# EUROPEAN PATENT SPECIFICATION

(11) **EP 1 384 788 B1**
(45) Date of publication and mention of the grant of the patent: **24.11.2010**
(21) Application number: 02708661.0
(22) Date of filing: 26.03.2002
(51) Int. Cl.: C12Q 1/68, C12N 15/09, G01N 33/15, G01N 33/50

(54) **Screening Method of measuring curative medicines for stomach cancer patients**
Screening-Verfahren zur Untersuchnung von Heilmitteln im Zusammenhang mit Magenkrebs
Procedé de selection d'un remed contre le cancer de l'estomac

(30) Priority: 10.04.2001 JP 2001111856; 04.09.2001 JP 2001267524
(43) Date of publication of application: 28.01.2004
(73) Proprietor: Ogoshi, Kyoji, Sagamihara-shi, Kanagawa 228-0802 (JP)
(72) Inventor: Ogoshi, Kyoji, Sagamihara-shi, Kanagawa 228-0802 (JP)
(74) Representative: Forstmeyer, Dietmar
(86) International application number: PCT/JP2002/002894
(87) International publication number: WO 2002/083947

(56) References cited:
- OZDEMIR E ET AL: "HLA-DRB1*0101 and *0405 as protective alleles in Japanese patients with renal cell carcinoma" CANCER RESEARCH, AMERICAN ASSOCIATION FOR CANCER RESEARCH, BALTIMORE, MD, US, vol. 57, no. 4, 1997, pages 742-746, XP002985932 ISSN: 0008-5472
- NAGAOKA YOSHIKO ET AL: "Ovine MHC Class II DRB1 alleles associated with resistance or susceptibility to development of bovine leukemia virus-induced ovine lymphoma" CANCER RESEARCH, vol. 59, no. 4, 15 February 1999 (1999-02-15), pages 975-981, XP002367590 ISSN: 0008-5472
- GHADERI A ET AL: "HLA-DRB 1 ALLELES AND THE SUSCEPTIBILITY OF IRANIAN PATIENTS WITH BREAST CANCER" PATHOLOGY ONCOLOGY RESEARCH, TUD. KIADO, BUDAPEST, HU, vol. 7, no. 1, 2001, pages 39-41, XP009004935 ISSN: 1219-4956
- KYOJI OGOSHI: 'Atarashii shuyo maker' JAPANESE JOURNAL OF CLINICAL MEDICINE vol. 59, no. SUPPL. 4, 30 April 2001, pages 513 - 520, XP002957153
- KYOJI OGOSHI ET AL.: 'HLA idenshi joho ni motozuita geka chiryo senryaku' JOURNAL OF JAPAN SURGICAL SOCIETY vol. 101, 2000, page 56, XP002957154
- OGOSHI K. ET AL.: 'HLA-A2 antigen status predicts metastasis and response to immunotherapy in gastric cancer' CANCER IMMUNOL. IMMUNOTHER. vol. 45, no. 1, 1997, pages 53 - 59, XP002952747
- KYOGO ITO ET AL.: 'Genome tayosei to kino kaiseki MHC-tagata to shikkan kanjusei HLA-tagata to gan chiryo yadonushi meneki kino o jushi sita gan chiryoho no kakuritsu ni mukete' MOL. MED. vol. 37, no. 5, 2000, pages 590 - 596, XP002957155
- KIM C.J. ET AL.: 'Immunodominance across HLA polymorphism: implications for cancer immunotherapy' J. IMMUNOTHER. vol. 21, no. 1, 1998, pages 1 - 16, XP002952748
- KYOJI OGOSE ET AL.: 'PSK no shokakigan (shokudo, I) ni okeru rinsho koka' JOURNAL OF JAPAN SURGICAL SOCIETY vol. 90, no. 9, pages 1443 - 1446, XP002957156

## Description

### Technical Field

The field relates to using identified amino acids at specific region of genes and corresponding base sequences as markers to screen for cancer curative medicines.

### Background of the Invention

When a gene characteristic that is controlled by a single locus has several phenotypes, which are genetically balanced, it is called a polymorphism. Each variable of the polymorphism is called an allele. A polymorphism is attributed not only by phenotype characteristics, or variety of amino acid sequences constructing proteins, but also by DNA base sequences where an amino acid sequence does not have varieties. In most cases, it is detected as cleavage positions of DNA, created by restriction enzymes, that differ from the others.

The HLA molecule of Human MHC molecule (major histocompatibility complex) was found as an antigen against the antibody that reacts in leukoagglutinin during serum treatment, in 1952. The HLA molecule is a gene product controlled by a gene cluster coded by the MHC region, within about 4000kbp on the 6^{th} chromosome short arm, 6p21.3. The MHC region includes the following 3 regions: 1) Class I gene region controlling HLA-A, B, and C and antigens, which are found on eucaryotic cell membranes, 2) Class II region controlling cell-specific HLA-DP, DQ, and DR antigens, which are found on particular tissues or cells, such as B-cell and macrophage, and 3) Class III gene region controlling complement ingredients and 21-hydroxyilaze.

The Class II molecule is a non-covalent cell membrane antigen made of glycoprotein of 34kDa (α-chain) and glycoprotein of 29kDa (β-chain). 7 pieces of α-chain gene and 9 to 12 pieces of β-chain (16 kinds) form clusters to construct a multigene family. On the Class II gene region, each gene lines up as DP-DN-DM-DO-DQ-DR from the centromere side. HLA-DP, DQ, and DR antigens include multiple alloantigens, and mainly the sequences of amino acids of the β-chain (B1) cause a polymorphism. DR and DQ antigens are epitope that react with antibodiesproduced from B-cells.

Each HLA molecule includes a form of domain construction with 260 to 370 amino acids. α1(β1), α2(β2) domain, compounded peptide (CP), TM, and CY regions construct the Class II molecule, and α1 and β1 domains configure polymorphism while α2 and β2 domains compose the base of the Class II molecule.

A genetic polymorphism of the HLA molecule is caused by different amino acid sequences coded by the corresponding HLA gene (Gene information regulating the amino acid sequences is written as the base sequence on DNA. A group of three bases, called a "Codon," is connected as one unit to form a single amino acid.). This is a reflection of an alloantigen with different base sequences, and currently most of the base sequences for alloantigens have been identified. (Tissue Antigen, 45, 258-280, 1995) Regions of polymorphism are found mainly in the α1 and α2 domains of the Class I molecule, and single common variable regions exist on each α1 C-terminal domain and α2 N-terminal domain. In the Class II molecule, the variable regions are found mainly in the α1 domain of the DQα-chain and the β1 domain of DRβ, DQβ, DPβ-chains. (Proc. Natl. Acad. Sci. USA, 84, 6234-6238, 1987). Substitution of the amino acid residue on the variable regions or differences in alloantigens have a direct effect on the affinity of HLA molecules against antigens. Substitution of the amino acid residue on the variable regions or differences in alloantigens also effects an affinity of TCR, which changes the ability of antigen presentations. The fact differentiates immune responses against an exogenous antigen and an autoantigen among individuals with diverse HLA antigens and can induce variety of immune responses.

Brief Description One object of this invention is to elucidate functions controlled by variations of the amino acids on particular positions of particular regions on the HLA genes and the base sequences and to provide usages of the functions in medical field.

This invention has clarified the relationship of particular positions of the amino acids and base sequences and cancer by analysis of clinical phenomenon of cancer patients based on analysis of polymorphisms of the HLA gene.

The following method is provided:
1. A screening method to determined effective stomach cancer curative medicines, wherein amino acid variations of DQB1*gene are used as a marker, and wherein said amino acid variations are at positions 57 and 67 of the amino acid of HLA DQB1*gene, and wherein methods for treatment of the human or animal body by surgery or therapy and diagnostic methods practised on the human or animal body are excluded.
2. The method according to topic 1, wherein said effective cancer treatment medicines are immunological medicines, with the proviso that Asp is encoded at position 57 and Val is encoded at position 67 of the HLA DQB1*gene.

### Brief Description of the Drawings

Fig. 1: Base sequence at Position 57 and 67 of DQB1*gene cluster and corresponding amino acids;
Fig. 2: Result of the stomach cancer resection alone in patients a) with DQB1*05031 gene (Asp at Position 57, Val at Position 67) and b) without DQB1*05031 gene;
Fig. 3: Result of the anticancer chemotherapy after the stomach cancer resection in patients a) with DQB1*05031 gene (Asp at Position 57, Val at Position 67) and b) without DQB1*05031 gene;
Fig. 4: Result of the anticancer immunotherapy after the stomach cancer resection in patients in patients a) with DQB1*05031 gene (Asp at Position 57, Val at Position (7) and b) without DQB1*05031 gene;
Fig. 5: Result of PHA stimulating test in patients with the presence of DQB1*05031;
Fig. 6: Graph showing effectiveness of immunotherapy in patients with GUA or GUG at Position 27 (Val) of the amino acid sequence of DQB1*gene cluster of HLA Class II. The vertical axis shows accumulated survival rate, while the cross axis shows survived days;
Fig. 7: Graph showing effectiveness of immunotherapy in patients with CUG or UUG at Position 91 (Leu) of the amino acid sequence of DQB1*gene cluster of HLA Class II. The vertical axis shows accumulated survival rate, while the lateral axis shows survived days;
Fig. 8: Graph showing effectiveness of immunotherapy in patients with AAA or AAG at Position 12 (Lys) of the amino acid sequence of DQB1*gene cluster of HLA Class II. The vertical axis shows accumulated survival rate, while the cross axis shows survived days;
Fig. 9: Result of the treatment effects in all cancer cases and the variations of the amino acid sequences at Position 9 of DQB1*;
Fig. 10: Result of the treatment effects in all cancer cases and the variations of the amino acid sequences at Position 67 of DQB1*;
Fig. 11: Result of the treatment effects in stomach cancer cases and the variations of the amino acid sequences at Position 9 of DQB1*;
Fig. 12: Result of the treatment effects in stomach cancer cases and the variations of the amino acid sequences at Position 67 of DQB1*;
Fig. 13: Result of the treatment effects in stomach cancer cases and the variations of the amino acid sequences at Position 74 of DQB1*;
Fig. 14: Table showing effectiveness of the cancer resection alone (no adjuvant therapy) in Working Example 7;
Fig. 15: Table showing effectiveness of the anticancer chemotherapy after the cancer resection (Chemotherapy) in Working Example 7;
Fig. 16: Table showing effectiveness of the anticancer immunotherapy after cancer resection (Immunotherapy) in Working Example 7;
Fig. 17: Analysis of DQB1*gene equivalence (1) in Working Example 8;
Fig. 18: Analysis of DQB1*gene equivalence (2) in Working Example 8;
Fig. 19: Prognosis and treatment effect in patients with DQB1*gene (1) in Working Example 9;
Fig. 20: Prognosis and treatment effect in patients with DQB1*gene (2) in Working Example 9;
Fig. 21: Prognosis and treatment effect in patients with DQB1*gene (3) in Working Example 9;
Fig. 22: Prognosis and treatment effect in patients with DQB1*gene (4) in Working Example 9;
Fig. 23: Prognosis and treatment effect in patients with DQB1*gene (5) in Working Example 9;
Fig. 24: Prognosis and treatment effect in patients with DQB1*gene (6) in Working Example 9;
Fig. 25: Prognosis and treatment effect in patients with DQB1*gene (7) in Working Example 9;
Fig. 26: Prognosis and treatment effect in patients with DQB1*gene (8) in Working Example 9;
Fig. 27: Prognosis and treatment effect in patients with DQB1*gene (9) in Working Example 9;
Fig. 28: Base sequence analysis in patients with DQB1*gene (1) in Working Example 10;
Fig. 29: Base sequence analysis in patients with DQB1*gene (2) in Working Example 10;
Fig. 30: Base sequence analysis in patients with DQB1*gene (3) in Working Example 10;
Fig. 31: Base sequence analysis in patients with DQB1*gene (4) in Working Example 10;
Fig. 32: Optimum amino acid sequence in patients with DQB1*gene (1) in Working Example 11;
Fig. 33: Optimum amino acid sequence in patients with DQB1*gene (2) in Working Example 11;
Fig. 34: Optimum amino acid sequence inpatients with DQB1*gene (3) in Working Example 11;
Fig. 35: Optimum amino acid sequence in patients with DQB1*gene (4) in Working Example 11;
Fig. 36: Optimum amino acid sequence in patients with DQB1*gene (5) in Working Example 11;
Fig. 37: Relationship between DQB1*gene and cancer metastases (1) in Working Example 12;
Fig. 38: Relationship between DQB1*gene and cancer metastases (2) in Working Example 12;
Fig. 39: Relationship between DQB1*gene and cancer metastases (3) in Working Example 12;
Fig. 40: Relationship between DQB1*gene and cancer metastases (4) in Working Example 12;
Fig. 41: Relationship between DQB1*gene and cancer metastases (5) in Working Example 12;
Fig. 42: Relationship between DQB1*gene and tumor advancement (1) in Working Example 12;
Fig. 43: Relationship between DQB1*gene and tumor advancement (2) in Working Example 12;
Fig. 44: Relationship between DQB1*gene and tumor advancement (3) in Working Example 12;
Fig. 45: Relationship between DQB1*gene and tumor advancement (4) in Working Example 12;
Fig: 46: Relationship between DQB1*gene and tumor advancement (5) in Working Example 12;

### [Legend for Symbols and Marks]

Fig. 1
   A, D, V, S, I: Single character codes for the amino acid
Fig. 2
   a: patients without DQRB1*05031 gene
   b: patients with DQRB1*05031 gene (Asp at Position 57, Val at Position 67)
Fig. 3
   a: patients without DQRB1*05031 gene
   b: patients with DQRB1*05031 gene (Asp at Position 57, Val at Position 67)
Fig. 4
   a: patients without DQRB1*05031 gene
   b: patients with DQRB1*05031 gene (Asp at Position 57, Val at Position 67)
Fig. 5
   a: patients with neither Asp at Position 57 nor Val at Position 67 (DQ B 1 *05031 (-))
   b: patients with Asp at Position 57 and Val at Position 67 (DQB 1 *05031 (+))
      PSK: I-1 Group
      Different OK: II-2 Group
      Different PSK: I-3 Group
      Same Mix: II-1 Group
      Same Mix2: II-4 Group
      Same OK: II-3 Group
      Same PSK: I-2 Group
Fig. 6
   B: Heterozygote of vGUA and vGUG
   G: Homozygote of vGUG
   R: Homozygote of vGUA
Fig. 7
   B: Heterozygote of 1 CUG and 1 UUG
   G: Homozygote of 1 UUG
   R: Homozygote of 1 CUG
Fig. 8
   B: Heterozygote of kAAG and kAAA
      G: Homozygote of kAAG
      R: Homozygote of kAAA
Fig. 27-75, 85-129 9-27, 32-46
   Upper case letters shown on the Figures are single character codes of the amino acids
Fig. 28-31
   Lower case letters shown on Figures are single character codes of the amino acids

### Detailed Description

Gene specified in this invention is DQB1*gene of HLA. Variations of the amino acids coded on the diversity positions of this gene have important meanings. Variations of such amino acids effect interactions with several amino acids. Variations of the amino acids can be used as a marker for screening of effective cancer curative medicines.
1. Positions of note of the amino acid sequences of DQB1*gene of HLA Class II are as follows: -21, (-9), -6, -5, -4, 3, 9, 14, (19), 23, (26), 30, 37, 38, 45, 53, 55, 56, 57, 66, 67, 70, 71, 74, 77, 84, (85), 86, 87, (89), (90), (116), 125, 130, 140, 182, 197, 220, 221, and 224. Bracketed numbers have 2^{nd} level of importance or to tend to have predominance over others.
2. Amino acid variations in the amino acid sequences of DQB1*gene at Position 3, 14, 19, 26, 30, 66, 67, 71, 77, 87, 116, 125, 185, 203, and 224 have functions to restrict and control the metastases of cancer cells. Especially, the variations such as (LM: single character codes of the amino acid) and (LL) at Position 14, (GLY) at Position 26, (DE) at Position 66, (IV) at Position 67, (RT) and (RR) at Position 67, (FLY) and (YY) at Position 87, (LV) at Position 116, (SS) at Position 125, (IT) at Position 185, (IV) at Position 203, and (RR) at Position 224 indicate significant tendency. The term "significant tendency" means, for example, that experimental results indicate a stronger than average positive correlation between the amino acid sequence position number and functions to restrict and control the metastases of cancer cells.
3. Amino acid variations in the amino acid sequences of DQB1*gene at Position 5, 9, 30, 57, 66, 67, 86, 87, and 130 are found to have important relationship with the immunotherapy. Especially, the variations such as (PP) at Position 5, (LY) and (YY) at Position 9, (HSY) and (HY) at Position 30, (AA) at Position 57, (EE) and (DE) at Position 66, (VV) and (IV) at Position 67, (EG) at Position 86, (LY) at Position 87, and (QR) at Position 130 indicate significant tendency. The terms "important relationship" and "significant tendency" mean, for example, that the noted amino acid positions have a positive correlation with effective immunotherapy and that experimental results indicate a stronger than average positive correlation between the amino acid sequence position number and functions to restrict and control the metastases of cancer cells, respectively. Furthermore, the terms "the same," "different," "significantly different," "longer," "shorter," or the like, refer to the statistical probability that the values of the items being compared or referred to are the same, different, significantly different, etc.
4. Amino acid variations in the amino acid sequences of DQB1*gene at Position (-5), 9, 30, 37, 38, 66, 67, 86, 87, and 130 are found to have important relationship with the chemotherapy. Especially, the variations such as (PP) at Position -5, (LY) and (YY) at Position 9, (HY) at Position 30, (DY) at Position 37, (AV) at Position 38, (DE) and (EE) at Position 66, (IV) and (VV) at Position 67, (EG) at Position 86, (LY) at Position 87, and (QR) and (RR) at Position 130 indicate significant tendency. The terms "important relationship" and "significant tendency" mean, for example, that the noted amino acid positions have a positive correlation with effective immunotherapy and that experimental results indicate a stronger than average positive correlation between the amino acid sequence position number and functions to restrict and control the metastases of cancer cells, respectively.

The method provided teaches easy screening of stomach cancer curative medicines by inspecting interactions with the above amino acids positions. Methods for drug-designing by comparison of three-dimensional structures of the candidate compounds, based on the three-dimensional structures and each amino acid's positions and variations, are provided. Effectiveness of the stomach cancer treatment medications can be measured by selecting from the conditions which allow interaction with three-dimensional structures by positions and variations of each amino acid with the candidate compounds, estimating the interaction, and detecting signals from interaction.

New compounds identified from the above information and methods should be effective stomach cancer medicines. The term "effective" means that experimental data indicates a positive correlation exists between a cancer medicine and reduction or lack of growth of cancer cells and/or tumors or reduction in the rate of cancer metastases. Medicines for anti-metastases can contain the compounds according to one of topics 1 or 2. Medicines for immunotherapy can contain the compounds according to one of topics 1 or 2. Medicines used for chemotherapy can contain the compounds according to topic 1.

Information is provided about gene variations suitable for effective stomach cancer treatments. The information relating to the relationship, or statistical correlation, between amino acid positions and variations provides methods of measuring the significance, or effectiveness, of anticancer treatments. For example, examining the genes or the amino acid variations coded by the genes of patients enables the estimation of the rate of metastases of cancer cells and the effectiveness of the immunotherapy, the chemotherapy, or the cancer resection, alone. When using at least one of the below amino acid variations of DQB1*gene of HLA as a marker, it is possible to provide statistically significant or statistically meaningful examination methods.
1) Positions of the amino acid sequences of HLA Class II, DQB1*gene: -21, -6, -5, -4, 3, 9, 14, (19), 23, 30, 37, 38, 45, 53, 55, 56, 57, 66, 67, 70, 71, 74, 77, 84, (85), 86, 87, (89, 90, 116), 125, 130, 140, 182, 197, 220, 221, and 224.
2) Base variations of HLA Class II, DQB1*gene: CCU and CCC at Position -23, CCU and CCC at Position -15, AAC and AAU at Position 19, ACG and ACC at Position 21, GUA or GUG at Position 27 (Val), GCA and GCG at Position 38, AAC and AAU at Position 62, CGG and CGA at Position 72, ACC, ACG and AGA at Position 77, GUA and GUG at Position 78, CUG and UUG at Position 91 (Leu), GAC and GAU at Position 135, GCC, GCU, ACC and ACU at Position 140, GAC and GAU at Position 169, CUC and CUG at Position 210, CUC and CUU at Position 213, and CUU and CUG at Position 215.

Additionally, reagent kits to measure diversity of the amino acids or the base sequences of these specified genes can be provided. Clinical measuring reagents which estimate the results of the treatments accurately can be provided.

The following are the details of clinical results of the invention. It is, however, not limited to the reported cases only.
Methods used herein are as follows:
1) Genetic polymorphisms are based on the public literature. (WHO HLA Nomenclature Committee For Factors of the HLA system, IMGT/HLA Sequence Database, http://www.ebi.ac.uk/imgt/HLA/algn.html, Tissue Antigens, 1998;51:417-466)
2) Clinical experiments, including 344 patients with the cancer resection alone, 394 patients with anticancer chemotherapy after the cancer resection (therapy: fluoropyrimidines such as 5-FU, mitomycin or adriamycin), and 241 patients with immunotherapy after the cancer resection (therapy: immuneopotentiator such as PSK or OK432).
3) Standard methods were used for collecting genes from the patients, identifying genes, and specifying diverse amino acids and base sequences. (MCH & IRS, Supplement Vol.1 73-95, 1994. Tissue Antigens 39:187-202, 1992. 38 ;53-59,1991, 38 :60-71,1991, 40 ;100-103,1992). Analyses positions were from -32 to 237th on DQB1*. .
4) Metastases of all kinds of cancers included 1649 cases, of which 504 cases had metastases and 1145 cases did not have metastases. "Metastases" as used herein refer to lymph node metastases and remote metastases.
5) Analysis of influence of the variations of the amino acids on metastases and the treatment was performed as followings: after a provision of the treatments described at 2), the follow-up research of the patients was conducted for about 10 years, and the statistical analysis of the mortality rate was carried out. The amino acid positions which are distinguishable with a statistically significant difference by the amino acid types (types of amino acids: heterozygote or homozygote) have been identified for each given treatment (the cancer resection alone, anticancer chemotherapy after cancer resection, and anticancer immunotherapy after cancer resection). In the summary tables, the results are organized by the types of amino acids, effect on metastases, and the treatment effect at each amino acid position.

### [Clinical Cases]

### [Results of Clinical Cases]

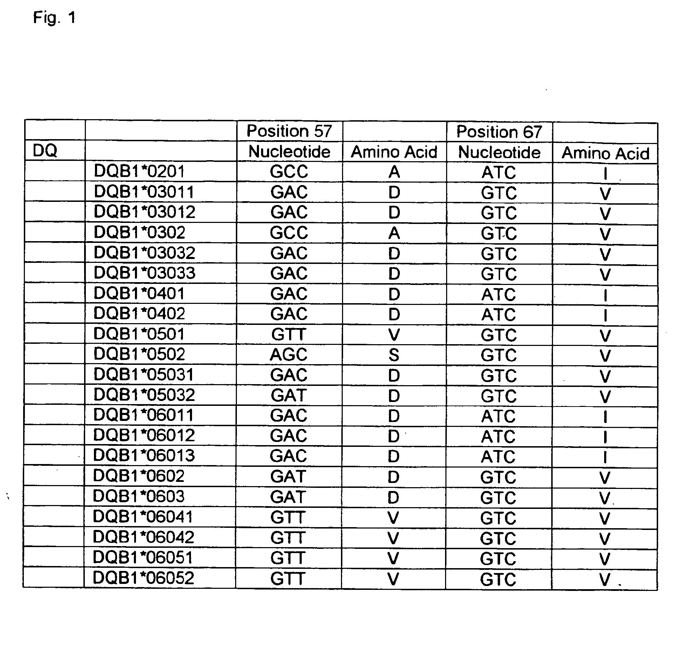

Fig. 1: Table shows the base sequence of DQB1*gene and corresponding amino acids to analyze polymorphic amino acids at Position 57 and 67. As a result, Asp, Ala, Ser, and Val are found at Position 57 while Ile and Val are found at Position 67.

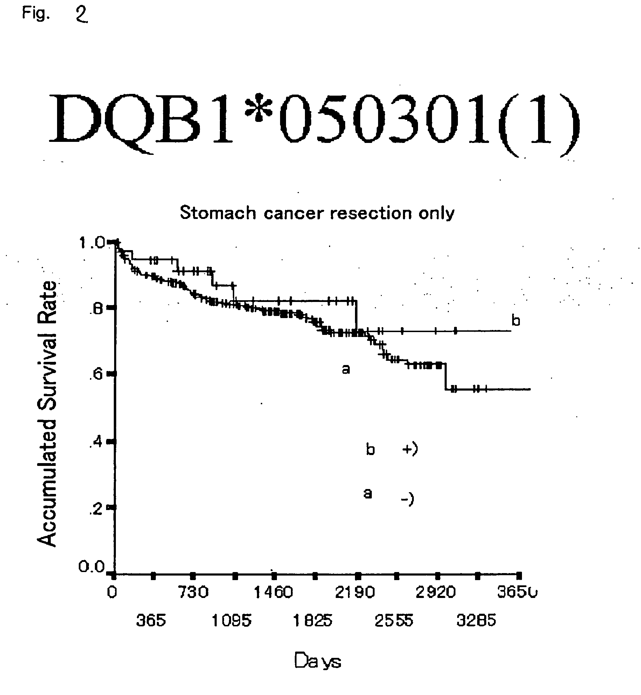

Fig. 2 shows the accumulated survival curvets in patients with DQB1*05031 gene (Asp at Position 57, Val at Position 67) ("b" group) and the "a" group without DQB1* 05031 gene among patients with DQB1*gene cluster with the stomach cancer resection alone. The vertical axis indicates the accumulated survival rate (Kaplan-Meier Method) (1.0 = 100% survived) while the lateral axis shows the number of survived days. As the result, there is only a slight difference at the 1825^{th} day (5^{th} year) between the 2 groups, but shows a good survival rate at the 7^{th} and 8^{th} year for the "b" group with (+) (the patient group with DQB1*05031 gene). Thus, it can be concluded that the patients with DQB1*05031 gene (Asp at Position 57, Val at Position 67) have slightly better survival rate after stomach cancer resection. (DQB1*05031(-)(n=306), (+) (n=38)

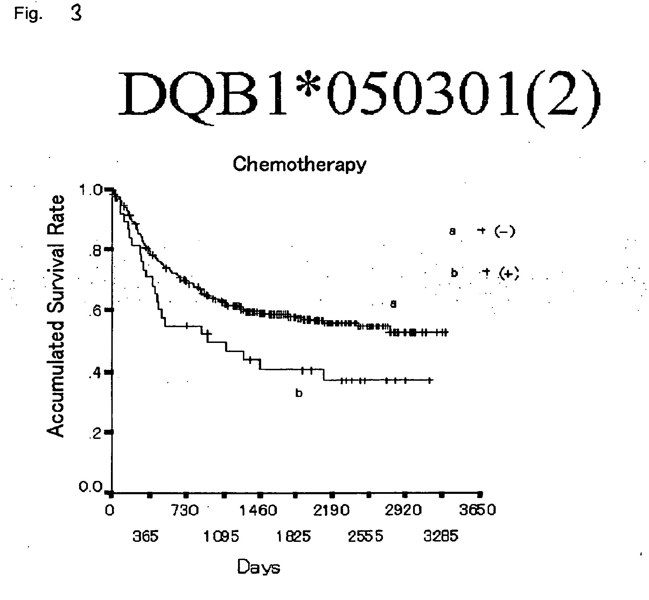

Fig. 3 shows the accumulated survival curves in patients with DQB*05031 gene ("b" group) (Asp at Position 57, Val at Position 67) and the "a" group without DQB1*gene among patients with DQB1*gene cluster with anticancer chemotherapy after cancer resection. Medications used for anticancer chemotherapy in this report are treatments with prescription anticancer chemicals well known in the clinical field, such as, for example, 5-FU, Adriamycin, and others. As clearly shown in the figure, the (+) (b) patient group is not suitable for the chemotherapy. Thus, if verifying existence of DQB1*05031 gene (Asp at Position 57, Val at Position 67) by gene examination before beginning the treatments, prescribing such chemical treatments to those patients can be avoided. In contrast, the chemotherapy is suitable for the patients without DQB1*05031 gene (Asp at Position 67, other than Val at Position 67). Moreover, if the effective examination of the anticancer chemotherapy is given to these (-) patients, the effectiveness rate would improve drastically (DQB1*05031(-) (n=356), (+) (n=38))

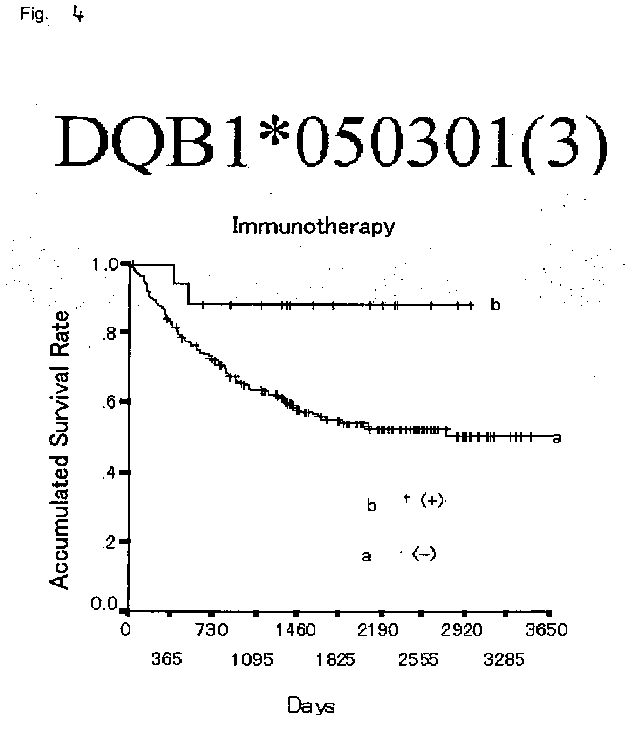

Fig. 4 shows the accumulated survival curves in patients with DQB*05031 gene ("b" group) (Asp at Position 57, Val at Position 67) and the "a" group without DQB1*gene among patients with DQB1*gene with anticancer immunotherapy after cancer resection. Medications used for anticancer immunotherapy in this report are treatments with prescription anticancer immune materials well known in the clinical field, such as, for example, krestin (PSK), OK432, and others. The figure clearly shows that the survival rate of the (+) (b) patient group is statistically longer (log rank test p<0.05) than that of the (-) (a) patient group without such gene. Five year-survival rates were 90% and 50% in patients with positive and negative DQB*05031 gene, respectively. Thus, if patients could be confirmed as not having DQB1*05031 gene (Asp at Position 57, other than Val at Position 67), the immunotherapy is not a recommended treatment for them. If the immunotherapy treatment is given only to the (+) patients or excluding the (-) patients, the effectiveness rate would improve. (DQB1*05031(-) (n=233), (+) (n=18))

From Figs. 3 and 4, it is clear that the (+) patients and the (-) patients have opposite outcomes from the anticancer treatments. It is possible to select patients who respond positively to therapies by using this gene as a marker and to provide the most appropriate treatments to those patients. In other words, "order-made" treatment is possible.

The efficacy of medicines for anticancer immunotherapy is such that immunotherapy treatment should be carried out in patients without Ile at Position 67 but with Asp at Position 57.

The above analyses show that effective treatment for cancer patients can be dependent upon the amino acids that are in Position 57 and 67 on DQB*1gene. It is possible to choose the appropriate therapy and to provide appropriate medications after resections (e.g., surgery) by identifying and recognizing the amino acids of the patients. Also, this invention demonstrates that the amino acids in Position 57 and 67 on DQB*1 gene can be used as markers to select patients who respond to anticancer therapy, thereby enhancing treatment efficacy.

### [Experimental results]

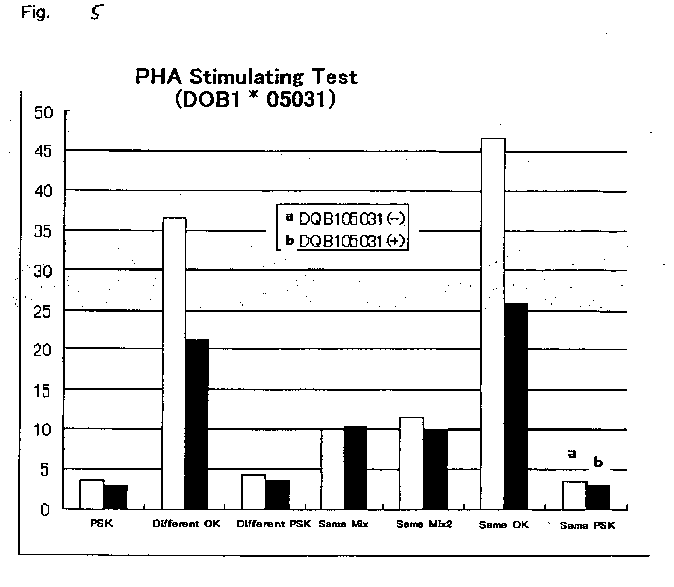

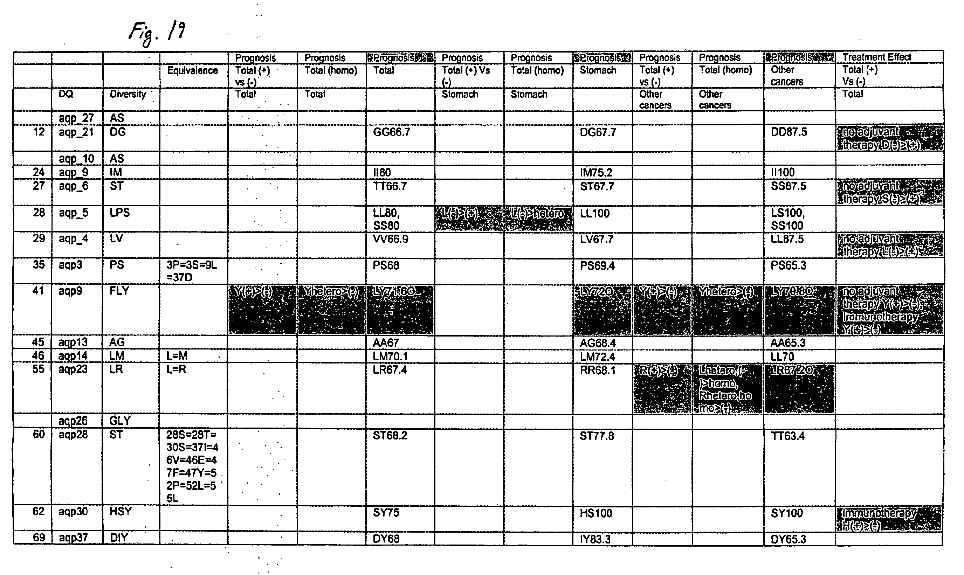

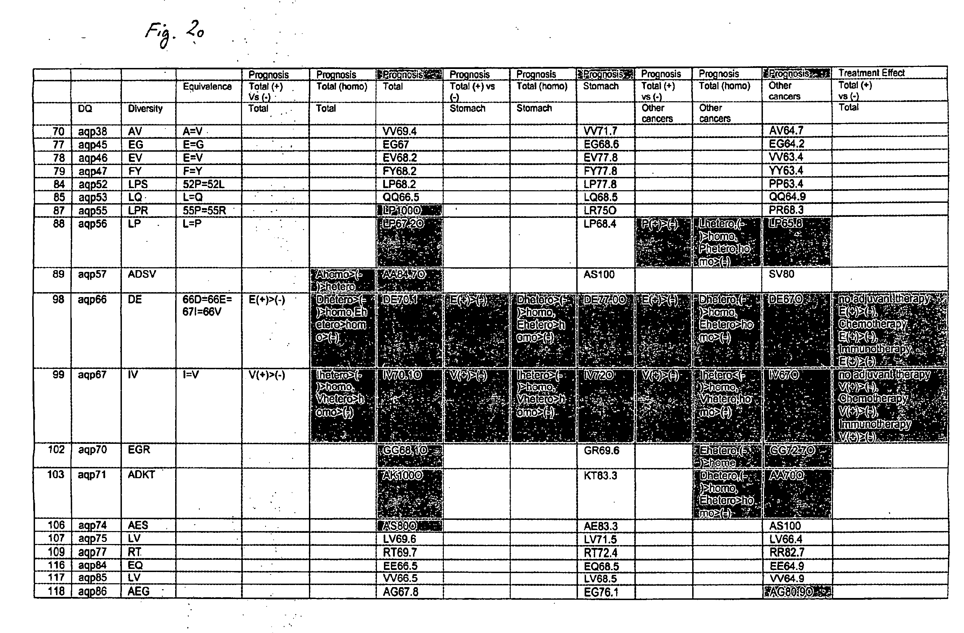

The phytohemagglutinin (PHA) Stimulating Test (lymphocyte proliferation reaction in stomach cancer cases) was performed as follows. Results are shown in Figure 5. Stimulation index was calculated as follows; the Ficollo-Conray gravity centrifugation was used to separate lymphocytes from peripheral blood with heparin. RPMI-1640 was added to adjust to 6.0 x 10⁶/ml. It was separated into 0.1ml/well on a 96-hole U-base microplate (corning #2850) for the stimulating test. The I Group was added with PSK (1mg/ml, 0.1ml/well), the II Group was added with OK-432 (1/200 KE/ml, 0.1ml/well), and the III Group was on medium only. The I Group was separated into 3 aliquots and tested: the I-1 Group (indicated as PSK on Figure 19 and 20) was incubated for 5 days, the I-2 Group (indicated as Same PSK on Figures) for 2 more days of incubation upon adding 0.1mg/well of PSK after the first 3-day incubation, and the I-3 Group (indicated as Different PSK on Figures) for 2 more days of incubation upon adding 0.005 KE/well of OK432 after the first 3-day incubation. Similarly, the II Group was subdivided and classified into the II-1 Group (indicated as Same Mix), the II-2 Group (indicated as Different OK), and the II-3 Group (indicated as Same OK).

Among this group, a double amount of OK432 was added to the II-4 Group (indicated as Mix2). The III Group was incubated for 5 days.

Then, 1 micro Ci/well of ³H-thymidine was added to the samples and incubated for 24 hours to measure lymphocytes on the harvest scintillation counter.

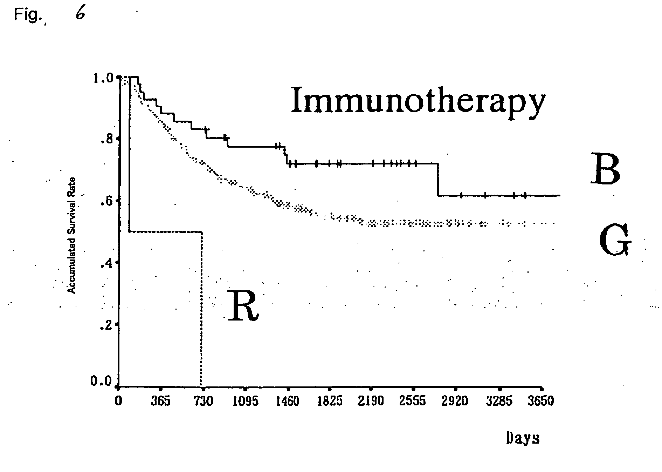

Fig. 5 shows the result of the PHA stimulating test in patients with/without Asp at Position 57 and Val at Position 67 of DQB*1 gene (i.e. DQB1*05031 patients). The vertical axis is for SI and lateral axis is for the methods used for stimulation. The left side of the coupled bars is the a Group and the right side represents the b Group. Similar reactions to the stimulus are noted. The patients with Asp at Position 57 but not Val at Position 67 (a Group) are more immune responsive than the patients with Asp at Position 57 and Val at Position 67 (b Group). The results from this experiment and the fact that the anticancer immunotherapy is effective to the patient with Asp at Position 57 and Val at Position 67 (referring to Figure 6) proves that anticancer immunotherapy is not effective on the patients with high immune response.

### [Working Example 1]

Table 1 shows statistical analysis of the polymorphic amino acids on DQB1*gene. It shows the results of the amino acid variations at Position 3, 14, 19, 26, 30, 38, 53, 57, 66, 67, 77, 85, 86, 87, 89, 90, 116, 125, 140, 182, 185, 203, 220, and 221, the effectiveness of the anticancer immunotherapy and the anticancer chemotherapy, the metastases (total), lymph node metastases, and remote metastases. Letters in the brackets shown in the position of the amino acid columns represent single character codes of the amino acids which may be combined. For example, Position 30 (HSY) means that amino acids may be H, S, or Y. "H" (with the same H for the complementary amino acid) in the immunotherapy of (HSY) column means that there is a tendency for immunotherapy to be effective on patients with H at that position, while "HY hetero•" means that there is a statistically significant tendency for Immunotherapy to be effective on patients with H and Y, different kinds of the amino acids variation. "hetero" is an abbreviation of "heterozygote," and marked with "•" means that there is a statistically significant tendency of the figures. In addition, "Y homo•" in the column indicates that there is a statistically significant tendency for Immunotherapy to be effective on patients with Y at Position 30 (with the same Y for the complementary amino acid. "homo" is an abbreviation of "homozygote").

"AV hetero •" in the 38 (AV) row and in the Chemotherapy column shows that there is a statistically significant tendency for Chemotherapy to be effective on patients with A at Position 38 and V for the complementary amino acid. "V homo" in the Chemotherapy column shows that there is a statistically significant tendency for anticancer chemotherapy to be effective on patients with V at Position 38 (with the same amino acid for the complementary amino acid).

"V hetero •" in the 57(ADSV) row and in the Immunotherapy column shows that there is a statistically significant tendency for anticancer immunotherapy to be effective on patients with V at Position 57 and A, D, or S for the complementary amino acids. Hereafter, the meaning of each gene is shown by same relation.

"PS 34.2" in the 3 (PS) row and in the Metastases (total) column shows that the complementary amino acids are P and S, and the rate of metastases is 34.2% in total and is increasing. "PP 26.8" means that the complementary of the amino acids are same P and P, and the rate of metastases is 26.8% in total on a decreasing trend. Additionally, the • mark represents statistical significance in the figures.

The results show that cancer metastases has statistically significant relationship with Positions 3, 14, 19, 26, 30, 77, 87, 116, 125, and 203 of DQB1*gene, and either homozygotic or heterozygotic type of the complementary amino acids at corresponding positions has important effects on the metastases. It is likely to have cancer metastases in patients with heterozygotes at Position 3 and 19 and homozygotes at Position 14, 26, 30, 77, 87, 116, 125, and 203. In particular, LL homozygote at Position 14 and 26, RR homozygote at Position 77, YY homozygote at Position 87, II homozygote at Position 116, SS homozygote at Position 125, and VV homozygote at Position 203 shows statistically significant differences of the effect. Therefore, the possibility of providing the means of suppressing cancer metastases using these amino acids as a marker is shown.

There is a statistically significant relationship between the effectiveness of Immunotherapy and Position 30, 57, 66, 67, 85, 86, and 87 of DQB1*gene. Especially, Y homozygote and heterozygote at Position 30, H heterozygote on Position 30, V heterozygote on Position 57 have statistically significant results. Position 38, 66, 67, 86, and 87 has a statistically significant relationship with the Chemotherapy. Especially, A heterozygote and V heterozygote on Position 38, D heterozygote and E heterozygote on Position 66, I heterozygote and V heterozygote on Position 67, A homozygote on Position 86, and F homozygote on Position 87 shows statistically significant results. Therefore, the possibility of providing the means of the anticancer chemotherapy and the anticancer immunotherapy in cancer treatment using these amino acids as a marker is shown.

Differences between the Working Examples and the statistical analysis report can be explained as follows:

The survival rates were examined when applying the priority demand and 1 year after. All cancers were treated as one group on applying the priority demand, but it was separated into the stomach cancer cases and the other cancer cases in this experiment. Since all positions of cancer cases published at present were examined for this report, there was some difference in the result. Moreover, since the lymph node metastases and the metastases of remote organs (liver, lungs and others) were divided and examined on the priority demands for the metastases, there was some difference.

### [Working Example 4]

It is confirmed that the differences in the base sequence on genes affect the effectiveness of anticancer treatments by the following statistics processing. The corresponding base sequences are GUA or GUG of the base sequences at Position 27 (Val) on DQB1 gene of HLA Class II, CUG or UUG of the base sequences at Position 91 (Leu) on DQB1 gene of HLA Class II, and AAA or AAG of the base sequences at Position 12 (Lys), and UAC or UAU at Position 78 (Tyr).

Fig. 6 shows that GUA or GUG at Position 27 (Val) of the base sequences on DQB1*gene has a statistically significant relationship with the response to immunotherapy. Immunotherapy is not effective for vGUG homozygote but is for vGUG homozygote or vGUA and vGUG heterozygote. The result means that the patients who respond to immunotherapy can be determined by measuring the base sequence of Position 27 (Val) on DQB1*gene.

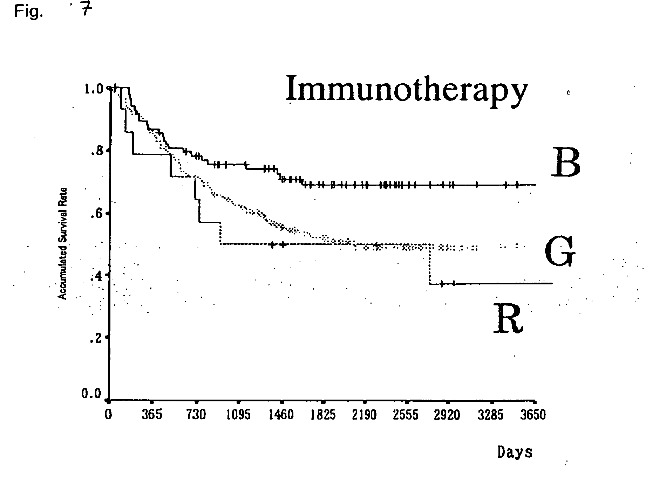

Fig. 7 shows that CUG or UUG at Position 91 (Leu) of the base sequences on DQB1*gene has a statistically significant relationship with the response to immunotherapy. Immunotherapy is not effective for 1UUG homozygote or 1CUG homozygote but is for 1CUG and 1UUG heterozygote. As a result, it can be predicted which patients respond to immunotherapy by examining the base sequence of Position 91 (Leu) on DQB1*gene.

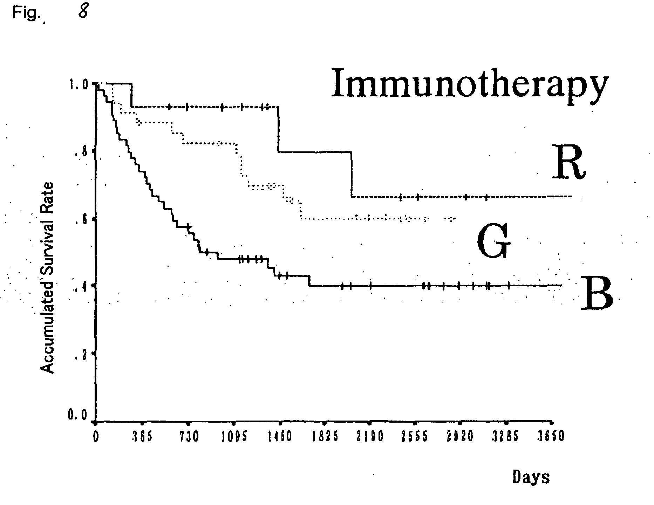

Fig. 8 shows that AAG or AAA at Position 12 (Lys) of the base sequence on DRB1*gene has a statistically significant relationship with the response to Immunotherapy with a significant difference. Immunotherapy is effective on kAAA homozygote or kAAG homozygote but not for kAAA and kAAG heterozygote. The result means that the patients who respond to immunotherapy can be determined by examining the base sequence of Position 12 (Lys) on DRB1*gene.

### [Working Example 5]

### (Relationship between polymorphism and treatment in the specified positions)

With respect to the variations of the amino acids of the specific part of the polymorphic amino acids of DQB1* gene in all cases, the variations of the amino acids and the medical treatment effect of treatments [The cancer resection alone (no adjuvant therapy), anticancer chemotherapy after the cancer resection (Chemotherapy), and anticancer immunotherapy after the cancer resection (Immunotherapy)] were confirmed. (Graphs include the survival rate for the vertical axis (1.0 = 100%) and survived days for the lateral axis.) The base data was collected from the above clinical cases.

### 1) DQ9 (All cases)

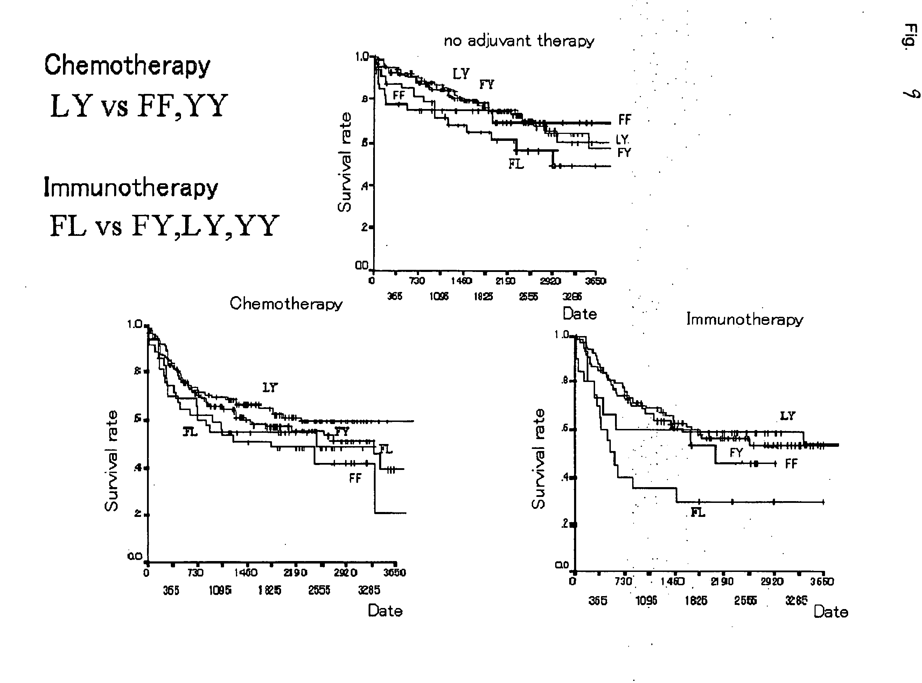

The remarkable point of Fig. 9 is that the immunotherapy after the cancer resection (Immunotherapy) is not suitable for patients with the variation FL (Single character code of the amino acid) at Position 9 of the amino acid sequence on DQB1*gene.

### 2) DQ67 (All cases)

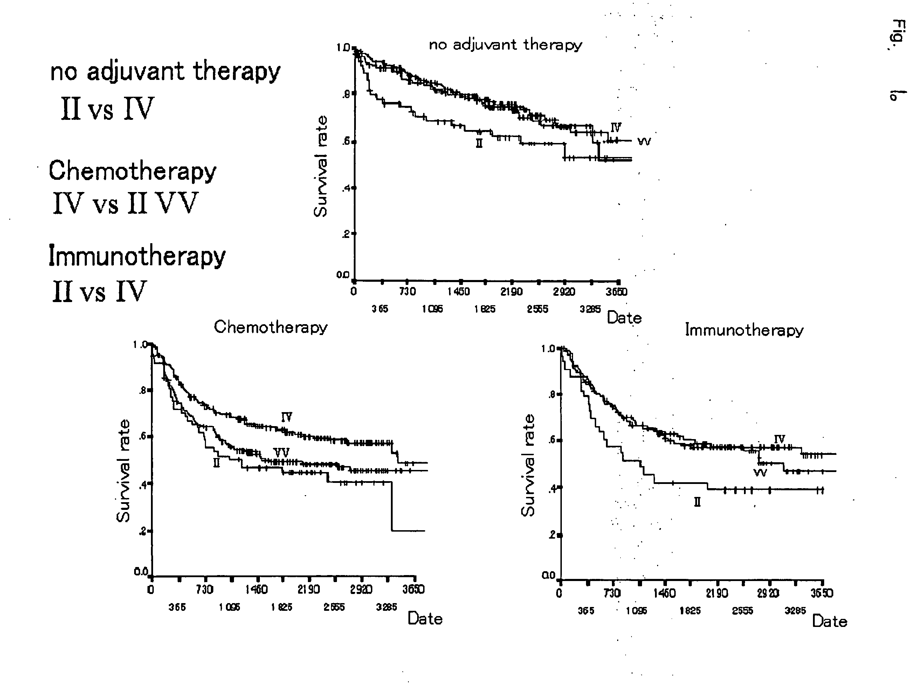

Fig. 10 shows that none of the treatments [The cancer resection alone (no adjuvant therapy), anticancer chemotherapy after the cancer resection (Chemotherapy), and anticancer immunotherapy after the cancer resection (Immunotherapy)] is suitable for the patients with II at Position 67 of the amino acid sequence on DQB1*gene.

### [Working Example 6]

### (Relationship analysis among polymorphisms, treatment and the specified positions on each gene in stomach cancer patients)

With respect to the variations of the amino acids of the specific part of the polymorphic amino acids of DQB1* gene in stomach cancer cases, the variations of the amino acids and the medical effect of treatments [The cancer resection alone (no adjuvant therapy), anticancer chemotherapy after the cancer resection (Chemotherapy), and anticancer immunotherapy after the cancer resection (Immunotherapy)] were confirmed. (Graphs include the survival rate for the vertical axis (1.0 = 100%) and survived days for the lateral axis.) The base data was collected from the previous clinical cases.

### 1) DQ9 (Stomach cancer)

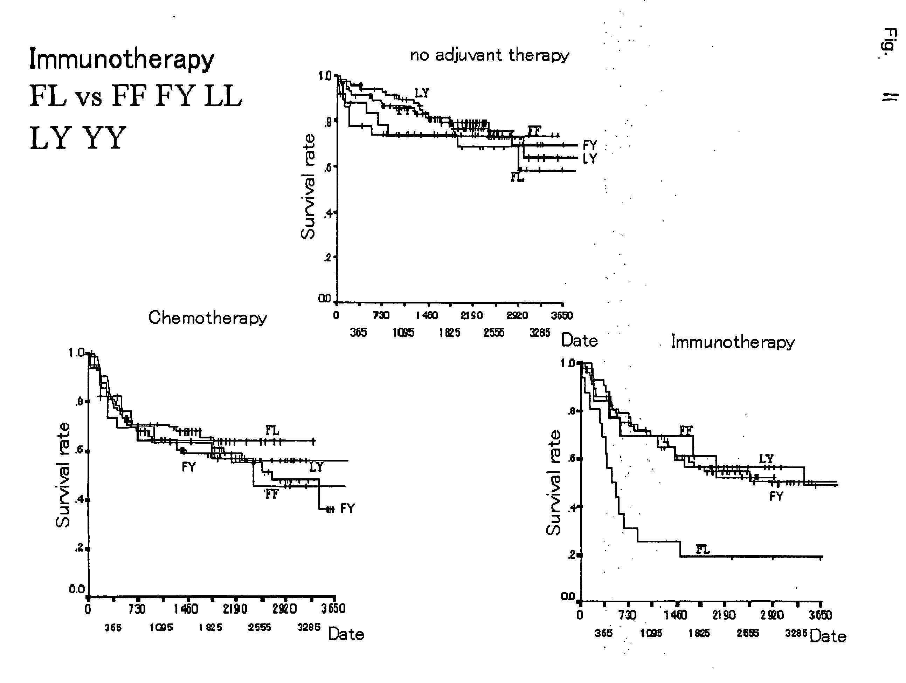

Fig. 11 shows that the patients with the anticancer immunotherapy after the cancer resection (Immunotherapy) is not suitable for patients with FL at Position 9 of the amino acid sequence on DQB1*gene.

### 2) DQ67 (Stomach cancer)

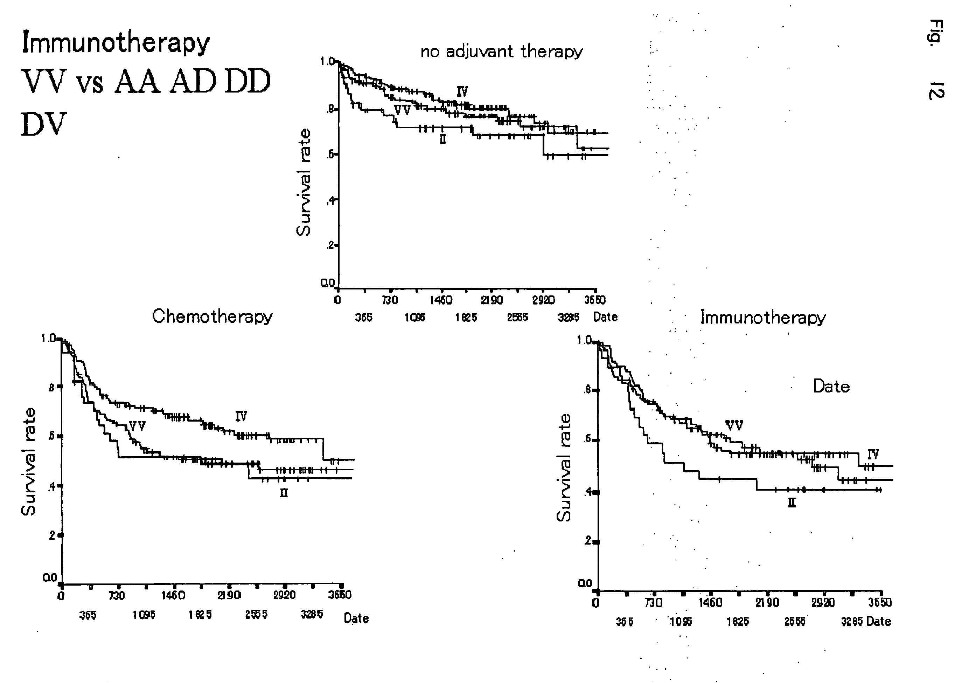

Fig. 12 shows that the anticancer immunotherapy after the cancer resection (Immunotherapy) is not suitable for patients with II at Position 67 of the amino acid sequence on DQB1*gene. The anticancer chemotherapy after the cancer resection (Chemotherapy) IV is suitable for patients with II at Position 6.

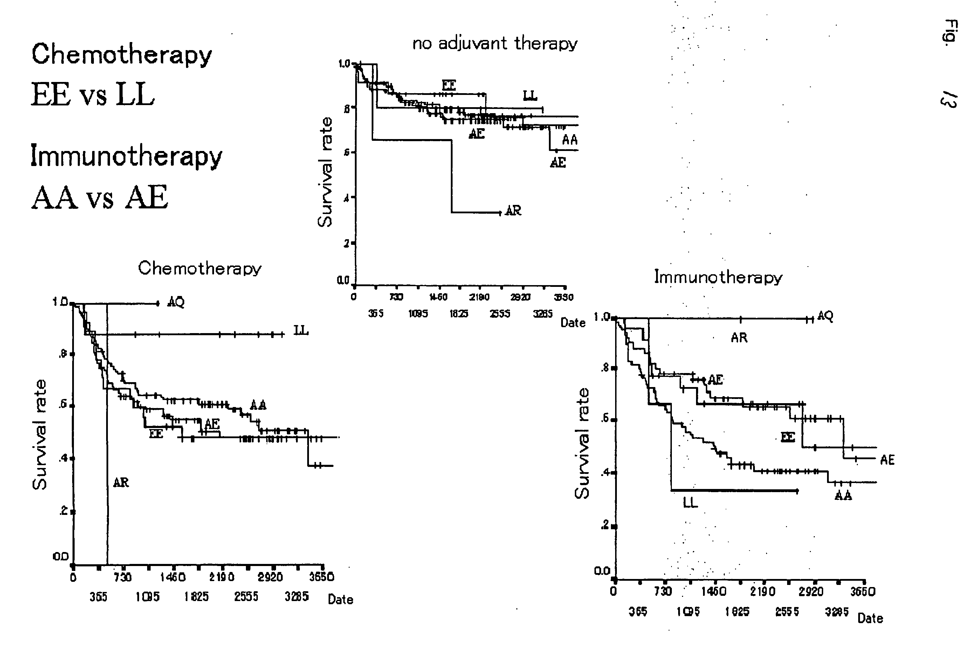

Fig. 13 shows that cancer resection alone (no adjuvant therapy) and the anticancer chemotherapy after the cancer resection (Chemotherapy) are not suitable for patients with AR at Position 74 of the amino acid sequence on DRB1*gene, while the anticancer immunotherapy after the cancer resection (Immunotherapy) is suitable for them. Cancer resection alone (no adjuvant therapy) and the anticancer immunotherapy after cancer resection (Immunotherapy) are suitable for patients with AQ. The anticancer immunotherapy after cancer resection (Immunotherapy) is not suitable but the anticancer chemotherapy after cancer resection (Chemotherapy) is suitable for patients with LL.

### [Working Example 7]

The treatment effects (5-year survival rate) with the amino acid variations of the specific part of the polymorphic amino acids of each DPB1*, DQB1* and DRB1*gene were analyzed. Data from a previous clinical example were used.

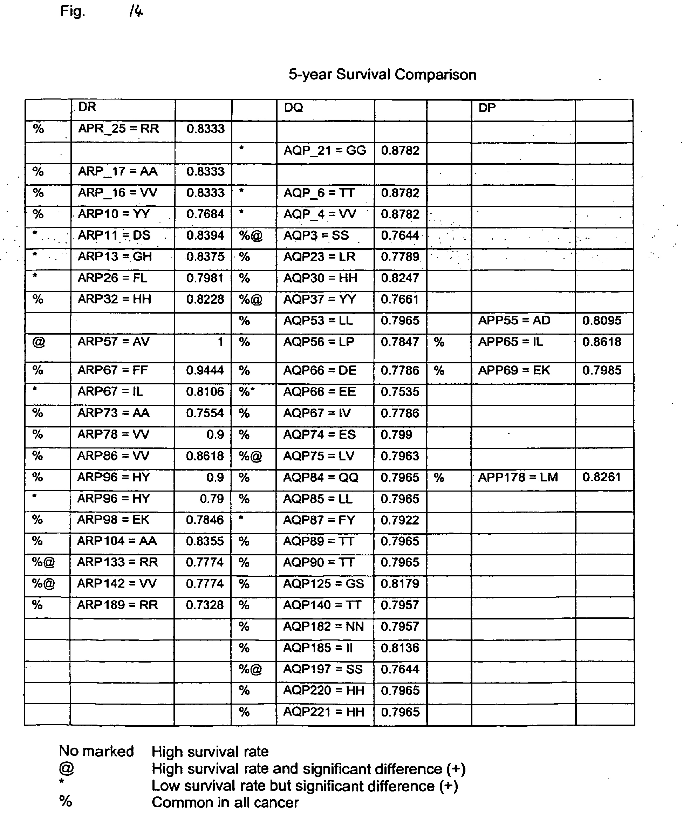

Fig. 14 shows the best survival rate (5-year survival) of the amino acid variations of the specific parts (polymorphic parts) of the amino acids of DRB1*, DQB1*, and DPB1*genes with the cancer resections alone. Positions marked with @ * in table are the positions with a statistically significant difference. Displayed as "APR-25=RR 0.8333" means the 5-year survival rate of 83.33% in patients with the RR of the amino acid variations at Position -25 (QR) on DRB1*gene. This table shows that the cancer resection alone is advantageous to patients with the amino acid variations such as (DS) at Position 11, (GH) at Position 13, (FL) at Position 26, (AV) at Position 57, (IL) at Position 67, (HY) at Position 96, (RR) at Position 133, and (VV) at Position 142 on the DR gene with a significant statistical difference. On DQB1*gene, the cancer resection alone is advantageous to patients with the amino acid variations such as (SS) at Position 3, (VV) at Position 4, (TT) at Position 6, (YY) at Position 37, (EE) at Position 66, (IV) at Position 67, (LV) at Position 75, and (SS) at Position 197 with a significant statistical difference. On DPB1*gene, the cancer resection alone is advantageous to patients with the amino acid variations of (AD) at Position 55 and (EK) at Position 69 with a significant statistical difference.

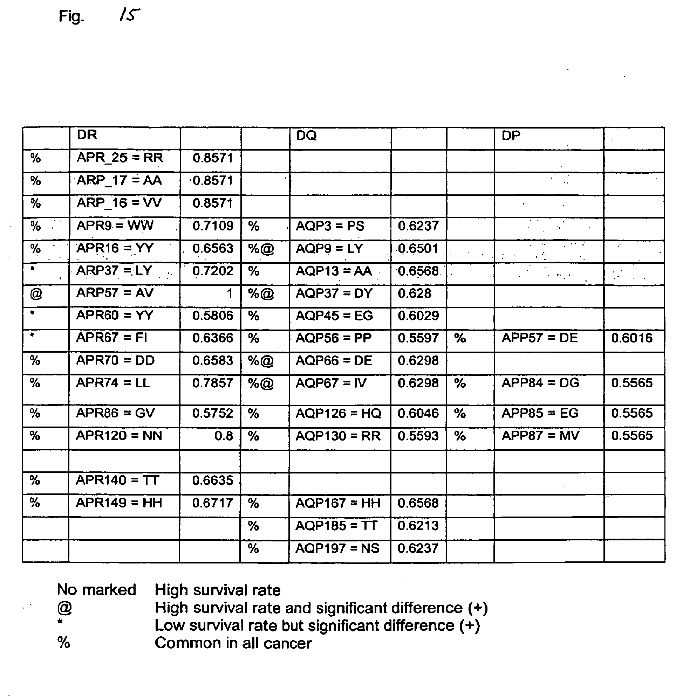

Fig. 15 shows the best survival rate (5-year survival) with the amino acid variation of the specific parts (polymorphic parts) of the amino acids of DRB1*, DQB1*, and DPB1*genes with the anticancer chemotherapy after cancer resection. Positions marked with @* in the table are positions with a statistically significant difference. Displayed as "APR-25=RR 0.8571" means the best 5-year survival rate of 85.71% in patients with the RR of the amino acid variations (QR) at Position -25 on DRB1*gene. This table shows that anticancer chemotherapy after cancer resection is advantageous to patients with the variations such as (LY) at Position 37, (AV) at Position 57, and anticancer chemotherapy after the cancer resection is advantageous to patients with amino acid variations such as (YY) at Position 60, and (FI) at Position 67, with a significant statistical difference. On DQB1*gene, anticancer chemotherapy after the cancer resection is advantageous to patients with the amino acid variations such as (LY) at Position 9, (DY) at Position 37, (DE) at Position 66, and (IV) at Position 67 with a significant statistical difference.

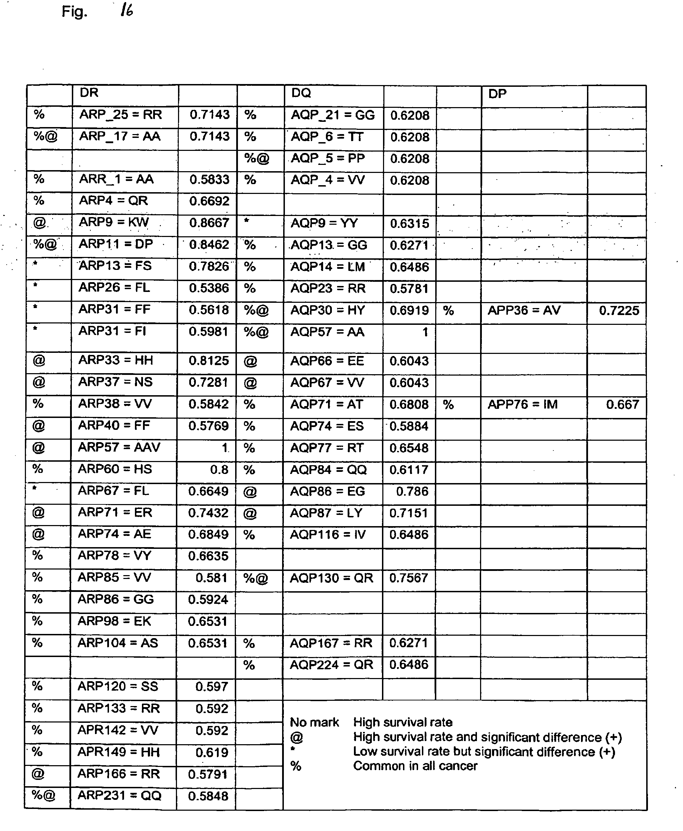

Fig. 16 shows the best survival rate (5-year survival) with the amino acid variation of the specific parts (polymorphic parts) on DRB1*, DQB1*, and DPB1*genes with the immunotherapy after the cancer resection. Positions marked with @* in table are positions with a statistically significant difference. Displayed as "APR-25=RR 0.7143" means that the best 5-year survival rate of patients with the variation of RR is 71.43% at Position -25 (QR) of DRB1*gene. This table shows that anticancer immunotherapy after cancer resection is advantageous to patients with amino acid variations such as the DR genes of (AA) at Position -17, (KW) at Position 9, (DP) at Position 11, (FS) at Position 13, (FL) at Position 26, (FF) at Position 31, (FI) at Position 31, (HH) at Position 3, (NS) at Position 37, (FF) at Position 40, (AV) at Position 57, (ER) at Position 71, (AE) at Position 74, and (QQ) at Position 231 with statistically significant difference. On DQB1*gene, the anticancer immunotherapy after the cancer resection is advantageous to patients with amino acid variations such as (PP) at Position -5, (YY) at Position 9, (HY) at Position 30, (AA) at Position 57, (EE) at Position 66, (VV) at Position 67, (EG) at Position 86, (LY) at Position 87, and (QR) at Position 310.

### [Working Example 8]

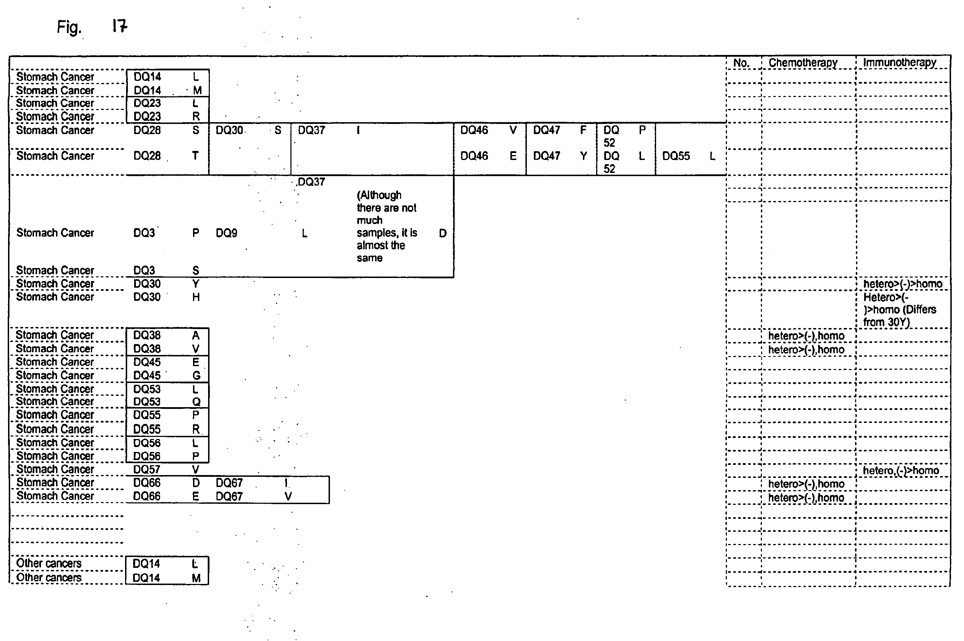

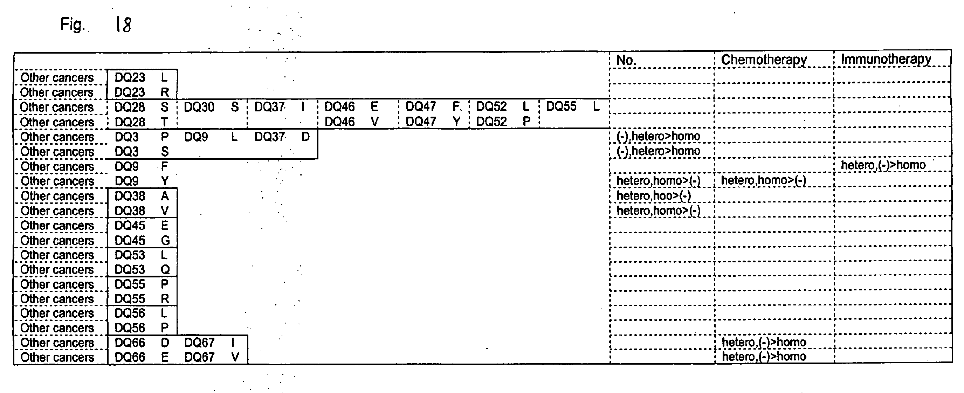

Fig. 17 and 18 display the result of the 5-year survival rate relating to the amino acid polymorphism of DQB*1 gene.

### (Patients with stomach cancers)

Equivalences are confirmed in the following 4 groups: 1) DQ14L and 14M, 23L and 23R, 38A and 38V, 45E and 45G, 53L and 53Q, 55P and 55R, and 56L and 56P, 2) DQ28S, 28T, 30S, 37I, 46V, 46E, 47F, 47Y, 53P, 52L, and 55L, 3) DQ3P, 3S, 9L, 37D, and 4) DQ66D, 66E, 67I, and 67V. On DQ30Y, the survival rate ofYH (heterozygote) is significantly longest, and that of Y (-) is significantly longer than that of YY (homozygote) with the anticancer immunotherapy after the cancer resection (Immunotherapy). On DQ30H, the survival rate of HY (heterozygote) is significantly longest, and that of H (-) is significantly longer than that ofHH (heterozygote) with anticancer immunotherapy after the cancer resection (Immunotherapy). On DQ38A, the survival rate of AY (heterozygote) is significantly longer than that of A (-) or AA (homozygote) with the anticancer chemotherapy after the cancer resection (Chemotherapy). On DQ38V the survival rate of VH (heterozygote) is significantly longer than that of V (-) or W (homozygote) with the anticancer chemotherapy after the cancer resection (Chemotherapy). On DQ57V, the survival rate of (heterozygote) is significantly longer than that of V (-) and W (homozygote) with the anticancer immunotherapy after the cancer resection (Immunotherapy). On DQ66D, the survival rate of DE (heterozygote) is significantly longer than that of D (-) or DD (homozygote) with the anticancer chemotherapy after the cancer resection (Chemotherapy). On DQ66E, the survival rate of ED (heterozygote) is significantly longer than that of E (-) or EE (homozygote) with the anticancer chemotherapy after the cancer resection (Chemotherapy). On DQ67I, the survival rate of IV (heterozygote) is significantly longer than that of I (-) or II (homozygote) with the anticancer chemotherapy after the cancer resection (Chemotherapy). On DQ67V, the survival rate of VI (heterozygote) is significantly longer than that of V (-) or W (homozygote) with the anticancer chemotherapy after the cancer resection (Chemotherapy).

These results indicate that Positions at 30, 38, 57, 66, and 67 on the amino acid sequences of DQB*1 gene are important and statistically significant in each stomach cancer treatment. Also, the positions where the polymorphic amino acids have equivalent significance are confirmed.

### (Patients of Cancers other than stomach cancer)

Equivalences are confirmed in the following 4 groups: 1) DQ14L and14M, 23L and 23R, 45E and 45G, 53L and 53Q, 55P and 55R, and 56L and 56P, 2) DQ28S, 28T, 30S, 371, 46E, 46V, 47F, 47Y, 52L, 52P, and 55L, 3) DQ3P, 3S, 9L, and 37D, and 4) DQ66D, 66E, 67I, and 67V.

On DQ3P, the survival rate of P (-) or PS (heterozygote) is significantly longer than that of PP (homozygote) with the cancer resection alone (no adjuvant therapy). On DQ3S, the survival rate of S (-) or PS (heterozygote) is significantly longer than that of SS (homozygote) with the cancer resection alone (no adjuvant therapy). On DQ9L, the survival rate of L (-) or (heterozygote) such as LF/LY is significantly longer than that of LL (homozygote) with the cancer resection alone (no adjuvant therapy). On DQ37D, the survival rate of D (-) or DI (heterozygote) is significantly longer than that of DD (homozygote) with the cancer resection alone (no adjuvant therapy). On DQ9F, the survival rate of FY (heterozygote) or F (-) is significantly longer than that of FF (homozygote) with the anticancer immunotherapy after the cancer resection (Immunotherapy). On DQ9Y, the survival rate of YF(heterozygote) or YY(homozygote) is significantly longer than that of Y(-) with both the cancer resection alone (no adjuvant therapy) and the anticancer chemotherapy after the cancer resection (Chemotherapy). On DQ38A, the survival rate of AV(heterozygote) and AA(homozygote) is significantly longer than that of A(-) with the cancer resection alone (no adjuvant therapy). On DQ38V, the survival rate of VA(heterozygote) or VV(homozygote) is significantly longer than that of V(-) with the cancer resection alone (no adjuvant therapy). On DQ66D, the survival rate of DE (heterozygote) or D (-) is significantly longer than that of DD (homozygote) with the anticancer chemotherapy after the cancer resection (Chemotherapy). On DQ67I, the survival rate of IV (heterozygote) or I (-) is significantly longer than that of II (homozygote) with the anticancer chemotherapy after the cancer resection (Chemotherapy). On DQ66E, the survival rate of ED (heterozygote) or E (-) is significantly longer than that of EE (homozygote) with the anticancer chemotherapy after the cancer resection (Chemotherapy). On DQ67V, the survival rate of VI (heterozygote) and V (-) is significantly longer than that of VV (homozygote) with the anticancer chemotherapy after the cancer resection (Chemotherapy).

These results indicate that Positions at 3, 9, 37, 38, 66, and 67 on the amino acid sequences on DQ gene play an important and statistically significant role in each treatment for cancers other than stomach cancers. Also, several positions where the polymorphic amino acids have equivalent significance are confirmed.

### [Working Example 9]

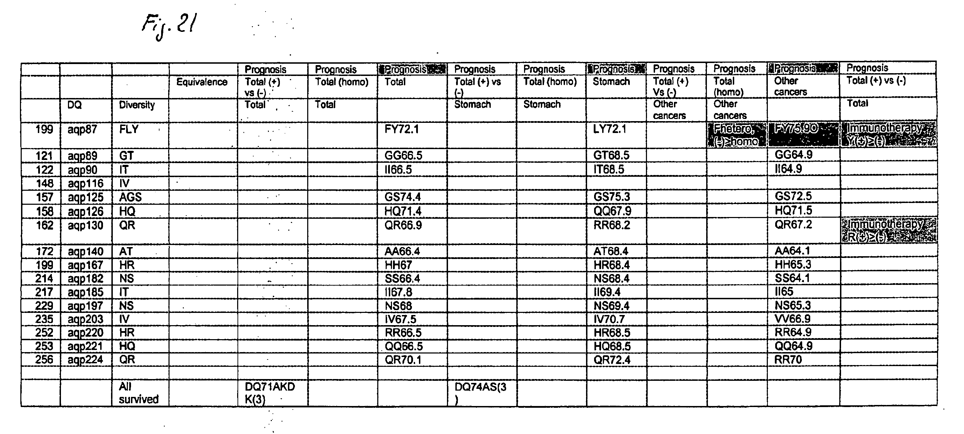

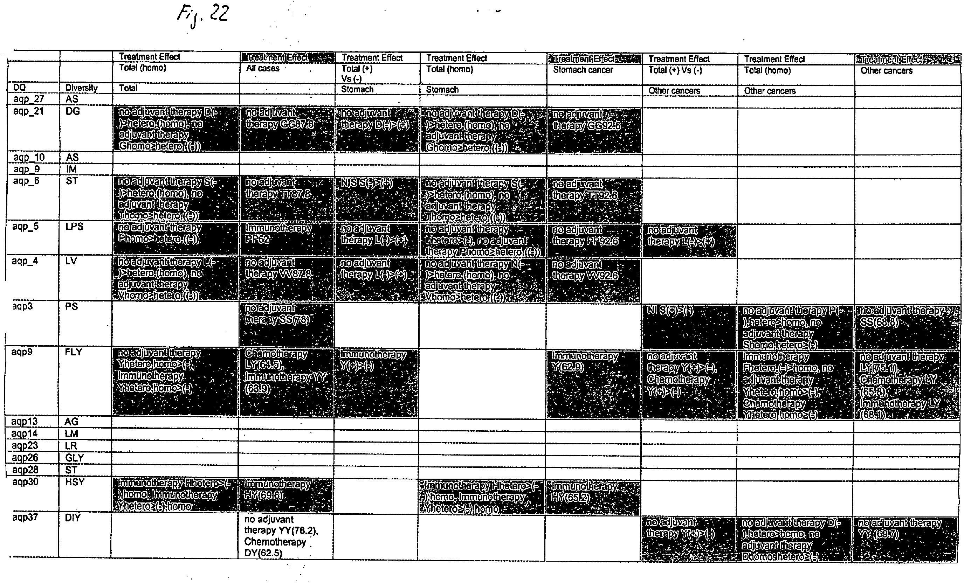

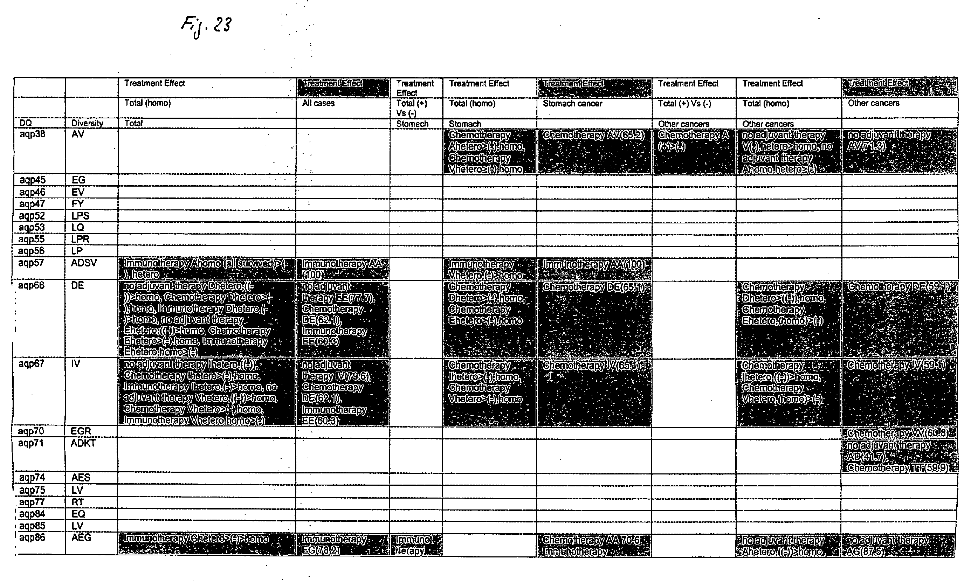

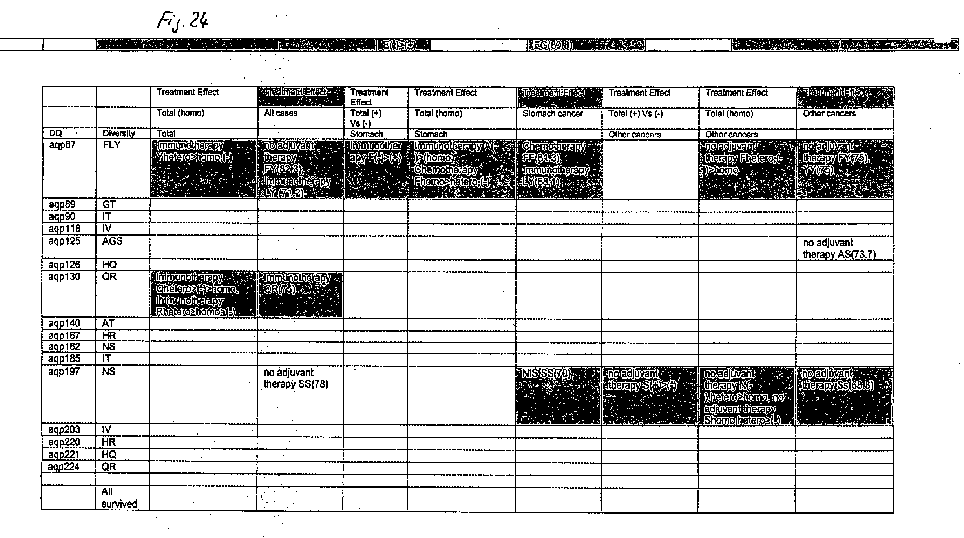

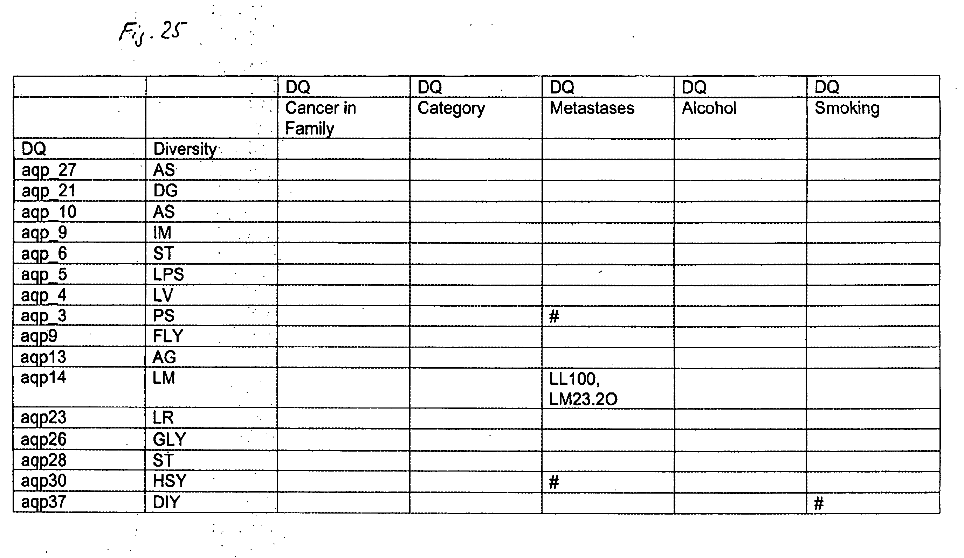

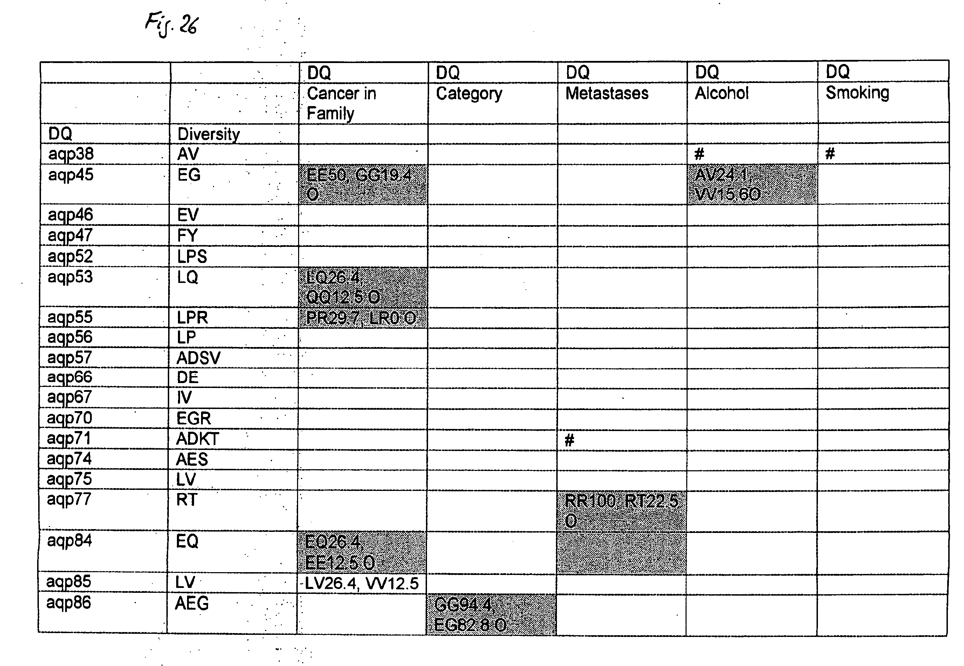

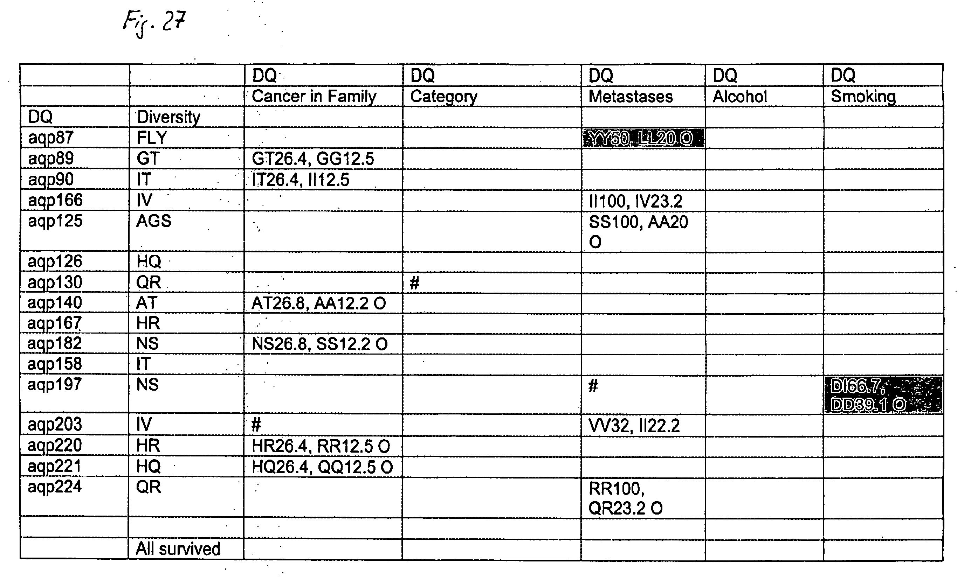

In Fig. 19 to 27, the polymorphic amino acids at the amino acid sequences on gene DQB1* and prognosis of the variations [5-year survival rates in all cancer cases (not categorize them according to treatments)], treatment effects, 5-year survival rate, and other possible effects are shown. Basic data is based on the above clinical cases. Shadowed cells indicate the results with statistically significant differences.

In the row of "arp-25" in the Figure, data of the amino acid at Position -25 is shown. In the "Diversity" column, a polymorphism (e.g. KR) of the amino acid at each position is shown. "Equivalence" column shows whether the amino acid has equivalence (e.g. K=R: equivalence). In the "Prognosis", "Total (homozygote)" and "Total" column, the prognoses in all cases with presence/absence/homozygote/hete.rozygote of polymorphic amino acid on the amino acid sequence positions are shown. In the "Prognosis", "Total", and a blank cell column, the survival rate of the best amino acid variations for good prognosis in all cases is shown, (e.g. "RR78.8" means that the 5-year survival rate of the patients with RR homozygote variation in the corresponding sequence is 78.8%, and the prognosis is good.) In the "Prognosis", "Total (+) vs. (-)", and "Stomach" column, only stomach cancer patients are totaled, and the prognoses by the presence or absence of the polymorphic amino acid are shown. (e.g. F(-)>(+) means that the prognosis in stomach cancer cases of the patients without F is better than that of the patients with F. ) In the "Prognosis", "Total (homozygote)", and "Stomach" column, only stomach cancer patients are totaled, and prognoses with presence/ absence/ homozygote/ heterozygote of polymorphic amino acid are shown. (e.g. "V heterozygote > (-)" means that the prognosis of the patients with (heterozygote) such as VG/ VD/VL is better than that of the patients without heterozygote.

In the "Prognosis", "Stomach" and a blank cell column, only stomach cancer patients are totaled, and the survival rate of the best amino acid variations for good prognosis in all cases is shown. (e.g. "RR71.4" means that the 5-year survival rate of the patients with RR variation in the sequence is 71.4%, and the prognosis is good.) In the "Prognosis", "Total (+) vs. (-)", and "Other cancer" column, only patients of cancers other than stomach cancers are totaled, the prognosis with the presence or absence of the polymorphic amino acid is shown. (e.g. A(+)>(-) means that the prognosis of the patients with A is better than that of the patients without A in other cancers cases. In the "Prognosis", "Total (homozygote)", and "Other Cancer" column, only patients of cancers other than stomach cancers are totaled, and prognosis with presence/ absence/ homozygote/ heterozygote of polymorphic amino acids are shown. (e.g. "A, S homozygote, heterozygote> (-)" means that the prognosis of the patients with AA (homozygote), SS (homozygote), or AS (heterozygote) is better than that of the patients without A or S. In the "Prognosis", "Other Cancers", and a blank cell column, only patients of cancers other than stomach cancers are totaled, and the survival rate of the best amino acid variations for good prognosis in all cases is shown. (e.g. "RR100" means that the 5-year survival rate of the patients with RR variation in the sequence is 100%, and the prognosis is good.) In the "Treatment Effect", "Total (+) vs. (-)", and "Total" column, all kinds of cancer including stomach cancer and other cancers patients are totaled, the polymorphic amino acids presence and treatment effects of the post-treatments after the cancer resections [the cancer resection alone (no adjuvant therapy), and the anticancer chemotherapy after the cancer resection (Chemotherapy), and the anticancer immunotherapy after the cancer resection (Immunotherapy)] are compared and examined. (e.g. "Immunotherapy E (-)> (+)" means that the amino acids of the specific sequence position has EQY and the treatment effect of the patients without E (E (-)) is better than that of the patients with E with the anticancer immunotherapy after the cancer resection (Immunotherapy).

In the "Treatment Effect", "Total (homozygote)" and "Total" column, all kinds of cancer including stomach cancer and other cancers patients are totaled, and treatment effects with presence/ absence/ homozygote/ heterozygote of polymorphic amino acids are shown. (e.g. "Immunotherapy E(-)>heterozygote" means that the amino acids of the specific sequence position has EQY and the treatment effect of the patients without E is better than that of the patients with (heterozygote) such as EQ and EY with the anticancer immunotherapy after the cancer resection (Immunotherapy). In the "Treatment Effect", "All Cases", and a blank cell column, the survival rate of the best amino acid variations for good prognosis in all cases is shown. (e.g. "AA71.4" means that the 5-year survival rate of the patients with AA variation in the sequence t is 71.4%, and the anticancer immunotherapy after the cancer resection (Immunotherapy) is effective on the patients.) In the "Treatment Effect", "Total (+) vs. (-)", "Stomach" column, stomach cancer patients is totaled, and treatment effects with the polymorphic amino acids presence and the type of the post-treatments after the cancer resections are compared and examined. (e.g. "Immunotherapy E (-)>(+)" means that the treatment effect of the patients without E in the sequence is better than that of the patients with E with the anticancer immunotherapy after the cancer resection (Immunotherapy) in stomach cancer cases.) In the "Treatment Effect", "Total (homozygote)", and "Stomach" column, stomach cancer patients are totaled, and the treatment effects of each treatment with presence/ absence/ homozygote/ heterozygote of polymorphic amino acids are shown. (e.g. "Immunotherapy E(-), homozygote>heterozygote" means that the amino acids of the specific sequence position has EKW, and the treatment effect of the patients with E or EE(homozygote) is better than that of the patients with (heterozygote) such as EK and EW with the anticancer immunotherapy after the cancer resection (Immunotherapy).) In the "Treatment Effect", "Stomach Cancer", and a blank cell column, stomach cancer patients are totaled, and the survival rate of the best amino acid variations for good prognosis in all cases is shown. (e.g. "KW84.6" means the 5-year survival rate of the stomach cancer patients with KW variation in the sequence is 84.6%, and the anticancer immunotherapy after the cancer resection (Immunotherapy) is effective on the patients.) In the "Treatment Effect", "Total (+) vs. (-)", "Other Cancer" column, cancers other than stomach cancer patients are totaled, and treatment effects with the polymorphic amino acids presence and the type of the post-treatments after the cancer resections are compared and examined. (e.g. "Chemotherapy Y (+) > (-)" means that the treatment effect of patients with Y is better, or more effective, than that of the patients without Y with the anticancer chemotherapy after the cancer resection (Chemotherapy). In the "Treatment Effect", "Total (homozygote)" and "Other Cancer" column, cancers other than stomach cancers are totaled, and the treatment effects with presence/ absence/ homozygote/ heterozygote of polymorphic amino acids are shown. (e.g. "Chemotherapy H(-), heterozygote>homozygote" means that the amino acids of the specific sequence position has HY, and the treatment effect of the patients without H or HY(heterozygote) is better than that of the patients with HH(homozygote) with the anticancer chemotherapy after the cancer resection (Chemotherapy). In the "Treatment Effect", "Other Cancers", and a blank cell column, the survival rate of the best amino acid variations for good prognosis in all cases is shown. (e.g. "HY55.6" means that 5-year survival rate of the patients with HY variation in the sequence is 55.6% in other cancer cases, the anticancer chemotherapy after the cancer resection (Chemotherapy) is effective on the patients.)

The "DR" and "Cancer in Family" column shows the particular amino acids at the specified amino acid sequences of the family with a history of having cancer patients. ("Cancer in Family" means that the patients have cancer patients within their 2 degrees of consanguinity.) Marked `#' indicates that no significance is found in this case study, although there was in the previous study. Marked `O' means that there is significant difference of the amino acid variation in the column. (e.g. "AV 38.7" means significant difference that the variation of AV of the specified amino acid sequence has 38.7% while AA has 0%. It means that the families of the patients of AV tend to have more cancer patients.)

The "DR" and "Metastases" column indicates the tendency of the cancer metastases. ("Metastases" in this report refers cancer metastases to lymph nodes, liver, and lungs.) Marked 'O' means that there is the significant difference in the column, while marked '#' means not much difference although there was in the previous case. (e.g. "FL80" means that since metastases are observed in 80% of patients with FL amino acid variation while it is 22.9% with LL variation, there is significant difference between them. Patients with an FL variation tend to have more cancer metastases.)

In "DR" and "Total t" (Ratio of advanced cancer) column, the rates of advanced cancer patients are shown. Marked 'O' means that there is the significant difference in the column, while marked '#' means that there is not much difference, even though there was in the previous case. (e.g. "FT 22.6" means that advanced cancer is seen in 22.6% of the patients with FT amino acid variation, while in the patients with FF or GR variations there is not advanced cancer but early stage cancers only (early stage cancers is invasive cancers to mucosa or submucosa with few or no lymph node metastases. Advanced cancer mentioned in this report means other cancer cases.),

In "DR" and "Smoking" column, the rate of smokers is shown ("Smoker" is defined as the patient who has a smoking habit before the treatments and keeps the habit.) Marked 'O' means that there is the significant difference, while marked '#' means that there is not much difference, even though there was in the previous case. (e.g. "AS 73.3" means that 77.3% of patients with AS amino acid variation are smokers while 44.4% for AA variation significant differences is noted between the groups.

### DQB1*gene

### (Influences of polymorphic amino acids on the specific positions of DQB1*gene on prognosis and treatment effects)

### Prognosis Analysis of DQB1*gene in All Cancer Cases

### (Fig. 19 to 21)

From the figures, it is confirmed that the polymorphic amino acids at Position 9, 55, 56, 57, 66, 67, 70, 71, and 74 have significance in prognosis in all cancer cases.

### Prognosis Analysis of DQB1*gene in Stomach Cancer Cases

### (Fig. 19 to 21)

From the figures, it is confirmed that the polymorphic amino acids at Position -5, 9, 55, 66, and 67 have significance in prognosis in stomach cancer cases.

### Prognosis analysis of DQB1*gene in Other Cancer Cases

### (Fig. 19 to 21)

From the figures, it is confirmed that the polymorphic amino acids at Position 9, 23, 56, 66, 67, 70, 71, 86, and 87 of the polymorphic amino acids have significance in prognosis in other cancer cases (other than stomach cancer cases).

### Treatment Effect Analysis of DQB1*Gene in All Cancer Cases

### (Fig. 19 to 21) (Figure 22 to 24)

From the figures, it is confirmed that in all cancer cases the polymorphic amino acids at Position -21, -6, -5, -4, 3, 9, 30, 57, 66, 67, 86, 87, and 130 have significance in treatment effects with the cancer resection alone (no adjuvant therapy), the anticancer chemotherapy after the cancer resection (Chemotherapy), and the anticancer immunotherapy after the cancer resection (Immunotherapy).

### Treatment Effect Analysis of DQB1*Gene in Stomach Cancer Cases

### (Fig. 22 to 24)

From the figures, it is confirmed that in stomach cancer cases the polymorphic amino acids at Position -21, -6, -5, -4, 9, 30, 38, 57, 66, 67, 86, 87, and 197 have significance in treatment effects with the cancer resection alone (no adjuvant therapy), the anticancer chemotherapy after the cancer resection (Chemotherapy), and the anticancer immunotherapy after the cancer resection (Immunotherapy).

### Treatment Effect Analysis of DQB1*Gene in Other Cancer Cases

### (Fig. 22 to 24)

From the figures, it is confirmed that in other cancer cases (excluding stomach cancer cases) the polymorphic amino acids at Position -5, -3, 9, 37, 38, 66, 67, 70, 71, 86, 87, and 197 significance in treatment effects with the cancer resection alone (no adjuvant therapy), the anticancer chemotherapy after the cancer resection (Chemotherapy), and the anticancer immunotherapy after the cancer resection (Immunotherapy).

### Other analysis of DQB1*gene

### (Fig. 25 to 27)

From the figures, it is confirmed that polymorphic amino acids at Position 45, 53, 55, 84,140,182,220, and 221 have significance in Cancer in Family.

### [Performed Case 10]

### Relational Analysis of Variation in the Base Sequences of the Specific Amino Acid Position of DQB1* gene with Treatment Effects

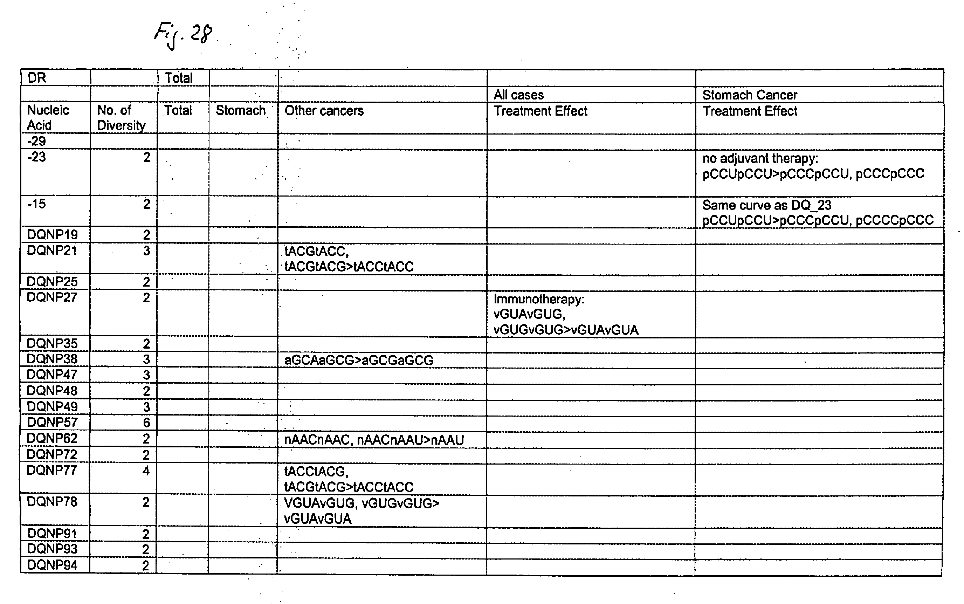

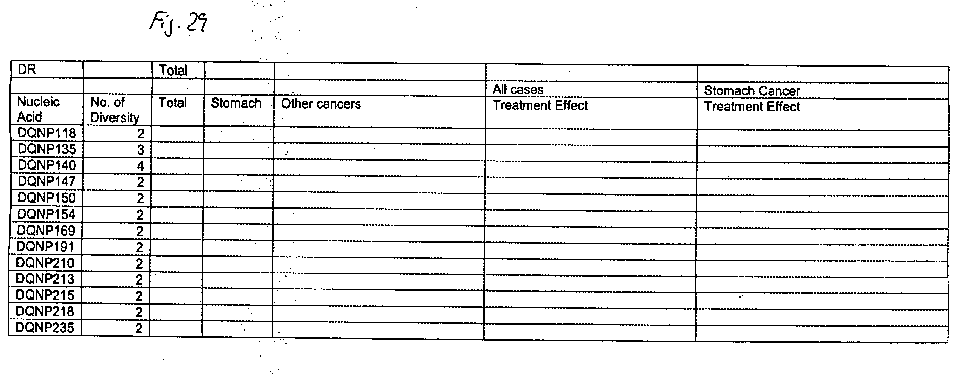

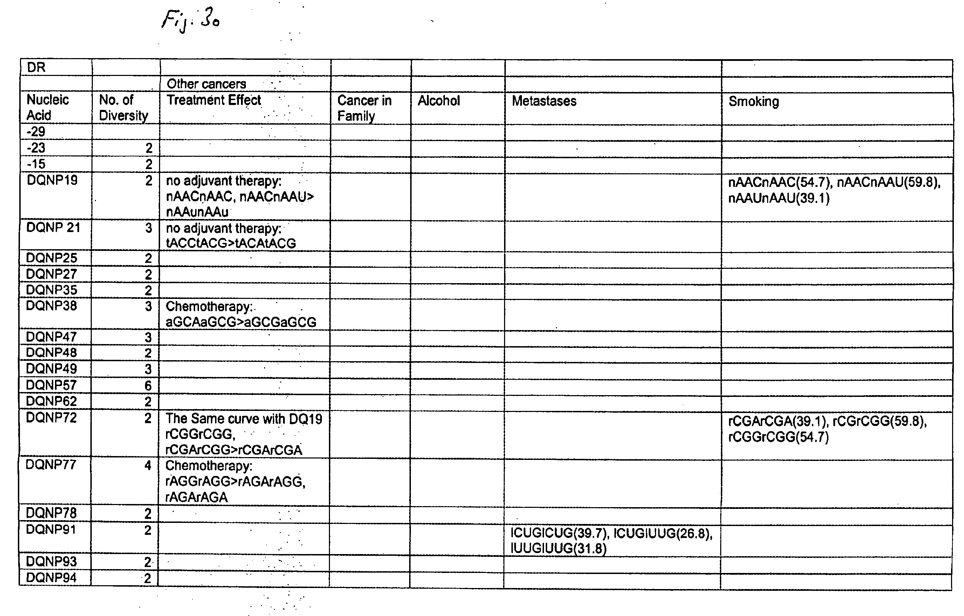

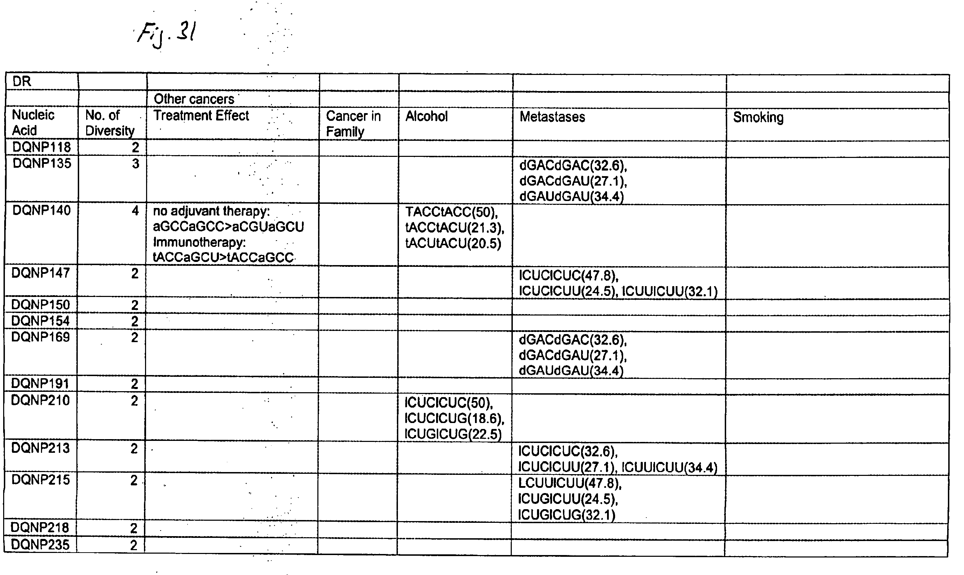

Fig. 28 - 31 shows, from left, Nucleic Acid (Position of Amino Acid Sequence, "-16" means the pre-area of the gene and "4" means Position 4.), Number of Diversity (Number of polymorphic amino acids at the position, "3" means that 3 kinds of amino acids exist), Total (total number of stomach cancer and other cancer cases), Stomach (Stomach cancer cases), Other cancer (other cancer cases), Treatment Effect [rate of 5-year survival (Total Case, Stomach Cancer, Other Cancer)], Cancer In Family (family with history of having cancer patients), Alcohol (patients with a habit of drinking alcohol), Metastases (patients with cancer metastases), and Smoking (patients with a habit of smoking). For example, "aGCGaGCG>aGCGaGCU" under DRB1*gene (column: -16 and row: Total Stomach) means that the lower-case alphabet "a" is the single character code of the amino acid which is followed with-the corresponding base sequence GCG. Thus, it means that on the whole homozygote is more significant than heterozygote. Also, "kAAAkAAA>kAAGkAAG>kAAAkAAG" under DRB1*gene (column: 12, row: stomach cancer treatment effect) means that the lower-case alphabet "k" is a single character code of the amino acid which is followed with the corresponding base sequence AAA or AAG. Both kAAAkAAA and kAAGkAAG are homozygotic while kAAAkAAG is heterozygotic. Thus, it indicates that the treatment effects of kAAAkAAA and kAAGkAAG (homozygote) are significantly better than that of kAAAkAAG (heterozygote) with the anticancer immunotherapy after the cancer resection (Immunotherapy). And so forth, the relationship of the base sequences with the treatments [the cancer resection alone (no adjuvant therapy), the anticancer chemotherapy after the cancer resection (Chemotherapy), and the anticancer immunotherapy after the cancer resection (Immunotherapy)] are shown.
Data was collected from the above clinical data analysis.

### Effect of The Polymorphic amino acids of DQB1*Gene and Corresponding Base Sequence on Prognosis in Total , Treatment Effect, And Other Analysis

### Analysis of DQB1*gene in All and Stomach Cancer Cases

### (Fig. 28 to 29)

From the figures, the significance of the polymorphic base sequence is not confirmed.

### Analysis of DQB1*gene in Other Cancer Cases

### (Fig. 28 to 29)

From the figures, it is confirmed that the polymorphic base sequences of the amino acid at Position 21t, 38a, 62n, 77t, and 78v have significance in other cancer cases.

### Treatment Effect Analysis of DQB1*Gene in All Cancer Cases

### (Fig. 28 to 29)

From the figures, it is confirmed that the polymorphic base sequence of the amino acid at Position 27v has significance in treatment effects of the anticancer immunotherapy after the cancer resection (Immunotherapy) in all cancer cases.

### Treatment Effect Analysis of DQB1*Gene in Stomach Cancer Cases

### (Fig. 28 to 29)

From the figures, it is confirmed that the polymorphic base sequences of the amino acid at Position -23 and -15 have significance in treatment effects of the cancer resection alone (no adjuvant therapy) in stomach cancer cases.

### Treatment Effect Analysis of DQB1*Gene in Other Cancer Cases

### (Fig. 30 to 31)

From the figures, it is confirmed that the polymorphic base sequences of the amino acid at Position 19n, 21t, 38a, 72r, 77r, and 104a have significance in treatment effects of the cancer resection alone (no adjuvant therapy), the anticancer chemotherapy after the cancer resection (Chemotherapy), and the anticancer immunotherapy after the cancer resection (Immunotherapy) in other cancer cases.

### Other Analysis of DB1*Gene

### (Fig. 30 to 31)

From the figures, it is confirmed that the polymorphic base sequences of the amino acid have significance at Position 140t and 2101 in drinking, at Position 911, 135d, 1471, 169d, 2131, and 2151 in cancer metastases, and at Position 19n and 72r in smoking.

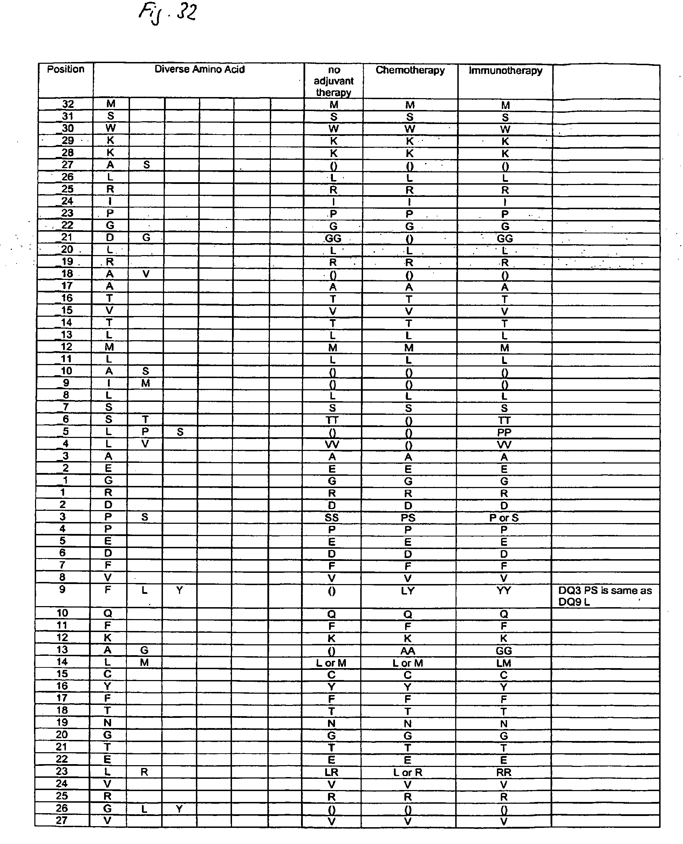

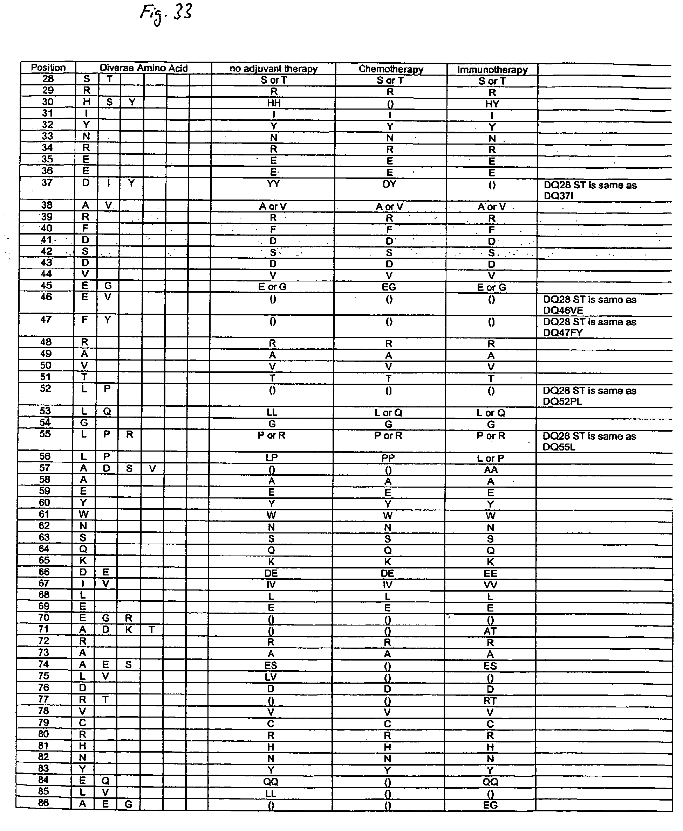

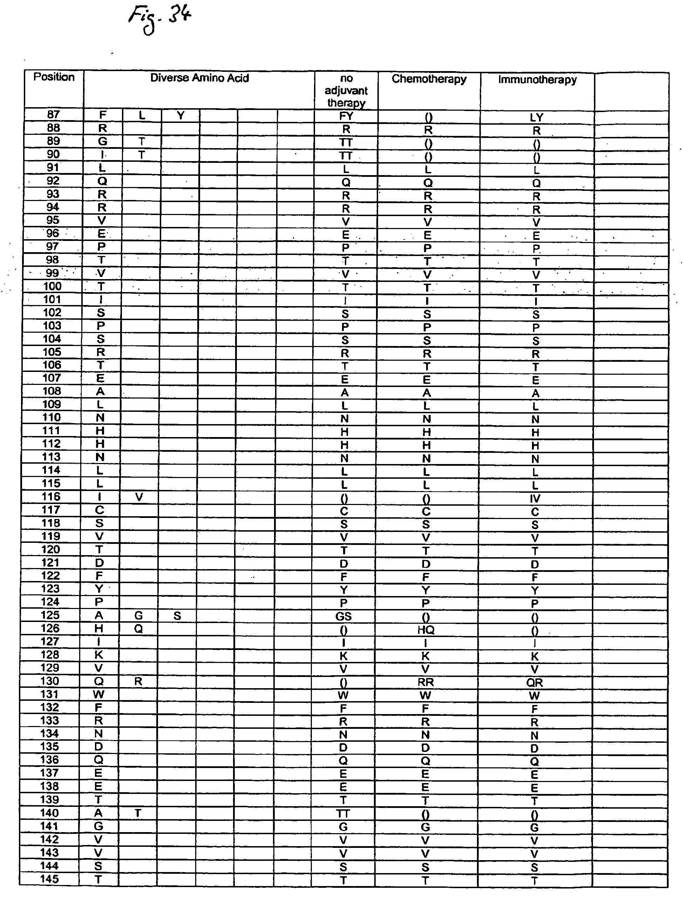

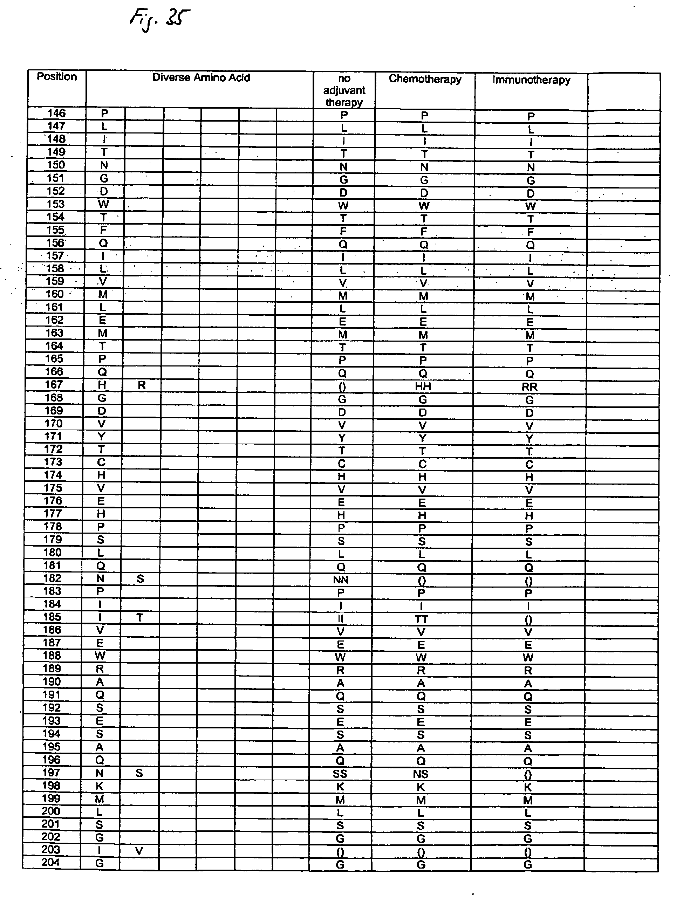

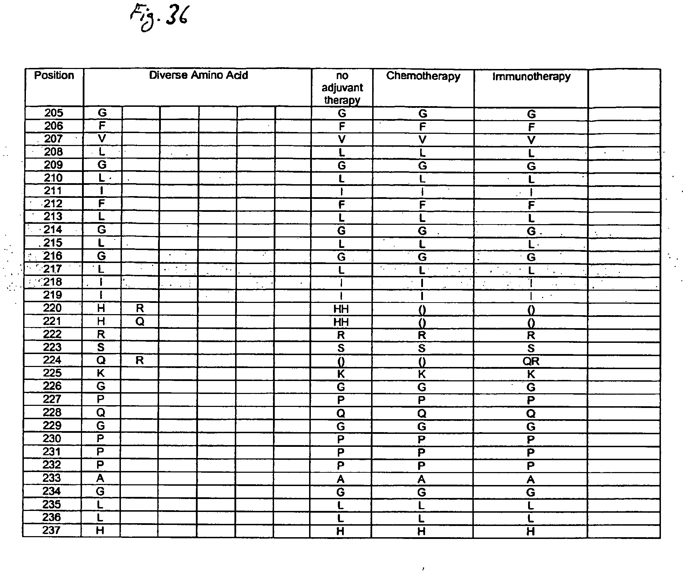

### Optimum Amino Acid of DQB1*gene (Fig. 32 to 36)

Polymorphisms are found at Position -27, -21, -18, -10, -9, -6, -5, -4, 3, 9, 13, 14, 23, 26, 28, 30, 37, 38, 45, 46, 47, 52, 53, 55, 56, 57, 66, 67, 70, 71, 74, 75, 77, 84, 85, 86, 87, 89, 90, 116, 125, 126, 130, 140, 167, 182, 185, 197, 203, 220, 221, and 224. Particularly important positions are Position -21, -6, -5, -4, 3, 9, 13, 14, 23, 30, 37, 45, 53, 56, 57, 66, 67, 71, 74, 75, 77, 84, 85, 86, 87, 89, 90, 116, 125, 126, 130, 140, 167, 182, 185, 197, 220, 221, 224, having significance in the treatments [the cancer resection alone (no adjuvant therapy ), the anticancer chemotherapy after the cancer resection (Chemotherapy), and the anticancer immunotherapy after the cancer resection (Immunotherapy)].

### [Performed Case 12]

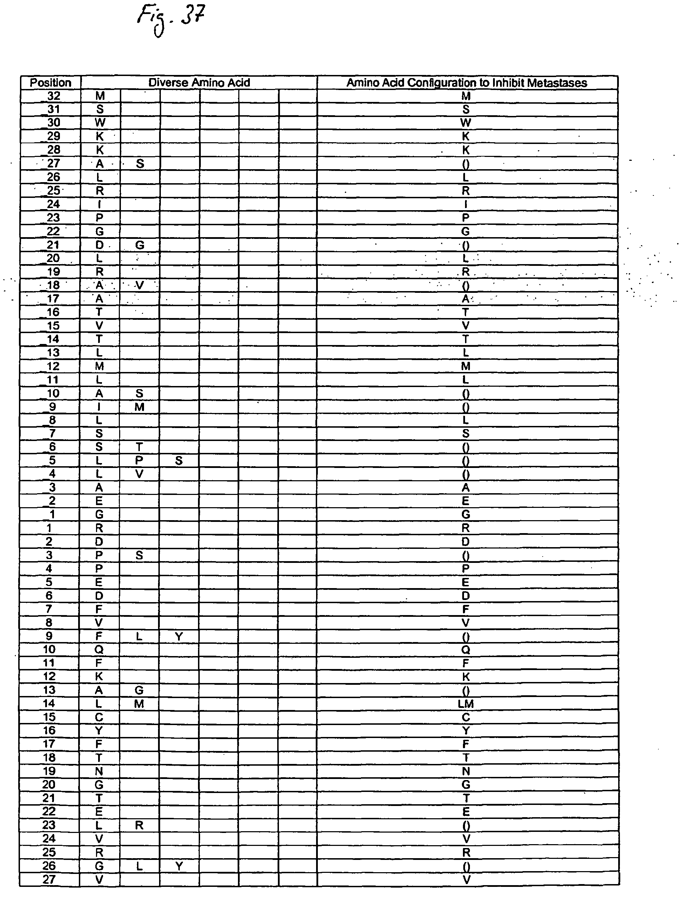

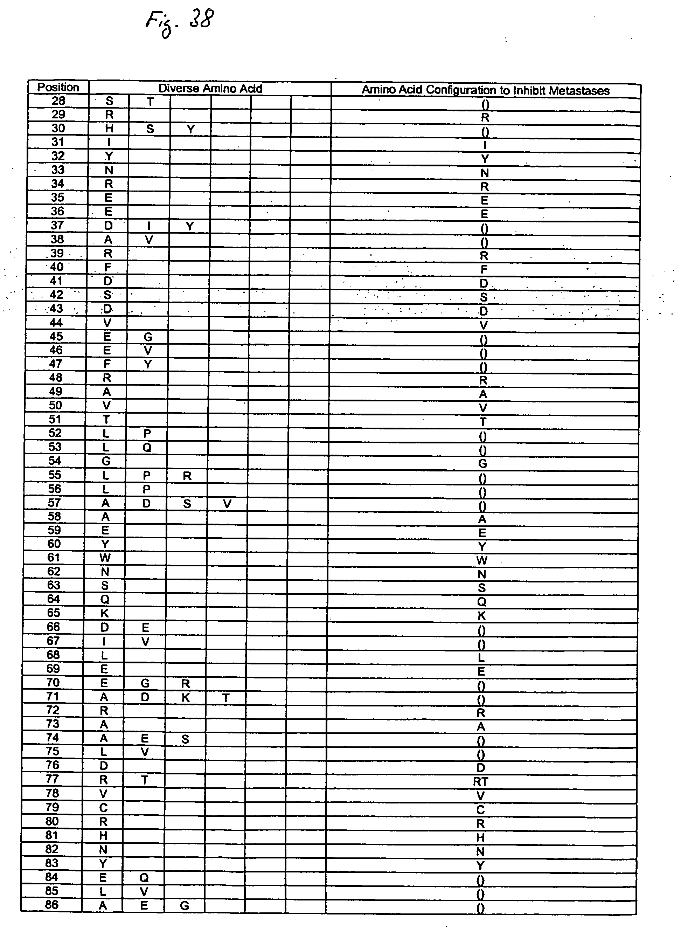

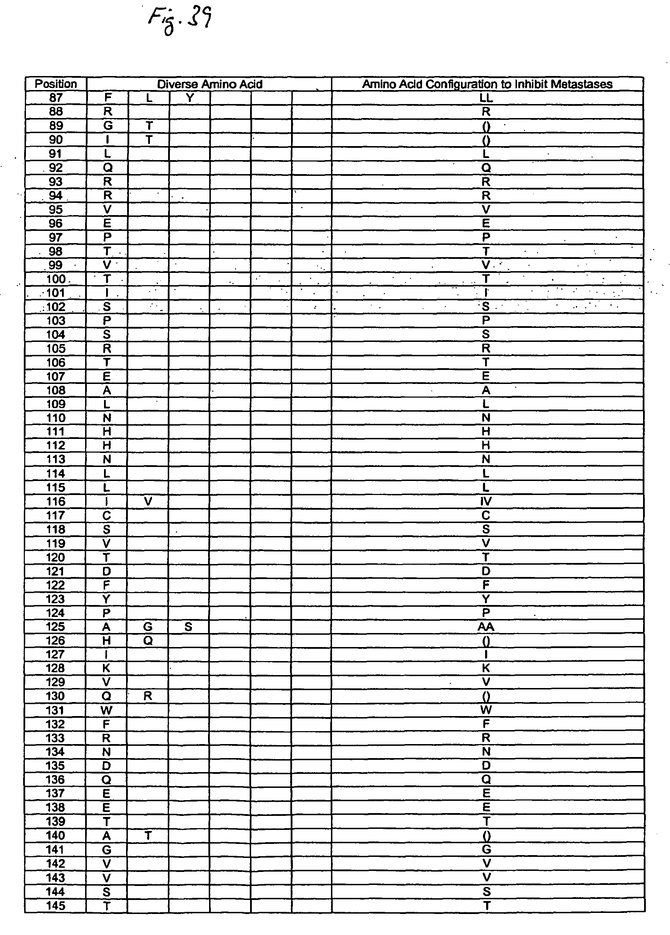

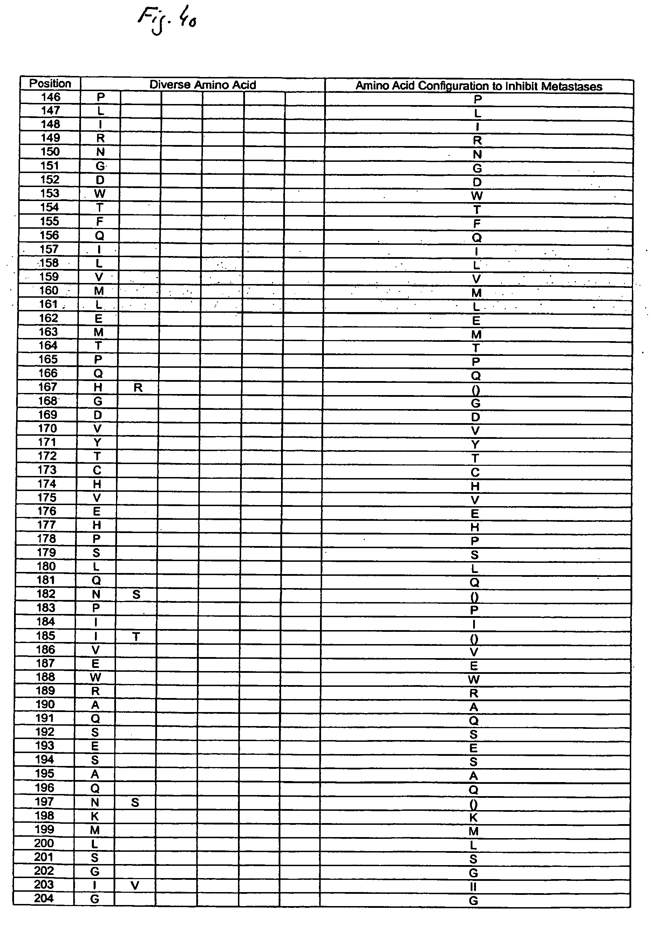

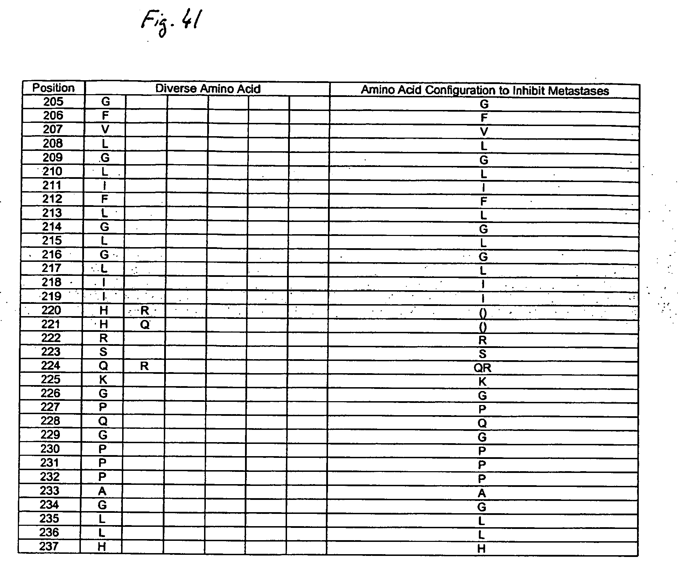

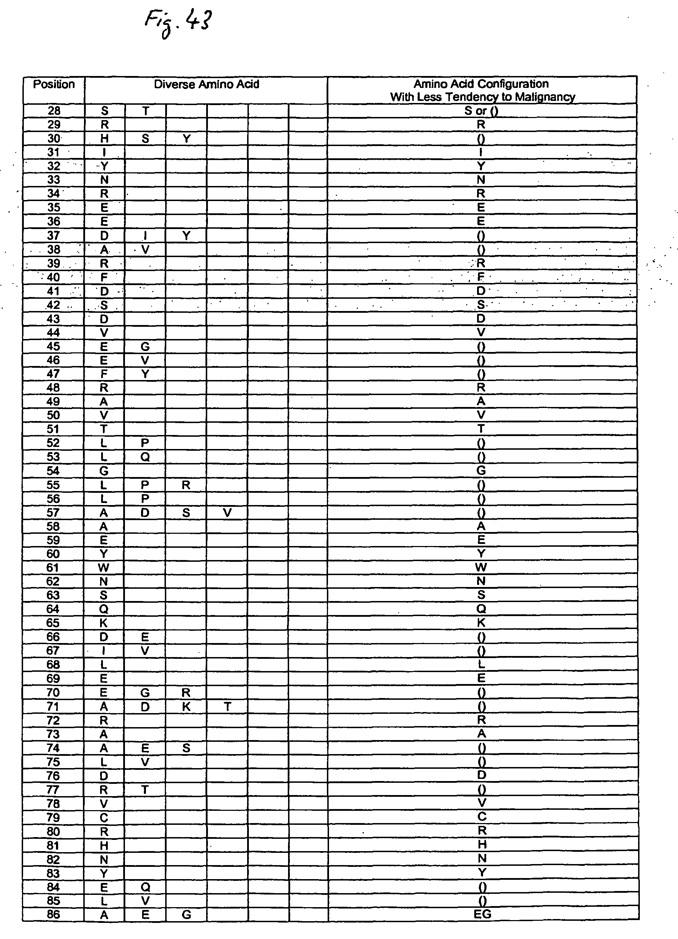

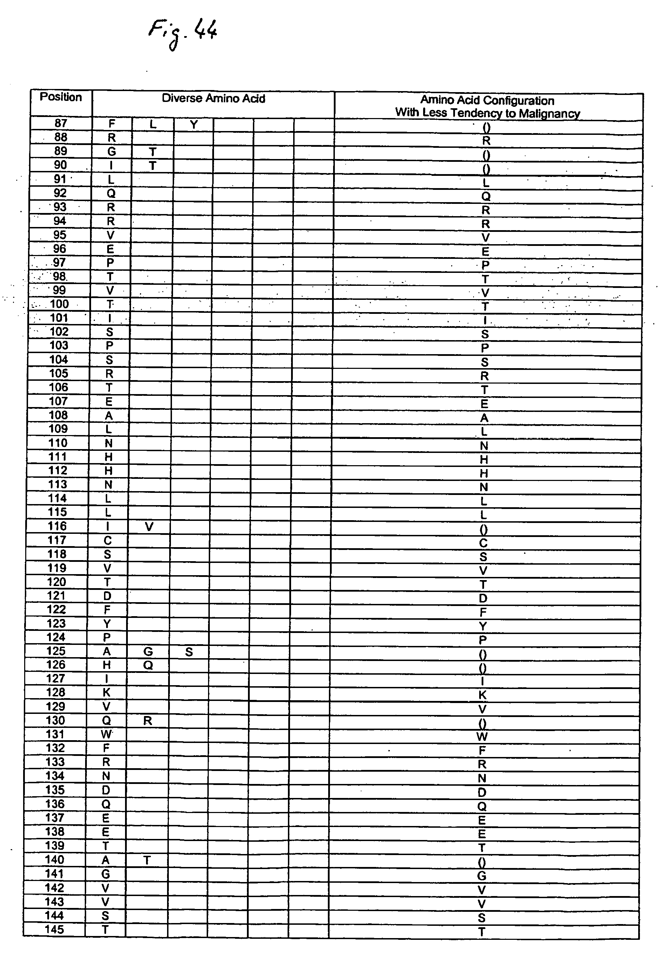

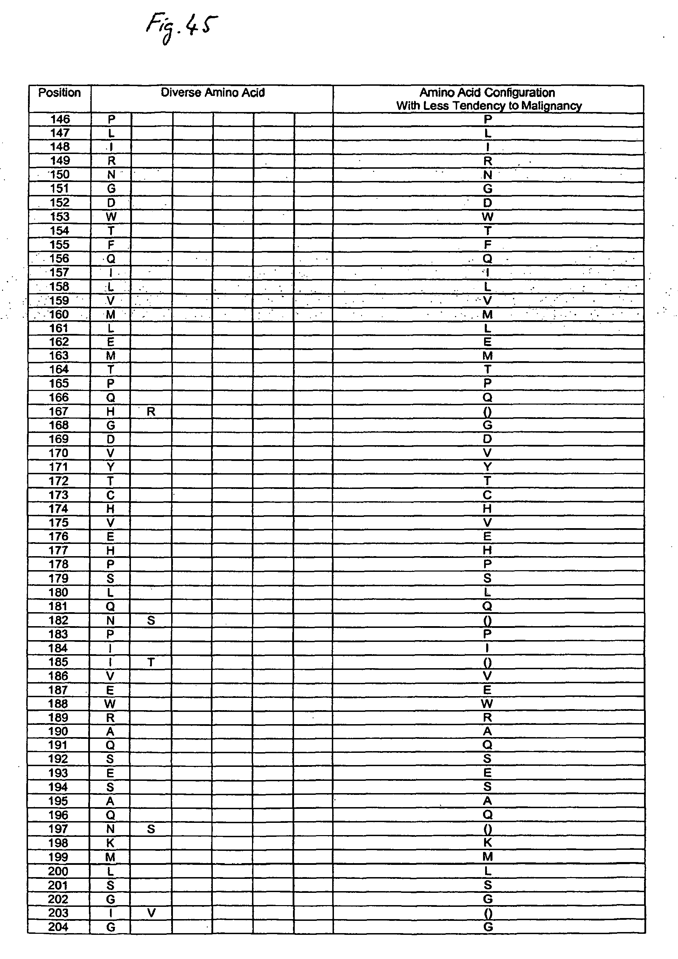

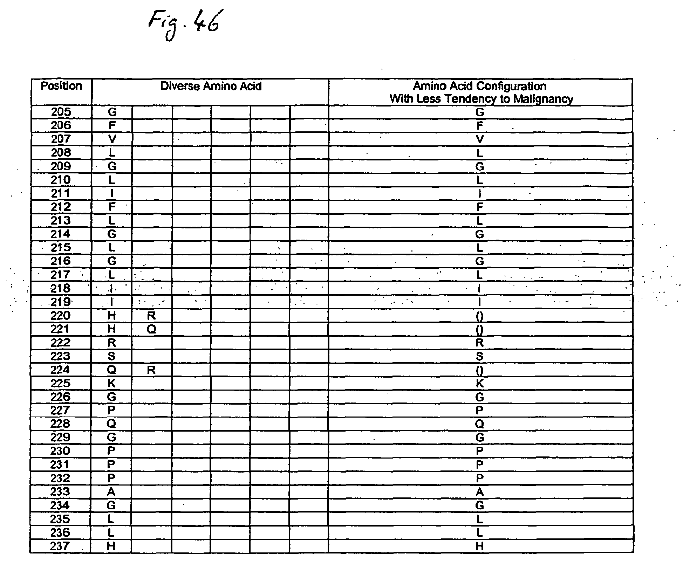

### Other Analysis (Fig. 37 to 46)

Statistical analysis about the polymorphisms on DQB1*genes with respective treatment effects (controlling cancer metastases and being hard to develop to malignant tumor) are carried out. By statistical analysis about the relationships of the amino acid variations on the polymorphic positions with treatment effects, the significance of corresponding positions in the treatment effects are confirmed. This analysis enables choosing the most appropriate treatments for the patients by knowing the diverse amino acids at the polymorphic positions, and analysis of three-dimensional structure of the amino acids can be useful in drug designing. For example, three-dimensional structural analysis by pinpointing the specific amino acids which could suppress cancer metastases is very useful as a method for screening new medicines. Also, there is no doubt that this analysis provides very important significance for gene diagnosis. Signs with brackets in figures mean that the result of either amino acid is the same and there is little significance of diversity. Also, shaded cells in figures mean the positioning at the same level.

### Optimum Amino Acids of DQB1*gene to Inhibit Cancer Metastases

### (Fig. 37 to 41)

Position 14, 77, 87, 116, 125, 203, and 224 are the most important position for variation of optimum amino acids for inhibiting cancer metastases.

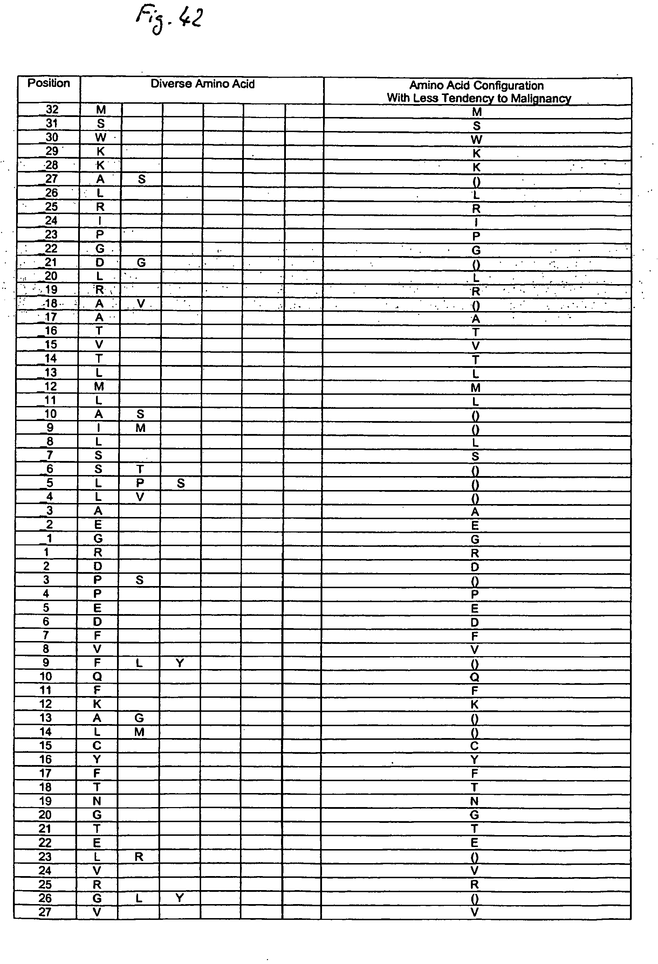

### Optimum Amino Acids of DQB1*gene to Suppress Development of Malignant Tumors (Fig. 42 to 46)

Position 86 is the most important position for variation of optimum amino acids for suppressing development of malignant tumors.

### Industrial Applicability

In accordance with the teaching herein, there is provided a screening method of developing new stomach cancer curative medicines by analyzing polymorphisms at specific positions of DQB1*gene. Since the relationship of the specific positions of the polymorphic amino acid variations with respect to the effect of cancer treatments together with immune ability is clearly realized, very useful means for stomach cancer treatments as cancer treatment sensitivity (patients on whom cancer curative medicines tend to effect), cancer metastasis (patients having more cancer metastases), and more personalized cancer treatment are provided by using variations at the position as markers. It is also useful for developing new medicines with computer graphic technology, and also it improves the extreme efficiency in the efficiency parameters.

**[Table 1]**

| DQ Gene | | | | | | | | |
|---|---|---|---|---|---|---|---|---|
| | Treatment Method | | Rate of Metastases(Total)(%) | | Rate of Lymph Node Metastases(%) | | Rate of Remote Metastases(%) | |
| | Immunotherapy | Chemotherapy | Increase | Decrease | Increase | Decrease | Increase | Decrease |
| 3(PS) | | | PS 342 | PP 26.8 | | | | |
| 14(LM) | | | LL 47.8% | LM 24.5 | LL 36.4% | LM 19.2 | LL 20% | LM 9.6 |
| 19(NS) | | | NS 34.2 | NN 16.8 | | | | |
| 26(GLY) | | | | | LL 30 % | GL 20.1 | | |
| 30(HSY) | H homozygote. HY heterozygote% Y homozygote% | | HH 38.9 | HY 26.9 | | | | |
| 38(AV) | | AV heterozygote%, V homozygote | | | | | | |
| 53(LQ) | | | | | | | | |
| 57(ADS) V) | V heterozygote % | | | | | | | |
| 66(DE) | E homozygote | E homozygote. DE heterozygote% E homozygote | | | | | DD 18.9 | DE11.7 |
| 67(IV) | V homozygote | I homozyogote, IV heterozygote% | | | | | II 18.9 % | IV 11.7 |
| 77(RT) | | | RR 44 % | RT 24.5 | RR 33.3 | RT 19.3 | RR 18.5 | RT 9.6 |
| 85(LV) | G homozygote | | | | | | | |
| 86(AEG) | E homozygote | A homozygote% | | | | | | |
| 87(FLY) | L homozygote. Y homozygote | F homozygote% | YY 39.7% | LY 24.3 | | | | |
| 89(GT) | | | | | | | | |
| 90(IT) | | | | | | | | |
| 116(LV) | | | II 47.8% | IV 24.5 | II 36.4 % | IV 19.2 | II 20 % | IV 9.6 |
| 125(AGS) | | | SS 47.8% | AS 23.9 | | | | |
| 140(AT) | | | | | | | | |
| 182(NS) | | | | | | | | |
| 185(IT) | | | | | | | IT 15.3 % | TT 10 |
| 203(IV) | | | VV 34.4 | IV 27 | | | W 15.6 | IV 11 |
| 220(HR) | | | | | | | | |
| 221(HQ) | | | | | | | | |

### SEQUENCE LISTING

<110> OGOSHI, KYOJI
<120> Examintion method for judging treatment for cancer
<130> GP01-1038
<140> PCT/JP2002/002894
   <141> 2002-03-26
<160> 872
<170> PatentIn version 3.1
<210> 1
   <211> 258
   <212> PRT
   <213> Homo sapiens
<400> 1
<210> 2
   <211> 87
   <212> PRT
   <213> Homo sapiens
<400> 2
   Asn Tyr Val Tyr Gln Gly Arg Gln Glu Cys Tyr Ala Phe Asn Gly Thr
   1               5                   10                  15
   Gln Arg Phe Leu Glu Arg Tyr Ile Tyr Asn Arg Glu Glu Tyr Ala Arg
               20                  25                  30
   Phe Asp Ser Asp Val Gly Glu Phe Arg Ala Val Thr Glu Leu Gly Arg
           35                  40                  45
   Pro Ala Ala Glu Tyr Trp Asn Ser Gln Lys Asp Ile Leu Glu Glu Lys
       50                  55                  60
   Arg Ala Val Pro Asp Arg Val Cys Arg His Asn Tyr Glu Leu Asp Glu
   65                  70                  75                  80
   Ala Val Thr Leu Gln Arg Arg
                   85
<210> 3
   <211> 258
   <212> PRT
   <213> Homo sapiens
<400> 3
   Met Met Val Leu Gln Val Ser Ala Ala Pro Arg Thr Val Ala Leu Thr
   1               5                   10                  15
   Ala Leu Leu Met Val Leu Leu Thr Ser Val Val Gln Gly Arg Ala Thr
               20                  25                  30
   Pro Glu Asn Tyr Leu Phe Gln Gly Arg Gln Glu Cys Tyr Ala Phe Asn
           35                  40                  45
   Gly Thr Gln Arg Phe Leu Glu Arg Tyr Ile Tyr Asn Arg Glu Glu Phe
   50 55 60
   Val Arg Phe Asp Ser Asp Val Gly Glu Phe Arg Ala Val Thr Glu Leu
   65 70 75 80
   Gly Arg Pro Asp Glu Glu Tyr Trp Asn Ser Gln Lys Asp Ile Leu Glu
   85 90 95
   Glu Glu Arg Ala Val Pro Asp Arg Met Cys Arg His Asn Tyr Glu Leu
   100 105 110
   Gly Gly Pro Met Thr Leu Gln Arg Arg Val Gln Pro Arg Val Asn Val
   115 120 125
   Ser Pro Ser Lys Lys Gly Pro Leu Gln His His Asn Leu Leu Val Cys
   130 135 140
   His Val Thr Asp Phe Tyr Pro Gly Ser Ile Gln Val Arg Trp Phe Leu
   145 150 155 160
   Asn Gly Gln Glu Glu Thr Ala Gly Val Val Ser Thr Asn Leu Ile Arg
   165 170 175
   Asn Gly Asp Trp Thr Phe Gln Ile Leu Val Met Leu Glu Met Thr Pro
   180 185 190
   Gln Gln Gly Asp Val Tyr Thr Cys Gln Val Glu His Thr Ser Leu Asp
   195 200 205
   Ser Pro Val Thr Val Glu Trp Lys Ala Gln Ser Asp Ser Ala Arg Ser
   210 215 220
   Lys Thr Leu Thr Gly Ala Gly Gly Phe Val Leu Gly Leu Ile Ile Cys
   225 230 235 240
   Gly Val Gly Ile Phe Met His Arg Arg Ser Lys Lys Val Gln Arg Gly
   245 250 255
   Ser Ala
<210> 4
   <211> 87
   <212> PRT
   <213> Homo sapiens
<400> 4
   Asn Tyr Leu Phe Gln Gly Arg Gln Glu Cys Tyr Ala Phe Asn Gly Thr
   1               5                   10                  15
   Gln Arg Phe Leu Glu Arg Tyr Ile Tyr Asn Arg Glu Glu Phe Val Arg
               20                  25                  30
   Phe Asp Ser Asp Val Gly Glu Phe Arg Ala Val Thr Glu Leu Gly Arg
           35                  40                  45
   Pro Asp Glu Glu Tyr Trp Asn Ser Gln Lys Asp Ile Leu Glu Glu Glu
       50                  55                  60
   Arg Ala Val Pro Asp Arg Met Cys Arg His Asn Tyr Glu Leu Gly Gly
   65                  70                  75                  80
   Pro Met Thr Leu Gln Arg Arg
                   85
<210> 5
   <211> 87
   <212> PRT
   <213> Homo sapiens
<400> 5
   Asn Tyr Leu Phe Gln Gly Arg Gln Glu Cys Tyr Ala Phe Asn Gly Thr
   1               5                   10                  15
   Gln Arg Phe Leu Glu Arg Tyr Ile Tyr Asn Arg Glu Glu Phe Val Arg
               20                  25                  30
   Phe Asp Ser Asp Val Gly Glu Phe Arg Ala Val Thr Glu Leu Gly Arg
           35                  40                  45
   Pro Asp Glu Glu Tyr Trp Asn Ser Gln Lys Asp Ile Leu Glu Glu Glu
       50                  55                  60
   Arg Ala Val Pro Asp Arg Met Cys Arg His Asn Tyr Glu Leu Gly Gly
   65                  70                  75                  80
   Pro Met Thr Leu Gln Arg Arg
                   85
<210> 6
   <211> 85
   <212> PRT
   <213> Homo sapiens
<400> 6
   Asn Tyr Leu Phe Gln Gly Arg Gln Glu Cys Tyr Ala Phe Asn Gly Thr
   1 5 10 15
   Gln Arg Phe Leu Glu Arg Tyr Ile Tyr Asn Arg Glu Glu Leu Val Arg
   20 25 30
   Phe Asp Ser Asp Val Gly Glu Phe Arg Ala Val Thr Glu Leu Gly Arg
   35 40 45
   Pro Glu Ala Glu Tyr Trp Asn Ser Gln Lys Asp Ile Leu Glu Glu Glu
   50 55 60
   Arg Ala Val Pro Asp Arg Met Cys Arg His Asn Tyr Glu Leu Gly Gly
   65 70 75 80
   Pro Met Thr Leu Gln
   85
<210> 7
   <211> 258
   <212> PRT
   <213> Homo sapiens
<400> 7
   Met Met Val Leu Gln Val Ser Ala Ala Pro Arg Thr Val Ala Leu Thr
   1 5 10 15
   Ala Leu Leu Met Val Leu Leu Thr Ser Val Val Gln Gly Arg Ala Thr
   20 25 30
   Pro Glu Asn Tyr Val Tyr Gln Leu Arg Gln Glu Cys Tyr Ala Phe Asn
   35 40 45
   Gly Thr Gln Arg Phe Leu Glu Arg Tyr Ile Tyr Asn Arg Glu Glu Phe
   50 55 60
   Val Arg Phe Asp Ser Asp Val Gly Glu Phe Arg Ala Val Thr Glu Leu
   65 70 75 80
   Gly Arg Pro Asp Glu Asp Tyr Trp Asn Ser Gln Lys Asp Leu Leu Glu
   85 90 95
   Glu Lys Arg Ala Val Pro Asp Arg Val Cys Arg His Asn Tyr Glu Leu
   100 105 110
   Asp Glu Ala Val Thr Leu Gln Arg Arg Val Gln Pro Lys Val Asn Val
   115 120 125
   Ser Pro Ser Lys Lys Gly Pro Leu Gln His His Asn Leu Leu Val Cys
   130 135 140
   His Val Thr Asp Phe Tyr Pro Gly Ser Ile Gln Val Arg Trp Phe Leu
   145 150 155 160
   Asn Gly Gln Glu Glu Thr Ala Gly Val Val Ser Thr Asn Leu Ile Arg
   165 170 175
   Asn Gly Asp Trp Thr Phe Gln Ile Leu Val Met Leu Glu Met Thr Pro
   180 185 190
   Gln Gln Gly Asp Val Tyr Ile Cys Gln Val Glu His Thr Ser Leu Asp
   195 200 205
   Ser Pro Val Thr Val Glu Trp Lys Ala Gln Ser Asp Ser Ala Arg Ser
   210 215 220
   Lys Thr Leu Thr Gly Ala Gly Gly Phe Val Leu Gly Leu Ile Ile Cys
   225 230 235 240
   Gly Val Gly Ile Phe Met His Arg Arg Ser Lys Lys Val Gln Arg Gly
   245 250 255
   Ser Ala
<210> 8
   <211> 87
   <212> PRT
   <213> Homo sapiens
<400> 8
   Asn Tyr Val Tyr Gln Leu Arg Gln Glu Cys Tyr Ala Phe Asn Gly Thr
   1 5 10 15
   Gln Arg Phe Leu Glu Arg Tyr Ile Tyr Asn Arg Glu Glu Phe Val Arg
   20 25 30
   Phe Asp Ser Asp Val Gly Glu Phe Arg Ala Val Thr Glu Leu Gly Arg
   35 40 45
   Pro Asp Glu Asp Tyr Trp Asn Ser Gln Lys Asp Leu Leu Glu Glu Lys
   50 55 60
   Arg Ala Val Pro Asp Arg Val Cys Arg His Asn Tyr Glu Leu Asp Glu
   65 70 75 80
   Ala Val Thr Leu Gln Arg Arg
   85
<210> 9
   <211> 258
   <212> PRT
   <213> Homo sapiens
<400> 9
   Met Met Val Leu Gln Val Ser Ala Ala Pro Arg Thr Val Ala Leu Thr
   1               5                   10                  15
   Ala Leu Leu Met Val Leu Leu Thr Ser Val Val Gln Gly Arg Ala Thr
               20                  25                  30
   Pro Glu Asn Tyr Leu Phe Gln Gly Arg Gln Glu Cys Tyr Ala Phe Asn
           35                  40                  45
   Gly Thr Gln Arg Phe Leu Glu Arg Tyr Ile Tyr Asn Arg Glu Glu Phe
       50                  55                  60
   Ala Arg Phe Asp Ser Asp Val Gly Glu Phe Arg Ala Val Thr Glu Leu
   65                  70                  75                  80
   Gly Arg Pro Ala Ala Glu Tyr Trp Asn Ser Gln Lys Asp Ile Leu Glu
                   85                  90                  95
   Glu Lys Arg Ala Val Pro Asp Arg Met Cys Arg His Asn Tyr Glu Leu
               100                 105                 110
   Gly Gly Pro Met Thr Leu Gln Arg Arg Val Gln Pro Arg Val Asn Val
           115                 120                 125
   Ser Pro Ser Lys Lys Gly Pro Leu Gln His His Asn Leu Leu Val Cys
       130                 135                 140
   His Val Thr Asp Phe Tyr Pro Gly Ser Ile Gln Val Arg Trp Phe Leu
   145                 150                 155                 160
   Asn Gly Gln Glu Glu Thr Ala Gly Val Val Ser Thr Asn Leu Ile Arg
                   165                 170                 175
   Asn Gly Asp Trp Thr Phe Gln Ile Leu Val Met Leu Glu Met Thr Pro
               180                 185                 190
   Gln Gln Gly Asp Val Tyr Thr Cys Gln Val Glu His Thr Ser Leu Asp
           195                 200                 205
   Ser Pro Val Thr Val Glu Trp Lys Ala Gln Ser Asp Ser Ala Arg Ser
       210                 215                 220
   Lys Thr Leu Thr Gly Ala Gly Gly Phe Val Leu Gly Leu Ile Ile Cys
   225                 230                 235                 240
   Gly Val Gly Ile Phe Met His Arg Arg Ser Lys Lys Val Gln Arg Gly
                   245                 250                 255
   Ser Ala
<210> 10
   <211> 229
   <212> PRT
   <213> Homo sapiens
<400> 10
   Arg Ala Thr Pro Glu Asn Tyr Leu Phe Gln Gly Arg Gln Glu Cys Tyr
   1               5                   10                  15
   Ala Phe Asn Gly Thr Gln Arg Phe Leu Glu Arg Tyr Ile Tyr Asn Arg
               20                  25                  30
   Glu Glu Phe Val Arg Phe Asp Ser Asp Val Gly Glu Phe Arg Ala Val
           35                  40                  45
   Thr Glu Leu Gly Arg Pro Asp Glu Glu Tyr Trp Asn Ser Gln Lys Asp
       50                  55                  60
   Ile Leu Glu Glu Lys Arg Ala Val Pro Asp Arg Met Cys Arg His Asn
   65                  70                  75                  80
   Tyr Glu Leu Gly Gly Pro Met Thr Leu Gln Arg Arg Val Gln Pro Arg
                   85                  90                  95
   Val Asn Val Ser Pro Ser Lys Lys Gly Pro Leu Gln His His Asn Leu
               100                 105                 110
   Leu Val Cys His Val Thr Asp Phe Tyr Pro Gly Ser Ile Gln Val Arg
           115                 120                 125
   Trp Phe Leu Asn Gly Gln Glu Glu Thr Ala Gly Val Val Ser Thr Asn
       130                 135                 140
   Leu Ile Arg Asn Gly Asp Trp Thr Phe Gln Ile Leu Val Met Leu Glu
   145                 150                 155                 160
   Met Thr Pro Gln Gln Gly Asp Val Tyr Thr Cys Gln Val Glu His Thr
                   165                 170                 175
   Ser Met Asp Ser Pro Val Thr Val Glu Trp Lys Ala Gln Ser Asp Ser
               180                 185                 190
   Ala Arg Ser Lys Thr Leu Thr Gly Ala Gly Gly Phe Val Leu Gly Leu
           195                 200                 205
   Ile Ile Cys Gly Val Gly Ile Phe Met His Arg Arg Ser Lys Lys Val
       210                 215                 220
   Gln Arg Gly Ser Ala
   225
<210> 11
   <211> 85
   <212> PRT
   <213> DPB1*0501
<400> 11
   Asn Tyr Leu Phe Gln Gly Arg Gln Glu Cys Tyr Ala Phe Asn Gly Thr
   1               5                   10                  15
   Gln Arg Phe Leu Glu Arg Tyr Ile Tyr Asn Arg Glu Glu Leu Val Arg
               20                  25                  30
   Phe Asp Ser Asp Val Gly Glu Phe Arg Ala Val Thr Glu Leu Gly Arg
           35                  40                  45
   Pro Glu Ala Glu Tyr Trp Asn Ser Gln Lys Asp Ile Leu Glu Glu Lys
       50                  55                  60
   Arg Ala Val Pro Asp Arg Met Cys Arg His Asn Tyr Glu Leu Asp Glu
   65                  70                  75                  80
   Ala Val Thr Leu Gln
                   85
<210> 12
   <211> 85
   <212> PRT
   <213> Homo sapiens
<400> 12
   Asn Tyr Val Tyr Gln Leu Arg Gln Glu Cys Tyr Ala Phe Asn Gly Thr
   1               5                   10                  15
   Gln Arg Phe Leu Glu Arg Tyr Ile Tyr Asn Arg Glu Glu Phe Val Arg
               20                  25                  30
   Phe Asp Ser Asp Val Gly Glu Phe Arg Ala Val Thr Glu Leu Gly Arg
           35                  40                  45
   Pro Asp Glu Asp Tyr Trp Asn Ser Gln Lys Asp Leu Leu Glu Glu Glu
       50                  55                  60
   Arg Ala Val Pro Asp Arg Met Cys Arg His Asn Tyr Glu Leu Asp Glu
   65                  70                  75                  80
   Ala Val Thr Leu Gln
                   85
<210> 13
   <211> 83
   <212> PRT
   <213> Homo sapiens
<400> 13
   Leu Phe Gln Gly Arg Gln Glu Cys Tyr Ala Phe Asn Gly Thr Gln Arg
   1               5                   10                  15
   Phe Leu Glu Arg Tyr Ile Tyr Asn Arg Glu Glu Phe Val Arg Phe Asp
               20                  25                  30
   Ser Asp Val Gly Glu Phe Arg Ala Val Thr Glu Leu Gly Arg Pro Asp
           35                  40                  45
   Glu Glu Tyr Trp Asn Ser Gln Lys Asp Ile Leu Glu Glu Glu Arg Ala
       50                  55                  60
   Val Pro Asp Arg Val Cys Arg His Asn Tyr Glu Leu Asp Glu Ala Val
   65                  70                  75                  80
   Thr Leu Gln
<210> 14
   <211> 85
   <212> PRT
   <213> Homo sapiens
<400> 14
   Asn Tyr Val His Gln Leu Arg Gln Glu Cys Tyr Ala Phe Asn Gly Thr
   1               5                   10                  15
   Gln Arg Phe Leu Glu Arg Tyr Ile Tyr Asn Arg Glu Glu Phe Val Arg
               20                  25                  30
   Phe Asp Ser Asp Val Gly Glu Phe Arg Ala Val Thr Glu Leu Gly Arg
           35                  40                  45
   Pro Asp Glu Asp Tyr Trp Asn Ser Gln Lys Asp Ile Leu Glu Glu Glu
       50                  55                  60
   Arg Ala Val Pro Asp Arg Val Cys Arg His Asn Tyr Glu Leu Asp Glu
   65                  70                  75                  80
   Ala Val Thr Leu Gln
                   85
<210> 15
   <211> 85
   <212> PRT
   <213> Homo sapiens
<400> 15
   Asn Tyr Val His Gln Leu Arg Gln Glu Cys Tyr Ala Phe Asn Gly Thr
   1               5                   10                  15
   Gln Arg Phe Leu Glu Arg Tyr Ile Tyr Asn Arg Glu Glu Phe Val Arg
               20                  25                  30
   Phe Asp Ser Asp Val Gly Glu Phe Arg Ala Val Thr Glu Leu Gly Arg
           35                  40                  45
   Pro Asp Glu Glu Tyr Trp Asn Ser Gln Lys Asp Ile Leu Glu Glu Glu
       50                  55                  60
   Arg Ala Val Pro Asp Arg Val Cys Arg His Asn Tyr Glu Leu Asp Glu
   65                  70                  75                  80
   Ala Val Thr Leu Gln
                   85

<210> 16
   <211> 83
   <212> PRT
   <213> Homo sapiens
<400> 16
   Val Tyr Gln Leu Arg Gln Glu Cys Tyr Ala Phe Asn Gly Thr Gln Arg
   1               5                   10                  15
   Phe Leu Glu Arg Tyr Ile Tyr Asn Arg Gln Glu Tyr Ala Arg Phe Asp
               20                  25                  30
   Ser Asp Val Gly Glu Phe Arg Ala Val Thr Glu Leu Gly Arg Pro Ala
           35                  40                  45
   Ala Glu Tyr Trp Asn Ser Gln Lys Asp Leu Leu Glu Glu Arg Arg Ala
       50                  55                  60
   Val Pro Asp Arg Met Cys Arg His Asn Tyr Glu Leu Asp Glu Ala Val
   65                  70                  75                  80
   Thr Leu Gln
<210> 17
   <211> 85
   <212> PRT
   <213> Homo sapiens
<400> 17
   Asn Tyr Val Tyr Gln Leu Arg Gln Glu Cys Tyr Ala Phe Asn Gly Thr
   1               5                   10                  15
   Gln Arg Phe Leu Glu Arg Tyr Ile Tyr Asn Arg Gln Glu Tyr Ala Arg
               20                  25                  30
   Phe Asp Ser Asp Val Gly Glu Phe Arg Ala Val Thr Glu Leu Gly Arg
           35                  40                  45
   Pro Ala Ala Glu Tyr Trp Asn Ser Gln Lys Asp Leu Leu Glu Glu Arg
       50                  55                  60
   Arg Ala Val Pro Asp Arg Met Cys Arg His Asn Tyr Glu Leu Asp Glu
   65                  70                  75                  80
   Ala Val Thr Leu Gln
                   85
<210> 18
   <211> 85
   <212> PRT
   <213> Homo sapiens
<400> 18
   Asn Tyr Val Tyr Gln Leu Arg Gln Glu Cys Tyr Ala Phe Asn Gly Thr
   1               5                   10                  15
   Gln Arg Phe Leu Glu Arg Tyr Ile Tyr Asn Arg Glu Glu Tyr Ala Arg
               20                  25                  30
   Phe Asp Ser Asp Val Gly Glu Phe Arg Ala Val Thr Glu Leu Gly Arg
           35                  40                  45
   Pro Ala Ala Glu Tyr Trp Asn Ser Gln Lys Asp Ile Leu Glu Glu Glu
       50                  55                  60
   Arg Ala Val Pro Asp Arg Ile Cys Arg His Asn Tyr Glu Leu Asp Glu
   65                  70                  75                  80
   Ala Val Thr Leu Gln
                   85
<210> 19
   <211> 85
   <212> PRT
   <213> Homo sapiens
<400> 19
   Asn Tyr Val His Gln Leu Arg Gln Glu Cys Tyr Ala Phe Asn Gly Thr
   1 5 10 15
   Gln Arg Phe Leu Glu Arg Tyr Ile Tyr Asn Arg Glu Glu Phe Val Arg
   20 25 30
   Phe Asp Ser Asp Val Gly Glu Phe Arg Ala Val Thr Glu Leu Gly Arg
   35 40 45
   Pro Asp Glu Asp Tyr Trp Asn Ser Gln Lys Asp Leu Leu Glu Glu Lys
   50 55 60
   Arg Ala Val Pro Asp Arg Val Cys Arg His Asn Tyr Glu Leu Asp Glu
   65 70 75 80
   Ala Val Thr Leu Gln
   85
<210> 20
   <211> 85
   <212> PRT
   <213> Homo sapiens
<400> 20
   Asn Tyr Val Tyr Gln Gly Arg Gln Glu Cys Tyr Ala Phe Asn Gly Thr
   1 5 10 15
   Gln Arg Phe Leu Glu Arg Tyr Ile Tyr Asn Arg Gln Glu Tyr Ala Arg
   20 25 30
   Phe Asp Ser Asp Val Gly Glu Phe Arg Ala Val Thr Glu Leu Gly Arg
   35 40 45
   Pro Ala Ala Glu Tyr Trp Asn Ser Gln Lys Asp Leu Leu Glu Glu Arg
   50 55 60
   Arg Ala Val Pro Asp Arg Met Cys Arg His Asn Tyr Glu Leu Val Gly
   65 70 75 80
   Pro Met Thr Leu Gln
   85
<210> 21
   <211> 85
   <212> PRT
   <213> Homo sapiens
<400> 21
   Asn Tyr Leu Phe Gln Gly Arg Gln Glu Cys Tyr Ala Phe Asn Gly Thr
   1               5                   10                  15
   Gln Arg Phe Leu Glu Arg Tyr Ile Tyr Asn Arg Glu Glu Phe Val Arg
               20                  25                  30
   Phe Asp Ser Asp Val Gly Glu Phe Arg Ala Val Thr Glu Leu Gly Arg
           35                  40                  45
   Pro Asp Glu Glu Tyr Trp Asn Ser Gln Lys Asp Ile Leu Glu Glu Glu
       50                  55                  60
   Arg Ala Val Pro Asp Arg Met Cys Arg His Asn Tyr Glu Leu Asp Glu
   65                  70                  75                  80
   Ala Val Thr Leu Gln
                   85
<210> 22
   <211> 85
   <212> PRT
   <213> Homo sapiens
<400> 22
   Asn Tyr Val His Gln Leu Arg Gln Glu Cys Tyr Ala Phe Asn Gly Thr
   1               5                   10                  15
   Gln Arg Phe Leu Glu Arg Tyr Ile Tyr Asn Arg Glu Glu Phe Val Arg
               20                  25                  30
   Phe Asp Ser Asp Val Gly Glu Phe Arg Ala Val Thr Glu Leu Gly Arg
           35                  40                  45
   Pro Asp Glu Asp Tyr Trp Asn Ser Gln Lys Asp Ile Leu Glu Glu Glu
       50                  55                  60
   Arg Ala Val Pro Asp Arg Met Cys Arg His Asn Tyr Glu Leu Asp Glu
   65                  70                  75                  80
   Ala Val Thr Leu Gln
                   85
<210> 23
   <211> 83
   <212> PRT
   <213> Homo sapiens
<400> 23
   Val Tyr Gln Gly Arg Gln Glu Cys Tyr Ala Phe Asn Gly Thr Gln Arg
   1               5                   10                  15
   Phe Leu Glu Arg Tyr Ile Tyr Asn Arg Glu Glu Phe Val Arg Phe Asp
               20                  25                  30
   Ser Asp Val Gly Glu Phe Arg Ala Val Thr Glu Leu Gly Arg Pro Asp
           35                  40                  45
   Glu Glu Tyr Trp Asn Ser Gln Lys Asp Ile Leu Glu Glu Lys Arg Ala
       50                  55                  60
   Val Pro Asp Arg Met Cys Arg His Asn Tyr Glu Leu Val Gly Pro Met
   65                  70                  75                  80
   Thr Leu Gln
<210> 24
   <211> 85
   <212> PRT
   <213> Homo sapiens
<400> 24
   Asn Tyr Leu Phe Gln Gly Arg Gln Glu Cys Tyr Ala Phe Asn Gly Thr
   1               5                   10                  15
   Gln Arg Phe Leu Glu Arg Tyr Ile Tyr Asn Arg Glu Glu Phe Val Arg
               20                  25                  30
   Phe Asp Ser Asp Val Gly Glu Phe Arg Ala Val Thr Glu Leu Gly Arg
           35                  40                  45
   Pro Glu Ala Glu Tyr Trp Asn Ser Gln Lys Asp Ile Leu Glu Glu Glu
       50                  55                  60
   Arg Ala Val Pro Asp Arg Ile Cys Arg His Asn Tyr Glu Leu Asp Glu
   65                  70                  75                  80
   Ala Val Thr Leu Gln
                   85
<210> 25
   <211> 87
   <212> PRT
   <213> Homo sapiens
<400> 25
   Asn Tyr Val Tyr Gln Leu Arg Gln Glu Cys Tyr Ala Phe Asn Gly Thr
   1               5                   10                  15
   Gln Arg Phe Leu Glu Arg Tyr Ile Tyr Asn Arg Glu Glu Phe Val Arg
               20                  25                  30
   Phe Asp Ser Asp Val Gly Glu Phe Arg Ala Val Thr Glu Leu Gly Arg
           35                  40                  45
   Pro Asp Glu Asp Tyr Trp Asn Ser Gln Lys Asp Leu Leu Glu Glu Lys
       50                  55                  60
   Arg Ala Val Pro Asp Arg Met Cys Arg His Asn Tyr Glu Leu Asp Glu
   65                  70                  75                  80
   Ala Val Thr Leu Gln Arg Arg
                   85
<210> 26
   <211> 87
   <212> PRT
   <213> Homo sapiens
<400> 26
   Asn Tyr Val Tyr Gln Leu Arg Gln Glu Cys Tyr Ala Phe Asn Gly Thr
   1               5                   10                  15
   Gln Arg Phe Leu Glu Arg Tyr Ile Tyr Asn Arg Glu Glu Phe Val Arg
               20                  25                  30
   Phe Asp Ser Asp Val Gly Glu Phe Arg Ala Val Thr Glu Leu Gly Arg
           35                  40                  45
   Pro Asp Glu Asp Tyr Trp Asn Ser Gln Lys Asp Leu Leu Glu Glu Lys
       50                  55                  60
   Arg Ala Val Pro Asp Arg Met Cys Arg His Asn Tyr Glu Leu Asp Glu
   65                  70                  75                  80
   Ala Val Thr Leu Gln Arg Arg
                   85
<210> 27
   <211> 87
   <212> PRT
   <213> Homo sapiens
<400> 27
   Asn Tyr Val Tyr Gln Leu Arg Gln Glu Cys Tyr Ala Phe Asn Gly Thr
   1 5 10 15
   Gln Arg Phe Leu Glu Arg Tyr Ile Tyr Asn Arg Glu Glu Leu Val Arg
   20 25 30
   Phe Asp Ser Asp Val Gly Glu Phe Arg Ala Val Thr Glu Leu Gly Arg
   35 40 45
   Pro Glu Ala Glu Tyr Trp Asn Ser Gln Lys Asp Ile Leu Glu Glu Glu
   50 55 60
   Arg Ala Val Pro Asp Arg Met Cys Arg His Asn Tyr Glu Leu Asp Glu
   65 70 75 80
   Ala Val Thr Leu Gln Arg Arg
   85
<210> 28
   <211> 87
   <212> PRT
   <213> Homo sapiens
<400> 28
   Asn Tyr Leu Phe Gln Gly Arg Gln Glu Cys Tyr Ala Phe Asn Gly Thr
   1 5 10 15
   Gln Arg Phe Leu Glu Arg Tyr Ile Tyr Asn Arg Glu Glu Leu Val Arg
   20 25 30
   Phe Asp Ser Asp Val Gly Glu Phe Arg Ala Val Thr Glu Leu Gly Arg
   35 40 45
   Pro Glu Ala Glu Tyr Trp Asn Ser Gln Lys Asp Ile Leu Glu Glu Glu
   50 55 60
   Arg Ala Val Pro Asp Arg Met Cys Arg His Asn Tyr Glu Leu Asp Glu
   65 70 75 80
   Ala Val Thr Leu Gln Arg Arg
   85
<210> 29
   <211> 87
   <212> PRT
   <213> Homo sapiens
<400> 29
   Asn Tyr Leu Phe Gln Gly Arg Gln Glu Cys Tyr Ala Phe Asn Gly Thr
   1               5                   10                  15
   Gln Arg Phe Leu Glu Arg Tyr Ile Tyr Asn Arg Glu Glu Phe Val Arg
               20                  25                  30
   Phe Asp Ser Asp Val Gly Glu Phe Arg Ala Val Thr Glu Leu Gly Arg
           35                  40                  45
   Pro Ala Ala Glu Tyr Trp Asn Ser Gln Lys Asp Ile Leu Glu Glu Lys
       50                  55                  60
   Arg Ala Val Pro Asp Arg Met Cys Arg His Asn Tyr Glu Leu Gly Gly
   65                  70                  75                  80
   Pro Met Thr Leu Gln Arg Arg
                   85
<210> 30
   <211> 87
   <212> PRT
   <213> Homo sapiens
<400> 30
   Asn Tyr Leu Phe Gln Gly Arg Gln Glu Cys Tyr Ala Phe Asn Gly Thr
   1               5                   10                  15
   Gln Arg Phe Leu Glu Arg Tyr Ile Tyr Asn Arg Glu Glu Phe Ala Arg
               20                  25                  30
   Phe Asp Ser Asp Val Gly Glu Phe Arg Ala Val Thr Glu Leu Gly Arg
           35                  40                  45
   Pro Glu Ala Glu Tyr Trp Asn Ser Gln Lys Asp Ile Leu Glu Glu Lys
       50                  55                  60
   Arg Ala Val Pro Asp Arg Met Cys Arg His Asn Tyr Glu Leu Gly Gly
   65                  70                  75                  80
   Pro Met Thr Leu Gln Arg Arg
                   85
<210> 31
   <211> 87
   <212> PRT
   <213> Homo sapiens
<400> 31
   Asn Tyr Val Tyr Gln Leu Arg Gln Glu Cys Tyr Ala Phe Asn Gly Thr
   1               5                   10                  15
   Gln Arg Phe Leu Glu Arg Tyr Ile Tyr Asn Arg Glu Glu Phe Val Arg
               20                  25                  30
   Phe Asp Ser Asp Val Gly Glu Phe Arg Ala Val Thr Glu Leu Gly Arg
           35                  40                  45
   Pro Asp Glu Glu Tyr Trp Asn Ser Gln Lys Asp Leu Leu Glu Glu Lys
       50                  55                  60
   Arg Ala Val Pro Asp Arg Val Cys Arg His Asn Tyr Glu Leu Asp Glu
   65                  70                  75                  80
   Ala Val Thr Leu Gln Arg Arg
                   85
<210> 32
   <211> 85
   <212> PRT
   <213> Homo sapiens
<400> 32
   Val Tyr Gln Leu Arg Gln Glu Cys Tyr Ala Phe Asn Gly Thr Gln Arg
   1               5                   10                  15
   Phe Leu Glu Arg Tyr Ile Tyr Asn Arg Glu Glu Tyr Ala Arg Phe Asp
               20                  25                  30
   Ser Asp Val Gly Glu Phe Arg Ala Val Thr Glu Leu Gly Arg Pro Ala
           35                  40                  45
   Ala Glu Tyr Trp Asn Ser Gln Lys Asp Ile Leu Glu Glu Lys Arg Ala
       50                  55                  60
   Val Pro Asp Arg Val Cys Arg His Asn Tyr Glu Leu Asp Glu Ala Val
   65                  70                  75                  80
   Thr Leu Gln Arg Arg
                   85
<210> 33
   <211> 85
   <212> PRT
   <213> Homo sapiens
<400> 33
   Val Tyr Gln Leu Arg Gln Glu Cys Tyr Ala Phe Asn Gly Thr Gln Arg
   1 5 10 15
   Phe Leu Glu Arg Tyr Ile Tyr Asn Arg Glu Glu Tyr Ala Arg Phe Asp
   20 25 30
   Ser Asp Val Gly Glu Phe Arg Ala Val Thr Glu Leu Gly Arg Pro Ala
   35 40 45
   Ala Glu Tyr Trp Asn Ser Gln Lys Asp Ile Leu Glu Glu Lys Arg Ala
   50 55 60
   Val Pro Asp Arg Val Cys Arg His Asn Tyr Glu Leu Asp Glu Ala Val
   65 70 75 80
   Thr Leu Gln Arg Arg
   85
<210> 34
   <211> 87
   <212> PRT
   <213> Homo sapiens
<400> 34
   Asn Tyr Val Tyr Gln Leu Arg Gln Glu Cys Tyr Ala Phe Asn Gly Thr
   1 5 10 15
   Gln Arg Phe Leu Glu Arg Tyr Ile Tyr Asn Arg Glu Glu Tyr Ala Arg
   20 25 30
   Phe Asp Ser Asp Val Gly Glu Phe Arg Ala Val Thr Glu Leu Gly Arg
   35 40 45
   Pro Ala Ala Glu Tyr Trp Asn Ser Gln Lys Asp Ile Leu Glu Glu Lys
   50 55 60
   Arg Ala Val Pro Asp Arg Met Cys Arg His Asn Tyr Glu Leu Asp Glu
   65 70 75 80
   Ala Val Thr Leu Gln Arg Arg
   85
<210> 35
   <211> 87
   <212> PRT
   <213> Homo sapiens
<400> 35
   Asn Tyr Leu Phe Gln Gly Arg Gln Glu Cys Tyr Ala Phe Asn Gly Thr
   1 5 10 15
   Gln Arg Phe Leu Glu Arg Tyr Ile Tyr Asn Arg Glu Glu Phe Ala Arg
   20 25 30
   Phe Asp Ser Asp Val Gly Glu Phe Arg Ala Val Thr Glu Leu Gly Arg
   35 40 45
   Pro Asp Glu Glu Tyr Trp Asn Ser Gln Lys Asp Leu Leu Glu Glu Lys
   50 55 60
   Arg Ala Val Pro Asp Arg Met Cys Arg His Asn Tyr Glu Leu Val Gly
   65 70 75 80
   Pro Met Thr Leu Gln Arg Arg
   85
<210> 36
   <211> 87
   <212> PRT
   <213> Homo sapiens
<400> 36
   Asn Tyr Val Tyr Gln Leu Arg Gln Glu Cys Tyr Ala Phe Asn Gly Thr
   1 5 10 15
   Gln Arg Phe Leu Glu Arg Tyr Ile Tyr Asn Arg Glu Glu Phe Val Arg
   20 25 30
   Phe Asp Ser Asp Val Gly Glu Phe Arg Ala Val Thr Glu Leu Gly Arg
   35 40 45
   Pro Asp Glu Asp Tyr Trp Asn Ser Gln Lys Asp Leu Leu Glu Glu Glu
   50 55 60
   Arg Ala Val Pro Asp Arg Val Cys Arg His Asn Tyr Glu Leu Asp Glu
   65 70 75 80
   Ala Val Thr Leu Gln Arg Arg
   85
<210> 37
   <211> 85
   <212> PRT
   <213> Homo sapiens
<400> 37
   Asn Tyr Val His Gln Leu Arg Gln Glu Cys Tyr Ala Phe Asn Gly Thr
   1               5                   10                  15
   Gln Arg Phe Leu Glu Arg Tyr Ile Tyr Asn Arg Glu Glu Phe Val Arg
               20                  25                  30
   Phe Asp Ser Asp Val Gly Glu Phe Arg Ala Val Thr Glu Leu Gly Arg
           35                  40                  45
   Pro Glu Ala Glu Tyr Trp Asn Ser Gln Lys Asp Ile Leu Glu Glu Glu
       50                  55                  60
   Arg Ala Val Pro Asp Arg Met Cys Arg His Asn Tyr Glu Leu Asp Glu
   65                  70                  75                  80
   Ala Val Thr Leu Gln
                   85
<210> 38
   <211> 87
   <212> PRT
   <213> Homo sapiens
<400> 38
   Asn Tyr Leu Phe Gln Gly Arg Gln Glu Cys Tyr Ala Phe Asn Gly Thr
   1               5                   10                  15
   Gln Arg Phe Leu Glu Arg Tyr Ile Tyr Asn Arg Glu Glu Phe Ala Arg
               20                  25                  30
   Phe Asp Ser Asp Val Gly Glu Phe Arg Ala Val Thr Glu Leu Gly Arg
           35                  40                  45
   Pro Ala Ala Glu Tyr Trp Asn Ser Gln Lys Asp Leu Leu Glu Glu Lys
       50                  55                  60
   Arg Ala Leu Pro Asp Arg Met Cys Arg His Asn Tyr Glu Leu Asp Glu
   65                  70                  75                  80
   Ala Val Thr Leu Gln Arg Arg
                   85
<210> 39
   <211> 87
   <212> PRT
   <213> Homo sapiens
<400> 39
   Asn Tyr Leu Phe Gln Gly Arg Gln Glu Cys Tyr Ala Phe Asn Gly Thr
   1 5 10 15
   Gln Arg Phe Leu Glu Arg Tyr Ile Tyr Asn Arg Glu Glu Phe Val Arg
   20 25 30
   Phe Asp Ser Asp Val Gly Glu Phe Arg Ala Val Thr Glu Leu Gly Arg
   35 40 45
   Pro Asp Glu Val Tyr Trp Asn Ser Gln Lys Asp Ile Leu Glu Glu Glu
   50 55 60
   Arg Ala Val Pro Asp Arg Met Cys Arg His Asn Tyr Glu Leu Gly Gly
   65 70 75 80
   Pro Met Thr Leu Gln Arg Arg
   85
<210> 40
   <211> 85
   <212> PRT
   <213> Homo sapiens
<400> 40
   Asn Tyr Leu Phe Gln Gly Arg Gln Glu Cys Tyr Ala Phe Asn Gly Thr
   1 5 10 15
   Gln Arg Phe Leu Glu Arg Tyr Ile Tyr Asn Arg Glu Glu Phe Ala Arg
   20 25 30
   Phe Asp Ser Asp Val Gly Glu Phe Arg Ala Val Thr Glu Leu Gly Arg
   35 40 45
   Pro Ala Ala Glu Tyr Trp Asn Ser Gln Lys Asp Ile Leu Glu Glu Glu
   50 55 60
   Arg Ala Val Pro Asp Arg Met Cys Arg His Asn Tyr Glu Leu Gly Gly
   65 70 75 80
   Pro Met Thr Leu Gln
   85
<210> 41
   <211> 85
   <212> PRT
   <213> Homo sapiens
<400> 41
   Asn Tyr Leu Phe Gln Gly Arg Gln Glu Cys Tyr Ala Phe Asn Gly Thr
   1 5 10 15
   Gln Arg Phe Leu Glu Arg Tyr Ile Tyr Asn Arg Glu Glu Leu Val Arg
   20 25 30
   Phe Asp Ser Asp Val Gly Glu Phe Arg Ala Val Thr Glu Leu Gly Arg
   35 40 45
   Pro Ala Ala Glu Tyr Trp Asn Ser Gln Lys Asp Leu Leu Glu Glu Lys
   50 55 60
   Arg Ala Leu Pro Asp Arg Met Cys Arg His Asn Tyr Glu Leu Val Gly
   65 70 75 80
   Pro Met Thr Leu Gln
   85
<210> 42
   <211> 85
   <212> PRT
   <213> Homo sapiens
<400> 42
   Asn Tyr Val His Gln Leu Arg Gln Glu Cys Tyr Ala Phe Asn Gly Thr
   1 5 10 15
   Gln Arg Phe Leu Glu Arg Tyr Ile Tyr Asn Arg Glu Glu Phe Val Arg
   20 25 30
   Phe Asp Ser Asp Val Gly Glu Phe Arg Ala Val Thr Glu Leu Gly Arg
   35 40 45
   Pro Asp Glu Asp Tyr Trp Asn Ser Gln Lys Asp Ile Leu Glu Glu Lys
   50 55 60
   Arg Ala Val Pro Asp Arg Val Cys Arg His Asn Tyr Glu Leu Asp Glu
   65 70 75 80
   Ala Val Thr Leu Gln
   85

<210> 43
   <211> 87
   <212> PRT
   <213> Homo sapiens
<400> 43
   Asn Tyr Val Tyr Gln Leu Arg Gln Glu Cys Tyr Ala Phe Asn Gly Thr
   1 5 10 15
   Gln Arg Phe Leu Glu Arg Tyr Ile Tyr Asn Arg Glu Glu Leu Val Arg
   20 25 30
   Phe Asp Ser Asp Val Gly Glu Phe Arg Ala Val Thr Glu Leu Gly Arg
   35 40 45
   Pro Glu Ala Glu Tyr Trp Asn Ser Gln Lys Asp Ile Leu Glu Glu Lys
   50 55 60
   Arg Ala Val Pro Asp Arg Met Cys Arg His Asn Tyr Glu Leu Asp Glu
   65 70 75 80
   Ala Val Thr Leu Gln Arg Arg
   85
<210> 44
   <211> 85
   <212> PRT
   <213> Homo sapiens
<400> 44
   Val Tyr Gln Leu Arg Gln Glu Cys Tyr Ala Phe Asn Gly Thr Gln Arg
   1 5 10 15
   Phe Leu Glu Arg Tyr Ile Tyr Asn Arg Glu Glu Phe Val Arg Phe Asp
   20 25 30
   Ser Asp Val Gly Glu Phe Arg Ala Val Thr Glu Leu Gly Arg Pro Asp
   35 40 45
   Glu Glu Tyr Trp Asn Ser Gln Lys Asp Ile Leu Glu Glu Glu Arg Ala
   50 55 60
   Val Pro Asp Arg Val Cys Arg His Asn Tyr Glu Leu Asp Glu Ala Val
   65 70 75 80
   Thr Leu Gln Arg Arg
   85
<210> 45
   <211> 83
   <212> PRT
   <213> Homo sapiens
<400> 45
   Leu Phe Gln Gly Arg Gln Glu Cys Tyr Pro Phe Asn Gly Thr Gln Arg
   1 5 10 15
   Phe Leu Glu Arg Tyr Ile Tyr Asn Arg Glu Glu Leu Val Arg Phe Asp
   20 25 30
   Ser Asp Val Gly Glu Phe Arg Ala Val Thr Glu Leu Gly Arg Pro Glu
   35 40 45
   Ala Glu Tyr Trp Asn Ser Gln Lys Asp Ile Leu Glu Glu Lys Arg Ala
   50 55 60
   Val Pro Asp Arg Met Cys Arg His Asn Tyr Glu Leu Asp Glu Ala Val
   65 70 75 80
   Thr Leu Gln
<210> 46
   <211> 87
   <212> PRT
   <213> Homo sapiens
<400> 46
   Asn Tyr Leu Phe Gln Gly Arg Gln Glu Cys Tyr Ala Phe Asn Gly Thr
   1 5 10 15
   Gln Arg Phe Leu Glu Arg Tyr Ile Tyr Asn Arg Glu Glu Tyr Ala Arg
   20 25 30
   Phe Asp Ser Asp Val Gly Glu Phe Arg Ala Val Thr Glu Leu Gly Arg
   35 40 45
   Pro Ala Ala Glu Tyr Trp Asn Ser Gln Lys Asp Ile Leu Glu Glu Lys
   50 55 60
   Arg Ala Val Pro Asp Arg Met Cys Arg His Asn Tyr Glu Leu Gly Gly
   65 70 75 80
   Pro Met Thr Leu Gln Arg Arg
   85
<210> 47
   <211> 87
   <212> PRT
   <213> Homo sapiens
<400> 47
   Asn Tyr Leu Phe Gln Gly Arg Gln Glu Cys Tyr Ala Phe Asn Gly Thr
   1               5                   10                  15
   Gln Arg Phe Leu Glu Arg Tyr Ile Tyr Asn Arg Glu Glu Tyr Ala Arg
               20                  25                  30
   Phe Asp Ser Asp Val Gly Glu Phe Arg Ala Val Thr Glu Leu Gly Arg
           35                  40                  45
   Pro Ala Ala Glu Tyr Trp Asn Ser Gln Lys Asp Ile Leu Glu Glu Lys
       50                  55                  60
   Arg Ala Val Pro Asp Arg Met Cys Arg His Asn Tyr Glu Leu Val Gly
   65                  70                  75                  80
   Pro Met Thr Leu Gln Arg Arg
                   85
<210> 48
   <211> 87
   <212> PRT
   <213> Homo sapiens
<400> 48
   Asn Tyr Leu Phe Gln Gly Arg Gln Glu Cys Tyr Ala Phe Asn Gly Thr
   1               5                   10                  15
   Gln Arg Phe Leu Glu Arg Tyr Ile Tyr Asn Arg Glu Glu Phe Val Arg
               20                  25                  30
   Phe Asp Ser Asp Val Gly Glu Phe Arg Ala Val Thr Glu Leu Gly Arg
           35                  40                  45
   Pro Asp Glu Glu Tyr Trp Asn Ser Gln Lys Asp Phe Leu Glu Glu Glu
       50                  55                  60
   Arg Ala Val Pro Asp Arg Met Cys Arg His Asn Tyr Glu Leu Gly Gly
   65                  70                  75                  80
   Pro Met Thr Leu Gln Arg Arg
                   85
<210> 49
   <211> 87
   <212> PRT
   <213> Homo sapiens
<400> 49
   Asn Tyr Val Tyr Gln Leu Arg Gln Glu Cys Tyr Ala Phe Asn Gly Thr
   1               5                   10                  15
   Gln Arg Phe Leu Glu Arg Tyr Ile Tyr Asn Arg Glu Glu Leu Val Arg
               20                  25                  30
   Phe Asp Ser Asp Val Gly Glu Phe Arg Ala Val Thr Glu Leu Gly Arg
           35                  40                  45
   Pro Asp Glu Asp Tyr Trp Asn Ser Gln Lys Asp Leu Leu Glu Glu Glu
       50                  55                  60
   Arg Ala Val Pro Asp Arg Val Cys Arg His Asn Tyr Glu Leu Asp Glu
   65                  70                  75                  80
   Ala Val Thr Leu Gln Arg Arg
                   85
<210> 50
   <211> 83
   <212> PRT
   <213> Homo sapiens
<400> 50
   Val His Gln Leu Arg Gln Glu Cys Tyr Ala Phe Asn Gly Thr Gln Arg
   1               5                   10                  15
   Phe Leu Glu Arg Tyr Ile Tyr Asn Arg Glu Glu Phe Val Arg Phe Asp
               20                  25                  30
   Ser Asp Val Gly Glu Phe Arg Ala Val Thr Glu Leu Gly Arg Pro Asp
           35                  40                  45
   Glu Glu Tyr Trp Asn Ser Gln Lys Asp Leu Leu Glu Glu Lys Arg Ala
       50                  55                  60
   Val Pro Asp Arg Val Cys Arg His Asn Tyr Glu Leu Asp Glu Ala Val
   65                  70                  75                  80
   Thr Leu Gln
<210> 51
   <211> 87
   <212> PRT
   <213> Homo sapiens
<400> 51
   Asn Tyr Leu Phe Gln Gly Arg Gln Glu Cys Tyr Ala Phe Asn Gly Thr
   1               5                   10                  15
   Gln Arg Phe Leu Glu Arg Tyr Ile Tyr Asn Arg Glu Glu Phe Val Arg
               20                  25                  30
   Phe Asp Ser Asp Val Gly Glu Phe Arg Ala Val Thr Glu Leu Gly Arg
           35                  40                  45
   Pro Asp Glu Asp Tyr Trp Asn Ser Gln Lys Asp Ile Leu Glu Glu Glu
       50                  55                  60
   Arg Ala Val Pro Asp Arg Met Cys Arg His Asn Tyr Glu Leu Gly Gly
   65                  70                  75                  80
   Pro Met Thr Leu Gln Arg Arg
                   85
<210> 52
   <211> 87
   <212> PRT
   <213> Homo sapiens
<400> 52
   Asn Tyr Leu Phe Gln Gly Arg Gln Glu Cys Tyr Ala Phe Asn Gly Thr
   1               5                   10                  15
   Gln Arg Phe Leu Glu Arg Tyr Ile Tyr Asn Arg Glu Glu Phe Val Arg
               20                  25                  30
   Phe Asp Ser Asp Val Gly Glu Phe Arg Ala Val Thr Glu Leu Gly Arg
           35                  40                  45
   Pro Glu Ala Glu Tyr Trp Asn Ser Gln Lys Asp Ile Leu Glu Glu Glu
       50                  55                  60
   Arg Ala Val Pro Asp Arg Met Cys Arg His Asn Tyr Glu Leu Gly Gly
   65                  70                  75                  80
   Pro Met Thr Leu Gln Arg Arg
                   85
<210> 53
   <211> 85
   <212> PRT
   <213> Homo sapiens
<400> 53
   Asn Tyr Leu Phe Gln Gly Arg Gln Glu Cys Tyr Ala Phe Asn Gly Thr
   1 5 10 15
   Gln Arg Phe Leu Glu Arg Tyr Ile Tyr Asn Arg Glu Glu Leu Val Arg
   20 25 30
   Phe Asp Ser Asp Val Gly Glu Phe Arg Ala Val Thr Glu Leu Gly Arg
   35 40 45
   Pro Asp Glu Glu Tyr Trp Asn Ser Gln Lys Asp Ile Leu Glu Glu Glu
   50 55 60
   Arg Ala Val Pro Asp Arg Met Cys Arg His Asn Tyr Glu Leu Gly Gly
   65 70 75 80
   Pro Met Thr Leu Gln
   85
<210> 54
   <211> 85
   <212> PRT
   <213> Homo sapiens
<400> 54
   Asn Tyr Leu Phe Gln Gly Arg Gln Glu Cys Tyr Ala Phe Asn Gly Thr
   1 5 10 15
   Gln Arg Phe Leu Glu Arg Tyr Ile Tyr Asn Arg Glu Glu Tyr Ala Arg
   20 25 30
   Phe Asp Ser Asp Val Gly Glu Phe Arg Ala Val Thr Glu Leu Gly Arg
   35 40 45
   Pro Asp Glu Glu Tyr Trp Asn Ser Gln Lys Asp Ile Leu Glu Glu Lys
   50 55 60
   Arg Ala Val Pro Asp Arg Met Cys Arg His Asn Tyr Glu Leu Gly Gly
   65 70 75 80
   Pro Met Thr Leu Gln
   85
<210> 55
   <211> 85
   <212> PRT
   <213> Homo sapiens
<400> 55
   Asn Tyr Val Tyr Gln Gly Arg Gln Glu Cys Tyr Ala Phe Asn Gly Thr
   1               5                   10                  15
   Gln Arg Phe Leu Glu Arg Tyr Ile Tyr Asn Arg Glu Glu Phe Val Arg
               20                  25                  30
   Phe Asp Ser Asp Val Gly Glu Phe Arg Ala Val Thr Glu Leu Gly Arg
           35                  40                  45
   Pro Asp Glu Asp Tyr Trp Asn Ser Gln Lys Asp Leu Leu Glu Glu Lys
       50                  55                  60
   Arg Ala Val Pro Asp Arg Val Cys Arg His Asn Tyr Glu Leu Asp Glu
   65                  70                  75                  80
   Ala Val Thr Leu Gln
                   85
<210> 56
   <211> 87
   <212> PRT
   <213> Homo sapiens
<400> 56
   Asn Tyr Leu Phe Gln Gly Arg Gln Glu Cys Tyr Ala Phe Asn Gly Thr
   1               5                   10                  15
   Gln Arg Phe Leu Glu Arg Tyr Ile Tyr Asn Arg Glu Glu Phe Ala Arg
               20                  25                  30
   Phe Asp Ser Asp Val Gly Glu Phe Arg Ala Val Thr Glu Leu Gly Arg
           35                  40                  45
   Pro Asp Glu Glu Tyr Trp Asn Ser Gln Lys Asp Ile Leu Glu Glu Lys
       50                  55                  60
   Arg Ala Val Pro Asp Arg Met Cys Arg His Asn Tyr Glu Leu Gly Gly
   65                  70                  75                  80
   Pro Met Thr Leu Gln Arg Arg
                   85
<210> 57
   <211> 85
   <212> PRT
   <213> Homo sapiens
<400> 57
   Asn Tyr Val Tyr Gln Leu Arg Gln Glu Cys Tyr Ala Phe Asn Gly Thr
   1               5                   10                  15
   Gln Arg Phe Leu Glu Arg Tyr Ile Tyr Asn Arg Glu Glu Phe Val Arg
               20                  25                  30
   Phe Asp Ser Asp Val Gly Glu Phe Arg Ala Val Thr Glu Leu Gly Arg
           35                  40                  45
   Pro Ala Ala Glu Tyr Trp Asn Ser Gln Lys Asp Leu Leu Glu Glu Lys
       50                  55                  60
   Arg Ala Val Pro Asp Arg Val Cys Arg His Asn Tyr Glu Leu Asp Glu
   65                  70                  75                  80
   Ala Val Thr Leu Gln
                   85
<210> 58
   <211> 85
   <212> PRT
   <213> Homo sapiens
<400> 58
   Asn Tyr Leu Phe Gln Gly Arg Gln Glu Cys Tyr Ala Phe Asn Gly Thr
   1               5                   10                  15
   Gln Arg Phe Leu Glu Arg Tyr Ile Tyr Asn Arg Glu Glu Tyr Ala Arg
               20                  25                  30
   Phe Asp Ser Asp Val Gly Glu Phe Arg Ala Val Thr Glu Leu Gly Arg
           35                  40                  45
   Pro Asp Glu Glu Tyr Trp Asn Ser Gln Lys Asp Ile Leu Glu Glu Lys
       50                  55                  60
   Arg Ala Val Pro Asp Arg Met Cys Arg His Asn Tyr Glu Leu Val Gly
   65                  70                  75                  80
   Pro Met Thr Leu Gln
                   85
<210> 59
   <211> 85
   <212> PRT
   <213> Homo sapiens
<400> 59
   Asn Tyr Val His Gln Leu Arg Gln Glu Cys Tyr Ala Phe Asn Gly Thr
   1               5                   10                  15
   Gln Arg Phe Leu Glu Arg Tyr Ile Tyr Asn Arg Glu Glu Phe Val Arg
               20                  25                  30
   Phe Asp Ser Asp Val Gly Glu Phe Arg Ala Val Thr Glu Leu Gly Arg
           35                  40                  45
   Pro Glu Ala Glu Tyr Trp Asn Ser Gln Lys Asp Ile Leu Glu Glu Glu
       50                  55                  60
   Arg Ala Val Pro Asp Arg Val Cys Arg His Asn Tyr Glu Leu Asp Glu
   65                  70                  75                  80
   Ala Val Thr Leu Gln
                   85
<210> 60
   <211> 85
   <212> PRT
   <213> Homo sapiens
<400> 60
   Asn Tyr Val His Gln Leu Arg Gln Glu Cys Tyr Ala Phe Asn Gly Thr
   1               5                   10                  15
   Gln Arg Phe Leu Glu Arg Tyr Ile Tyr Asn Arg Glu Glu Phe Val Arg
               20                  25                  30
   Phe Asp Ser Asp Val Gly Glu Phe Arg Ala Val Thr Glu Leu Gly Arg
           35                  40                  45
   Pro Ala Ala Glu Tyr Trp Asn Ser Gln Lys Asp Ile Leu Glu Glu Glu
       50                  55                  60
   Arg Ala Val Pro Asp Arg Met Cys Arg His Asn Tyr Glu Leu Asp Glu
   65                  70                  75                  80
   Ala Val Thr Leu Gln
                   85
<210> 61
   <211> 83
   <212> PRT
   <213> Homo sapiens
<400> 61
   Val Tyr Gln Leu Arg Gln Glu Cys Tyr Ala Phe Asn Gly Thr Gln Arg
   1               5                   10                  15
   Phe Leu Glu Arg Tyr Ile Tyr Asn Arg Glu Glu Phe Ala Arg Phe Asp
               20                  25                  30
   Ser Asp Val Gly Glu Phe Arg Ala Val Thr Glu Leu Gly Arg Pro Ala
           35                  40                  45
   Ala Glu Tyr Trp Asn Ser Gln Lys Asp Leu Leu Glu Glu Lys Arg Ala
       50                  55                  60
   Val Pro Asp Arg Val Cys Arg His Asn Tyr Glu Leu Asp Glu Ala Val
   65                  70                  75                  80
   Thr Leu Gln
<210> 62
   <211> 79
   <212> PRT
   <213> Homo sapiens
<400> 62
   Leu Phe Gln Gly Arg Gln Glu Cys Tyr Ala Phe Asn Gly Thr Gln Arg
   1               5                   10                  15
   Phe Leu Glu Arg Tyr Ile Tyr Asn Arg Glu Glu Phe Val Arg Phe Asp
               20                  25                  30
   Ser Asp Val Gly Glu Phe Arg Ala Val Thr Glu Leu Gly Arg Pro Asp
           35                  40                  45
   Glu Asp Tyr Trp Asn Ser Gln Lys Asp Leu Leu Glu Glu Lys Arg Ala
       50                  55                  60
   Val Pro Asp Arg Val Cys Arg His Asn Tyr Glu Leu Asp Glu Ala
   65                  70                  75
<210> 63
   <211> 85
   <212> PRT
   <213> Homo sapiens
<400> 63
   Asn Tyr Val His Gln Leu Arg Gln Glu Cys Tyr Ala Phe Asn Gly Thr
   1               5                   10                  15
   Gln Arg Phe Leu Glu Arg Tyr Ile Tyr Asn Arg Glu Glu Leu Val Arg
               20                  25                  30
   Phe Asp Ser Asp Val Gly Glu Phe Arg Ala Val Thr Glu Leu Gly Arg
           35                  40                  45
   Pro Ala Ala Glu Tyr Trp Asn Ser Gln Lys Asp Ile Leu Glu Glu Glu
       50                  55                  60
   Arg Ala Val Pro Asp Arg Met Cys Arg His Asn Tyr Glu Leu Asp Glu
   65                  70                  75                  80
   Ala Val Thr Leu Gln
                   85
<210> 64
   <211> 85
   <212> PRT
   <213> Homo sapiens
<400> 64
   Asn Tyr Leu Phe Gln Gly Arg Gln Glu Cys Tyr Ala Phe Asn Gly Thr
   1               5                   10                  15
   Gln Arg Phe Leu Glu Arg Tyr Ile Tyr Asn Arg Glu Glu Phe Val Arg
               20                  25                  30
   Phe Asp Ser Asp Val Gly Glu Phe Arg Ala Val Thr Glu Leu Gly Arg
           35                  40                  45
   Pro Asp Glu Glu Tyr Trp Asn Ser Gln Lys Asp Leu Leu Glu Glu Lys
       50                  55                  60
   Arg Ala Val Pro Asp Arg Met Cys Arg His Asn Tyr Glu Leu Gly Gly
   65                  70                  75                  80
   Pro Met Thr Leu Gln
                   85
<210> 65
   <211> 85
   <212> PRT
   <213> Homo sapiens

<400> 65
   Asn Tyr Leu Phe Gln Gly Arg Gln Glu Cys Tyr Ala Phe Asn Gly Thr
   1               5                   10                  15
   Gln Arg Phe Leu Glu Arg Tyr Ile Tyr Asn Arg Glu Glu Phe Val Arg
               20                  25                  30
   Phe Asp Ser Asp Val Gly Glu Phe Arg Ala Val Thr Glu Leu Gly Arg
           35                  40                  45
   Pro Asp Glu Glu Tyr Trp Asn Ser Gln Lys Asp Asn Leu Glu Glu Lys
       50                  55                  60
   Arg Ala Val Pro Asp Arg Met Cys Arg His Asn Tyr Glu Leu Gly Gly
   65                  70                  75                  80
   Pro Met Thr Leu Gln
                   85
<210> 66
   <211> 63
   <212> PRT
   <213> Homo sapiens
<220>
   <221> MISC_FEATURE
   <222> (63)..(63)
   <223> "Xaa" is a changeable amino acid.
<400> 66
   Asn Tyr Val Tyr Gln Leu Arg Gln Glu Cys Tyr Ala Phe Asn Gly Thr
   1               5                   10                  15
   Gln Arg Phe Leu Glu Arg Tyr Ile Tyr Asn Arg Glu Glu Phe Val Arg
               20                  25                  30
   Phe Asp Ser Asp Val Gly Glu Phe Arg Ala Val Thr Glu Leu Gly Arg
           35                  40                  45
   Pro Asp Glu Asp Tyr Trp Asn Ser Gln Lys Asp Leu Leu Glu Xaa
       50                  55                  60
<210> 67
   <211> 85
   <212> PRT
   <213> Homo sapiens
<400> 67
   Asn Tyr Leu Phe Gln Gly Arg Gln Glu Cys Tyr Ala Phe Asn Gly Thr
   1               5                   10                  15
   Gln Arg Phe Leu Glu Arg Tyr Ile Tyr Asn Arg Glu Glu Leu Val Arg
               20                  25                  30
   Phe Asp Ser Asp Val Gly Glu Phe Arg Ala Val Thr Glu Leu Gly Arg
           35                  40                  45
   Pro Ala Ala Glu Tyr Trp Asn Ser Gln Lys Asp Ile Leu Glu Glu Lys
       50                  55                  60
   Arg Ala Val Pro Asp Arg Met Cys Arg His Asn Tyr Glu Leu Val Gly
   65                  70                  75                  80
   Pro Met Thr Leu Gln
                   85
<210> 68
   <211> 85
   <212> PRT
   <213> Homo sapiens
<400> 68
   Asn Tyr Leu Phe Gln Gly Arg Gln Glu Cys Tyr Ala Phe Asn Gly Thr
   1               5                   10                  15
   Gln Arg Phe Leu Glu Arg Tyr Ile Tyr Asn Arg Glu Glu Leu Val Arg
               20                  25                  30
   Phe Asp Ser Asp Val Gly Glu Phe Arg Ala Val Thr Glu Leu Gly Arg
           35                  40                  45
   Pro Ala Ala Glu Tyr Trp Asn Ser Gln Lys Asp Ile Leu Glu Glu Lys
       50                  55                  60
   Arg Ala Val Pro Asp Arg Met Cys Arg His Asn Tyr Glu Leu Asp Glu
   65                  70                  75                  80
   Ala Val Thr Leu Gln
                   85
<210> 69
   <211> 2
   <212> PRT
   <213> Homo sapiens
<220>
   <221> MISC_FEATURE
   <222> (2)..(2)
   <223> "Xaa" is a changeable amino acid.
<400> 69
   Asn Xaa
   1
<210> 70
   <211> 87
   <212> PRT
   <213> Homo sapiens
<400> 70
   Asn Tyr Leu Phe Gln Gly Arg Gln Glu Cys Tyr Ala Phe Asn Gly Thr
   1               5                   10                  15
   Gln Arg Phe Leu Glu Arg Tyr Ile Tyr Asn Arg Glu Glu Tyr Ala Arg
               20                  25                  30
   Phe Asp Ser Asp Val Gly Glu Phe Arg Ala Val Thr Glu Leu Gly Arg
           35                  40                  45
   Pro Ala Ala Glu Tyr Trp Asn Ser Gln Lys Asp Ile Leu Glu Glu Lys
       50                  55                  60
   Arg Ala Val Pro Asp Arg Val Cys Arg His Asn Tyr Glu Leu Asp Glu
   65                  70                  75                  80
   Ala Val Thr Leu Gln Arg Arg
                   85
<210> 71
   <211> 87
   <212> PRT
   <213> Homo sapiens
<400> 71
   Asn Tyr Val His Gln Leu Arg Gln Glu Cys Tyr Ala Phe Asn Gly Thr
   1               5                   10                  15
   Gln Arg Phe Leu Glu Arg Tyr Ile Tyr Asn Arg Glu Glu Phe Ala Arg
               20                  25                  30
   Phe Asp Ser Asp Val Gly Glu Phe Arg Ala Val Thr Glu Leu Gly Arg
           35                  40                  45
   Pro Ala Ala Glu Tyr Trp Asn Ser Gln Lys Asp Ile Leu Glu Glu Lys
       50                  55                  60
   Arg Ala Val Pro Asp Arg Met Cys Arg His Asn Tyr Glu Leu Gly Gly
   65                  70                  75                  80
   Pro Met Thr Leu Gln Arg Arg
                   85
<210> 72
   <211> 87
   <212> PRT
   <213> Homo sapiens
<400> 72
   Asn Tyr Val His Gln Leu Arg Gln Glu Cys Tyr Ala Phe Asn Gly Thr
   1               5                   10                  15
   Gln Arg Phe Leu Glu Arg Tyr Ile Tyr Asn Arg Glu Glu Phe Val Arg
               20                  25                  30
   Phe Asp Ser Asp Val Gly Glu Phe Arg Ala Val Thr Glu Leu Gly Arg
           35                  40                  45
   Pro Ala Ala Glu Tyr Trp Asn Ser Gln Lys Asp Leu Leu Glu Glu Lys
       50                  55                  60
   Arg Ala Val Pro Asp Arg Val Cys Arg His Asn Tyr Glu Leu Asp Glu
   65                  70                  75                  80
   Ala Val Thr Leu Gln Arg Arg
                   85
<210> 73
   <211> 87
   <212> PRT
   <213> Homo sapiens
<400> 73
   Asn Tyr Leu Phe Gln Gly Arg Gln Glu Cys Tyr Ala Phe Asn Gly Thr
   1               5                   10                  15
   Gln Arg Phe Leu Glu Arg Tyr Ile Tyr Asn Arg Glu Glu Phe Val Arg
               20                  25                  30
   Phe Asp Ser Asp Val Gly Glu Phe Arg Ala Val Thr Glu Leu Gly Arg
           35                  40                  45
   Pro Asp Glu Glu Tyr Trp Asn Ser Gln Lys Asp Ile Leu Glu Glu Lys
       50                  55                  60
   Arg Ala Val Pro Asp Arg Val Cys Arg His Asn Tyr Glu Leu Asp Glu
   65                  70                  75                  80
   Ala Val Thr Leu Gln Arg Arg
                   85
<210> 74
   <211> 83
   <212> PRT
   <213> Homo sapiens
<400> 74
   Asn Tyr Val Tyr Gln Leu Arg Gln Glu Cys Tyr Ala Phe Asn Gly Thr
   1               5                   10                  15
   Gln Arg Phe Leu Glu Arg Tyr Ile Tyr Asn Arg Glu Glu Phe Val Arg
               20                  25                  30
   Phe Asp Ser Asp Val Gly Glu Phe Arg Ala Val Thr Glu Leu Gly Arg
           35                  40                  45
   Pro Asp Glu Asp Tyr Trp Asn Ser Gln Lys Asp Leu Leu Glu Glu Arg
       50                  55                  60
   Arg Ala Val Pro Asp Arg Met Cys Arg His Asn Tyr Glu Leu Asp Glu
   65                  70                  75                  80
   Ala Val Thr
<210> 75
   <211> 85
   <212> PRT
   <213> Homo sapiens
<400> 75
   Asn Tyr Val Asp Gln Leu Arg Gln Glu Cys Tyr Ala Phe Asn Gly Thr
   1               5                   10                  15
   Gln Arg Phe Leu Glu Arg Tyr Ile Tyr Asn Arg Glu Glu Phe Val Arg
               20                  25                  30
   Phe Asp Ser Asp Val Gly Glu Phe Arg Ala Val Thr Glu Leu Gly Arg
           35                  40                  45
   Pro Asp Glu Asp Tyr Trp Asn Ser Gln Lys Asp Leu Leu Glu Glu Lys
       50                  55                  60
   Arg Ala Val Pro Asp Arg Val Cys Arg His Asn Tyr Glu Leu Asp Glu
   65                  70                  75                  80
   Ala Val Thr Leu Gln
                   85
<210> 76
   <211> 85
   <212> PRT
   <213> Homo sapiens
<400> 76
   Asn Tyr Leu Phe Gln Gly Arg Gln Glu Cys Tyr Ala Phe Asn Gly Thr
   1               5                   10                  15
   Gln Arg Phe Leu Glu Arg Tyr Ile Tyr Asn Arg Glu Glu Phe Val Arg
               20                  25                  30
   Phe Asp Ser Asp Val Gly Glu Phe Arg Ala Val Thr Glu Leu Gly Arg
           35                  40                  45
   Pro Ala Ala Glu Tyr Trp Asn Ser Gln Lys Asp Ile Leu Glu Glu Glu
       50                  55                  60
   Arg Ala Val Pro Asp Arg Met Cys Arg His Asn Tyr Glu Leu Gly Gly
   65                  70                  75                  80
   Pro Met Thr Leu Gln
                   85
<210> 77
   <211> 85
   <212> PRT
   <213> Homo sapiens
<400> 77
   Asn Tyr Leu Phe Gln Gly Arg Gln Glu Cys Tyr Ala Phe Asn Gly Thr
   1               5                   10                  15
   Gln Arg Phe Leu Glu Arg Tyr Ile Tyr Asn Arg Glu Glu Phe Ala Arg
               20                  25                  30
   Phe Asp Ser Asp Val Gly Glu Phe Arg Ala Val Thr Glu Leu Gly Arg
           35                  40                  45
   Pro Ala Ala Glu Tyr Trp Asn Ser Gln Lys Asp Leu Leu Glu Glu Lys
       50                  55                  60
   Arg Ala Val Pro Asp Arg Met Cys Arg His Asn Tyr Glu Leu Gly Gly
   65                  70                  75                  80
   Pro Met Thr Leu Gln
                   85
<210> 78
   <211> 85
   <212> PRT
   <213> Homo sapiens
<400> 78
   Asn Tyr Leu Phe Gln Gly Arg Gln Glu Cys Tyr Ala Phe Asn Gly Thr
   1               5                   10                  15
   Gln Arg Phe Leu Glu Arg Tyr Ile Tyr Asn Arg Glu Glu Phe Val Arg
               20                  25                  30
   Phe Asp Ser Asp Val Gly Glu Phe Arg Ala Val Thr Glu Leu Gly Arg
           35                  40                  45
   Pro Asp Glu Glu Tyr Trp Asn Ser Gln Lys Asp Leu Leu Glu Glu Lys
       50                  55                  60
   Arg Ala Val Pro Asp Arg Val Cys Arg His Asn Tyr Glu Leu Gly Gly
   65                  70                  75                  80
   Pro Met Thr Leu Gln
   85
<210> 79
   <211> 83
   <212> PRT
   <213> Homo sapiens
<400> 79
   Val Tyr Gln Leu Arg Gln Glu Cys Tyr Ala Phe Asn Gly Thr Gln Arg
   1 5 10 15
   Phe Leu Glu Arg Tyr Ile Tyr Asn Arg Gln Glu Tyr Ala Arg Phe Asp
   20 25 30
   Ser Asp Val Gly Glu Phe Arg Ala Val Thr Glu Leu Gly Arg Pro Ala
   35 40 45
   Ala Glu Tyr Trp Asn Ser Gln Lys Asp Leu Leu Glu Glu Arg Arg Ala
   50 55 60
   Val Pro Asp Arg Met Cys Arg His Asn Tyr Glu Leu Val Gly Pro Met
   65 70 75 80
   Thr Leu Gln
<210> 80
   <211> 83
   <212> PRT
   <213> Homo sapiens
<400> 80
   Leu Phe Gln Gly Arg Gln Glu Cys Tyr Ala Phe Asn Gly Thr Gln Arg
   1 5 10 15
   Phe Leu Glu Arg Tyr Ile Tyr Asn Arg Glu Glu Phe Val Arg Phe Asp
   20 25 30
   Ser Asp Val Gly Glu Phe Arg Ala Val Thr Glu Leu Gly Arg Pro Asp
   35 40 45
   Glu Glu Tyr Trp Asn Ser Gln Lys Asp Ile Leu Glu Glu Lys Arg Ala
   50 55 60
   Val Pro Asp Arg Val Cys Arg His Asn Tyr Glu Leu Gly Gly Pro Met
   65 70 75 80
   Thr Leu Gln
<210> 81
   <211> 85
   <212> PRT
   <213> Homo sapiens
<400> 81
   Asn Tyr Val His Gln Leu Arg Gln Glu Cys Tyr Ala Phe Asn Gly Thr
   1 5 10 15
   Gln Arg Phe Leu Glu Arg Tyr Ile Tyr Asn Arg Glu Glu Phe Ala Arg
   20 25 30
   Phe Asp Ser Asp Val Gly Glu Phe Arg Ala Val Thr Glu Leu Gly Arg
   35 40 45
   Pro Asp Glu Asp Tyr Trp Asn Ser Gln Lys Asp Leu Leu Glu Glu Lys
   50 55 60
   Arg Ala Val Pro Asp Arg Val Cys Arg His Asn Tyr Glu Leu Asp Glu
   65 70 75 80
   Ala Val Thr Leu Gln
   85
<210> 82
   <211> 87
   <212> PRT
   <213> Homo sapiens
<400> 82
   Asn Tyr Leu Phe Gln Gly Leu Gln Glu Cys Tyr Ala Phe Asn Gly Thr
   1 5 10 15
   Gln Arg Phe Leu Glu Arg Tyr Ile Tyr Asn Arg Glu Glu Phe Val Arg
   20 25 30
   Phe Asp Ser Asp Val Gly Glu Phe Arg Ala Val Thr Glu Leu Gly Arg
   35 40 45
   Pro Asp Glu Glu Tyr Trp Asn Ser Gln Lys Asp Ile Leu Glu Glu Lys
   50 55 60
   Arg Ala Val Pro Asp Arg Met Cys Arg His Asn Tyr Glu Leu Gly Gly
   65 70 75 80
   Pro Met Thr Leu Gln Arg Arg
   85
<210> 83
   <211> 87
   <212> PRT
   <213> Homo sapiens
<400> 83
   Asn Tyr Val Tyr Gln Leu Arg Gln Glu Cys Tyr Ala Phe Asn Gly Thr
   1 5 10 15
   Gln Arg Phe Leu Glu Arg Tyr Ile Tyr Asn Arg Glu Glu Phe Val Arg
   20 25 30
   Phe Asp Ser Asp Val Gly Glu Phe Arg Ala Val Thr Glu Leu Gly Arg
   35 40 45
   Pro Asp Glu Asp Tyr Trp Asn Ser Gln Lys Asp Leu Leu Glu Glu Lys
   50 55 60
   Arg Ala Val Leu Asp Arg Val Cys Arg His Asn Tyr Glu Leu Asp Glu
   65 70 75 80
   Ala Val Thr Leu Gln Arg Arg
   85
<210> 84
   <211> 87
   <212> PRT
   <213> Homo sapiens
<400> 84
   Asn Tyr Val Tyr Gln Leu Arg Gln Glu Cys Tyr Ala Phe Asn Gly Thr
   1 5 10 15
   Gln Arg Phe Leu Glu Arg Tyr Ile Tyr Asn Arg Glu Glu Phe Val Arg
   20 25 30
   Phe Asp Ser Asp Val Gly Glu Phe Arg Ala Val Thr Glu Leu Gly Arg
   35 40 45
   Pro Asp Glu Glu Tyr Trp Asn Ser Gln Lys Asp Ile Leu Glu Glu Lys
   50 55 60
   Arg Ala Val Pro Asp Arg Val Cys Arg His Asn Tyr Glu Leu Asp Glu
   65 70 75 80
   Ala Val Thr Leu Gln Arg Arg
   85
<210> 85
   <211> 83
   <212> PRT
   <213> Homo sapiens
<400> 85
   Asn Tyr Leu Phe Gln Gly Arg Gln Glu Cys Tyr Ala Phe Asn Gly Thr
   1               5                   10                  15
   Gln Arg Phe Leu Glu Arg Tyr Ile Tyr Asn Arg Glu Glu Phe Val Arg
               20                  25                  30
   Phe Asp Ser Asp Val Gly Glu Phe Arg Ala Val Thr Glu Leu Gly Arg
           35                  40                  45
   Pro Asp Glu Asp Tyr Trp Asn Ser Gln Lys Asp Ile Leu Glu Glu Lys
       50                  55                  60
   Arg Ala Val Pro Asp Arg Met Cys Arg His Asn Tyr Glu Leu Gly Gly
   65                  70                  75                  80
   Pro Met Thr
<210> 86
   <211> 87
   <212> PRT
   <213> Homo sapiens
<400> 86
   Asn Tyr Leu Phe Gln Gly Arg Gln Glu Cys Tyr Ala Phe Asn Gly Thr
   1               5                   10                  15
   Gln Arg Phe Leu Glu Arg Tyr Ile Tyr Asn Arg Glu Glu Phe Ala Arg
               20                  25                  30
   Phe Asp Ser Asp Val Gly Glu Phe Arg Ala Val Thr Glu Leu Gly Arg
           35                  40                  45
   Pro Asp Glu Glu Tyr Trp Asn Ser Gln Lys Asp Ile Leu Glu Glu Glu
       50                  55                  60
   Arg Ala Val Pro Asp Arg Met Cys Arg His Asn Tyr Glu Leu Gly Gly
   65                  70                  75                  80
   Pro Met Thr Leu Gln Arg Arg
                   85
<210> 87
   <211> 87
   <212> PRT
   <213> Homo sapiens
<400> 87
   Asn Tyr Leu Phe Gln Gly Arg Gln Glu Cys Tyr Ala Phe Asn Gly Thr
   1               5                   10                  15
   Gln Arg Phe Leu Glu Arg Tyr Ile Tyr Asn Arg Glu Glu Phe Val Arg
               20                  25                  30
   Phe Asp Ser Asp Val Gly Glu Phe Arg Ala Val Thr Glu Leu Gly Arg
           35                  40                  45
   Pro Asp Glu Glu Tyr Trp Asn Ser Gln Lys Asp Ile Leu Glu Glu Lys
       50                  55                  60
   Arg Ala Val Pro Asp Arg Met Cys Arg His Asn Tyr Glu Leu Gly Gly
   65                  70                  75                  80
   Pro Met Thr Leu Gln His Arg
                   85

<210> 88
   <211> 87
   <212> PRT
   <213> Homo sapiens
<400> 88
   Asn Tyr Leu Phe Gln Gly Arg Gln Glu Cys Tyr Ala Phe Asn Gly Thr
   1               5                   10                  15
   Gln Arg Phe Leu Glu Arg Tyr Ile Tyr Asn Arg Glu Glu Phe Val Arg
               20                  25                  30
   Phe Asp Ser Asp Val Gly Glu Phe Arg Ala Val Thr Glu Leu Gly Arg
           35                  40                  45
   Pro Asp Glu Glu Tyr Trp Asn Ser Gln Lys Asp Phe Leu Glu Glu Lys
       50                  55                  60
   Arg Ala Val Pro Asp Arg Met Cys Arg His Asn Tyr Glu Leu Gly Gly
   65                  70                  75                  80
   Pro Met Thr Leu Gln Arg Arg
                   85
<210> 89
   <211> 87
   <212> PRT
   <213> Homo sapiens
<400> 89
   Asn Tyr Leu Phe Gln Gly Arg Gln Glu Cys Tyr Ala Phe Asn Gly Thr
   1               5                   10                  15
   Gln Arg Phe Leu Glu Arg Tyr Ile Tyr Asn Arg Glu Glu Phe Val Arg
               20                  25                  30
   Phe Asp Ser Asp Val Gly Glu Phe Arg Ala Val Thr Glu Leu Gly Arg
           35                  40                  45
   Pro Glu Glu Glu Tyr Trp Asn Ser Gln Lys Asp Ile Leu Glu Glu Lys
       50                  55                  60
   Arg Ala Val Pro Asp Arg Val Cys Arg His Asn Tyr Glu Leu Asp Glu
   65                  70                  75                  80
   Ala Val Thr Leu Gln Arg Arg
                   85
<210> 90
   <211> 87
   <212> PRT
   <213> Homo sapiens
<400> 90
   Asn Tyr Val Tyr Gln Leu Arg Gln Glu Cys Tyr Ala Phe Asn Gly Thr
   1               5                   10                  15
   Gln Arg Phe Leu Glu Arg Tyr Ile Tyr Asn Arg Glu Glu Tyr Ala Arg
               20                  25                  30
   Phe Asp Ser Asp Val Gly Glu Phe Arg Ala Val Thr Glu Leu Gly Arg
           35                  40                  45
   Pro Ala Ala Glu Tyr Trp Asn Ser Gln Lys Asp Ile Leu Glu Glu Lys
       50                  55                  60
   Arg Ala Val Pro Asp Arg Met Cys Arg His Asn Tyr Glu Leu Asp Glu
   65                  70                  75                  80
   Ala Val Thr Leu Gln His Arg
                   85
<210> 91
   <211> 87
   <212> PRT
   <213> Homo sapiens
<400> 91
   Asn Tyr Val His Gln Leu Arg Gln Glu Cys Tyr Ala Phe Asn Gly Thr
   1 5 10 15
   Gln Arg Phe Leu Glu Arg Tyr Ile Tyr Asn Arg Glu Glu Phe Val Arg
   20 25 30
   Phe Asp Ser Asp Val Gly Glu Phe Arg Ala Val Thr Glu Leu Gly Arg
   35 40 45
   Pro Asp Glu Asp Tyr Trp Asn Ser Gln Lys Asp Ile Leu Glu Glu Glu
   50 55 60
   Arg Ala Val Pro Asp Arg Met Cys Arg His Asn Tyr Glu Leu Gly Gly
   65 70 75 80
   Pro Met Thr Leu Gln Arg Arg
   85
<210> 92
   <211> 87
   <212> PRT
   <213> Homo sapiens
<400> 92
   Asn Tyr Val Tyr Gln Leu Arg Gln Glu Cys Tyr Ala Phe Asn Gly Thr
   1 5 10 15
   Gln Arg Phe Leu Glu Arg Tyr Ile Tyr Asn Arg Glu Glu Phe Val Arg
   20 25 30
   Phe Asp Ser Asp Val Gly Glu Phe Arg Ala Val Thr Glu Leu Gly Arg
   35 40 45
   Pro Ala Ala Glu Tyr Trp Asn Ser Gln Lys Asp Leu Leu Glu Glu Lys
   50 55 60
   Arg Ala Val Pro Asp Arg Met Cys Arg His Asn Tyr Glu Leu Asp Glu
   65 70 75 80
   Ala Val Thr Leu Gln Arg Arg
   85
<210> 93
   <211> 87
   <212> PRT
   <213> Homo sapiens
<400> 93
   Asn Tyr Val Tyr Gln Leu Arg Gln Glu Cys Tyr Ala Phe Asn Gly Thr
   1               5                   10                  15
   Gln Arg Phe Leu Glu Arg Tyr Ile Tyr Asn Arg Glu Glu Phe Val Arg
               20                  25                  30
   Phe Asp Ser Asp Val Gly Glu Phe Arg Ala Val Thr Glu Leu Gly Arg
           35                  40                  45
   Pro Asp Glu Asp Tyr Trp Asn Ser Gln Lys Asp Ile Leu Glu Glu Glu
       50                  55                  60
   Arg Ala Val Pro Asp Arg Val Cys Arg His Asn Tyr Glu Leu Asp Glu
   65                  70                  75                  80
   Ala Val Thr Leu Gln Arg Arg
                   85
<210> 94
   <211> 87
   <212> PRT
   <213> Homo sapiens
<400> 94
   Asn Tyr Val Tyr Gln Gly Arg Gln Glu Cys Tyr Ala Phe Asn Gly Thr
   1               5                   10                  15
   Gln Arg Phe Leu Glu Arg Tyr Ile Tyr Asn Arg Glu Glu Tyr Ala Arg
               20                  25                  30
   Phe Asp Ser Asp Val Gly Glu Phe Arg Ala Val Thr Glu Leu Gly Arg
           35                  40                  45
   Pro Ala Ala Glu Tyr Trp Asn Ser Gln Lys Asp Ile Leu Glu Glu Lys
       50                  55                  60
   Arg Ala Val Pro Asp Arg Met Cys Arg His Asn Tyr Glu Leu Asp Glu
   65                  70                  75                  80
   Ala Val Thr Leu Gln Arg Arg
                   85
<210> 95
   <211> 87
   <212> PRT
   <213> Homo sapiens
<400> 95
   Asn Tyr Val Tyr Gln Gly Arg Gln Glu Cys Tyr Ala Phe Asn Gly Thr
   1               5                   10                  15
   Gln Arg Phe Leu Glu Arg Tyr Ile Tyr Asn Arg Glu Glu Phe Ala Arg
               20                  25                  30
   Phe Asp Ser Asp Val Gly Glu Phe Arg Ala Val Thr Glu Leu Gly Arg
           35                  40                  45
   Pro Ala Ala Glu Tyr Trp Asn Ser Gln Lys Asp Ile Leu Glu Glu Lys
       50                  55                  60
   Arg Ala Val Pro Asp Arg Val Cys Arg His Asn Tyr Glu Leu Asp Glu
   65                  70                  75                  80
   Ala Val Thr Leu Gln Arg Arg
                   85
<210> 96
   <211> 87
   <212> PRT
   <213> Homo sapiens
<400> 96
   Asn Tyr Val His Gln Leu Arg Gln Glu Cys Tyr Ala Phe Asn Gly Thr
   1               5                   10                  15
   Gln Arg Phe Leu Glu Arg Tyr Ile Tyr Asn Arg Glu Glu Phe Val Arg
               20                  25                  30
   Phe Asp Ser Asp Val Gly Glu Phe Arg Ala Val Thr Glu Leu Gly Arg
           35                  40                  45
   Pro Asp Glu Asp Tyr Trp Asn Ser Gln Lys Asp Leu Leu Glu Glu Lys
       50                  55                  60
   Arg Ala Val Pro Asp Arg Met Cys Arg His Asn Tyr Glu Leu Asp Glu
   65                  70                  75                  80
   Ala Val Thr Leu Gln Arg Arg
                   85
<210> 97
   <211> 777
   <212> DNA
   <213> Homo sapiens
<400> 97
   atgatggttc tgcaggtttc tgcggccccc cggacagtgg ctctgacggc gttactgatg 60
   gtgctgctca catctgtggt ccagggcagg gccactccag agaattacgt gtaccaggga 120
   cggcaggaat gctacgcgtt taatgggaca cagcgcttcc tggagagata catctacaac 180
   cgggaggagt acgcgcgctt cgacagcgac gtgggggagt tccgggcggt gacggagctg 240
   gggcggcctg ctgcggagta ctggaacagc cagaaggaca tcctggagga gaagcgggca 300
   gtgccggaca gggtatgcag acacaactac gagctggacg aggccgtgac cctgcagcgc 360
   cgagtccagc ctaaggtgaa cgtttccccc tccaagaagg ggcccctgca gcaccacaac 420
   ctgcttgtct gccacgtgac agatttctac ccaggcagca ttcaagtccg atggttcctg 480
   aatggacagg aggaaacagc tggggtcgtg tccaccaacc tgatccgtaa tggagactgg 540
   accttccaga tcctggtgat gctggaaatg accccccagc agggagacgt ctacatctgc 600
   caagtggagc acaccagcct ggacagtcct gtcaccgtgg agtggaaggc acagtctgat 660
   tctgcccaga gtaagacatt gacgggagct gggggcttcg tgctggggct catcatctgt 720
   ggagtgggca tcttcatgca caggaggagc aagaaagttc aacgaggatc tgcataa 777
<210> 98
   <211> 261
   <212> DNA
   <213> Homo sapiens
<400> 98
   aattacgtgt accagggacg gcaggaatgc tacgcgttta atgggacaca gcgcttcctg 60
   gagagataca tctacaaccg ggaggagtac gcgcgcttcg acagcgacgt gggagagttc 120
   cgggcggtga cggagctggg gcggcctgct gcggagtact ggaacagcca gaaggacatc 180
   ctggaggaga agcgggcagt gccggacagg gtatgcagac acaactacga gctggacgag 240
   gccgtgaccc tgcagcgccg a 261
<210> 99
   <211> 777
   <212> DNA
   <213> Homo sapiens
<400> 99
   atgatggttc tgcaggtttc tgcggccccc cggacagtgg ctctgacggc gttactgatg 60
   gtgctgctca catctgtggt ccagggcagg gccactccag agaattacct tttccaggga 120
   cggcaggaat gctacgcgtt taatgggaca cagcgcttcc tggagagata catctacaac 180
   cgggaggagt tcgtgcgctt cgacagcgac gtgggggagt tccgggcggt gacggagctg 240
   gggcggcctg atgaggagta ctggaacagc cagaaggaca tcctggagga ggagcgggca 300
   gtgccggaca ggatgtgcag acacaactac gagctgggcg ggcccatgac cctgcagcgc 360
   cgagtccagc ctagggtgaa tgtttccccc tccaagaagg ggcccttgca gcaccacaac 420
   ctgcttgtct gccacgtgac ggatttctac ccaggcagca ttcaagtccg atggttcctg 480
   aatggacagg aggaaacagc tggggtcgtg tccaccaacc tgatccgtaa tggagactgg 540
   accttccaga tcctggtgat gctggaaatg accccccagc agggagatgt ctacacctgc 600
   caagtggagc acaccagcct ggatagtcct gtcaccgtgg agtggaaggc acagtctgat 660
   tctgcccgga gtaagacatt gacgggagct gggggcttcg tgctggggct catcatctgt 720
   ggagtgggca tcttcatgca caggaggagc aagaaagttc aacgaggatc tgcataa 777
<210> 100
   <211> 264
   <212> DNA
   <213> Homo sapiens
<400> 100
   agaattacct tttccaggga cggcaggaat gctacgcgtt taatgggaca cagcgcttcc 60
   tggagagata catctacaac cgggaggagt tcgtgcgctt cgacagcgac gtgggggagt 120
   tccgggcggt gacggagctg gggcggcctg acgaggagta ctggaacagc cagaaggaca 180
   tcctggagga ggagcgggca gtgccggaca ggatgtgcag acacaactac gagctgggcg 240
   ggcccatgac cctgcagcgc cgag 264
<210> 101
   <211> 263
   <212> DNA
   <213> Homo sapiens
<400> 101
   agaattacct tttccaggga cggcaggaat gctacgcgtt taatgggaca cagcgcttcc 60
   tggagagata catctacaac cgggaggagt tcgtgcgctt cgacagcgac gtgggggagt 120
   tccgggcggt gacggagctg gggcggcctg atgaggagta ctggaacagc cagaaggaca 180
   tcctggagga ggagcgggca gttccggaca ggatgtgcag acacaactac gagctgggcg 240
   ggcccatgac cctgcagcgc cga 263
<210> 102
   <211> 257
   <212> DNA
   <213> Homo sapiens
<400> 102
   agaattacct tttccaggga cggcaggaat gctacgcgtt taatgggaca cagcgcttcc 60
   tggagagata catctacaac cgggaggagc tcgtgcgctt cgacagcgac gtgggggagt 120
   tccgggcggt gacggagctg gggcggcctg aggcggagta ctggaacagc cagaaggaca 180
   tcctggagga ggagcgggca gtgccggaca ggatgtgcag acacaactac gagctgggcg 240
   ggcccatgac cctgcag 257
<210> 103
   <211> 777
   <212> DNA
   <213> Homo sapiens
<400> 103
   atgatggttc tgcaggtttc tgcggccccc cggacagtgg ctctgacggc gttactgatg 60
   gtgctgctca catctgtggt ccagggcagg gccactccag agaattacgt gtaccagtta 120
   cggcaggaat gctacgcgtt taatgggaca cagcgcttcc tggagagata catctacaac 180
   cgggaggagt tcgtgcgctt cgacagcgac gtgggggagt tccgggcggt gacggagctg 240
   gggcggcctg atgaggacta ctggaacagc cagaaggacc tcctggagga gaagcgggca 300
   gtgccggaca gggtatgcag acacaactac gagctggacg aggccgtgac cctgcagcgc 360
   cgagtccagc ctaaggtgaa cgtttccccc tccaagaagg ggcccctgca gcaccacaac 420
   ctgcttgtct gccacgtgac agatttctac ccaggcagca ttcaagtccg atggttcctg 480
   aatggacagg aggaaacagc tggggtcgtg tccaccaacc tgatccgtaa tggagactgg 540
   accttccaga tcctggtgat gctggaaatg accccccagc agggagacgt ctacatctgc 600
   caagtggagc acaccagcct ggacagtcct gtcaccgtgg agtggaaggc acagtctgat 660
   tctgcccgga gtaagacatt gacgggagct gggggcttcg tgctggggct catcatctgt 720
   ggagtgggca tcttcatgca caggaggagc aagaaagttc aacgaggatc tgcataa 777
<210> 104
   <211> 264
   <212> DNA
   <213> Homo sapiens
<400> 104
   agaattacgt gtaccagtta cggcaggaat gctacgcgtt taatgggaca cagcgcttcc 60
   tggagagata catctacaac cgggaggagt tcgtgcgctt cgacagcgac gtgggggagt 120
   tccgggcggt gacggagctg gggcggcctg atgaggacta ctggaacagc cagaaggacc 180
   tcctggagga gaagcgggca gtgccggaca gggtatgcag acacaactac gagctggacg 240
   aggccgtgac cctacagcgc cgag 264
<210> 105
   <211> 777
   <212> DNA
   <213> Homo sapiens
<400> 105
   atgatggttc tgcaggtttc tgcggccccc cggacagtgg ctctgacggc gttactgatg 60
   gtgctgctca catctgtggt ccagggcagg gccactccag agaattacct tttccaggga 120
   cggcaggaat gctacgcgtt taatgggaca cagcgcttcc tggagagata catctacaac 180
   cgggaggagt tcgcgcgctt cgacagcgac gtgggggagt tccgggcggt gacggagctg 240
   gggcggcctg ctgcggagta ctggaacagc cagaaggaca tcctggagga gaagcgggca 300
   gtgccggaca ggatgtgcag acacaactac gagctgggcg ggcccatgac cctgcagcgc 360
   cgagtccagc ctagggtgaa tgtttccccc tccaagaagg ggcccttgca gcaccacaac 420
   ctgcttgtct gccacgtgac ggatttctac ccaggcagca ttcaagtccg atggttcctg 480
   aatggacagg aggaaacagc tggggtcgtg tccaccaacc tgatccgtaa tggagactgg 540
   accttccaga tcctggtgat gctggaaatg accccccagc agggagatgt ctacacctgc 600
   caagtggagc acaccagcct ggatagtcct gtcaccgtgg agtggaaggc acagtctgat 660
   tctgcccgga gtaagacatt gacgggagct gggggcttcg tgctggggct catcatctgt 720
   ggagtgggca tcttcatgca caggaggagc aagaaagttc aacgaggatc tgcataa 777
<210> 106
   <211> 690
   <212> DNA
   <213> Homo sapiens
<400> 106
   agggccactc cagagaatta ccttttccag ggacggcagg aatgctacgc gtttaatggg 60
   acacagcgct tcctggagag atacatctac aaccgggagg agttcgtgcg cttcgacagc 120
   gacgtggggg agttccgggc ggtgacggag ctggggcggc ctgatgagga gtactggaac 180
   agccagaagg acatcctgga ggagaagcgg gcagtgccgg acaggatgtg cagacacaac 240
   tacgagctgg gcgggcccat gaccctgcag cgccgagtcc agcctagggt gaatgtttcc 300
   ccctccaaga aggggccctt gcagcaccac aacctgcttg tctgccacgt gacggatttc 360
   tacccaggca gcattcaagt ccgatggttc ctgaatggac aggaggaaac agctggggtc 420
   gtgtccacca acctgatccg taatggagac tggaccttcc agatcctggt gatgctggaa 480
   atgacccccc agcagggaga tgtctacacc tgccaagtgg agcacaccag catggatagt 540
   cctgtcaccg tggagtggaa ggcacagtct gattctgccc ggagtaagac attgacggga 600
   gctgggggct tcgtgctggg gctcatcatc tgtggagtgg gcatcttcat gcacaggagg 660
   agcaagaaag ttcaacgagg atctgcataa 690
<210> 107
   <211> 257
   <212> DNA
   <213> Homo sapiens
<400> 107
   agaattacct tttccaggga cggcaggaat gctacgcgtt taatgggaca cagcgcttcc 60
   tggagagata catctacaac cgggaggagc tcgtgcgctt cgacagcgac gtgggggagt 120
   tccgggcggt gacggagctg gggcggcctg aggcggagta ctggaacagc cagaaggaca 180
   tcctggagga gaagcgggca gtgccggaca ggatgtgcag acacaactac gagctggacg 240
   aggccgtgac cctgcag 257
<210> 108
   <211> 257
   <212> DNA
   <213> Homo sapiens
<400> 108
   agaattacgt gtaccagtta cggcaggaat gctacgcgtt taatgggaca cagcgcttcc 60
   tggagagata catctacaac cgggaggagt tcgtgcgctt cgacagcgac gtgggggagt 120
   tccgggcggt gacggagctg gggcggcctg atgaggacta ctggaacagc cagaaggacc 180
   tcctggagga ggagcgggca gtgccggaca ggatgtgcag acacaactac gagctggacg 240
   aggccgtgac cctgcag 257
<210> 109
   <211> 249
   <212> DNA
   <213> Homo sapiens
<400> 109
   cttttccagg gacggcagga atgctacgcg tttaatggga cacagcgctt cctggagaga     60
   tacatctaca accgggagga gttcgtgcgc ttcgacagcg acgtggggga gttccgggcg    120
   gtgacggagc tggggcggcc tgatgaggag tactggaaca gccagaagga catcctggag    180
   gaggagcggg cagtgccgga cagggtatgc agacacaact acgagctgga cgaggccgtg    240
   accctgcag                                                            249
<210> 110
   <211> 257
   <212> DNA
   <213> Homo sapiens
<400> 110
   agaattacgt gcaccagtta cggcaggaat gctacgcgtt taatgggaca cagcgcttcc     60
   tggagagata catctacaac cgggaggagt tcgtgcgctt cgacagcgac gtgggggagt    120
   tccgggcggt gacggagctg gggcggcctg atgaggacta ctggaacagc cagaaggaca    180
   tcctggagga ggagcgggca gtgccggaca gggtatgcag acacaactac gagctggacg    240
   aggccgtgac cctgcag                                                   257
<210> 111
   <211> 257
   <212> DNA
   <213> Homo sapiens
<400> 111
   agaattacgt gcaccagtta cggcaggaat gctacgcgtt taatgggaca cagcgcttcc     60
   tggagagata catctacaac cgggaggagt tcgtgcgctt cgacagcgac gtgggggagt    120
   tccgggcggt gacggagctg gggcggcctg atgaggagta ctggaacagc cagaaggaca    180
   tcctggagga ggagcgggca gtgccggaca gggtatgcag acacaactac gagctggacg    240
   aggccgtgac cctgcag                                                   257
<210> 112
   <211> 249
   <212> DNA
   <213> Homo sapiens
<400> 112
   gtgtaccagt tacggcagga atgctacgcg tttaatggga cacagcgctt cctggagaga 60
   tacatctaca accggcagga gtacgcgcgc ttcgacagcg acgtgggaga gttccgggcg 120
   gtgacggagc tggggcggcc tgctgcggag tactggaaca gccagaagga cctcctggag 180
   gagaggcggg cagtgccgga caggatgtgc agacacaact acgagctgga cgaggccgtg 240
   accctgcag 249
<210> 113
   <211> 257
   <212> DNA
   <213> Homo sapiens
<400> 113
   agaattacgt gtaccagtta cggcaggaat gctacgcgtt taatgggaca cagcgcttcc 60
   tggagagata catctacaac aggcaggagt acgcgcgctt cgacagcgac gtgggagagt 120
   tccgggcggt gacggagctg gggcggcctg ctgcggagta ctggaacagc cagaaggacc 180
   tcctggagga gaggcgggca gtgccggaca ggatgtgcag acacaactac gagctggacg 240
   aggccgtgac cctgcag 257
<210> 114
   <211> 257
   <212> DNA
   <213> Homo sapiens
<400> 114
   agaattacgt gtaccagtta cggcaggaat gctacgcgtt taatgggaca cagcgcttcc 60
   tggagagata catctacaac cgggaggagt acgcgcgctt cgacagcgac gtgggggagt 120
   tccgggcggt gacggagctg gggcggcctg ctgcggagta ctggaacagc cagaaggaca 180
   tcctggagga ggagcgggca gtgccggaca ggatatgcag acacaactac gagctggacg 240
   aggccgtgac cctgcag 257
<210> 115
   <211> 257
   <212> DNA
   <213> Homo sapiens
<400> 115
   agaattacgt gcaccagtta cggcaggaat gctacgcgtt taatgggaca cagcgcttcc 60
   tggagagata catctacaac cgggaggagt tcgtgcgctt cgacagcgac gtgggggagt 120
   tccgggcggt gacggagctg gggcggcctg atgaggacta ctggaacagc cagaaggacc 180
   tcctggagga gaagcgggca gtgccggaca gggtatgcag acacaactac gagctggacg 240
   aggccgtgac cctgcag 257
<210> 116
   <211> 257
   <212> DNA
   <213> Homo sapiens

<400> 116
   agaattacgt gtaccaggga cggcaggaat gctacgcgtt taatgggaca cagcgcttcc 60
   tggagagata catctacaac cggcaggagt acgcgcgctt cgacagcgac gtgggagagt 120
   tccgggcggt gacggagctg gggcggcctg ctgcggagta ctggaacagc cagaaggacc 180
   tcctggagga gaggcgggca gtgccggaca ggatgtgcag acacaactac gagctggtcg 240
   ggcccatgac cctgcag 257
<210> 117
   <211> 257
   <212> DNA
   <213> Homo sapiens
<400> 117
   agaattacct tttccaggga cggcaggaat gctacgcgtt taatgggaca cagcgcttcc 60
   tggagagata catctacaac cgggaggagt tcgtgcgctt cgacagcgac gtgggggagt 120
   tccgggcggt gacggagctg gggcggcctg atgaggagta ctggaacagc cagaaggaca 180
   tcctggagga ggagcgggca gtgccggaca ggatgtgcag acacaactac gagctggacg 240
   aggccgtgac cctgcag 257
<210> 118
   <211> 257
   <212> DNA
   <213> Homo sapiens
<400> 118
   agaattacgt gcaccagtta cggcaggaat gctacgcgtt taatgggaca cagcgcttcc 60
   tggagagata catctacaac cgggaggagt tcgtgcgctt cgacagcgac gtgggggagt 120
   tccgggcggt gacggagctg gggcggcctg atgaggacta ctggaacagc cagaaggaca 180
   tcctggagga ggagcgggca gtgccggaca ggatgtgcag acacaactac gagctggacg 240
   aggccgtgac cctgcag 257
<210> 119
   <211> 249
   <212> DNA
   <213> Homo sapiens
<400> 119
   gtgtaccagg gacggcagga atgctacgcg tttaatggga cacagcgctt cctggagaga 60
   tacatctaca accgggagga gttcgtgcgc ttcgacagcg acgtggggga gttccgggcg 120
   gtgacggagc tggggcggcc tgatgaggag tactggaaca gccagaagga catcctggag 180
   gagaagcggg cagtgccgga caggatgtgc agacacaact acgagctggt cgggcccatg 240
   accctgcag 249
<210> 120
   <211> 257
   <212> DNA
   <213> Homo sapiens
<400> 120
   agaattacct tttccaggga cggcaggaat gctacgcgtt taatgggaca cagcgcttcc 60
   tggagagata catctacaac cgggaggagt tcgtgcgctt cgacagcgac gtgggggagt 120
   tccgggcggt gacggagctg gggcggcctg aggcggagta ctggaacagc cagaaggaca 180
   tcctggagga ggagcgggca gtgccggaca ggatatgcag acacaactac gagctggacg 240
   aggccgtgac cctgcag 257
<210> 121
   <211> 264
   <212> DNA
   <213> Homo sapiens
<400> 121
   agaattacgt gtaccagtta cggcaggaat gctacgcgtt taatgggaca cagcgcttcc 60
   tggagagata catctacaac cgggaggagt tcgtgcgctt cgacagcgac gtgggggagt 120
   tccgggcggt gacggagctg gggcggcctg atgaggacta ctggaacagc cagaaggacc 180
   tcctggagga gaagcgggca gtgccggaca ggatgtgcag acacaactac gagctggacg 240
   aggccgtgac cctgcagcgc cgag 264
<210> 122
   <211> 263
   <212> DNA
   <213> Homo sapiens
<400> 122
   agaattacgt gtaccagtta cggcaggaat gctacgcgtt taatgggaca cagcgcttcc 60
   tggagagata catctacaac cgggaggagt tcgtgcgctt cgacagcgac gtgggggagt 120
   tccgggcggt gacggagctg gggcggcctg atgaggacta ctggaacagc cagaaggacc 180
   tcctggagga gaagcgggca gtgccggaca ggatgtgcag acacaactac gagctggacg 240
   aggccgtgac cctgcagcgt cga 263
<210> 123
   <211> 264
   <212> DNA
   <213> Homo sapiens
<400> 123
   agaattacgt gtaccagtta cggcaggaat gctacgcgtt taatgggaca cagcgcttcc 60
   tggagagata catctacaac cgggaggagc tcgtgcgctt cgacagcgac gtgggggagt 120
   tccgggcggt gacggagctg gggcggcctg aggcggagta ctggaacagc cagaaggaca 180
   tcctggagga ggagcgggca gtgccggaca ggatgtgcag acacaactac gagctggacg 240
   aggccgtgac cctgcagcgc cgag 264
<210> 124
   <211> 264
   <212> DNA
   <213> Homo sapiens
<400> 124
   agaattacct tttccaggga cggcaggaat gctacgcgtt taatgggaca cagcgcttcc 60
   tggagagata catctacaac cgggaggagc tcgtgcgctt cgacagcgac gtgggggagt 120
   tccgggcggt gacggagctg gggcggcctg aggcggagta ctggaacagc cagaaggaca 180
   tcctggagga ggagcgggca gtgccggaca ggatgtgcag acacaactac gagctggacg 240
   aggccgtgac cctgcagcgc cgag 264
<210> 125
   <211> 264
   <212> DNA
   <213> Homo sapiens
<400> 125
   agaattacct tttccaggga cggcaggaat gctacgcgtt taatgggaca cagcgcttcc 60
   tggagagata catctacaac cgggaggagt tcgtgcgctt cgacagcgac gtgggggagt 120
   tccgggcggt gacggagctg gggcggcctg ctgcggagta ctggaacagc cagaaggaca 180
   tcctggagga gaagcgggca gtgccggaca ggatgtgcag acacaactac gagctgggcg 240
   ggcccatgac cctgcagcgc cgag 264
<210> 126
   <211> 264
   <212> DNA
   <213> Homo sapiens
<400> 126
   agaattacct tttccaggga cggcaggaat gctacgcgtt taatgggaca cagcgcttcc 60
   tggagagata catctacaac cgggaggagt tcgcgcgctt cgacagcgac gtgggggagt 120
   tccgggcggt gacggagctg gggcggcctg aggcggagta ctggaacagc cagaaggaca 180
   tcctggagga gaagcgggca gtgccggaca ggatgtgcag acacaactac gagctgggcg 240
   ggcccatgac cctgcagcgc cgag 264
<210> 127
   <211> 264
   <212> DNA
   <213> Homo sapiens
<400> 127
   agaattacgt gtaccagtta cggcaggaat gctacgcgtt taatgggaca cagcgcttcc 60
   tggagagata catctacaac cgggaggagt tcgtgcgctt cgacagcgac gtgggggagt 120
   tccgggcggt gacggagctg gggcggcctg atgaggagta ctggaacagc cagaaggacc 180
   tcctggagga gaagcgggca gtgccggaca gggtatgcag acacaactac gagctggacg 240
   aggccgtgac cctgcagcgc cgag 264
<210> 128
   <211> 256
   <212> DNA
   <213> Homo sapiens
<400> 128
   gtgtaccagt tacggcagga atgctacgcg tttaatggga cacagcgctt cctggagaga 60
   tacatctaca accgggagga gtacgcgcgc ttcgacagcg acgtgggaga gttccgggcg 120
   gtgacggagc tggggcggcc tgctgcggag tactggaaca gccagaagga catcctggag 180
   gagaagcggg cagtgccgga cagagtatgc agacacaact acgagctgga cgaggccgtg 240
   accctgcagc gccgag 256
<210> 129
   <211> 255
   <212> DNA
   <213> Homo sapiens
<400> 129
   gtgtaccagt tacggcagga atgctacgcg tttaatggga cacagcgctt cctggagaga      60
   tacatctaca accgggagga gtacgcgcgc ttcgacagcg acgtggggga gttccgggcg     120
   gtgacggagc tggggcggcc tgctgcggag tactggaaca gccagaagga catcctggag 180
   gagaagcggg cagtgccgga cagggtatgc agacacaact acgagctgga cgaggccgtg 240
   accctgcagc gccga 255
<210> 130
   <211> 263
   <212> DNA
   <213> Homo sapiens
<400> 130
   agaattacgt gtaccagtta cggcaggaat gctacgcgtt taatgggaca cagcgcttcc 60
   tggagagata catctacaac cgggaggagt acgcgcgctt cgacagcgac gtgggggagt 120
   tccgggcggt gacggagctg gggcggcctg ctgcggagta ctggaacagc cagaaggaca 180
   tcctggagga gaagcgggca gtgccggaca ggatgtgcag acacaactac gagctggacg 240
   aggccgtgac cctgcagcgc cga 263
<210> 131
   <211> 264
   <212> DNA
   <213> Homo sapiens
<400> 131
   agaattacct tttccaggga cggcaggaat gctacgcgtt taatgggaca cagcgcttcc 60
   tggagagata catctacaac cgggaggagt tcgcgcgctt cgacagcgac gtgggggagt 120
   tccgggcggt gacggagctg gggcggcctg atgaggagta ctggaacagc cagaaggacc 180
   tcctggagga gaagcgggca gtgccggaca ggatgtgcag acacaactac gagctggtcg 240
   ggcccatgac cctgcagcgc cgag 264
<210> 132
   <211> 264
   <212> DNA
   <213> Homo sapiens
<400> 132
   agaattacgt gtaccagtta cggcaggaat gctacgcgtt taatgggaca cagcgcttcc 60
   tggagagata catctacaac cgggaggagt tcgtgcgctt cgacagcgac gtgggggagt 120
   tccgggcggt gacggagctg gggcggcctg atgaggacta ctggaacagc cagaaggacc 180
   tcctggagga ggagcgggca gtgccggaca gggtatgcag acacaactac gagctggacg 240
   aggccgtgac cctgcagcgc cgag 264
<210> 133
   <211> 257
   <212> DNA
   <213> Homo sapiens
<400> 133
   agaattacgt gcaccagtta cggcaggaat gctacgcgtt taatgggaca cagcgcttcc 60
   tggagagata catctacaac cgggaggagt tcgtgcgctt cgacagcgac gtgggggagt 120
   tccgggcggt gacggagctg gggcggcctg aggcggagta ctggaacagc cagaaggaca 180
   tcctggagga ggagcgggca gtgccggaca ggatgtgcag acacaactac gagctggacg 240
   aggccgtgac cctgcag 257
<210> 134
   <211> 264
   <212> DNA
   <213> Homo sapiens
<400> 134
   agaattacct tttccaggga cggcaggaat gctacgcgtt taatgggaca cagcgcttcc 60
   tggagagata catctacaac cgggaggagt tcgcgcgctt cgacagcgac gtgggggagt 120
   tccgggcggt gacggagctg gggcggcctg ctgcggagta ctggaacagc cagaaggacc 180
   tcctggagga gaagcgggca ttgccggaca ggatgtgcag acacaactac gagctggacg 240
   aggccgtgac cctgcagcgc cgag 264
<210> 135
   <211> 264
   <212> DNA
   <213> Homo sapiens
<400> 135
   agaattacct tttccaggga cggcaggaat gctacgcgtt taatgggaca cagcgcttcc 60
   tggagagata catctacaac cgggaggagt tcgtgcgctt cgacagcgac gtgggggagt 120
   tccgggcggt gacggagctg gggcggcctg atgaggtgta ctggaacagc cagaaggaca 180
   tcctggagga ggagcgggca gtgccggaca ggatgtgcag acacaactac gagctgggcg 240
   ggcccatgac cctgcagcgc cgag 264
<210> 136
   <211> 257
   <212> DNA
   <213> Homo sapiens
<400> 136
   agaattacct tttccaggga cggcaggaat gctacgcgtt taatgggaca cagcgcttcc 60
   tggagagata catctacaac cgggaggagt tcgcgcgctt cgacagcgac gtgggggagt 120
   tccgggcggt gacggagctg gggcggcctg ctgcggagta ctggaacagc cagaaggaca 180
   tcctggagga ggagcgggca gtgccggaca ggatgtgcag acacaactac gagctgggcg 240
   ggcccatgac cctgcag 257
<210> 137
   <211> 257
   <212> DNA
   <213> Homo sapiens
<400> 137
   agaattacct tttccaggga cggcaggaat gctacgcgtt taatgggaca cagcgcttcc 60
   tggagagata catctacaac cgggaggagc tcgtgcgctt cgacagcgac gtgggggagt 120
   tccgggcggt gacggagctg gggcggcctg ctgcggagta ctggaacagc cagaaggacc 180
   tcctggagga gaagcgggca ttgccggaca ggatgtgcag acacaactac gagctggtcg 240
   ggcccatgac cctgcag 257
<210> 138
   <211> 257
   <212> DNA
   <213> Homo sapiens
<400> 138
   agaattacgt gcaccagtta cggcaggaat gctacgcgtt taatgggaca cagcgcttcc 60
   tggagagata catctacaac cgggaggagt tcgtgcgctt cgacagcgac gtgggggagt 120
   tccgggcggt gacggagctg gggcggcctg atgaggacta ctggaacagc cagaaggaca 180
   tcctggagga gaagcgggca gtgccggaca gggtatgcag acacaactac gagctggacg 240
   aggccgtgac cctgcag 257
<210> 139
   <211> 264
   <212> DNA
   <213> Homo sapiens
<400> 139
   agaattacgt gtaccagtta cggcaggaat gctacgcgtt taatgggaca cagcgcttcc 60
   tggagagata catctacaac cgggaggagc tcgtgcgctt cgacagcgac gtgggggagt 120
   tccgggcggt gacggagctg gggcggcctg aggcggagta ctggaacagc cagaaggaca 180
   tcctggagga gaagcgggca gtgccggaca ggatgtgcag acacaactac gagctggacg 240
   aggccgtgac cctgcagcgc cgag 264
<210> 140
   <211> 256
   <212> DNA
   <213> Homo sapiens
<400> 140
   gtgtaccagt tacggcagga atgctacgcg tttaatggga cacagcgctt cctggagaga      60
   tacatctaca accgggagga gttcgtgcgc ttcgacagcg acgtggggga gttccgggcg     120
   gtgacggagc tggggcggcc tgatgaggag tactggaaca gccagaagga catcctggag     180
   gaggagcggg cagtgccgga cagggtatgc agacacaact acgagctgga cgaggccgtg     240
   accctgcagc gccgag                                                     256
<210> 141
   <211> 249
   <212> DNA
   <213> Homo sapiens
<400> 141
   cttttccagg gacggcagga atgctacccg tttaatggga cacagcgctt cctggagaga      60
   tacatctaca accgggagga gctcgtgcgc ttcgacagcg acgtggggga gttccgggcg     120
   gtgacggagc tggggcggcc tgaggcggag tactggaaca gccagaagga catcctggag     180
   gagaagcggg cagtgccgga caggatgtgc agacacaact acgagctgga cgaggccgtg     240
   accctgcag                                                             249
<210> 142
   <211> 263
   <212> DNA
   <213> Homo sapiens
<400> 142
   agaattacct tttccaggga cggcaggaat gctacgcgtt taatgggaca cagcgcttcc      60
   tggagagata catctacaac cgggaggagt acgcgcgctt cgacagcgac gtgggggagt     120
   tccgggcggt gacggagctg ggcgcgcctg ctgcggagta ctggaacagc cagaaggaca     180
   tcctggagga gaagcgggca gtgccggaca ggatgtgcag acacaactac gagctgggcg     240
   ggcccatgac cctgcagcgc cga                                             263
<210> 143
   <211> 263
   <212> DNA
   <213> Homo sapiens
<400> 143
   agaattacct tttccaggga cggcaggaat gctacgcgtt taatgggaca cagcgcttcc 60
   tggagagata catctacaac cgggaggagt acgcgcgctt cgacagcgac gtgggggagt 120
   tccgggcggt gacggagctg gggcggcctg ctgcggagta ctggaacagc cagaaggaca 180
   tcctggagga gaagcgggca gtgccggaca ggatgtgcag acacaactac gagctggtcg 240
   ggcccatgac cctgcagcgc cga 263
<210> 144
   <211> 261
   <212> DNA
   <213> Homo sapiens
<400> 144
   aattaccttt tccagggacg gcaggaatgc tacgcgttta atgggacaca gcgcttcctg 60
   gagagataca tctacaaccg ggaggagttc gtgcgcttcg acagcgacgt gggggagttc 120
   cgggcggtga cggagctggg gcggcctgat gaggagtact ggaacagcca gaaggacttc 180
   ctggaggagg agcgggcagt gccggacagg atgtgcagac acaactacga gctgggcggg 240
   cccatgaccc tgcagcgccg a 261
<210> 145
   <211> 264
   <212> DNA
   <213> Homo sapiens
<400> 145
   agaattacgt gtaccagtta cggcaggaat gctacgcgtt taatgggaca cagcgcttcc 60
   tggagagata catctacaac cgggaggagc tcgtgcgctt cgacagcgac gtgggggagt 120
   tccgggcggt gacggagctg gggcggcctg atgaggacta ctggaacagc cagaaggacc 180
   tcctggagga ggagcgggca gtgccggaca gggtatgcag acacaactac gagctggacg 240
   aggccgtgac cctgcagcgc cgag 264
<210> 146
   <211> 249
   <212> DNA
   <213> Homo sapiens
<400> 146
   gtgcaccagt tacggcagga atgctacgcg tttaatggga cacagcgctt cctggagaga 60
   tacatctaca accgggagga gttcgtgcgc ttcgacagcg acgtggggga gttccgggcg 120
   gtgacggagc tggggcggcc tgatgaggag tactggaaca gccagaagga cctcctggag 180
   gagaagcggg cagtgccgga cagggtatgc agacacaact acgagctgga cgaggccgtg 240
   accctgcag 249
<210> 147
   <211> 264
   <212> DNA
   <213> Homo sapiens
<400> 147
   agaattacct tttccaggga cggcaggaat gctacgcgtt taatgggaca cagcgcttcc 60
   tggagagata catctacaac cgggaggagt tcgtgcgctt cgacagcgac gtgggggagt 120
   tccgggcggt gacggagctg gggcggcctg atgaggacta ctggaacagc cagaaggaca 180
   tcctggagga ggagcgggca gtgccggaca ggatgtgcag acacaactac gagctgggcg 240
   ggcccatgac cctgcagcgc cgag 264
<210> 148
   <211> 264
   <212> DNA
   <213> Homo sapiens
<400> 148
   agaattacct tttccaggga cggcaggaat gctacgcgtt taatgggaca cagcgcttcc 60
   tggagagata catctacaac cgggaggagt tcgtgcgctt cgacagcgac gtgggggagt 120
   tccgggcggt gacggagctg gggcggcctg aggcggagta ctggaacagc cagaaggaca 180
   tcctggagga ggagcgggca gtgccggaca ggatgtgcag acacaactac gagctgggcg 240
   ggcccatgac cctgcagcgc cgag 264
<210> 149
   <211> 255
   <212> DNA
   <213> Homo sapiens
<400> 149
   aattaccttt tccagggacg gcaggaatgc tacgcgttta atgggacaca gcgcttcctg 60
   gagagataca tctacaaccg ggaggagctc gtgcgcttcg acagcgacgt gggggagttc 120
   cgggcggtga cggagctggg gcggcctgat gaggagtact ggaacagcca gaaggacatc 180
   ctggaggagg agcgggcagt gccggacagg atgtgcagac acaactacga gctgggcggg 240
   cccatgaccc tgcag 255
<210> 150
   <211> 255
   <212> DNA
   <213> Homo sapiens
<400> 150
   aattaccttt tccagggacg gcaggaatgc tacgcgttta atgggacaca gcgcttcctg 60
   gagagataca tctacaaccg ggaggagtac gcgcgcttcg acagcgacgt gggggagttc 120
   cgggcggtga cggagctggg gcggcctgat gaggagtact ggaacagcca gaaggacatc 180
   ctggaggaga agcgggcagt gccggacagg atgtgcagac acaactacga gctgggcggg 240
   cccatgaccc tgcag 255
<210> 151
   <211> 255
   <212> DNA
   <213> Homo sapiens
<400> 151
   aattacgtgt accagggacg gcaggaatgc tacgcgttta atgggacaca gcgcttcctg 60
   gagagataca tctacaaccg ggaggagttc gtgcgcttcg acagcgacgt gggggagttc 120
   cgggcggtga cggagctggg gcggcctgat gaggactact ggaacagcca gaaggacctc 180
   ctggaggaga agcgggcagt gccggacagg gtatgcagac acaactacga gctggacgag 240
   gccgtgaccc tgcag 255
<210> 152
   <211> 264
   <212> DNA
   <213> Homo sapiens
<400> 152
   agaattacct tttccaggga cggcaggaat gctacgcgtt taatgggaca cagcgcttcc 60
   tggagagata catctacaac cgggaggagt tcgcgcgctt cgacagcgac gtgggggagt 120
   tccgggcggt gacggagctg gggcggcctg atgaggagta ctggaacagc cagaaggaca 180
   tcctggagga gaagcgggca gtgccggaca ggatgtgcag acacaactac gagctgggcg 240
   ggcccatgac cctgcagcgc cgag 264
<210> 153
   <211> 257
   <212> DNA
   <213> Homo sapiens
<400> 153
   agaattacgt gtaccagtta cggcaggaat gctacgcgtt taatgggaca cagcgcttcc 60
   tggagagata catctacaac cgggaggagt tcgtgcgctt cgacagcgac gtgggggagt 120
   tccgggcggt gacggagctg gggcggcctg ctgcggagta ctggaacagc cagaaggacc 180
   tcctggagga gaagcgggca gtgccggaca gggtatgcag acacaactac gagctggacg 240
   aggccgtgac cctgcag 257
<210> 154
   <211> 257
   <212> DNA
   <213> Homo sapiens
<400> 154
   agaattacct tttccaggga cggcaggaat gctacgcgtt taatgggaca cagcgcttcc 60
   tggagagata catctacaac cgggaggagt acgcgcgctt cgacagcgac gtgggggagt 120
   tccgggcggt gacggagctg gggcggcctg atgaggagta ctggaacagc cagaaggaca 180
   tcctggagga gaagcgggca gtgccggaca ggatgtgcag acacaactac gagctggtcg 240
   ggcccatgac cctgcag 257
<210> 155
   <211> 257
   <212> DNA
   <213> Homo sapiens
<400> 155
   agaattacgt gcaccagtta cggcaggaat gctacgcgtt taatgggaca cagcgcttcc 60
   tggagagata catctacaac cgggaggagt tcgtgcgctt cgacagcgac gtgggggagt 120
   tccgggcggt gacggagctg gggcggcctg aggcggagta ctggaacagc cagaaggaca 180
   tcctggagga ggagcgggca gtgccggaca gggtatgcag acacaactac gagctggacg 240
   aggccgtgac cctgcag 257
<210> 156
   <211> 257
   <212> DNA
   <213> Homo sapiens
<400> 156
   agaattacgt gcaccagtta cggcaggaat gctacgcgtt taatgggaca cagcgcttcc 60
   tggagagata catctacaac cgggaggagt tcgtgcgctt cgacagcgac gtgggggagt 120
   tccgggcggt gacggagctg gggcggcctg ctgcggagta ctggaacagc cagaaggaca 180
   tcctggagga ggagcgggca gtgccggaca ggatgtgcag acacaactac gagctggacg 240
   aggccgtgac cctgcag 257

<210> 157
   <211> 257
   <212> DNA
   <213> Homo sapiens
<400> 157
   agaattacgt gcaccagtta cggcaggaat gctacgcgtt taatgggaca cagcgcttcc 60
   tggagagata catctacaac cgggaggagt tcgtgcgctt cgacagcgac gtgggggagt 120
   tccgggcggt gacggagctg gggcggcctg ctgcggagta ctggaacagc cagaaggaca 180
   tcctggagga ggagcgggca gtgccggaca ggatgtgcag acacaactac gagctggacg 240
   aggccgtgac cctgcag 257
<210> 158
   <211> 249
   <212> DNA
   <213> Homo sapiens
<400> 158
   gtgtaccagt tacggcagga atgctacgcg tttaatggga cacagcgctt cctggagaga 60
   tacatctaca accgggagga gttcgcgcgc ttcgacagcg acgtggggga gttccgggcg 120
   gtgacggagc tggggcggcc tgctgcggag tactggaaca gccagaagga cctcctggag 180
   gagaagcggg cagtgccgga cagggtatgc agacacaact acgagctgga cgaggccgtg 240
   accctgcag 249
<210> 159
   <211> 238
   <212> DNA
   <213> Homo sapiens
<400> 159
   cttttccagg gacggcagga atgctacgcg tttaatggga cacagcgctt cctggagaga 60
   tacatctaca accgggagga gttcgtgcgc ttcgacagcg acgtggggga gttccgggcg 120
   gtgacggagc tggggcggcc tgatgaggac tactggaaca gccagaagga cctcctggag 180
   gagaagcggg cagtgccgga cagggtatgc agacacaact acgagctgga cgaggccg 238
<210> 160
   <211> 255
   <212> DNA
   <213> Homo sapiens
<400> 160
   aattacgtgc accagttacg gcaggaatgc tacgcgttta atgggacaca gcgcttcctg 60
   gagagataca tctacaaccg ggaggagctc gtgcgcttcg acagcgacgt gggggagttc 120
   cgggcggtga cggagctggg gcggcctgct gcggagtact ggaacagcca gaaggacatc 180
   ctggaggagg agcgggcagt gccggacagg atgtgcagac acaactacga gctggacgag 240
   gccgtgaccc tgcag 255
<210> 161
   <211> 257
   <212> DNA
   <213> Homo sapiens
<400> 161
   agaattacct tttccaggga cggcaggaat gctacgcgtt taatgggaca cagcgcttcc 60
   tggagagata catctacaac cgggaggagt tcgtgcgctt cgacagcgac gtgggggagt 120
   tccgggcggt gacggagctg gggcggcctg atgaggagta ctggaacagc cagaaggacc 180
   tcctggagga gaagcgggca gtgccggaca ggatgtgcag acacaactac gagctgggcg 240
   ggcccatgac cctgcag 257
<210> 162
   <211> 257
   <212> DNA
   <213> Homo sapiens
<400> 162
   agaattacct tttccaggga cggcaggaat gctacgcgtt taatgggaca cagcgcttcc 60
   tggagagata catctacaac cgggaggagt tcgtgcgctt cgacagcgac gtgggggagt 120
   tccgggcggt gacggagctg gggcggcctg atgaggagta ctggaacagc cagaaggaca 180
   acctggagga gaagcgggca gtgccggaca ggatgtgcag acacaactac gagctgggcg 240
   ggcccatgac cctgcag 257
<210> 163
   <211> 260
   <212> DNA
   <213> Homo sapiens
<400> 163
   agaattacgt gtaccagtta cggcaggaat gctacgcgtt taatgggaca cagcgcttcc 60
   tggagagata catctacaac cgggaggagt tcgtgcgctt cgacagcgac gtgggggagt 120
   tccgggcggt gacggagctg gggcggcctg atgaggacta ctggaacagc cagaaggacc 180
   tcctgtagga gaagcgggca gtgccggaca gggtatgcag acacaactac gagctggacg 240
   aggccgtgac cctgcagcgc 260
<210> 164
   <211> 257
   <212> DNA
   <213> Homo sapiens
<400> 164
   agaattacct tttccaggga cggcaggaat gctacgcgtt taatgggaca cagcgcttcc 60
   tggagagata catctacaac cgggaggagc tcgtgcgctt cgacagcgac gtgggggagt 120
   tccgggcggt gacggagctg gggcggcctg ctgcggagta ctggaacagc cagaaggaca 180
   tcctggagga gaagcgggca gtgccggaca ggatgtgcag acacaactac gagctggtcg 240
   ggcccatgac cctgcag 257
<210> 165
   <211> 255
   <212> DNA
   <213> Homo sapiens
<400> 165
   aattaccttt tccagggacg gcaggaatgc tacgcgttta atgggacaca gcgcttcctg 60
   gagagataca tctacaaccg ggaggagctc gtgcgcttcg acagcgacgt gggggagttc 120
   cgggcggtga cggagctggg gcggcctgct gcggagtact ggaacagcca gaaggacatc 180
   ctggaggaga agcgggcagt gccggacagg atgtgcagac acaactacga gctggacgag 240
   gccgtgaccc tgcag 255
<210> 166
   <211> 255
   <212> DNA
   <213> Homo sapiens
<400> 166
   aattaagtgt accagttacg gcaggaatgc tacgcgttta atgggacaca gcgcttcctg 60
   gagagataca tctacaaccg ggaggagttc gtgcgcttcg acagcgacgt gggggagttc 120
   cgggcggtga cggagctggg gcggcctgat gaggactact ggaacagcca gaaggacctc 180
   ctggaggagg agcgggcagt gccggacagg atgtgcagac acaactacga gctggacgag 240
   gccgtgaccc tgcag 255
<210> 167
   <211> 264
   <212> DNA
   <213> Homo sapiens
<400> 167
   agaattacct tttccaggga cggcaggaat gctacgcgtt taatgggaca cagcgcttcc 60
   tggagagata catctacaac cgggaggagt acgcgcgctt cgacagcgac gtgggggagt 120
   tccgggcggt gacggagctg gggcggcctg ctgcggagta ctggaacagc cagaaggaca 180
   tcctggagga gaagcgggca gtgccggaca gggtatgcag acacaactac gagctggacg 240
   aggccgtgac cctgcagcgc cgag 264
<210> 168
   <211> 264
   <212> DNA
   <213> Homo sapiens
<400> 168
   agaattacgt gcaccagtta cggcaggaat gctacgcgtt taatgggaca cagcgcttcc 60
   tggagagata catctacaac cgggaggagt tcgcgcgctt cgacagcgac gtgggggagt 120
   tccgggcggt gacggagctg gggcggcctg ctgcggagta ctggaacagc cagaaggaca 180
   tcctggagga gaagcgggca gtgccggaca ggatgtgcag acacaactac gagctgggcg 240
   ggcccatgac cctgcagcgc cgag 264
<210> 169
   <211> 264
<212> DNA
   <213> Homo sapiens
<400> 169
   agaattacgt gcaccagtta cggcaggaat gctacgcgtt taatgggaca cagcgcttcc 60
   tggagagata catctacaac cgggaggagt tcgtgcgctt cgacagcgac gtgggggagt 120
   tccgggcggt gacggagctg gggcggcctg ctgcggagta ctggaacagc cagaaggacc 180
   tcctggagga gaagcgggca gtgccggaca gggtatgcag acacaactac gagctggacg 240
   aggccgtgac cctgcagcgc cgag 264
<210> 170
   <211> 263
   <212> DNA
   <213> Homo sapiens
<400> 170
   agaattacct tttccaggga cggcaggaat gctacgcgtt taatgggaca cagcgcttcc 60
   tggagagata catctacaac cgggaggagt tcgtgcgctt cgacagcgac gtgggggagt 120
   tccgggcggt gacggagctg gggcggcctg atgaggagta ctggaacagc cagaaggaca 180
   tcctggagga gaagcgggca gtgccggaca gggtatgcag acacaactac gagctggacg 240
   aggccgtgac cctgcagcgc cga 263
<210> 171
   <211> 251
   <212> DNA
   <213> Homo sapiens
<400> 171
   agaattacgt gtaccagtta cggcaggaat gctacgcgtt taatgggaca cagcgcttcc 60
   tggagagata catctacaac cgggaggagt tcgtgcgctt cgacagcgac gtgggggagt 120
   tccgggcggt gacggagctg gggcggcctg atgaggacta ctggaacagc cagaaggacc 180
   tcctggagga gaggcgggca gtgccggaca ggatgtgcag acacaactac gagctggacg 240
   aggccgtgac c 251
<210> 172
   <211> 255
   <212> DNA
   <213> Homo sapiens
<400> 172
   aattaccttt tccagggacg gcaggaatgc tacgcgttta atgggacaca gcgcttcctg      60
   gagagataca tctacaaccg ggaggagttc gtgcgcttcg acagcgacgt gggggagttc     120
   cgggcggtga cggagctggg gcggcctgct gcggagtact ggaacagcca gaaggacatc     180
   ctggaggagg agcgggcagt gccggacagg atgtgcagac acaactacga gctgggcggg     240
   cccatgaccc tgcag                                                      255
<210> 173
   <211> 255
   <212> DNA
   <213> Homo sapiens
<400> 173
   aattaccttt tccagggacg gcaggaatgc tacgcgttta atgggacaca gcgcttcctg      60
   gagagataca tctacaaccg ggaggagttc gcgcgcttcg acagcgacgt gggggagttc     120
   cgggcggtga cggagctggg gcggcctgct gcggagtact ggaacagcca gaaggacctc     180
   ctggaggaga agcgggcagt gccggacagg atgtgcagac acaactacga gctgggcggg     240
   cccatgaccc tgcag                                                      255
<210> 174
   <211> 255
   <212> DNA
   <213> Homo sapiens
<400> 174
   aattaccttt tccagggacg gcaggaatgc tacgcgttta atgggacaca gcgcttcctg      60
   gagagataca tctacaaccg ggaggagttc gtgcgcttcg acagcgacgt gggggagttc     120
   cgggcggtga cggagctggg gcggcctgat gaggagtact ggaacagcca gaaggacctc     180
   ctggaggaga agcgggcagt gccggacagg gtatgcagac acaactacga gctgggcggg     240
   cccatgaccc tgcag                                                      255
<210> 175
   <211> 249
   <212> DNA
   <213> Homo sapiens
<400> 175
   gtgtaccagt tacggcagga atgctacgcg tttaatggga cacagcgctt cctggagaga 60
   tacatctaca accggcagga gtacgcgcgc ttcgacagcg acgtgggaga gttccgggcg 120
   gtgacggagc tggggcggcc tgctgcggag tactggaaca gccagaagga cctcctggag 180
   gagaggcggg cagtgccgga caggatgtgc agacacaact acgagctggt cgggcccatg 240
   accctgcag 249
<210> 176
   <211> 249
   <212> DNA
   <213> Homo sapiens
<400> 176
   cttttccagg gacggcagga atgctacgcg tttaatggga cacagcgctt cctggagaga 60
   tacatctaca accgggagga gttcgtgcgc ttcgacagcg acgtggggga gttccgggcg 120
   gtgacggagc tggggcggcc tgatgaggag tactggaaca gccagaagga catcctggag 180
   gagaagcggg cagtgccgga cagggtatgc agacacaact acgagctggg cgggcccatg 240
   accctgcag 249
<210> 177
   <211> 257
   <212> DNA
   <213> Homo sapiens
<400> 177
   agaattacgt gcaccagtta cggcaggaat gctacgcgtt taatgggaca cagcgcttcc 60
   tggagagata catctacaac cgggaggagt tcgcgcgctt cgacagcgac gtgggggagt 120
   tccgggcggt gacggagctg gggcggcctg atgaggacta ctggaacagc cagaaggacc 180
   tcctggagga gaagcgggca gtgccggaca gggtatgcag acacaactac gagctggacg 240
   aggccgtgac cctgcag 257
<210> 178
   <211> 264
   <212> DNA
   <213> Homo sapiens
<400> 178
   agaattacct tttccaggga ctgcaggaat gctacgcgtt taatgggaca cagcgcttcc 60
   tggagagata catctacaac cgggaggagt tcgtgcgctt cgacagcgac gtgggggagt 120
   tccgggcggt gacggagctg gggcggcctg atgaggagta ctggaacagc cagaaggaca 180
   tcctggagga gaagcgggca gtgccggaca ggatgtgcag acacaactac gagctgggcg 240
   ggcccatgac cctgcagcgc cgag 264
<210> 179
   <211> 264
   <212> DNA
   <213> Homo sapiens
<400> 179
   agaattacgt gtaccagtta cggcaggaat gctacgcgtt taatgggaca cagcgcttcc 60
   tggagagata catctacaac cgggaggagt tcgtgcgctt cgacagcgac gtgggggagt 120
   tccgggcggt gacggagctg gggcggcctg atgaggacta ctggaacagc cagaaggacc 180
   tcctggagga gaagcgggca gtgctggaca gggtatgcag acacaactac gagctggacg 240
   aggccgtgac cctgcagcgc cgag 264
<210> 180
   <211> 264
   <212> DNA
   <213> Homo sapiens
<400> 180
   agaattacgt gtaccagtta cggcaggaat gctacgcgtt taatgggaca cagcgcttcc 60
   tggagagata catctacaac cgggaggagt tcgtgcgctt cgacagcgac gtgggggagt 120
   tccgggcggt gacggagctg gggcggcctg atgaggagta ctggaacagc cagaaggaca 180
   tcctggagga gaagcgggca gtgccggaca gggtatgcag acacaactac gagctggacg 240
   aggccgtgac cctgcagcgc cgag 264
<210> 181
   <211> 251
   <212> DNA
   <213> Homo sapiens
<400> 181
   agaattacct tttccaggga cggcaggaat gctacgcgtt taatgggaca cagcgcttcc 60
   tggagagata catctacaac cgggaggagt tcgtgcgctt cgacagcgac gtgggggagt 120
   tccgggcggt gacggagctg gggcggcctg atgaggacta ctggaacagc cagaaggaca 180
   tcctggagga gaagcgggca gtgccggaca ggatgtgcag acacaactac gagctgggcg 240
   ggcccatgac c 251
<210> 182
   <211> 264
   <212> DNA
   <213> Homo sapiens
<400> 182
   agaattacct tttccaggga cggcaggaat gctacgcgtt taatgggaca cagcgcttcc 60
   tggagagata catctacaac cgggaggagt tcgcgcgctt cgacagcgac gtgggggagt 120
   tccgggcggt gacggagctg gggcggcctg atgaggagta ctggaacagc cagaaggaca 180
   tcctggagga ggagcgggca gtgccggaca ggatgtgcag acacaactac gagctgggcg 240
   ggcccatgac cctgcagcgc cgag 264
<210> 183
   <211> 264
   <212> DNA
   <213> Homo sapiens
<400> 183
   agaattacct tttccaggga cggcaggaat gctacgcgtt taatgggaca cagcgcttcc 60
   tggagagata catctacaac cgggaggagt tcgtgcgctt cgacagcgac gtgggggagt 120
   tccgggcggt gacggagctg gggcggcctg atgaggagta ctggaacagc cagaaggaca 180
   tcctggagga gaagcgggca gtgccggaca ggatgtgcag acacaactac gagctgggcg 240
   ggcccatgac cctgcagcac cgag 264
<210> 184
   <211> 264
   <212> DNA
   <213> Homo sapiens
<400> 184
   agaattacct tttccaggga cggcaggaat gctacgcgtt taatgggaca cagcgcttcc     60
   tggagagata catctacaac cgggaggagt tcgtgcgctt cgacagcgac gtgggggagt    120
   tccgggcggt gacggagctg gggcggcctg atgaggagta ctggaacagc cagaaggact    180
   tcctggagga gaagcgggca gtgccggaca ggatgtgcag acacaactac gagctgggcg    240
   ggcccatgac cctgcagcgc cgag                                           264
<210> 185
   <211> 263
   <212> DNA
   <213> Homo sapiens
<400> 185
   agaattacct tttccaggga cggcaggaat gctacgcgtt taatgggaca cagcgcttcc     60
   tggagagata catctacaac cgggaggagt tcgtgcgctt cgacagcgac gtgggggagt    120
   tccgggcggt gacggagctg gggcggcctg aggaggagta ctggaacagc cagaaggaca    180
   tcctggagga gaagcgggca gtgccggaca gggtatgcag acacaactac gagctggacg    240
   aggccgtgac cctgcagcgc cga                                            263
<210> 186
   <211> 264
   <212> DNA
   <213> Homo sapiens
<400> 186
   agaattacgt gtaccagtta cggcaggaat gctacgcgtt taatgggaca cagcgcttcc     60
   tggagagata catctacaac cgggaggagt acgcgcgctt cgacagcgac gtgggggagt    120
   tccgggcggt gacggagctg gggcggcctg ctgcggagta ctggaacagc cagaaggaca    180
   tcctggagga gaagcgggca gtgccggaca ggatgtgcag acacaactac gagctggacg    240
   aggccgtgac cctgcagcac cgag                                           264
<210> 187
   <211> 262
   <212> DNA
   <213> Homo sapiens
<400> 187
   gaattacgtg caccagttac ggcaggaatg ctacgcgttt aatgggacac agcgcttcct 60
   ggagagatac atctacaacc gggaggagtt cgtgcgcttc gacagcgacg tgggggagtt 120
   ccgggcggtg acggagctgg ggcggcctga tgaggactac tggaacagcc agaaggacat 180
   cctggaggag gagcgggcag tgccggacag gatgtgcaga cacaactacg agctgggcgg 240
   gcccatgacc ctgcagcgcc ga 262
<210> 188
   <211> 264
   <212> DNA
   <213> Homo sapiens
<400> 188
   agaattacgt gtaccagtta cggcaggaat gctacgcgtt taatgggaca cagcgcttcc 60
   tggagagata catctacaac cgggaggagt tcgtgcgctt cgacagcgac gtgggggagt 120
   tccgggcggt gacggagctg gggcggcctg ctgcggagta ctggaacagc cagaaggacc 180
   tcctggagga gaagcgggca gtgccggaca ggatgtgcag acacaactac gagctggacg 240
   aggccgtgac cctgcagcgc cgag 264
<210> 189
   <211> 264
   <212> DNA
   <213> Homo sapiens
<400> 189
   agaattacgt gtaccagtta cggcaggaat gctacgcgtt taatgggaca cagcgcttcc 60
   tggagagata catctacaac cgggaggagt tcgtgcgctt cgacagcgac gtgggggagt 120
   tccgggcggt gacggagctg gggcggcctg atgaggacta ctggaacagc cagaaggaca 180
   tcctggagga ggagcgggca gtgccggaca gggtatgcag acacaactac gagctggacg 240
   aggccgtgac cctgcagcgc cgag 264
<210> 190
   <211> 264
   <212> DNA
   <213> Homo sapiens
<400> 190
   agaattacgt gtaccaggga cggcaggaat gctacgcgtt taatgggaca cagcgcttcc 60
   tggagagata catctacaac cgggaggagt acgcgcgctt cgacagcgac gtgggggagt 120
   tccgggcggt gacggagctg gggcggcctg ctgcggagta ctggaacagc cagaaggaca 180
   tcctggagga gaagcgggca gtgccggaca ggatgtgcag acacaactac gagctggacg 240
   aggccgtgac cctgcagcgc cgag 264
<210> 191
   <211> 264
   <212> DNA
   <213> Homo sapiens
<400> 191
   agaattacgt gtaccaggga cggcaggaat gctacgcgtt taatgggaca cagcgcttcc 60
   tggagagata catctacaac cgggaggagt tcgcgcgctt cgacagcgac gtgggggagt 120
   tccgggcggt gacggagctg gggcggcctg ctgcggagta ctggaacagc cagaaggaca 180
   tcctggagga gaagcgggca gtgccggaca gggtatgcag acacaactac gagctggacg 240
   aggccgtgac cctgcagcgc cgag 264
<210> 192
   <211> 264
   <212> DNA
   <213> Homo sapiens
<400> 192
   agaattacgt gcaccagtta cggcaggaat gctacgcgtt taatgggaca cagcgcttcc 60
   tggagagata catctacaac cgggaggagt tcgtgcgctt cgacagcgac gtgggggagt 120
   tccgggcggt gacggagctg gggcggcctg atgaggacta ctggaacagc cagaaggacc 180
   tcctggagga gaagcgggca gtgccggaca ggatgtgcag acacaactac gagctggacg 240
   aggccgtgac cctgcagcgc cgag 264
<210> 193
   <211> 261
   <212> PRT
   <213> Homo sapiens
<400> 193
<210> 194
   <211> 90

<212> PRT
   <213> Homo sapiens
<400> 194
   Pro Glu Asp Phe Val Tyr Gln Phe Lys Gly Leu Cys Tyr Phe Thr Asn
   1               5                   10                  15
   Gly Thr Glu Arg Val Arg Gly Val Thr Arg His Ile Tyr Asn Arg Glu
               20                  25                  30
   Glu Tyr Val Arg Phe Asp Ser Asp Val Gly Val Tyr Arg Ala Val Thr
           35                  40                  45
   Pro Gln Gly Arg Pro Val Ala Glu Tyr Trp Asn Ser Gln Lys Glu Val
       50                  55                  60
   Leu Glu Gly Ala Arg Ala Ser Val Asp Arg Val Cys Arg His Asn Tyr
   65                  70                  75                  80
   Glu Val Ala Tyr Arg Gly Ile Leu Gln Arg
                   85                  90
<210> 195
   <211> 229
   <212> PRT
   <213> Homo sapiens
<400> 195
   Arg Asp Ser Pro Glu Asp Phe Val Tyr Gln Phe Lys Gly Leu Cys Tyr
   1 5 10 15
   Phe Thr Asn Gly Thr Glu Arg Val Arg Gly Val Thr Arg His Ile Tyr
   20 25 30
   Asn Arg Glu Glu Tyr Val Arg Phe Asp Ser Asp Val Gly Val Tyr Arg
   35 40 45
   Ala Val Thr Pro Gln Gly Arg Pro Ser Ala Glu Tyr Trp Asn Ser Gln
   50 55 60
   Lys Glu Val Leu Glu Gly Ala Arg Ala Ser Val Asp Arg Val Cys Arg
   65 70 75 80
   His Asn Tyr Glu Val Ala Tyr Arg Gly Ile Leu Gln Arg Arg Val Glu
   85 90 95
   Pro Thr Val Thr Ile Ser Pro Ser Arg Thr Glu Ala Leu Asn His His
   100 105 110
   Asn Leu Leu Ile Cys Ser Val Thr Asp Phe Tyr Pro Ser His Ile Lys
   115 120 125
   Val Arg Trp Phe Arg Asn Asp Gln Glu Glu Thr Ala Gly Val Val Ser
   130 135 140
   Thr Pro Leu Ile Arg Asn Gly Asp Trp Thr Phe Gln Ile Leu Val Met
   145 150 155 160
   Leu Glu Met Thr Pro Gln Arg Gly Asp Val Tyr Thr Cys His Val Glu
   165 170 175
   His Pro Ser Leu Gln Ser Pro Ile Thr Val Glu Trp Arg Ala Gln Ser
   180 185 190
   Glu Ser Ala Gln Ser Lys Met Leu Ser Gly Val Gly Gly Phe Val Leu
   195 200 205
   Gly Leu Ile Phe Leu Gly Leu Gly Leu Ile Ile Arg Gln Arg Ser Arg
   210 215 220
   Lys Gly Leu Leu His
   225
<210> 196
   <211> 237
   <212> PRT
   <213> Homo sapiens
<400> 196
   Arg Asp Ser Pro Glu Asp Phe Val Tyr Gln Phe Lys Gly Leu Cys Tyr
   1 5 10 15
   Phe Thr Asn Gly Thr Glu Arg Val Arg Gly Val Thr Arg His Ile Tyr
   20 25 30
   Asn Arg Glu Glu Tyr Val Arg Phe Asp Ser Asp Val Gly Val Tyr Arg
   35 40 45
   Ala Val Thr Pro Gln Gly Arg Pro Asp Ala Glu Tyr Trp Asn Ser Gln
   50 55 60
   Lys Glu Val Leu Glu Gly Ala Arg Ala Ser Val Asp Arg Val Cys Arg
   65 70 75 80
   His Asn Tyr Glu Val Ala Tyr Arg Gly Ile Leu Gln Arg Arg Val Glu
   85 90 95
   Pro Thr Val Thr Ile Ser Pro Ser Arg Thr Glu Ala Leu Asn His His
   100 105 110
   Asn Leu Leu Ile Cys Ser Val Thr Asp Phe Tyr Pro Ser Gln Ile Lys
   115 120 125
   Val Arg Trp Phe Arg Asn Asp Gln Glu Glu Thr Ala Gly Val Val Ser
   130 135 140
   Thr Pro Leu Ile Arg Asn Gly Asp Trp Thr Phe Gln Ile Leu Val Met
   145 150 155 160
   Leu Glu Met Thr Pro Gln Arg Gly Asp Val Tyr Thr Cys His Val Glu
   165 170 175
   His Pro Ser Leu Gln Ser Pro Ile Thr Val Glu Trp Arg Ala Gln Ser
   180 185 190
   Glu Ser Ala Gln Ser Lys Met Leu Ser Gly Val Gly Gly Phe Val Leu
   195 200 205
   Gly Leu Ile Phe Leu Gly Leu Gly Leu Ile Ile Arg Gln Arg Ser Arg
   210 215 220
   Lys Gly Pro Gln Gly Pro Pro Pro Ala Gly Leu Leu His
   225 230 235
<210> 197
   <211> 49
   <212> PRT
   <213> Homo sapiens
<400> 197
   Thr Glu Arg Val Arg Gly Val Thr Arg His Ile Tyr Asn Arg Glu Glu
   1               5                   10                  15
   Tyr Val Arg Phe Asp Ser Asp Val Gly Val Tyr Arg Ala Val Thr Pro
               20                  25                  30
   Gln Gly Arg Pro Asp Ala Glu Tyr Trp Asn Ser Gln Lys Glu Val Leu
           35                  40                  45
   Glu
<210> 198
   <211> 74
   <212> PRT
   <213> Homo sapiens
<400> 198
   Tyr Gln Phe Lys Gly Leu Cys Tyr Phe Thr Asn Gly Thr Glu Arg Val
   1               5                   10                  15
   Arg Gly Val Thr Arg Tyr Ile Tyr Asn Arg Glu Glu Tyr Val Arg Phe
               20                  25                  30
   Asp Ser Asp Val Gly Val Tyr Arg Ala Val Thr Pro Gln Gly Arg Pro
           35                  40                  45
   Ser Ala Glu Tyr Trp Asn Ser Gln Lys Asp Ile Leu Glu Glu Asp Arg
       50                  55                  60
   Ala Ser Val Asp Arg Val Cys Arg His Asn
   65                  70
<210> 199
   <211> 261
   <212> PRT
   <213> Homo sapiens
<400> 199
   Met Ser Trp Lys Lys Ala Leu Arg Ile Pro Gly Gly Leu Arg Ala Ala
   1               5                   10                  15
   Thr Val Thr Leu Met Leu Ser Met Leu Ser Thr Pro Val Ala Glu Gly
               20                  25                  30
   Arg Asp Ser Pro Glu Asp Phe Val Tyr Gln Phe Lys Gly Met Cys Tyr
           35                  40                  45
   Phe Thr Asn Gly Thr Glu Arg Val Arg Leu Val Ser Arg Ser Ile Tyr
       50                  55                  60
   Asn Arg Glu Glu Ile Val Arg Phe Asp Ser Asp Val Gly Glu Phe Arg
   65                  70                  75                  80
   Ala Val Thr Leu Leu Gly Leu Pro Ala Ala Glu Tyr Trp Asn Ser Gln
                   85                  90                  95
   Lys Asp Ile Leu Glu Arg Lys Arg Ala Ala Val Asp Arg Val Cys Arg
               100                 105                 110
   His Asn Tyr Gln Leu Glu Leu Arg Thr Thr Leu Gln Arg Arg Val Glu
           115                 120                 125
   Pro Thr Val Thr Ile Ser Pro Ser Arg Thr Glu Ala Leu Asn His His
       130                 135                 140
   Asn Leu Leu Val Cys Ser Val Thr Asp Phe Tyr Pro Ala Gln Ile Lys
   145                 150                 155                 160
   Val Arg Trp Phe Arg Asn Asp Gln Glu Glu Thr Ala Gly Val Val Ser
                   165                 170                 175
   Thr Pro Leu Ile Arg Asn Gly Asp Trp Thr Phe Gln Ile Leu Val Met
               180                 185                 190
   Leu Glu Met Thr Pro Gln Arg Gly Asp Val Tyr Thr Cys His Val Glu
           195                 200                 205
   His Pro Ser Leu Gln Ser Pro Ile Thr Val Glu Trp Arg Ala Gln Ser
       210                 215                 220
   Glu Ser Ala Gln Ser Lys Met Leu Ser Gly Ile Gly Gly Phe Val Leu
   225                 230                 235                 240
   Gly Leu Ile Phe Leu Gly Leu Gly Leu Ile Ile His His Arg Ser Gln
                   245                 250                 255
   Lys Gly Leu Leu His
               260
<210> 200
   <211> 261
   <212> PRT
   <213> Homo sapiens
<400> 200
   Met Ser Trp Lys Lys Ala Leu Arg Ile Pro Gly Gly Leu Arg Ala Ala
   1               5                   10                  15
   Thr Val Thr Leu Met Leu Ser Met Leu Ser Thr Pro Val Ala Glu Gly
               20                  25                  30
   Arg Asp Ser Pro Glu Asp Phe Val Tyr Gln Phe Lys Gly Met Cys Tyr
           35                  40                  45
   Phe Thr Asn Gly Thr Glu Arg Val Arg Leu Val Ser Arg Ser Ile Tyr
       50                  55                  60
   Asn Arg Glu Glu Ile Val Arg Phe Asp Ser Asp Val Gly Glu Phe Arg
   65                  70                  75                  80
   Ala Val Thr Leu Leu Gly Leu Pro Ala Ala Glu Tyr Trp Asn Ser Gln
                   85                  90                  95
   Lys Asp Ile Leu Glu Arg Lys Arg Ala Ala Val Asp Arg Val Cys Arg
               100                 105                 110
   His Asn Tyr Gln Leu Glu Leu Arg Thr Thr Leu Gln Arg Arg Val Glu
           115                 120                 125
   Pro Thr Val Thr Ile Ser Pro Ser Arg Thr Glu Ala Leu Asn His His
       130                 135                 140
   Asn Leu Leu Val Cys Ser Val Thr Asp Phe Tyr Pro Ala Gln Ile Lys
   145                 150                 155                 160
   Val Arg Trp Phe Arg Asn Gly Gln Glu Glu Thr Ala Gly Val Val Ser
                   165                 170                 175
   Thr Pro Leu Ile Arg Asn Gly Asp Trp Thr Phe Gln Ile Leu Val Met
               180                 185                 190
   Leu Glu Met Thr Pro Gln Arg Gly Asp Val Tyr Thr Cys His Val Glu
           195                 200                 205
   His Pro Ser Leu Gln Ser Pro Ile Thr Val Glu Trp Arg Ala Gln Ser
   210 215 220
   Glu Ser Ala Gln Ser Lys Met Leu Ser Gly Ile Gly Gly Phe Val Leu
   225 230 235 240
   Gly Leu Ile Phe Leu Gly Leu Gly Leu Ile Ile His His Arg Ser Gln
   245 250 255
   Lys Gly Leu Leu His
   260
<210> 201
   <211> 122
   <212> PRT
   <213> Homo sapiens
<400> 201
   Asp Phe Val Tyr Gln Phe Lys Gly Met Cys Tyr Phe Thr Asn Gly Thr
   1 5 10 15
   Glu Arg Val Arg Leu Val Ser Arg Ser Ile Tyr Asn Arg Glu Glu Ile
   20 25 30
   Val Arg Phe Asp Ser Asp Val Gly Glu Phe Arg Ala Val Thr Leu Leu
   35 40 45
   Gly Leu Pro Asp Ala Glu Tyr Trp Asn Ser Gln Lys Asp Ile Leu Glu
   50 55 60
   Arg Lys Arg Ala Ala Val Asp Arg Val Cys Arg His Asn Tyr Gln Leu
   65 70 75 80
   Glu Leu Arg Thr Thr Leu Gln Arg Arg Pro Ser Arg Thr Glu Ala Leu
   85 90 95
   Asn His His Asn Leu Leu Val Cys Ser Val Thr Asp Phe Tyr Pro Ala
   100 105 110
   Gln Ile Lys Val Arg Trp Phe Arg Asn Gly
   115 120
<210> 202
   <211> 229
   <212> PRT
   <213> Homo sapiens
<400> 202
   Arg Asp Ser Pro Glu Asp Phe Val Tyr Gln Phe Lys Ala Met Cys Tyr
   1 5 10 15
   Phe Thr Asn Gly Thr Glu Arg Val Arg Tyr Val Thr Arg Tyr Ile Tyr
   20 25 30
   Asn Arg Glu Glu Tyr Ala Arg Phe Asp Ser Asp Val Glu Val Tyr Arg
   35 40 45
   Ala Val Thr Pro Leu Gly Pro Pro Asp Ala Glu Tyr Trp Asn Ser Gln
   50 55 60
   Lys Glu Val Leu Glu Arg Thr Arg Ala Glu Leu Asp Thr Val Cys Arg
   65 70 75 80
   His Asn Tyr Gln Leu Glu Leu Arg Thr Thr Leu Gln Arg Arg Val Glu
   85 90 95
   Pro Thr Val Thr Ile Ser Pro Ser Arg Thr Glu Ala Leu Asn His His
   100 105 110
   Asn Leu Leu Val Cys Ser Val Thr Asp Phe Tyr Pro Ala Gln Ile Lys
   115 120 125
   Val Arg Trp Phe Arg Asn Asp Gln Glu Glu Thr Thr Gly Val Val Ser
   130 135 140
   Thr Pro Leu Ile Arg Asn Gly Asp Trp Thr Phe Gln Ile Leu Val Met
   145 150 155 160
   Leu Glu Met Thr Pro Gln His Gly Asp Val Tyr Thr Cys His Val Glu
   165 170 175
   His Pro Ser Leu Gln Asn Pro Ile Thr Val Glu Trp Arg Ala Gln Ser
   180 185 190
   Glu Ser Ala Gln Ser Lys Met Leu Ser Gly Ile Gly Gly Phe Val Leu
   195 200 205
   Gly Leu Ile Phe Leu Gly Leu Gly Leu Ile Ile His His Arg Ser Gln
   210 215 220
   Lys Gly Leu Leu His
   225
<210> 203
   <211> 86
   <212> PRT
   <213> Homo sapiens
<400> 203
   Tyr Gln Phe Lys Ala Met Cys Tyr Phe Thr Asn Gly Thr Glu Arg Val
   1               5                   10                  15
   Arg Tyr Val Thr Arg Tyr Ile Tyr Asn Arg Glu Glu Tyr Ala Arg Phe
               20                  25                  30
   Asp Ser Asp Val Glu Val Tyr Arg Ala Val Thr Pro Leu Gly Pro Pro
           35                  40                  45
   Asp Ala Glu Tyr Trp Asn Ser Gln Lys Glu Val Leu Glu Arg Thr Arg
       50                  55                  60
   Ala Glu Leu Asp Thr Val Cys Arg His Asn Tyr Gln Leu Glu Leu Arg
   65                  70                  75                  80
   Thr Thr Leu Gln Arg Arg
                   85
<210> 204
   <211> 261
   <212> PRT
   <213> Homo sapiens
<400> 204
<210> 205
   <211> 261
   <212> PRT
   <213> Homo sapiens
<400> 205
<210> 206
   <211> 89
   <212> PRT
   <213> Homo sapiens
<400> 206
   Asp Phe Val Tyr Gln Phe Lys Gly Met Cys Tyr Phe Thr Asn Gly Thr
   1               5                   10                  15
   Glu Arg Val Arg Leu Val Thr Arg Tyr Ile Tyr Asn Arg Glu Glu Tyr
               20                  25                  30
   Ala Arg Phe Asp Ser Asp Val Gly Val Tyr Arg Ala Val Thr Pro Leu
           35                  40                  45
   Gly Pro Pro Asp Ala Glu Tyr Trp Asn Ser Gln Lys Glu Val Leu Glu
       50                  55                  60
   Arg Thr Arg Ala Glu Leu Asp Thr Val Cys Arg His Asn Tyr Gln Leu
   65                  70                  75                  80
   Glu Leu Arg Thr Thr Leu Gln Arg Arg
                   85
<210> 207
   <211> 199
   <212> PRT
   <213> Homo sapiens
<400> 207
   Arg Asp Ser Pro Glu Asp Phe Val Tyr Gln Phe Lys Ala Met Cys Tyr
   1               5                   10                  15
   Phe Thr Asn Gly Thr Glu Arg Val Arg Tyr Val Thr Arg Tyr Ile Tyr
               20                  25                  30
   Asn Arg Glu Glu Tyr Ala Arg Phe Asp Ser Asp Val Glu Val Tyr Arg
           35                  40                  45
   Ala Val Thr Pro Leu Gly Pro Pro Ala Ala Glu Tyr Trp Asn Ser Gln
       50                  55                  60
   Lys Glu Val Leu Glu Arg Thr Arg Ala Glu Leu Asp Thr Val Cys Arg
   65                  70                  75                  80
   His Asn Tyr Gln Leu Glu Leu Arg Thr Thr Leu Gln Arg Arg Val Glu
                   85                  90                  95
   Pro Thr Val Thr Ile Ser Pro Ser Arg Thr Glu Ala Leu Asn His His
               100                 105                 110
   Asn Leu Leu Val Cys Ser Val Thr Asp Phe Tyr Pro Ala Gln Ile Lys
           115                 120                 125
   Val Arg Trp Phe Arg Asn Asp Gln Glu Glu Thr Thr Gly Val Val Ser
       130                 135                 140
   Thr Pro Leu Ile Arg Asn Gly Asp Trp Thr Phe Gln Ile Leu Val Met
   145                 150                 155                 160
   Leu Glu Met Thr Pro Gln His Gly Asp Val Tyr Thr Cys His Val Glu
                   165                 170                 175
   His Pro Ser Leu Gln Asn Pro Ile Thr Val Glu Trp Arg Ala Gln Ser
               180                 185                 190
   Glu Ser Ala Gln Ser Lys Met
           195

<210> 208
   <211> 195
   <212> PRT
   <213> Homo sapiens
<400> 208
   Glu Asp Phe Val Tyr Gln Phe Lys Gly Met Cys Tyr Phe Thr Asn Gly
   1               5                   10                  15
   Thr Glu Arg Val Arg Gly Val Thr Arg Tyr Ile Tyr Asn Arg Glu Glu
               20                  25                  30
   Tyr Ala Arg Phe Asp Ser Asp Val Gly Val Tyr Arg Ala Val Thr Pro
           35                  40                  45
   Leu Gly Pro Pro Ala Ala Glu Tyr Trp Asn Ser Gln Lys Glu Val Leu
       50                  55                  60
   Glu Arg Thr Arg Ala Glu Leu Asp Thr Val Cys Arg His Asn Tyr Gln
   65                  70                  75                  80
   Leu Glu Leu Arg Thr Thr Leu Gln Arg Arg Val Glu Pro Thr Val Thr
                   85                  90                  95
   Ile Ser Pro Ser Arg Thr Glu Ala Leu Asn His His Asn Leu Leu Val
               100                 105                 110
   Cys Ser Val Thr Asp Phe Tyr Pro Ala Gln Ile Lys Val Arg Trp Phe
           115                 120                 125
   Arg Asn Asp Gln Glu Glu Thr Thr Gly Val Val Ser Thr Pro Leu Ile
       130                 135                 140
   Arg Asn Gly Asp Trp Thr Phe Gln Ile Leu Val Met Leu Glu Met Thr
   145                 150                 155                 160
   Pro Gln Arg Gly Asp Val Tyr Thr Cys His Val Glu His Pro Ser Leu
                   165                 170                 175
   Gln Asn Pro Ile Ile Val Glu Trp Arg Ala Gln Ser Glu Ser Ala Gln
               180                 185                 190
   Ser Lys Met
           195
<210> 209
   <211> 89
   <212> PRT
   <213> Homo sapiens
<400> 209
   Asp Phe Val Tyr Gln Phe Lys Gly Met Cys Tyr Phe Thr Asn Gly Thr
   1               5                   10                  15
   Glu Arg Val Arg Gly Val Thr Arg Tyr Ile Tyr Asn Arg Glu Glu Tyr
               20                  25                  30
   Ala Arg Phe Asp Ser Asp Val Gly Val Tyr Arg Ala Val Thr Pro Leu
           35                  40                  45
   Gly Pro Pro Ala Ala Glu Tyr Trp Asn Ser Gln Lys Glu Val Leu Glu
       50                  55                  60
   Arg Thr Arg Ala Glu Leu Asp Thr Val Cys Arg His Asn Tyr Gln Leu
   65                  70                  75                  80
   Glu Leu Arg Thr Thr Leu Gln Arg Arg
                   85
<210> 210
   <211> 89
   <212> PRT
   <213> Homo sapiens
<400> 210
   Asp Phe Val Tyr Gln Phe Lys Gly Met Cys Tyr Phe Thr Asn Gly Thr
   1               5                   10                  15
   Glu Arg Val Arg Leu Val Thr Arg Tyr Ile Tyr Asn Arg Glu Glu Tyr
               20                  25                  30
   Ala Arg Phe Asp Ser Asp Val Gly Val Tyr Arg Ala Val Thr Pro Leu
           35                  40                  45
   Gly Pro Pro Asp Ala Glu Tyr Trp Asn Ser Gln Lys Asp Ile Leu Glu
       50                  55                  60
   Glu Asp Arg Ala Ser Val Asp Thr Val Cys Arg His Asn Tyr Gln Leu
   65                  70                  75                  80
   Glu Leu Arg Thr Thr Leu Gln Arg Arg
                   85
<210> 211
   <211> 90
   <212> PRT
   <213> Homo sapiens
<400> 211
   Glu Asp Phe Val Tyr Gln Phe Lys Gly Met Cys Tyr Phe Thr Asn Gly
   1 5 10 15
   Thr Glu Arg Val Arg Leu Val Thr Arg Tyr Ile Tyr Asn Arg Glu Glu
   20 25 30
   Tyr Ala Arg Phe Asp Ser Asp Val Gly Val Tyr Arg Val Val Thr Pro
   35 40 45
   Leu Gly Pro Pro Ala Ala Glu Tyr Trp Asn Ser Gln Lys Glu Val Leu
   50 55 60
   Glu Arg Thr Arg Ala Glu Leu Asp Thr Val Cys Arg His Asn Tyr Gln
   65 70 75 80
   Leu Glu Leu Arg Thr Thr Leu Gln Arg Arg
   85 90
<210> 212
   <211> 89
   <212> PRT
   <213> Homo sapiens
<400> 212
   Asp Phe Val Tyr Gln Phe Lys Gly Met Cys Tyr Phe Thr Asn Gly Thr
   1 5 10 15
   Glu Arg Val Arg Leu Val Thr Arg Tyr Ile Tyr Asn Arg Glu Glu Tyr
   20 25 30
   Ala Arg Phe Asp Ser Asp Val Gly Val Tyr Arg Ala Val Thr Pro Leu
   35 40 45
   Gly Pro Pro Ala Ala Glu Tyr Trp Asn Ser Gln Lys Glu Val Leu Glu
   50 55 60
   Gly Thr Arg Ala Glu Leu Asp Thr Val Cys Arg His Asn Tyr Gln Leu
   65 70 75 80
   Glu Leu Arg Thr Thr Leu Gln Arg Arg
   85
<210> 213
   <211> 228
   <212> PRT
   <213> Homo sapiens
<400> 213
   Arg Asp Ser Pro Glu Asp Phe Val Tyr Gln Phe Lys Ala Met Cys Tyr
   1               5                   10                  15
   Phe Thr Asn Gly Thr Glu Arg Val Arg Tyr Val Thr Arg Tyr Ile Tyr
               20                  25                  30
   Asn Arg Glu Glu Tyr Ala Arg Phe Asp Ser Asp Val Glu Val Tyr Arg
           35                  40                  45
   Ala Val Thr Pro Leu Gly Pro Pro Asp Ala Glu Tyr Trp Asn Ser Gln
       50                  55                  60
   Lys Glu Val Leu Glu Arg Thr Arg Ala Glu Leu Asp Thr Val Cys Arg
   65                  70                  75                  80
   His Asn Tyr Gln Leu Glu Leu Arg Thr Thr Leu Gln Arg Arg Val Glu
                   85                  90                  95
   Pro Thr Val Thr Ile Ser Pro Ser Arg Thr Glu Ala Leu Asn His His
               100                 105                 110
   Asn Leu Leu Val Cys Ser Val Thr Asp Phe Tyr Pro Ala Gln Ile Lys
           115                 120                 125
   Val Arg Trp Phe Arg Asn Asp Gln Glu Glu Thr Thr Gly Val Val Ser
       130                 135                 140
   Thr Pro Leu Ile Arg Asn Gly Asp Trp Thr Phe Gln Ile Leu Val Met
   145                 150                 155                 160
   Leu Glu Met Thr Pro Gln His Ala Val Tyr Thr Cys His Val Glu His
                   165                 170                 175
   Pro Ser Leu Gln Asn Pro Ile Thr Val Glu Trp Arg Ala Gln Ser Glu
               180                 185                 190
   Ser Ala Gln Ser Lys Met Leu Ser Gly Ile Gly Gly Phe Val Leu Gly
           195                 200                 205
   Leu Ile Phe Leu Gly Leu Gly Leu Ile Ile His His Arg Ser Gln Lys
       210                 215                 220
   Gly Leu Leu His
   225
<210> 214
   <211> 229
   <212> PRT
   <213> Homo sapiens
<400> 214
   Arg Asp Ser Pro Glu Asp Phe Val Tyr Gln Phe Lys Ala Met Cys Tyr
   1               5                   10                  15
   Phe Thr Asn Gly Thr Glu Arg Val Arg Tyr Val Thr Arg Tyr Ile Tyr
               20                  25                  30
   Asn Arg Glu Glu Tyr Ala Arg Phe Asp Ser Asp Val Gly Val Tyr Arg
           35                  40                  45
   Ala Val Thr Pro Leu Gly Pro Pro Asp Ala Glu Tyr Trp Asn Ser Gln
       50                  55                  60
   Lys Glu Val Leu Glu Arg Thr Arg Ala Glu Leu Asp Thr Val Cys Arg
   65                  70                  75                  80
   His Asn Tyr Gln Leu Glu Leu Arg Thr Thr Leu Gln Arg Arg Val Glu
                   85                  90                  95
   Pro Thr Val Thr Ile Ser Pro Ser Arg Thr Glu Ala Leu Asn His His
               100                 105                 110
   Asn Leu Leu Val Cys Ser Val Thr Asp Phe Tyr Pro Ala Gln Ile Lys
           115                 120                 125
   Val Arg Trp Phe Arg Asn Asp Gln Glu Glu Thr Thr Gly Val Val Ser
       130                 135                 140
   Thr Pro Leu Ile Arg Asn Gly Asp Trp Thr Phe Gln Ile Leu Val Met
   145                 150                 155                 160
   Leu Glu Met Thr Pro Gln His Gly Asp Val Tyr Thr Cys His Val Glu
                   165                 170                 175
   His Pro Ser Leu Gln Asn Pro Ile Thr Val Glu Trp Arg Ala Gln Ser
               180                 185                 190
   Glu Ser Ala Gln Ser Lys Met Leu Ser Gly Ile Gly Gly Phe Val Leu
           195                 200                 205
   Gly Leu Ile Phe Leu Gly Leu Gly Leu Ile Ile His His Arg Ser Gln
       210                 215                 220
   Lys Gly Leu Leu His
   225
<210> 215
   <211> 229
   <212> PRT
   <213> Homo sapiens
<400> 215
   Arg Asp Ser Pro Glu Asp Phe Val Phe Gln Phe Lys Gly Met Cys Tyr
   1               5                   10                  15
   Phe Thr Asn Gly Thr Glu Leu Val Arg Gly Val Thr Arg Tyr Ile Tyr
               20                  25                  30
   Asn Arg Glu Glu Tyr Ala Arg Phe Asp Ser Asp Val Gly Val Tyr Arg
           35                  40                  45
   Ala Val Thr Pro Leu Gly Arg Leu Asp Ala Glu Tyr Trp Asn Ser Gln
       50                  55                  60
   Lys Asp Ile Leu Glu Glu Asp Arg Ala Ser Val Asp Thr Val Cys Arg
   65                  70                  75                  80
   His Asn Tyr Gln Leu Glu Leu Arg Thr Thr Leu Gln Arg Arg Val Glu
                   85                  90                  95
   Pro Thr Val Thr Ile Ser Pro Ser Arg Thr Glu Ala Leu Asn His His
               100                 105                 110
   Asn Leu Leu Val Cys Ser Val Thr Asp Phe Tyr Pro Ala Gln Ile Lys
           115                 120                 125
   Val Arg Trp Phe Arg Asn Asp Gln Glu Glu Thr Thr Gly Val Val Ser
       130                 135                 140
   Thr Pro Leu Ile Arg Asn Gly Asp Trp Thr Phe Gln Ile Leu Val Met
   145                 150                 155                 160
   Leu Glu Met Thr Pro Gln Arg Gly Asp Val Tyr Thr Cys His Val Glu
                   165                 170                 175
   His Pro Ser Leu Gln Asn Pro Ile Ile Val Glu Trp Arg Ala Gln Ser
               180                 185                 190
   Glu Ser Ala Gln Ser Lys Met Leu Ser Gly Ile Gly Gly Phe Val Leu
           195                 200                 205
   Gly Leu Ile Phe Leu Gly Leu Gly Leu Ile Ile His His Arg Ser Gln
       210                 215                 220
   Lys Gly Leu Leu His
   225
<210> 216
   <211> 261
   <212> PRT
   <213> Homo sapiens
<400> 216
<210> 217
   <211> 237
   <212> PRT
   <213> Homo sapiens
<400> 217
   Arg Asp Pro Pro Glu Asp Phe Val Leu Gln Phe Lys Ala Met Cys Tyr
   1               5                   10                  15
   Phe Thr Asn Gly Thr Glu Arg Val Arg Tyr Val Thr Arg Tyr Ile Tyr
               20                  25                  30
   Asn Arg Glu Glu Asp Val Arg Phe Asp Ser Asp Val Gly Val Tyr Arg
           35                  40                  45
   Ala Val Thr Pro Gln Gly Arg Pro Asp Ala Glu Tyr Trp Asn Ser Gln
       50                  55                  60
   Lys Asp Ile Leu Glu Arg Thr Arg Ala Glu Leu Asp Thr Val Cys Arg
   65                  70                  75                  80
   His Asn Tyr Glu Val Ala Phe Arg Gly Ile Leu Gln Arg Arg Val Glu
                   85                  90                  95
   Pro Thr Val Thr Ile Ser Pro Ser Arg Thr Glu Ala Leu Asn His His
               100                 105                 110
   Asn Leu Leu Val Cys Ser Val Thr Asp Phe Tyr Pro Gly Gln Ile Lys
           115                 120                 125
   Val Arg Trp Phe Arg Asn Asp Gln Glu Glu Thr Ala Gly Val Val Ser
       130                 135                 140
   Thr Pro Leu Ile Arg Asn Gly Asp Trp Thr Phe Gln Ile Leu Val Met
   145                 150                 155                 160
   Leu Glu Met Thr Pro Gln His Gly Asp Val Tyr Thr Cys His Val Glu
                   165                 170                 175
   His Pro Ser Leu Gln Ser Pro Ile Thr Val Glu Trp Arg Ala Gln Ser
               180                 185                 190
   Glu Ser Ala Gln Asn Lys Met Leu Ser Gly Ile Gly Gly Phe Val Leu
           195                 200                 205
   Gly Leu Ile Phe Leu Gly Leu Gly Leu Ile Ile Arg Gln Arg Ser Gln
       210                 215                 220
   Lys Gly Pro Gln Gly Pro Pro Pro Ala Gly Leu Leu His
   225                 230                 235
<210> 218
   <211> 68
   <212> PRT
   <213> Homo sapiens
<400> 218
   Ala Met Cys Tyr Phe Thr Asn Gly Thr Glu Arg Val Arg Tyr Val Thr
   1               5                   10                  15
   Arg Tyr Ile Tyr Asn Arg Glu Glu Asp Val Arg Phe Asp Ser Asp Val
               20                  25                  30
   Gly Val Tyr Arg Ala Val Thr Pro Gln Gly Arg Pro Asp Ala Glu Tyr
           35                  40                  45
   Trp Asn Ser Gln Lys Asp Ile Leu Glu Arg Thr Arg Ala Glu Leu Asp
       50                  55                  60
   Thr Val Cys Arg
   65
<210> 219
   <211> 225
   <212> PRT
   <213> Homo sapiens
<400> 219
   Arg Asp Pro Pro Glu Asp Phe Val Leu Gln Phe Lys Ala Met Cys Tyr
   1               5                   10                  15
   Phe Thr Asn Gly Thr Glu Arg Val Arg Tyr Val Thr Arg Tyr Ile Tyr
               20                  25                  30
   Asn Arg Glu Glu Asp Val Arg Phe Asp Ser Asp Val Gly Val Tyr Arg
           35                  40                  45
   Ala Val Thr Pro Gln Gly Arg Pro Asp Ala Glu Tyr Trp Asn Ser Gln
       50                  55                  60
   Lys Asp Ile Leu Glu Arg Thr Arg Ala Glu Leu Asp Thr Val Cys Arg
   65                  70                  75                  80
   His Asn Tyr Glu Val Ala Phe Arg Gly Ile Leu Gln Arg Arg Val Glu
                   85                  90                  95
   Pro Thr Val Thr Ile Ser Pro Ser Arg Thr Glu Ala Leu Asn His His
               100                 105                 110
   Asn Leu Leu Val Cys Ser Val Thr Asp Phe Tyr Pro Gly Gln Ile Lys
           115                 120                 125
   Val Arg Trp Phe Arg Asn Asp Gln Glu Glu Thr Ala Gly Val Val Ser
       130                 135                 140
   Thr Pro Leu Ile Arg Asn Gly Asp Trp Thr Phe Gln Ile Leu Val Met
   145                 150                 155                 160
   Leu Glu Met Thr Pro Gln His Gly Asp Val Tyr Thr Cys His Val Glu
                   165                 170                 175
   His Pro Ser Leu Gln Ser Pro Ile Thr Val Glu Trp Arg Ala Gln Ser
               180                 185                 190
   Glu Ser Ala Gln Asn Lys Met Leu Ser Gly Ile Gly Gly Phe Val Leu
           195                 200                 205
   Gly Leu Ile Phe Leu Gly Leu Gly Leu Ile Ile Arg Gln Arg Ser Gln
       210                 215                 220
   Lys
   225
<210> 220
   <211> 261
   <212> PRT
   <213> Homo sapiens
<400> 220
   Met Ser Trp Lys Lys Ala Leu Arg Ile Pro Gly Asp Leu Arg Val Ala
   1 5 10 15
   Thr Val Thr Leu Met Leu Ala Met Leu Ser Ser Leu Leu Ala Glu Gly
   20 25 30
   Arg Asp Ser Pro Glu Asp Phe Val Phe Gln Phe Lys Gly Met Cys Tyr
   35 40 45
   Phe Thr Asn Gly Thr Glu Arg Val Arg Leu Val Thr Arg Tyr Ile Tyr
   50 55 60
   Asn Arg Glu Glu Tyr Ala Arg Phe Asp Ser Asp Val Gly Val Tyr Arg
   65 70 75 80
   Ala Val Thr Pro Gln Gly Arg Pro Asp Ala Glu Tyr Trp Asn Ser Gln
   85 90 95
   Lys Glu Val Leu Glu Gly Thr Arg Ala Glu Leu Asp Thr Val Cys Arg
   100 105 110
   His Asn Tyr Glu Val Ala Phe Arg Gly Ile Leu Gln Arg Arg Val Glu
   115 120 125
   Pro Thr Val Thr Ile Ser Pro Ser Arg Thr Glu Ala Leu Asn His His
   130 135 140
   Asn Leu Leu Val Cys Ser Val Thr Asp Phe Tyr Pro Gly Gln Ile Lys
   145 150 155 160
   Val Arg Trp Phe Arg Asn Asp Gln Glu Glu Thr Ala Gly Val Val Ser
   165 170 175
   Thr Pro Leu Ile Arg Asn Gly Asp Trp Thr Phe Gln Ile Leu Val Met
   180 185 190
   Leu Glu Met Thr Pro Gln Arg Gly Asp Val Tyr Thr Cys His Val Glu
   195 200 205
   His Pro Ser Leu Gln Ser Pro Ile Thr Val Glu Trp Arg Ala Gln Ser
   210 215 220
   Glu Ser Ala Gln Ser Lys Met Leu Ser Gly Val Gly Gly Phe Val Leu
   225 230 235 240
   Gly Leu Ile Phe Leu Gly Leu Gly Leu Ile Ile Arg Gln Arg Ser Gln
   245 250 255
   Lys Gly Leu Leu His
   260

<210> 221
   <211> 229
   <212> PRT
   <213> Homo sapiens
<400> 221
   Arg Asp Ser Pro Glu Asp Phe Val Tyr Gln Phe Lys Gly Met Cys Tyr
   1               5                   10                  15
   Phe Thr Asn Gly Thr Glu Arg Val Arg Leu Val Thr Arg His Ile Tyr
               20                  25                  30
   Asn Arg Glu Glu Tyr Ala Arg Phe Asp Ser Asp Val Gly Val Tyr Arg
           35                  40                  45
   Ala Val Thr Pro Gln Gly Arg Pro Asp Ala Glu Tyr Trp Asn Ser Gln
       50                  55                  60
   Lys Glu Val Leu Glu Gly Thr Arg Ala Glu Leu Asp Thr Val Cys Arg
   65                  70                  75                  80
   His Asn Tyr Glu Val Ala Phe Arg Gly Ile Leu Gln Arg Arg Val Glu
                   85                  90                  95
   Pro Thr Val Thr Ile Ser Pro Ser Arg Thr Glu Ala Leu Asn His His
               100                 105                 110
   Asn Leu Leu Val Cys Ser Val Thr Asp Phe Tyr Pro Gly Gln Ile Lys
           115                 120                 125
   Val Arg Trp Phe Arg Asn Asp Gln Glu Glu Thr Ala Gly Val Val Ser
       130                 135                 140
   Thr Pro Leu Ile Arg Asn Gly Asp Trp Thr Phe Gln Ile Leu Val Met
   145                 150                 155                 160
   Leu Glu Met Thr Pro Gln Arg Gly Asp Val Tyr Thr Cys His Val Glu
                   165                 170                 175
   His Pro Ser Leu Gln Ser Pro Ile Thr Val Glu Trp Arg Ala Gln Ser
               180                 185                 190
   Glu Ser Ala Gln Ser Lys Met Leu Ser Gly Val Gly Gly Phe Val Leu
           195                 200                 205
   Gly Leu Ile Phe Leu Gly Leu Gly Leu Ile Ile Arg Gln Arg Ser Gln
       210                 215                 220
   Lys Gly Leu Leu His
   225
<210> 222
   <211> 229
   <212> PRT
   <213> Homo sapiens
<400> 222
   Arg Asp Ser Pro Glu Asp Phe Val Tyr Gln Phe Lys Gly Met Cys Tyr
   1               5                   10                  15
   Phe Thr Asn Gly Thr Glu Arg Val Arg Leu Val Thr Arg His Ile Tyr
               20                  25                  30
   Asn Arg Glu Glu Tyr Ala Arg Phe Asp Ser Asp Val Gly Val Tyr Arg
                   35                  40                  45
   Ala Val Thr Pro Gln Gly Arg Pro Val Ala Glu Tyr Trp Asn Ser Gln
       50                  55                  60
   Lys Glu Val Leu Glu Arg Thr Arg Ala Glu Leu Asp Thr Val Cys Arg
   65                  70                  75                  80
   His Asn Tyr Glu Val Gly Tyr Arg Gly Ile Leu Gln Arg Arg Val Glu
                   85                  90                  95
   Pro Thr Val Thr Ile Ser Pro Ser Arg Thr Glu Ala Leu Asn His His
               100                 105                 110
   Asn Leu Leu Val Cys Ser Val Thr Asp Phe Tyr Pro Gly Gln Ile Lys
           115                 120                 125
   Val Gln Trp Phe Arg Asn Asp Gln Glu Glu Thr Ala Gly Val Val Ser
       130                 135                 140
   Thr Pro Leu Ile Arg Asn Gly Asp Trp Thr Phe Gln Ile Leu Val Met
   145                 150                 155                 160
   Leu Glu Met Thr Pro Gln Arg Gly Asp Val Tyr Thr Cys His Val Glu
                   165                 170                 175
   His Pro Ser Leu Gln Ser Pro Ile Thr Val Glu Trp Arg Ala Gln Ser
               180                 185                 190
   Glu Ser Ala Gln Ser Lys Met Leu Ser Gly Val Gly Gly Phe Val Leu
           195                 200                 205
   Gly Leu Ile Phe Leu Gly Leu Gly Leu Ile Ile Arg Gln Arg Ser Gln
       210                 215                 220
   Lys Gly Leu Leu His
   225
<210> 223
   <211> 106
   <212> PRT
   <213> Homo sapiens
<400> 223
   Ser Pro Glu Asp Phe Val Tyr Gln Phe Lys Gly Met Cys Tyr Phe Thr
   1               5                   10                  15
   Asn Gly Thr Glu Arg Val Arg Leu Val Thr Arg His Ile Tyr Asn Arg
               20                  25                  30
   Glu Glu Tyr Ala Arg Phe Asp Ser Asp Val Gly Val Tyr Arg Ala Val
           35                  40                  45
   Thr Pro Gln Gly Arg Pro Val Ala Glu Tyr Trp Asn Ser Gln Lys Glu
       50                  55                  60
   Val Leu Glu Arg Thr Arg Ala Glu Leu Asp Thr Val Cys Arg His Asn
   65                  70                  75                  80
   Tyr Glu Val Gly Tyr Arg Gly Ile Leu Gln Arg Arg Val Glu Pro Thr
                   85                  90                  95
   Val Thr Ile Ser Pro Ser Arg Thr Glu Ala
               100                 105
<210> 224
   <211> 108
   <212> PRT
   <213> Homo sapiens
<400> 224
   Arg Asp Ser Pro Glu Asp Phe Val Tyr Gln Phe Lys Gly Leu Cys Tyr
   1               5                   10                  15
   Phe Thr Asn Gly Thr Glu Arg Val Arg Leu Val Thr Arg Tyr Ile Tyr
               20                  25                  30
   Asn Arg Glu Glu Tyr Ala Arg Phe Asp Ser Asp Val Gly Val Tyr Arg
           35                  40                  45
   Ala Val Thr Pro Gln Gly Arg Pro Val Ala Glu Tyr Trp Asn Ser Gln
       50                  55                  60
   Lys Glu Val Leu Glu Arg Thr Arg Ala Glu Leu Asp Thr Val Cys Arg
   65                  70                  75                  80
   His Asn Tyr Glu Val Gly Tyr Arg Gly Ile Leu Gln Arg Arg Val Glu
                   85                  90                  95
   Pro Thr Val Thr Ile Ser Pro Ser Arg Thr Glu Ala
               100                 105
<210> 225
   <211> 57
   <212> PRT
   <213> Homo sapiens
<400> 225
   Thr Glu Arg Val Arg Leu Val Thr Arg Tyr Ile Tyr Asn Arg Glu Glu
   1               5                   10                  15
   Tyr Ala Arg Phe Asp Ser Asp Val Gly Val Tyr Arg Ala Val Thr Pro
               20                  25                  30
   Gln Gly Arg Pro Val Ala Glu Tyr Trp Asn Ser Gln Lys Glu Val Leu
           35                  40                  45
   Glu Arg Thr Arg Ala Glu Leu Asp Thr
       50                  55
<210> 226
   <211> 58
   <212> PRT
   <213> Homo sapiens
<400> 226
   Thr Glu Arg Val Arg Leu Val Thr Arg Tyr Ile Tyr Asn Arg Glu Glu
   1               5                   10                  15
   Tyr Ala Arg Phe Asp Ser Asp Val Gly Val Tyr Arg Ala Val Thr Pro
               20                  25                  30
   Gln Gly Arg Pro Val Ala Glu Tyr Trp Asn Ser Gln Lys Glu Val Leu
           35                  40                  45
   Glu Arg Thr Arg Ala Ala Val Asp Arg Val
       50                  55
<210> 227
   <211> 82
   <212> PRT
   <213> Homo sapiens
<400> 227
   Tyr Gln Phe Lys Gly Met Cys Tyr Phe Thr Asn Gly Thr Glu Arg Val
   1 5 10 15
   Arg Leu Val Thr Arg His Ile Tyr Asn Arg Glu Glu Tyr Ala Arg Phe
   20 25 30
   Asp Ser Asp Val Gly Val Tyr Arg Ala Val Thr Pro Gln Gly Arg Pro
   35 40 45
   Asp Ala Glu Tyr Trp Asn Ser Gln Lys Glu Val Leu Glu Arg Thr Arg
   50 55 60
   Ala Glu Leu Asp Thr Val Cys Arg His Asn Tyr Glu Val Gly Tyr Arg
   65 70 75 80
   Gly Ile
<210> 228
   <211> 82
   <212> PRT
   <213> Homo sapiens
<400> 228
   Tyr Gln Phe Lys Gly Met Cys Tyr Phe Thr Asn Gly Thr Glu Arg Val
   1 5 10 15
   Arg Leu Val Thr Arg His Ile Tyr Asn Arg Glu Glu Tyr Ala Arg Phe
   20 25 30
   Asp Ser Asp Val Gly Val Tyr Arg Ala Val Thr Pro Gln Gly Arg Pro
   35 40 45
   Val Ala Glu Tyr Trp Asn Ser Gln Lys Glu Val Leu Glu Gly Thr Arg
   50 55 60
   Ala Glu Leu Asp Thr Val Cys Arg His Asn Tyr Glu Val Ala Phe Arg
   65 70 75 80
   Gly Ile
<210> 229
   <211> 229
   <212> PRT
   <213> Homo sapiens
<400> 229
   Arg Asp Ser Pro Glu Asp Phe Val Tyr Gln Phe Lys Gly Met Cys Tyr
   1               5                   10                  15
   Phe Thr Asn Gly Thr Glu Arg Val Arg Leu Val Thr Arg Tyr Ile Tyr
               20                  25                  30
   Asn Arg Glu Glu Tyr Ala Arg Phe Asp Ser Asp Val Gly Val Tyr Arg
           35                  40                  45
   Ala Val Thr Pro Gln Gly Arg Pro Val Ala Glu Tyr Trp Asn Ser Gln
       50                  55                  60
   Lys Glu Val Leu Glu Arg Thr Arg Ala Glu Leu Asp Thr Val Cys Arg
   65                  70                  75                  80
   His Asn Tyr Glu Val Gly Tyr Arg Gly Ile Leu Gln Arg Arg Val Glu
                   85                  90                  95
   Pro Thr Val Thr Ile Ser Pro Ser Arg Thr Glu Ala Leu Asn His His
               100                 105                 110
   Asn Leu Leu Val Cys Ser Val Thr Asp Phe Tyr Pro Gly Gln Ile Lys
           115                 120                 125
   Val Gln Trp Phe Arg Asn Asp Gln Glu Glu Thr Ala Gly Val Val Ser
       130                 135                 140
   Thr Pro Leu Ile Arg Asn Gly Asp Trp Thr Phe Gln Ile Leu Val Met
   145                 150                 155                 160
   Leu Glu Met Thr Pro Gln Arg Gly Asp Val Tyr Thr Cys His Val Glu
                   165                 170                 175
   His Pro Ser Leu Gln Ser Pro Ile Thr Val Glu Trp Arg Ala Gln Ser
               180                 185                 190
   Glu Ser Ala Gln Ser Lys Met Leu Ser Gly Val Gly Gly Phe Val Leu
           195                 200                 205
   Gly Leu Ile Phe Leu Gly Leu Gly Leu Ile Ile Arg Gln Arg Ser Gln
       210                 215                 220
   Lys Gly Leu Leu His
   225
<210> 230
   <211> 89
   <212> PRT
   <213> Homo sapiens
<400> 230
   Asp Phe Val Phe Gln Phe Lys Gly Met Cys Tyr Phe Thr Asn Gly Thr
   1               5                   10                  15
   Glu Arg Val Arg Leu Val Thr Arg Tyr Ile Tyr Asn Arg Glu Glu Tyr
               20                  25                  30
   Ala Arg Phe Asp Ser Asp Val Gly Val Tyr Arg Ala Val Thr Pro Gln
           35                  40                  45
   Gly Arg Pro Ser Ala Glu Tyr Trp Asn Ser Gln Lys Glu Val Leu Glu
       50                  55                  60
   Gly Thr Arg Ala Glu Leu Asp Thr Val Cys Arg His Asn Tyr Glu Val
   65                  70                  75                  80
   Ala Phe Arg Gly Ile Leu Gln Arg Arg
                   85
<210> 231
   <211> 85
   <212> PRT
   <213> Homo sapiens
<400> 231
   Tyr Gln Phe Lys Gly Met Cys Tyr Phe Thr Asn Gly Thr Glu Arg Val
   1               5                   10                  15
   Arg Leu Val Thr Arg Tyr Ile Tyr Asn Arg Glu Glu Tyr Ala Arg Phe
               20                  25                  30
   Asp Ser Asp Val Gly Val Tyr Arg Ala Val Thr Pro Gln Gly Arg Pro
           35                  40                  45
   Asp Ala Glu Tyr Trp Asn Ser Gln Lys Glu Val Leu Glu Gly Thr Arg
       50                  55                  60
   Ala Glu Leu Asp Thr Val Cys Arg His Asn Tyr Glu Val Ala Phe Arg
   65                  70                  75                  80
   Gly Ile Leu Gln Arg
                   85
<210> 232
   <211> 89
   <212> PRT
   <213> Homo sapiens
<400> 232
   Asp Phe Val Tyr Gln Phe Lys Gly Met Cys Tyr Phe Thr Asn Gly Thr
   1               5                   10                  15
   Glu Arg Val Arg Leu Val Thr Arg Tyr Ile Tyr Asn Arg Glu Glu Tyr
               20                  25                  30
   Ala Arg Phe Asp Ser Asp Val Gly Val Tyr Arg Ala Val Thr Pro Gln
           35                  40                  45
   Gly Arg Pro Asp Ala Glu Tyr Trp Asn Ser Gln Lys Glu Val Leu Glu
       50                  55                  60
   Gly Thr Arg Ala Glu Leu Asp Thr Val Cys Arg His Asn Tyr Glu Val
   65                  70                  75                  80
   Ala Phe Arg Gly Ile Leu Gln Arg Arg
                   85
<210> 233
   <211> 261
   <212> PRT
   <213> Homo sapiens
<400> 233
<210> 234
   <211> 85
   <212> PRT
   <213> Homo sapiens
<400> 234
   Glu Asp Phe Val Phe Gln Phe Lys Gly Met Cys Tyr Phe Thr Asn Gly
   1 5 10 15
   Thr Glu Arg Val Arg Leu Val Thr Arg Tyr Ile Tyr Asn Arg Glu Glu
   20 25 30
   Tyr Ala Arg Phe Asp Ser Asp Val Gly Val Tyr Arg Ala Val Thr Pro
   35 40 45
   Gln Gly Arg Pro Val Ala Glu Tyr Trp Asn Ser Gln Lys Glu Val Leu
       50                  55                  60
   Glu Gly Thr Arg Ala Glu Leu Asp Thr Val Cys Arg His Asn Tyr Glu
   65                  70                  75                  80
   Val Ala Phe Arg Gly
                   85
<210> 235
   <211> 86
   <212> PRT
   <213> Homo sapiens
<400> 235
   Phe Gln Phe Lys Gly Met Cys Tyr Phe Thr Asn Gly Thr Glu Arg Val
   1               5                   10                  15
   Arg Leu Val Thr Arg His Ile Tyr Asn Arg Glu Glu Tyr Ala Arg Phe
               20                  25                  30
   Asp Ser Asp Val Gly Val Tyr Arg Ala Val Thr Pro Gln Gly Arg Pro
           35                  40                  45
   Asp Ala Glu Tyr Trp Asn Ser Gln Lys Glu Val Leu Glu Gly Thr Arg
       50                  55                  60
   Ala Glu Leu Asp Thr Val Cys Arg His Asn Tyr Glu Val Ala Phe Arg
   65                  70                  75                  80
   Gly Ile Leu Gln Arg Arg
                   85
<210> 236
   <211> 89
   <212> PRT
   <213> Homo sapiens
<400> 236
   Asp Phe Val Phe Gln Phe Lys Gly Met Cys Tyr Phe Thr Asn Gly Thr
   1 5 10 15
   Glu Arg Val Arg Leu Val Thr Arg Tyr Ile Tyr Asn Arg Glu Glu Tyr
   20 25 30
   Ala Arg Phe Asp Ser Asp Val Gly Val Tyr Arg Ala Val Thr Pro Gln
   35 40 45
   Gly Arg Pro Asp Ala Glu Tyr Trp Asn Ser Gln Lys Glu Val Leu Glu
   50 55 60
   Arg Thr Arg Ala Glu Leu Asp Thr Val Cys Arg His Asn Tyr Glu Val
   65 70 75 80
   Gly Tyr Arg Gly Ile Leu Gln Arg Arg
   85

<210> 237
   <211> 89
   <212> PRT
   <213> Homo sapiens
<400> 237
   Asp Phe Val Phe Gln Phe Lys Gly Met Cys Tyr Phe Thr Asn Gly Thr
   1 5 10 15
   Glu Arg Val Arg Leu Val Thr Arg Tyr Ile Tyr Asn Arg Glu Glu Tyr
   20 25 30
   Ala Arg Phe Asp Ser Asp Val Gly Val Tyr Arg Ala Val Thr Pro Gln
   35 40 45
   Gly Arg Pro Asp Ala Glu Asn Trp Asn Ser Gln Lys Glu Val Leu Glu
   50 55 60
   Gly Thr Arg Ala Glu Leu Asp Thr Val Cys Arg His Asn Tyr Glu Val
   65 70 75 80
   Ala Phe Arg Gly Ile Leu Gln Arg Arg
   85
<210> 238
   <211> 80
   <212> PRT
   <213> Homo sapiens
<400> 238
   Tyr Gln Phe Lys Gly Met Cys Tyr Phe Thr Asn Gly Thr Glu Arg Val
   1               5                   10                  15
   Arg Leu Val Thr Arg His Ile Tyr Asn Arg Glu Glu Tyr Ala Arg Phe
               20                  25                  30
   Asp Ser Asp Val Gly Val Tyr Arg Ala Val Thr Pro Gln Gly Arg Pro
           35                  40                  45
   Val Ala Glu Tyr Trp Asn Ser Gln Lys Glu Val Leu Glu Gly Ala Arg
       50                  55                  60
   Ala Glu Leu Asp Thr Val Cys Arg His Asn Tyr Glu Val Gly Tyr Arg
   65                  70                  75                  80
<210> 239
   <211> 89
   <212> PRT
   <213> Homo sapiens
<400> 239
   Asp Phe Val Phe Gln Phe Lys Gly Met Cys Tyr Phe Thr Asn Gly Thr
   1               5                   10                  15
   Glu Arg Val Arg Leu Val Thr Arg Tyr Ile Tyr Asn Arg Glu Glu Tyr
               20                  25                  30
   Ala Arg Phe Asp Ser Asp Val Gly Val Tyr Arg Ala Val Thr Pro Leu
           35                  40                  45
   Gly Arg Pro Asp Ala Glu Tyr Trp Asn Ser Gln Lys Glu Val Leu Glu
       50                  55                  60
   Gly Thr Arg Ala Glu Leu Asp Thr Val Cys Arg His Asn Tyr Glu Val
   65                  70                  75                  80
   Ala Phe Arg Gly Ile Leu Gln Arg Arg
                   85
<210> 240
   <211> 786
   <212> DNA
   <213> Homo sapiens
<400> 240
   atgtcttgga agaagtcttt gcggatcccc ggagaccttc gggtagcaac tgtcaccttg 60
   atgctggcga tcctgagctc ctcactggct gagggcagag actctcccga ggatttcgtg 120
   taccagttta agggcctgtg ctacttcacc aacgggacgg agcgcgtgcg gggtgtgacc 180
   agacacatct ataaccgaga ggagtacgtg cgcttcgaca gcgacgtggg ggtgtaccgg 240
   gcagtgacgc cgcaggggcg gcctgttgcc gagtactgga acagccagaa ggaagtcctg 300
   gagggggccc gggcgtcggt ggacagggtg tgcagacaca actacgaggt ggcgtaccgc 360
   gggatcctgc agaggagagt ggagcccaca gtgaccatct ccccatccag gacagaggcc 420
   ctcaaccacc acaacctgct gatctgctcg gtgacagatt tctatccaag ccagatcaaa 480
   gtccggtggt ttcggaatga tcaggaggag acagccggcg ttgtgtccac ccccctcatt 540
   aggaacggtg actggacctt ccagatcctg gtgatgctgg aaatgactcc ccagcgtgga 600
   gatgtctaca cctgccacgt ggagcacccc agcctccaga gccccatcac cgtggagtgg 660
   cgggctcagt ctgaatctgc ccagagcaag atgctgagtg gcgttggagg cttcgtgctg 720
   gggctgatct tccttgggct tggccttatc atccgtcaaa ggagtcggaa agggcttctg 780
   cactga 786
<210> 241
   <211> 271
   <212> DNA
   <213> Homo sapiens
<400> 241
   tcccgaggat ttcgtgtacc agtttaaggg cctgtgctac ttcaccaacg ggacggagcg 60
   cgtgcggggt gtgaccagac acatctataa ccgagaggag tacgtgcgct tcgacagcga 120
   cgtgggggtg taccgggcgg tgacgccgca ggggcggcct gttgccgagt actggaacag 180
   ccagaaggaa gtcctggagg gggcccgggc gtcggtggac agagtgtgca gacacaacta 240
   cgaggtggcg taccgcggga tcctgcagag g 271
<210> 242
   <211> 690
   <212> DNA
   <213> Homo sapiens
<400> 242
   agagactctc ccgaggattt cgtgtaccag tttaagggcc tgtgctactt caccaacggg 60
   acggagcgcg tgcggggtgt gaccagacac atctataacc gagaggagta cgtgcgcttc 120
   gacagcgacg tgggggtgta ccgggcggtg acgccgcagg ggcggcctag cgccgagtac 180
   tggaacagcc agaaggaagt cctggagggg gcccgggcgt cggtggacag agtgtgcaga 240
   cacaactacg aggtggcgta ccgcgggatc ctgcagagga gagtggagcc cacagtgacc 300
   atctccccat ccaggacaga ggccctcaac caccacaacc tgctgatctg ctcggtgaca 360
   gatttctatc caagccacat caaagtccgg tggtttcgga atgatcagga ggagacagcc 420
   ggcgttgtgt ccacccccct cattaggaac ggtgactgga ccttccagat cctggtgatg 480
   ctggaaatga ctccccagcg tggagatgtc tacacctgcc acgtggagca ccccagcctc 540
   cagagcccca tcaccgtgga gtggcgggct cagtctgaat ctgcccagag caagatgctg 600
   agtggcgttg gaggcttcgt gctggggctg atcttccttg ggcttggcct tatcatccgt 660
   caaaggagtc ggaaagggct tctgcactga 690
<210> 243
   <211> 714
   <212> DNA
   <213> Homo sapiens
<400> 243
   agagactctc ccgaggattt cgtgtaccag tttaagggcc tgtgctactt caccaacggg 60
   acggagcgcg tgcggggtgt gaccagacac atctataacc gagaggagta cgtgcgcttc 120
   gacagcgacg tgggggtgta tcgggcggtg acgccgcagg ggcggcctga cgccgagtac 180
   tggaacagcc agaaggaagt cctggagggg gcccgggcgt cggtggacag agtgtgcaga 240
   cacaactacg aggtggcgta ccgcgggatc ctgcagagga gagtggagcc cacagtgacc 300
   atctccccat ccaggacaga ggccctcaac caccacaacc tgctgatctg ctcggtgaca 360
   gatttctatc caagccagat caaagtccgg tggtttcgga atgatcagga ggagacagcc 420
   ggcgttgtgt ccacccccct cattaggaac ggtgactgga ccttccagat cctggtgatg 480
   ctggaaatga ctccccagcg tggagatgtc tacacctgcc acgtggagca ccccagcctc 540
   cagagcccca tcaccgtgga gtggcgggct cagtctgaat ctgcccagag caagatgctg 600
   agtggcgttg gaggcttcgt gctggggctg atcttccttg ggcttggcct tatcatccgt 660
   caaaggagtc ggaaaggacc tcaagggcct ccaccagcag ggcttctgca ctga 714
<210> 244
   <211> 148
   <212> DNA
   <213> Homo sapiens
<400> 244
   gacggagcgc gtgcggggtg tgaccagaca catctataac cgagaggagt acgtgcgctt 60
   cgacagcgac gtgggggtgt atcgggcggt gacgccgcag gggcggcctg atgccgagta 120
   ctggaacagc cagaaggaag tcctggag 148
<210> 245
   <211> 223
   <212> DNA
   <213> Homo sapiens
<400> 245
   taccagttta agggcctgtg ctacttcacc aacgggacgg agcgcgtgcg gggtgtgacc 60
   agatacatct ataaccgaga agagtacgtg cgcttcgaca gcgacgtggg ggtgtaccgg 120
   gcggtgacgc cgcaggggcg gcctagcgcc gagtactgga acagccagaa ggacatcctg 180
   gaggaggacc gggcgtcggt ggacagggtg tgcagacaca act 223
<210> 246
   <211> 786
   <212> DNA
   <213> Homo sapiens
<400> 246
   atgtcttgga aaaaggcttt gcggatcccc ggaggccttc gggcagcaac tgtgaccttg 60
   atgctgtcga tgctgagcac cccagtggct gagggcagag actctcccga ggatttcgtg 120
   taccagttta agggcatgtg ctacttcacc aacgggacag agcgcgtgcg tcttgtgagc 180
   agaagcatct ataaccgaga agagatcgtg cgcttcgaca gcgacgtggg ggagttccgg 240
   gcggtgacgc tgctggggct gcctgccgcc gagtactgga acagccagaa ggacatcctg 300
   gagaggaaac gggcggcggt ggacagggtg tgcagacaca actaccagtt ggagctccgc 360
   acgaccttgc agcggcgagt ggagcccaca gtgaccatct ccccatccag gacagaggcc 420
   ctcaaccacc acaacctgct ggtctgctcg gtgacagatt tctatccagc ccagatcaaa 480
   gtccggtggt ttcggaatga ccaggaggag acagctggcg ttgtgtccac cccccttatt 540
   aggaatggtg actggacctt ccagatcctg gtgatgctgg aaatgactcc ccagcgtgga 600
   gacgtctaca cctgccacgt ggagcacccc agcctccaga gccccatcac cgtggagtgg 660
   cgggctcaat ctgaatctgc ccagagcaag atgctgagtg gcattggagg cttcgtgctg 720
   gggctgatct tcctcgggct gggccttatc atccatcaca ggagtcagaa agggctcctg 780
   cactga 786
<210> 247
   <211> 786
   <212> DNA
   <213> Homo sapiens
<400> 247
   atgtcttgga aaaaggcttt gcggatcccc ggaggccttc gggcagcaac tgtgaccttg      60
   atgctgtcga tgctgagcac cccagtggct gagggcagag actctcccga ggatttcgtg     120
   taccagttta agggcatgtg ctacttcacc aacgggacag agcgcgtgcg tcttgtgagc     180
   agaagcatct ataaccgaga agagatcgtg cgcttcgaca gcgacgtggg ggagttccgg     240
   gcggtgacgc tgctggggct gcctgccgcc gagtactgga acagccagaa ggacatcctg     300
   gagaggaaac gggcggcggt ggacagggtg tgcagacaca actaccagtt ggagctccgc     360
   acgaccttgc agcggcgagt ggagcccaca gtgaccatct ccccatccag gacagaggcc     420
   ctcaaccacc acaacctgct ggtctgctcg gtgacagatt tctatccagc ccagatcaaa     480
   gtccggtggt ttcggaatgg ccaggaggag acagctggcg ttgtgtccac cccccttatt     540
   aggaatggtg actggacctt ccagatcctg gtgatgctgg aaatgactcc ccagcgtgga     600
   gacgtctaca cctgccacgt ggagcacccc agcctccaga gccccatcac cgtggagtgg     660
   cgggctcaat ctgaatctgc ccagagcaag atgctgagtg gcattggagg cttcgtgctg     720
   gggctgatct tcctcgggct gggccttatc atccatcaca ggagtcagaa agggctcctg     780
   cactga                                                                786
<210> 248
   <211> 471
   <212> DNA
   <213> Homo sapiens
<400> 248
   aggatttcgt gtaccagttt aagggcatgt gctacttcac caacgggaca gagcgcgtgc      60
   gtcttgtgag cagaagcatc tataaccgag aagagatcgt gcgcttcgac agcgacgtgg     120
   gggagttccg ggcggtgacg ctgctggggc tgcctgacgc cgagtactgg aacagccaga     180
   aggacatcct ggagaggaaa cgggcggcgg tggacagggt gtgcagacac aactaccagt     240
   tggagctccg cacgaccttg cagcggcgac cccatccagg acagaggccc tcaaccacca     300
   caacctgctg gtctgctcgg tgacagattt ctatccagcc cagatcaaag tccggtggtt     360
   tcggaatggc caggaggaga cagctggcgt tgtgtccacc ccccttatta ggaatggtga     420
   ctggaccttc cagatcctgg tgatgctgga aatgactccc cagcgtggag a              471
<210> 249
   <211> 690
   <212> DNA
   <213> Homo sapiens
<400> 249
   agagactctc ccgaggattt cgtgtaccag tttaaggcca tgtgctactt caccaacggg 60
   acggagcgcg tgcgttatgt gaccagatac atctataacc gagaggagta cgcacgcttc 120
   gacagcgacg tggaggtgta ccgggcggtg acgccgctgg ggccgcctga cgccgagtac 180
   tggaacagcc agaaggaagt cctggagagg acccgggcgg agttggacac ggtgtgcaga 240
   cacaactacc agttggagct ccgcacgacc ttgcagcggc gagtggagcc cacagtgacc 300
   atctccccat ccaggacaga ggccctcaac caccacaacc tgctggtctg ctcagtgaca 360
   gatttctatc cagcccagat caaagtccgg tggtttcgga atgaccagga ggagacaacc 420
   ggcgttgtgt ccacccccct tattaggaac ggtgactgga ccttccagat cctggtgatg 480
   ctggaaatga ctccccagca tggagacgtc tacacctgcc acgtggagca ccccagcctc 540
   cagaacccca tcaccgtgga gtggcgggct cagtctgaat ctgcccagag caagatgctg 600
   agtggcattg gaggcttcgt gctggggctc atcttcctcg ggctgggcct tattatccat 660
   cacaggagtc agaaagggct cctgcactga 690
<210> 250
   <211> 261
   <212> DNA
   <213> Homo sapiens
<400> 250
   tgtaccagtt taaggccatg tgctacttca ccaacgggac ggagcgcgtg cgttatgtga 60
   ccagatacat ctataaccga gaggagtacg cgcgcttcga cagcgacgtg gaggtgtacc 120
   gggcggtgac gccgctgggg ccgcctgacg ccgagtactg gaacagccag aaggaagtcc 180
   tggagaggac ccgggcggag ttggacacgg tgtgcagaca caactaccag ttggagctcc 240
   gcacgacctt gcagcggcga g 261
<210> 251
   <211> 528
   <212> DNA
   <213> Homo sapiens
<400> 251
   atgtcttgga agaaggcttt gcggatccct ggaggccttc gggtagcaac tgtgaccttg      60
   atgctggcga tgctgagcac cccggtggct gagggcatgt cttggaagaa ggctttgcgg     120
   atccctggag gccttcgggt agcaactgtg accttgatgc tggcgatgct gagcaccccg     180
   gtggctgagg gcgtgacaga tttctatcca gcccagatca aagtccggtg gtttcggaat     240
   gaccaggagg agacaactgg cgttgtgtcc acccccctta ttaggaacgg tgactggacc     300
   ttccagatcc tggtgatgct ggaaatgact ccccagcgtg gagacgtcta cacctgccac     360
   gtggagcacc ccagcctcca gaaccccatc atcgtggagt ggcgggctca gtctgaatct     420
   gcccagagca agatgctgag tggcattgga ggcttcgtgc tggggctgat cttcctcggg     480
   ctgggcctta ttatccatca caggagtcag aaagggctcc tgcactga                  528
<210> 252
   <211> 786
   <212> DNA
   <213> Homo sapiens
<400> 252
   atgtcttgga agaaggcttt gcggatccct ggaggccttc gggtagcaac tgtgaccttg      60
   atgctggcga tgctgagcac cccggtggct gagggcagag actctcccga ggatttcgtg     120
   taccagttta agggcatgtg ctacttcacc aacgggacgg agcgcgtgcg tcttgtgacc     180
   agatacatct ataaccgaga ggagtacgca cgcttcgaca gcgacgtggg ggtgtatcgg     240
   gcggtgacgc cgctggggcc gcctgacgcc gagtactgga acagccagaa ggaagtcctg     300
   gagaggaccc gggcggagtt ggacacggtg tgcagacaca actaccagtt ggagctccgc     360
   acgaccttgc agcggcgagt ggagcccaca gtgaccatct ccccatccag gacagaggcc     420
   ctcaaccacc acaacctgct ggtctgctca gtgacagatt tctatccagc ccagatcaaa     480
   gtccggtggt ttcggaatga ccaggaggag acaactggcg ttgtgtccac cccccttatt     540
   aggaacggtg actggacctt ccagatcctg gtgatgctgg aaatgactcc ccagcgtgga     600
   gacgtctaca cctgccacgt ggagcacccc agcctccaga accccatcat cgtggagtgg     660
   cgggctcagt ctgaatctgc ccagagcaag atgctgagtg gcattggagg cttcgtgctg     720
   gggctgatct tcctcgggct gggccttatt atccatcaca ggagtcagaa agggctcctg     780
   cactga                                                                786
<210> 253
   <211> 270
   <212> DNA
   <213> Homo sapiens
<400> 253
   aggatttcgt gtaccagttt aagggcatgt gctacttcac caacgggacg gagcgcgtgc 60
   gtcttgtgac cagatacatc tataaccgag aggagtacgc gcgcttcgac agcgacgtgg 120
   gggtgtatcg ggcggtgacg ccgctggggc cgcctgacgc cgagtactgg aacagccaga 180
   aggaagtcct ggagaggacc cgggcggagt tggacacggt gtgcagacac aactaccagt 240
   tggagctccg cacgaccttg cagcggcgag 270
<210> 254
   <211> 786
   <212> DNA
   <213> Homo sapiens
<400> 254
   atgtcttgga agaaggcttt gcggatccct ggaggccttc gggtagcaac tgtgaccttg 60
   atgctggcga tgctgagcac cccggtggct gagggcagag actctcccga ggatttcgtg 120
   taccagttta agggcatgtg ctacttcacc aacgggacgg agcgcgtgcg tcttgtgacc 180
   agatacatct ataaccgaga ggagtacgca cgcttcgaca gcgacgtggg ggtgtatcgg 240
   gcggtgacgc cgctggggcc gcctgacgcc gagtactgga acagccagaa ggaagtcctg 300
   gagaggaccc gggcggagtt ggacacggtg tgcagacaca actaccagtt ggagctccgc 360
   acgaccttgc agcggcgagt ggagcccaca gtgaccatct ccccatccag gacagaggcc 420
   ctcaaccacc acaacctgct ggtctgctca gtgacagatt tctatccagc ccagatcaaa     480
   gtccggtggt ttcggaatga ccaggaggag acaactggcg ttgtgtccac cccccttatt     540
   aggaacggtg actggacctt ccagatcctg gtgatgctgg aaatgactcc ccagcgtgga 600
   gacgtctaca cctgccacgt ggagcacccc agcctccaga accccatcat cgtggagtgg 660
   cgggctcagt ctgaatctgc ccagagcaag atgctgagtg gcattggagg cttcgtgctg 720
   gggctgatct tcctcgggct gggccttatt atccatcaca ggagtcagaa agggctcctg 780
   cactga 786
<210> 255
   <211> 585
   <212> DNA
   <213> Homo sapiens
<400> 255
   gaggatttcg tgtaccagtt taagggcatg tgctacttca ccaacgggac cgagcgcgtg 60
   cggggtgtga ccagatacat ctataaccga gaggagtacg cgcgcttcga cagcgacgtg 120
   ggggtgtatc gggcggtgac gccgctgggg ccgcctgccg ccgagtactg gaacagccag 180
   aaggaagtcc tggagaggac ccgggcggag ttggacacgg tgtgcagaca caactaccag 240
   ttggagctcc gcacgacctt gcagcggcga gtggagccca cagtgaccat ctccccatcc 300
   aggacagagg ccctcaacca ccacaacctg ctggtctgct cagtgacaga tttctatcca 360
   gcccagatca aagtccggtg gtttcggaat gaccaggagg agacaactgg cgttgtgtcc 420
   acccccctta ttaggaacgg tgactggacc ttccagatcc tggtgatgct ggaaatgact 480
   ccccagcgtg gagacgtcta cacctgccac gtggagcacc ccagcctcca gaaccccatc 540
   atcgtggagt ggcgggctca gtctgaatct gcccagagca agatg 585
<210> 256
   <211> 270
   <212> DNA
   <213> Homo sapiens
<400> 256
   aggatttcgt gtaccagttt aagggcatgt gctacttcac caacgggacg gagcgcgtgc 60
   ggggtgtgac cagatacatc tataaccgag aggagtacgc gcgcttcgac agcgacgtgg 120
   gggtgtatcg ggcggtgacg ccgctggggc cgcctgccgc cgagtactgg aacagccaga 180
   aggaagtcct ggagaggacc cgggcggagt tggacacggt gtgcagacac aactaccagt 240
   tggagctccg cacgaccttg cagcggcgag 270
<210> 257
   <211> 270
   <212> DNA
   <213> Homo sapiens
<400> 257
   aggatttcgt gtaccagttt aagggcatgt gctacttcac caacgggacg gagcgcgtgc 60
   gtcttgtgac cagatacatc tataaccgag aggagtacgc acgcttcgac agcgacgtgg 120
   gggtgtatcg ggcggtgacg ccgctggggc cgcctgacgc cgagtactgg aatagccaga 180
   aggacatcct ggaggaggac cgggcgtcgg tggacaccgt atgcagacac aactaccagt 240
   tggagctccg cacgaccttg cagcggcgag 270
<210> 258
   <211> 270
<212> DNA
   <213> Homo sapiens
<400> 258
   gaggatttcg tgtaccagtt taagggcatg tgctacttca ccaacgggac ggagcgcgtg      60
   cgtcttgtga ccagatacat ctataaccga gaggagtacg cacgcttcga cagcgacgtg     120
   ggggtgtatc gggtggtgac gccgctgggg ccgcctgccg ccgagtactg gaacagccag     180
   aaggaagtcc tggagaggac ccgggcggag ttggacacgg tgtgcagaca caactaccag     240
   ttggagctcc gcacgacctt gcagcggcga                                      270
<210> 259
   <211> 270
   <212> DNA
   <213> Homo sapiens
<400> 259
   aggatttcgt gtaccagttt aagggcatgt gctacttcac caacgggacg gagcgcgtgc      60
   gtcttgtgac cagatacatc tataaccgag aggagtacgc acgcttcgac agcgacgtgg     120
   gggtgtatcg ggcggtgacg ccgctggggc cgcctgccgc cgagtactgg aacagccaga     180
   aggaagtcct ggaggggacc cgggcggagt tggacacggt gtgcagacac aactaccagt     240
   tggagctccg cacgaccttg cagcggcgag                                      270
<210> 260
   <211> 687
   <212> DNA
   <213> Homo sapiens
<400> 260
   agagactctc ccgaggattt cgtgtaccag tttaaggcca tgtgctactt caccaacggg      60
   acggagcgcg tgcgttatgt gaccagatac atctataacc gagaggagta cgcacgcttc     120
   gacagcgacg tggaggtgta ccgggcggtg acgccgctgg ggccgcctga cgccgagtac     180
   tggaacagcc agaaggaagt cctggagagg acccgggcgg agttggacac ggtgtgcaga     240
   cacaactacc agttggagct ccgcacgacc ttgcagcggc gagtggagcc cacagtgacc     300
   atctccccat ccaggacaga ggccctcaac caccacaacc tgctggtctg ctcagtgaca     360
   gatttctatc cagcccagat caaagtccgg tggtttcgga atgaccagga ggagacaacc     420
   ggcgttgtgt ccacccccct tattaggaac ggtgactgga ccttccagat cctggtgatg     480
   ctggaaatga ctccccagca tgccgtctac acctgccacg tggagcaccc cagcctccag     540
   aaccccatca ccgtggagtg gcgggctcag tctgaatctg cccagagcaa gatgctgagt     600
   ggcattggag gcttcgtgct ggggctcatc ttcctcgggc tgggccttat tatccatcac     660
   aggagtcaga aagggctcct gcactga                                         687
<210> 261
   <211> 690
   <212> DNA
   <213> Homo sapiens
<400> 261
   agagactctc ccgaggattt cgtgtaccag tttaaggcca tgtgctactt caccaacggg    60
   acggagcgcg tgcgttatgt gaccagatac atctataacc gagaggagta cgcacgcttc   120
   gacagcgacg tgggggtgta tcgggcggtg acgccgctgg ggccgcctga cgccgagtac   180
   tggaacagcc agaaggaagt cctggagagg acccgggcgg agttggacac ggtgtgcaga   240
   cacaactacc agttggagct ccgcacgacc ttgcagcggc gagtggagcc cacagtgacc   300
   atctccccat ccaggacaga ggccctcaac caccacaacc tgctggtctg ctcagtgaca   360
   gatttctatc cagcccagat caaagtccgg tggtttcgga atgaccagga ggagacaacc   420
   ggcgttgtgt ccacccccct tattaggaac ggtgactgga ccttccagat cctggtgatg   480
   ctggaaatga ctccccagca tggagacgtc tacacctgcc acgtggagca ccccagcctc   540
   cagaacccca tcaccgtgga gtggcgggct cagtctgaat ctgcccagag caagatgctg   600
   agtggcattg gaggcttcgt gctggggctc atcttcctcg ggctgggcct tattatccat   660
   cacaggagtc agaaagggct cctgcactga                                    690
<210> 262
   <211> 690
   <212> DNA
   <213> Homo sapiens
<400> 262
   agagactctc ccgaggattt cgtgttccag tttaagggca tgtgctactt caccaacggg    60
   accgagctcg tgcggggtgt gaccagatac atctataacc gagaggagta cgcgcgcttc   120
   gacagcgacg tgggggtgta tcgggcggtg acgccgctgg ggcggcttga cgccgagtac   180
   tggaatagcc agaaggacat cctggaggag gaccgggcgt cggtggacac cgtatgcaga   240
   cacaactacc agttggagct ccgcacgacc ttgcagcggc gagtggagcc cacagtgacc   300
   atctccccat ccaggacaga ggccctcaac caccacaacc tgctggtctg ctcagtgaca   360
   gatttctatc cagcccagat caaagtccgg tggtttcgga atgaccagga ggagacaact   420
   ggcgttgtgt ccacccccct tattaggaac ggtgactgga ccttccagat cctggtgatg   480
   ctggaaatga ctccccagcg tggagacgtc tacacctgcc acgtggagca ccccagcctc   540
   cagaacccca tcatcgtgga gtggcgggct cagtctgaat ctgcccagag caagatgctg   600
   agtggcattg gaggcttcgt gctggggctg atcttcctcg ggctgggcct tattatccat   660
   cacaggagtc agaaagggct cctgcactga                                    690
<210> 263
   <211> 786
   <212> DNA
   <213> Homo sapiens
<400> 263
   atgtcttgga agaaggcttt gcggatccct ggaggccttc gggtagcaac tgtgaccttg      60
   atgctggcga tgctgagcac cccggtggct gagggcagag actctcccga ggatttcgtg     120
   ttccagttta agggcatgtg ctacttcacc aacgggaccg agcgcgtgcg gggtgtgacc     180
   agatacatct ataaccgaga ggagtacgcg cgcttcgaca gcgacgtggg ggtgtatcgg     240
   gcggtgacgc cgctggggcg gcttgacgcc gagtactgga atagccagaa ggacatcctg     300
   gaggaggacc gggcgtcggt ggacaccgta tgcagacaca actaccagtt ggagctccgc     360
   acgaccttgc agcggcgagt ggagcccaca gtgaccatct ccccatccag gacagaggcc     420
   ctcaaccacc acaacctgct ggtctgctca gtgacagatt tctatccagc ccagatcaaa     480
   gtccggtggt ttcggaatga ccaggaggag acaactggcg ttgtgtccac cccccttatt    540
   aggaacggtg actggacctt ccagatcctg gtgatgctgg aaatgactcc ccagcgtgga     600
   gacgtctaca cctgccacgt ggagcacccc agcctccaga accccatcat cgtggagtgg     660
   cgggctcagt ctgaatctgc ccagagcaag atgctgagtg gcattggagg cttcgtgctg     720
   gggctgatct tcctcgggct gggccttatt atccatcaca ggagtcagaa agggctcctg     780
   cactga                                                                786

<210> 264
   <211> 714
   <212> DNA
   <213> Homo sapiens
<400> 264
   agagaccctc ccgaggattt cgtgctccag tttaaggcca tgtgctactt caccaatggg      60
   acggagcgcg tgcgttatgt gaccagatac atctataacc gagaggagga cgtgcgcttc     120
   gacagcgacg tgggggtgta tcgggcggtg acgccgcagg ggcggcctga cgccgagtac     180
   tggaacagcc agaaggacat cctggagagg acccgagcgg agttggacac ggtgtgcaga     240
   cacaactacg aggtggcgtt ccgcgggatc ttgcagagga gagtggagcc cacagtgacc     300
   atctccccat ccaggacaga ggccctcaac caccacaacc tgctggtctg ctcggtgaca     360
   gatttctatc caggccagat caaagtccgg tggtttcgga atgaccagga ggagacagct     420
   ggcgttgtgt ccacccccct tattaggaac ggtgactgga ccttccagat cctggtgatg     480
   ctggaaatga ctccccagca tggagacgtc tacacctgcc acgtggagca ccccagcctc     540
   cagagcccca tcaccgtgga gtggcgggct cagtctgaat ctgcccagaa caagatgctg     600
   agtggcattg gaggcttcgt gctggggctg atcttcctcg ggctgggcct tatcatccgt     660
   caaaggagtc agaaaggacc tcaagggcct ccaccagcag ggcttctgca ctga           714
<210> 265
   <211> 204
   <212> DNA
   <213> Homo sapiens
<400> 265
   gccatgtgct acttcaccaa cgggacggag cgcgtgcgtt atgtgaccag atacatctat      60
   aaccgagagg aggacgtgcg cttcgacagc gacgtggggg tgtatcgggc ggtgaccccg     120
   caggggcggc ctgacgccga gtactggaac agccagaagg acatcctgga gaggacccga     180
   gcggagttgg acacggtgtg caga                                            204
<210> 266
   <211> 880
   <212> DNA
   <213> Homo sapiens
<400> 266
   gccatgtgct acttcaccaa cgggacggag cgcgtgcgtt atgtgaccag atacatctat      60
   aaccgagagg aggacgtgcg cttcgacagc gacgtggggg tgtatcgggc ggtgaccccg     120
   caggggcggc ctgacgccga gtactggaac agccagaagg acatcctgga gaggacccga     180
   gcggagttgg acacggtgtg cagaagagac cctcccgagg atttcgtgct ccagtttaag     240
   gccatgtgct acttcaccaa tgggacggag cgcgtgcgtt atgtgaccag atacatctat     300
   aaccgagagg aggacgtgcg cttcgacagc gacgtggggg tgtatcgggc ggtgacgccg     360
   caggggcggc ctgacgccga gtactggaac agccagaagg acatcctgga gaggacccga     420
   gcggagttgg acacggtgtg cagacacaac tacgaggtgg cgttccgcgg gatcttgcag     480
   aggagagtgg agcccacagt gaccatctcc ccatccagga cagaggccct caaccaccac     540
   aacctgctgg tctgctcggt gacagatttc tatccaggcc agatcaaagt ccggtggttt     600
   cggaatgacc aggaagagac agctggcgtt gtgtccaccc cccttattag gaacggtgac     660
   tggaccttcc agatcctggt gatgctggaa atgactcccc agcatggaga cgtctacacc     720
   tgccacgtgg agcaccccag cctccagagc cccatcaccg tggagtggcg ggctcagtct     780
   gaatctgccc agaacaagat gctgagtggc attggaggct tcgtgctggg gctgatcttc     840
   ctcgggctgg gccttatcat ccgtcaaagg agtcagaaag                           880
<210> 267
   <211> 786
   <212> DNA
   <213> Homo sapiens
<400> 267
   atgtcttgga agaaggcttt gcggatcccc ggagaccttc gggtagcaac tgtcaccttg 60
   atgctggcga tgctgagctc cctactggct gagggcagag actctcccga ggatttcgtg 120
   ttccagttta agggcatgtg ctacttcacc aacgggacgg agcgcgtgcg tcttgtgacc 180
   agatacatct ataaccgaga ggagtacgcg cgcttcgaca gcgacgtggg ggtgtaccgc 240
   gcggtgacgc cgcaggggcg gcctgatgcc gagtactgga acagccagaa ggaagtcctg 300
   gaggggaccc gggcggagtt ggacacggtg tgcagacaca actacgaggt ggcgttccgc 360
   gggatcttgc agaggagagt ggagcccaca gtgaccatct ccccatccag gacagaggcc 420
   ctcaaccacc acaacctgct ggtctgctcg gtgacagatt tctatccagg ccagatcaaa 480
   gtccggtggt ttcggaatga tcaggaggag acagccggcg ttgtgtccac cccccttatt 540
   aggaatggtg actggacttt ccagatcctg gtgatgctgg aaatgactcc ccagcgtgga 600
   gatgtctaca cctgccacgt ggagcacccc agcctccaga gccccatcac cgtggagtgg 660
   cgggctcagt ctgaatctgc ccagagcaag atgctgagtg gcgttggagg cttcgtgctg 720
   gggctgatct tccttgggct gggccttatc atccgtcaaa ggagtcagaa agggcttctg 780
   cactga 786
<210> 268
   <211> 690
   <212> DNA
   <213> Homo sapiens
<400> 268
   agagactctc ccgaggattt cgtgtaccag tttaagggca tgtgctactt caccaacggg 60
   acggagcgcg tgcgtcttgt aaccagacac atctataacc gagaggagta cgcgcgcttc 120
   gacagcgacg tgggggtgta ccgcgcggtg acgccgcagg ggcggcctga tgccgagtac 180
   tggaacagcc agaaggaagt cctggagggg acccgggcgg agttggacac ggtgtgcaga 240
   cacaactacg aggtggcgtt ccgcgggatc ttgcagagga gagtggagcc cacagtgacc 300
   atctccccat ccaggacaga ggccctcaac caccacaacc tgctggtctg ctcggtgaca 360
   gatttctatc caggccagat caaagtccgg tggtttcgga atgatcagga ggagacagcc 420
   ggcgttgtgt ccacccccct tattaggaat ggtgactgga ctttccagat cctggtgatg 480
   ctggaaatga ctccccagcg tggagatgtc tacacctgcc acgtggagca ccccagcctc 540
   cagagcccca tcaccgtgga gtggcgggct cagtctgaat ctgcccagag caagatgctg 600
   agtggcgttg gaggcttcgt gctggggctg atcttccttg ggctgggcct tatcatccgt 660
   caaaggagtc agaaagggct tctgcactga 690
<210> 269
   <211> 690
   <212> DNA
   <213> Homo sapiens
<400> 269
   agagactctc ccgaggattt cgtgtaccag tttaagggca tgtgctactt caccaacggg 60
   acggagcgcg tgcgtcttgt aaccagacac atctataacc gagaggagta cgcgcgcttc 120
   gacagcgacg tgggggtgta ccgggcggtg acgccgcagg ggcggcctgt tgccgagtac 180
   tggaacagcc agaaggaagt cctggagagg acccgggcgg agttggacac ggtgtgcaga 240
   cacaactacg aggtggggta ccgcgggatc ctgcagagga gagtggagcc cacagtgacc 300
   atctccccat ccaggacaga ggccctcaac caccacaacc tgctggtctg ctcggtgaca 360
   gatttctatc caggccagat caaagtccag tggtttcgga atgatcagga ggagacagcc 420
   ggcgttgtgt ccacccccct tattaggaat ggtgactgga ctttccagat cctggtgatg 480
   ctggaaatga ctccccagcg tggagatgtc tacacctgcc acgtggagca ccccagcctc 540
   cagagcccca tcaccgtgga gtggcgggct cagtctgaat ctgcccagag caagatgctg 600
   agtggcgttg gaggcttcgt gctggggctg atcttccttg ggctgggcct tatcatccgt 660
   caaaggagtc agaaagggct tctgcactga 690
<210> 270
   <211> 318
   <212> DNA
   <213> Homo sapiens
<400> 270
   tctcccgagg atttcgtgta ccagtttaag ggcatgtgct acttcaccaa cgggacggag 60
   cgcgtgcgtc ttgtaaccag acacatctat aaccgagagg agtacgcgcg cttcgacagc 120
   gacgtggggg tgtaccgcgc ggtgacgccg caggggcggc ctgttgccga gtactggaac 180
   agccagaagg aagtcctgga gaggacccgg gcggagttgg acacggtgtg cagacacaac 240
   tacgaggtgg ggtaccgcgg gatcctgcag aggagagtgg agcccacagt gaccatctcc 300
   ccatccagga cagaggcc 318
<210> 271
   <211> 324
   <212> DNA
   <213> Homo sapiens
<400> 271
   agagactctc ccgaggattt cgtgtaccag tttaagggcc tgtgctactt caccaacggg 60
   acggagcgcg tgcgtcttgt aaccagatac atctataacc gagaggagta cgcgcgcttc 120
   gacagcgacg tgggggtgta ccgggcggtg acgccgcagg ggcggcctgt tgccgagtac 180
   tggaacagcc agaaggaagt cctggagagg acccgggcgg agttggacac ggtgtgcaga 240
   cacaactacg aggtggggta ccgcgggatc ctgcagagga gagtggagcc cacagtgacc 300
   atctccccat ccaggacaga ggcc 324
<210> 272
   <211> 173
   <212> DNA
   <213> Homo sapiens
<400> 272
   ggacggagcg cgtgcgtctt gtaaccagat acatctataa ccgagaggag tacgcgcgct 60
   tcgacagcga cgtgggggtg taccgggcgg tgacgccgca ggggcggcct gtcgccgagt 120
   actggaacag ccagaaggaa gtcctggaga ggacccgggc ggagttggac acg 173
<210> 273
   <211> 176
   <212> DNA
   <213> Homo sapiens
<400> 273
   ggacggagcg cgtgcgtctt gtaaccagat acatctataa ccgagaggag tacgcgcgct 60
   tcgacagcga cgtgggggtg taccgggcgg tgacgccgca ggggcggcct gttgccgagt 120
   actggaacag ccagaaggaa gtcctggaga ggacccgggc ggcggtggac agggtg 176
<210> 274
   <211> 247
   <212> DNA
   <213> Homo sapiens
<400> 274
   taccagttta agggcatgtg ctacttcacc aacgggacgg agcgcgtgcg tcttgtaacc 60
   agacacatct ataaccgaga ggagtacgcg cgcttcgaca gcgacgtggg ggtgtaccgc 120
   gcggtgacgc cgcaggggcg gcctgatgcc gagtactgga acagccagaa ggaagtcctg 180
   gagaggaccc gggcggagtt ggacacggtg tgcagacaca actacgaggt ggggtaccgc 240
   gggatcc 247
<210> 275
   <211> 247
   <212> DNA
   <213> Homo sapiens
<400> 275
   taccagttta agggcatgtg ctacttcacc aacgggacgg agcgcgtgcg tcttgtaacc 60
   agacacatct ataaccgaga ggagtacgcg cgcttcgaca gcgacgtggg ggtgtaccgc 120
   gcggtgacgc cgcaggggcg gcctgttgcc gagtactgga acagccagaa ggaagtcctg 180
   gaggggaccc gggcggagtt ggacacggtg tgcagacaca actacgaggt ggcgttccgc 240
   gggatct 247
<210> 276
   <211> 690
   <212> DNA
   <213> Homo sapiens
<400> 276
   agagactctc ccgaggattt cgtgtaccag tttaagggca tgtgctactt caccaacggg 60
   acggagcgcg tgcgtcttgt aaccagatac atctataacc gagaggagta cgcgcgcttc 120
   gacagcgacg tgggggtgta ccgggcggtg acgccgcagg ggcggcctgt tgccgagtac 180
   tggaacagcc agaaggaagt cctggagagg acccgggcgg agttggacac ggtgtgcaga 240
   cacaactacg aggtggggta ccgcgggatc ctgcagagga gagtggagcc cacagtgacc 300
   atctccccat ccaggacaga ggccctcaac caccacaacc tgctggtctg ctcggtgaca 360
   gatttctatc caggccagat caaagtccag tggtttcgga atgatcagga ggagacagcc 420
   ggcgttgtgt ccacccccct tattaggaat ggtgactgga ctttccagat cctggtgatg 480
   ctggaaatga ctccccagcg tggagatgtc tacacctgcc acgtggagca ccccagcctc 540
   cagagcccca tcaccgtgga gtggcgggct cagtctgaat ctgcccagag caagatgctg 600
   agtggcgttg gaggcttcgt gctggggctg atcttccttg ggctgggcct tatcatccgt 660
   caaaggagtc agaaagggct tctgcactga 690
<210> 277
   <211> 270
   <212> DNA
   <213> Homo sapiens
<400> 277
   aggatttcgt gttccagttt aagggcatgt gctacttcac caacgggacg gagcgcgtgc 60
   gtcttgtgac cagatacatc tataaccgag aggagtacgc gcgcttcgac agcgacgtgg 120
   gggtgtaccg cgcggtgacg ccgcaggggc ggcctagcgc cgagtactgg aacagccaga 180
   aggaagtcct ggaggggacc cgggcggagt tggacacggt gtgcagacac aactacgagg 240
   tggcgttccg cgggatcttg cagaggagag 270
<210> 278
   <211> 257
   <212> DNA
   <213> Homo sapiens
<400> 278
   tgtaccagtt taagggcatg tgctacttca ccaacgggac ggagcgcgtg cgtcttgtga     60
   ccagatacat ctataaccga gaggagtacg cgcgcttcga cagcgacgtg ggggtgtacc     120
   gcgcggtgac gccgcagggg cggcctgatg ccgagtactg gaacagccag aaggaagtcc    180
   tggaggggac ccgggcggag ttggacacgg tgtgcagaca caactacgag gtggcgttcc    240
   gcgggatctt gcagagg                                                   257
<210> 279
   <211> 270
   <212> DNA
   <213> Homo sapiens
<400> 279
   aggatttcgt gtaccagttt aagggcatgt gctacttcac caacgggacg gagcgcgtgc     60
   gtcttgtaac cagatacatc tataaccgag aggagtacgc gcgcttcgac agcgacgtgg    120
   gggtgtaccg cgcggtgacg ccgcaggggc ggcctgatgc cgagtactgg aacagccaga    180
   aggaagtcct ggaggggacc cgggcggagt tggacacggt gtgcagacac aactacgagg    240
   tggcgttccg cgggatcttg cagaggagag                                     270
<210> 280
   <211> 786
   <212> DNA
   <213> Homo sapiens
<400> 280
   atgtcttgga agaaggcttt gcggatcccc ggagaccttc gggtagcaac tgtcaccttg 60
   atgctggcga tgctgagctc cctactggct gagggcagag actctcccga ggatttcgtg 120
   taccagttta agggcatgtg ctacttcacc aacgggacgg agcgcgtgcg tcttgtaacc 180
   agatacatct ataaccgaga ggagtacgcg cgcttcgaca gcgacgtggg ggtgtaccgg 240
   gcggtgacgc cgcaggggcg gcctgttgcc gagtactgga acagccagaa ggaagtcctg 300
   gaggggaccc gggcggagtt ggacacggtg tgcagacaca actacgaggt ggggtaccgc 360
   gggatcctgc agaggagagt ggagcccaca gtgaccatct ccccatccag gacagaggcc 420
   ctcaaccacc acaacctgct ggtctgctcg gtgacagatt tctatccagg ccagatcaaa 480
   gtccagtggt ttcggaatga tcaggaggag acagccggcg ttgtgtccac cccccttatt 540
   aggaatggtg actggacttt ccagatcctg gtgatgctgg aaatgactcc ccagcgtgga 600
   gatgtctaca cctgccacgt ggagcacccc agcctccaga gccccatcac cgtggagtgg 660
   cgggctcagt ctgaatctgc ccagagcaag atgctgagtg gcgttggagg cttcgtgctg 720
   gggctgatct tccttgggct gggccttatc atccgtcaaa ggagtcagaa agggcttctg 780
   cactga 786
<210> 281
   <211> 257
   <212> DNA
   <213> Homo sapiens
<400> 281
   gaggatttcg tgttccagtt taagggcatg tgctacttca ccaacgggac ggagcgcgtg 60
   cgtcttgtga ccagatacat ctataaccga gaggagtacg cgcgcttcga cagcgacgtg 120
   ggggtgtacc gcgcggtgac gccgcagggg cggcctgttg ccgagtactg gaacagccag 180
   aaggaagtcc tggaggggac ccgggcggag ttggacacgg tgtgcagaca caactacgag 240
   gtggcgttcc gcgggat 257
<210> 282
   <211> 261
   <212> DNA
   <213> Homo sapiens
<400> 282
   tgttccagtt taagggcatg tgctacttca ccaacgggac ggagcgcgtg cgtcttgtaa 60
   ccagacacat ctataaccga gaggagtacg cgcgcttcga cagcgacgtg ggggtgtacc 120
   gcgcggtgac gccgcagggg cggcctgatg ccgagtactg gaacagccag aaggaagtcc 180
   tggaggggac ccgggcggag ttggacacgg tgtgcagaca caactacgag gtggcgttcc 240
   gcgggatctt gcagaggaga g 261
<210> 283
   <211> 270
   <212> DNA
   <213> Homo sapiens
<400> 283
   aggatttcgt gttccagttt aagggcatgt gctacttcac caacgggacg gagcgcgtgc 60
   gtcttgtgac cagatacatc tataaccgag aggagtacgc gcgcttcgac agcgacgtgg 120
   gggtgtaccg cgcggtgacg ccgcaggggc ggcctgatgc cgagtactgg aacagccaga 180
   aggaagtcct ggagaggacc cgggcggagt tggacacggt gtgcagacac aactacgagg 240
   tggggtaccg cgggatcctg cagaggagag 270
<210> 284
   <211> 270
   <212> DNA
   <213> Homo sapiens
<400> 284
   aggatttcgt gttccagttt aagggcatgt gctacttcac caacgggacg gagcgcgtgc 60
   gtcttgtgac cagatacatc tataaccgag aggagtacgc gcgcttcgac agcgacgtgg 120
   gggtgtaccg cgcggtgacg ccgcaggggc ggcctgatgc cgagaactgg aacagccaga 180
   aggaagtcct ggaggggacc cgggcggagt tggacacggt gtgcagacac aactacgagg 240
   tggcgttccg cgggatcttg cagaggagag 270
<210> 285
   <211> 242
   <212> DNA
   <213> Homo sapiens
<400> 285
   tgtaccagtt taagggcatg tgctacttca ccaacgggac ggagcgcgtg cgtcttgtaa 60
   ccagacacat ctataaccga gaggagtacg cgcgcttcga cagcgacgtg ggggtgtacc 120
   gggcggtgac gccgcagggg cggcctgttg ccgagtactg gaacagccag aaggaagtcc 180
   tggagggggc ccgggcggag ttggacacgg tgtgcagaca caactacgag gtggggtacc 240
   gc 242
<210> 286
   <211> 270
   <212> DNA
   <213> Homo sapiens
<400> 286
   aggatttcgt gttccagttt aagggcatgt gctacttcac caacgggacg gagcgcgtgc     60
   gtcttgtgac cagatacatc tataaccgag aggagtacgc gcgcttcgac agcgacgtgg    120
   gggtgtatcg ggcggtgacg ccgctggggc ggcctgatgc cgagtactgg aacagccaga    180
   aggaagtcct ggaggggacc cgggcggagt tggacacggt gtgcagacac aactacgagg    240
   tggcgttccg cgggatcttg cagaggagag                                     270
<210> 287
   <211> 266
   <212> PRT
   <213> Homo sapiens
<400> 287
   Met Val Cys Leu Lys Leu Pro Gly Gly Ser Cys Met Thr Ala Leu Thr
   1               5                   10                  15
   Val Thr Leu Met Val Leu Ser Ser Pro Leu Ala Leu Ala Gly Asp Thr
               20                  25                  30
   Arg Pro Arg Phe Leu Trp Gln Leu Lys Phe Glu Cys His Phe Phe Asn
           35                  40                  45
   Gly Thr Glu Arg Val Arg Leu Leu Glu Arg Cys Ile Tyr Asn Gln Glu
       50                  55                  60
   Glu Ser Val Arg Phe Asp Ser Asp Val Gly Glu Tyr Arg Ala Val Thr
   65                  70                  75                  80
   Glu Leu Gly Arg Pro Asp Ala Glu Tyr Trp Asn Ser Gln Lys Asp Leu
                   85                  90                  95
   Leu Glu Gln Arg Arg Ala Ala Val Asp Thr Tyr Cys Arg His Asn Tyr
               100                 105                 110
   Gly Val Gly Glu Ser Phe Thr Val Gln Arg Arg Val Glu Pro Lys Val
   115 120 125
   Thr Val Tyr Pro Ser Lys Thr Gln Pro Leu Gln His His Asn Leu Leu
   130 135 140
   Val Cys Ser Val Ser Gly Phe Tyr Pro Gly Ser Ile Glu Val Arg Trp
   145 150 155 160
   Phe Arg Asn Gly Gln Glu Glu Lys Ala Gly Val Val Ser Thr Gly Leu
   165 170 175
   Ile Gln Asn Gly Asp Trp Thr Phe Gln Thr Leu Val Met Leu Glu Thr
   180 185 190
   Val Pro Arg Ser Gly Glu Val Tyr Thr Cys Gln Val Glu His Pro Ser
   195 200 205
   Val Thr Ser Pro Leu Thr Val Glu Trp Arg Ala Arg Ser Glu Ser Ala
   210 215 220
   Gln Ser Lys Met Leu Ser Gly Val Gly Gly Phe Val Leu Gly Leu Leu
   225 230 235 240
   Phe Leu Gly Ala Gly Leu Phe Ile Tyr Phe Arg Asn Gln Lys Gly His
   245 250 255
   Ser Gly Leu Gln Pro Thr Gly Phe Leu Ser
   260 265
<210> 288
   <211> 237
   <212> PRT
   <213> Homo sapiens
<400> 288
   Gly Asp Thr Arg Pro Arg Phe Leu Trp Gln Leu Lys Phe Glu Cys His
   1               5                   10                  15
   Phe Phe Asn Gly Thr Glu Arg Val Arg Leu Leu Glu Arg Cys Ile Tyr
               20                  25                  30
   Asn Gln Glu Glu Ser Val Arg Phe Asp Ser Asp Val Gly Glu Tyr Arg
           35                  40                  45
   Ala Val Thr Glu Leu Gly Arg Pro Asp Ala Glu Tyr Trp Asn Ser Gln
       50                  55                  60
   Lys Asp Leu Leu Glu Gln Arg Arg Ala Ala Val Asp Thr Tyr Cys Arg
   65                  70                  75                  80
   His Asn Tyr Gly Ala Val Glu Ser Phe Thr Val Gln Arg Arg Val Glu
                   85                  90                  95
   Pro Lys Val Thr Val Tyr Pro Ser Lys Thr Gln Pro Leu Gln His His
               100                 105                 110
   Asn Leu Leu Val Cys Ser Val Ser Gly Phe Tyr Pro Gly Ser Ile Glu
           115                 120                 125
   Val Arg Trp Phe Arg Asn Gly Gln Glu Glu Lys Ala Gly Val Val Ser
       130                 135                 140
   Thr Gly Leu Ile Gln Asn Gly Asp Trp Thr Phe Gln Thr Leu Val Met
   145                 150                 155                 160
   Leu Glu Thr Val Pro Arg Ser Gly Glu Val Tyr Thr Cys Gln Val Glu
                   165                 170                 175
   His Pro Ser Val Thr Ser Pro Leu Thr Val Glu Trp Arg Ala Arg Ser
               180                 185                 190
   Glu Ser Ala Gln Ser Lys Met Leu Ser Gly Val Gly Gly Phe Val Leu
           195                 200                 205
   Gly Leu Leu Phe Leu Gly Ala Gly Leu Phe Ile Tyr Phe Arg Asn Gln
       210                 215                 220
   Lys Gly His Ser Gly Leu Gln Pro Thr Gly Phe Leu Ser
   225                 230                 235
<210> 289
   <211> 81
   <212> PRT
   <213> Homo sapiens
<400> 289
   Arg Phe Leu Trp Gln Leu Lys Phe Glu Cys His Phe Phe Asn Gly Thr
   1               5                   10                  15
   Glu Arg Val Arg Leu Leu Glu Arg Cys Ile Tyr Asn Gln Glu Glu Ser
               20                  25                  30
   Val Arg Phe Asp Ser Asp Val Gly Glu Tyr Arg Ala Val Thr Glu Leu
           35                  40                  45
   Gly Arg Pro Asp Ala Glu Tyr Trp Asn Ser Gln Lys Asp Leu Leu Glu
       50                  55                  60
   Gln Arg Arg Ala Ala Val Asp Thr Tyr Cys Arg His Asn Tyr Gly Ala
   65                  70                  75                  80
   Val

<210> 290
   <211> 266
   <212> PRT
   <213> Homo sapiens
<400> 290
<210> 291
   <211> 225
   <212> PRT
   <213> Homo sapiens
<400> 291
   Gly Asp Thr Arg Pro Arg Phe Leu Trp Gln Leu Lys Phe Glu Cys His
   1               5                   10                  15
   Phe Phe Asn Gly Thr Glu Arg Val Arg Leu Leu Glu Arg Cys Ile Tyr
               20                  25                  30
   Asn Gln Glu Glu Ser Val Arg Phe Asp Ser Asp Val Gly Glu Tyr Arg
           35                  40                  45
   Ala Val Thr Glu Leu Gly Arg Pro Asp Ala Glu Tyr Trp Asn Ser Gln
       50                  55                  60
   Lys Asp Leu Leu Glu Gln Arg Arg Ala Ala Val Asp Asn Tyr Cys Arg
   65                  70                  75                  80
   His Asn Tyr Gly Val Val Glu Ser Phe Thr Val Gln Arg Arg Val Glu
                   85                  90                  95
   Pro Lys Val Thr Val Tyr Pro Ser Lys Thr Gln Pro Leu Gln His His
               100                 105                 110
   Asn Leu Leu Val Cys Ser Val Ser Gly Phe Tyr Pro Gly Ser Ile Glu
           115                 120                 125
   Val Arg Trp Phe Arg Asn Gly Gln Glu Glu Lys Ala Gly Val Val Ser
       130                 135                 140
   Thr Gly Leu Ile Gln Asn Gly Asp Trp Thr Phe Gln Thr Leu Val Met
   145                 150                 155                 160
   Leu Glu Thr Val Pro Arg Ser Gly Glu Val Tyr Thr Cys Gln Val Glu
                   165                 170                 175
   His Pro Ser Val Thr Ser Pro Leu Thr Val Glu Trp Arg Ala Arg Ser
               180                 185                 190
   Glu Ser Ala Gln Ser Lys Met Leu Ser Gly Val Gly Gly Phe Val Leu
           195                 200                 205
   Gly Leu Leu Phe Leu Gly Ala Gly Leu Phe Ile Tyr Phe Arg Asn Gln
       210                 215                 220
   Lys
   225
<210> 292
   <211> 89
   <212> PRT
   <213> Homo sapiens
<400> 292
   Arg Phe Leu Trp Gln Leu Lys Phe Glu Cys His Phe Phe Asn Gly Thr
   1               5                   10                  15
   Glu Arg Val Arg Leu Leu Glu Arg Cys Ile Tyr Asn Gln Glu Glu Ser
               20                  25                  30
   Val Arg Phe Asp Ser Asp Val Arg Glu Tyr Arg Ala Val Thr Glu Leu
           35                  40                  45
   Gly Arg Pro Asp Ala Glu Tyr Trp Asn Ser Gln Lys Asp Leu Leu Glu
       50                  55                  60
   Gln Arg Arg Ala Ala Val Asp Thr Tyr Cys Arg His Asn Tyr Gly Val
   65                  70                  75                  80
   Gly Glu Ser Phe Thr Val Gln Arg Arg
                   85
<210> 293
   <211> 89
   <212> PRT
   <213> Homo sapiens
<400> 293
   Arg Phe Leu Trp Gln Leu Lys Phe Glu Cys His Phe Phe Asn Gly Thr
   1               5                   10                  15
   Glu Arg Val Arg Leu Leu Glu Arg Cys Ile Tyr Asn Gln Glu Glu Ser
               20                  25                  30
   Val Arg Phe Asp Ser Asp Val Gly Glu Tyr Arg Ala Val Thr Glu Leu
           35                  40                  45
   Gly Arg Pro Asp Ala Glu Tyr Trp Asn Ser Gln Lys Asp Leu Leu Glu
       50                  55                  60
   Gln Ala Arg Ala Ala Val Asp Thr Tyr Cys Arg His Asn Tyr Gly Val
   65                  70                  75                  80
   Val Glu Ser Phe Thr Val Gln Arg Arg
                   85
<210> 294
   <211> 89
   <212> PRT
   <213> Homo sapiens
<400> 294
   Arg Phe Leu Trp Glu Leu Lys Phe Glu Cys His Phe Phe Asn Gly Thr
   1               5                   10                  15
   Glu Arg Val Arg Leu Leu Glu Arg Cys Ile Tyr Asn Gln Glu Glu Ser
               20                  25                  30
   Val Arg Phe Asp Ser Asp Val Gly Glu Tyr Arg Ala Val Thr Glu Leu
           35                  40                  45
   Gly Arg Pro Asp Ala Glu Tyr Trp Asn Ser Gln Lys Asp Leu Leu Glu
       50                  55                  60
   Gln Arg Arg Ala Ala Val Asp Thr Tyr Cys Arg His Asn Tyr Gly Val
   65                  70                  75                  80
   Gly Glu Ser Phe Thr Val Gln Arg Arg
                   85
<210> 295
   <211> 89
   <212> PRT
   <213> Homo sapiens
<400> 295
   Arg Phe Leu Trp Gln Leu Lys Phe Glu Cys His Phe Phe Asn Gly Thr
   1               5                   10                  15
   Glu Arg Val Arg Leu Leu Glu Arg Cys Ile Tyr Asn Gln Glu Glu Tyr
               20                  25                  30
   Val Arg Phe Asp Ser Asp Val Gly Glu Tyr Arg Ala Val Thr Glu Leu
           35                  40                  45
   Gly Arg Pro Asp Ala Glu Tyr Trp Asn Ser Gln Lys Asp Leu Leu Glu
       50                  55                  60
   Gln Arg Arg Ala Ala Val Asp Thr Tyr Cys Arg His Asn Tyr Gly Val
   65                  70                  75                  80
   Gly Glu Ser Phe Thr Val Gln Arg Arg
                   85
<210> 296
   <211> 237
   <212> PRT
   <213> Homo sapiens
<400> 296
   Gly Asp Thr Arg Pro Arg Phe Leu Glu Tyr Ser Thr Ser Glu Cys His
   1 5 10 15
   Phe Phe Asn Gly Thr Glu Arg Val Arg Tyr Leu Asp Arg Tyr Phe His
   20 25 30
   Asn Gln Glu Glu Asn Val Arg Phe Asp Ser Asp Val Gly Glu Phe Arg
   35 40 45
   Ala Val Thr Glu Leu Gly Arg Pro Asp Ala Glu Tyr Trp Asn Ser Gln
   50 55 60
   Lys Asp Leu Leu Glu Gln Lys Arg Gly Arg Val Asp Asn Tyr Cys Arg
   65 70 75 80
   His Asn Tyr Gly Val Val Glu Ser Phe Thr Val Gln Arg Arg Val His
   85 90 95
   Pro Lys Val Thr Val Tyr Pro Ser Lys Thr Gln Pro Leu Gln His His
   100 105 110
   Asn Leu Leu Val Cys Ser Val Ser Gly Phe Tyr Pro Gly Ser Ile Glu
   115 120 125
   Val Arg Trp Phe Arg Asn Gly Gln Glu Glu Lys Thr Gly Val Val Ser
   130 135 140
   Thr Gly Leu Ile His Asn Gly Asp Trp Thr Phe Gln Thr Leu Val Met
   145 150 155 160
   Leu Glu Thr Val Pro Arg Ser Gly Glu Val Tyr Thr Cys Gln Val Glu
   165 170 175
   His Pro Ser Val Thr Ser Pro Leu Thr Val Glu Trp Arg Ala Arg Ser
   180 185 190
   Glu Ser Ala Gln Ser Lys Met Leu Ser Gly Val Gly Gly Phe Val Leu
   195 200 205
   Gly Leu Leu Phe Leu Gly Ala Gly Leu Phe Ile Tyr Phe Arg Asn Gln
   210 215 220
   Lys Gly His Ser Gly Leu Gln Pro Arg Gly Phe Leu Ser
   225 230 235
<210> 297
   <211> 87
   <212> PRT
   <213> Homo sapiens
<400> 297
   Arg Phe Leu Glu Tyr Ser Thr Ser Glu Cys His Phe Phe Asn Gly Thr
   1               5                   10                  15
   Glu Arg Val Arg Tyr Leu Asp Arg Tyr Phe His Asn Gln Glu Glu Asn
               20                  25                  30
   Val Arg Phe Asp Ser Asp Val Gly Glu Phe Arg Ala Val Thr Glu Leu
           35                  40                  45
   Gly Arg Pro Asp Ala Glu Tyr Trp Asn Ser Gln Lys Asp Leu Leu Glu
       50                  55                  60
   Gln Lys Arg Gly Arg Val Asp Asn Tyr Cys Arg His Asn Tyr Gly Val
   65                  70                  75                  80
   Val Glu Ser Phe Thr Val Gln
                   85
<210> 298
   <211> 237
   <212> PRT
   <213> Homo sapiens
<400> 298
   Gly Asp Thr Arg Pro Arg Phe Leu Glu Tyr Ser Thr Ser Glu Cys His
   1               5                   10                  15
   Phe Phe Asn Gly Thr Glu Arg Val Arg Phe Leu Glu Arg Tyr Phe His
               20                  25                  30
   Asn Gln Glu Glu Asn Val Arg Phe Asp Ser Asp Val Gly Glu Tyr Arg
           35                  40                  45
   Ala Val Thr Glu Leu Gly Arg Pro Asp Ala Glu Tyr Trp Asn Ser Gln
       50                  55                  60
   Lys Asp Leu Leu Glu Gln Lys Arg Gly Arg Val Asp Asn Tyr Cys Arg
   65                  70                  75                  80
   His Asn Tyr Gly Val Gly Glu Ser Phe Thr Val Gln Arg Arg Val His
                   85                  90                  95
   Pro Lys Val Thr Val Tyr Pro Ser Lys Thr Gln Pro Leu Gln His His
               100                 105                 110
   Asn Leu Leu Val Cys Ser Val Ser Gly Phe Tyr Pro Gly Ser Ile Glu
           115                 120                 125
   Val Arg Trp Phe Arg Asn Gly Gln Glu Glu Lys Thr Gly Val Val Ser
       130                 135                 140
   Thr Gly Leu Ile His Asn Gly Asp Trp Thr Phe Gln Thr Leu Val Met
   145                 150                 155                 160
   Leu Glu Thr Val Pro Arg Ser Gly Glu Val Tyr Thr Cys Gln Val Glu
                   165                 170                 175
   His Pro Ser Val Thr Ser Pro Leu Thr Val Glu Trp Arg Ala Arg Ser
               180                 185                 190
   Glu Ser Ala Gln Ser Lys Met Leu Ser Gly Val Gly Gly Phe Val Leu
           195                 200                 205
   Gly Leu Leu Phe Leu Gly Ala Gly Leu Phe Ile Tyr Phe Arg Asn Gln
       210                 215                 220
   Lys Gly His Ser Gly Leu Gln Pro Arg Gly Phe Leu Ser
   225                 230                 235
<210> 299
   <211> 100
   <212> PRT
   <213> Homo sapiens
<400> 299
   Gly Asp Thr Arg Pro Arg Phe Leu Glu Tyr Ser Thr Ser Glu Cys His
   1               5                   10                  15
   Phe Phe Asn Gly Thr Glu Arg Val Arg Phe Leu Glu Arg Tyr Phe His
               20                  25                  30
   Asn Gln Glu Glu Asn Val Arg Phe Asp Ser Asp Val Gly Glu Tyr Arg
           35                  40                  45
   Ala Val Thr Glu Leu Gly Arg Pro Asp Ala Glu Tyr Trp Asn Ser Gln
       50                  55                  60
   Lys Asp Leu Leu Glu Gln Lys Arg Gly Arg Val Asp Asn Tyr Cys Arg
   65                  70                  75                  80
   His Asn Tyr Gly Val Gly Glu Ser Phe Thr Val Gln Arg Arg Val His
                   85                  90                  95
   Pro Lys Val Thr
               100
<210> 300
   <211> 85
   <212> PRT
   <213> Homo sapiens
<400> 300
   Tyr Ser Thr Ser Glu Cys His Phe Phe Asn Gly Thr Glu Arg Val Arg
   1               5                   10                  15
   Phe Leu Glu Arg Tyr Phe His Asn Gln Glu Glu Asn Val Arg Phe Asp
               20                  25                  30
   Ser Asp Val Gly Glu Tyr Arg Ala Val Thr Glu Leu Gly Arg Pro Asp
           35                  40                  45
   Ala Glu Tyr Trp Asn Ser Gln Lys Asp Leu Leu Glu Gln Lys Arg Gly
       50                  55                  60
   Arg Val Asp Asn Tyr Cys Arg His Asn Tyr Gly Val Val Glu Ser Phe
   65                  70                  75                  80
   Thr Val Gln Arg Arg
                   85
<210> 301
   <211> 89
   <212> PRT
   <213> Homo sapiens
<400> 301
   Arg Phe Leu Glu Tyr Ser Thr Ser Glu Cys His Phe Phe Asn Gly Thr
   1               5                   10                  15
   Glu Arg Val Arg Tyr Leu Asp Arg Tyr Phe His Asn Gln Glu Glu Ser
               20                  25                  30
   Val Arg Phe Asp Ser Asp Val Gly Glu Phe Arg Ala Val Thr Glu Leu
           35                  40                  45
   Gly Arg Pro Asp Ala Glu Tyr Trp Asn Ser Gln Lys Asp Leu Leu Glu
       50                  55                  60
   Gln Lys Arg Gly Arg Val Asp Asn Tyr Cys Arg His Asn Tyr Gly Val
   65                  70                  75                  80
   Val Glu Ser Phe Thr Val Gln Arg Arg
                   85
<210> 302
   <211> 89
   <212> PRT
   <213> Homo sapiens
<400> 302
   Arg Phe Leu Glu Tyr Ser Thr Ser Glu Cys His Phe Phe Asn Gly Thr
   1               5                   10                  15
   Glu Arg Val Arg Tyr Leu Asp Arg Tyr Phe His Asn Gln Glu Glu Asn
               20                  25                  30
   Val Arg Phe Asp Ser Asp Val Gly Glu Phe Arg Ala Val Thr Glu Leu
           35                  40                  45
   Gly Arg Pro Asp Ala Glu Tyr Trp Asn Ser Gln Lys Asp Leu Leu Glu
       50                  55                  60
   Gln Lys Arg Gly Arg Val Asp Asn Tyr Cys Arg His Asn Tyr Gly Val
   65                  70                  75                  80
   Gly Glu Ser Phe Thr Val Gln Arg Arg
                   85
<210> 303
   <211> 89
   <212> PRT
   <213> Homo sapiens
<400> 303
   Arg Phe Leu Glu Tyr Ser Thr Ser Glu Cys His Phe Phe Asn Gly Thr
   1               5                   10                  15
   Glu Arg Val Arg Tyr Leu Asp Arg Tyr Phe His Asn Gln Glu Glu Asn
               20                  25                  30
   Val Arg Phe Asp Ser Asp Val Gly Glu Phe Arg Ala Val Thr Glu Leu
           35                  40                  45
   Gly Arg Pro Asp Ala Glu Tyr Trp Asn Ser Gln Lys Asp Leu Leu Glu
       50                  55                  60
   Gln Lys Arg Gly Arg Val Asp Asn Tyr Cys Arg His Asn Tyr Gly Val
   65                  70                  75                  80
   Gly Glu Ser Phe Thr Val Gln Arg Arg
                   85
<210> 304
   <211> 86
   <212> PRT
   <213> Homo sapiens
<400> 304
   Phe Leu Glu Tyr Ser Thr Ser Glu Cys His Phe Phe Asn Gly Thr Glu
   1               5                   10                  15
   Arg Val Arg Tyr Leu Asp Arg Tyr Phe His Asn Gln Glu Glu Asn Val
               20                  25                  30
   Arg Phe Asp Ser Asp Val Gly Glu Tyr Arg Ala Val Thr Glu Leu Gly
           35                  40                  45
   Arg Pro Asp Ala Glu Tyr Trp Asn Ser Gln Lys Asp Leu Leu Glu Gln
       50                  55                  60
   Lys Arg Gly Arg Val Asp Asn Tyr Cys Arg His Asn Tyr Gly Val Val
   65                  70                  75                  80
   Glu Ser Phe Thr Val Gln
                   85
<210> 305
   <211> 102
   <212> PRT
   <213> Homo sapiens
<400> 305
   Gly Asp Thr Arg Pro Arg Phe Leu Glu Tyr Ser Thr Ser Glu Cys His
   1               5                   10                  15
   Phe Phe Asn Gly Thr Glu Arg Val Arg Phe Leu Asp Arg Tyr Phe His
               20                  25                  30
   Asn Gln Glu Glu Asn Val Arg Phe Asp Ser Asp Val Gly Glu Phe Arg
           35                  40                  45
   Ala Val Thr Glu Leu Gly Arg Pro Asp Ala Glu Tyr Trp Asn Ser Gln
       50                  55                  60
   Lys Asp Leu Leu Glu Gln Lys Arg Gly Arg Val Asp Asn Tyr Cys Arg
   65                  70                  75                  80
   His Asn Tyr Gly Val Val Glu Ser Phe Thr Val Gln Arg Arg Val His
                   85                  90                  95
   Pro Lys Val Thr Val Tyr
               100
<210> 306
   <211> 102
   <212> PRT
   <213> Homo sapiens
<400> 306
   Gly Asp Thr Arg Pro Arg Phe Leu Glu Tyr Ser Thr Ser Glu Cys His
   1               5                   10                  15
   Phe Phe Asn Gly Thr Glu Arg Val Arg Tyr Leu Asp Arg Tyr Phe His
               20                  25                  30
   Asn Gln Glu Glu Asn Val Arg Phe Asp Ser Asp Val Gly Glu Phe Arg
           35                  40                  45
   Ala Val Thr Glu Leu Gly Arg Pro Asp Glu Glu Tyr Trp Asn Ser Gln
       50                  55                  60
   Lys Asp Leu Leu Glu Gln Lys Arg Gly Arg Val Asp Asn Tyr Cys Arg
   65                  70                  75                  80
   His Asn Tyr Gly Val Val Glu Ser Phe Thr Val Gln Arg Arg Val His
                   85                  90                  95
   Pro Lys Val Thr Val Tyr
   100

<210> 307
   <211> 86
   <212> PRT
   <213> Homo sapiens
<400> 307
   Leu Glu Tyr Ser Thr Ser Glu Cys His Phe Phe Asn Gly Thr Glu Arg
   1               5                   10                  15
   Val Arg Tyr Leu Asp Arg Tyr Phe His Asn Arg Glu Glu Asn Val Arg
               20                  25                  30
   Phe Asp Ser Asp Val Gly Glu Phe Arg Ala Val Thr Glu Leu Gly Arg
           35                  40                  45
   Pro Asp Ala Glu Tyr Trp Asn Ser Gln Lys Asp Leu Leu Glu Gln Lys
       50                  55                  60
   Arg Gly Arg Val Asp Asn Tyr Cys Arg His Asn Tyr Gly Val Gly Glu
   65                  70                  75                  80
   Ser Phe Thr Val Gln Arg
                   85
<210> 308
   <211> 237
   <212> PRT
   <213> Homo sapiens
<400> 308
   Gly Asp Thr Arg Pro Arg Phe Leu Glu Tyr Ser Thr Ser Glu Cys His
   1               5                   10                  15
   Phe Phe Asn Gly Thr Glu Arg Val Arg Tyr Leu Asp Arg Tyr Phe His
               20                  25                  30
   Asn Gln Glu Glu Asn Val Arg Phe Asp Ser Asp Val Gly Glu Phe Arg
           35                  40                  45
   Ala Val Thr Glu Leu Gly Arg Pro Ala Ala Glu His Trp Asn Ser Gln
       50                  55                  60
   Lys Asp Leu Leu Glu Gln Lys Arg Gly Arg Val Asp Asn Tyr Cys Arg
   65                  70                  75                  80
   His Asn Tyr Gly Val Val Glu Ser Phe Thr Val Gln Arg Arg Val His
                   85                  90                  95
   Pro Lys Val Thr Val Tyr Pro Ser Lys Thr Gln Pro Leu Gln His His
               100                 105                 110
   Asn Leu Leu Val Cys Ser Val Ser Gly Phe Tyr Pro Gly Ser Ile Glu
           115                 120                 125
   Val Arg Trp Phe Arg Asn Gly Gln Glu Glu Lys Thr Gly Val Val Ser
       130                 135                 140
   Thr Gly Leu Ile His Asn Gly Asp Trp Thr Phe Gln Thr Leu Val Met
   145                 150                 155                 160
   Leu Glu Thr Val Pro Arg Ser Gly Glu Val Tyr Thr Cys Gln Val Glu
                   165                 170                 175
   His Pro Ser Val Thr Ser Pro Leu Thr Val Glu Trp Arg Ala Arg Ser
               180                 185                 190
   Glu Ser Ala Gln Ser Lys Met Leu Ser Gly Val Gly Gly Phe Val Leu
           195                 200                 205
   Gly Leu Leu Phe Leu Gly Ala Gly Leu Phe Ile Tyr Phe Arg Asn Gln
       210                 215                 220
   Lys Gly His Ser Gly Leu Gln Pro Arg Gly Phe Leu Ser
   225                 230                 235
<210> 309
   <211> 89
   <212> PRT
   <213> Homo sapiens
<400> 309
   Arg Phe Leu Glu Tyr Ser Thr Ser Glu Cys His Phe Phe Asn Gly Thr
   1 5 10 15
   Glu Arg Val Arg Tyr Leu Asp Arg Tyr Phe His Asn Gln Glu Glu Asn
   20 25 30
   Val Arg Phe Asp Ser Asp Val Gly Glu Phe Arg Ala Val Thr Glu Leu
   35 40 45
   Gly Arg Pro Asp Ala Glu Tyr Trp Asn Ser Gln Lys Asp Leu Leu Glu
   50 55 60
   Gln Lys Arg Gly Gln Val Asp Asn Tyr Cys Arg His Asn Tyr Gly Val
   65 70 75 80
   Val Glu Ser Phe Thr Val Gln Arg Arg
   85
<210> 310
   <211> 80
   <212> PRT
   <213> Homo sapiens
<400> 310
   Leu Glu Tyr Ser Thr Ser Glu Cys His Phe Phe Asn Gly Thr Glu Arg
   1 5 10 15
   Val Arg Tyr Leu Asp Arg Tyr Phe His Asn Gln Glu Glu Asn Val Arg
   20 25 30
   Phe Asp Ser Asp Val Gly Glu Phe Arg Ala Val Thr Glu Leu Gly Arg
   35 40 45
   Pro Ser Ala Glu Tyr Trp Asn Ser Gln Lys Asp Leu Leu Glu Gln Lys
   50 55 60
   Arg Gly Arg Val Asp Asn Tyr Cys Arg His Asn Tyr Gly Val Val Glu
   65 70 75 80
<210> 311
   <211> 89
   <212> PRT
   <213> Homo sapiens
<400> 311
   Arg Phe Leu Glu Tyr Ser Thr Ser Glu Cys His Phe Phe Asn Gly Thr
   1               5                   10                  15
   Glu Arg Val Arg Tyr Leu Asp Arg Tyr Phe His Asn Gln Glu Glu Asn
               20                  25                  30
   Val Arg Phe Asp Ser Asp Val Gly Glu Phe Arg Ala Val Thr Glu Leu
           35                  40                  45
   Gly Arg Pro Asp Ala Glu Ser Trp Asn Ser Gln Lys Asp Leu Leu Glu
       50                  55                  60
   Gln Lys Arg Gly Arg Val Asp Asn Tyr Cys Arg His Asn Tyr Gly Val
   65                  70                  75                  80
   Val Glu Ser Phe Thr Val Gln Arg Arg
                   85
<210> 312
   <211> 89
   <212> PRT
   <213> Homo sapiens
<400> 312
   Arg Phe Leu Glu Tyr Ser Thr Ser Glu Cys His Phe Phe Asn Gly Thr
   1               5                   10                  15
   Glu Arg Val Arg Tyr Leu Asp Arg Tyr Phe His Asn Gln Glu Glu Asn
               20                  25                  30
   Val Arg Phe Asp Ser Asp Val Gly Glu Phe Arg Ala Val Thr Glu Leu
           35                  40                  45
   Gly Arg Pro Asp Ala Glu Tyr Trp Asn Ser Gln Lys Asp Leu Leu Glu
       50                  55                  60
   Gln Lys Arg Gly Arg Val Asp Thr Tyr Cys Arg His Asn Tyr Gly Val
   65                  70                  75                  80
   Gly Glu Ser Phe Thr Val Gln Arg Arg
                   85
<210> 313
   <211> 89
   <212> PRT
   <213> Homo sapiens
<400> 313
   Arg Phe Leu Glu Tyr Ser Thr Ser Glu Cys His Phe Phe Asn Gly Thr
   1               5                   10                  15
   Glu Arg Val Arg Tyr Leu Asp Arg Tyr Phe His Asn Gln Glu Glu Asn
               20                  25                  30
   Val Arg Phe Asp Ser Asp Val Gly Glu Phe Arg Ala Val Thr Glu Leu
           35                  40                  45
   Gly Arg Pro Asp Ala Glu Tyr Trp Asn Ser Gln Lys Asp Leu Leu Glu
       50                  55                  60
   Gln Lys Arg Gly Arg Val Asp Thr Tyr Cys Arg His Asn Tyr Gly Val
   65                  70                  75                  80
   Val Glu Ser Phe Thr Val Gln Arg Arg
                   85
<210> 314
   <211> 81
   <212> PRT
   <213> Homo sapiens
<400> 314
   Arg Phe Leu Glu Tyr Ser Thr Ser Glu Cys His Phe Phe Asn Gly Thr
   1               5                   10                  15
   Glu Arg Val Arg Tyr Leu Asp Arg Tyr Phe His Asn Gln Glu Glu Asn
               20                  25                  30
   Val Arg Phe Asp Ser Asp Val Gly Glu Phe Trp Ala Val Thr Glu Leu
           35                  40                  45
   Gly Arg Pro Asp Ala Glu Tyr Trp Asn Ser Gln Lys Asp Leu Leu Glu
       50                  55                  60
   Gln Lys Arg Gly Arg Val Asp Asn Tyr Cys Arg His Asn Tyr Gly Val
   65                  70                  75                  80
   Val
<210> 315
   <211> 89
   <212> PRT
   <213> Homo sapiens
<400> 315
   Arg Phe Leu Glu Tyr Ser Thr Ser Glu Cys His Phe Phe Asn Gly Thr
   1               5                   10                  15
   Glu Arg Val Arg Phe Leu Asp Arg Tyr Phe Tyr Asn Gln Glu Glu Tyr
               20                  25                  30
   Val Arg Phe Asp Ser Asp Val Gly Glu Tyr Arg Ala Val Arg Glu Leu
           35                  40                  45
   Gly Arg Pro Asp Ala Glu Tyr Trp Asn Ser Gln Lys Asp Leu Leu Glu
       50                  55                  60
   Gln Lys Arg Gly Gln Val Asp Asn Tyr Cys Arg His Asn Tyr Gly Val
   65                  70                  75                  80
   Gly Glu Ser Phe Thr Val Gln Arg Arg
                   85
<210> 316
   <211> 89
   <212> PRT
   <213> Homo sapiens
<400> 316
   Arg Phe Leu Glu Tyr Ser Thr Ser Glu Cys His Phe Phe Asn Gly Thr
   1               5                   10                  15
   Glu Arg Val Arg Tyr Leu Asp Arg Tyr Phe His Asn Gln Glu Glu Asn
               20                  25                  30
   Val Arg Phe Asp Ser Asp Val Arg Glu Phe Arg Ala Val Thr Glu Leu
           35                  40                  45
   Gly Arg Pro Asp Ala Glu Tyr Trp Asn Ser Gln Lys Asp Leu Leu Glu
       50                  55                  60
   Gln Lys Arg Gly Arg Val Asp Asn Tyr Cys Arg His Asn Tyr Gly Val
   65                  70                  75                  80
   Val Glu Ser Phe Thr Val Gln Arg Arg
                   85
<210> 317
   <211> 89
   <212> PRT
   <213> Homo sapiens
<400> 317
   Arg Phe Leu Glu Tyr Ser Thr Ser Glu Cys His Phe Phe Asn Gly Thr
   1               5                   10                  15
   Glu Arg Val Arg Tyr Leu Asp Arg Tyr Phe His Asn Gln Glu Glu Asn
               20                  25                  30
   Val Arg Phe Asp Ser Asp Val Gly Glu Phe Arg Ala Val Thr Glu Leu
           35                  40                  45
   Gly Arg Pro Asp Ala Glu Tyr Trp Asn Ser Gln Lys Asp Ile Leu Glu
       50                  55                  60
   Gln Lys Arg Gly Arg Val Asp Asn Tyr Cys Arg His Asn Tyr Gly Val
   65                  70                  75                  80
   Val Glu Ser Phe Thr Val Gln Arg Arg
                   85
<210> 318
   <211> 88
   <212> PRT
   <213> Homo sapiens
<400> 318
   Arg Phe Leu Glu Tyr Ser Thr Ser Glu Cys His Phe Phe Asn Gly Thr
   1               5                   10                  15
   Glu Arg Val Arg Tyr Leu Asp Arg Tyr Phe His Asn Gln Glu Glu Asn
               20                  25                  30
   Val Arg Phe Asp Ser Asp Val Gly Glu Phe Arg Ala Val Thr Glu Leu
           35                  40                  45
   Gly Arg Pro Asp Ala Glu Tyr Trp Asn Ser Gln Lys Asp Leu Leu Glu
       50                  55                  60
   Gln Lys Arg Gly Arg Val Asp Asn Tyr Cys Arg His Asn Tyr Gly Ala
   65                  70                  75                  80
   Val Glu Ser Phe Thr Val Gln Arg
                   85
<210> 319
   <211> 266
   <212> PRT
   <213> Homo sapiens
<400> 319
<210> 320
   <211> 89
   <212> PRT
   <213> Homo sapiens
<400> 320
   Arg Phe Leu Glu Gln Val Lys His Glu Cys His Phe Phe Asn Gly Thr
   1               5                   10                  15
   Glu Arg Val Arg Phe Leu Asp Arg Tyr Phe Tyr His Gln Glu Glu Tyr
               20                  25                  30
   Val Arg Phe Asp Ser Asp Val Gly Glu Tyr Arg Ala Val Thr Glu Leu
           35                  40                  45
   Gly Arg Pro Asp Ala Glu Tyr Trp Asn Ser Gln Lys Asp Leu Leu Glu
       50                  55                  60
   Gln Lys Arg Ala Ala Val Asp Thr Tyr Cys Arg His Asn Tyr Gly Val
   65                  70                  75                  80
   Gly Glu Ser Phe Thr Val Gln Arg Arg
                   85
<210> 321
   <211> 264
   <212> PRT
   <213> Homo sapiens
<400> 321
   Cys Leu Lys Phe Pro Gly Gly Ser Cys Met Ala Ala Leu Thr Val Thr
   1               5                   10                  15
   Leu Met Val Leu Ser Ser Pro Leu Ala Leu Ala Gly Asp Thr Arg Pro
               20                  25                  30
   Arg Phe Leu Glu Gln Val Lys His Glu Cys His Phe Phe Asn Gly Thr
           35                  40                  45
   Glu Arg Val Arg Phe Leu Asp Arg Tyr Phe Tyr His Gln Glu Glu Tyr
       50                  55                  60
   Val Arg Phe Asp Ser Asp Val Gly Glu Tyr Arg Ala Val Thr Glu Leu
   65                  70                  75                  80
   Gly Arg Pro Asp Ala Glu Tyr Trp Asn Ser Gln Lys Asp Ile Leu Glu
                   85                  90                  95
   Asp Glu Arg Ala Ala Val Asp Thr Tyr Cys Arg His Asn Tyr Gly Val
               100                 105                 110
   Val Glu Ser Phe Thr Val Gln Arg Arg Val Tyr Pro Glu Val Thr Val
           115                 120                 125
   Tyr Pro Ala Lys Thr Gln Pro Leu Gln His His Asn Leu Leu Val Cys
       130                 135                 140
   Ser Val Asn Gly Phe Tyr Pro Gly Ser Ile Glu Val Arg Trp Phe Arg
   145                 150                 155                 160
   Asn Gly Gln Glu Glu Lys Thr Gly Val Val Ser Thr Gly Leu Ile Gln
                   165                 170                 175
   Asn Gly Asp Trp Thr Phe Gln Thr Leu Val Met Leu Glu Thr Val Pro
               180                 185                 190
   Arg Ser Gly Glu Val Tyr Thr Cys Gln Val Glu His Pro Ser Leu Thr
           195                 200                 205
   Ser Pro Leu Thr Val Glu Trp Arg Ala Arg Ser Glu Ser Ala Gln Ser
       210                 215                 220
   Lys Met Leu Ser Gly Val Gly Gly Phe Val Leu Gly Leu Leu Phe Leu
   225                 230                 235                 240
   Gly Ala Gly Leu Phe Ile Tyr Phe Arg Asn Gln Lys Gly His Ser Gly
                   245                 250                 255
   Leu Gln Pro Thr Gly Phe Leu Ser
               260
<210> 322
   <211> 237
   <212> PRT
   <213> Homo sapiens
<400> 322
   Gly Asp Thr Arg Pro Arg Phe Leu Glu Gln Val Lys His Glu Cys His
   1               5                   10                  15
   Phe Phe Asn Gly Thr Glu Arg Val Arg Phe Leu Asp Arg Tyr Phe Tyr
               20                  25                  30
   His Gln Glu Glu Tyr Val Arg Phe Asp Ser Asp Val Gly Glu Tyr Arg
           35                  40                  45
   Ala Val Thr Glu Leu Gly Arg Pro Asp Ala Glu Tyr Trp Asn Ser Gln
       50                  55                  60
   Lys Asp Leu Leu Glu Gln Arg Arg Ala Glu Val Asp Thr Tyr Cys Arg
   65                  70                  75                  80
   His Asn Tyr Gly Val Val Glu Ser Phe Thr Val Gln Arg Arg Val Tyr
                   85                  90                  95
   Pro Glu Val Thr Val Tyr Pro Ala Lys Thr Gln Pro Leu Gln His His
               100                 105                 110
   Asn Leu Leu Val Cys Ser Val Asn Gly Phe Tyr Pro Gly Ser Ile Glu
           115                 120                 125
   Val Arg Trp Phe Arg Asn Gly Gln Glu Glu Lys Thr Gly Val Val Ser
       130                 135                 140
   Thr Gly Leu Ile Gln Asn Gly Asp Trp Thr Phe Gln Thr Leu Val Met
   145                 150                 155                 160
   Leu Glu Thr Val Pro Arg Ser Gly Glu Val Tyr Thr Cys Gln Val Glu
                   165                 170                 175
   His Pro Ser Leu Thr Ser Pro Leu Thr Val Glu Trp Arg Ala Arg Ser
               180                 185                 190
   Glu Ser Ala Gln Ser Lys Met Leu Ser Gly Val Gly Gly Phe Val Leu
           195                 200                 205
   Gly Leu Leu Phe Leu Gly Ala Gly Leu Phe Ile Tyr Phe Arg Asn Gln
       210                 215                 220
   Lys Gly His Ser Gly Leu Gln Pro Thr Gly Phe Leu Ser
   225                 230                 235
<210> 323
   <211> 89
   <212> PRT
   <213> Homo sapiens
<400> 323
   Arg Phe Leu Glu Gln Val Lys His Glu Cys His Phe Phe Asn Gly Thr
   1               5                   10                  15
   Glu Arg Val Arg Phe Leu Asp Arg Tyr Phe Tyr His Gln Glu Glu Tyr
               20                  25                  30
   Val Arg Phe Asp Ser Asp Val Gly Glu Tyr Arg Ala Val Thr Glu Leu
           35                  40                  45
   Gly Arg Pro Asp Ala Glu Tyr Trp Asn Ser Gln Lys Asp Leu Leu Glu
       50                  55                  60
   Gln Arg Arg Ala Glu Val Asp Thr Tyr Cys Arg His Asn Tyr Gly Val
   65                  70                  75                  80
   Val Glu Ser Phe Thr Val Gln Arg Arg
                   85
<210> 324
   <211> 266
   <212> PRT
   <213> Homo sapiens

<400> 324
<210> 325
   <211> 94
   <212> PRT
   <213> Homo sapiens
<400> 325
   Gly Asp Thr Arg Pro Arg Phe Leu Glu Gln Val Lys His Glu Cys His
   1               5                   10                  15
   Phe Phe Asn Gly Thr Glu Arg Val Arg Phe Leu Asp Arg Tyr Phe Tyr
               20                  25                  30
   His Gln Glu Glu Tyr Val Arg Phe Asp Ser Asp Val Gly Glu Tyr Arg
           35                  40                  45
   Ala Val Thr Glu Leu Gly Arg Pro Ser Ala Glu Tyr Trp Asn Ser Gln
       50                  55                  60
   Lys Asp Leu Leu Glu Gln Arg Arg Ala Ala Val Asp Thr Tyr Cys Arg
   65                  70                  75                  80
   His Asn Tyr Gly Val Gly Glu Ser Phe Thr Val Gln Arg Arg
                   85                  90
<210> 326
   <211> 88
   <212> PRT
   <213> Homo sapiens
<400> 326
   Phe Leu Glu Gln Val Lys His Glu Cys His Phe Phe Asn Gly Thr Glu
   1               5                   10                  15
   Arg Val Arg Phe Leu Asp Arg Tyr Phe Tyr His Gln Glu Glu Tyr Val
               20                  25                  30
   Arg Phe Asp Ser Asp Val Gly Glu Tyr Arg Ala Val Thr Glu Leu Gly
           35                  40                  45
   Arg Pro Ser Ala Glu Tyr Trp Asn Ser Gln Lys Asp Leu Leu Glu Gln
       50                  55                  60
   Arg Arg Ala Ala Val Asp Thr Tyr Cys Arg His Asn Tyr Gly Val Gly
   65                  70                  75                  80
   Glu Ser Phe Thr Val Gln Arg Arg
                   85
<210> 327
   <211> 237
   <212> PRT
   <213> Homo sapiens
<400> 327
   Gly Asp Thr Arg Pro Arg Phe Leu Glu Gln Val Lys His Glu Cys His
   1               5                   10                  15
   Phe Phe Asn Gly Thr Glu Arg Val Arg Phe Leu Asp Arg Tyr Phe Tyr
               20                  25                  30
   His Gln Glu Glu Ser Val Arg Phe Asp Ser Asp Val Gly Glu Tyr Arg
           35                  40                  45
   Ala Val Thr Glu Leu Gly Arg Pro Asp Ala Glu Tyr Trp Asn Ser Gln
       50                  55                  60
   Lys Asp Leu Leu Glu Gln Arg Arg Ala Glu Val Asp Thr Tyr Cys Arg
   65                  70                  75                  80
   His Asn Tyr Gly Val Val Glu Ser Phe Thr Val Gln Arg Arg Val Tyr
                   85                  90                  95
   Pro Glu Val Thr Val Tyr Pro Ala Lys Thr Gln Pro Leu Gln His His
               100                 105                 110
   Asn Leu Leu Val Cys Ser Val Asn Gly Phe Tyr Pro Gly Ser Ile Glu
           115                 120                 125
   Val Arg Trp Phe Arg Asn Gly Gln Glu Glu Lys Thr Gly Val Val Ser
       130                 135                 140
   Thr Gly Leu Ile Gln Asn Gly Asp Trp Thr Phe Gln Thr Leu Val Met
   145                 150                 155                 160
   Leu Glu Thr Val Pro Arg Ser Gly Glu Val Tyr Thr Cys Gln Val Glu
                   165                 170                 175
   His Pro Ser Leu Thr Ser Pro Leu Thr Val Glu Trp Arg Ala Arg Ser
               180                 185                 190
   Glu Ser Ala Gln Ser Lys Met Leu Ser Gly Val Gly Gly Phe Val Leu
           195                 200                 205
   Gly Leu Leu Phe Leu Gly Ala Gly Leu Phe Ile Tyr Phe Arg Asn Gln
       210                 215                 220
   Lys Gly His Ser Gly Leu Gln Pro Thr Gly Phe Leu Ser
   225                 230                 235
<210> 328
   <211> 94
   <212> PRT
   <213> Homo sapiens
<400> 328
   Gly Asp Thr Arg Pro Arg Phe Leu Glu Gln Val Lys His Glu Cys His
   1               5                   10                  15
   Phe Phe Asn Gly Thr Glu Arg Val Arg Phe Leu Asp Arg Tyr Phe Tyr
               20                  25                  30
   His Gln Glu Glu Tyr Val Arg Phe Asp Ser Asp Val Gly Glu Tyr Arg
           35                  40                  45
   Ala Val Thr Glu Leu Gly Arg Pro Asp Ala Glu Tyr Trp Asn Ser Gln
       50                  55                  60
   Lys Asp Leu Leu Glu Gln Arg Arg Ala Glu Val Asp Thr Tyr Cys Arg
   65                  70                  75                  80
   His Asn Tyr Gly Val Gly Glu Ser Phe Thr Val Gln Arg Arg
                   85                  90
<210> 329
   <211> 88
   <212> PRT
   <213> Homo sapiens
<400> 329
   Arg Phe Leu Glu Gln Val Lys His Glu Cys His Phe Phe Asn Gly Thr
   1               5                   10                  15
   Glu Arg Val Arg Phe Leu Asp Arg Tyr Phe Tyr His Gln Glu Glu Tyr
               20                  25                  30
   Val Arg Phe Asp Ser Asp Val Gly Glu Tyr Arg Ala Val Thr Glu Leu
           35                  40                  45
   Gly Arg Pro Asp Ala Glu Tyr Trp Asn Ser Gln Lys Asp Leu Leu Glu
       50                  55                  60
   Gln Arg Arg Ala Glu Val Asp Thr Tyr Cys Arg His Asn Tyr Gly Val
   65                  70                  75                  80
   Gly Glu Ser Phe Thr Val Gln Arg
                   85
<210> 330
   <211> 88
   <212> PRT
   <213> Homo sapiens
<400> 330
   Phe Leu Glu Gln Val Lys His Glu Cys His Phe Phe Asn Gly Thr Glu
   1               5                   10                  15
   Arg Val Arg Phe Leu Asp Arg Tyr Phe Tyr His Gln Glu Glu Tyr Val
               20                  25                  30
   Arg Phe Asp Ser Asp Val Gly Glu Tyr Arg Ala Val Thr Glu Leu Gly
           35                  40                  45
   Arg Pro Asp Ala Glu Tyr Trp Asn Ser Gln Lys Asp Leu Leu Glu Gln
       50                  55                  60
   Arg Arg Ala Ala Val Asp Thr Tyr Cys Arg His Asn Tyr Gly Val Gly
   65                  70                  75                  80
   Glu Ser Phe Thr Val Gln Arg Arg
                   85
<210> 331
   <211> 74
   <212> PRT
   <213> Homo sapiens
<400> 331
   Glu Cys His Phe Phe Asn Gly Thr Glu Arg Val Arg Phe Leu Asp Arg
   1               5                   10                  15
   Tyr Phe Tyr His Gln Glu Glu Tyr Val Arg Phe Asp Ser Asp Val Gly
               20                  25                  30
   Glu Tyr Arg Ala Val Thr Glu Leu Gly Arg Pro Ser Ala Glu Tyr Trp
           35                  40                  45
   Asn Ser Gln Lys Asp Leu Leu Glu Gln Lys Arg Ala Ala Val Asp Thr
       50                  55                  60
   Tyr Cys Arg His Asn Tyr Gly Val Gly Glu
   65                  70
<210> 332
   <211> 84
   <212> PRT
   <213> Homo sapiens
<400> 332
   Phe Leu Glu Gln Val Lys His Glu Cys His Phe Phe Asn Gly Thr Glu
   1               5                   10                  15
   Arg Val Arg Phe Leu Asp Arg Tyr Phe Tyr His Gln Glu Glu Tyr Val
               20                  25                  30
   Arg Phe Asp Ser Asp Val Gly Glu Tyr Arg Ala Val Thr Glu Leu Gly
           35                  40                  45
   Arg Pro Ser Ala Glu Tyr Trp Asn Ser Gln Lys Asp Leu Leu Glu Gln
       50                  55                  60
   Arg Arg Ala Ala Val Asp Thr Tyr Cys Arg His Asn Tyr Gly Val Val
   65                  70                  75                  80
   Glu Ser Phe Thr
<210> 333
   <211> 266
   <212> PRT
   <213> Homo sapiens
<400> 333
<210> 334
   <211> 87
   <212> PRT
   <213> Homo sapiens
<400> 334
   Phe Leu Glu Gln Val Lys His Glu Cys His Phe Phe Asn Gly Thr Glu
   1               5                   10                  15
   Arg Val Arg Phe Leu Asp Arg Tyr Phe Tyr His Gln Glu Glu Tyr Val
               20                  25                  30
   Arg Phe Asp Ser Asp Val Gly Glu Tyr Arg Ala Val Thr Glu Leu Gly
           35                  40                  45
   Arg Pro Ser Ala Glu Tyr Trp Asn Ser Gln Lys Asp Ile Leu Glu Asp
       50                  55                  60
   Arg Arg Ala Leu Val Asp Thr Tyr Cys Arg His Asn Tyr Gly Val Val
   65                  70                  75                  80
   Glu Ser Phe Thr Val Gln Arg
                   85
<210> 335
   <211> 78
   <212> PRT
   <213> Homo sapiens
<400> 335
   His Glu Cys His Phe Phe Asn Gly Thr Glu Arg Val Arg Phe Leu Asp
   1               5                   10                  15
   Arg Tyr Phe Tyr His Gln Glu Glu Tyr Val Arg Phe Asp Ser Asp Val
               20                  25                  30
   Gly Glu Tyr Arg Ala Val Thr Glu Leu Gly Arg Pro Asp Ala Glu Tyr
           35                  40                  45
   Trp Asn Ser Gln Lys Asp Leu Leu Glu Gln Lys Arg Ala Ala Val Asp
       50                  55                  60
   Thr Tyr Cys Arg His Asn Tyr Gly Val Val Glu Ser Phe Thr
   65                  70                  75
<210> 336
   <211> 74
   <212> PRT
   <213> Homo sapiens
<400> 336
   Glu Cys His Phe Phe Asn Gly Thr Glu Arg Val Arg Phe Leu Asp Arg
   1               5                   10                  15
   Tyr Phe Tyr His Gln Glu Glu Tyr Val Arg Phe Asp Ser Asp Val Gly
               20                  25                  30
   Glu Tyr Arg Ala Val Thr Glu Leu Gly Arg Pro Asp Ala Glu Tyr Trp
           35                  40                  45
   Asn Ser Gln Lys Asp Ile Leu Glu Asp Glu Arg Ala Ala Val Asp Thr
       50                  55                  60
   Tyr Cys Arg His Asn Tyr Gly Val Gly Glu
   65                  70
<210> 337
   <211> 82
   <212> PRT
   <213> Homo sapiens
<400> 337
   Arg Phe Leu Glu Gln Val Lys His Glu Cys His Phe Phe Asn Gly Thr
   1               5                   10                  15
   Glu Arg Val Arg Phe Leu Asp Arg Tyr Phe Tyr His Gln Glu Glu Tyr
               20                  25                  30
   Val Arg Phe Asp Ser Asp Val Gly Glu Tyr Arg Ala Val Thr Glu Leu
           35                  40                  45
   Gly Arg Pro Asp Glu Glu Tyr Trp Asn Ser Gln Lys Asp Phe Leu Glu
       50                  55                  60
   Asp Arg Arg Ala Ala Val Asp Thr Tyr Cys Arg His Asn Tyr Gly Val
   65                  70                  75                  80
   Val Glu
<210> 338
   <211> 73
   <212> PRT
   <213> Homo sapiens
<400> 338
   Glu Cys His Phe Phe Asn Gly Thr Glu Arg Val Arg Phe Leu Asp Arg
   1               5                   10                  15
   Tyr Phe Tyr His Gln Glu Glu Tyr Val Arg Phe Asp Ser Asp Val Gly
   20 25 30
   Glu Tyr Arg Ala Val Thr Glu Leu Gly Arg Pro Asp Ala Gln Tyr Trp
   35 40 45
   Asn Ser Gln Lys Asp Leu Leu Glu Gln Lys Arg Ala Ala Val Asp Thr
   50 55 60
   Tyr Cys Arg His Asn Tyr Gly Val Gly
   65 70
<210> 339
   <211> 73
   <212> PRT
   <213> Homo sapiens
<400> 339
   Glu Cys His Phe Phe Asn Gly Thr Glu Arg Val Arg Phe Leu Asp Arg
   1 5 10 15
   Tyr Phe Tyr His Gln Glu Glu Tyr Val Arg Phe Asp Ser Asp Val Gly
   20 25 30
   Glu Tyr Arg Ala Val Thr Glu Leu Gly Arg Pro Ser Ala Glu Tyr Trp
   35 40 45
   Asn Ser Gln Lys Asp Leu Leu Glu Gln Arg Arg Ala Glu Val Asp Thr
   50 55 60
   Tyr Cys Arg His Asn Tyr Gly Val Gly
   65 70
<210> 340
   <211> 78
   <212> PRT
   <213> Homo sapiens
<400> 340
   Glu Cys His Phe Phe Asn Gly Thr Glu Arg Val Arg Phe Leu Asp Arg
   1               5                   10                  15
   Tyr Phe Tyr His Gln Glu Glu Tyr Val Arg Phe Asp Ser Asp Val Gly
               20                  25                  30
   Glu Tyr Arg Ala Val Thr Glu Leu Gly Arg Pro Asp Ala Glu Tyr Trp
           35                  40                  45
   Asn Ser Gln Lys Asp Ile Leu Glu Asp Arg Arg Ala Leu Val Asp Thr
       50                  55                  60
   Tyr Cys Arg His Asn Tyr Gly Val Val Glu Ser Phe Thr Val
   65                  70                  75
<210> 341
   <211> 79
   <212> PRT
   <213> Homo sapiens
<400> 341
   His Glu Cys His Phe Phe Asn Gly Thr Glu Arg Val Arg Phe Leu Asp
   1               5                   10                  15
   Arg Tyr Phe Tyr His Gln Glu Glu Ser Val Arg Phe Asp Ser Asp Val
               20                  25                  30
   Gly Glu Tyr Arg Ala Val Thr Glu Leu Gly Arg Pro Asp Ala Glu Tyr
           35                  40                  45
   Trp Asn Ser Gln Lys Asp Leu Leu Glu Gln Arg Arg Ala Ala Val Asp
       50                  55                  60
   Thr Tyr Cys Arg His Asn Tyr Gly Val Gly Glu Ser Phe Thr Val
   65                  70                  75
<210> 342
   <211> 73
   <212> PRT
   <213> Homo sapiens
<400> 342
   Glu Cys His Phe Phe Asn Gly Thr Glu Arg Val Arg Phe Leu Asp Arg
   1               5                   10                  15
   Tyr Phe Tyr His Gln Glu Glu Ser Val Arg Phe Asp Ser Asp Val Gly
               20                  25                  30
   Glu Tyr Arg Ala Val Thr Glu Leu Gly Arg Pro Asp Ala Glu Tyr Trp
           35                  40                  45
   Asn Ser Gln Lys Asp Leu Leu Glu Gln Arg Arg Ala Glu Val Asp Thr
       50                  55                  60
   Tyr Cys Arg His Asn Tyr Gly Val Gly
   65                  70
<210> 343
   <211> 83
   <212> PRT
   <213> Homo sapiens
<400> 343
   Glu Gln Val Lys His Glu Cys His Phe Phe Asn Gly Thr Glu Arg Val
   1               5                   10                  15
   Arg Phe Leu Asp Arg Tyr Phe Tyr His Gln Glu Glu Ser Val Arg Phe
               20                  25                  30
   Asp Ser Asp Val Gly Glu Tyr Arg Ala Val Thr Glu Leu Gly Arg Pro
           35                  40                  45
   Asp Ala Glu Tyr Trp Asn Ser Gln Lys Asp Leu Leu Glu Gln Lys Arg
       50                  55                  60
   Ala Ala Val Asp Thr Tyr Cys Arg His Asn Tyr Gly Val Gly Glu Ser
   65                  70                  75                  80
   Phe Thr Val

<210> 344
   <211> 82
   <212> PRT
   <213> Homo sapiens
<400> 344
   Glu Gln Val Lys His Glu Cys His Phe Phe Asn Gly Thr Glu Arg Val
   1               5                   10                  15
   Arg Phe Leu Asp Arg Tyr Phe Tyr His Gln Glu Glu Tyr Val Arg Phe
               20                  25                  30
   Asp Ser Asp Val Gly Glu Tyr Arg Ala Val Thr Glu Leu Gly Arg Pro
           35                  40                  45
   Asp Ala Glu Tyr Trp Asn Ser Gln Lys Asp Leu Leu Glu Gln Lys Arg
       50                  55                  60
   Gly Arg Val Asp Asn Tyr Cys Arg His Asn Tyr Gly Val Val Glu Ser
   65                  70                  75                  80
   Phe Thr
<210> 345
   <211> 89
   <212> PRT
   <213> Homo sapiens
<400> 345
   Arg Phe Leu Glu Gln Val Lys His Glu Cys His Phe Phe Asn Gly Thr
   1               5                   10                  15
   Glu Arg Val Arg Phe Leu Asp Arg Tyr Phe Tyr His Gln Glu Glu Tyr
               20                  25                  30
   Val Arg Phe Asp Ser Asp Val Gly Glu Tyr Arg Ala Val Thr Glu Leu
           35                  40                  45
   Gly Arg Pro Asp Ala Glu Tyr Trp Asn Ser Gln Lys Asp Leu Leu Glu
       50                  55                  60
   Gln Arg Arg Ala Ala Val Asp Thr Tyr Cys Arg His Asn Tyr Gly Val
   65                  70                  75                  80
   Val Glu Arg Phe Thr Val Gln Arg Arg
                   85
<210> 346
   <211> 89
   <212> PRT
   <213> Homo sapiens
<400> 346
   Arg Phe Leu Glu Gln Val Lys His Glu Cys His Phe Phe Asn Gly Thr
   1 5 10 15
   Glu Arg Val Arg Phe Leu Asp Arg Tyr Phe Tyr His Gln Glu Glu Tyr
   20 25 30
   Val Arg Phe Asp Ser Asp Val Gly Glu Tyr Arg Ala Val Thr Glu Leu
   35 40 45
   Gly Arg Pro Ser Ala Glu Tyr Trp Asn Ser Gln Lys Asp Leu Leu Glu
   50 55 60
   Arg Arg Arg Ala Ala Val Asp Thr Tyr Cys Arg His Asn Tyr Gly Val
   65 70 75 80
   Gly Glu Ser Phe Thr Val Gln Arg Arg
   85
<210> 347
   <211> 80
   <212> PRT
   <213> Homo sapiens
<400> 347
   Leu Glu Gln Val Lys His Glu Cys His Phe Phe Asn Gly Thr Glu Arg
   1 5 10 15
   Val Arg Phe Leu Asp Arg Tyr Phe Tyr His Gln Glu Glu Tyr Val Arg
   20 25 30
   Phe Asp Ser Asp Val Gly Glu Tyr Arg Ala Val Thr Glu Leu Gly Arg
   35 40 45
   Pro Asp Ala Glu Tyr Trp Asn Ser Gln Lys Asp Phe Leu Glu Asp Arg
   50 55 60
   Arg Ala Leu Val Asp Thr Tyr Cys Arg His Asn Tyr Gly Val Val Glu
   65 70 75 80
<210> 348
   <211> 81
   <212> PRT
   <213> Homo sapiens
<400> 348
   Arg Phe Leu Glu Gln Val Lys His Glu Cys His Phe Phe Asn Gly Thr
   1 5 10 15
   Glu Arg Val Arg Phe Leu Asp Arg Tyr Phe Tyr His Gln Glu Glu Tyr
   20 25 30
   Val Arg Phe Asp Ser Asp Val Gly Glu Tyr Arg Ala Val Thr Glu Leu
   35 40 45
   Gly Arg Pro Asp Thr Glu Tyr Trp Asn Ser Gln Lys Asp Leu Leu Glu
   50 55 60
   Gln Lys Arg Ala Ala Val Asp Thr Tyr Cys Arg His Asn Tyr Gly Val
   65 70 75 80
   Gly
<210> 349
   <211> 86
   <212> PRT
   <213> Homo sapiens
<400> 349
   Arg Phe Leu Glu Gln Val Lys His Glu Cys His Phe Phe Asn Gly Thr
   1 5 10 15
   Glu Arg Val Arg Phe Leu Asp Arg Tyr Phe Tyr His Gln Glu Glu Tyr
   20 25 30
   Val Arg Phe Asp Ser Asp Val Gly Glu Tyr Arg Ala Val Thr Glu Leu
   35 40 45
   Gly Arg Pro Asp Ala Glu Tyr Trp Asn Ser Gln Lys Asp Leu Leu Glu
   50 55 60
   Gln Arg Arg Ala Glu Val Asp Thr Tyr Cys Arg His Asn Tyr Gly Ala
   65 70 75 80
   Val Glu Ser Phe Thr Val
   85
<210> 350
   <211> 86
   <212> PRT
   <213> Homo sapiens
<400> 350
   Arg Phe Leu Glu Gln Val Lys His Glu Cys His Phe Phe Asn Gly Thr
   1               5                   10                  15
   Glu Arg Val Arg Phe Leu Asp Arg Tyr Phe Tyr His Gln Glu Glu Tyr
               20                  25                  30
   Val Arg Phe Asp Ser Asp Val Gly Glu Tyr Arg Ala Val Thr Glu Leu
           35                  40                  45
   Gly Arg Pro Asp Ala Glu Tyr Trp Asn Ser Gln Lys Asp Leu Leu Glu
       50                  55                  60
   Gln Arg Arg Ala Glu Val Asp Thr Tyr Cys Arg His Asn Tyr Gly Ala
   65                  70                  75                  80
   Val Glu Ser Phe Thr Val
                   85
<210> 351
   <211> 89
   <212> PRT
   <213> Homo sapiens
<400> 351
   Arg Phe Leu Glu Gln Val Lys His Glu Cys His Phe Phe Asn Gly Thr
   1               5                   10                  15
   Glu Arg Val Arg Phe Leu Asp Arg Tyr Phe Tyr His Gln Glu Glu Tyr
               20                  25                  30
   Val Arg Phe Asp Ser Asp Val Gly Glu Tyr Arg Ala Val Met Glu Leu
           35                  40                  45
   Gly Arg Pro Ser Ala Glu Tyr Trp Asn Ser Gln Lys Asp Leu Leu Glu
       50                  55                  60
   Gln Arg Arg Ala Ala Val Asp Thr Tyr Cys Arg His Asn Tyr Gly Val
   65                  70                  75                  80
   Gly Glu Ser Phe Thr Val Gln Arg Arg
                   85
<210> 352
   <211> 89
   <212> PRT
   <213> Homo sapiens
<400> 352
   Arg Phe Leu Glu Gln Val Lys His Glu Cys His Phe Phe Asn Gly Thr
   1 5 10 15
   Glu Arg Val Arg Phe Leu Asp Arg Tyr Phe Tyr His Gln Glu Glu Tyr
   20 25 30
   Val Arg Phe Asp Ser Asp Val Gly Glu Tyr Arg Val Val Thr Glu Leu
   35 40 45
   Gly Arg Pro Ser Ala Glu Tyr Trp Asn Ser Gln Lys Asp Leu Leu Glu
   50 55 60
   Gln Arg Arg Ala Ala Val Asp Thr Tyr Cys Arg His Asn Tyr Gly Val
   65 70 75 80
   Gly Glu Ser Phe Thr Val Gln Arg Arg
   85
<210> 353
   <211> 89
   <212> PRT
   <213> Homo sapiens
<400> 353
   Arg Phe Leu Glu Gln Val Lys His Glu Cys His Phe Phe Asn Gly Thr
   1 5 10 15
   Glu Arg Val Arg Phe Leu Asp Arg Tyr Phe Tyr His Gln Glu Glu Tyr
   20 25 30
   Val Arg Phe Asp Ser Asp Val Gly Glu Tyr Arg Ala Val Thr Glu Leu
   35 40 45
   Gly Arg Pro Asp Ala Glu Tyr Trp Asn Ser Gln Lys Asp Leu Leu Glu
   50 55 60
   Gln Arg Arg Ala Leu Val Asp Thr Tyr Cys Arg His Asn Tyr Gly Val
   65 70 75 80
   Gly Glu Ser Phe Thr Val Gln Arg Arg
   85
<210> 354
   <211> 80
   <212> PRT
   <213> Homo sapiens
<400> 354
   Leu Glu Gln Val Lys His Glu Cys His Phe Phe Asn Gly Thr Glu Arg
   1               5                   10                  15
   Val Arg Phe Leu Asp Arg Tyr Phe Tyr His Gln Glu Glu Tyr Val Arg
               20                  25                  30
   Phe Asp Ser Asp Val Gly Glu Tyr Arg Ala Val Thr Glu Leu Gly Arg
           35                  40                  45
   Pro Asp Ala Glu Tyr Trp Asn Ser Gln Lys Asp Leu Leu Glu Gln Arg
       50                  55                  60
   Gln Ala Ala Val Asp Thr Tyr Cys Arg His Asn Tyr Gly Val Val Glu
   65                  70                  75                  80
<210> 355
   <211> 81
   <212> PRT
   <213> Homo sapiens
<400> 355
   Arg Phe Leu Glu Gln Val Lys His Glu Cys His Phe Phe Asn Gly Thr
   1               5                   10                  15
   Glu Arg Val Arg Phe Leu Asp Arg Tyr Phe Tyr His Gln Glu Glu Tyr
               20                  25                  30
   Val His Phe Asp Ser Asp Val Gly Glu Tyr Arg Ala Val Thr Glu Leu
           35                  40                  45
   Gly Arg Pro Asp Ala Glu Tyr Trp Asn Ser Gln Lys Asp Leu Leu Glu
       50                  55                  60
   Gln Lys Arg Ala Ala Val Asp Thr Tyr Cys Arg His Asn Tyr Gly Val
   65                  70                  75                  80
   Gly
<210> 356
   <211> 80
   <212> PRT
   <213> Homo sapiens
<400> 356
   Phe Leu Glu Gln Val Lys Pro Glu Cys His Phe Phe Asn Gly Thr Glu
   1               5                   10                  15
   Arg Val Arg Phe Leu Asp Arg Tyr Phe Tyr His Gln Glu Glu Tyr Val
               20                  25                  30
   Arg Phe Asp Ser Asp Val Gly Glu Tyr Arg Ala Val Thr Glu Leu Gly
           35                  40                  45
   Arg Pro Asp Ala Glu Tyr Trp Asn Ser Gln Lys Asp Leu Leu Glu Gln
       50                  55                  60
   Lys Arg Ala Ala Val Asp Thr Tyr Cys Arg His Asn Tyr Gly Val Gly
   65                  70                  75                  80
<210> 357
   <211> 86
   <212> PRT
   <213> Homo sapiens
<400> 357
   Arg Phe Leu Glu Gln Val Lys His Glu Cys His Phe Phe Asn Gly Thr
   1               5                   10                  15
   Glu Arg Val Arg Phe Leu Asp Arg Tyr Phe Tyr His Gln Glu Glu Tyr
               20                  25                  30
   Val Arg Phe Asp Ser Asp Val Gly Glu Phe Arg Ala Val Thr Glu Leu
           35                  40                  45
   Gly Arg Pro Asp Ala Glu Tyr Trp Asn Ser Gln Lys Asp Leu Leu Glu
       50                  55                  60
   Gln Lys Arg Ala Ala Val Asp Thr Tyr Cys Arg His Asn Tyr Gly Val
   65                  70                  75                  80
   Gly Glu Ser Phe Thr Val
                   85
<210> 358
   <211> 89
   <212> PRT
   <213> Homo sapiens
<400> 358
   Arg Phe Leu Glu Gln Val Lys His Glu Cys His Phe Phe Asn Gly Thr
   1 5 10 15
   Glu Arg Val Arg Phe Leu Asp Arg Tyr Phe Tyr His Gln Glu Glu Tyr
   20 25 30
   Val Arg Phe Asp Ser Asp Val Gly Glu Tyr Arg Ala Val Thr Glu Leu
   35 40 45
   Gly Arg Pro Asp Ala Glu Tyr Trp Asn Ser Gln Lys Asp Phe Leu Glu
   50 55 60
   Asp Arg Arg Ala Ala Val Asp Thr Tyr Cys Arg His Asn Tyr Gly Val
   65 70 75 80
   Val Glu Ser Phe Thr Val Gln Arg Arg
   85
<210> 359
   <211> 89
   <212> PRT
   <213> Homo sapiens
<400> 359
   Arg Phe Leu Glu Gln Val Lys His Glu Cys His Phe Phe Asn Gly Thr
   1 5 10 15
   Glu Arg Val Arg Phe Leu Asp Arg Tyr Phe Tyr His Gln Glu Glu Tyr
   20 25 30
   Val Arg Phe Asp Ser Asp Val Gly Glu Tyr Arg Ala Val Thr Glu Leu
   35 40 45
   Gly Arg Pro Asp Ala Glu Tyr Trp Asn Ser Gln Lys Asp Leu Leu Glu
   50 55 60
   Asp Glu Arg Ala Ala Val Asp Thr Tyr Cys Arg His Asn Tyr Gly Val
   65 70 75 80
   Val Glu Ser Phe Thr Val Gln Arg Arg
   85
<210> 360
   <211> 89
   <212> PRT
   <213> Homo sapiens
<400> 360
   Arg Phe Leu Glu Gln Val Lys His Glu Cys His Phe Phe Asn Gly Thr
   1               5                   10                  15
   Glu Arg Val Arg Phe Leu Asp Arg Tyr Phe Tyr His Gln Glu Glu Tyr
               20                  25                  30
   Val Arg Phe Asp Ser Asp Val Gly Glu Tyr Arg Ala Val Thr Glu Leu
           35                  40                  45
   Gly Arg Pro Asp Ala Glu Tyr Trp Asn Ser Gln Lys Asp Ile Leu Glu
       50                  55                  60
   Gln Lys Arg Ala Ala Val Asp Thr Tyr Cys Arg His Asn Tyr Gly Val
   65                  70                  75                  80
   Gly Glu Ser Phe Thr Val Gln Arg Arg
                   85
<210> 361
   <211> 88
   <212> PRT
   <213> Homo sapiens
<400> 361
   Arg Phe Leu Glu Gln Val Lys His Glu Cys His Phe Phe Asn Gly Thr
   1               5                   10                  15
   Glu Arg Val Arg Phe Leu Asp Arg Tyr Phe Tyr His Gln Glu Glu Tyr
               20                  25                  30
   Val Arg Phe Asp Ser Asp Val Gly Asp Tyr Arg Ala Val Thr Glu Leu
           35                  40                  45
   Gly Arg Pro Asp Ala Glu Tyr Trp Asn Ser Gln Lys Asp Leu Leu Glu
       50                  55                  60
   Gln Arg Arg Ala Glu Val Asp Thr Tyr Cys Arg His Asn Tyr Gly Val
   65                  70                  75                  80
   Val Glu Ser Phe Thr Val Gln Arg
                   85
<210> 362
   <211> 88
   <212> PRT
   <213> Homo sapiens
<400> 362
   Arg Phe Leu Glu Gln Val Lys His Glu Cys His Phe Phe Asn Gly Thr
   1               5                   10                  15
   Glu Arg Val Arg Phe Leu Asp Arg Tyr Phe Tyr His Gln Glu Glu Tyr
               20                  25                  30
   Val Arg Phe Asp Ser Asp Val Gly Glu Tyr Arg Ala Val Thr Glu Leu
           35                  40                  45
   Gly Arg Pro Asp Gly Glu Tyr Trp Asn Ser Gln Lys Asp Leu Leu Glu
       50                  55                  60
   Gln Arg Arg Ala Ala Val Asp Thr Tyr Cys Arg His Asn Tyr Gly Val
   65                  70                  75                  80
   Val Glu Ser Phe Thr Val Gln Arg
                   85
<210> 363
   <211> 88
   <212> PRT
   <213> Homo sapiens
<400> 363
   Arg Phe Leu Glu Gln Val Lys His Glu Cys His Phe Phe Asn Gly Thr
   1               5                   10                  15
   Glu Arg Val Arg Phe Leu Asp Arg Tyr Phe Tyr His Gln Glu Glu Asn
               20                  25                  30
   Val Arg Phe Asp Ser Asp Val Gly Glu Tyr Arg Ala Val Thr Glu Leu
           35                  40                  45
   Gly Arg Pro Asp Ala Glu Tyr Trp Asn Ser Gln Lys Asp Leu Leu Glu
       50                  55                  60
   Gln Arg Arg Ala Glu Val Asp Thr Tyr Cys Arg His Asn Tyr Gly Val
   65                  70                  75                  80
   Val Glu Ser Phe Thr Val Gln Arg
                   85
<210> 364
   <211> 89
   <212> PRT
   <213> Homo sapiens
<400> 364
   Arg Phe Leu Glu Gln Val Lys His Glu Cys His Phe Phe Asn Gly Thr
   1               5                   10                  15
   Glu Arg Val Arg Phe Leu Asp Arg Tyr Phe Tyr His Gln Glu Glu Tyr
               20                  25                  30
   Val Arg Phe Asp Ser Asp Val Gly Glu Phe Arg Ala Val Thr Glu Leu
           35                  40                  45
   Gly Arg Pro Asp Ala Glu Tyr Trp Asn Ser Gln Lys Asp Leu Leu Glu
       50                  55                  60
   Gln Arg Arg Ala Ala Val Asp Thr Tyr Cys Arg His Asn Tyr Gly Val
   65                  70                  75                  80
   Val Glu Ser Phe Thr Val Gln Arg Arg
                   85
<210> 365
   <211> 266
   <212> PRT
   <213> Homo sapiens
<400> 365
   Met Val Cys Leu Lys Leu Pro Gly Gly Ser Cys Met Ala Ala Leu Thr
   1               5                   10                  15
   Val Thr Leu Met Val Leu Ser Ser Pro Leu Ala Leu Ala Gly Asp Thr
               20                  25                  30
   Gln Pro Arg Phe Leu Trp Gln Gly Lys Tyr Lys Cys His Phe Phe Asn
           35                  40                  45
   Gly Thr Glu Arg Val Gln Phe Leu Glu Arg Leu Phe Tyr Asn Gln Glu
       50                  55                  60
   Glu Phe Val Arg Phe Asp Ser Asp Val Gly Glu Tyr Arg Ala Val Thr
   65                  70                  75                  80
   Glu Leu Gly Arg Pro Val Ala Glu Ser Trp Asn Ser Gln Lys Asp Ile
                   85                  90                  95
   Leu Glu Asp Arg Arg Gly Gln Val Asp Thr Val Cys Arg His Asn Tyr
               100                 105                 110
   Gly Val Gly Glu Ser Phe Thr Val Gln Arg Arg Val His Pro Glu Val
           115                 120                 125
   Thr Val Tyr Pro Ala Lys Thr Gln Pro Leu Gln His His Asn Leu Leu
       130                 135                 140
   Val Cys Ser Val Ser Gly Phe Tyr Pro Gly Ser Ile Glu Val Arg Trp
   145                 150                 155                 160
   Phe Arg Asn Gly Gln Glu Glu Lys Ala Gly Val Val Ser Thr Gly Leu
                   165                 170                 175
   Ile Gln Asn Gly Asp Trp Thr Phe Gln Thr Leu Val Met Leu Glu Thr
               180                 185                 190
   Val Pro Arg Ser Gly Glu Val Tyr Thr Cys Gln Val Glu His Pro Ser
           195                 200                 205
   Val Met Ser Pro Leu Thr Val Glu Trp Arg Ala Arg Ser Glu Ser Ala
       210                 215                 220
   Gln Ser Lys Met Leu Ser Gly Val Gly Gly Phe Val Leu Gly Leu Leu
   225                 230                 235                 240
   Phe Leu Gly Ala Gly Leu Phe Ile Tyr Phe Arg Asn Gln Lys Gly His
                   245                 250                 255
   Ser Gly Leu Gln Pro Thr Gly Phe Leu Ser
               260                 265

<210> 366
   <211> 81
   <212> PRT
   <213> Homo sapiens
<400> 366
   Arg Phe Leu Trp Gln Gly Lys Tyr Lys Cys His Phe Phe Asn Gly Thr
   1               5                   10                  15
   Glu Arg Val Gln Phe Leu Glu Arg Leu Phe Tyr Asn Gln Glu Glu Phe
               20                  25                  30
   Val Arg Phe Asp Ser Asp Val Gly Glu Tyr Arg Ala Val Thr Glu Leu
           35                  40                  45
   Gly Arg Pro Val Ala Glu Ser Trp Asn Ser Gln Lys Asp Ile Leu Glu
       50                  55                  60
   Asp Arg Arg Gly Gln Val Asp Thr Val Cys Arg His Asn Tyr Gly Val
   65                  70                  75                  80
   Gly
<210> 367
   <211> 81
   <212> PRT
   <213> Homo sapiens
<400> 367
   Arg Phe Leu Trp Gln Gly Lys Tyr Lys Cys His Phe Phe Asn Gly Thr
   1               5                   10                  15
   Glu Arg Val Gln Phe Leu Glu Ser Leu Phe Tyr Asn Gln Glu Glu Phe
               20                  25                  30
   Val Arg Phe Asp Ser Asp Val Gly Glu Tyr Arg Ala Val Thr Glu Leu
           35                  40                  45
   Gly Arg Pro Val Ala Glu Ser Trp Asn Ser Gln Lys Asp Ile Leu Glu
       50                  55                  60
   Asp Arg Arg Gly Gln Val Asp Thr Val Cys Arg His Asn Tyr Gly Val
   65                  70                  75                  80
   Gly
<210> 368
   <211> 82
   <212> PRT
   <213> Homo sapiens
<400> 368
   Phe Leu Trp Gln Gly Lys Tyr Lys Cys His Phe Phe Asn Gly Thr Glu
   1 5 10 15
   Arg Val Gln Phe Leu Glu Arg Leu Phe Tyr Asn Gln Glu Glu Phe Val
   20 25 30
   Arg Phe Asp Ser Asp Val Gly Glu Tyr Arg Ala Val Thr Glu Leu Gly
   35 40 45
   Arg Pro Val Ala Glu Ser Trp Asn Ser Gln Lys Asp Ile Leu Glu Asp
   50 55 60
   Arg Arg Gly Gln Val Asp Asn Tyr Cys Arg His Asn Tyr Gly Val Gly
   65 70 75 80
   Glu Ser
<210> 369
   <211> 85
   <212> PRT
   <213> Homo sapiens
<400> 369
   Arg Phe Leu Trp Gln Gly Lys Tyr Lys Cys His Phe Phe Asn Gly Thr
   1 5 10 15
   Glu Arg Val Gln Phe Leu Glu Arg Leu Phe Tyr Asn Gln Glu Glu Phe
   20 25 30
   Val Arg Phe Asp Ser Asp Val Gly Glu Tyr Arg Ala Val Thr Glu Leu
   35 40 45
   Gly Arg Pro Val Ala Glu Ser Trp Asn Ser Arg Lys Asp Ile Leu Glu
   50 55 60
   Asp Arg Arg Gly Gln Val Asp Thr Val Cys Arg His Asn Tyr Gly Val
   65 70 75 80
   Gly Glu Ser Phe Thr
   85
<210> 370
   <211> 179
   <212> PRT
   <213> Homo sapiens
<400> 370
   Gly Asp Thr Arg Pro Arg Phe Leu Glu Tyr Ser Thr Gly Glu Cys Tyr
   1 5 10 15
   Phe Phe Asn Gly Thr Glu Arg Val Arg Phe Leu Asp Arg Tyr Phe Tyr
   20 25 30
   Asn Gln Glu Glu Tyr Val Arg Phe Asp Ser Asp Val Gly Glu Tyr Arg
   35 40 45
   Ala Val Thr Glu Leu Gly Arg Pro Ser Ala Glu Tyr Trp Asn Ser Gln
   50 55 60
   Lys Asp Phe Leu Glu Asp Arg Arg Ala Leu Val Asp Thr Tyr Cys Arg
   65 70 75 80
   His Asn Tyr Gly Val Gly Glu Ser Phe Thr Val Gln Arg Arg Val His
   85 90 95
   Pro Lys Val Thr Val Tyr Pro Ser Lys Thr Gln Pro Leu Gln His His
   100 105 110
   Asn Leu Leu Val Cys Ser Val Ser Gly Phe Tyr Pro Gly Ser Ile Glu
   115 120 125
   Val Arg Trp Phe Arg Asn Gly Gln Glu Glu Lys Thr Gly Val Val Ser
   130 135 140
   Thr Gly Leu Ile His Asn Gly Asp Trp Thr Phe Gln Thr Leu Val Met
   145 150 155 160
   Leu Glu Thr Val Pro Arg Ser Gly Glu Val Tyr Thr Cys Gln Val Glu
   165 170 175
   His Pro Ser
<210> 371
   <211> 183
   <212> PRT
   <213> Homo sapiens
<400> 371
   Arg Phe Leu Glu Tyr Ser Thr Gly Glu Cys Tyr Phe Phe Asn Gly Thr
   1 5 10 15
   Glu Arg Val Arg Phe Leu Asp Arg Tyr Phe Tyr Asn Gln Glu Glu Tyr
   20 25 30
   Val Arg Phe Asp Ser Asp Val Gly Glu Tyr Arg Ala Val Thr Glu Leu
   35 40 45
   Gly Arg Pro Asp Ala Glu Tyr Trp Asn Ser Gln Lys Asp Phe Leu Glu
   50 55 60
   Asp Arg Arg Ala Leu Val Asp Thr Tyr Cys Arg His Asn Tyr Gly Val
   65 70 75 80
   Gly Glu Ser Phe Thr Val Gln Arg Arg Val His Pro Lys Val Thr Val
   85 90 95
   Tyr Pro Ser Lys Thr Gln Pro Leu Gln His His Asn Leu Leu Val Cys
   100 105 110
   Ser Val Ser Gly Phe Tyr Pro Gly Ser Ile Glu Val Arg Trp Phe Arg
   115 120 125
   Asn Gly Gln Glu Glu Lys Thr Gly Val Val Ser Thr Gly Leu Ile His
   130 135 140
   Asn Gly Asp Trp Thr Phe Gln Thr Leu Val Met Leu Glu Thr Val Pro
   145 150 155 160
   Arg Ser Gly Glu Val Tyr Thr Cys Gln Val Glu His Pro Ser Val Thr
   165 170 175
   Ser Pro Leu Thr Val Glu Trp
   180
<210> 372
   <211> 183
   <212> PRT
   <213> Homo sapiens
<400> 372
   Arg Phe Leu Glu Tyr Ser Thr Gly Glu Cys Tyr Phe Phe Asn Gly Thr
   1               5                   10                  15
   Glu Arg Val Arg Phe Leu Asp Arg Tyr Phe Tyr Asn Gln Glu Glu Tyr
               20                  25                  30
   Val Arg Phe Asp Ser Asp Val Gly Glu Tyr Arg Ala Val Thr Glu Leu
           35                  40                  45
   Gly Arg Pro Asp Ala Glu Tyr Trp Asn Ser Gln Lys Asp Phe Leu Glu
       50                  55                  60
   Asp Arg Arg Ala Leu Val Asp Thr Tyr Cys Arg His Asn Tyr Gly Val
   65                  70                  75                  80
   Gly Glu Ser Phe Thr Val Gln Arg Arg Val His Pro Lys Val Thr Val
                   85                  90                  95
   Tyr Pro Ser Lys Thr Gln Pro Leu Gln His His Asn Leu Leu Val Cys
               100                 105                 110
   Ser Val Ser Gly Phe Tyr Pro Gly Ser Ile Glu Val Arg Trp Phe Arg
           115                 120                 125
   Asn Gly Gln Glu Glu Lys Thr Gly Val Val Ser Thr Gly Leu Ile His
       130                 135                 140
   Asn Gly Asp Trp Thr Phe Gln Thr Leu Val Met Leu Glu Thr Val Pro
   145                 150                 155                 160
   Arg Ser Gly Glu Val Tyr Thr Cys Gln Val Glu His Pro Ser Val Thr
                   165                 170                 175
   Ser Pro Leu Thr Val Glu Trp
               180
<210> 373
   <211> 86
   <212> PRT
   <213> Homo sapiens
<400> 373
   Arg Phe Leu Glu Tyr Ser Thr Gly Glu Cys Tyr Phe Phe Asn Gly Thr
   1               5                   10                  15
   Glu Arg Val Arg Phe Leu Asp Arg Tyr Phe Tyr Asn Gln Glu Glu Tyr
               20                  25                  30
   Val Arg Phe Asp Ser Asp Val Gly Glu Tyr Arg Ala Val Thr Glu Leu
   35 40 45
   Gly Arg Pro Asp Ala Glu Tyr Trp Asn Ser Gln Lys Asp Phe Leu Glu
   50 55 60
   Asp Arg Arg Ala Leu Val Asp Thr Tyr Cys Arg His Asn Tyr Gly Val
   65 70 75 80
   Gly Glu Ser Phe Thr Val
   85
<210> 374
   <211> 188
   <212> PRT
   <213> Homo sapiens
<400> 374
   Gly Asp Thr Arg Pro Arg Phe Leu Glu Tyr Ser Thr Gly Glu Cys Tyr
   1               5                   10                  15
   Phe Phe Asn Gly Thr Glu Arg Val Arg Phe Leu Asp Arg Tyr Phe Tyr
               20                  25                  30
   Asn Gln Glu Glu Tyr Val Arg Phe Asp Ser Asp Val Gly Glu Tyr Arg
           35                  40                  45
   Ala Val Thr Glu Leu Gly Arg Pro Ser Ala Glu Tyr Trp Asn Ser Gln
       50                  55                  60
   Lys Asp Ile Leu Glu Asp Arg Arg Ala Leu Val Asp Thr Tyr Cys Arg
   65                  70                  75                  80
   His Asn Tyr Gly Val Gly Glu Ser Phe Thr Val Gln Arg Arg Val His
                   85                  90                  95
   Pro Lys Val Thr Val Tyr Pro Ser Lys Thr Gln Pro Leu Gln His His
               100                 105                 110
   Asn Leu Leu Val Cys Ser Val Ser Gly Phe Tyr Pro Gly Ser Ile Glu
           115                 120                 125
   Val Arg Trp Phe Arg Asn Gly Gln Glu Glu Lys Thr Gly Val Val Ser
       130                 135                 140
   Thr Gly Leu Ile His Asn Gly Asp Trp Thr Phe Gln Thr Leu Val Met
   145                 150                 155                 160
   Leu Glu Thr Val Pro Arg Ser Gly Glu Val Tyr Thr Cys Gln Val Glu
                   165                 170                 175
   His Pro Ser Val Thr Ser Pro Leu Thr Val Glu Trp
           180                 185
<210> 375
   <211> 101
   <212> PRT
   <213> Homo sapiens
<400> 375
   Gly Asp Thr Arg Pro Arg Phe Leu Glu Tyr Ser Thr Gly Glu Cys Tyr
   1               5                   10                  15
   Phe Phe Asn Gly Thr Glu Arg Val Arg Phe Leu Asp Arg Tyr Phe Tyr
               20                  25                  30
   Asn Gln Glu Glu Tyr Val Arg Phe Asp Ser Asp Val Gly Glu Tyr Arg
           35                  40                  45
   Ala Val Thr Glu Leu Gly Arg Pro Asp Ala Glu Tyr Trp Asn Ser Gln
       50                  55                  60
   Lys Asp Phe Leu Glu Asp Arg Arg Ala Leu Val Asp Thr Tyr Cys Arg
   65                  70                  75                  80
   His Asn Tyr Gly Val Val Glu Ser Phe Thr Val Gln Arg Arg Val His
                   85                  90                  95
   Pro Lys Val Thr Val
               100
<210> 376
   <211> 76
   <212> PRT
   <213> Homo sapiens
<400> 376
   Phe Asn Gly Thr Glu Arg Val Arg Phe Leu Asp Arg Tyr Phe Tyr Asn
   1               5                   10                  15
   Gln Glu Glu Tyr Val Arg Phe Asp Ser Asp Val Gly Glu Tyr Arg Ala
               20                  25                  30
   Val Thr Glu Leu Gly Arg Pro Asp Ala Glu Tyr Trp Asn Ser Gln Lys
           35                  40                  45
   Asp Phe Leu Glu Asp Arg Arg Ala Leu Val Asp Thr Tyr Cys Arg His
       50                  55                  60
   Asn Tyr Gly Val Val Glu Ser Phe Thr Val Gln Arg
   65                  70                  75
<210> 377
   <211> 89
   <212> PRT
   <213> Homo sapiens
<400> 377
   Arg Phe Leu Glu Tyr Ser Thr Gly Glu Cys Tyr Phe Phe Asn Gly Thr
   1               5                   10                  15
   Glu Arg Val Arg Phe Leu Asp Arg Tyr Phe Tyr Asn Gln Glu Glu Tyr
               20                  25                  30
   Val Arg Phe Asp Ser Asp Val Gly Glu Tyr Arg Ala Val Thr Glu Leu
           35                  40                  45
   Gly Arg Pro Asp Ala Glu Tyr Trp Asn Ser Gln Lys Asp Phe Leu Glu
       50                  55                  60
   Asp Arg Arg Ala Leu Val Asp Thr Tyr Cys Arg His Asn Tyr Gly Val
   65                  70                  75                  80
   Val Glu Ser Phe Thr Val Gln Arg Arg
                   85
<210> 378
   <211> 89
   <212> PRT
   <213> Homo sapiens
<400> 378
   Arg Phe Leu Glu Tyr Ser Thr Gly Glu Cys Tyr Phe Phe Asn Gly Thr
   1               5                   10                  15
   Glu Arg Val Arg Phe Leu Asp Arg Tyr Phe Tyr Asn Gln Glu Glu Tyr
               20                  25                  30
   Val Arg Phe Asp Ser Asp Val Gly Glu Tyr Arg Ala Val Thr Glu Leu
           35                  40                  45
   Gly Arg Pro Asp Ala Glu Tyr Trp Asn Ser Gln Lys Asp Phe Leu Glu
       50                  55                  60
   Asp Arg Arg Ala Leu Val Asp Thr Tyr Cys Arg His Asn Tyr Gly Val
   65                  70                  75                  80
   Val Glu Ser Phe Thr Val Gln Arg Arg
                   85
<210> 379
   <211> 81
   <212> PRT
   <213> Homo sapiens
<400> 379
   Arg Phe Leu Glu Tyr Ser Thr Gly Glu Cys Tyr Phe Phe Asn Gly Thr
   1               5                   10                  15
   Glu Arg Val Arg Phe Leu Asp Arg Tyr Phe Tyr Asn Gln Glu Glu Tyr
               20                  25                  30
   Val Arg Phe Asp Ser Asp Val Gly Glu Tyr Arg Ala Val Thr Glu Leu
           35                  40                  45
   Gly Arg Pro Ser Ala Glu Tyr Trp Asn Ser Gln Lys Asp Phe Leu Glu
       50                  55                  60
   Asp Arg Arg Ala Ala Val Asp Thr Tyr Cys Arg His Asn Tyr Gly Val
   65                  70                  75                  80
   Gly
<210> 380
   <211> 90
   <212> PRT
   <213> Homo sapiens
<400> 380
   Pro Arg Phe Leu Glu Tyr Ser Thr Gly Glu Cys Tyr Phe Phe Asn Gly
   1               5                   10                  15
   Thr Glu Arg Val Arg Phe Leu Asp Arg Tyr Phe Tyr Asn Gln Glu Glu
               20                  25                  30
   Tyr Val Arg Phe Asp Ser Asp Val Gly Glu Tyr Arg Ala Val Thr Glu
           35                  40                  45
   Leu Gly Arg Pro Ser Ala Glu Tyr Trp Asn Ser Gln Lys Asp Phe Leu
       50                  55                  60
   Glu Asp Arg Arg Ala Leu Val Asp Thr Tyr Cys Arg His Asn Tyr Gly
   65                  70                  75                  80
   Val Val Glu Ser Phe Thr Val Gln Arg Arg
                   85                  90
<210> 381
   <211> 86
   <212> PRT
   <213> Homo sapiens
<400> 381
   Arg Phe Leu Glu Tyr Ser Thr Gly Glu Cys Tyr Phe Phe Asn Gly Thr
   1               5                   10                  15
   Glu Arg Val Arg Phe Leu Asp Arg Tyr Phe Tyr Asn Gln Glu Glu Tyr
               20                  25                  30
   Val Arg Phe Asp Ser Asp Val Gly Glu Tyr Arg Ala Val Thr Glu Leu
           35                  40                  45
   Gly Arg Pro Val Ala Glu Tyr Trp Asn Ser Gln Lys Asp Phe Leu Glu
       50                  55                  60
   Asp Arg Arg Ala Leu Val Asp Thr Tyr Cys Arg His Asn Tyr Gly Val
   65                  70                  75                  80
   Gly Glu Ser Phe Thr Val
                   85
<210> 382
   <211> 80
   <212> PRT
   <213> Homo sapiens
<400> 382
   Leu Glu Tyr Ser Thr Gly Glu Cys Tyr Phe Phe Asn Gly Thr Glu Arg
   1               5                   10                  15
   Val Arg Phe Leu Asp Arg Tyr Phe Tyr Asn Gln Glu Glu Tyr Val Arg
               20                  25                  30
   Phe Asp Ser Asp Val Gly Glu Tyr Arg Ala Val Thr Glu Leu Gly Arg
           35                  40                  45
   Pro Ala Ala Glu His Trp Asn Ser Gln Lys Asp Phe Leu Glu Asp Arg
       50                  55                  60
   Arg Ala Leu Val Asp Thr Tyr Cys Arg His Asn Tyr Gly Val Gly Glu
   65                  70                  75                  80
<210> 383
   <211> 89
   <212> PRT
   <213> Homo sapiens
<400> 383
   Arg Phe Leu Glu Tyr Ser Thr Gly Glu Cys Tyr Phe Phe Asn Gly Thr
   1               5                   10                  15
   Glu Arg Val Arg Phe Leu Asp Arg Tyr Phe His Asn Gln Glu Glu Phe
               20                  25                  30
   Val Arg Phe Asp Ser Asp Val Gly Glu Tyr Arg Ala Val Thr Glu Leu
           35                  40                  45
   Gly Arg Pro Asp Ala Glu Tyr Trp Asn Ser Gln Lys Asp Phe Leu Glu
       50                  55                  60
   Asp Arg Arg Ala Leu Val Asp Thr Tyr Cys Arg His Asn Tyr Gly Val
   65                  70                  75                  80
   Gly Glu Ser Phe Thr Val Gln Arg Arg
                   85

<210> 384
   <211> 89
   <212> PRT
   <213> Homo sapiens
<400> 384
   Arg Phe Leu Glu Tyr Ser Thr Gly Glu Cys Tyr Phe Phe Asn Gly Thr
   1               5                   10                  15
   Glu Arg Val Arg Phe Leu Asp Arg Tyr Phe Tyr Asn Gln Glu Glu Tyr
               20                  25                  30
   Val Arg Phe Asp Ser Asp Val Gly Glu Tyr Arg Ala Val Thr Glu Leu
           35                  40                  45
   Gly Arg Pro Ser Ala Glu Tyr Trp Asn Ser Gln Lys Asp Ile Leu Glu
       50                  55                  60
   Asp Arg Arg Ala Leu Val Asp Thr Tyr Cys Arg His Asn Tyr Gly Val
   65                  70                  75                  80
   Val Glu Ser Phe Thr Val Gln Arg Arg
                   85
<210> 385
   <211> 86
   <212> PRT
   <213> Homo sapiens
<400> 385
   Arg Phe Leu Glu Tyr Ser Thr Gly Glu Cys Tyr Phe Phe Asn Gly Thr
   1               5                   10                  15
   Glu Arg Val Arg Phe Leu Asp Arg Tyr Phe Tyr Asn Gln Glu Glu Tyr
               20                  25                  30
   Val Arg Phe Asp Ser Asp Val Gly Glu Tyr Arg Ala Val Thr Glu Leu
           35                  40                  45
   Gly Arg Pro Ala Ala Glu Tyr Trp Asn Ser Gln Lys Asp Phe Leu Glu
       50                  55                  60
   Asp Arg Arg Ala Leu Val Asp Thr Tyr Cys Arg His Asn Tyr Gly Val
   65                  70                  75                  80
   Gly Glu Ser Phe Thr Val
                   85
<210> 386
   <211> 89
   <212> PRT
   <213> Homo sapiens
<400> 386
   Arg Phe Leu Glu Tyr Ser Thr Gly Glu Cys Tyr Phe Phe Asn Gly Thr
   1               5                   10                  15
   Glu Arg Val Arg Phe Leu Asp Arg Tyr Phe Tyr Asn Gln Glu Glu Tyr
               20                  25                  30
   Val Arg Phe Asp Ser Asp Val Gly Glu Tyr Arg Ala Val Thr Glu Leu
           35                  40                  45
   Gly Arg Pro Ser Ala Glu Tyr Trp Asn Ser Gln Lys Asp Ile Leu Glu
       50                  55                  60
   Asp Arg Arg Ala Leu Val Asp Thr Tyr Cys Arg His Asn Tyr Gly Ala
   65                  70                  75                  80
   Val Glu Ser Phe Thr Val Gln Arg Arg
                   85
<210> 387
   <211> 80
   <212> PRT
   <213> Homo sapiens
<400> 387
   Leu Glu Tyr Ser Thr Gly Glu Cys Tyr Phe Phe Asn Gly Thr Glu Arg
   1 5 10 15
   Val Arg Phe Leu Asp Arg Tyr Phe Tyr Asn Gln Glu Glu Tyr Val Arg
   20 25 30
   Phe Asp Ser Asp Val Gly Glu Tyr Arg Ala Val Thr Glu Leu Gly Arg
   35 40 45
   Pro Asp Ala Glu Tyr Trp Asn Ser Gln Lys Asp Leu Leu Glu Asp Arg
   50 55 60
   Arg Ala Leu Val Asp Thr Tyr Cys Arg His Asn Tyr Gly Val Gly Glu
   65 70 75 80
<210> 388
   <211> 86
   <212> PRT
   <213> Homo sapiens
<400> 388
   Arg Phe Leu Glu Tyr Ser Arg Gly Glu Cys Tyr Phe Phe Asn Gly Thr
   1 5 10 15
   Glu Arg Val Arg Phe Leu Asp Arg Tyr Phe Tyr Asn Gln Glu Glu Tyr
   20 25 30
   Val Arg Phe Asp Ser Asp Val Gly Glu Tyr Arg Ala Val Thr Glu Leu
   35 40 45
   Gly Arg Pro Ser Ala Glu Tyr Trp Asn Ser Gln Lys Asp Ile Leu Glu
   50 55 60
   Asp Arg Arg Ala Leu Val Asp Thr Tyr Cys Arg His Asn Tyr Gly Val
   65 70 75 80
   Gly Glu Ser Phe Thr Val
   85
<210> 389
   <211> 84
   <212> PRT
   <213> Homo sapiens
<400> 389
   Phe Leu Glu Tyr Ser Thr Gly Glu Cys Tyr Phe Phe Asn Gly Thr Glu
   1               5                   10                  15
   Arg Val Arg Phe Leu Asp Arg Tyr Phe Tyr Asn Gln Glu Glu Tyr Val
               20                  25                  30
   Arg Phe Asp Ser Asp Val Gly Glu Tyr Arg Ala Val Thr Glu Leu Gly
           35                  40                  45
   Arg Pro Asp Ala Glu His Trp Asn Ser Gln Lys Asp Ile Leu Glu Asp
       50                  55                  60
   Arg Arg Ala Leu Val Asp Thr Tyr Cys Arg His Asn Tyr Gly Val Gly
   65                  70                  75                  80
   Glu Ser Phe Thr
<210> 390
   <211> 89
   <212> PRT
   <213> Homo sapiens
<400> 390
   Arg Phe Leu Glu Tyr Ser Thr Gly Glu Cys Tyr Phe Phe Asn Gly Thr
   1               5                   10                  15
   Glu Arg Val Arg Phe Leu Asp Arg Tyr Phe Tyr Asn Gln Glu Glu Asp
               20                  25                  30
   Val Arg Phe Asp Ser Asp Val Gly Glu Tyr Arg Ala Val Thr Glu Leu
           35                  40                  45
   Gly Arg Pro Ser Ala Glu Tyr Trp Asn Ser Gln Lys Asp Phe Leu Glu
       50                  55                  60
   Asp Arg Arg Ala Leu Val Asp Thr Tyr Cys Arg His Asn Tyr Gly Val
   65                  70                  75                  80
   Gly Glu Ser Phe Thr Val Gln Arg Arg
                   85
<210> 391
   <211> 81
   <212> PRT
   <213> Homo sapiens
<400> 391
   Arg Phe Leu Glu Tyr Ser Thr Gly Glu Cys Tyr Phe Phe Asn Gly Thr
   1               5                   10                  15
   Glu Arg Val Arg Phe Leu Asp Arg Tyr Phe Tyr Asn Gln Glu Glu Tyr
               20                  25                  30
   Val Arg Phe Asp Ser Asp Val Gly Glu Phe Arg Ala Val Thr Glu Leu
           35                  40                  45
   Gly Arg Pro Ser Ala Glu Tyr Trp Asn Ser Gln Lys Asp Phe Leu Glu
       50                  55                  60
   Asp Arg Arg Ala Leu Val Asp Thr Tyr Cys Arg His Asn Tyr Gly Val
   65                  70                  75                  80
   Gly
<210> 392
   <211> 89
   <212> PRT
   <213> Homo sapiens
<400> 392
   Arg Phe Leu Glu Tyr Ser Thr Gly Glu Cys Tyr Phe Phe Asn Gly Thr
   1               5                   10                  15
   Glu Arg Val Arg Phe Leu Asp Arg Tyr Phe Tyr Asn Gln Glu Glu Tyr
               20                  25                  30
   Val Arg Phe Asp Ser Asp Val Gly Glu Tyr Arg Ala Val Thr Glu Leu
           35                  40                  45
   Gly Arg Pro Ser Ala Glu Tyr Trp Asn Ser Gln Lys Asp Ile Leu Glu
       50                  55                  60
   Asp Arg Arg Ala Ala Val Asp Thr Tyr Cys Arg His Asn Tyr Gly Val
   65                  70                  75                  80
   Gly Glu Ser Phe Thr Val Gln Arg Arg
                   85
<210> 393
   <211> 85
   <212> PRT
   <213> Homo sapiens
<400> 393
   Phe Leu Glu Tyr Ser Thr Gly Glu Cys Tyr Phe Phe Asn Gly Thr Glu
   1 5 10 15
   Arg Val Arg Phe Leu Asp Arg Tyr Phe Tyr Asn Gln Glu Glu Tyr Val
   20 25 30
   Arg Phe Asp Ser Asp Val Gly Glu Tyr Arg Ala Val Thr Glu Leu Gly
   35 40 45
   Arg Pro Ile Ala Glu Tyr Trp Asn Ser Gln Lys Asp Ile Leu Glu Asp
   50 55 60
   Arg Arg Ala Leu Val Asp Thr Tyr Cys Arg His Asn Tyr Gly Val Gly
   65 70 75 80
   Glu Ser Phe Thr Val
   85
<210> 394
   <211> 89
   <212> PRT
   <213> Homo sapiens
<400> 394
   Arg Phe Leu Glu Tyr Ser Thr Ser Glu Cys His Phe Phe Asn Gly Thr
   1 5 10 15
   Glu Arg Val Arg Phe Leu Asp Arg Tyr Phe Tyr Asn Gln Glu Glu Tyr
   20 25 30
   Val Arg Phe Asp Ser Asp Val Gly Glu Tyr Arg Ala Val Thr Glu Leu
   35 40 45
   Gly Arg Pro Asp Ala Glu Tyr Trp Asn Ser Gln Lys Asp Phe Leu Glu
   50 55 60
   Asp Arg Arg Ala Leu Val Asp Thr Tyr Cys Arg His Asn Tyr Gly Val
   65 70 75 80
   Val Glu Ser Phe Thr Val Gln Arg Arg
   85
<210> 395
   <211> 89
   <212> PRT
   <213> Homo sapiens
<400> 395
   Arg Phe Leu Glu Tyr Ser Met Gly Glu Cys Tyr Phe Phe Asn Gly Thr
   1               5                   10                  15
   Glu Arg Val Arg Phe Leu Asp Arg Tyr Phe His Asn Gln Glu Glu Phe
               20                  25                  30
   Val Arg Phe Asp Ser Asp Val Gly Glu Tyr Arg Ala Val Thr Glu Leu
           35                  40                  45
   Gly Arg Pro Asp Ala Glu Tyr Trp Asn Ser Gln Lys Asp Phe Leu Glu
       50                  55                  60
   Asp Arg Arg Ala Leu Val Asp Thr Tyr Cys Arg His Asn Tyr Gly Val
   65                  70                  75                  80
   Gly Glu Ser Phe Thr Val Gln Arg Arg
                   85
<210> 396
   <211> 89
   <212> PRT
   <213> Homo sapiens
<400> 396
   Arg Phe Leu Glu Tyr Ser Thr Gly Glu Cys Tyr Phe Phe Asn Gly Thr
   1               5                   10                  15
   Glu Arg Val Arg Phe Leu Asp Arg Tyr Phe Tyr Asn Gln Glu Glu Tyr
               20                  25                  30
   Val Arg Phe Asp Ser Asp Val Gly Glu Tyr Arg Ala Val Thr Glu Leu
           35                  40                  45
   Gly Arg Pro Ser Ala Glu Tyr Trp Asn Ser Gln Lys Asp Phe Leu Glu
       50                  55                  60
   Asp Arg Arg Ala Leu Val Asp Thr Tyr Cys Arg His Asn Tyr Gly Ala
   65                  70                  75                  80
   Val Glu Ser Phe Thr Val Gln Arg Arg
                   85
<210> 397
   <211> 89
   <212> PRT
   <213> Homo sapiens
<400> 397
   Arg Phe Leu Glu Tyr Ser Thr Gly Glu Cys Tyr Phe Phe Asn Gly Thr
   1 5 10 15
   Glu Arg Val Arg Phe Leu Asp Arg Tyr Phe Tyr Asn Gln Glu Glu Tyr
   20 25 30
   Val Arg Phe Asp Ser Asp Val Arg Glu Tyr Arg Ala Val Thr Glu Leu
   35 40 45
   Gly Arg Pro Ser Ala Glu Tyr Trp Asn Ser Gln Lys Asp Ile Leu Glu
   50 55 60
   Asp Arg Arg Ala Leu Val Asp Thr Tyr Cys Arg His Asn Tyr Gly Val
   65 70 75 80
   Gly Glu Ser Phe Thr Val Gln Arg Arg
   85
<210> 398
   <211> 88
   <212> PRT
   <213> Homo sapiens
<400> 398
   Arg Phe Leu Glu Tyr Ser Thr Gly Glu Cys Tyr Phe Phe Asn Gly Thr
   1 5 10 15
   Glu Arg Val Arg Phe Leu Asp Arg Tyr Phe Tyr Asn Gln Glu Glu Tyr
   20 25 30
   Val Arg Phe Asp Ser Asp Val Gly Glu Tyr Arg Ala Val Thr Glu Leu
   35 40 45
   Gly Arg Pro Asp Ala Glu Tyr Trp Asn Ser Gln Lys Asp Phe Leu Glu
   50 55 60
   Asp Arg Arg Ala Ala Val Asp Thr Tyr Cys Arg His Asn Tyr Gly Val
   65 70 75 80
   Gly Glu Ser Phe Thr Val Gln Arg
   85
<210> 399
   <211> 237
   <212> PRT
   <213> Homo sapiens
<400> 399
   Gly Asp Thr Gln Pro Arg Phe Leu Lys Gln Asp Lys Phe Glu Cys His
   1               5                   10                  15
   Phe Phe Asn Gly Thr Glu Arg Val Arg Tyr Leu His Arg Gly Ile Tyr
               20                  25                  30
   Asn Gln Glu Glu Asn Val Arg Phe Asp Ser Asp Val Gly Glu Tyr Arg
           35                  40                  45
   Ala Val Thr Glu Leu Gly Arg Pro Val Ala Glu Ser Trp Asn Ser Gln
       50                  55                  60
   Lys Asp Phe Leu Glu Arg Arg Arg Ala Glu Val Asp Thr Val Cys Arg
   65                  70                  75                  80
   His Asn Tyr Gly Val Gly Glu Ser Phe Thr Val Gln Arg Arg Val His
                   85                  90                  95
   Pro Glu Val Thr Val Tyr Pro Ala Lys Thr Gln Pro Leu Gln His His
               100                 105                 110
   Asn Leu Leu Val Cys Ser Val Ser Gly Phe Tyr Pro Gly Ser Ile Glu
           115                 120                 125
   Val Arg Trp Phe Arg Asn Gly Gln Glu Glu Lys Ala Gly Val Val Ser
       130                 135                 140
   Thr Gly Leu Ile Gln Asn Gly Asp Trp Thr Phe Gln Thr Leu Val Met
   145                 150                 155                 160
   Leu Glu Thr Val Pro Arg Ser Gly Glu Val Tyr Thr Cys Gln Val Glu
                   165                 170                 175
   His Pro Ser Val Met Ser Pro Leu Thr Val Glu Trp Arg Ala Arg Ser
               180                 185                 190
   Glu Ser Ala Gln Ser Lys Met Leu Ser Gly Val Gly Gly Phe Val Leu
           195                 200                 205
   Gly Leu Leu Phe Leu Gly Ala Gly Leu Phe Ile Tyr Phe Arg Asn Gln
       210                 215                 220
   Lys Gly His Ser Gly Leu Gln Pro Thr Gly Phe Leu Ser
   225                 230                 235
<210> 400
   <211> 266
   <212> PRT
   <213> Homo sapiens
<400> 400
<210> 401
   <211> 89
   <212> PRT
   <213> Homo sapiens
<400> 401
   Arg Phe Leu Glu Glu Val Lys Phe Glu Cys His Phe Phe Asn Gly Thr
   1 5 10 15
   Glu Arg Val Arg Leu Leu Glu Arg Arg Val His Asn Gln Glu Glu Tyr
   20 25 30
   Ala Arg Tyr Asp Ser Asp Val Gly Glu Tyr Arg Ala Val Thr Glu Leu
   35 40 45
   Gly Arg Pro Asp Ala Glu Tyr Trp Asn Ser Gln Lys Asp Leu Leu Glu
   50 55 60
   Arg Arg Arg Ala Ala Val Asp Thr Tyr Cys Arg His Asn Tyr Gly Val
   65 70 75 80
   Gly Glu Ser Phe Thr Val Gln Arg Arg
   85
<210> 402
   <211> 266
   <212> PRT
   <213> Homo sapiens
<400> 402

<210> 403
   <211> 101
   <212> PRT
   <213> Homo sapiens
<400> 403
   Gly Asp Thr Arg Pro Arg Phe Leu Glu Tyr Ser Thr Ser Glu Cys His
   1               5                   10                  15
   Phe Phe Asn Gly Thr Glu Arg Val Arg Phe Leu Asp Arg Tyr Phe Tyr
               20                  25                  30
   Asn Gln Glu Glu Tyr Val Arg Phe Asp Ser Asp Val Gly Glu Phe Arg
           35                  40                  45
   Ala Val Thr Glu Leu Gly Arg Pro Asp Glu Glu Tyr Trp Asn Ser Gln
       50                  55                  60
   Lys Asp Phe Leu Glu Asp Arg Arg Ala Ala Val Asp Thr Tyr Cys Arg
   65                  70                  75                  80
   His Asn Tyr Gly Val Gly Glu Ser Phe Thr Val Gln Arg Arg Val His
                   85                  90                  95
   Pro Lys Val Thr Val
               100
<210> 404
   <211> 89
   <212> PRT
   <213> Homo sapiens
<400> 404
   Arg Phe Leu Glu Tyr Ser Thr Ser Glu Cys His Phe Phe Asn Gly Thr
   1               5                   10                  15
   Glu Arg Val Arg Phe Leu Asp Arg Tyr Phe Tyr Asn Gln Glu Glu Tyr
               20                  25                  30
   Val Arg Phe Asp Ser Asp Val Gly Glu Phe Arg Ala Val Thr Glu Leu
           35                  40                  45
   Gly Arg Pro Asp Glu Glu Tyr Trp Asn Ser Gln Lys Asp Phe Leu Glu
       50                  55                  60
   Asp Arg Arg Ala Ala Val Asp Thr Tyr Cys Arg His Asn Tyr Gly Val
   65                  70                  75                  80
   Gly Glu Ser Phe Thr Val Gln Arg Arg
                   85
<210> 405
   <211> 89
   <212> PRT
   <213> Homo sapiens
<400> 405
   Arg Phe Leu Glu Tyr Ser Thr Ser Glu Cys His Phe Phe Asn Gly Thr
   1               5                   10                  15
   Glu Arg Val Arg Phe Leu Asp Arg Tyr Phe Tyr Asn Gln Glu Glu Tyr
               20                  25                  30
   Val Arg Phe Asp Ser Asp Val Gly Glu Phe Arg Ala Val Thr Glu Leu
           35                  40                  45
   Gly Arg Pro Asp Glu Glu Tyr Trp Asn Ser Gln Lys Asp Phe Leu Glu
       50                  55                  60
   Asp Arg Arg Ala Ala Val Asp Thr Tyr Cys Arg His Asn Tyr Gly Val
   65                  70                  75                  80
   Gly Glu Ser Phe Thr Val Gln Arg Arg
                   85
<210> 406
   <211> 237
   <212> PRT
   <213> Homo sapiens
<400> 406
   Gly Asp Thr Arg Pro Arg Phe Leu Glu Tyr Ser Thr Ser Glu Cys His
   1 5 10 15
   Phe Phe Asn Gly Thr Glu Arg Val Arg Phe Leu Asp Arg Tyr Phe Tyr
   20 25 30
   Asn Gln Glu Glu Tyr Val Arg Phe Asp Ser Asp Val Gly Glu Phe Arg
   35 40 45
   Ala Val Thr Glu Leu Gly Arg Pro Asp Glu Glu Tyr Trp Asn Ser Gln
   50 55 60
   Lys Asp Ile Leu Glu Asp Glu Arg Ala Ala Val Asp Thr Tyr Cys Arg
   65 70 75 80
   His Asn Tyr Gly Val Val Glu Ser Phe Thr Val Gln Arg Arg Val His
   85 90 95
   Pro Lys Val Thr Val Tyr Pro Ser Lys Thr Gln Pro Leu Gln His His
   100 105 110
   Asn Leu Leu Val Cys Ser Val Ser Gly Phe Tyr Pro Gly Ser Ile Glu
   115 120 125
   Val Arg Trp Phe Arg Asn Gly Gln Glu Glu Lys Thr Gly Val Val Ser
   130 135 140
   Thr Gly Leu Ile His Asn Gly Asp Trp Thr Phe Gln Thr Leu Val Met
   145 150 155 160
   Leu Glu Thr Phe Pro Arg Ser Gly Glu Val Tyr Thr Cys Gln Val Glu
   165 170 175
   His Pro Ser Val Thr Ser Pro Leu Thr Val Glu Trp Arg Ala Arg Ser
   180 185 190
   Glu Ser Ala Gln Ser Lys Met Leu Ser Gly Val Gly Gly Phe Val Leu
   195 200 205
   Gly Leu Leu Phe Leu Gly Ala Gly Leu Phe Ile Tyr Phe Arg Asn Gln
   210 215 220
   Lys Gly His Ser Gly Leu Gln Pro Arg Gly Phe Leu Ser
   225 230 235
<210> 407
   <211> 266
   <212> PRT
   <213> Homo sapiens
<400> 407
   Met Val Cys Leu Arg Leu Pro Gly Gly Ser Cys Met Ala Val Leu Thr
   1               5                   10                  15
   Val Thr Leu Met Val Leu Ser Ser Pro Leu Ala Leu Ala Gly Asp Thr
               20                  25                  30
   Arg Pro Arg Phe Leu Glu Tyr Ser Thr Ser Glu Cys His Phe Phe Asn
           35                  40                  45
   Gly Thr Glu Arg Val Arg Phe Leu Asp Arg Tyr Phe Tyr Asn Gln Glu
       50                  55                  60
   Glu Tyr Val Arg Phe Asp Ser Asp Val Gly Glu Phe Arg Ala Val Thr
   65                  70                  75                  80
   Glu Leu Gly Arg Pro Asp Glu Glu Tyr Trp Asn Ser Gln Lys Asp Phe
                   85                  90                  95
   Leu Glu Asp Glu Arg Ala Ala Val Asp Thr Tyr Cys Arg His Asn Tyr
               100                 105                 110
   Gly Val Val Glu Ser Phe Thr Val Gln Arg Arg Val His Pro Lys Val
           115                 120                 125
   Thr Val Tyr Pro Ser Lys Thr Gln Pro Leu Gln His His Asn Leu Leu
       130                 135                 140
   Val Cys Ser Val Ser Gly Phe Tyr Pro Gly Ser Ile Glu Val Arg Trp
   145                 150                 155                 160
   Phe Arg Asn Gly Gln Glu Glu Lys Thr Gly Val Val Ser Thr Gly Leu
                   165                 170                 175
   Ile His Asn Gly Asp Trp Thr Phe Gln Thr Leu Val Met Leu Glu Thr
               180                 185                 190
   Val Pro Arg Ser Gly Glu Val Tyr Thr Cys Gln Val Glu His Pro Ser
           195                 200                 205
   Val Thr Ser Pro Leu Thr Val Glu Trp Arg Ala Arg Ser Glu Ser Ala
       210                 215                 220
   Gln Ser Lys Met Leu Ser Gly Val Gly Gly Phe Val Leu Gly Leu Leu
   225                 230                 235                 240
   Phe Leu Gly Ala Gly Leu Phe Ile Tyr Phe Arg Asn Gln Lys Gly His
                   245                 250                 255
   Ser Gly Leu Gln Pro Arg Gly Phe Leu Ser
               260                 265
<210> 408
   <211> 94
   <212> PRT
   <213> Homo sapiens
<400> 408
   Gly Asp Thr Arg Pro Arg Phe Leu Glu Tyr Ser Thr Ser Glu Cys His
   1               5                   10                  15
   Phe Phe Asn Gly Thr Glu Arg Val Arg Phe Leu Asp Arg Tyr Phe Tyr
               20                  25                  30
   Asn Gln Glu Glu Tyr Val Arg Phe Asp Ser Asp Val Gly Glu Phe Arg
           35                  40                  45
   Ala Val Thr Glu Leu Gly Arg Pro Asp Glu Glu Tyr Trp Asn Ser Gln
       50                  55                  60
   Lys Asp Phe Leu Glu Asp Arg Arg Ala Ala Val Asp Thr Tyr Cys Arg
   65                  70                  75                  80
   His Asn Tyr Gly Val Val Glu Ser Phe Thr Val Gln Arg Arg
                   85                  90
<210> 409
   <211> 103
   <212> PRT
   <213> Homo sapiens
<400> 409
   Gly Asp Thr Arg Pro Arg Phe Leu Glu Tyr Ser Thr Ser Glu Cys His
   1               5                   10                  15
   Phe Phe Asn Gly Thr Glu Arg Val Arg Phe Leu Asp Arg Tyr Phe Tyr
               20                  25                  30
   Asn Gln Glu Glu Tyr Val Arg Phe Asp Ser Asp Val Gly Glu Phe Arg
           35                  40                  45
   Ala Val Thr Glu Leu Gly Arg Pro Asp Glu Glu Tyr Trp Asn Ser Gln
       50                  55                  60
   Lys Asp Phe Leu Glu Asp Arg Arg Ala Ala Val Asp Thr Tyr Cys Arg
   65                  70                  75                  80
   His Asn Tyr Gly Val Val Glu Ser Phe Thr Val Gln Arg Arg Val His
                   85                  90                  95
   Pro Lys Val Thr Val Tyr Pro
               100
<210> 410
   <211> 90
   <212> PRT
   <213> Homo sapiens
<400> 410
   Pro Arg Phe Leu Glu Tyr Ser Thr Gly Glu Cys His Phe Phe Asn Gly
   1               5                   10                  15
   Thr Glu Arg Val Arg Phe Leu Asp Arg Tyr Phe Tyr Asn Gln Glu Glu
               20                  25                  30
   Tyr Val Arg Phe Asp Ser Asp Val Gly Glu Phe Arg Ala Val Thr Glu
           35                  40                  45
   Leu Gly Arg Pro Asp Glu Glu Tyr Trp Asn Ser Gln Lys Asp Phe Leu
       50                  55                  60
   Glu Asp Arg Arg Ala Ala Val Asp Thr Tyr Cys Arg His Asn Tyr Gly
   65                  70                  75                  80
   Val Gly Glu Ser Phe Thr Val Gln Arg Arg
                   85                  90
<210> 411
   <211> 85
   <212> PRT
   <213> Homo sapiens
<400> 411
   Arg Phe Leu Glu Tyr Ser Thr Ser Glu Cys His Phe Phe Asn Gly Thr
   1               5                   10                  15
   Glu Arg Val Arg Phe Leu Asp Arg Tyr Phe Tyr Asn Gln Glu Glu Tyr
               20                  25                  30
   Val Arg Phe Asp Ser Asp Val Gly Glu Phe Arg Ala Val Thr Glu Leu
           35                  40                  45
   Gly Arg Pro Asp Glu Glu Tyr Trp Asn Ser Gln Lys Asp Phe Leu Glu
       50                  55                  60
   Asp Arg Arg Ala Ala Val Asp Thr Tyr Cys Arg His Asn Tyr Gly Ala
   65                  70                  75                  80
   Val Glu Ser Phe Thr
                   85
<210> 412
   <211> 87
   <212> PRT
   <213> Homo sapiens
<400> 412
   Leu Glu Tyr Ser Thr Ser Glu Cys His Phe Phe Asn Gly Thr Glu Arg
   1               5                   10                  15
   Val Arg Phe Leu Asp Arg Tyr Phe Tyr Asn Gln Glu Glu Tyr Val Arg
               20                  25                  30
   Phe Asp Ser Asp Val Gly Glu Phe Arg Ala Val Thr Glu Leu Gly Arg
           35                  40                  45
   Pro Asp Glu Glu Tyr Trp Asn Ser Gln Lys Asp Leu Leu Glu Gln Lys
       50                  55                  60
   Arg Gly Arg Val Asp Asn Tyr Cys Arg His Asn Tyr Gly Val Val Glu
   65                  70                  75                  80
   Ser Phe Thr Val Gln Arg Arg
                   85
<210> 413
   <211> 79
   <212> PRT
   <213> Homo sapiens
<400> 413
   Ser Glu Cys His Phe Phe Asn Gly Thr Glu Arg Val Arg Phe Leu Asp
   1               5                   10                  15
   Arg Tyr Phe Tyr Asn Gln Glu Glu Tyr Val Arg Phe Asp Ser Asp Val
               20                  25                  30
   Gly Glu Phe Arg Ala Val Thr Glu Leu Gly Arg Pro Asp Glu Glu Tyr
           35                  40                  45
   Trp Asn Ser Gln Lys Asp Leu Leu Glu Asp Arg Arg Ala Ala Val Asp
       50                  55                  60
   Thr Tyr Cys Arg His Asn Tyr Gly Val Gly Glu Ser Phe Thr Val
   65                  70                  75
<210> 414
   <211> 79
   <212> PRT
   <213> Homo sapiens
<400> 414
   Ser Glu Cys His Phe Phe Asn Gly Thr Glu Arg Val Arg Phe Leu Asp
   1 5 10 15
   Arg Tyr Phe Tyr Asn Gln Glu Glu Tyr Val Arg Phe Asp Ser Asp Val
   20 25 30
   Gly Glu Phe Arg Ala Val Thr Glu Leu Gly Arg Pro Asp Glu Glu Tyr
   35 40 45
   Trp Asn Ser Gln Lys Asp Leu Leu Glu Asp Arg Arg Ala Ala Val Asp
   50 55 60
   Thr Tyr Cys Arg His Asn Tyr Gly Val Gly Glu Ser Phe Thr Val
   65 70 75
<210> 415
   <211> 77
   <212> PRT
   <213> Homo sapiens
<400> 415
   His Phe Phe Asn Gly Thr Glu Arg Val Arg Phe Leu Asp Arg Tyr Phe
   1 5 10 15
   His Asn Gln Glu Glu Asn Val Arg Phe Asp Ser Asp Val Gly Glu Phe
   20 25 30
   Arg Ala Val Thr Glu Leu Gly Arg Pro Asp Glu Glu Tyr Trp Asn Ser
   35 40 45
   Gln Lys Asp Phe Leu Glu Asp Arg Arg Ala Ala Val Asp Thr Tyr Cys
   50 55 60
   Arg His Asn Tyr Gly Val Gly Glu Ser Phe Thr Val Gln
   65 70 75
<210> 416
   <211> 73
   <212> PRT
   <213> Homo sapiens
<400> 416
   Glu Cys His Phe Phe Asn Gly Thr Glu Arg Val Arg Phe Leu Asp Arg
   1               5                   10                  15
   Tyr Phe His Asn Gln Glu Glu Phe Val Arg Phe Asp Ser Asp Val Gly
               20                  25                  30
   Glu Phe Arg Ala Val Thr Glu Leu Gly Arg Pro Asp Glu Glu Tyr Trp
           35                  40                  45
   Asn Ser Gln Lys Asp Phe Leu Glu Asp Arg Arg Ala Ala Val Asp Thr
       50                  55                  60
   Tyr Cys Arg His Asn Tyr Gly Val Gly
   65                  70
<210> 417
   <211> 79
   <212> PRT
   <213> Homo sapiens
<400> 417
   Ser Glu Cys His Phe Phe Asn Gly Thr Glu Arg Val Arg Phe Leu Asp
   1               5                   10                  15
   Arg Tyr Phe Tyr Asn Gln Glu Glu Tyr Val Arg Phe Asp Ser Asp Val
               20                  25                  30
   Gly Glu Phe Arg Ala Val Thr Glu Leu Gly Arg Pro Asp Glu Glu Tyr
           35                  40                  45
   Trp Asn Ser Gln Lys Asp Phe Leu Glu Asp Glu Arg Ala Ala Val Asp
       50                  55                  60
   Thr Tyr Cys Arg His Asn Tyr Gly Val Gly Glu Ser Phe Thr Val
   65                  70                  75
<210> 418
   <211> 73
   <212> PRT
   <213> Homo sapiens
<400> 418
   Glu Cys His Phe Phe Asn Gly Thr Glu Arg Val Arg Phe Leu Asp Arg
   1               5                   10                  15
   Tyr Phe Tyr Asn Gln Glu Glu Phe Val Arg Phe Asp Ser Asp Val Gly
               20                  25                  30
   Glu Phe Arg Ala Val Thr Glu Leu Gly Arg Pro Asp Glu Glu Tyr Trp
           35                  40                  45
   Asn Ser Gln Lys Asp Phe Leu Glu Asp Arg Arg Ala Ala Val Asp Thr
       50                  55                  60
   Tyr Cys Arg His Asn Tyr Gly Val Gly
   65                  70
<210> 419
   <211> 89
   <212> PRT
   <213> Homo sapiens
<400> 419
   Arg Phe Leu Glu Tyr Ser Thr Ser Glu Cys His Phe Phe Asn Gly Thr
   1               5                   10                  15
   Glu Arg Val Arg Phe Leu Asp Arg Tyr Phe Tyr Asn Gln Glu Glu Phe
               20                  25                  30
   Val Arg Phe Asp Ser Asp Val Gly Glu Phe Arg Ala Val Thr Glu Leu
           35                  40                  45
   Gly Arg Pro Asp Glu Glu Tyr Trp Asn Ser Gln Lys Asp Phe Leu Glu
       50                  55                  60
   Asp Arg Arg Ala Ala Val Asp Thr Tyr Cys Arg His Asn Tyr Gly Val
   65                  70                  75                  80
   Gly Glu Ser Phe Thr Val Gln Arg Arg
                   85
<210> 420
   <211> 94
   <212> PRT
   <213> Homo sapiens
<400> 420
   Gly Asp Thr Arg Pro Arg Phe Leu Glu Tyr Ser Thr Ser Glu Cys His
   1               5                   10                  15
   Phe Phe Asn Gly Thr Glu Arg Val Arg Phe Leu Asp Arg Tyr Phe His
   20 25 30
   Asn Gln Glu Glu Phe Val Arg Phe Asp Ser Asp Val Gly Glu Phe Arg
   35 40 45
   Ala Val Thr Glu Leu Gly Arg Pro Asp Glu Glu Tyr Trp Asn Ser Gln
   50 55 60
   Lys Asp Leu Leu Glu Arg Arg Arg Ala Ala Val Asp Thr Tyr Cys Arg
   65 70 75 80
   His Asn Tyr Gly Val Val Glu Ser Phe Thr Val Gln Arg Arg
   85 90
<210> 421
   <211> 93
   <212> PRT
   <213> Homo sapiens
<400> 421
   Gly Asp Thr Arg Pro Arg Phe Leu Glu Tyr Ser Thr Ser Glu Cys His
   1 5 10 15
   Phe Phe Asn Gly Thr Glu Arg Val Arg Phe Leu Asp Arg Tyr Phe Tyr
   20 25 30
   Asn Gln Glu Glu Tyr Val Arg Phe Asp Ser Asp Val Gly Glu Phe Arg
   35 40 45
   Ala Val Thr Glu Leu Gly Arg Pro Asp Glu Glu Tyr Trp Asn Ser Gln
   50 55 60
   Lys Asp Ile Leu Glu Asp Glu Arg Ala Ala Val Asp Thr Tyr Cys Arg
   65 70 75 80
   His Asn Tyr Gly Val Gly Glu Ser Phe Thr Val Gln Arg
   85 90
<210> 422
   <211> 112
   <212> PRT
   <213> Homo sapiens
<400> 422
   Gly Asp Thr Arg Pro Arg Phe Leu Glu Tyr Ser Thr Ser Glu Cys His
   1 5 10 15
   Phe Phe Asn Gly Thr Glu Arg Val Arg Phe Leu Asp Arg Tyr Phe Tyr
   20 25 30
   Asn Gln Glu Glu Asp Leu Arg Phe Asp Ser Asp Val Gly Glu Phe Arg
   35 40 45
   Ala Val Thr Glu Leu Gly Arg Pro Asp Glu Glu Tyr Trp Asn Ser Gln
   50 55 60
   Lys Asp Phe Leu Glu Asp Arg Arg Ala Ala Val Asp Thr Tyr Cys Arg
   65 70 75 80
   His Asn Tyr Gly Val Gly Glu Ser Phe Thr Val Gln Arg Arg Val His
   85 90 95
   Pro Lys Val Thr Val Tyr Pro Ser Lys Thr Gln Pro Leu Gln His His
   100 105 110

<210> 423
   <211> 89
   <212> PRT
   <213> Homo sapiens
<400> 423
   Arg Phe Leu Glu Tyr Ser Thr Ser Glu Cys His Phe Phe Asn Gly Thr
   1 5 10 15
   Glu Arg Val Arg Phe Leu Asp Arg Tyr Phe His Asn Gln Glu Glu Asn
   20 25 30
   Val Arg Phe Asp Ser Asp Val Gly Glu Phe Arg Ala Val Thr Glu Leu
   35 40 45
   Gly Arg Pro Asp Glu Glu Tyr Trp Asn Ser Gln Lys Asp Ile Leu Glu
   50 55 60
   Asp Glu Arg Ala Ala Val Asp Thr Tyr Cys Arg His Asn Tyr Gly Val
   65 70 75 80
   Val Glu Ser Phe Thr Val Gln Arg Arg
   85
<210> 424
   <211> 112
   <212> PRT
   <213> Homo sapiens
<400> 424
   Gly Asp Thr Arg Pro Arg Phe Leu Glu Tyr Ser Thr Ser Glu Cys His
   1               5                   10                  15
   Phe Phe Asn Gly Thr Glu Arg Val Arg Phe Leu Asp Arg Tyr Phe His
               20                  25                  30
   Asn Gln Glu Glu Phe Val Arg Phe Asp Ser Asp Val Gly Glu Tyr Arg
           35                  40                  45
   Ala Val Thr Glu Leu Gly Arg Pro Asp Glu Glu Tyr Trp Asn Ser Gln
       50                  55                  60
   Lys Asp Leu Leu Glu Arg Arg Arg Ala Glu Val Asp Thr Tyr Cys Arg
   65                  70                  75                  80
   His Asn Tyr Gly Val Val Glu Ser Phe Thr Val Gln Arg Arg Val His
                   85                  90                  95
   Pro Lys Val Thr Val Tyr Pro Ser Lys Thr Gln Pro Leu Gln His His
               100                 105                 110
<210> 425
   <211> 88
   <212> PRT
   <213> Homo sapiens
<400> 425
   Phe Leu Glu Tyr Ser Thr Ser Glu Cys His Phe Phe Asn Gly Thr Glu
   1               5                   10                  15
   Arg Val Arg Phe Leu Asp Arg Tyr Phe Tyr Asn Gln Glu Glu Tyr Val
               20                  25                  30
   Arg Phe Asp Ser Asp Val Gly Glu Phe Arg Ala Val Thr Glu Leu Gly
           35                  40                  45
   Arg Pro Asp Glu Glu Tyr Trp Asn Ser Gln Lys Asp Ile Leu Glu Asp
       50                  55                  60
   Arg Arg Ala Ala Val Asp Thr Tyr Cys Arg His Asn Tyr Gly Val Val
   65                  70                  75                  80
   Glu Ser Phe Thr Val Gln Arg Arg
                   85
<210> 426
   <211> 88
   <212> PRT
   <213> Homo sapiens
<400> 426
   Phe Leu Glu Tyr Ser Thr Ser Glu Cys His Phe Phe Asn Gly Thr Glu
   1               5                   10                  15
   Arg Val Arg Phe Leu Asp Arg Tyr Phe Tyr Asn Gln Glu Glu Tyr Val
               20                  25                  30
   Arg Phe Asp Ser Asp Val Gly Glu Phe Arg Ala Val Thr Glu Leu Gly
           35                  40                  45
   Arg Pro Asp Glu Glu Tyr Trp Asn Ser Gln Lys Asp Ile Leu Glu Asp
       50                  55                  60
   Arg Arg Ala Ala Val Asp Thr Tyr Cys Arg His Asn Tyr Gly Val Gly
   65                  70                  75                  80
   Glu Ser Phe Thr Val Gln Arg Arg
                   85
<210> 427
   <211> 85
   <212> PRT
   <213> Homo sapiens
<400> 427
   Leu Glu Tyr Ser Thr Ser Glu Cys His Phe Phe Asn Gly Thr Glu Arg
   1               5                   10                  15
   Val Arg Phe Leu Asp Arg Tyr Phe His Asn Gln Glu Glu Asn Val Arg
               20                  25                  30
   Phe Asp Ser Asp Val Gly Glu Phe Arg Ala Val Thr Glu Leu Gly Arg
           35                  40                  45
   Pro Asp Glu Glu Tyr Trp Asn Ser Gln Lys Asp Ile Leu Glu Asp Glu
       50                  55                  60
   Arg Ala Ala Val Asp Thr Tyr Cys Arg His Asn Tyr Gly Val Gly Glu
   65                  70                  75                  80
   Ser Phe Thr Val Gln
                   85
<210> 428
   <211> 80
   <212> PRT
   <213> Homo sapiens
<400> 428
   Leu Glu Tyr Ser Thr Ser Glu Cys His Phe Phe Asn Gly Thr Glu Arg
   1               5                   10                  15
   Val Arg Phe Leu Asp Arg Tyr Phe Tyr Asn Gln Glu Glu Tyr Val Arg
               20                  25                  30
   Phe Asp Ser Asp Val Gly Glu Phe Arg Ala Val Thr Glu Leu Gly Arg
           35                  40                  45
   Pro Asp Glu Glu Tyr Trp Asn Ser Gln Lys Asp Ile Leu Glu Asp Glu
       50                  55                  60
   Arg Ala Ala Val Asp Thr Tyr Cys Arg His Asn Tyr Gly Ala Val Glu
   65                  70                  75                  80
<210> 429
   <211> 82
   <212> PRT
   <213> Homo sapiens
<400> 429
   Arg Phe Leu Glu Gln Val Lys His Glu Cys His Phe Phe Asn Gly Thr
   1               5                   10                  15
   Glu Arg Val Arg Phe Leu Asp Arg Tyr Phe Tyr Asn Gln Glu Glu Tyr
               20                  25                  30
   Val Arg Phe Asp Ser Asp Val Gly Glu Phe Arg Ala Val Thr Glu Leu
           35                  40                  45
   Gly Arg Pro Asp Glu Glu Tyr Trp Asn Ser Gln Lys Asp Phe Leu Glu
       50                  55                  60
   Asp Arg Arg Ala Ala Val Asp Thr Tyr Cys Arg His Asn Tyr Gly Val
   65                  70                  75                  80
   Gly Glu
<210> 430
   <211> 82
   <212> PRT
   <213> Homo sapiens
<400> 430
   Pro Arg Phe Leu Glu Tyr Ser Thr Ser Glu Cys His Phe Phe Asn Gly
   1 5 10 15
   Thr Glu Arg Val Arg Phe Leu Asp Arg Tyr Phe Tyr Asn Gln Glu Glu
   20 25 30
   Tyr Val Arg Phe Asp Ser Asp Val Gly Glu Phe Arg Ala Val Thr Glu
   35 40 45
   Leu Gly Arg Pro Asp Glu Glu Tyr Trp Asn Ser Gln Lys Asp Phe Leu
   50 55 60
   Glu Asp Arg Arg Ala Leu Val Asp Thr Tyr Cys Arg His Asn Tyr Gly
   65 70 75 80
   Val Gly
<210> 431
   <211> 83
   <212> PRT
   <213> Homo sapiens
<400> 431
   Phe Leu Glu Tyr Ser Thr Ser Glu Cys His Phe Phe Asn Gly Thr Glu
   1 5 10 15
   Arg Val Arg Phe Leu Asp Arg Tyr Phe Tyr Asn Gln Glu Glu Asp Val
   20 25 30
   Arg Phe Asp Ser Asp Val Gly Glu Phe Arg Ala Val Thr Glu Leu Gly
   35 40 45
   Arg Pro Asp Glu Glu Tyr Trp Asn Ser Gln Lys Asp Phe Leu Glu Asp
   50 55 60
   Arg Arg Ala Ala Val Asp Thr Tyr Cys Arg His Asn Tyr Gly Val Gly
   65 70 75 80
   Glu Ser Phe
<210> 432
   <211> 89
   <212> PRT
   <213> Homo sapiens
<400> 432
   Arg Phe Leu Glu Tyr Ser Thr Ser Glu Cys His Phe Phe Asn Gly Thr
   1 5 10 15
   Glu Arg Val Arg Phe Leu Asp Arg Tyr Phe Tyr Asn Gln Glu Glu Tyr
   20 25 30
   Val Arg Phe Asp Ser Asp Val Gly Glu Phe Arg Ala Val Thr Glu Leu
   35 40 45
   Gly Arg Pro Asp Glu Glu Tyr Trp Asn Ser Gln Lys Asp Phe Leu Glu
   50 55 60
   Asp Arg Arg Ala Leu Val Asp Thr Tyr Cys Arg His Asn Tyr Gly Val
   65 70 75 80
   Val Glu Ser Phe Thr Val Gln Arg Arg
   85
<210> 433
   <211> 80
   <212> PRT
   <213> Homo sapiens
<400> 433
   Leu Glu Tyr Ser Thr Ser Glu Cys His Phe Phe Asn Gly Thr Glu Arg
   1 5 10 15
   Val Arg Phe Leu Asp Arg Tyr Phe Tyr Asn Gln Glu Glu Tyr Val Arg
   20 25 30
   Phe Asp Ser Asp Val Gly Glu Phe Arg Ala Val Thr Glu Leu Gly Arg
   35 40 45
   Pro Asp Glu Glu Tyr Trp Asn Ser Gln Lys Asp Leu Leu Glu Gln Arg
   50 55 60
   Arg Ala Ala Val Asp Thr Tyr Cys Arg His Asn Tyr Gly Val Gly Glu
   65 70 75 80
<210> 434
   <211> 81
   <212> PRT
   <213> Homo sapiens
<400> 434
   Phe Leu Glu Tyr Ser Thr Ser Glu Cys His Phe Phe Asn Gly Thr Glu
   1 5 10 15
   Arg Val Arg Phe Leu Asp Arg Tyr Phe Tyr Asn Gln Glu Glu Tyr Val
   20 25 30
   Arg Phe Asp Ser Asp Val Gly Glu Phe Arg Ala Val Thr Glu Leu Gly
   35 40 45
   Arg Pro Asp Glu Glu Tyr Trp Asn Ser Gln Lys Asp Phe Leu Glu Asp
   50 55 60
   Arg Arg Ala Ala Val Asp Asn Tyr Cys Arg His Asn Tyr Gly Val Gly
   65 70 75 80
   Glu
<210> 435
   <211> 89
   <212> PRT
   <213> Homo sapiens
<400> 435
   Arg Phe Leu Glu Tyr Ser Thr Ser Glu Cys His Phe Phe Asn Gly Thr
   1 5 10 15
   Glu Arg Val Arg Phe Leu Asp Arg Tyr Phe Tyr Asn Gln Glu Glu Tyr
   20 25 30
   Val Arg Phe Asp Ser Asp Val Gly Glu Phe Arg Ala Val Thr Glu Leu
   35 40 45
   Gly Arg Pro Asp Glu Glu Tyr Trp Asn Ser Gln Lys Asp Phe Leu Glu
   50 55 60
   Asp Arg Arg Ala Ala Val Asp Asn Tyr Cys Arg His Asn Tyr Gly Val
   65 70 75 80
   Gly Glu Ser Phe Thr Val Gln Arg Arg
   85
<210> 436
   <211> 89
   <212> PRT
   <213> Homo sapiens
<400> 436
   Arg Phe Leu Glu Tyr Ser Thr Ser Glu Cys His Phe Phe Asn Gly Thr
   1               5                   10                  15
   Glu Arg Val Arg Phe Leu Asp Arg Tyr Phe Tyr Asn Gln Glu Glu Asn
               20                  25                  30
   Val Arg Phe Asp Ser Asp Val Gly Glu Phe Arg Ala Val Thr Glu Leu
           35                  40                  45
   Gly Arg Pro Asp Glu Glu Tyr Trp Asn Ser Gln Lys Asp Phe Leu Glu
       50                  55                  60
   Asp Arg Arg Ala Ala Val Asp Thr Tyr Cys Arg His Asn Tyr Gly Val
   65                  70                  75                  80
   Gly Glu Ser Phe Thr Val Gln Arg Arg
                   85
<210> 437
   <211> 89
   <212> PRT
   <213> Homo sapiens
<400> 437
   Arg Phe Leu Glu Tyr Ser Thr Ser Glu Cys His Phe Phe Asn Gly Thr
   1               5                   10                  15
   Glu Arg Val Arg Phe Leu Asp Arg Tyr Phe Tyr Asn Gln Glu Glu Ser
               20                  25                  30
   Val Arg Phe Asp Ser Asp Val Gly Glu Phe Arg Ala Val Thr Glu Leu
           35                  40                  45
   Gly Arg Pro Asp Glu Glu Tyr Trp Asn Ser Gln Lys Asp Phe Leu Glu
       50                  55                  60
   Asp Arg Arg Ala Ala Val Asp Thr Tyr Cys Arg His Asn Tyr Gly Val
   65                  70                  75                  80
   Gly Glu Ser Phe Thr Val Gln Arg Arg
                   85
<210> 438
   <211> 89
   <212> PRT
   <213> Homo sapiens
<400> 438
   Arg Phe Leu Glu Leu Leu Lys Ser Glu Cys His Phe Phe Asn Gly Thr
   1               5                   10                  15
   Glu Arg Val Arg Phe Leu Asp Arg Tyr Phe Tyr Asn Gln Glu Glu Tyr
               20                  25                  30
   Val Arg Phe Asp Ser Asp Val Gly Glu Phe Arg Ala Val Thr Glu Leu
           35                  40                  45
   Gly Arg Pro Asp Glu Glu Tyr Trp Asn Ser Gln Lys Asp Phe Leu Glu
       50                  55                  60
   Asp Arg Arg Ala Ala Val Asp Thr Tyr Cys Arg His Asn Tyr Gly Val
   65                  70                  75                  80
   Gly Glu Ser Phe Thr Val Gln Arg Arg
                   85
<210> 439
   <211> 120
   <212> PRT
   <213> Homo sapiens
<400> 439
   Gly Asp Thr Arg Pro Arg Phe Leu Glu Tyr Ser Thr Ser Glu Cys His
   1               5                   10                  15
   Phe Phe Asn Gly Thr Glu Arg Val Arg Phe Leu Asp Arg Tyr Phe Tyr
               20                  25                  30
   Asn Gln Glu Glu Tyr Val Arg Phe Asp Ser Asp Val Gly Glu Phe Arg
           35                  40                  45
   Ala Val Thr Glu Leu Gly Arg Pro Asp Glu Glu His Trp Asn Ser Gln
       50                  55                  60
   Lys Asp Ile Leu Glu Asp Arg Arg Ala Ala Val Asp Thr Tyr Cys Arg
   65                  70                  75                  80
   His Asn Tyr Gly Val Gly Glu Ser Phe Thr Val Gln Arg Arg Val His
                   85                  90                  95
   Pro Lys Val Thr Val Tyr Pro Ser Lys Thr Gln Pro Leu Gln His His
               100                 105                 110
   Asn Leu Leu Val Cys Ser Val Ser
           115                 120
<210> 440
   <211> 89
   <212> PRT
   <213> Homo sapiens
<400> 440
   Arg Phe Leu Glu Tyr Ser Thr Ser Glu Cys His Phe Phe Asn Gly Thr
   1               5                   10                  15
   Glu Arg Val Arg Phe Leu Asp Arg Tyr Phe Tyr Asn Gln Glu Glu Tyr
               20                  25                  30
   Val Arg Phe Asp Ser Asp Val Gly Glu Phe Arg Ala Val Thr Glu Leu
           35                  40                  45
   Gly Arg Pro Asp Glu Glu Tyr Trp Asn Ser Gln Lys Asp Phe Leu Glu
       50                  55                  60
   Asp Arg Arg Ala Val Val Asp Thr Tyr Cys Arg His Asn Tyr Gly Val
   65                  70                  75                  80
   Gly Glu Ser Phe Thr Val Gln Arg Arg
                   85
<210> 441
   <211> 86
   <212> PRT
   <213> Homo sapiens
<400> 441
   Leu Glu Tyr Ser Thr Ser Glu Cys His Phe Phe Asn Gly Thr Glu Arg
   1               5                   10                  15
   Val Arg Phe Leu Asp Arg Tyr Phe Tyr Asn Gln Glu Glu Tyr Val Arg
               20                  25                  30
   Phe Asp Ser Asp Val Gly Glu Phe Arg Ala Val Thr Glu Leu Gly Arg
           35                  40                  45
   Pro Asp Glu Asp Tyr Trp Asn Ser Gln Lys Asp Phe Leu Glu Asp Arg
       50                  55                  60
   Arg Ala Ala Val Asp Thr Tyr Cys Arg His Asn Tyr Gly Val Gly Glu
   65                  70                  75                  80
   Ser Phe Thr Val Gln Arg
                   85
<210> 442
   <211> 89
   <212> PRT
   <213> Homo sapiens
<400> 442
   Arg Phe Leu Glu Tyr Ser Thr Ser Glu Cys His Phe Phe Asn Gly Thr
   1               5                   10                  15
   Glu Arg Val Arg Phe Leu Asp Arg Tyr Phe Tyr Asn Gln Glu Glu Tyr
               20                  25                  30
   Val Arg Phe Asp Ser Asp Val Gly Glu Phe Arg Ala Val Thr Glu Leu
           35                  40                  45
   Gly Arg Pro Asp Glu Glu Tyr Trp Asn Ser Gln Lys Asp Leu Leu Glu
       50                  55                  60
   Gln Arg Arg Ala Ala Val Asp Thr Tyr Cys Arg His Asn Tyr Gly Val
   65                  70                  75                  80
   Val Glu Ser Phe Thr Val Gln Arg Arg
                   85
<210> 443
   <211> 86
   <212> PRT
   <213> Homo sapiens
<400> 443
   Leu Glu Tyr Ser Thr Ser Glu Cys His Phe Phe Asn Gly Thr Glu Arg
   1               5                   10                  15
   Val Arg Phe Leu Asp Arg Tyr Phe Tyr Asn Gln Glu Glu Tyr Val Arg
               20                  25                  30
   Phe Asp Ser Asp Val Gly Glu Phe Arg Ala Val Thr Glu Leu Gly Arg
           35                  40                  45
   Pro Asp Glu Asp Tyr Trp Asn Ser Gln Lys Asp Phe Leu Glu Asp Arg
       50                  55                  60
   Arg Ala Ala Val Asp Thr Tyr Cys Arg His Asn Tyr Gly Val Val Glu
   65                  70                  75                  80
   Ser Phe Thr Val Gln Arg
                   85
<210> 444
   <211> 89
   <212> PRT
   <213> Homo sapiens
<400> 444
   Arg Phe Leu Glu Tyr Ser Thr Ser Glu Cys His Phe Phe Asn Gly Thr
   1               5                   10                  15
   Glu Arg Val Arg Phe Leu Asp Arg Tyr Phe Tyr Asn Gln Glu Glu Tyr
               20                  25                  30
   Val Arg Phe Asp Ser Asp Val Gly Glu Phe Arg Ala Val Thr Glu Leu
           35                  40                  45
   Gly Arg Pro Asp Glu Glu Tyr Trp Asn Ser Gln Lys Asp Leu Leu Glu
       50                  55                  60
   Asp Glu Arg Ala Ala Val Asp Thr Tyr Cys Arg His Asn Tyr Gly Val
   65                  70                  75                  80
   Val Glu Ser Phe Thr Val Gln Arg Arg
                   85

<210> 445
   <211> 89
   <212> PRT
   <213> Homo sapiens
<400> 445
   Arg Phe Leu Glu Tyr Ser Thr Ser Glu Cys His Phe Phe Asn Gly Thr
   1               5                   10                  15
   Glu Arg Val Arg Phe Leu Asp Arg Tyr Phe Tyr Asn Gln Glu Glu Tyr
               20                  25                  30
   Val Arg Phe Asp Ser Asp Val Gly Glu Tyr Arg Ala Val Thr Glu Leu
           35                  40                  45
   Gly Arg Pro Asp Glu Glu Tyr Trp Asn Ser Gln Lys Asp Phe Leu Glu
       50                  55                  60
   Asp Arg Arg Ala Ala Val Asp Thr Tyr Cys Arg His Asn Tyr Gly Val
   65                  70                  75                  80
   Gly Glu Ser Phe Thr Val Gln Arg Arg
                   85
<210> 446
   <211> 89
   <212> PRT
   <213> Homo sapiens
<400> 446
   Arg Phe Leu Glu Tyr Ser Thr Ser Glu Cys His Phe Phe Asn Gly Thr
   1 5 10 15
   Glu Arg Val Arg Phe Leu Asp Arg Tyr Phe Tyr Asn Gln Glu Glu Tyr
   20 25 30
   Val Arg Phe Asp Ser Asp Val Gly Glu Phe Arg Ala Val Thr Glu Leu
   35 40 45
   Gly Arg Pro Asp Glu Gly Tyr Trp Asn Ser Gln Lys Asp Phe Leu Glu
   50 55 60
   Asp Arg Arg Ala Ala Val Asp Thr Tyr Cys Arg His Asn Tyr Gly Val
   65 70 75 80
   Val Glu Ser Phe Thr Val Gln Arg Arg
   85
<210> 447
   <211> 89
   <212> PRT
   <213> Homo sapiens
<400> 447
   Arg Phe Leu Glu Tyr Ser Thr Ser Glu Cys His Phe Phe Asn Gly Thr
   1 5 10 15
   Glu Arg Val Arg Phe Leu Asp Arg Tyr Phe Tyr Asn Gln Glu Glu Tyr
   20 25 30
   Val Arg Phe Asp Ser Asp Val Gly Glu Phe Arg Ala Val Arg Glu Leu
   35 40 45
   Gly Arg Pro Asp Glu Glu Tyr Trp Asn Ser Gln Lys Asp Phe Leu Glu
   50 55 60
   Asp Arg Arg Ala Ala Val Asp Thr Tyr Cys Arg His Asn Tyr Gly Val
   65 70 75 80
   Gly Glu Ser Phe Thr Val Gln Arg Arg
   85
<210> 448
   <211> 81
   <212> PRT
   <213> Homo sapiens
<400> 448
   Arg Phe Leu Glu Tyr Ser Thr Ser Glu Cys His Phe Phe Asn Gly Thr
   1               5                   10                  15
   Glu Arg Val Arg Phe Leu Asp Arg Tyr Phe His Asn Gln Glu Glu Asn
               20                  25                  30
   Val Arg Phe Asp Ser Asp Val Gly Glu Phe Arg Ala Val Thr Glu Leu
           35                  40                  45
   Gly Arg Pro Asp Glu Glu Tyr Trp Asn Ser Gln Lys Asp Phe Leu Glu
       50                  55                  60
   Asp Glu Arg Ala Ala Val Asp Thr Tyr Cys Arg His Asn Tyr Gly Val
   65                  70                  75                  80
   Val
<210> 449
   <211> 88
   <212> PRT
   <213> Homo sapiens
<400> 449
   Phe Leu Glu Tyr Ser Thr Ser Glu Cys His Phe Phe Asn Gly Thr Glu
   1               5                   10                  15
   Arg Val Arg Phe Leu Asp Arg Tyr Phe Tyr Asn Gln Glu Glu Tyr Val
               20                  25                  30
   Arg Phe Asp Ser Asp Val Gly Glu Tyr Arg Ala Val Thr Glu Leu Gly
           35                  40                  45
   Arg Pro Asp Glu Glu Tyr Trp Asn Ser Gln Lys Asp Phe Leu Glu Asp
       50                  55                  60
   Glu Arg Ala Ala Val Asp Thr Tyr Cys Arg His Asn Tyr Gly Val Val
   65                  70                  75                  80
   Glu Ser Phe Thr Val Gln Arg Arg
                   85
<210> 450
   <211> 89
   <212> PRT
   <213> Homo sapiens
<400> 450
   Arg Phe Leu Glu Tyr Ser Thr Ser Glu Cys His Phe Phe Asn Gly Thr
   1               5                   10                  15
   Glu Arg Val Arg Phe Leu Asp Arg Tyr Phe Tyr Asn Gln Glu Glu Tyr
               20                  25                  30
   Val Arg Phe Asp Ser Asp Val Gly Glu Phe Arg Ala Val Thr Glu Leu
           35                  40                  45
   Gly Arg Pro Asp Glu Glu Tyr Trp Asn Ser Gln Lys Asp Leu Leu Glu
       50                  55                  60
   Asp Arg Arg Ala Ala Val Asp Thr Tyr Cys Arg His Asn Tyr Gly Val
   65                  70                  75                  80
   Val Glu Ser Phe Thr Val Gln Arg Arg
                   85
<210> 451
   <211> 266
   <212> PRT
   <213> Homo sapiens
<400> 451
<210> 452
   <211> 266
   <212> PRT
   <213> Homo sapiens
<400> 452
<210> 453
   <211> 81
   <212> PRT
   <213> Homo sapiens
<400> 453
   Arg Phe Leu Glu Tyr Ser Thr Gly Glu Cys Tyr Phe Phe Asn Gly Thr
   1               5                   10                  15
   Glu Arg Val Arg Leu Leu Glu Arg His Phe His Asn Gln Glu Glu Leu
               20                  25                  30
   Leu Arg Phe Asp Ser Asp Val Gly Glu Phe Arg Ala Val Thr Glu Leu
           35                  40                  45
   Gly Arg Pro Val Ala Glu Ser Trp Asn Ser Gln Lys Asp Phe Leu Glu
       50                  55                  60
   Asp Arg Arg Ala Ala Val Asp Thr Tyr Cys Arg His Asn Tyr Gly Ala
   65                  70                  75                  80
   Val
<210> 454
   <211> 81
   <212> PRT
   <213> Homo sapiens
<400> 454
   Phe Leu Glu Tyr Ser Thr Gly Glu Cys Tyr Phe Phe Asn Gly Thr Glu
   1               5                   10                  15
   Arg Val Arg Leu Leu Glu Arg His Phe His Asn Gln Glu Glu Leu Leu
               20                  25                  30
   Arg Phe Asp Ser Asp Val Gly Glu Phe Arg Ala Val Thr Glu Leu Gly
           35                  40                  45
   Arg Pro Val Ala Glu Ser Trp Asn Ser Gln Lys Asp Phe Leu Glu Asp
       50                  55                  60
   Arg Arg Ala Ala Val Asp Thr Tyr Cys Arg His Asn Tyr Gly Ala Val
   65                  70                  75                  80
   Glu
<210> 455
   <211> 88
   <212> PRT
   <213> Homo sapiens
<400> 455
   Arg Phe Leu Glu Tyr Ser Thr Gly Glu Cys Tyr Phe Phe Asn Gly Thr
   1               5                   10                  15
   Glu Arg Val Arg Leu Leu Glu Arg His Phe His Asn Gln Glu Glu Leu
               20                  25                  30
   Leu Arg Phe Asp Ser Asp Val Gly Glu Phe Arg Ala Val Thr Glu Leu
           35                  40                  45
   Gly Arg Pro Val Ala Glu Ser Trp Asn Ser Gln Lys Asp Ile Leu Glu
       50                  55                  60
   Asp Arg Arg Ala Ala Val Asp Thr Tyr Cys Arg His Asn Tyr Gly Val
   65                  70                  75                  80
   Val Glu Ser Phe Thr Val Gln Arg
                   85
<210> 456
   <211> 78
   <212> PRT
   <213> Homo sapiens
<400> 456
   Glu Tyr Ser Thr Gly Glu Cys Tyr Phe Phe Asn Gly Thr Glu Arg Val
   1               5                   10                  15
   Arg Leu Leu Glu Arg His Phe His Asn Gln Glu Glu Leu Leu Arg Phe
               20                  25                  30
   Asp Ser Asp Val Gly Glu Phe Arg Ala Val Thr Glu Leu Gly Arg Pro
           35                  40                  45
   Asp Glu Glu Tyr Trp Asn Ser Gln Lys Asp Ile Leu Glu Asp Arg Arg
       50                  55                  60
   Ala Ala Val Asp Thr Tyr Cys Arg His Asn Tyr Gly Ala Val
   65                  70                  75
<210> 457
   <211> 89
   <212> PRT
   <213> Homo sapiens
<400> 457
   Arg Phe Leu Glu Tyr Ser Thr Gly Glu Cys Tyr Phe Phe Asn Gly Thr
   1 5 10 15
   Glu Arg Val Arg Leu Leu Glu Arg His Phe His Asn Gln Glu Glu Phe
   20 25 30
   Leu Arg Phe Asp Ser Asp Val Gly Glu Phe Arg Ala Val Thr Glu Leu
   35 40 45
   Gly Arg Pro Val Ala Glu Ser Trp Asn Ser Gln Lys Asp Ile Leu Glu
   50 55 60
   Asp Arg Arg Ala Ala Val Asp Thr Tyr Cys Arg His Asn Tyr Gly Ala
   65 70 75 80
   Val Glu Ser Phe Thr Val Gln Arg Arg
   85
<210> 458
   <211> 209
   <212> PRT
   <213> Homo sapiens
<400> 458
   Gly Asp Thr Arg Pro Arg Phe Leu Glu Tyr Ser Thr Gly Glu Cys Tyr
   1 5 10 15
   Phe Phe Asn Gly Thr Glu Arg Val Arg Leu Leu Glu Arg His Phe His
   20 25 30
   Asn Gln Glu Glu Leu Leu Arg Phe Asp Ser Asp Val Gly Glu Phe Arg
   35 40 45
   Ala Val Thr Glu Leu Gly Arg Pro Val Ala Glu Ser Trp Asn Ser Gln
   50 55 60
   Lys Asp Ile Leu Glu Asp Arg Arg Ala Ala Val Asp Thr Tyr Cys Arg
   65 70 75 80
   His Asn Tyr Gly Ala Val Glu Ser Phe Thr Val Gln Arg Arg Val His
   85 90 95
   Pro Lys Val Thr Val Tyr Pro Ser Lys Thr Gln Pro Leu Gln His His
   100 105 110
   Asn Leu Leu Val Cys Ser Val Ser Gly Phe Tyr Pro Gly Ser Ile Glu
   115 120 125
   Val Arg Trp Phe Arg Asn Gly Gln Glu Glu Lys Thr Gly Val Val Ser
   130 135 140
   Thr Gly Leu Ile Gln Asn Gly Asp Trp Thr Phe Gln Thr Leu Val Met
   145 150 155 160
   Leu Glu Thr Val Pro Arg Ser Gly Glu Val Tyr Thr Cys Gln Val Glu
   165 170 175
   His Pro Ser Val Thr Ser Pro Leu Thr Val Glu Trp Arg Ala Arg Ser
   180 185 190
   Glu Ser Ala Gln Ser Lys Met Leu Ser Gly Val Gly Gly Phe Val Leu
   195 200 205
   Gly
<210> 459
   <211> 174
   <212> PRT
   <213> Homo sapiens
<400> 459
   Arg Phe Leu Glu Tyr Ser Thr Gly Glu Cys Tyr Phe Phe Asn Gly Thr
   1               5                   10                  15
   Glu Arg Val Arg Leu Leu Glu Arg His Phe His Asn Gln Glu Glu Leu
               20                  25                  30
   Leu Arg Phe Asp Ser Asp Val Gly Glu Phe Arg Ala Val Thr Glu Leu
           35                  40                  45
   Gly Arg Pro Val Ala Glu Ser Trp Asn Ser Gln Lys Asp Ile Leu Gly
       50                  55                  60
   Asp Arg Arg Ala Ala Val Asp Thr Tyr Cys Arg His Asn Tyr Gly Ala
   65                  70                  75                  80
   Val Glu Ser Phe Thr Val Gln Arg Arg Val His Pro Lys Val Thr Val
                   85                  90                  95
   Tyr Pro Ser Lys Thr Gln Pro Leu Gln His His Asn Leu Leu Val Cys
               100                 105                 110
   Ser Val Ser Gly Phe Tyr Pro Gly Ser Ile Glu Val Arg Trp Phe Arg
           115                 120                 125
   Asn Gly Gln Glu Glu Lys Thr Gly Val Val Ser Thr Gly Leu Ile His
       130                 135                 140
   Asn Gly Asp Trp Thr Phe Gln Thr Leu Val Met Leu Glu Thr Val Pro
   145                 150                 155                 160
   Arg Ser Gly Glu Val Tyr Thr Cys Gln Val Glu His Pro Ser
                   165                 170
<210> 460
   <211> 89
   <212> PRT
   <213> Homo sapiens
<400> 460
   Arg Phe Leu Glu Tyr Ser Thr Gly Glu Cys Tyr Phe Phe Asn Gly Thr
   1               5                   10                  15
   Glu Arg Val Arg Phe Leu Glu Arg His Phe His Asn Gln Glu Glu Leu
               20                  25                  30
   Leu Arg Phe Asp Ser Asp Val Gly Glu Phe Arg Ala Val Thr Glu Leu
           35                  40                  45
   Gly Arg Pro Val Ala Glu Ser Trp Asn Ser Gln Lys Asp Ile Leu Glu
       50                  55                  60
   Asp Arg Arg Ala Ala Val Asp Thr Tyr Cys Arg His Asn Tyr Gly Ala
   65                  70                  75                  80
   Val Glu Ser Phe Thr Val Gln Arg Arg
                   85
<210> 461
   <211> 237
   <212> PRT
   <213> Homo sapiens
<400> 461
   Gly Asp Thr Arg Pro Arg Phe Leu Glu Tyr Ser Thr Ser Glu Cys His
   1               5                   10                  15
   Phe Phe Asn Gly Thr Glu Arg Val Arg Phe Leu Asp Arg Tyr Phe His
               20                  25                  30
   Asn Gln Glu Glu Asn Val Arg Phe Asp Ser Asp Val Gly Glu Phe Arg
           35                  40                  45
   Ala Val Thr Glu Leu Gly Arg Pro Asp Ala Glu Tyr Trp Asn Ser Gln
       50                  55                  60
   Lys Asp Ile Leu Glu Asp Glu Arg Ala Ala Val Asp Thr Tyr Cys Arg
   65                  70                  75                  80
   His Asn Tyr Gly Val Val Glu Ser Phe Thr Val Gln Arg Arg Val His
                   85                  90                  95
   Pro Lys Val Thr Val Tyr Pro Ser Lys Thr Gln Pro Leu Gln His His
               100                 105                 110
   Asn Leu Leu Val Cys Ser Val Ser Gly Phe Tyr Pro Gly Ser Ile Glu
           115                 120                 125
   Val Arg Trp Phe Arg Asn Gly Gln Glu Glu Lys Thr Gly Val Val Ser
       130                 135                 140
   Thr Gly Leu Ile His Asn Gly Asp Trp Thr Phe Gln Thr Leu Val Met
   145                 150                 155                 160
   Leu Glu Thr Val Pro Arg Ser Gly Glu Val Tyr Thr Cys Gln Val Glu
                   165                 170                 175
   His Pro Ser Val Thr Ser Pro Leu Thr Val Glu Trp Arg Ala Arg Ser
               180                 185                 190
   Glu Ser Ala Gln Ser Lys Met Leu Ser Gly Val Gly Gly Phe Val Leu
           195                 200                 205
   Gly Leu Leu Phe Leu Gly Ala Gly Leu Phe Ile Tyr Phe Arg Asn Gln
       210                 215                 220
   Lys Gly His Ser Gly Leu Gln Pro Arg Gly Phe Leu Ser
   225                 230                 235

<210> 462
   <211> 89
   <212> PRT
   <213> Homo sapiens
<400> 462
   Arg Phe Leu Glu Tyr Ser Thr Ser Glu Cys His Phe Phe Asn Gly Thr
   1               5                   10                  15
   Glu Arg Val Arg Phe Leu Asp Arg Tyr Phe His Asn Gln Glu Glu Asn
               20                  25                  30
   Val Arg Phe Asp Ser Asp Val Gly Glu Phe Arg Ala Val Thr Glu Leu
           35                  40                  45
   Gly Arg Pro Asp Ala Glu Tyr Trp Asn Ser Gln Lys Asp Ile Leu Glu
       50                  55                  60
   Asp Glu Arg Ala Ala Val Asp Thr Tyr Cys Arg His Asn Tyr Gly Val
   65                  70                  75                  80
   Val Glu Ser Phe Thr Val Gln Arg Arg
                   85
<210> 463
   <211> 237
   <212> PRT
   <213> Homo sapiens
<400> 463
   Gly Asp Thr Arg Pro Arg Phe Leu Glu Tyr Ser Thr Ser Glu Cys His
   1               5                   10                  15
   Phe Phe Asn Gly Thr Glu Arg Val Arg Phe Leu Asp Arg Tyr Phe His
               20                  25                  30
   Asn Gln Glu Glu Asn Val Arg Phe Asp Ser Asp Val Gly Glu Phe Arg
   35 40 45
   Ala Val Thr Glu Leu Gly Arg Pro Asp Ala Glu Tyr Trp Asn Ser Gln
   50 55 60
   Lys Asp Ile Leu Glu Asp Glu Arg Ala Ala Val Asp Thr Tyr Cys Arg
   65 70 75 80
   His Asn Tyr Gly Val Gly Glu Ser Phe Thr Val Gln Arg Arg Val His
   85 90 95
   Pro Lys Val Thr Val Tyr Pro Ser Lys Thr Gln Pro Leu Gln His His
   100 105 110
   Asn Leu Leu Val Cys Ser Val Ser Gly Phe Tyr Pro Gly Ser Ile Glu
   115 120 125
   Val Arg Trp Phe Arg Asn Gly Gln Glu Glu Lys Thr Gly Val Val Ser
   130 135 140
   Thr Gly Leu Ile His Asn Gly Asp Trp Thr Phe Gln Thr Leu Val Met
   145 150 155 160
   Leu Glu Thr Val Pro Arg Ser Gly Glu Val Tyr Thr Cys Gln Val Glu
   165 170 175
   His Pro Ser Val Thr Ser Pro Leu Thr Val Glu Trp Arg Ala Arg Ser
   180 185 190
   Glu Ser Ala Gln Ser Lys Met Leu Ser Gly Val Gly Gly Phe Val Leu
   195 200 205
   Gly Leu Leu Phe Leu Gly Ala Gly Leu Phe Ile Tyr Phe Arg Asn Gln
   210 215 220
   Lys Gly His Ser Gly Leu Gln Pro Arg Gly Phe Leu Ser
   225 230 235
<210> 464
   <211> 88
   <212> PRT
   <213> Homo sapiens
<400> 464
   Arg Phe Leu Glu Tyr Ser Thr Ser Glu Cys His Phe Phe Asn Gly Thr
   1 5 10 15
   Glu Arg Val Arg Phe Leu Asp Arg Tyr Phe His Asn Gln Glu Glu Asn
   20 25 30
   Val Arg Phe Asp Ser Asp Val Gly Glu Phe Arg Ala Val Thr Glu Leu
   35 40 45
   Gly Arg Pro Asp Ala Glu Tyr Trp Asn Ser Gln Lys Asp Ile Leu Glu
   50 55 60
   Asp Glu Arg Ala Ala Val Asp Thr Tyr Cys Arg His Asn Tyr Gly Val
   65 70 75 80
   Gly Glu Ser Phe Thr Val Gln Arg
   85
<210> 465
   <211> 101
   <212> PRT
   <213> Homo sapiens
<400> 465
   Gly Asp Thr Arg Pro Arg Phe Leu Glu Tyr Ser Thr Ser Glu Cys His
   1 5 10 15
   Phe Phe Asn Gly Thr Glu Arg Val Arg Phe Leu Asp Arg Tyr Phe Tyr
   20 25 30
   Asn Gln Glu Glu Tyr Val Arg Phe Asp Ser Asp Val Gly Glu Tyr Arg
   35 40 45
   Ala Val Thr Glu Leu Gly Arg Pro Ser Ala Glu Tyr Trp Asn Ser Gln
   50 55 60
   Lys Asp Ile Leu Glu Asp Lys Arg Ala Ala Val Asp Thr Tyr Cys Arg
   65 70 75 80
   His Asn Tyr Gly Val Gly Glu Ser Phe Thr Val Gln Arg Arg Val His
   85 90 95
   Pro Lys Val Thr Val
   100
<210> 466
   <211> 87
   <212> PRT
   <213> Homo sapiens
<400> 466
   Leu Glu Tyr Ser Thr Ser Glu Cys His Phe Phe Asn Gly Thr Glu Arg
   1 5 10 15
   Val Arg Phe Leu Asp Arg Tyr Phe Tyr Asn Gln Glu Glu Tyr Val Arg
   20 25 30
   Phe Asp Ser Asp Val Gly Glu Tyr Arg Ala Val Thr Glu Leu Gly Arg
   35 40 45
   Pro Ser Ala Glu Tyr Trp Asn Ser Gln Lys Asp Ile Leu Glu Asp Lys
   50 55 60
   Arg Ala Ala Val Asp Thr Tyr Cys Arg His Asn Tyr Gly Val Gly Glu
   65 70 75 80
   Ser Phe Thr Val Gln Arg Arg
   85
<210> 467
   <211> 101
   <212> PRT
   <213> Homo sapiens
<400> 467
   Gly Asp Thr Arg Pro Arg Phe Leu Glu Tyr Ser Thr Ser Glu Cys His
   1 5 10 15
   Phe Phe Asn Gly Thr Glu Arg Val Arg Phe Leu Asp Arg Tyr Phe Tyr
   20 25 30
   Asn Gln Glu Glu Tyr Val Arg Phe Asp Ser Asp Val Gly Glu Phe Arg
   35 40 45
   Ala Val Thr Glu Leu Gly Arg Pro Ser Ala Glu Tyr Trp Asn Ser Gln
   50 55 60
   Lys Asp Ile Leu Glu Asp Glu Arg Ala Ala Val Asp Thr Tyr Cys Arg
   65 70 75 80
   His Asn Tyr Gly Val Val Glu Ser Phe Thr Val Gln Arg Arg Val His
   85 90 95
   Pro Lys Val Thr Val
   100
<210> 468
   <211> 89
   <212> PRT
   <213> Homo sapiens
<400> 468
   Arg Phe Leu Glu Tyr Ser Thr Ser Glu Cys His Phe Phe Asn Gly Thr
   1 5 10 15
   Glu Arg Val Arg Phe Leu Asp Arg Tyr Phe His Asn Gln Glu Glu Asn
   20 25 30
   Val Arg Phe Asp Ser Asp Val Gly Glu Phe Arg Ala Val Thr Glu Leu
   35 40 45
   Gly Arg Pro Asp Ala Glu Tyr Trp Asn Ser Gln Lys Asp Phe Leu Glu
   50 55 60
   Asp Arg Arg Ala Ala Val Asp Thr Tyr Cys Arg His Asn Tyr Gly Val
   65 70 75 80
   Gly Glu Ser Phe Thr Val Gln Arg Arg
   85
<210> 469
   <211> 76
   <212> PRT
   <213> Homo sapiens
<400> 469
   Cys His Phe Phe Asn Gly Thr Glu Arg Val Arg Phe Leu Asp Arg Tyr
   1 5 10 15
   Phe His Asn Gln Glu Glu Asn Val Arg Phe Asp Ser Asp Val Gly Glu
   20 25 30
   Phe Arg Ala Val Thr Glu Leu Gly Arg Pro Asp Ala Glu Tyr Trp Asn
   35 40 45
   Ser Gln Lys Asp Ile Leu Glu Asp Arg Arg Ala Ala Val Asp Thr Tyr
   50 55 60
   Cys Arg His Asn Tyr Gly Val Val Glu Ser Phe Thr
   65 70 75
<210> 470
   <211> 89
   <212> PRT
   <213> Homo sapiens
<400> 470
   Arg Phe Leu Glu Tyr Ser Thr Ser Glu Cys His Phe Phe Asn Gly Thr
   1               5                   10                  15
   Glu Arg Val Arg Phe Leu Asp Arg Tyr Phe Tyr Asn Gln Glu Glu Tyr
               20                  25                  30
   Val Arg Phe Asp Ser Asp Val Gly Glu Tyr Arg Ala Val Thr Glu Leu
           35                  40                  45
   Gly Arg Pro Asp Ala Glu Tyr Trp Asn Ser Gln Lys Asp Phe Leu Glu
       50                  55                  60
   Asp Arg Arg Ala Ala Val Asp Thr Tyr Cys Arg His Asn Tyr Gly Val
   65                  70                  75                  80
   Gly Glu Ser Phe Thr Val Gln Arg Arg
                   85
<210> 471
   <211> 89
   <212> PRT
   <213> Homo sapiens
<400> 471
   Arg Phe Leu Glu Tyr Ser Thr Ser Glu Cys His Phe Phe Asn Gly Thr
   1               5                   10                  15
   Glu Arg Val Arg Phe Leu Asp Arg Tyr Phe Tyr Asn Gln Glu Glu Tyr
               20                  25                  30
   Val Arg Phe Asp Ser Asp Val Gly Glu Tyr Arg Ala Val Thr Glu Leu
           35                  40                  45
   Gly Arg Pro Asp Ala Glu Tyr Trp Asn Ser Gln Lys Asp Phe Leu Glu
       50                  55                  60
   Asp Arg Arg Ala Ala Val Asp Thr Tyr Cys Arg His Asn Tyr Gly Val
   65                  70                  75                  80
   Gly Glu Ser Phe Thr Val Gln Arg Arg
                   85
<210> 472
   <211> 85
   <212> PRT
   <213> Homo sapiens
<400> 472
   Phe Leu Glu Tyr Ser Thr Ser Glu Cys His Phe Phe Asn Gly Thr Glu
   1 5 10 15
   Arg Val Arg Phe Leu Asp Arg Tyr Phe His Asn Gln Glu Glu Phe Val
   20 25 30
   Arg Phe Asp Ser Asp Val Gly Glu Tyr Arg Ala Val Thr Glu Leu Gly
   35 40 45
   Arg Pro Asp Ala Glu Tyr Trp Asn Ser Gln Lys Asp Ile Leu Glu Asp
   50 55 60
   Glu Arg Ala Ala Val Asp Thr Tyr Cys Arg His Asn Tyr Gly Val Val
   65 70 75 80
   Glu Ser Phe Thr Val
   85
<210> 473
   <211> 85
   <212> PRT
   <213> Homo sapiens
<400> 473
   Phe Leu Glu Tyr Ser Thr Ser Glu Cys His Phe Phe Asn Gly Thr Glu
   1 5 10 15
   Arg Val Arg Phe Leu Asp Arg Tyr Phe His Asn Gln Glu Glu Asn Val
   20 25 30
   Arg Phe Asp Ser Asp Val Gly Glu Phe Arg Ala Val Thr Glu Leu Gly
   35 40 45
   Arg Pro Asp Ala Glu Tyr Trp Asn Ser Gln Lys Asp Ile Leu Glu Gln
   50 55 60
   Ala Arg Ala Ala Val Asp Thr Tyr Cys Arg His Asn Tyr Gly Val Val
   65 70 75 80
   Glu Ser Phe Thr Val
   85
<210> 474
   <211> 89
   <212> PRT
   <213> Homo sapiens
<400> 474
   Arg Phe Leu Glu Tyr Ser Thr Ser Glu Cys His Phe Phe Asn Gly Thr
   1 5 10 15
   Glu Arg Val Arg Phe Leu Asp Arg Tyr Phe His Asn Gln Glu Glu Asn
   20 25 30
   Val Arg Phe Asp Ser Asp Val Gly Glu Phe Arg Ala Val Thr Glu Leu
   35 40 45
   Gly Arg Pro Asp Ala Glu Tyr Trp Asn Ser Gln Lys Asp Ile Leu Glu
   50 55 60
   Asp Lys Arg Ala Ala Val Asp Thr Tyr Cys Arg His Asn Tyr Gly Val
   65 70 75 80
   Val Glu Ser Phe Thr Val Gln Arg Arg
   85
<210> 475
   <211> 89
   <212> PRT
   <213> Homo sapiens
<400> 475
   Arg Phe Leu Glu Tyr Ser Thr Ser Glu Cys His Phe Phe Asn Gly Thr
   1 5 10 15
   Glu Arg Val Arg Phe Leu Asp Arg Tyr Phe Tyr Asn Gln Glu Glu Tyr
   20 25 30
   Val Arg Phe Asp Ser Asp Val Gly Glu Phe Arg Ala Val Thr Glu Leu
   35 40 45
   Gly Arg Pro Asp Ala Glu Tyr Trp Asn Ser Gln Lys Asp Phe Leu Glu
   50 55 60
   Asp Arg Arg Ala Ala Val Asp Thr Tyr Cys Arg His Asn Tyr Gly Val
   65 70 75 80
   Val Glu Ser Phe Thr Val Gln Arg Arg
   85
<210> 476
   <211> 89
   <212> PRT
   <213> Homo sapiens
<400> 476
   Arg Phe Leu Glu Tyr Ser Thr Ser Glu Cys His Phe Phe Asn Gly Thr
   1               5                   10                  15
   Glu Arg Val Arg Phe Leu Asp Arg Tyr Phe Tyr Asn Gln Glu Glu Tyr
               20                  25                  30
   Val Arg Phe Asp Ser Asp Val Gly Glu Tyr Arg Ala Val Thr Glu Leu
           35                  40                  45
   Gly Arg Pro Ser Ala Glu Tyr Trp Asn Ser Gln Lys Asp Ile Leu Glu
       50                  55                  60
   Asp Arg Arg Ala Ala Val Asp Thr Tyr Cys Arg His Asn Tyr Gly Val
   65                  70                  75                  80
   Gly Glu Ser Phe Thr Val Gln Arg Arg
                   85
<210> 477
   <211> 86
   <212> PRT
   <213> Homo sapiens
<400> 477
   Arg Phe Leu Glu Tyr Ser Thr Ser Glu Cys His Phe Phe Asn Gly Thr
   1               5                   10                  15
   Glu Arg Val Arg Phe Leu Asp Arg Tyr Phe Tyr Asn Gln Glu Glu Tyr
               20                  25                  30
   Val Arg Phe Asp Ser Asp Val Gly Glu Tyr Arg Ala Val Thr Glu Leu
           35                  40                  45
   Gly Arg Pro Ser Ala Glu Tyr Trp Asn Ser Gln Lys Asp Ile Leu Glu
       50                  55                  60
   Asp Arg Arg Ala Leu Val Asp Thr Tyr Cys Arg His Asn Tyr Gly Val
   65                  70                  75                  80
   Gly Glu Ser Phe Thr Val
                   85
<210> 478
   <211> 75
   <212> PRT
   <213> Homo sapiens
<400> 478
   Thr Ser Glu Cys His Phe Phe Asn Gly Thr Glu Arg Val Arg Phe Leu
   1 5 10 15
   Asp Arg Tyr Phe Tyr Asn Gln Glu Glu Tyr Val Arg Phe Asp Ser Asp
   20 25 30
   Val Gly Glu Phe Arg Ala Val Thr Glu Leu Gly Arg Pro Asp Ala Glu
   35 40 45
   Tyr Trp Asn Ser Gln Lys Asp Phe Leu Glu Asp Arg Arg Ala Ala Val
   50 55 60
   Asp Thr Tyr Cys Arg His Asn Tyr Gly Val Gly
   65 70 75
<210> 479
   <211> 89
   <212> PRT
   <213> Homo sapiens
<400> 479
   Arg Phe Leu Glu Tyr Ser Thr Ser Glu Cys His Phe Phe Asn Gly Thr
   1 5 10 15
   Glu Arg Val Arg Phe Leu Asp Arg Tyr Phe Tyr Asn Gln Glu Glu Tyr
   20 25 30
   Val Arg Phe Asp Ser Asp Val Gly Glu Phe Arg Ala Val Thr Glu Leu
   35 40 45
   Gly Arg Pro Asp Ala Glu Tyr Trp Asn Ser Gln Lys Asp Phe Leu Glu
   50 55 60
   Asp Arg Arg Ala Ala Val Asp Thr Tyr Cys Arg His Asn Tyr Gly Val
   65 70 75 80
   Gly Glu Ser Phe Thr Val Gln Arg Arg
   85
<210> 480
   <211> 84
   <212> PRT
   <213> Homo sapiens
<400> 480
   Phe Leu Glu Tyr Ser Thr Ser Glu Cys His Phe Phe Asn Gly Thr Glu
   1 5 10 15
   Arg Val Arg Phe Leu Glu Arg Tyr Phe His Asn Gln Glu Glu Asn Val
   20 25 30
   Arg Phe Asp Ser Asp Val Gly Glu Phe Arg Ala Val Thr Glu Leu Gly
   35 40 45
   Arg Pro Asp Ala Glu Tyr Trp Asn Ser Gln Lys Asp Ile Leu Glu Asp
   50 55 60
   Glu Arg Ala Ala Val Asp Thr Tyr Cys Arg His Asn Tyr Gly Val Val
   65 70 75 80
   Glu Ser Phe Thr
<210> 481
   <211> 82
   <212> PRT
   <213> Homo sapiens
<400> 481
   Glu Tyr Ser Thr Ser Glu Cys His Phe Phe Asn Gly Thr Glu Arg Val
   1 5 10 15
   Arg Phe Leu Asp Arg Tyr Phe His Asn Gln Glu Glu Asn Val Arg Phe
   20 25 30
   Asp Ser Asp Val Gly Glu Phe Arg Ala Val Thr Glu Leu Gly Arg Pro
   35 40 45
   Asp Ala Glu Tyr Trp Asn Ser Gln Lys Asp Ile Leu Glu Asp Glu Arg
   50 55 60
   Ala Ala Val Asp Thr Tyr Cys Arg His Asn Tyr Gly Val Asp Glu Ser
   65 70 75 80
   Phe Thr
<210> 482
   <211> 114
   <212> PRT
   <213> Homo sapiens
<400> 482
   Gly Asp Thr Arg Pro Arg Phe Leu Glu Tyr Ser Thr Gly Glu Cys Tyr
   1 5 10 15
   Phe Phe Asn Gly Thr Glu Arg Val Arg Phe Leu Asp Arg Tyr Phe Tyr
   20 25 30
   Asn Gln Glu Glu Tyr Val Arg Phe Asp Ser Asp Val Gly Glu Phe Arg
   35 40 45
   Ala Val Thr Glu Leu Gly Arg Pro Asp Ala Glu Tyr Trp Asn Ser Gln
   50 55 60
   Lys Asp Ile Leu Glu Asp Glu Arg Ala Ala Val Asp Thr Tyr Cys Arg
   65 70 75 80
   His Asn Tyr Gly Val Val Glu Ser Phe Thr Val Gln Arg Arg Val His
   85 90 95
   Pro Lys Val Thr Val Tyr Pro Ser Lys Thr Gln Pro Leu Gln His His
   100 105 110
   Asn Leu

<210> 483
   <211> 89
   <212> PRT
   <213> Homo sapiens
<400> 483
   Arg Phe Leu Glu Tyr Ser Thr Ser Glu Cys His Phe Phe Asn Gly Thr
   1 5 10 15
   Glu Arg Val Arg Phe Leu Asp Arg Tyr Phe His Asn Gln Glu Glu Asn
   20 25 30
   Val Arg Phe Asp Ser Asp Val Gly Glu Phe Arg Ala Val Thr Glu Leu
   35 40 45
   Gly Arg Pro Asp Ala Glu Tyr Trp Asn Ser Gln Lys Asp Phe Leu Glu
   50 55 60
   Asp Arg Arg Ala Leu Val Asp Thr Tyr Cys Arg His Asn Tyr Gly Val
   65 70 75 80
   Val Glu Ser Phe Thr Val Gln Arg Arg
   85
<210> 484
   <211> 108
   <212> PRT
   <213> Homo sapiens
<400> 484
   Gly Asp Thr Arg Pro Arg Phe Leu Glu Tyr Ser Thr Ser Glu Cys His
   1               5                   10                  15
   Phe Phe Asn Gly Thr Glu Arg Val Arg Phe Leu Glu Arg Tyr Phe His
               20                  25                  30
   Asn Gln Glu Glu Phe Val Arg Phe Asp Ser Asp Val Gly Glu Tyr Arg
           35                  40                  45
   Ala Val Thr Glu Leu Gly Arg Pro Asp Ala Glu Tyr Trp Asn Ser Gln
       50                  55                  60
   Lys Asp Ile Leu Glu Asp Glu Arg Ala Ala Val Asp Thr Tyr Cys Arg
   65                  70                  75                  80
   His Asn Tyr Gly Val Val Glu Ser Phe Thr Val Gln Arg Arg Val His
                   85                  90                  95
   Pro Lys Val Thr Val Tyr Pro Ser Lys Thr Gln Pro
               100                 105
<210> 485
   <211> 88
   <212> PRT
   <213> Homo sapiens
<400> 485
   Arg Phe Leu Glu Tyr Ser Thr Ser Glu Cys His Phe Phe Asn Gly Thr
   1               5                   10                  15
   Glu Arg Val Arg Phe Leu Asp Arg Tyr Phe His Asn Gln Glu Glu Asn
               20                  25                  30
   Val Arg Phe Asp Ser Asp Val Gly Glu Phe Arg Ala Val Thr Glu Leu
           35                  40                  45
   Gly Arg Pro Asp Ala Glu Tyr Trp Asn Ser Gln Lys Asp Leu Leu Glu
       50                  55                  60
   Asp Glu Arg Ala Ala Val Asp Thr Tyr Cys Arg His Asn Tyr Gly Val
   65                  70                  75                  80
   Val Glu Ser Phe Thr Val Gln Arg
                   85
<210> 486
   <211> 119
   <212> PRT
   <213> Homo sapiens
<400> 486
   Gly Asp Thr Arg Pro Arg Phe Leu Glu Tyr Ser Thr Ser Glu Cys His
   1               5                   10                  15
   Phe Phe Asn Gly Thr Glu Arg Val Arg Phe Leu Asp Arg Tyr Phe Tyr
               20                  25                  30
   Asn Gln Glu Glu Tyr Val Arg Phe Asp Ser Asp Val Gly Glu Phe Arg
           35                  40                  45
   Ala Val Thr Glu Leu Gly Arg Pro Ser Ala Glu Tyr Trp Asn Ser Gln
       50                  55                  60
   Lys Asp Phe Leu Glu Asp Arg Arg Ala Ala Val Asp Thr Tyr Cys Arg
   65                  70                  75                  80
   His Asn Tyr Gly Val Gly Glu Ser Phe Thr Val Gln Arg Arg Val His
                   85                  90                  95
   Pro Lys Val Thr Val Tyr Pro Ser Lys Thr Gln Pro Leu Gln His His
               100                 105                 110
   Asn Leu Leu Val Cys Ser Val
           115
<210> 487
   <211> 90
   <212> PRT
   <213> Homo sapiens
<400> 487
   Pro Arg Phe Leu Glu Tyr Ser Thr Ser Glu Cys His Phe Phe Asn Gly
   1               5                   10                  15
   Thr Glu Arg Val Arg Phe Leu Asp Arg Tyr Phe Tyr Asn Gln Glu Glu
               20                  25                  30
   Tyr Val Arg Phe Asp Ser Asp Val Gly Glu Phe Arg Ala Val Thr Glu
           35                  40                  45
   Leu Gly Arg Pro Asp Ala Glu Tyr Trp Asn Ser Gln Lys Asp Ile Leu
       50                  55                  60
   Glu Asp Glu Arg Ala Ala Val Asp Thr Tyr Cys Arg His Asn Tyr Gly
   65                  70                  75                  80
   Val Val Glu Ser Phe Thr Val Gln Arg Arg
                   85                  90
<210> 488
   <211> 88
   <212> PRT
   <213> Homo sapiens
<400> 488
   Arg Phe Leu Glu Tyr Ser Thr Ser Glu Cys His Phe Phe Asn Gly Thr
   1               5                   10                  15
   Glu Arg Val Arg Phe Leu Asp Arg Tyr Phe Tyr Asn Gln Glu Glu Tyr
               20                  25                  30
   Val Arg Phe Asp Ser Asp Val Gly Glu Phe Arg Ala Val Thr Glu Leu
           35                  40                  45
   Gly Arg Pro Asp Ala Glu Tyr Trp Asn Ser Gln Lys Asp Ile Leu Glu
       50                  55                  60
   Asp Glu Arg Ala Ala Val Asp Thr Tyr Cys Arg His Asn Tyr Gly Val
   65                  70                  75                  80
   Gly Glu Ser Phe Thr Val Gln Arg
                   85
<210> 489
   <211> 88
   <212> PRT
   <213> Homo sapiens
<400> 489
   Arg Phe Leu Glu Tyr Ser Thr Ser Glu Cys His Phe Phe Asn Gly Thr
   1 5 10 15
   Glu Arg Val Arg Phe Leu Asp Arg Tyr Phe Tyr Asn Gln Glu Glu Tyr
   20 25 30
   Val Arg Phe Asp Ser Asp Val Gly Glu Phe Arg Ala Val Thr Glu Leu
   35 40 45
   Gly Arg Pro Asp Ala Glu Tyr Trp Asn Ser Gln Lys Asp Phe Leu Glu
   50 55 60
   Asp Glu Arg Ala Ala Val Asp Thr Tyr Cys Arg His Asn Tyr Gly Val
   65 70 75 80
   Val Glu Ser Phe Thr Val Gln Arg
   85
<210> 490
   <211> 82
   <212> PRT
   <213> Homo sapiens
<400> 490
   Arg Phe Leu Glu Tyr Ser Thr Ser Glu Cys His Phe Phe Asn Gly Thr
   1 5 10 15
   Glu Arg Val Arg Phe Leu Asp Arg Tyr Phe Tyr Asn Gln Glu Glu Tyr
   20 25 30
   Val Arg Phe Asp Ser Asp Val Gly Glu Phe Arg Ala Val Thr Glu Leu
   35 40 45
   Gly Arg Pro Asp Ala Glu Tyr Trp Asn Ser Gln Lys Asp Leu Leu Glu
   50 55 60
   Asp Arg Arg Ala Ala Val Asp Thr Tyr Cys Arg His Asn Tyr Gly Val
   65 70 75 80
   Gly Glu
<210> 491
   <211> 89
   <212> PRT
   <213> Homo sapiens
<400> 491
   Arg Phe Leu Glu Tyr Ser Thr Ser Glu Cys His Phe Phe Asn Gly Thr
   1               5                   10                  15
   Glu Arg Val Arg Phe Leu Glu Arg Tyr Phe His Asn Gln Glu Glu Asn
               20                  25                  30
   Val Arg Phe Asp Ser Asp Val Gly Glu Tyr Arg Ala Val Thr Glu Leu
           35                  40                  45
   Gly Arg Pro Asp Ala Glu Tyr Trp Asn Ser Gln Lys Asp Phe Leu Glu
       50                  55                  60
   Asp Arg Arg Ala Ala Val Asp Thr Tyr Cys Arg His Asn Tyr Gly Val
   65                  70                  75                  80
   Gly Glu Ser Phe Thr Val Gln Arg Arg
                   85
<210> 492
   <211> 89
   <212> PRT
   <213> Homo sapiens
<400> 492
   Arg Phe Leu Glu Tyr Ser Thr Ser Glu Cys His Phe Phe Asn Gly Thr
   1               5                   10                  15
   Glu Arg Val Arg Tyr Leu Asp Arg Tyr Phe His Asn Gln Glu Glu Asn
               20                  25                  30
   Val Arg Phe Asp Ser Asp Val Gly Glu Phe Arg Ala Val Thr Glu Leu
           35                  40                  45
   Gly Arg Pro Asp Ala Glu Tyr Trp Asn Ser Gln Lys Asp Ile Leu Glu
       50                  55                  60
   Asp Glu Arg Ala Ala Val Asp Thr Tyr Cys Arg His Asn Tyr Gly Val
   65                  70                  75                  80
   Val Glu Ser Phe Thr Val Gln Arg Arg
                   85
<210> 493
   <211> 82
   <212> PRT
   <213> Homo sapiens
<400> 493
   Leu Glu Tyr Ser Thr Ser Glu Cys His Phe Phe Asn Gly Thr Glu Arg
   1               5                   10                  15
   Val Arg Phe Leu Asp Arg Tyr Phe His Asn Gln Glu Glu Asn Val Arg
               20                  25                  30
   Phe Asp Ser Asp Val Gly Glu Phe Arg Ala Val Thr Glu Leu Gly Arg
           35                  40                  45
   Pro Asp Ala Glu Tyr Trp Asn Ser Gln Lys Asp Ile Leu Glu Asp Glu
       50                  55                  60
   Arg Ala Ala Val Asp Thr Tyr Cys Arg His Asn Tyr Arg Val Val Glu
   65                  70                  75                  80
   Ser Phe
<210> 494
   <211> 89
   <212> PRT
   <213> Homo sapiens
<400> 494
   Arg Phe Leu Glu Tyr Ser Thr Ser Glu Cys His Phe Phe Asn Gly Thr
   1               5                   10                  15
   Glu Arg Val Arg Phe Leu Asp Arg Tyr Phe His Asn Gln Glu Glu Asn
               20                  25                  30
   Val Arg Phe Asp Ser Asp Val Gly Glu Phe Arg Ala Val Thr Glu Leu
           35                  40                  45
   Gly Arg Pro Asp Ala Glu Tyr Trp Asn Ser Gln Lys Asp Leu Leu Glu
       50                  55                  60
   Asp Glu Arg Ala Ala Val Asp Thr Tyr Cys Arg His Asn Tyr Gly Val
   65                  70                  75                  80
   Gly Glu Ser Phe Thr Val Gln Arg Arg
                   85
<210> 495
   <211> 84
   <212> PRT
   <213> Homo sapiens
<400> 495
   Phe Leu Glu Tyr Ser Thr Ser Glu Cys His Phe Phe Asn Gly Thr Glu
   1 5 10 15
   Arg Val Arg Phe Leu Asp Arg Tyr Phe Tyr Asn Gln Glu Glu Tyr Val
   20 25 30
   Arg Phe Asp Ser Asp Val Gly Glu Phe Arg Ala Val Thr Glu Leu Gly
   35 40 45
   Arg Pro Ser Ala Glu Tyr Trp Asn Ser Gln Lys Asp Ile Leu Glu Asp
   50 55 60
   Arg Arg Ala Ala Val Asp Thr Tyr Cys Arg His Asn Tyr Gly Val Gly
   65 70 75 80
   Glu Ser Phe Thr
<210> 496
   <211> 89
   <212> PRT
   <213> Homo sapiens
<400> 496
   Arg Phe Leu Glu Tyr Ser Thr Ser Glu Cys His Phe Phe Asn Gly Thr
   1 5 10 15
   Glu Arg Val Arg Phe Leu Asp Arg Tyr Phe His Asn Gln Glu Glu Asn
   20 25 30
   Val Arg Phe Asp Ser Asp Val Gly Glu Phe Arg Ala Val Thr Glu Leu
   35 40 45
   Gly Arg Pro Val Ala Glu Tyr Trp Asn Ser Gln Lys Asp Ile Leu Glu
   50 55 60
   Asp Glu Arg Ala Ala Val Asp Thr Tyr Cys Arg His Asn Tyr Gly Val
   65 70 75 80
   Gly Glu Ser Phe Thr Val Gln Arg Arg
   85
<210> 497
   <211> 89
   <212> PRT
   <213> Homo sapiens
<400> 497
   Arg Phe Leu Glu Tyr Ser Thr Ser Glu Cys His Phe Phe Asn Gly Thr
   1 5 10 15
   Glu Arg Val Arg Phe Leu Asp Arg Tyr Phe His Asn Gln Glu Glu Asn
   20 25 30
   Val Arg Phe Asp Ser Asp Val Gly Glu Tyr Arg Ala Val Thr Glu Leu
   35 40 45
   Gly Arg Pro Ser Ala Glu Tyr Trp Asn Ser Gln Lys Asp Ile Leu Glu
   50 55 60
   Asp Glu Arg Ala Ala Val Asp Thr Tyr Cys Arg His Asn Tyr Gly Val
   65 70 75 80
   Val Glu Ser Phe Thr Val Gln Arg Arg
   85
<210> 498
   <211> 81
   <212> PRT
   <213> Homo sapiens
<400> 498
   Arg Phe Leu Glu Tyr Ser Thr Ser Glu Cys His Phe Phe Asn Gly Thr
   1 5 10 15
   Glu Arg Val Arg Phe Leu Asp Arg Tyr Phe Tyr Asn Gln Glu Glu Tyr
   20 25 30
   Val Arg Phe Asp Ser Asp Val Gly Glu Tyr Arg Ala Val Thr Glu Leu
   35 40 45
   Gly Arg Pro Ser Ala Glu Tyr Trp Asn Ser Gln Lys Asp Ile Leu Glu
   50 55 60
   Asp Lys Arg Ala Ala Val Asp Asn Tyr Cys Arg His Asn Tyr Gly Val
   65 70 75 80
   Gly
<210> 499
   <211> 89
   <212> PRT
   <213> Homo sapiens
<400> 499
   Arg Phe Leu Glu Tyr Ser Thr Ser Glu Cys His Phe Phe Asn Gly Thr
   1 5 10 15
   Glu Arg Val Arg Phe Leu Asp Arg Tyr Phe His Asn Gln Glu Glu Asn
   20 25 30
   Leu Arg Phe Asp Ser Asp Val Gly Glu Phe Arg Ala Val Thr Glu Leu
   35 40 45
   Gly Arg Pro Asp Ala Glu Tyr Trp Asn Ser Gln Lys Asp Ile Leu Glu
   50 55 60
   Asp Glu Arg Ala Ala Val Asp Thr Tyr Cys Arg His Asn Tyr Gly Val
   65 70 75 80
   Gly Glu Ser Phe Thr Val His Arg Arg
   85
<210> 500
   <211> 88
   <212> PRT
   <213> Homo sapiens
<400> 500
   Arg Phe Leu Glu Tyr Ser Thr Ser Glu Cys His Phe Phe Asn Gly Thr
   1 5 10 15
   Glu Arg Val Arg Phe Leu Asp Arg Tyr Phe His Asn Gln Glu Glu Asn
   20 25 30
   Val Arg Phe Asp Ser Asp Val Gly Glu Phe Leu Ala Val Thr Glu Leu
   35 40 45
   Gly Arg Pro Asp Ala Glu Tyr Trp Asn Ser Gln Lys Asp Ile Leu Glu
   50 55 60
   Asp Glu Arg Ala Ala Val Asp Thr Tyr Cys Arg His Asn Tyr Gly Val
   65 70 75 80
   Val Glu Ser Phe Thr Val Gln Arg
   85
<210> 501
   <211> 89
   <212> PRT
   <213> Homo sapiens
<400> 501
   Arg Phe Leu Glu Tyr Ser Thr Ser Glu Cys His Phe Phe Asn Gly Thr
   1 5 10 15
   Glu Arg Val Arg Phe Leu Asp Arg Tyr Phe His Asn Gln Glu Glu Asn
   20 25 30
   Val Arg Phe Asp Ser Asp Val Gly Glu Tyr Arg Ala Val Thr Glu Leu
   35 40 45
   Gly Arg Pro Asp Ala Glu Tyr Trp Asn Ser Gln Lys Asp Ile Leu Glu
   50 55 60
   Asp Glu Arg Ala Ala Val Asp Thr Tyr Cys Arg His Asn Tyr Gly Val
   65 70 75 80
   Gly Glu Ser Phe Thr Val Gln Arg Arg
   85
<210> 502
   <211> 89
   <212> PRT
   <213> Homo sapiens
<400> 502
   Arg Phe Leu Glu Tyr Ser Thr Ser Glu Cys His Phe Phe Asn Gly Thr
   1 5 10 15
   Glu Arg Val Arg Phe Leu Asp Arg Tyr Phe Tyr Asn Gln Glu Glu Tyr
   20 25 30
   Val Arg Phe Asp Ser Asp Val Gly Glu Tyr Arg Ala Val Thr Glu Leu
   35 40 45
   Gly Arg Pro Asp Ala Glu Tyr Trp Asn Ser Gln Lys Asp Ile Leu Glu
   50 55 60
   Asp Lys Arg Ala Ala Val Asp Thr Tyr Cys Arg His Asn Tyr Gly Val
   65 70 75 80
   Gly Glu Ser Phe Thr Val Gln Arg Arg
   85
<210> 503
   <211> 81
   <212> PRT
   <213> Homo sapiens
<400> 503
   Arg Phe Leu Glu Tyr Ser Thr Ser Glu Cys His Phe Phe Asn Gly Thr
   1               5                   10                  15
   Glu Arg Val Arg Phe Leu Asp Arg Tyr Phe Tyr Asn Gln Glu Glu Tyr
               20                  25                  30
   Val Arg Phe Asp Ser Asp Val Gly Glu Tyr Arg Ala Val Thr Glu Leu
           35                  40                  45
   Gly Arg Pro Ser Ala Glu Tyr Trp Asn Ser Gln Lys Asp Ile Leu Glu
       50                  55                  60
   Asp Glu Arg Ala Ala Val Asp Thr Tyr Cys Arg His Asn Tyr Gly Val
   65                  70                  75                  80
   Gly

<210> 504
   <211> 89
   <212> PRT
   <213> Homo sapiens
<400> 504
   Arg Phe Leu Glu Tyr Ser Thr Ser Glu Cys His Phe Phe Asn Gly Thr
   1               5                   10                  15
   Glu Arg Val Arg Phe Leu Asp Arg Tyr Phe His Asn Gln Glu Glu Asn
               20                  25                  30
   Val Arg Phe Asp Ser Asp Val Gly Glu Phe Arg Ala Val Thr Glu Leu
           35                  40                  45
   Gly Arg Pro Asp Ala Glu Ser Trp Asn Ser Gln Lys Asp Ile Leu Glu
       50                  55                  60
   Asp Glu Arg Ala Ala Val Asp Thr Tyr Cys Arg His Asn Tyr Gly Val
   65                  70                  75                  80
   Gly Glu Ser Phe Thr Val Gln Arg Arg
                   85
<210> 505
   <211> 87
   <212> PRT
   <213> Homo sapiens
<400> 505
   Leu Glu Tyr Ser Thr Ser Glu Cys His Phe Phe Asn Gly Thr Glu Arg
   1 5 10 15
   Val Arg Phe Leu Asp Arg Tyr Phe His Asn Gln Glu Glu Asn Val Arg
   20 25 30
   Phe Asp Ser Asp Val Gly Glu Tyr Arg Ala Val Thr Glu Leu Gly Arg
   35 40 45
   Pro Asp Ala Glu Tyr Trp Asn Ser Gln Lys Asp Ile Leu Glu Asp Glu
   50 55 60
   Arg Ala Ala Val Asp Thr Tyr Cys Arg His Asn Tyr Gly Val Val Glu
   65 70 75 80
   Ser Phe Thr Val Gln Arg Arg
   85
<210> 506
   <211> 89
   <212> PRT
   <213> Homo sapiens
<400> 506
   Arg Phe Leu Glu Tyr Ser Thr Ser Glu Cys His Phe Phe Asn Gly Thr
   1 5 10 15
   Glu Arg Val Arg Tyr Leu Asp Arg Tyr Phe His Asn Gln Glu Glu Asn
   20 25 30
   Val Arg Phe Asp Ser Asp Val Gly Glu Phe Arg Ala Val Thr Glu Leu
   35 40 45
   Gly Arg Pro Asp Ala Glu Tyr Trp Asn Ser Gln Lys Asp Ile Leu Glu
   50 55 60
   Asp Glu Arg Ala Ala Val Asp Thr Tyr Cys Arg His Asn Tyr Gly Val
   65 70 75 80
   Gly Glu Ser Phe Thr Val Gln Arg Arg
   85
<210> 507
   <211> 89
   <212> PRT
   <213> Homo sapiens
<400> 507
   Arg Phe Leu Glu Tyr Ser Thr Ser Glu Cys His Phe Phe Asn Gly Thr
   1               5                   10                  15
   Glu Arg Val Arg Phe Leu Asp Arg Tyr Phe His Asn Gln Glu Glu Asn
               20                  25                  30
   Val Arg Phe Asp Ser Asp Val Gly Glu Phe Arg Ala Val Thr Glu Leu
           35                  40                  45
   Gly Arg Pro Asp Ala Glu Tyr Trp Asn Ser Gln Lys Asp Phe Leu Glu
       50                  55                  60
   Asp Arg Arg Ala Ala Val Asp Thr Tyr Cys Arg His Asn Tyr Gly Val
   65                  70                  75                  80
   Val Glu Ser Phe Thr Val Gln Arg Arg
                   85
<210> 508
   <211> 89
   <212> PRT
   <213> Homo sapiens
<400> 508
   Arg Phe Leu Glu Tyr Ser Thr Ser Glu Cys His Phe Phe Asn Gly Thr
   1               5                   10                  15
   Glu Arg Val Arg Phe Leu Asp Arg Tyr Phe His Asn Gln Glu Glu Asn
               20                  25                  30
   Val Arg Phe Asp Ser Asp Val Gly Glu Phe Arg Ala Val Thr Glu Leu
           35                  40                  45
   Gly Arg Pro Ala Ala Glu His Trp Asn Ser Gln Lys Asp Leu Leu Glu
       50                  55                  60
   Asp Glu Arg Ala Ala Val Asp Thr Tyr Cys Arg His Asn Tyr Gly Val
   65                  70                  75                  80
   Val Glu Ser Phe Thr Val Gln Arg Arg
                   85
<210> 509
   <211> 89
   <212> PRT
   <213> Homo sapiens
<400> 509
   Arg Phe Leu Glu Tyr Ser Thr Ser Glu Cys His Phe Phe Asn Gly Thr
   1               5                   10                  15
   Glu Arg Val Arg Phe Leu Asp Arg Tyr Phe Tyr Asn Gln Glu Glu Tyr
               20                  25                  30
   Val Arg Phe Asp Ser Asp Val Gly Glu Phe Arg Ala Val Thr Glu Leu
           35                  40                  45
   Gly Arg Pro Asp Ala Glu Tyr Trp Asn Ser Gln Lys Asp Leu Leu Glu
       50                  55                  60
   Gln Arg Arg Ala Ala Val Asp Thr Tyr Cys Arg His Asn Tyr Gly Val
   65                  70                  75                  80
   Val Glu Ser Phe Thr Val Gln Arg Arg
                   85
<210> 510
   <211> 80
   <212> PRT
   <213> Homo sapiens
<400> 510
   Leu Glu Tyr Ser Thr Ser Glu Cys His Phe Phe Asn Gly Thr Glu Arg
   1               5                   10                  15
   Val Arg Phe Leu Asp Arg Tyr Phe Tyr Asn Gln Glu Glu Tyr Val Arg
               20                  25                  30
   Phe Asp Ser Asp Val Gly Glu Phe Arg Ala Val Thr Glu Leu Gly Arg
           35                  40                  45
   Pro Ala Ala Glu His Trp Asn Ser Gln Lys Asp Ile Leu Glu Asp Glu
       50                  55                  60
   Arg Ala Ala Val Asp Thr Tyr Cys Arg His Asn Tyr Gly Val Gly Glu
   65                  70                  75                  80
<210> 511
   <211> 89
   <212> PRT
   <213> Homo sapiens
<400> 511
   Arg Phe Leu Glu Tyr Ser Thr Ser Glu Cys His Phe Phe Asn Gly Thr
   1               5                   10                  15
   Glu Arg Val Arg Phe Leu Asp Arg Tyr Phe Tyr Asn Gln Glu Glu Tyr
               20                  25                  30
   Val Arg Phe Asp Ser Asp Val Gly Glu Phe Arg Ala Val Thr Glu Leu
           35                  40                  45
   Gly Arg Pro Val Ala Glu Tyr Trp Asn Ser Gln Lys Asp Phe Leu Glu
       50                  55                  60
   Asp Arg Arg Ala Ala Val Asp Thr Tyr Cys Arg His Asn Tyr Gly Val
   65                  70                  75                  80
   Gly Glu Ser Phe Thr Val Gln Arg Arg
                   85
<210> 512
   <211> 89
   <212> PRT
   <213> Homo sapiens
<400> 512
   Arg Phe Leu Glu Tyr Ser Thr Ser Glu Cys His Phe Phe Asn Gly Thr
   1               5                   10                  15
   Glu Arg Val Arg Phe Leu Asp Arg Tyr Phe Tyr Asn Gln Glu Glu Tyr
               20                  25                  30
   Val Arg Phe Asp Ser Asp Val Gly Glu Tyr Arg Ala Val Thr Glu Leu
           35                  40                  45
   Gly Arg Pro Asp Ala Glu Tyr Trp Asn Ser Gln Lys Asp Phe Leu Glu
       50                  55                  60
   Asp Arg Arg Ala Leu Val Asp Thr Tyr Cys Arg His Asn Tyr Gly Val
   65                  70                  75                  80
   Gly Glu Ser Phe Thr Val Gln Arg Arg
                   85
<210> 513
   <211> 89
   <212> PRT
   <213> Homo sapiens
<400> 513
   Arg Phe Leu Glu Tyr Ser Thr Ser Glu Cys His Phe Phe Asn Gly Thr
   1               5                   10                  15
   Glu Arg Val Arg Phe Leu Asp Arg Tyr Phe Tyr Asn Gln Glu Glu Tyr
               20                  25                  30
   Val Arg Phe Asp Ser Asp Val Gly Glu Tyr Arg Ala Val Thr Glu Leu
           35                  40                  45
   Gly Arg Pro Ser Ala Glu Tyr Trp Asn Ser Gln Lys Asp Ile Leu Glu
       50                  55                  60
   Asp Glu Arg Ala Ala Val Asp Thr Tyr Cys Arg His Asn Tyr Gly Val
   65                  70                  75                  80
   Val Glu Ser Phe Thr Val Gln Arg Arg
                   85
<210> 514
   <211> 88
   <212> PRT
   <213> Homo sapiens
<400> 514
   Arg Phe Leu Glu Tyr Ser Thr Ser Glu Cys His Phe Phe Asn Gly Thr
   1               5                   10                  15
   Glu Arg Val Arg Phe Leu Asp Arg Tyr Phe Tyr Asn Gln Glu Glu Tyr
               20                  25                  30
   Val Arg Phe Asp Ser Asp Val Gly Glu Tyr Arg Ala Val Thr Glu Leu
           35                  40                  45
   Gly Arg Pro Ser Ala Glu Tyr Trp Asn Ser Gln Lys Asp Phe Leu Glu
       50                  55                  60
   Asp Arg Arg Ala Ala Val Asp Thr Tyr Cys Arg His Asn Tyr Gly Val
   65                  70                  75                  80
   Gly Glu Ser Phe Thr Val Gln Arg
                   85
<210> 515
   <211> 94
   <212> PRT
   <213> Homo sapiens
<400> 515
   Gly Asp Thr Arg Pro Arg Phe Leu Glu Tyr Ser Thr Ser Glu Cys His
   1 5 10 15
   Phe Phe Asn Gly Thr Glu Arg Val Arg Phe Leu Asp Arg Tyr Phe His
   20 25 30
   Asn Gln Glu Glu Phe Val Arg Phe Asp Ser Asp Val Gly Glu Tyr Arg
   35 40 45
   Ala Val Thr Glu Leu Gly Arg Pro Ala Ala Glu His Trp Asn Ser Gln
   50 55 60
   Lys Asp Leu Leu Glu Arg Arg Arg Ala Glu Val Asp Thr Tyr Cys Arg
   65 70 75 80
   His Asn Tyr Gly Val Val Glu Ser Phe Thr Val Gln Arg Arg
   85 90
<210> 516
   <211> 81
   <212> PRT
   <213> Homo sapiens
<400> 516
   Arg Phe Leu Glu Tyr Ser Thr Ser Glu Cys His Phe Phe Asn Gly Thr
   1 5 10 15
   Glu Arg Val Arg Phe Leu Asp Arg Tyr Phe His Asn Gln Glu Glu Phe
   20 25 30
   Val Arg Phe Asp Ser Asp Val Gly Glu Tyr Arg Ala Val Thr Glu Leu
   35 40 45
   Gly Arg Pro Ala Ala Glu His Trp Asn Ser Gln Lys Asp Leu Leu Glu
   50 55 60
   Arg Arg Arg Ala Glu Val Asp Thr Tyr Cys Arg His Asn Tyr Gly Val
   65 70 75 80
   Val
<210> 517
   <211> 94
   <212> PRT
   <213> Homo sapiens
<400> 517
   Gly Asp Thr Arg Pro Arg Phe Leu Glu Tyr Ser Thr Ser Glu Cys His
   1               5                   10                  15
   Phe Phe Asn Gly Thr Glu Arg Val Arg Phe Leu Glu Arg Tyr Phe His
               20                  25                  30
   Asn Gln Glu Glu Asn Val Arg Phe Asp Ser Asp Val Gly Glu Tyr Arg
           35                  40                  45
   Ala Val Thr Glu Leu Gly Arg Pro Asp Ala Glu Tyr Trp Asn Ser Gln
       50                  55                  60
   Lys Asp Leu Leu Glu Gln Arg Arg Ala Ala Val Asp Thr Tyr Cys Arg
   65                  70                  75                  80
   His Asn Tyr Gly Val Gly Glu Ser Phe Thr Val Gln Arg Arg
                   85                  90
<210> 518
   <211> 94
   <212> PRT
   <213> Homo sapiens
<400> 518
<210> 519
   <211> 89
   <212> PRT
   <213> Homo sapiens
<400> 519
   Arg Phe Leu Glu Tyr Ser Thr Gly Glu Cys Tyr Phe Phe Asn Gly Thr
   1 5 10 15
   Glu Arg Val Arg Phe Leu Asp Arg Tyr Phe His Asn Gln Glu Glu Phe
   20 25 30
   Val Arg Phe Asp Ser Asp Val Gly Glu Tyr Arg Ala Val Thr Glu Leu
   35 40 45
   Gly Arg Pro Ala Ala Glu His Trp Asn Ser Gln Lys Asp Leu Leu Glu
   50 55 60
   Arg Arg Arg Ala Glu Val Asp Thr Tyr Cys Arg His Asn Tyr Gly Val
   65 70 75 80
   Val Glu Ser Phe Thr Val Gln Arg Arg
   85
<210> 520
   <211> 88
   <212> PRT
   <213> Homo sapiens
<400> 520
   Phe Leu Glu Tyr Ser Thr Ser Glu Cys Gln Phe Phe Asn Gly Thr Glu
   1 5 10 15
   Arg Val Arg Phe Leu Asp Arg Tyr Phe His Asn Gln Glu Glu Phe Val
   20 25 30
   Arg Phe Asp Ser Asp Val Gly Glu Tyr Arg Ala Val Thr Glu Leu Gly
   35 40 45
   Arg Pro Asp Ala Glu Tyr Trp Asn Ser Gln Lys Asp Leu Leu Glu Arg
   50 55 60
   Arg Arg Ala Glu Val Asp Thr Tyr Cys Arg His Asn Tyr Gly Val Val
   65 70 75 80
   Glu Ser Phe Thr Val Gln Arg Arg
   85
<210> 521
   <211> 88
   <212> PRT
   <213> Homo sapiens
<400> 521
   Phe Leu Glu Tyr Ser Thr Ser Glu Cys His Phe Phe Asn Gly Thr Glu
   1               5                   10                  15
   Arg Val Arg Phe Leu Glu Arg Tyr Phe His Asn Gln Glu Glu Asn Val
               20                  25                  30
   Arg Phe Asp Ser Asp Val Gly Glu Tyr Arg Ala Val Thr Glu Leu Gly
           35                  40                  45
   Arg Pro Asp Ala Glu Tyr Trp Asn Ser Gln Lys Asp Leu Leu Glu Gln
       50                  55                  60
   Arg Arg Ala Ala Val Asp Thr Tyr Cys Arg His Asn Tyr Gly Val Val
   65                  70                  75                  80
   Glu Ser Phe Thr Val Gln Arg Arg
                   85
<210> 522
   <211> 78
   <212> PRT
   <213> Homo sapiens
<400> 522
   Glu Tyr Ser Thr Ser Glu Cys His Phe Phe Asn Gly Thr Glu Arg Val
   1               5                   10                  15
   Arg Phe Leu Asp Arg Tyr Phe His Asn Gln Glu Glu Phe Val Arg Phe
               20                  25                  30
   Asp Ser Asp Val Gly Glu Tyr Arg Ala Val Thr Glu Leu Gly Arg Pro
           35                  40                  45
   Ala Ala Glu His Trp Asn Ser Gln Lys Asp Leu Leu Glu Arg Arg Arg
       50                  55                  60
   Ala Glu Val Asp Thr Tyr Cys Arg His Asn Tyr Gly Val Gly
   65                  70                  75
<210> 523
   <211> 87
   <212> PRT
   <213> Homo sapiens
<400> 523
   Phe Leu Glu Tyr Ser Thr Ser Glu Cys His Phe Phe Asn Gly Thr Glu
   1               5                   10                  15
   Arg Val Arg Phe Leu Asp Arg Tyr Phe His Asn Gln Glu Glu Phe Val
               20                  25                  30
   Arg Phe Asp Ser Asp Val Gly Glu Tyr Arg Ala Val Thr Glu Leu Gly
           35                  40                  45
   Arg Pro Asp Ala Glu His Trp Asn Ser Gln Lys Asp Leu Leu Glu Arg
       50                  55                  60
   Arg Arg Ala Glu Val Asp Thr Tyr Cys Arg His Asn Tyr Gly Val Val
   65                  70                  75                  80
   Glu Ser Phe Thr Val Gln Arg
                   85
<210> 524
   <211> 87
   <212> PRT
   <213> Homo sapiens
<400> 524
   Phe Leu Glu Tyr Ser Thr Ser Glu Cys His Phe Phe Asn Gly Thr Glu
   1               5                   10                  15
   Arg Val Arg Phe Leu Asp Arg Tyr Phe His Asn Gln Glu Glu Asn Val
               20                  25                  30
   Arg Phe Asp Ser Asp Val Gly Glu Tyr Arg Ala Val Thr Glu Leu Gly
           35                  40                  45
   Arg Pro Asp Ala Glu Tyr Trp Asn Ser Gln Lys Asp Leu Leu Glu Gln
       50                  55                  60
   Arg Arg Ala Ala Val Asp Thr Tyr Cys Arg His Asn Tyr Gly Val Gly
   65                  70                  75                  80
   Glu Ser Phe Thr Val Gln Arg
                   85
<210> 525
   <211> 87
   <212> PRT
   <213> Homo sapiens
<400> 525
   Phe Leu Glu Gln Val Lys His Glu Cys His Phe Phe Asn Gly Thr Glu
   1               5                   10                  15
   Arg Val Arg Phe Leu Asp Arg Tyr Phe His Asn Gln Glu Glu Phe Val
               20                  25                  30
   Arg Phe Asp Ser Asp Val Gly Glu Tyr Arg Ala Val Thr Glu Leu Gly
           35                  40                  45
   Arg Pro Ala Ala Glu His Trp Asn Ser Gln Lys Asp Leu Leu Glu Arg
       50                  55                  60
   Arg Arg Ala Glu Val Asp Thr Tyr Cys Arg His Asn Tyr Gly Val Val
   65                  70                  75                  80
   Glu Ser Phe Thr Val Gln Arg
                   85
<210> 526
   <211> 78
   <212> PRT
   <213> Homo sapiens
<400> 526
   Glu Tyr Ser Thr Gly Glu Cys Tyr Phe Phe Asn Gly Thr Glu Arg Val
   1               5                   10                  15
   Arg Phe Leu Asp Arg Tyr Phe His Asn Gln Glu Glu Phe Val Arg Phe
               20                  25                  30
   Asp Ser Asp Val Gly Glu Tyr Arg Ala Val Thr Glu Leu Gly Arg Pro
           35                  40                  45
   Asp Glu Glu Tyr Trp Asn Ser Gln Lys Asp Leu Leu Glu Arg Arg Arg
       50                  55                  60
   Ala Glu Val Asp Thr Tyr Cys Arg His Asn Tyr Gly Val Val
   65                  70                  75

<210> 527
   <211> 74
   <212> PRT
   <213> Homo sapiens
<400> 527
   Ser Glu Cys His Phe Phe Asn Gly Thr Glu Arg Val Arg Phe Leu Glu
   1               5                   10                  15
   Arg Tyr Phe His Asn Gln Glu Glu Asn Val Arg Phe Asp Ser Asp Val
               20                  25                  30
   Gly Glu Tyr Arg Ala Val Thr Glu Leu Gly Arg Pro Asp Ala Glu Tyr
           35                  40                  45
   Trp Asn Ser Gln Lys Asp Leu Leu Glu Asp Arg Arg Ala Leu Val Asp
       50                  55                  60
   Thr Tyr Cys Arg His Asn Tyr Gly Val Val
   65                  70
<210> 528
   <211> 78
   <212> PRT
   <213> Homo sapiens
<400> 528
   Glu Tyr Ser Thr Ser Glu Cys His Phe Phe Asn Gly Thr Glu Arg Val
   1               5                   10                  15
   Arg Phe Leu Glu Arg Tyr Phe His Asn Gln Glu Glu Asn Val Arg Phe
               20                  25                  30
   Asp Ser Asp Val Gly Glu Tyr Arg Ala Val Thr Glu Leu Gly Arg Pro
           35                  40                  45
   Ser Ala Glu Tyr Trp Asn Ser Gln Lys Asp Leu Leu Glu Gln Arg Arg
       50                  55                  60
   Ala Ala Val Asp Thr Tyr Cys Arg His Asn Tyr Gly Val Gly
   65                  70                  75
<210> 529
   <211> 85
<212> PRT
   <213> Homo sapiens
<400> 529
   Phe Leu Glu Tyr Ser Thr Ser Glu Cys His Phe Phe Asn Gly Thr Glu
   1               5                   10                  15
   Arg Val Arg Phe Leu Asp Arg Tyr Phe His Asn Gln Glu Glu Phe Val
               20                  25                  30
   Arg Phe Asp Ser Asp Val Gly Glu Tyr Arg Ala Val Thr Glu Leu Gly
           35                  40                  45
   Arg Pro Asp Ala Glu Tyr Trp Asn Ser Gln Lys Asp Leu Leu Glu Arg
       50                  55                  60
   Arg Arg Ala Glu Val Asp Thr Tyr Cys Arg His Asn Tyr Gly Val Gly
   65                  70                  75                  80
   Glu Ser Phe Thr Val
                   85
<210> 530
   <211> 82
   <212> PRT
   <213> Homo sapiens
<400> 530
   Ser Thr Gly Glu Cys Tyr Phe Phe Asn Gly Thr Glu Arg Val Arg Phe
   1               5                   10                  15
   Leu Asp Arg Tyr Phe His Asn Gln Glu Glu Phe Val Arg Phe Asp Ser
               20                  25                  30
   Asp Val Gly Glu Tyr Arg Ala Val Thr Glu Leu Gly Arg Pro Asp Ala
           35                  40                  45
   Glu Tyr Trp Asn Ser Gln Lys Asp Phe Leu Glu Asp Arg Arg Ala Leu
       50                  55                  60
   Val Asp Thr Tyr Cys Arg His Asn Tyr Gly Val Val Glu Ser Phe Thr
   65                  70                  75                  80
   Val Gln
<210> 531
   <211> 80
   <212> PRT
   <213> Homo sapiens
<400> 531
   Leu Glu Tyr Ser Thr Ser Glu Cys His Phe Phe Asn Gly Thr Glu Arg
   1               5                   10                  15
   Val Arg Phe Leu Asp Arg Tyr Phe His Asn Gln Glu Glu Phe Val Arg
               20                  25                  30
   Phe Asp Ser Asp Val Gly Glu Tyr Arg Ala Val Thr Glu Leu Gly Arg
           35                  40                  45
   Pro Ala Ala Glu His Trp Asn Ser Gln Lys Asp Ile Leu Glu Asp Glu
       50                  55                  60
   Arg Ala Ala Val Asp Thr Tyr Cys Arg His Asn Tyr Gly Val Val Glu
   65                  70                  75                  80
<210> 532
   <211> 88
   <212> PRT
   <213> Homo sapiens
<400> 532
   Arg Phe Leu Glu Tyr Ser Thr Ser Glu Cys His Phe Phe Asn Gly Thr
   1               5                   10                  15
   Glu Arg Val Arg Phe Leu Asp Arg Tyr Phe His Asn Gln Glu Glu Asn
               20                  25                  30
   Val Arg Phe Asp Ser Asp Val Gly Glu Phe Arg Ala Val Thr Glu Leu
           35                  40                  45
   Gly Arg Pro Asp Ala Glu Tyr Trp Asn Ser Gln Lys Asp Leu Leu Glu
       50                  55                  60
   Gln Arg Arg Ala Ala Val Asp Thr Tyr Cys Arg His Asn Tyr Gly Val
   65                  70                  75                  80
   Val Glu Ser Phe Thr Val Gln Arg
                   85
<210> 533
   <211> 86
   <212> PRT
   <213> Homo sapiens
<400> 533
   Glu Tyr Ser Thr Ser Glu Cys His Phe Phe Asn Gly Thr Glu Arg Val
   1               5                   10                  15
   Arg Phe Leu Glu Arg Tyr Phe His Asn Gln Glu Glu Asn Val Arg Phe
               20                  25                  30
   Asp Ser Asp Val Gly Glu Tyr Arg Ala Val Thr Glu Leu Gly Arg Pro
           35                  40                  45
   Asp Ala Glu Tyr Trp Asn Ser Gln Lys Asp Leu Leu Glu Arg Arg Arg
       50                  55                  60
   Ala Glu Val Asp Thr Tyr Cys Arg His Asn Tyr Gly Val Val Glu Ser
   65                  70                  75                  80
   Phe Thr Val Gln Arg Arg
                   85
<210> 534
   <211> 90
   <212> PRT
   <213> Homo sapiens
<400> 534
   Gly Asp Thr Arg Pro Arg Phe Leu Glu Tyr Ser Thr Ser Glu Cys His
   1               5                   10                  15
   Phe Phe Asn Gly Thr Glu Arg Val Arg Phe Leu Glu Arg Tyr Phe His
               20                  25                  30
   Asn Gln Glu Glu Asn Val Arg Phe Asp Ser Asp Val Gly Glu Tyr Arg
           35                  40                  45
   Ala Val Thr Glu Leu Gly Arg Pro Asp Ala Glu Tyr Trp Asn Ser Gln
       50                  55                  60
   Lys Asp Leu Leu Glu Gln Lys Arg Ala Ala Val Asp Thr Tyr Cys Arg
   65                  70                  75                  80
   His Asn Tyr Gly Val Gly Glu Ser Phe Thr
                   85                  90
<210> 535
   <211> 80
   <212> PRT
   <213> Homo sapiens
<400> 535
   Leu Glu Tyr Ser Thr Ser Glu Cys His Phe Phe Asn Gly Thr Glu Arg
   1 5 10 15
   Val Arg Phe Leu Glu Arg Tyr Phe His Asn Gln Glu Glu Phe Val Arg
   20 25 30
   Phe Asp Ser Asp Val Gly Glu Tyr Arg Ala Val Thr Glu Leu Gly Arg
   35 40 45
   Pro Asp Ala Glu Tyr Trp Asn Ser Gln Lys Asp Leu Leu Glu Gln Arg
   50 55 60
   Arg Ala Ala Val Asp Thr Tyr Cys Arg His Asn Tyr Gly Val Val Glu
   65 70 75 80
<210> 536
   <211> 80
   <212> PRT
   <213> Homo sapiens
<400> 536
   Leu Glu Tyr Ser Thr Ser Glu Cys His Phe Phe Asn Gly Thr Glu Arg
   1 5 10 15
   Val Arg Phe Leu Asp Arg Tyr Phe His Asn Gln Glu Glu Asn Val Arg
   20 25 30
   Phe Asp Ser Asp Val Gly Glu Phe Arg Ala Val Thr Glu Leu Gly Arg
   35 40 45
   Pro Asp Ala Glu Tyr Trp Asn Ser Gln Lys Asp Leu Leu Glu Gln Lys
   50 55 60
   Arg Ala Ala Val Asp Thr Tyr Cys Arg His Asn Tyr Gly Val Val Glu
   65 70 75 80
<210> 537
   <211> 89
   <212> PRT
   <213> Homo sapiens
<400> 537
   Arg Phe Leu Glu Tyr Ser Thr Ser Glu Cys His Phe Phe Asn Gly Thr
   1               5                   10                  15
   Glu Arg Val Arg Phe Leu Asp Arg Tyr Phe His Asn Gln Glu Glu Phe
               20                  25                  30
   Val Arg Phe Asp Ser Asp Val Gly Glu Tyr Arg Ala Val Thr Glu Leu
           35                  40                  45
   Gly Arg Pro Ala Ala Glu His Trp Asn Ser Gln Lys Asp Phe Leu Glu
       50                  55                  60
   Asp Arg Arg Ala Ala Val Asp Thr Tyr Cys Arg His Asn Tyr Gly Val
   65                  70                  75                  80
   Gly Glu Ser Phe Thr Val Gln Arg Arg
                   85
<210> 538
   <211> 89
   <212> PRT
   <213> Homo sapiens
<400> 538
   Arg Phe Leu Glu Tyr Ser Thr Ser Glu Cys His Phe Phe Asn Gly Thr
   1               5                   10                  15
   Glu Arg Val Arg Phe Leu Asp Arg Tyr Phe His Asn Gln Glu Glu Phe
               20                  25                  30
   Val Arg Phe Asp Ser Asp Val Gly Glu Tyr Arg Ala Val Thr Glu Leu
           35                  40                  45
   Gly Arg Pro Asp Ala Glu Tyr Trp Asn Ser Gln Lys Asp Leu Leu Glu
       50                  55                  60
   Arg Arg Arg Ala Glu Val Asp Thr Tyr Cys Arg His Asn Tyr Gly Val
   65                  70                  75                  80
   Val Glu Ser Phe Thr Val Gln Arg Arg
                   85
<210> 539
   <211> 96
   <212> PRT
   <213> Homo sapiens
<400> 539
   Phe Leu Glu Tyr Ser Thr Ser Glu Cys His Phe Phe Asn Gly Thr Glu
   1 5 10 15
   Arg Val Arg Phe Leu Glu Arg Tyr Phe His Asn Gln Glu Glu Asn Val
   20 25 30
   Arg Phe Asp Ser Asp Val Gly Glu Tyr Arg Ala Val Thr Glu Leu Gly
   35 40 45
   Arg Pro Asp Ala Glu Tyr Trp Asn Ser Gln Lys Asp Ile Leu Glu Gln
   50 55 60
   Ala Arg Ala Ala Val Asp Thr Tyr Cys Arg His Asn Tyr Gly Val Gly
   65 70 75 80
   Glu Ser Phe Thr Val Gln Arg Arg Val His Pro Lys Val Thr Val Tyr
   85 90 95
<210> 540
   <211> 78
   <212> PRT
   <213> Homo sapiens
<400> 540
   Glu Tyr Ser Thr Ser Glu Cys His Phe Phe Asn Gly Thr Glu Arg Val
   1 5 10 15
   Arg Phe Leu Asp Arg Tyr Phe Tyr Asn Gln Glu Glu Tyr Val Arg Phe
   20 25 30
   Asp Ser Asp Val Gly Glu Tyr Arg Ala Val Thr Glu Leu Gly Arg Pro
   35 40 45
   Ala Ala Glu His Trp Asn Ser Gln Lys Asp Phe Leu Glu Asp Arg Arg
   50 55 60
   Ala Ala Val Asp Thr Tyr Cys Arg His Asn Tyr Gly Val Gly
   65 70 75
<210> 541
   <211> 89
   <212> PRT
   <213> Homo sapiens
<400> 541
   Arg Phe Leu Glu Tyr Ser Thr Ser Glu Cys His Phe Phe Asn Gly Thr
   1               5                   10                  15
   Glu Arg Val Gln Phe Leu Asp Arg Tyr Phe His Asn Gln Glu Glu Phe
               20                  25                  30
   Val Arg Phe Asp Ser Asp Val Gly Glu Tyr Arg Ala Val Thr Glu Leu
           35                  40                  45
   Gly Arg Pro Ala Ala Glu His Trp Asn Ser Gln Lys Asp Leu Leu Glu
       50                  55                  60
   Arg Arg Arg Ala Glu Val Asp Thr Tyr Cys Arg His Asn Tyr Gly Val
   65                  70                  75                  80
   Val Glu Ser Phe Thr Val Gln Arg Arg
                   85
<210> 542
   <211> 89
   <212> PRT
   <213> Homo sapiens
<400> 542
   Arg Phe Leu Glu Tyr Ser Thr Ser Glu Cys His Phe Phe Asn Gly Thr
   1               5                   10                  15
   Glu Arg Val Arg Phe Leu Glu Arg Tyr Phe His Asn Gln Glu Glu Asn
               20                  25                  30
   Val Arg Phe Asp Ser Asp Val Gly Glu Tyr Arg Ala Val Thr Glu Leu
           35                  40                  45
   Gly Arg Pro Asp Ala Glu Tyr Trp Asn Ser Gln Lys Asp Phe Leu Glu
       50                  55                  60
   Asp Arg Arg Ala Leu Val Asp Thr Tyr Cys Arg His Asn Tyr Gly Val
   65                  70                  75                  80
   Gly Glu Ser Phe Thr Val Gln Arg Arg
                   85
<210> 543
   <211> 81
   <212> PRT
   <213> Homo sapiens
<400> 543
   Arg Phe Leu Glu Tyr Ser Thr Gly Glu Cys Tyr Phe Phe Asn Gly Thr
   1               5                   10                  15
   Glu Arg Val Arg Phe Leu Asp Arg Tyr Phe His Asn Gln Glu Glu Phe
               20                  25                  30
   Val Arg Phe Asp Ser Asp Val Gly Glu Tyr Arg Ala Val Thr Glu Leu
           35                  40                  45
   Gly Arg Pro Ala Ala Glu His Trp Asn Ser Gln Lys Asp Leu Leu Glu
       50                  55                  60
   Arg Arg Arg Ala Glu Val Asp Thr Tyr Cys Arg His Asn Tyr Gly Ala
   65                  70                  75                  80
   Val
<210> 544
   <211> 89
   <212> PRT
   <213> Homo sapiens
<400> 544
   Arg Phe Leu Glu Tyr Ser Thr Ser Glu Cys His Phe Phe Asn Gly Thr
   1               5                   10                  15
   Glu Arg Val Arg Phe Leu Glu Arg Tyr Phe His Asn Gln Glu Glu Asn
               20                  25                  30
   Val Arg Phe Asp Ser Asp Val Gly Glu Tyr Arg Ala Val Thr Glu Leu
           35                  40                  45
   Gly Arg Pro Asp Ala Glu Tyr Trp Asn Ser Gln Lys Asp Leu Leu Glu
       50                  55                  60
   Gln Arg Arg Ala Ala Val Asp Thr Tyr Cys Arg His Asn Tyr Gly Ala
   65                  70                  75                  80
   Val Glu Ser Phe Thr Val Gln Arg Arg
                   85
<210> 545
   <211> 84
   <212> PRT
   <213> Homo sapiens
<400> 545
   Phe Leu Glu Tyr Ser Thr Ser Glu Cys His Phe Phe Asn Gly Thr Glu
   1               5                   10                  15
   Arg Val Arg Phe Leu Asp Arg Tyr Phe His Asn Gln Glu Glu Asn Val
               20                  25                  30
   Arg Phe Asp Ser Asp Val Gly Glu Phe Arg Ala Val Thr Glu Leu Gly
           35                  40                  45
   Arg Pro Asp Ala Glu Tyr Trp Asn Ser Gln Lys Asp Leu Leu Glu Gln
       50                  55                  60
   Arg Arg Ala Ala Val Asp Thr Tyr Cys Arg His Asn Tyr Gly Val Gly
   65                  70                  75                  80
   Glu Ser Phe Thr
<210> 546
   <211> 84
   <212> PRT
   <213> Homo sapiens
<400> 546
   Phe Leu Glu Tyr Ser Thr Gly Glu Cys Tyr Phe Phe Asn Gly Thr Glu
   1               5                   10                  15
   Arg Val Arg Phe Leu Asp Arg Tyr Phe His Asn Gln Glu Glu Phe Val
               20                  25                  30
   Arg Phe Asp Ser Asp Val Gly Glu Tyr Arg Ala Val Thr Glu Leu Gly
           35                  40                  45
   Arg Pro Ala Ala Glu His Trp Asn Ser Gln Lys Asp Leu Leu Glu Arg
       50                  55                  60
   Arg Arg Ala Ala Val Asp Thr Tyr Cys Arg His Asn Tyr Gly Val Val
   65                  70                  75                  80
   Glu Ser Phe Thr
<210> 547
   <211> 89
   <212> PRT
   <213> Homo sapiens
<400> 547
   Arg Phe Leu Glu Tyr Ser Thr Ser Glu Cys His Phe Phe Asn Gly Thr
   1               5                   10                  15
   Glu Arg Val Arg Phe Leu Asp Arg Tyr Phe His Asn Gln Glu Glu Phe
               20                  25                  30
   Val Arg Phe Asp Ser Asp Val Gly Glu Tyr Arg Ala Val Thr Glu Leu
           35                  40                  45
   Gly Arg Pro Ala Ala Glu His Trp Asn Ser Gln Lys Asp Leu Leu Glu
       50                  55                  60
   Arg Arg Arg Ala Ala Val Asp Thr Tyr Cys Arg His Asn Tyr Gly Val
   65                  70                  75                  80
   Val Glu Ser Phe Thr Val Gln Arg Arg
                   85
<210> 548
   <211> 86
   <212> PRT
   <213> Homo sapiens
<400> 548
   Leu Glu Tyr Ser Thr Ser Glu Cys His Phe Phe Asn Gly Thr Glu Arg
   1               5                   10                  15
   Val Arg Phe Leu Asp Arg Tyr Phe His Asn Gln Glu Glu Asn Val Arg
               20                  25                  30
   Phe Asp Ser Asp Val Gly Glu Phe Arg Ala Val Thr Glu Leu Gly Arg
           35                  40                  45
   Pro Asp Ala Glu Tyr Trp Asn Ser Gln Lys Asp Leu Leu Glu Gln Arg
       50                  55                  60
   Arg Ala Glu Val Asp Thr Tyr Cys Arg His Asn Tyr Gly Val Val Glu
   65                  70                  75                  80
   Ser Phe Thr Val Gln Arg
                   85
<210> 549
   <211> 89
   <212> PRT
   <213> Homo sapiens
<400> 549
   Arg Phe Leu Glu Tyr Ser Thr Ser Glu Cys His Phe Phe Asn Gly Thr
   1 5 10 15
   Glu Arg Val Arg Phe Leu Asp Arg Tyr Phe His Asn Gln Glu Glu Phe
   20 25 30
   Val Arg Phe Asp Ser Asp Val Gly Glu Tyr Arg Ala Val Thr Glu Leu
   35 40 45
   Gly Arg Pro Asp Ala Glu His Trp Asn Ser Gln Lys Asp Leu Leu Glu
   50 55 60
   Arg Arg Arg Ala Ala Val Asp Thr Tyr Cys Arg His Asn Tyr Gly Val
   65 70 75 80
   Val Glu Ser Phe Thr Val Gln Arg Arg
   85

<210> 550
   <211> 88
   <212> PRT
   <213> Homo sapiens
<400> 550
   Arg Phe Leu Glu Tyr Ser Thr Ser Glu Cys His Phe Phe Asn Gly Thr
   1 5 10 15
   Glu Arg Val Arg Phe Leu Asp Arg Tyr Phe His Asn Gln Glu Glu Phe
   20 25 30
   Val Arg Phe Asp Ser Asp Val Gly Glu Phe Arg Ala Val Thr Glu Leu
   35 40 45
   Gly Arg Pro Ala Ala Glu His Trp Asn Ser Gln Lys Asp Leu Leu Glu
   50 55 60
   Arg Arg Arg Ala Glu Val Asp Thr Tyr Cys Arg His Asn Tyr Gly Val
   65 70 75 80
   Val Glu Ser Phe Thr Val Gln Arg
   85
<210> 551
   <211> 89
   <212> PRT
   <213> Homo sapiens
<400> 551
   Arg Phe Leu Glu Tyr Ser Thr Ser Glu Cys His Phe Phe Asn Gly Thr
   1 5 10 15
   Glu Arg Val Arg Phe Leu Asp Arg Tyr Phe His Asn Gln Glu Glu Phe
   20 25 30
   Val Arg Phe Asp Ser Asp Val Arg Glu Tyr Arg Ala Val Thr Glu Leu
   35 40 45
   Gly Arg Pro Asp Ala Glu Tyr Trp Asn Ser Gln Lys Asp Leu Leu Glu
   50 55 60
   Arg Arg Arg Ala Glu Val Asp Thr Tyr Cys Arg His Asn Tyr Gly Val
   65 70 75 80
   Gly Glu Ser Phe Thr Val Gln Arg Arg
   85
<210> 552
   <211> 89
   <212> PRT
   <213> Homo sapiens
<400> 552
   Arg Phe Leu Glu Tyr Ser Thr Ser Glu Cys Gln Phe Phe Asn Gly Thr
   1 5 10 15
   Glu Arg Val Arg Phe Leu Asp Arg Tyr Phe His Asn Gln Glu Glu Phe
   20 25 30
   Val Arg Phe Asp Ser Asp Val Gly Glu Tyr Arg Ala Val Thr Glu Leu
   35 40 45
   Gly Arg Pro Asp Ala Glu Tyr Trp Asn Ser Gln Lys Asp Ile Leu Glu
   50 55 60
   Gln Ala Arg Ala Ala Val Asp Thr Tyr Cys Arg His Asn Tyr Gly Val
   65 70 75 80
   Val Glu Ser Phe Thr Val Gln Arg Arg
   85
<210> 553
   <211> 89
   <212> PRT
   <213> Homo sapiens
<400> 553
   Arg Phe Leu Glu Tyr Ser Thr Ser Glu Cys His Phe Phe Asn Gly Thr
   1               5                   10                  15
   Glu Arg Val Arg Phe Leu Asp Arg Tyr Phe His Asn Gln Glu Glu Phe
               20                  25                  30
   Val Arg Phe Asp Ser Asp Val Gly Glu Tyr Arg Ala Val Thr Glu Leu
           35                  40                  45
   Gly Arg Pro Ala Ala Glu His Trp Asn Ser Gln Lys Asp Leu Leu Glu
       50                  55                  60
   Arg Arg Arg Ala Glu Val Asp Asn Tyr Cys Arg His Asn Tyr Gly Val
   65                  70                  75                  80
   Val Glu Ser Phe Thr Val Gln Arg Arg
                   85
<210> 554
   <211> 89
   <212> PRT
   <213> Homo sapiens
<400> 554
   Arg Phe Leu Glu Tyr Pro Thr Ser Glu Cys His Phe Phe Asn Gly Thr
   1               5                   10                  15
   Glu Arg Val Arg Phe Leu Asp Arg Tyr Phe His Asn Gln Glu Glu Phe
               20                  25                  30
   Val Arg Phe Asp Ser Asp Val Gly Glu Tyr Arg Ala Val Thr Glu Leu
           35                  40                  45
   Gly Arg Pro Ala Ala Glu His Trp Asn Ser Gln Lys Asp Leu Leu Glu
       50                  55                  60
   Arg Arg Arg Ala Glu Val Asp Thr Tyr Cys Arg His Asn Tyr Gly Val
   65                  70                  75                  80
   Val Glu Ser Phe Thr Val Gln Arg Arg
                   85
<210> 555
   <211> 89
   <212> PRT
   <213> Homo sapiens
<400> 555
   Arg Phe Leu Glu Tyr Ser Thr Ser Glu Cys His Phe Phe Asn Gly Thr
   1 5 10 15
   Glu Arg Val Arg Phe Leu Glu Arg Tyr Phe His Asn Gln Glu Glu Phe
   20 25 30
   Val Arg Phe Asp Ser Asp Val Gly Glu Tyr Arg Ala Val Thr Glu Leu
   35 40 45
   Gly Arg Pro Asp Ala Glu Tyr Trp Asn Ser Gln Lys Asp Leu Leu Glu
   50 55 60
   Asp Arg Arg Ala Leu Val Asp Thr Tyr Cys Arg His Asn Tyr Gly Val
   65 70 75 80
   Gly Glu Ser Phe Thr Val Gln Arg Arg
   85
<210> 556
   <211> 266
   <212> PRT
   <213> Homo sapiens
<400> 556
   Met Val Cys Leu Lys Leu Pro Gly Gly Ser Cys Met Thr Ala Leu Thr
   1 5 10 15
   Val Thr Leu Met Val Leu Ser Ser Pro Leu Ala Leu Ser Gly Asp Thr
               20                  25                  30
   Arg Pro Arg Phe Leu Trp Gln Pro Lys Arg Glu Cys His Phe Phe Asn
           35                  40                  45
   Gly Thr Glu Arg Val Arg Phe Leu Asp Arg Tyr Phe Tyr Asn Gln Glu
       50                  55                  60
   Glu Ser Val Arg Phe Asp Ser Asp Val Gly Glu Phe Arg Ala Val Thr
   65                  70                  75                  80
   Glu Leu Gly Arg Pro Asp Ala Glu Tyr Trp Asn Ser Gln Lys Asp Ile
                   85                  90                  95
   Leu Glu Gln Ala Arg Ala Ala Val Asp Thr Tyr Cys Arg His Asn Tyr
               100                 105                 110
   Gly Val Val Glu Ser Phe Thr Val Gln Arg Arg Val Gln Pro Lys Val
           115                 120                 125
   Thr Val Tyr Pro Ser Lys Thr Gln Pro Leu Gln His His Asn Leu Leu
       130                 135                 140
   Val Cys Ser Val Ser Gly Phe Tyr Pro Gly Ser Ile Glu Val Arg Trp
   145                 150                 155                 160
   Phe Leu Asn Gly Gln Glu Glu Lys Ala Gly Met Val Ser Thr Gly Leu
                   165                 170                 175
   Ile Gln Asn Gly Asp Trp Thr Phe Gln Thr Leu Val Met Leu Glu Thr
               180                 185                 190
   Val Pro Arg Ser Gly Glu Val Tyr Thr Cys Gln Val Glu His Pro Ser
           195                 200                 205
   Val Thr Ser Pro Leu Thr Val Glu Trp Arg Ala Arg Ser Glu Ser Ala
       210                 215                 220
   Gln Ser Lys Met Leu Ser Gly Val Gly Gly Phe Val Leu Gly Leu Leu
   225                 230                 235                 240
   Phe Leu Gly Ala Gly Leu Phe Ile Tyr Phe Arg Asn Gln Lys Gly His
                   245                 250                 255
   Ser Gly Leu Gln Pro Thr Gly Phe Leu Ser
               260                 265
<210> 557
   <211> 88
   <212> PRT
   <213> Homo sapiens
<400> 557
   Arg Phe Leu Trp Gln Pro Lys Arg Glu Cys His Phe Phe Asn Gly Thr
   1 5 10 15
   Glu Arg Val Arg Phe Leu Asp Arg Tyr Phe Tyr Asn Gln Glu Glu Ser
   20 25 30
   Val Arg Phe Asp Ser Asp Val Gly Glu Phe Arg Ala Val Thr Glu Leu
   35 40 45
   Gly Arg Pro Asp Ala Glu Tyr Trp Asn Ser Gln Lys Asp Ile Leu Glu
   50 55 60
   Gln Ala Arg Ala Ala Val Asp Thr Tyr Cys Arg His Asn Tyr Gly Val
   65 70 75 80
   Val Glu Ser Phe Thr Val Gln Arg
   85
<210> 558
   <211> 88
   <212> PRT
   <213> Homo sapiens
<400> 558
   Arg Phe Leu Trp Gln Pro Lys Arg Glu Cys His Phe Phe Asn Gly Thr
   1 5 10 15
   Glu Arg Val Arg Phe Leu Asp Arg Tyr Phe Tyr Asn Gln Glu Glu Ser
   20 25 30
   Val Arg Phe Asp Ser Asp Val Gly Glu Phe Arg Ala Val Thr Glu Leu
   35 40 45
   Gly Arg Pro Asp Ala Glu Tyr Trp Asn Ser Gln Lys Asp Ile Leu Glu
   50 55 60
   Gln Ala Arg Ala Ala Val Asp Thr Tyr Cys Arg His Asn Tyr Gly Val
   65 70 75 80
   Val Glu Ser Phe Thr Val Gln Arg
   85
<210> 559
   <211> 237
   <212> PRT
   <213> Homo sapiens
<400> 559
   Gly Asp Thr Arg Pro Arg Phe Leu Trp Gln Pro Lys Arg Glu Cys His
   1               5                   10                  15
   Phe Phe Asn Gly Thr Glu Arg Val Arg Phe Leu Asp Arg Tyr Phe Tyr
               20                  25                  30
   Asn Gln Glu Glu Ser Val Arg Phe Asp Ser Asp Val Gly Glu Phe Arg
           35                  40                  45
   Ala Val Thr Glu Leu Gly Arg Pro Asp Ala Glu Tyr Trp Asn Ser Gln
       50                  55                  60
   Lys Asp Ile Leu Glu Gln Ala Arg Ala Ala Val Asp Thr Tyr Cys Arg
   65                  70                  75                  80
   His Asn Tyr Gly Val Gly Glu Ser Phe Thr Val Gln Arg Arg Val Gln
                   85                  90                  95
   Pro Lys Val Thr Val Tyr Pro Ser Lys Thr Gln Pro Leu Gln His His
               100                 105                 110
   Asn Leu Leu Val Cys Ser Val Ser Gly Phe Tyr Pro Gly Ser Ile Glu
           115                 120                 125
   Val Arg Trp Phe Leu Asn Gly Gln Glu Glu Lys Ala Gly Met Val Ser
       130                 135                 140
   Thr Gly Leu Ile Gln Asn Gly Asp Trp Thr Phe Gln Thr Leu Val Met
   145                 150                 155                 160
   Leu Glu Thr Val Pro Arg Ser Gly Glu Val Tyr Thr Cys Gln Val Glu
                   165                 170                 175
   His Pro Ser Val Thr Ser Pro Leu Thr Val Glu Trp Arg Ala Arg Ser
               180                 185                 190
   Glu Ser Ala Gln Ser Lys Met Leu Ser Gly Val Gly Gly Phe Val Leu
           195                 200                 205
   Gly Leu Leu Phe Leu Gly Ala Gly Leu Phe Ile Tyr Phe Arg Asn Gln
       210                 215                 220
   Lys Gly His Ser Gly Leu Gln Pro Thr Gly Phe Leu Ser
   225                 230                 235
<210> 560
   <211> 73
   <212> PRT
   <213> Homo sapiens
<400> 560
   Glu Cys His Phe Phe Asn Gly Thr Glu Arg Val Arg Phe Leu Asp Arg
   1               5                   10                  15
   Tyr Phe Tyr Asn Gln Glu Glu Ser Val Arg Phe Asp Ser Asp Val Gly
               20                  25                  30
   Glu Phe Arg Ala Val Thr Glu Leu Gly Arg Pro Asp Ala Glu Tyr Trp
           35                  40                  45
   Asn Ser Gln Lys Asp Ile Leu Glu Gln Ala Arg Ala Ala Val Asp Thr
       50                  55                  60
   Tyr Cys Arg His Asn Tyr Gly Val Gly
   65                  70
<210> 561
   <211> 81
   <212> PRT
   <213> Homo sapiens
<400> 561
   Arg Phe Leu Trp Gln Pro Lys Arg Glu Cys His Phe Phe Asn Gly Thr
   1               5                   10                  15
   Glu Arg Val Arg Phe Leu Asp Arg Tyr Phe Tyr Asn Gln Glu Glu Ser
               20                  25                  30
   Val Arg Phe Asp Ser Asp Val Gly Glu Phe Arg Ala Val Thr Glu Leu
           35                  40                  45
   Gly Arg Pro Asp Ala Glu Tyr Trp Asn Ser Gln Lys Asp Ile Leu Glu
       50                  55                  60
   Gln Ala Arg Ala Ala Val Asp Thr Tyr Cys Arg His Asn Tyr Gly Val
   65                  70                  75                  80
   Gly
<210> 562
   <211> 98
   <212> PRT
   <213> Homo sapiens
<400> 562
   Gly Asp Thr Arg Pro Arg Phe Leu Trp Gln Pro Lys Arg Glu Cys His
   1               5                   10                  15
   Phe Phe Asn Gly Thr Glu Arg Val Arg Phe Leu Asp Arg His Phe Tyr
               20                  25                  30
   Asn Gln Glu Glu Ser Val Arg Phe Asp Ser Asp Val Gly Glu Phe Arg
           35                  40                  45
   Ala Val Thr Glu Leu Gly Arg Pro Asp Ala Glu Tyr Trp Asn Ser Gln
       50                  55                  60
   Lys Asp Ile Leu Glu Gln Ala Arg Ala Ala Val Asp Thr Tyr Cys Arg
   65                  70                  75                  80
   His Asn Tyr Gly Val Val Glu Ser Phe Thr Val Gln Arg Arg Val Gln
                   85                  90                  95
   Pro Lys
<210> 563
   <211> 85
   <212> PRT
   <213> Homo sapiens
<400> 563
   Phe Leu Trp Gln Pro Lys Arg Glu Cys His Phe Phe Asn Gly Thr Glu
   1               5                   10                  15
   Arg Val Arg Phe Leu Asp Arg Tyr Phe Tyr Asn Gln Glu Glu Ser Val
               20                  25                  30
   Arg Phe Asp Ser Asp Val Gly Glu Phe Arg Ala Val Thr Glu Leu Gly
           35                  40                  45
   Arg Pro Asp Ala Glu Tyr Trp Asn Ser Gln Lys Asp Phe Leu Glu Gln
       50                  55                  60
   Ala Arg Ala Ala Val Asp Thr Tyr Cys Arg His Asn Tyr Gly Val Val
   65                  70                  75                  80
   Glu Ser Phe Thr Val
                   85
<210> 564
   <211> 87
   <212> PRT
   <213> Homo sapiens
<400> 564
   Phe Leu Trp Gln Pro Lys Arg Glu Cys His Phe Phe Asn Gly Thr Glu
   1 5 10 15
   Arg Val Arg Phe Leu Asp Arg Tyr Phe Tyr Asn Gln Glu Glu Ser Val
   20 25 30
   Arg Phe Asp Ser Asp Val Gly Glu Phe Arg Ala Val Thr Glu Leu Gly
   35 40 45
   Arg Pro Asp Ala Glu Tyr Trp Asn Ser Gln Lys Asp Leu Leu Glu Gln
   50 55 60
   Ala Arg Ala Ala Val Asp Thr Tyr Cys Arg His Asn Tyr Gly Val Val
   65 70 75 80
   Glu Ser Phe Thr Val Gln Arg
   85
<210> 565
   <211> 79
   <212> PRT
   <213> Homo sapiens
<400> 565
   Trp Gln Pro Lys Arg Glu Cys His Phe Phe Asn Gly Thr Glu Arg Val
   1 5 10 15
   Arg Phe Leu Asp Arg Tyr Phe Tyr Asn Gln Glu Glu Ser Val Arg Phe
   20 25 30
   Asp Ser Asp Val Gly Glu Phe Arg Ala Ala Thr Glu Leu Gly Arg Pro
   35 40 45
   Asp Ala Glu Tyr Trp Asn Ser Gln Lys Asp Ile Leu Glu Gln Ala Arg
   50 55 60
   Ala Ala Val Asp Thr Tyr Cys Arg His Asn Tyr Gly Val Val Glu
   65 70 75
<210> 566
   <211> 82
   <212> PRT
   <213> Homo sapiens
<400> 566
   Phe Leu Trp Gln Pro Lys Arg Glu Cys His Phe Phe Asn Gly Thr Glu
   1               5                   10                  15
   Arg Val Arg Phe Leu Asp Arg Tyr Phe Tyr Asn Gln Glu Glu Ser Val
               20                  25                  30
   Arg Phe Asp Ser Asp Val Gly Glu Tyr Arg Ala Val Thr Glu Leu Gly
           35                  40                  45
   Arg Pro Asp Ala Glu Tyr Trp Asn Ser Gln Lys Asp Ile Leu Glu Gln
       50                  55                  60
   Ala Arg Ala Ala Val Asp Thr Tyr Cys Arg His Asn Tyr Gly Val Val
   65                  70                  75                  80
   Glu Ser
<210> 567
   <211> 89
   <212> PRT
   <213> Homo sapiens
<400> 567
   Arg Phe Leu Trp Gln Pro Lys Arg Glu Cys His Phe Phe Asn Gly Thr
   1               5                   10                  15
   Glu Arg Val Arg Phe Leu Asp Arg Tyr Phe Tyr Asn Gln Glu Glu Ser
               20                  25                  30
   Val Arg Phe Asp Ser Asp Val Gly Glu Phe Arg Ala Val Thr Glu Leu
           35                  40                  45
   Gly Arg Pro Asp Ala Glu Tyr Trp Asn Ser Gln Lys Asn Ile Leu Glu
       50                  55                  60
   Gln Ala Arg Ala Ala Val Asp Thr Tyr Cys Arg His Asn Tyr Gly Val
   65                  70                  75                  80
   Gly Glu Ser Phe Thr Val Gln Arg Arg
                   85
<210> 568
   <211> 89
   <212> PRT
   <213> Homo sapiens
<400> 568
   Arg Phe Leu Trp Gln Pro Lys Arg Glu Cys His Phe Phe Asn Gly Thr
   1 5 10 15
   Glu Arg Val Arg Phe Leu Asp Arg Tyr Phe Tyr Asn Gln Glu Glu Ser
   20 25 30
   Val Arg Phe Asp Ser Asp Val Gly Glu Phe Gln Ala Val Thr Glu Leu
   35 40 45
   Gly Arg Pro Asp Ala Glu Tyr Trp Asn Ser Gln Lys Asp Ile Leu Glu
   50 55 60
   Gln Ala Arg Ala Ala Val Asp Thr Tyr Cys Arg His Asn Tyr Gly Val
   65 70 75 80
   Val Glu Ser Phe Thr Val Gln Arg Arg
   85
<210> 569
   <211> 82
   <212> PRT
   <213> Homo sapiens
<400> 569
   Phe Leu Trp Gln Pro Lys Arg Glu Cys His Phe Phe Asn Gly Thr Glu
   1 5 10 15
   Arg Val Arg Phe Leu Asp Arg Tyr Phe Tyr Asn Gln Glu Glu Ser Val
   20 25 30
   Arg Phe Asp Ser Asp Val Gly Glu Phe Arg Ala Val Thr Glu Leu Gly
   35 40 45
   Arg Pro Asp Ala Glu Tyr Trp Asn Ser Gln Lys Asp Ile Leu Glu Asp
   50 55 60
   Glu Arg Ala Ala Val Asp Thr Tyr Cys Arg His Asn Tyr Gly Val Val
   65 70 75 80
   Glu Ser

<210> 570
   <211> 89
   <212> PRT
   <213> Homo sapiens
<400> 570
   Arg Phe Leu Trp Gln Pro Lys Arg Glu Cys His Phe Phe Asn Gly Thr
   1               5                   10                  15
   Glu Arg Val Arg Phe Leu Asp Arg Tyr Phe Tyr Asn Gln Glu Glu Ser
               20                  25                  30
   Val Arg Phe Asp Ser Asp Val Gly Glu Tyr Arg Ala Val Thr Glu Leu
           35                  40                  45
   Gly Arg Pro Asp Ala Glu Tyr Trp Asn Ser Gln Lys Asp Ile Leu Glu
       50                  55                  60
   Gln Ala Arg Ala Ala Val Asp Thr Tyr Cys Arg His Asn Tyr Gly Val
   65                  70                  75                  80
   Gly Glu Ser Phe Thr Val Gln Arg Arg
                   85
<210> 571
   <211> 266
   <212> PRT
   <213> Homo sapiens
<400> 571
<210> 572
   <211> 85
   <212> PRT
   <213> Homo sapiens
<400> 572
   Arg Phe Leu Trp Gln Pro Lys Arg Glu Cys His Phe Phe Asn Gly Thr
   1               5                   10                  15
   Glu Arg Val Arg Phe Leu Asp Arg Tyr Phe Tyr Asn Gln Glu Glu Ser
               20                  25                  30
   Val Arg Phe Asp Ser Asp Val Gly Glu Tyr Arg Ala Val Thr Glu Leu
           35                  40                  45
   Gly Arg Pro Asp Ala Glu Tyr Trp Asn Ser Gln Lys Asp Phe Leu Glu
       50                  55                  60
   Asp Arg Arg Ala Ala Val Asp Thr Tyr Cys Arg His Asn Tyr Gly Val
   65                  70                  75                  80
   Gly Glu Ser Phe Thr
                   85
<210> 573
   <211> 266
   <212> PRT
   <213> Homo sapiens
<400> 573
<210> 574
   <211> 80
   <212> PRT
   <213> Homo sapiens
<400> 574
   Phe Leu Trp Gln Pro Lys Arg Glu Cys His Phe Phe Asn Gly Thr Glu
   1 5 10 15
   Arg Val Arg Phe Leu Asp Arg Tyr Phe Tyr Asn Gln Glu Glu Ser Val
   20 25 30
   Arg Phe Asp Ser Asp Val Gly Glu Tyr Arg Ala Val Thr Glu Leu Gly
   35 40 45
   Arg Pro Asp Ala Glu Tyr Trp Asn Ser Gln Lys Asp Leu Leu Glu Asp
   50 55 60
   Arg Arg Ala Ala Val Asp Thr Tyr Cys Arg His Asn Tyr Gly Val Gly
   65 70 75 80
<210> 575
   <211> 262
   <212> PRT
   <213> Homo sapiens
<400> 575
   Met Val Cys Leu Lys Leu Pro Gly Gly Ser Cys Met Thr Ala Leu Thr
   1 5 10 15
   Val Thr Leu Met Val Leu Ser Ser Pro Leu Ala Leu Ala Gly Asp Thr
   20 25 30
   Arg Pro Arg Phe Leu Trp Gln Pro Lys Arg Glu Cys His Phe Phe Asn
   35 40 45
   Gly Thr Glu Arg Val Arg Phe Leu Asp Arg Tyr Phe Tyr Asn Gln Glu
   50 55 60
   Glu Ser Val Arg Phe Asp Ser Asp Val Gly Glu Tyr Arg Ala Val Thr
   65 70 75 80
   Glu Leu Gly Arg Pro Asp Ala Glu Tyr Trp Asn Ser Gln Lys Asp Phe
   85 90 95
   Leu Glu Asp Arg Ala Ala Ala Val Asp Thr Tyr Cys Arg His Asn Tyr
   100 105 110
   Gly Val Gly Glu Ser Phe Thr Val Gln Arg Arg Val Gln Pro Lys Val
   115 120 125
   Thr Val Tyr Pro Ser Lys Thr Gln Pro Leu Gln His His Asn Leu Leu
   130 135 140
   Val Cys Ser Val Ser Gly Phe Tyr Pro Gly Ser Ile Glu Val Arg Trp
   145 150 155 160
   Phe Leu Asn Gly Gln Glu Glu Lys Ala Gly Met Val Ser Thr Gly Leu
   165 170 175
   Ile Gln Asn Gly Asp Trp Thr Phe Gln Thr Leu Val Met Leu Glu Thr
   180 185 190
   Val Pro Arg Ser Gly Glu Val Tyr Thr Cys Gln Val Glu His Pro Ser
   195 200 205
   Val Thr Ser Pro Leu Thr Val Glu Trp Arg Ala Arg Ser Glu Ser Ala
   210 215 220
   Gln Ser Lys Met Leu Ser Gly Val Gly Gly Phe Val Leu Gly Leu Leu
   225 230 235 240
   Phe Leu Gly Ala Gly Leu Phe Ile Tyr Phe Arg Asn Gln Lys Gly His
   245 250 255
   Ser Gly Leu Gln Pro Thr
   260
<210> 576
   <211> 78
   <212> PRT
   <213> Homo sapiens
<400> 576
   Trp Gln Pro Lys Arg Glu Cys His Phe Phe Asn Gly Thr Glu Arg Val
   1               5                   10                  15
   Arg Phe Leu Asp Arg Tyr Phe Tyr Asn Gln Glu Glu Ser Val Arg Phe
               20                  25                  30
   Asp Ser Asp Val Gly Glu Tyr Arg Ala Val Thr Glu Leu Gly Arg Pro
           35                  40                  45
   Asp Ala Glu Tyr Trp Asn Ser Gln Lys Asp Phe Leu Glu Asp Arg Arg
       50                  55                  60
   Ala Leu Val Asp Thr Tyr Cys Arg His Asn Tyr Gly Val Gly
   65                  70                  75
<210> 577
   <211> 80
   <212> PRT
   <213> Homo sapiens
<400> 577
   Leu Trp Gln Pro Lys Arg Glu Cys His Phe Phe Asn Gly Thr Glu Arg
   1               5                   10                  15
   Val Arg Phe Leu Asp Arg Tyr Phe Tyr Asn Gln Glu Glu Ser Val Arg
               20                  25                  30
   Phe Asp Ser Asp Val Gly Glu Tyr Arg Ala Val Thr Glu Leu Gly Arg
           35                  40                  45
   Pro Asp Ala Glu Tyr Trp Asn Ser Gln Lys Asp Ile Leu Glu Asp Arg
       50                  55                  60
   Arg Ala Ala Val Asp Thr Tyr Cys Arg His Asn Tyr Gly Val Gly Glu
   65                  70                  75                  80
<210> 578
   <211> 86
   <212> PRT
   <213> Homo sapiens
<400> 578
   Arg Phe Leu Trp Gln Pro Lys Arg Glu Cys His Phe Phe Asn Gly Thr
   1               5                   10                  15
   Glu Arg Val Arg Phe Pro Asp Arg Tyr Phe Tyr Asn Gln Glu Glu Ser
               20                  25                  30
   Val Arg Phe Asp Ser Asp Val Gly Glu Tyr Arg Ala Val Thr Glu Leu
           35                  40                  45
   Gly Arg Pro Asp Ala Glu Tyr Trp Asn Ser Gln Lys Asp Ile Leu Glu
       50                  55                  60
   Asp Arg Arg Ala Ala Val Asp Thr Tyr Cys Arg His Asn Tyr Gly Val
   65                  70                  75                  80
   Gly Glu Ser Phe Thr Val
               85
<210> 579
   <211> 89
   <212> PRT
   <213> Homo sapiens
<400> 579
   Arg Phe Leu Trp Gln Pro Lys Arg Glu Cys His Phe Phe Asn Gly Thr
   1               5                   10                  15
   Glu Arg Val Arg Phe Leu Asp Arg Tyr Phe Tyr Asn Gln Glu Glu Asn
               20                  25                  30
   Val Arg Phe Asp Ser Asp Val Gly Glu Tyr Arg Ala Val Thr Glu Leu
           35                  40                  45
   Gly Arg Pro Asp Ala Glu Tyr Trp Asn Ser Gln Lys Asp Phe Leu Glu
       50                  55                  60
   Asp Arg Arg Ala Ala Val Asp Thr Tyr Cys Arg His Asn Tyr Gly Val
   65                  70                  75                  80
   Gly Glu Ser Phe Thr Val Gln Arg Arg
                   85
<210> 580
   <211> 801
   <212> DNA
   <213> Homo sapiens
<400> 580
   atggtgtgtc tgaagctccc tggaggctcc tgcatgacag cgctgacagt gacactgatg     60
   gtgctgagct ccccactggc tttggctggg gacacccgac cacgtttctt gtggcagctt    120
   aagtttgaat gtcatttctt caatgggacg gagcgggtgc ggttgctgga aagatgcatc    180
   tataaccaag aggagtccgt gcgcttcgac agcgacgtgg gggagtaccg ggcggtgacg    240
   gagctggggc ggcctgatgc cgagtactgg aacagccaga aggacctcct ggagcagagg    300
   cgggccgcgg tggacaccta ctgcagacac aactacgggg ttggtgagag cttcacagtg    360
   cagcggcgag ttgagcctaa ggtgactgtg tatccttcaa agacccagcc cctgcagcac    420
   cacaacctcc tggtctgctc tgtgagtggt ttctatccag gcagcattga agtcaggtgg    480
   ttccggaacg gccaggaaga gaaggctggg gtggtgtcca caggcctgat ccagaatgga    540
   gattggacct tccagaccct ggtgatgctg gaaacagttc ctcggagtgg agaggtttac    600
   acctgccaag tggagcaccc aagtgtgacg agccctctca cagtggaatg gagagcacgg    660
   tctgaatctg cacagagcaa gatgctgagt ggagtcgggg gcttcgtgct gggcctgctc    720
   ttccttgggg ccgggctgtt catctacttc aggaatcaga aaggacactc tggacttcag    780
   ccaacaggat tcctgagctg a                                              801
<210> 581
   <211> 714
   <212> DNA
   <213> Homo sapiens
<400> 581
<210> 582
   <211> 246
   <212> DNA
   <213> Homo sapiens
<400> 582
   cacgtttctt gtggcagctt aagtttgaat gtcatttctt caatgggacg gagcgggtgc 60
   ggttgctgga aagatgcatc tataaccaag aggagtccgt gcgcttcgac agcgacgtgg 120
   gggagtaccg ggcggtgacg gagctggggc ggcctgatgc cgagtactgg aacagccaga 180
   aggacctcct ggagcagagg cgggccgccg tggacaccta ttgcagacac aactacgggg 240
   ctgtgg 246
<210> 583
   <211> 801
   <212> DNA
   <213> Homo sapiens
<400> 583
   atggtgtgtc tgaagctccc tggaggctcc tgcatgacag cgctgacagt gacactgatg 60
   gtgctgagct ccccactggc tttggctggg gacacccgac cacgtttctt gtggcagctt 120
   aagtttgaat gtcatttctt caatgggacg gagcgggtgc ggttgctgga aagatgcatc 180
   tataaccaag aggagtccgt gcgcttcgac agcgacgtgg gggagtaccg ggcggtgacg 240
   gagctggggc ggcctgatgc cgagtactgg aacagccaga aggacatcct ggaagacgag 300
   cgggccgcgg tggacaccta ctgcagacac aactacgggg ttggtgagag cttcacagtg 360
   cagcggcgag ttgagcctaa ggtgactgtg tatccttcaa agacccagcc cctgcagcac 420
   cacaacctcc tggtctgctc tgtgagtggt ttctatccag gcagcattga agtcaggtgg 480
   ttccggaacg gccaggaaga gaaggctggg gtggtgtcca caggcctgat ccagaatgga 540
   gattggacct tccagaccct ggtgatgctg gaaacagttc ctcggagtgg agaggtttac 600
   acctgccaag tggagcaccc aagtgtgacg agccctctca cagtggaatg gagagcacgg 660
   tctgaatctg cacagagcaa gatgctgagt ggagtcgggg gcttcgtgct gggcctgctc 720
   ttccttgggg ccgggctgtt catctacttc aggaatcaga aaggacactc tggacttcag 780
   ccaacaggat tcctgagctg a 801
<210> 584
   <211> 677
   <212> DNA
   <213> Homo sapiens
<400> 584
   ggggacaccc gaccacgttt cttgtggcag cttaagtttg aatgtcattt cttcaatggg 60
   acggagcggg tgcggttgct ggaaagatgc atctataacc aagaggagtc cgtgcgcttc 120
   gacagcgacg tgggggagta ccgggcggtg acggagctgg ggcggcctga tgccgagtac 180
   tggaacagcc agaaggacct cctggagcag aggcgggccg cggtggacaa ttactgcaga 240
   cacaactacg gggttgtgga gagcttcaca gtgcagcggc gagttgagcc taaggtgact 300
   gtgtatcctt caaagaccca gcccctgcag caccacaacc tcctggtctg ctctgtgagt 360
   ggtttctatc caggcagcat tgaagtcagg tggttccgga acggccagga agagaaggct 420
   ggggtggtgt ccacaggcct gatccagaat ggagattgga ccttccagac cctggtgatg 480
   ctggaaacag ttcctcggag tggagaggtt tacacctgcc aagtggagca cccaagtgtg 540
   acgagccctc tcacagtgga atggagagca cggtctgaat ctgcacagag caagatgctg 600
   agtggagtcg ggggcttcgt gctgggcctg ctcttccttg gggccgggct gttcatctac 660
   ttcaggaatc agaaagg 677
<210> 585
   <211> 270
   <212> DNA
   <213> Homo sapiens
<400> 585
   cacgtttctt gtggcagctt aagtttgaat gtcatttctt caatgggacg gagcgggtgc 60
   ggttgctgga aagatgcatc tataaccaag aggagtccgt gcgcttcgac agcgacgtga 120
   gggagtaccg ggcggtgacg gagctggggc ggcctgatgc cgagtactgg aacagccaga 180
   aggacctcct ggagcagagg cgggccgcgg tggacaccta ctgcagacac aactacgggg 240
   ttggtgagag cttcacagtg cagcggcgag 270
<210> 586
   <211> 270
   <212> DNA
   <213> Homo sapiens
<400> 586
   cacgtttctt gtggcagctt aagtttgaat gtcatttctt caatgggacg gagcgggtgc 60
   ggttgctgga aagatgcatc tataaccaag aggagtccgt gcgcttcgac agcgacgtgg 120
   gggagtaccg ggcggtgacg gagctggggc ggcctgatgc cgagtactgg aacagccaga 180
   aggacctcct ggagcaggcg cgggccgcgg tggacaccta ctgcagacac aactacgggg 240
   ttgtggagag cttcacagtg cagcggcgag 270

<210> 587
   <211> 270
   <212> DNA
   <213> Homo sapiens
<400> 587
   cacgtttctt gtgggagctt aagtttgaat gtcatttctt caatgggacg gagcgggtgc 60
   ggttgctgga aagatgcatc tataaccaag aggagtccgt gcgcttcgac agcgacgtgg 120
   gggagtaccg ggcggtgacg gagctggggc ggcctgatgc cgagtactgg aacagccaga 180
   aggacctcct ggagcagagg cgggccgcgg tggacaccta ctgcagacac aactacgggg 240
   ttggtgagag cttcacagtg cagcggcgag 270
<210> 588
   <211> 270
   <212> DNA
   <213> Homo sapiens
<400> 588
   cacgtttctt gtggcagctt aagtttgaat gtcatttctt caatgggacg gagcgggtgc 60
   ggttgctgga aagatgcatc tataaccaag aggagtacgt gcgcttcgac agcgacgtgg 120
   gggagtaccg ggcggtgacg gagctggggc ggcctgatgc cgagtactgg aacagccaga 180
   aggacctcct ggagcagagg cgggccgcgg tggacaccta ctgcagacac aactacgggg 240
   ttggtgagag cttcacagtg cagcggcgag 270
<210> 589
   <211> 714
   <212> DNA
   <213> Homo sapiens
<400> 589
   ggggacacca gaccacgttt cttggagtac tctacgtctg agtgtcattt cttcaatggg 60
   acggagcggg tgcggtacct ggacagatac ttccataacc aggaggagaa cgtgcgcttc 120
   gacagcgacg tgggggagtt ccgggcggtg acggagctgg ggcggcctga tgccgagtac 180
   tggaacagcc agaaggacct cctggagcag aagcggggcc gggtggacaa ctactgcaga 240
   cacaactacg gggttgtgga gagcttcaca gtgcagcggc gagtccatcc taaggtgact 300
   gtgtatcctt caaagaccca gcccctgcag caccataacc tcctggtctg ttctgtgagt 360
   ggtttctatc caggcagcat tgaagtcagg tggttccgga atggccagga agagaagact 420
   ggggtggtgt ccacaggcct gatccacaat ggagactgga ccttccagac cctggtgatg 480
   ctggaaacag ttcctcggag tggagaggtt tacacctgcc aagtggagca cccaagcgtg 540
   acaagccctc tcacagtgga atggagagca cggtctgaat ctgcacagag caagatgctg 600
   agtggagtcg ggggctttgt gctgggcctg ctcttccttg gggccgggct gttcatctac 660
   ttcaggaatc agaaaggaca ctctggactt cagccaagag gattcctgag ctga 714
<210> 590
   <211> 265
   <212> DNA
   <213> Homo sapiens
<400> 590
   cacgtttctt ggagtactct acgtctgagt gtcatttctt caatgggacg gagcgggtgc 60
   ggtacctgga cagatacttc cataaccagg aggagaacgt gcgcttcgac agcgacgtgg 120
   gggagttccg ggcggtgacg gagctggggc ggcctgatgc cgagtactgg aacagccaga 180
   aggacctcct ggagcagaag cggggccggg tggacaatta ctgcagacac aactacgggg 240
   ttgtggagag cttcacagtg cagcg 265
<210> 591
   <211> 714
   <212> DNA
   <213> Homo sapiens
<400> 591
   ggggacacca gaccacgttt cttggagtac tctacgtctg agtgtcattt cttcaatggg 60
   acggagcggg tgcggttcct ggagagatac ttccataacc aggaggagaa cgtgcgcttc 120
   gacagcgacg tgggggagta ccgggcggtg acggagctgg ggcggcctga tgccgagtac 180
   tggaacagcc agaaggacct cctggagcag aagcggggcc gggtggacaa ctactgcaga 240
   cacaactacg gggttggtga gagcttcaca gtgcagcggc gagtccatcc taaggtgact 300
   gtgtatcctt caaagaccca gcccctgcag caccacaacc tcctggtctg ttctgtgagt 360
   ggtttctatc caggcagcat tgaagtcagg tggttccgga atggccagga agagaagact 420
   ggggtggtgt ccacaggcct gatccacaat ggagactgga ccttccagac cctggtgatg 480
   ctggaaacag ttcctcggag tggagaggtt tacacctgcc aagtggagca cccaagcgtg 540
   acaagccctc tcacagtgga atggagagca cggtctgaat ctgcacagag caagatgctg 600
   agtggagtcg ggggctttgt gctgggcctg ctcttccttg gggccgggct gttcatctac 660
   ttcaggaatc agaaaggaca ctctggactt cagccaagag gattcctgag ctga 714
<210> 592
   <211> 300
   <212> DNA
   <213> Homo sapiens
<400> 592
   ggggacacca gaccacgttt cttggagtac tctacgtctg agtgtcattt cttcaatggg 60
   acggagcggg tgcggttcct ggagagatac ttccataacc aggaggagaa cgtgcgcttc 120
   gacagcgacg tgggggagta ccgggcggtg acggagctgg ggcggcctga tgccgagtac 180
   tggaacagcc agaaggacct cctggagcag aagcggggcc gggtggacaa ttactgcaga 240
   cacaactacg gggttggtga gagcttcaca gtgcagcggc gagtccatcc taaggtgact 300
<210> 593
   <211> 255
   <212> DNA
   <213> Homo sapiens
<400> 593
   tactctacgt ctgagtgtca tttcttcaat gggacggagc gggtgcggtt cctggagaga 60
   tacttccata accaggagga gaacgtgcgc ttcgacagcg acgtggggga gtaccgggcg 120
   gtgacggagc tggggcggcc tgatgccgag tactggaaca gccagaagga cctcctggag 180
   cagaagcggg gccgggtgga caactactgc agacacaact acggggttgt ggagagcttc 240
   acagtgcagc ggcga 255
<210> 594
   <211> 270
   <212> DNA
   <213> Homo sapiens
<400> 594
   cacgtttctt ggagtactct acgtctgagt gtcatttctt caatgggacg gagcgggtgc 60
   ggtacctgga cagatacttc cataaccagg aggagtccgt gcgcttcgac agcgacgtgg 120
   gggagttccg ggcggtgacg gagctggggc ggcctgatgc cgagtactgg aacagccaga 180
   aggacctcct ggagcagaag cggggccggg tggacaacta ctgcagacac aactacgggg 240
   ttgtggagag cttcacagtg cagcggcgag 270
<210> 595
   <211> 270
   <212> DNA
   <213> Homo sapiens
<400> 595
   cacgtttctt ggagtactct acgtctgagt gtcatttctt caatgggacg gagcgggtgc 60
   ggtacctgga cagatacttc cataaccagg aggagaacgt gcgcttcgac agcgacgtgg 120
   gggagttccg ggcggtgacg gagctggggc ggcctgatgc cgagtactgg aacagccaga 180
   aggacctcct ggagcagaag cggggccggg tggacaacta ctgcagacac aactacgggg 240
   ttggtgagag cttcacagtg cagcggcgag 270
<210> 596
   <211> 270
   <212> DNA
   <213> Homo sapiens
<400> 596
   cacgtttctt ggagtactct acgtctgagt gtcatttctt caatgggacg gagcgggtgc 60
   ggtacctgga cagatacttc cataaccagg aggagaacgt gcgcttcgac agcgacgtgg 120
   gggagttccg ggcggtgacg gagctggggc ggcctgatgc cgagtactgg aacagccaga 180
   aggacctcct ggagcagaag cggggccggg tggacaacta ctgcagacac aactacgggg 240
   ttggtgagag cttcacggtg cagcggcgag 270
<210> 597
   <211> 258
   <212> DNA
   <213> Homo sapiens
<400> 597
   ttcttggagt actctacgtc tgagtgtcat ttcttcaatg ggacggagcg ggtgcggtac 60
   ctggacagat acttccataa ccaggaggag aacgtgcgct tcgacagcga cgtgggggag 120
   taccgggcgg tgacggagct ggggcggcct gatgccgagt actggaacag ccagaaggac 180
   ctcctggagc agaagcgggg ccgggtggac aactactgca gacacaacta cggggttgtg 240
   gagagcttca cagtgcag 258
<210> 598
   <211> 306
   <212> DNA
   <213> Homo sapiens
<400> 598
   ggggacacca gaccacgttt cttggagtac tctacgtctg agtgtcattt cttcaatggg 60
   acggagcggg tgcggttcct ggacagatac ttccataacc aggaggagaa cgtgcgcttc 120
   gacagcgacg tgggggagtt ccgggcggtg acggagctgg ggcggcctga tgccgagtac 180
   tggaacagcc agaaggacct cctggagcag aagcggggcc gggtggacaa ctactgcaga 240
   cacaactacg gggttgtgga gagcttcaca gtgcagcggc gagtccatcc taaggtgact 300
   gtgtat 306
<210> 599
   <211> 306
   <212> DNA
   <213> Homo sapiens
<400> 599
   ggggacacca gaccacgttt cttggagtac tctacgtctg agtgtcattt cttcaatggg 60
   acggagcggg tgcggtacct ggacagatac ttccataacc aggaggagaa cgtgcgcttc 120
   gacagcgacg tgggggagtt ccgggcggtg acggagctgg ggcggcctga tgaggagtac 180
   tggaacagcc agaaggacct cctggagcag aagcggggcc gggtggacaa ctactgcaga 240
   cacaactacg gggttgtgga gagcttcaca gtgcagcggc gagtccatcc taaggtgact 300
   gtgtat 306
<210> 600
   <211> 258
   <212> DNA
   <213> Homo sapiens
<400> 600
   ttggagtact ctacgtctga gtgtcatttc ttcaatggga cggagcgggt gcggtacctg 60
   gacagatact tccataaccg ggaggagaac gtgcgcttcg acagcgacgt gggggagttc 120
   cgggcggtga cggagctggg gcggcctgat gccgagtact ggaacagcca gaaggacctc 180
   ctggagcaga agcggggccg ggtggacaac tactgcagac acaactacgg ggttggtgag 240
   agcttcacag tgcagcgg 258
<210> 601
   <211> 714
   <212> DNA
   <213> Homo sapiens
<400> 601
   ggggacacca gaccacgttt cttggagtac tctacgtctg agtgtcattt cttcaatggg 60
   acggagcggg tgcggtacct ggacagatac ttccataacc aggaggagaa cgtgcgcttc 120
   gacagcgacg tgggggagtt ccgggcggtg acggagctgg ggcggcctgc tgcggagcac 180
   tggaacagcc agaaggacct cctggagcag aagcggggcc gggtggacaa ctactgcaga 240
   cacaactacg gggttgtgga gagcttcaca gtgcagcggc gagtccatcc taaggtgact 300
   gtgtatcctt caaagaccca gcccctgcag caccataacc tcctggtctg ttctgtgagt 360
   ggtttctatc caggcagcat tgaagtcagg tggttccgga atggccagga agagaagact 420
   ggggtggtgt ccacaggcct gatccacaat ggagactgga ccttccagac cctggtgatg 480
   ctggaaacag ttcctcggag tggagaggtt tacacctgcc aagtggagca cccaagcgtg 540
   acaagccctc tcacagtgga atggagagca cggtctgaat ctgcacagag caagatgctg 600
   agtggagtcg ggggctttgt gctgggcctg ctcttccttg gggccgggct gttcatctac 660
   ttcaggaatc agaaaggaca ctctggactt cagccaagag gattcctgag ctga 714
<210> 602
   <211> 269
   <212> DNA
   <213> Homo sapiens
<400> 602
   cacgtttctt ggagtactct acgtctgagt gtcatttctt caatgggacg gagcgggtgc 60
   ggtacctgga cagatacttc cataaccagg aggagaacgt gcgcttcgac agcgacgtgg 120
   gggagttccg ggcggtgacg gagctggggc ggcctgatgc cgagtactgg aacagccaga 180
   aggacctcct ggagcagaag cggggccagg tggacaacta ctgcagacac aactacgggg 240
   ttgtggagag cttcacagtg cagcggcga 269
<210> 603
   <211> 240
   <212> DNA
   <213> Homo sapiens
<400> 603
   ttggagtact ctacgtctga gtgtcatttc ttcaatggga cggagcgggt gcggtacctg 60
   gacagatact tccataacca ggaggagaac gtgcgcttcg acagcgacgt gggggagttc 120
   cgggcggtga cggagctggg gcggcctagc gccgagtact ggaacagcca gaaggacctc 180
   ctggagcaga agcggggccg ggtggacaac tactgcagac acaactacgg ggttgtggag 240
<210> 604
   <211> 270
   <212> DNA
   <213> Homo sapiens
<400> 604
   cacgtttctt ggagtactct acgtctgagt gtcatttctt caatgggacg gagcgggtgc     60
   ggtacctgga cagatacttc cataaccagg aggagaacgt gcgcttcgac agcgacgtgg    120
   gggagttccg ggcggtgacg gagctggggc ggcctgatgc cgagtcctgg aacagccaga    180
   aggacctcct ggagcagaag cggggccggg tggacaacta ctgcagacac aactacgggg    240
   ttgtggagag cttcacagtg cagcggcgag                                     270
<210> 605
   <211> 270
   <212> DNA
   <213> Homo sapiens
<400> 605
   cacgtttctt ggagtactct acgtctgagt gtcatttctt caatgggacg gagcgggtgc     60
   ggtacctgga cagatacttc cataaccagg aggagaacgt gcgcttcgac agcgacgtgg    120
   gggagttccg ggcggtgacg gagctggggc ggcctgatgc cgagtactgg aacagccaga    180
   aggacctcct ggagcagaag cggggccggg tggacaccta ctgcagacac aactacgggg    240
   ttggtgagag cttcacagtg cagcggcgag                                     270
<210> 606
   <211> 269
   <212> DNA
   <213> Homo sapiens
<400> 606
   cacgtttctt ggagtactct acgtctgagt gtcatttctt caatgggacg gagcgggtgc     60
   ggtacctgga cagatacttc cataaccagg aggagaacgt gcgcttcgac agcgacgtgg    120
   gggagttccg ggcggtgacg gagctggggc ggcctgatgc cgagtactgg aacagccaga    180
   aggacctcct ggagcagaag cggggccggg tggacaccta ctgcagacac aactacgggg    240
   ttgtggagag cttcacagtg cagcggcga                                      269
<210> 607
   <211> 245
   <212> DNA
   <213> Homo sapiens
<400> 607
   cacgtttctt ggagtactct acgtctgagt gtcatttctt caatgggacg gagcgggtgc 60
   ggtacctgga cagatacttc cataaccagg aggagaacgt gcgcttcgac agcgacgtgg 120
   gggagttctg ggcggtgacg gagctggggc ggcctgatgc cgagtactgg aacagccaga 180
   aggacctcct ggagcagaag cggggccggg tggacaacta ctgcagacac aactacgggg 240
   ttgtg 245
<210> 608
   <211> 270
   <212> DNA
   <213> Homo sapiens
<400> 608
   cacgtttctt ggagtactct acgtctgagt gtcatttctt caatgggacg gagcgggtgc 60
   ggttcctgga cagatacttc tataaccaag aggagtacgt gcgcttcgac agcgacgtgg 120
   gggagtaccg ggcggtgagg gagctggggc ggcctgatgc cgagtactgg aacagccaga 180
   aggacctcct ggagcagaag cggggccagg tggacaatta ctgcagacac aactacgggg 240
   ttggtgagag cttcacagtg cagcggcgag 270
<210> 609
   <211> 270
   <212> DNA
   <213> Homo sapiens
<400> 609
   cacgtttctt ggagtactct acgtctgagt gtcatttctt caatgggacg gagcgggtgc 60
   ggtacctgga cagatacttc cataaccagg aggagaacgt gcgcttcgac agcgacgtgc 120
   gggagttccg ggcggtgacg gagctggggc ggcctgatgc cgagtactgg aacagccaga 180
   aggacctcct ggagcagaag cggggccggg tggacaacta ctgcagacac aactacgggg 240
   ttgtggagag cttcacagtg cagcggcgag 270
<210> 610
   <211> 270
   <212> DNA
   <213> Homo sapiens
<400> 610
   cacgtttctt ggagtactct acgtctgagt gtcatttctt caatgggacg gagcgggtgc 60
   ggtacctgga cagatacttc cataaccagg aggagaacgt gcgcttcgac agcgacgtgg 120
   gggagttccg ggcggtgacg gagctggggc ggcctgatgc cgagtactgg aacagccaga 180
   aggacatcct ggagcagaag cggggccggg tggacaacta ctgcagacac aactacgggg 240
   ttgtggagag cttcacagtg cagcggcgag 270
<210> 611
   <211> 266
   <212> DNA
   <213> Homo sapiens
<400> 611
   cacgtttctt ggagtactct acgtctgagt gtcatttctt caatgggacg gagcgggtgc 60
   ggtacctgga cagatacttc cataaccagg aggagaacgt gcgcttcgac agcgacgtgg 120
   gggagttccg ggcggtgacg gagctggggc ggcctgatgc cgagtactgg aacagccaga 180
   aggacctcct ggagcagaag cggggccggg tggacaacta ctgcagacac aactacgggg 240
   ctgtggagag cttcacagtg cagcgg 266
<210> 612
   <211> 801
   <212> DNA
   <213> Homo sapiens
<400> 612
   atggtgtgtc tgaagttccc tggaggctcc tgcatggcag ctctgacagt gacactgatg 60
   gtgctgagct ccccactggc tttggctggg gacacccgac cacgtttctt ggagcaggtt 120
   aaacatgagt gtcatttctt caacgggacg gagcgggtgc ggttcctgga cagatacttc 180
   tatcaccaag aggagtacgt gcgcttcgac agcgacgtgg gggagtaccg ggcggtgacg 240
   gagctggggc ggcctgatgc cgagtactgg aacagccaga aggacctcct ggagcagaag 300
   cgggccgcgg tggacaccta ctgcagacac aactacgggg ttggtgagag cttcacagtg 360
   cagcggcgag tctatcctga ggtgactgtg tatcctgcaa agacccagcc cctgcagcac 420
   cacaacctcc tggtctgctc tgtgaatggt ttctatccag gcagcattga agtcaggtgg 480
   ttccggaacg gccaggaaga gaagactggg gtggtgtcca caggcctgat ccagaatgga 540
   gactggacct tccagaccct ggtgatgctg gaaacagttc ctcggagtgg agaggtttac 600
   acctgccaag tggagcaccc aagcctgacg agccctctca cagtggaatg gagagcacgg 660
   tctgaatctg cacagagcaa gatgctgagt ggagtcgggg gcttcgtgct gggcctgctc 720
   ttccttgggg ccgggctgtt catctacttc aggaatcaga aaggacactc tggacttcag 780
   ccaacaggat tcctgagctg a 801
<210> 613
   <211> 270
   <212> DNA
   <213> Homo sapiens
<400> 613
   cacgtttctt ggagcaggtt aaacatgagt gtcatttctt caacgggacg gagcgggtgc 60
   ggttcctgga cagatacttc tatcaccaag aagagtacgt gcgcttcgac agcgacgtgg 120
   gggagtaccg ggcggtgacg gagctggggc ggcctgatgc cgagtactgg aacagccaga 180
   aggacctcct ggagcagaag cgggccgcgg tggacaccta ctgcagacac aactacgggg 240
   ttggtgagag cttcacagtg cagcggcgag 270
<210> 614
   <211> 795
   <212> DNA
   <213> Homo sapiens
<400> 614
   tgtctgaagt tccctggagg ctcctgcatg gcagctctga cagtgacact gatggtgctg 60
   agctccccac tggctttggc tggggacacc cgaccacgtt tcttggagca ggttaaacat 120
   gagtgtcatt tcttcaacgg gacggagcgg gtgcggttcc tggacagata cttctatcac 180
   caagaggagt acgtgcgctt cgacagcgac gtgggggagt accgggcggt gacggagctg 240
   gggcggcctg atgccgagta ctggaacagc cagaaggaca tcctggaaga cgagcgggcc 300
   gcggtggaca cctactgcag acacaactac ggggttgtgg agagcttcac agtgcagcgg 360
   cgagtctatc ctgaggtgac tgtgtatcct gcaaagaccc agcccctgca gcaccacaac 420
   ctcctggtct gctctgtgaa tggtttctat ccaggcagca ttgaagtcag gtggttccgg 480
   aacggccagg aagagaagac tggggtggtg tccacaggcc tgatccagaa tggagactgg 540
   accttccaga ccctggtgat gctggaaaca gttcctcgga gtggagaggt ttacacctgc 600
   caagtggagc acccaagcct gacgagccct ctcacagtgg aatggagagc acggtctgaa 660
   tctgcacaga gcaagatgct gagtggagtc gggggcttcg tgctgggcct gctcttcctt 720
   ggggccgggc tgttcatcta cttcaggaat cagaaaggac actctggact tcagccaaca 780
   ggattcctga gctga 795
<210> 615
   <211> 714
   <212> DNA
   <213> Homo sapiens
<400> 615
<210> 616
   <211> 270
   <212> DNA
   <213> Homo sapiens
<400> 616
   cacgtttctt ggagcaggtt aaacatgagt gtcatttctt caacgggacg gagcgggtgc 60
   ggttcctgga cagatacttc tatcaccaag aggagtacgt gcgcttcgac agcgacgtgg 120
   gggagtaccg ggcggtgacg gagctggggc ggcctgacgc tgagtactgg aacagccaga 180
   aggacctcct ggagcagagg cgggccgagg tggacaccta ctgcagacac aactacgggg 240
   ttgtggagag cttcacagtg cagcggcgag 270
<210> 617
   <211> 801
   <212> DNA
   <213> Homo sapiens
<400> 617
   atggtgtgtc tgaagttccc tggaggctcc tgcatggcag ctctgacagt gacactgatg 60
   gtgctgagct ccccactggc tttggctggg gacacccgac cacgtttctt ggagcaggtt 120
   aaacatgagt gtcatttctt caacgggacg gagcgggtgc ggttcctgga cagatacttc 180
   tatcaccaag aggagtacgt gcgcttcgac agcgacgtgg gggagtaccg ggcggtgacg 240
   gagctggggc ggcctgatgc cgagtactgg aacagccaga aggacctcct ggagcagagg 300
   cgggccgcgg tggacaccta ctgcagacac aactacgggg ttgtggagag cttcacagtg 360
   cagcggcgag tctatcctga ggtgactgtg tatcctgcaa agacccagcc cctgcagcac 420
   cacaacctcc tggtctgctc tgtgaatggt ttctatccag gcagcattga agtcaggtgg 480
   ttccggaacg gccaggaaga gaagactggg gtggtgtcca caggcctgat ccagaatgga 540
   gactggacct tccagaccct ggtgatgctg gaaacagttc ctcggagtgg agaggtttac 600
   acctgccaag tggagcaccc aagcctgacg agccctctca cagtggaatg gagagcacgg 660
   tctgaatctg cacagagcaa gatgctgagt ggagtcgggg gcttcgtgct gggcctgctc 720
   ttccttgggg ccgggctgtt catctacttc aggaatcaga aaggacactc tggacttcag 780
   ccaacaggat tcctgagctg a 801
<210> 618
   <211> 282
   <212> DNA
   <213> Homo sapiens
<400> 618
   ggggacaccc gaccacgttt cttggagcag gttaaacatg agtgtcattt cttcaacggg 60
   acggagcggg tgcggttcct ggacagatac ttctatcacc aagaggagta cgtgcgcttc 120
   gacagcgacg tgggggagta ccgggcggtg acggagctgg ggcggcctag cgccgagtac 180
   tggaacagcc agaaggacct cctggagcag aggcgggccg cggtggacac ctactgcaga 240
   cacaactacg gggttggtga gagcttcaca gtgcagcggc ga 282
<210> 619
   <211> 270
   <212> DNA
   <213> Homo sapiens
<400> 619
   cacgtttctt ggagcaggtt aaacatgagt gtcatttctt caacgggacg gagcgggtgc 60
   ggttcctgga cagatacttc tatcaccaag aggagtacgt gcggttcgac agcgacgtgg 120
   gggagtaccg ggcggtgacg gagctggggc ggcctagcgc cgagtactgg aacagccaga 180
   aggacctcct ggagcagagg cgggccgcgg tggacaccta ctgcagacac aactacgggg 240
   ttggtgagag cttcacagtg cagcggcgag 270
<210> 620
   <211> 714
   <212> DNA
   <213> Homo sapiens
<400> 620
   ggggacaccc gaccacgttt cttggagcag gttaaacatg agtgtcattt cttcaacggg 60
   acggagcggg tgcggttcct ggacagatac ttctatcacc aagaggagtc cgtgcgcttc 120
   gacagcgacg tgggggagta ccgggcggtg acggagctgg ggcggcctga tgccgagtac 180
   tggaacagcc agaaggacct cctggagcag aggcgggccg aggtggacac ctactgcaga 240
   cacaactacg gggttgtgga gagcttcaca gtgcagcggc gagtctatcc tgaggtgact 300
   gtgtatcctg caaagaccca gcccctgcag caccacaacc tcctggtctg ctctgtgaat 360
   ggtttctatc caggcagcat tgaagtcagg tggttccgga acggccagga agagaagact 420
   ggggtggtgt ccacaggcct gatccagaat ggagactgga ccttccagac cctggtgatg 480
   ctggaaacag ttcctcggag tggagaggtt tacacctgcc aagtggagca cccaagcctg 540
   acgagccctc tcacagtgga atggagagca cggtctgaat ctgcacagag caagatgctg 600
   agtggagtcg ggggcttcgt gctgggcctg ctcttccttg gggccgggct gttcatctac 660
   ttcaggaatc agaaaggaca ctctggactt cagccaacag gattcctgag ctga 714
<210> 621
   <211> 282
   <212> DNA
   <213> Homo sapiens
<400> 621
   ggggacaccc gaccacgttt cttggagcag gttaaacatg agtgtcattt cttcaacggg 60
   acggagcggg tgcggttcct ggacagatac ttctatcacc aagaggagta cgtgcgcttc 120
   gacagcgacg tgggggagta ccgggcggtg acggagctgg ggcggcctga tgccgagtac 180
   tggaacagcc agaaggacct cctggagcag aggcgggccg aggtggacac ctactgcaga 240
   cacaactacg gggttggtga gagcttcaca gtgcagcggc ga 282
<210> 622
   <211> 266
   <212> DNA
   <213> Homo sapiens
<400> 622
   cacgtttctt ggagcaggtt aaacatgagt gtcatttctt caacgggacg gagcgggtgc 60
   ggttcctgga cagatacttc tatcaccaag aggagtacgt gcgcttcgac agcgacgtgg 120
   gggagtaccg ggcggtgacg gagctggggc ggcctgatgc cgagtactgg aacagccaga 180
   aggacctcct ggagcagaga cgggccgagg tggacaccta ctgcagacac aactacgggg 240
   ttggtgagag cttcacagtg cagcgg 266
<210> 623
   <211> 266
   <212> DNA
   <213> Homo sapiens
<400> 623
   tttcttggag caggttaaac atgagtgtca tttcttcaac gggacggagc gggtgcggtt 60
   cctggacaga tacttctatc accaagagga gtacgtgcgc ttcgacagcg acgtggggga 120
   gtaccgggcg gtgacggagc tggggcggcc tgatgccgag tactggaaca gccagaagga 180
   cctcctggag cagaggcggg ccgcggtgga cacctactgc agacacaact acggggttgg 240
   tgagagcttc acagtgcagc ggcgag 266

<210> 624
   <211> 225
   <212> DNA
   <213> Homo sapiens
<400> 624
   tgagtgtcat ttcttcaacg ggacggagcg ggtgcggttc ctggacagat acttctatca 60
   ccaagaggag tacgtgcgct tcgacagcga cgtgggggag taccgggcgg tgacggagct 120
   ggggcggcct agcgccgagt actggaacag ccagaaggac ctcctggagc agaagcgggc 180
   cgcggtggac acctactgca gacacaacta cggggttggt gagag 225
<210> 625
   <211> 253
   <212> DNA
   <213> Homo sapiens
<400> 625
   tttcttggag caggttaaac atgagtgtca tttcttcaac gggacggagc gggtgcggtt 60
   cctggacaga tacttctatc accaagagga gtacgtgcgc ttcgacagcg acgtggggga 120
   gtaccgggcg gtgacggagc tggggcggcc tagcgccgag tactggaaca gccagaagga 180
   cctcctggag cagaggcggg ccgcggtgga cacctactgc agacacaact acggggttgt 240
   ggagagcttc aca 253
<210> 626
   <211> 801
   <212> DNA
   <213> Homo sapiens
<400> 626
   atggtgtgtc tgaagttccc tggaggctcc tgcatggcag ctctgacagt gacactgatg 60
   gtgctgagct ccccactggc tttggctggg gacacccgac cacgtttctt ggagcaggtt 120
   aaacatgagt gtcatttctt caacgggacg gagcgggtgc ggttcctgga cagatacttc 180
   tatcaccaag aggagtacgt gcgcttcgac agcgacgtgg gggagtaccg ggcggtgacg 240
   gagctggggc ggcctagcgc cgagtactgg aacagccaga aggacctcct ggagcagagg 300
   cgggccgagg tggacaccta ctgcagacac aactacgggg ttgtggagag cttcacagtg 360
   cagcggcgag tctatcctga ggtgactgtg tatcctgcaa agacccagcc cctgcagcac 420
   cacaacctcc tggtctgctc tgtgaatggt ttctatccag gcagcattga agtcaggtgg 480
   ttccggaacg gccaggaaga gaagactggg gtggtgtcca caggcctgat ccagaatgga 540
   gactggacct tccagaccct ggtgatgctg gaaacagttc ctcggagtgg agaggtttac 600
   acctgccaag tggagcaccc aagcctgacg agccctctca cagtggaatg gagagcacgg 660
   tctgaatctg cacagagcaa gatgctgagt ggagtcgggg gcttcgtgct gggcctgctc 720
   ttccttgggg ccgggctgtt catctacttc aggaatcaga aaggacactc tggacttcag 780
   ccaacaggat tcctgagctg a 801
<210> 627
   <211> 261
   <212> DNA
   <213> Homo sapiens
<400> 627
   ttcttggagc aggttaaaca tgagtgtcat ttcttcaacg ggacggagcg ggtgcggttc 60
   ctggacagat acttctatca ccaagaggag tacgtgcgct tcgacagcga cgtgggggag 120
   taccgggcgg tgacggagct ggggcggcct agcgccgagt actggaacag ccagaaggac 180
   atcctggaag acaggcgggc cctggtggac acctactgca gacacaacta cggggttgtg 240
   gagagcttca cagtgcagcg g 261
<210> 628
   <211> 234
   <212> DNA
   <213> Homo sapiens
<400> 628
   catgagtgtc atttcttcaa cgggacggag cgggtgcggt tcctggacag atacttctat 60
   caccaagagg agtacgtgcg cttcgacagc gacgtggggg agtaccgggc ggtgacggag 120
   ctggggcggc ctgatgccga gtactggaac agccagaagg acctcctgga gcagaagcgg 180
   gccgcggtgg acacctactg cagacacaac tacggggttg tggagagctt caca 234
<210> 629
   <211> 225
   <212> DNA
   <213> Homo sapiens
<400> 629
   tgagtgtcat ttcttcaacg ggacggagcg ggtgcggttc ctggacagat acttctatca 60
   ccaagaggag tacgtgcgct tcgacagcga cgtgggggag taccgggcgg tgacggagct 120
   ggggcggcct gatgccgagt actggaacag ccagaaggac atcctggaag acgagcgggc 180
   cgcggtggac acctactgca gacacaacta cggggttggt gagag 225
<210> 630
   <211> 250
   <212> DNA
   <213> Homo sapiens
<400> 630
   cacgtttctt ggagcaggtt aaacatgagt gtcatttctt caacgggacg gagcgggtgc 60
   ggttcctgga cagatacttc tatcaccaag aggagtacgt gcgcttcgac agcgacgtgg 120
   gggagtaccg ggcggtgacg gagctggggc ggcctgatga ggagtactgg aacagccaga 180
   aggacttcct ggaagacagg cgggccgcgg tggacaccta ctgcagacac aactacgggg 240
   ttgtggagag 250
<210> 631
   <211> 222
   <212> DNA
   <213> Homo sapiens
<400> 631
   atgagtgtca tttcttcaac gggacggagc gggtgcggtt cctggacaga tacttctatc      60
   accaagagga gtacgtgcgc ttcgacagcg acgtggggga gtaccgggcg gtgacggagc     120
   tggggcggcc tgatgcccag tactggaaca gccagaagga cctcctggag cagaagcggg     180
   ccgcggtgga cacctactgc agacacaact acggggttgg tg                        222
<210> 632
   <211> 221
   <212> DNA
   <213> Homo sapiens
<400> 632
   atgagtgtca tttcttcaac gggacggagc gggtgcggtt cctggacaga tacttctatc      60
   accaagagga gtacgtgcgc ttcgacagcg acgtggggga gtaccgggcg gtgacggagc     120
   tggggcggcc tagcgccgag tactggaaca gccagaagga cctcctggag cagaggcggg     180
   ccgaggtgga cacctactgc agacacaact acggggttgg t                         221
<210> 633
   <211> 238
   <212> DNA
   <213> Homo sapiens
<400> 633
   atgagtgtca tttcttcaac gggacggagc gggtgcggtt cctggacaga tacttctatc      60
   accaagagga gtacgtgcgc ttcgacagcg acgtggggga gtaccgggcg gtgacggagc     120
   tggggcggcc tgatgccgag tactggaaca gccagaagga catcctggaa gacaggcggg     180
   ccctggtgga cacctactgc agacacaact acggggttgt ggagagcttc acagtgca       238
<210> 634
   <211> 237
   <212> DNA
   <213> Homo sapiens
<400> 634
   catgagtgtc atttcttcaa cgggacggag cgggtgcggt tcctggacag atacttctat 60
   caccaagagg agtccgtgcg cttcgacagc gacgtggggg agtaccgggc ggtgacggag 120
   ctggggcggc ctgatgccga gtactggaac agccagaagg acctcctgga gcagaggcgg 180
   gccgcggtgg acacctactg cagacacaac tacggggttg gtgagagctt cacagtg 237
<210> 635
   <211> 222
   <212> DNA
   <213> Homo sapiens
<400> 635
   atgagtgtca tttcttcaac gggacggagc gggtgcggtt cctggacaga tacttctatc 60
   accaagagga gtccgtgcgc ttcgacagcg acgtggggga gtaccgggcg gtgacggagc 120
   tggggcggcc tgatgccgag tactggaaca gccagaagga cctcctggag cagaggcggg 180
   ccgaggtgga cacctactgc agacacaact acggggttgg tg 222
<210> 636
   <211> 249
   <212> DNA
   <213> Homo sapiens
<400> 636
   gagcaggtta aacatgagtg tcatttcttc aacgggacgg agcgggtgcg gttcctggac 60
   agatacttct atcaccaaga ggagtccgtg cgcttcgaca gcgacgtggg ggagtaccgg 120
   gcggtgacgg agctggggcg gcctgatgcc gagtactgga acagccagaa ggacctcctg 180
   gagcagaagc gggccgcggt ggacacctac tgcagacaca actacggggt tggtgagagc 240
   ttcacagtg 249
<210> 637
   <211> 246
   <212> DNA
   <213> Homo sapiens
<400> 637
   gagcaggtta aacatgagtg tcatttcttc aacgggacgg agcgggtgcg gttcctggac 60
   agatacttct atcaccaaga ggagtacgtg cgcttcgaca gcgacgtggg ggagtaccgg 120
   gcggtgacgg agctggggcg gcctgatgcc gagtactgga acagccagaa ggacctcctg 180
   gagcagaagc ggggccgggt ggacaactac tgcagacaca actacggggt tgtggagagc 240
   ttcaca 246
<210> 638
   <211> 270
   <212> DNA
   <213> Homo sapiens
<400> 638
   cacgtttctt ggagcaggtt aaacatgagt gtcatttctt caacgggacg gagcgggtgc 60
   ggttcctgga cagatacttc tatcaccaag aggagtacgt gcgcttcgac agcgacgtgg 120
   gggagtaccg ggcggtgacg gagctggggc ggcctgatgc cgagtactgg aacagccaga 180
   aggacctcct ggagcagagg cgggccgcgg tggacaccta ctgcagacac aactacgggg 240
   ttgtggagag attcacagtg cagcggcgag 270
<210> 639
   <211> 270
   <212> DNA
   <213> Homo sapiens
<400> 639
   cacgtttctt ggagcaggtt aaacatgagt gtcatttctt caacgggacg gagcgggtgc 60
   ggttcctgga cagatacttc tatcaccaag aggagtacgt gcgcttcgac agcgacgtgg 120
   gggagtaccg ggcggtgacg gagctggggc ggcctagcgc cgagtactgg aacagccaga 180
   aggacctcct ggagcggagg cgggccgcgg tggacaccta ctgcagacac aactacgggg 240
   ttggtgagag cttcacagtg cagcggcgag 270
<210> 640
   <211> 242
   <212> DNA
   <213> Homo sapiens
<400> 640
   ttggagcagg ttaaacatga gtgtcatttc ttcaacggga cggagcgggt gcggttcctg 60
   gacagatact tctatcacca agaggagtac gtgcgcttcg acagcgacgt gggggagtac 120
   cgggcggtga cggagctggg gcggcctgat gccgagtact ggaacagcca gaaggacttc 180
   ctggaagaca ggcgggccct ggtggacacc tactgcagac acaactacgg ggttgtggag 240
   ag 242
<210> 641
   <211> 246
   <212> DNA
   <213> Homo sapiens
<400> 641
   cacgtttctt ggagcaggtt aaacatgagt gtcatttctt caacgggacg gagcgggtgc 60
   ggttcctgga cagatacttc tatcaccaag aggagtacgt gcgcttcgac agcgacgtgg 120
   gggagtaccg ggcggtgacg gagctggggc ggcctgatac cgagtactgg aacagccaga 180
   aggacctcct ggagcagaag cgggccgcgg tggacaccta ctgcagacac aactacgggg 240
   ttggtg 246
<210> 642
   <211> 260
   <212> DNA
   <213> Homo sapiens
<400> 642
   cacgtttctt ggagcaggtt aaacatgagt gtcatttctt caacgggacg gagcgggtgc 60
   ggttcctgga cagatacttc tatcaccaag aggagtacgt gcgcttcgac agcgacgtgg 120
   gggagtaccg ggcggtgacg gagctggggc ggcctgatgc cgagtactgg aacagccaga 180
   aggacctcct ggagcagagg cgggccgagg tggacaccta ctgcagacac aactacgggg 240
   ctgtggagag cttcacagtg 260
<210> 643
   <211> 270
   <212> DNA
   <213> Homo sapiens
<400> 643
   cacgtttctt ggagcaggtt aaacatgagt gtcatttctt caacgggacg gagcgggtgc 60
   ggttcctgga cagatacttc tatcaccaag aggagtacgt gcgcttcgac agcgacgtgg 120
   gggagttccg ggcggtgacg gagctggggc ggcctagcgc cgagtactgg aacagccaga 180
   aggacctcct ggagcagagg cgggccgcgg tggacaccta ctgcagacac aactacgggg 240
   ttggtgagag cttcacagtg cagcggcgag 270
<210> 644
   <211> 270
   <212> DNA
   <213> Homo sapiens
<400> 644
   cacgtttctt ggagcaggtt aaacatgagt gtcatttctt caacgggacg gagcgggtgc 60
   ggttcctgga cagatacttc tatcaccaag aggagtacgt gcgcttcgac agcgacgtgg 120
   gggagtaccg ggcggtgatg gagctggggc ggcctagcgc cgagtactgg aacagccaga 180
   aggacctcct ggagcagagg cgggccgcgg tggacaccta ctgcagacac aactacgggg 240
   ttggtgagag cttcacagtg cagcggcgag 270
<210> 645
   <211> 270
   <212> DNA
   <213> Homo sapiens
<400> 645
   cacgtttctt ggagcaggtt aaacatgagt gtcatttctt caacgggacg gagcgggtgc 60
   ggttcctgga cagatacttc tatcaccaag aggagtacgt gcgcttcgac agcgacgtgg 120
   gggagtaccg ggtggtgacg gagctggggc ggcctagcgc cgagtactgg aacagccaga 180
   aggacctcct ggagcagagg cgggccgcgg tggacaccta ctgcagacac aactacgggg 240
   ttggtgagag cttcacagtg cagcggcgag 270
<210> 646
   <211> 270
   <212> DNA
   <213> Homo sapiens
<400> 646
   cacgtttctt ggagcaggtt aaacatgagt gtcatttctt caacgggacg gagcgggtgc 60
   ggttcctgga cagatacttc tatcaccaag aggagtacgt gcgcttcgac agcgacgtgg 120
   gggagtaccg ggcggtgacg gagctggggc ggcctgatgc cgagtactgg aacagccaga 180
   aggacctcct ggagcagagg cgggccctgg tggacaccta ctgcagacac aactacgggg 240
   ttggtgagag cttcacagtg cagcggcgag 270
<210> 647
   <211> 240
   <212> DNA
   <213> Homo sapiens
<400> 647
   ttggagcagg ttaaacatga gtgtcatttc ttcaacggga cggagcgggt gcggttcctg 60
   gacagatact tctatcacca agaggagtac gtgcgcttcg acagcgacgt gggggagtac 120
   cgggcggtga cggagctggg gcggcctgat gccgagtact ggaacagcca gaaggacctc 180
   ctggagcaga ggcaggccgc ggtggacacc tactgcagac acaactacgg ggttgtggag 240
<210> 648
   <211> 270
   <212> DNA
   <213> Homo sapiens
<400> 648
   cacgtttctt ggagcaggtt aaacatgagt gtcatttctt caacgggacg gagcgggtgc 60
   ggttcctgga cagatacttc tatcaccaag aggagtacgt gcacttcgac agcgacgtgg 120
   gggagtaccg ggcggtgacg gagctggggc ggcctgatgc cgagtactgg aacagccaga 180
   aggacctcct ggagcagaag cgggccgcgg tggacaccta ctgcagacac aactacgggg 240
   ttggtgagag cttcacagtg cagcggcgag 270
<210> 649
   <211> 243
   <212> DNA
   <213> Homo sapiens
<400> 649
   tttcttggag caggttaaac ctgagtgtca tttcttcaac gggacggagc gggtgcggtt 60
   cctggacaga tacttctatc accaagagga gtacgtgcgc ttcgacagcg acgtggggga 120
   gtaccgggcg gtgacggagc tggggcggcc tgatgccgag tactggaaca gccagaagga 180
   cctcctggag cagaagcggg ccgcggtgga cacctactgc agacacaact acggggttgg 240
   tga 243
<210> 650
   <211> 260
   <212> DNA
   <213> Homo sapiens
<400> 650
   cacgtttctt ggagcaggtt aaacatgagt gtcatttctt caacgggacg gagcgggtgc 60
   ggttcctgga cagatacttc tatcaccaag aggagtacgt gcgcttcgac agcgacgtgg 120
   gggagttccg ggcggtgacg gagctggggc ggcctgatgc cgagtactgg aacagccaga 180
   aggacctcct ggagcagaag cgggccgcgg tggacaccta ctgcagacac aactacgggg 240
   ttggtgagag cttcacagtg 260
<210> 651
   <211> 270
   <212> DNA
   <213> Homo sapiens
<400> 651
   cacgtttctt ggagcaggtt aaacatgagt gtcatttctt caacgggacg gagcgggtgc 60
   ggttcctgga cagatacttc tatcaccaag aggagtacgt gcgcttcgac agcgacgtgg 120
   gggagtaccg ggcggtgacg gagctggggc ggcctgatgc cgagtactgg aacagccaga 180
   aggacttcct ggaagacagg cgggccgcgg tggacaccta ctgcagacac aactacgggg 240
   ttgtggagag cttcacagtg cagcggcgag 270
<210> 652
   <211> 270
   <212> DNA
   <213> Homo sapiens
<400> 652
   cacgtttctt ggagcaggtt aaacatgagt gtcatttctt caacgggacg gagcgggtgc 60
   ggttcctgga cagatacttc tatcaccaag aggagtacgt gcgcttcgac agcgacgtgg 120
   gggagtaccg ggcggtgacg gagctggggc ggcctgatgc cgagtactgg aacagccaga 180
   aggacctcct ggaagacgag cgggccgcgg tggacaccta ctgcagacac aactacgggg 240
   ttgtggagag cttcacagtg cagcggcgag 270
<210> 653
   <211> 270
   <212> DNA
   <213> Homo sapiens
<400> 653
   cacgtttctt ggagcaggtt aaacatgagt gtcatttctt caacgggacg gagcgggtgc 60
   ggttcctgga cagatacttc tatcaccaag aggagtacgt gcgcttcgac agcgacgtgg 120
   gggagtaccg ggcggtgacg gagctggggc ggcctgatgc cgagtactgg aacagccaga 180
   aggacatcct ggagcagaag cgggccgcgg tggacaccta ctgcagacac aactacgggg 240
   ttggtgagag cttcacagtg cagcggcgag 270
<210> 654
   <211> 266
   <212> DNA
   <213> Homo sapiens
<400> 654
   cacgtttctt ggagcaggtt aaacatgagt gtcatttctt caacgggacg gagcgggtgc 60
   ggttcctgga cagatacttc tatcaccaag aggagtacgt gcgcttcgac agcgacgtgg 120
   gggactaccg ggcggtgacg gagctggggc ggcctgatgc cgagtactgg aacagccaga 180
   aggacctcct ggagcagagg cgggccgagg tggacaccta ctgcagacac aactacgggg 240
   ttgtggagag cttcacagtg cagcgg 266
<210> 655
   <211> 266
   <212> DNA
   <213> Homo sapiens
<400> 655
   cacgtttctt ggagcaggtt aaacatgagt gtcatttctt caacgggacg gagcgggtgc 60
   ggttcctgga cagatacttc tatcaccaag aggagtacgt gcgcttcgac agcgacgtgg 120
   gggagtaccg ggcggtgacg gagctggggc ggcctgatgg cgagtactgg aacagccaga 180
   aggacctcct ggagcagagg cgggccgcgg tggacaccta ctgcagacac aactacgggg 240
   ttgtggagag cttcacagtg cagcgg 266
<210> 656
   <211> 266
   <212> DNA
   <213> Homo sapiens
<400> 656
   cacgtttctt ggagcaggtt aaacatgagt gtcatttctt caacgggacg gagcgggtgc 60
   ggttcctgga cagatacttc tatcaccaag aggagaacgt gcgcttcgac agcgacgtgg 120
   gggagtaccg ggcggtgacg gagctggggc ggcctgatgc cgagtactgg aacagccaga 180
   aggacctcct ggagcagagg cgggccgagg tggacaccta ctgcagacac aactacgggg 240
   ttgtggagag cttcacagtg cagcgg 266
<210> 657
   <211> 270
   <212> DNA
   <213> Homo sapiens
<400> 657
   cacgtttctt ggagcaggtt aaacatgagt gtcatttctt caacgggacg gagcgggtgc 60
   ggttcctgga cagatacttc tatcaccaag aggagtacgt gcgcttcgac agcgacgtgg 120
   gggagttccg ggcggtgacg gagctggggc ggcctgatgc cgagtactgg aacagccaga 180
   aggacctcct ggagcagagg cgggccgcgg tggacaccta ctgcagacac aactacgggg 240
   ttgtggagag cttcacagtg cagcggcgag 270
<210> 658
   <211> 801
   <212> DNA
   <213> Homo sapiens
<400> 658
   atggtgtgtc tgaagctccc tggaggctcc tgcatggcag ctctgacagt gacactgatg 60
   gtgctgagct ccccactggc tttggctggg gacacccaac cacgtttcct gtggcagggt 120
   aagtataagt gtcatttctt caacgggacg gagcgggtgc agttcctgga aagactcttc 180
   tataaccagg aggagttcgt gcgcttcgac agcgacgtgg gggagtaccg ggcggtgacg 240
   gagctagggc ggcctgtcgc cgagtcctgg aacagccaga aggacatcct ggaggacagg 300
   cggggccagg tggacaccgt gtgcagacac aactacgggg ttggtgagag cttcacagtg 360
   cagcggcgag tccatcctga ggtgactgtg tatcctgcca agactcagcc cctgcagcac 420
   cacaacctcc tggtctgctc tgtgagtggt ttctatccag gcagcattga agtcaggtgg 480
   ttccggaacg gccaggaaga gaaggctggg gtggtgtcca caggcctgat ccagaatgga 540
   gactggacct tccagaccct ggtgatgctg gaaacagttc ctcggagtgg agaagtttac 600
   acctgccaag tggagcaccc aagtgtgatg agccctctca cagtggaatg gagagcacgg 660
   tctgaatctg cacagagcaa gatgctgagt ggagtcgggg gctttgtgct gggcctgctc 720
   ttccttgggg ccgggttgtt catctacttc aggaatcaga aaggacactc tggacttcag 780
   ccaacaggat tcctgagctg a 801
<210> 659
   <211> 246
<212> DNA
   <213> Homo sapiens
<400> 659
   cacgtttcct gtggcagggt aaatataagt gtcatttctt caacgggacg gagcgggtgc      60
   agttcctgga aagactcttc tataaccagg aggagttcgt gcgcttcgac agcgacgtgg     120
   gggagtaccg ggcggtgacg gagctagggc ggcctgtcgc cgagtcctgg aacagccaga     180
   aggacatcct ggaggacagg cggggccagg tggacaccgt gtgcagacac aactacgggg     240
   ttggtg                                                                246
<210> 660
   <211> 246
   <212> DNA
   <213> Homo sapiens
<400> 660
   cacgtttcct gtggcagggt aagtataagt gtcatttctt caacgggacg gagcgggtgc      60
   agttcctgga aagtctcttc tataaccagg aggagttcgt gcgcttcgac agcgacgtgg     120
   gggagtaccg ggcggtgacg gagctagggc ggcctgtcgc cgagtcctgg aacagccaga     180
   aggacatcct ggaggacagg cggggccagg tggacaccgt gtgcagacac aactacgggg     240
   ttggtg                                                                246
<210> 661
   <211> 247
   <212> DNA
   <213> Homo sapiens
<400> 661
   tttcctgtgg cagggtaagt ataagtgtca tttcttcaac gggacggagc gggtgcagtt      60
   cctggaaaga ctcttctata accaggagga gttcgtgcgc ttcgacagcg acgtggggga     120
   gtaccgggcg gtgacggagc tagggcggcc tgtcgccgag tcctggaaca gccagaagga     180
   catcctggag gacaggcggg gccaggtgga caattactgc agacacaact acggggttgg     240
   tgagagc                                                               247
<210> 662
   <211> 258
   <212> DNA
   <213> Homo sapiens
<400> 662
   cacgtttcct gtggcagggt aagtataagt gtcatttctt caacgggacg gagcgggtgc 60
   agttcctgga aagactcttc tataaccagg aggagttcgt gcgcttcgac agcgacgtgg 120
   gggagtaccg ggcggtgacg gagctagggc ggcctgtcgc cgagtcctgg aacagccgga 180
   aggacatcct ggaggacagg cggggccagg tggacaccgt gtgcagacac aactacgggg 240
   ttggtgagag cttcacag 258
<210> 663
   <211> 538
   <212> DNA
   <213> Homo sapiens

<400> 663
   ggggacaccc gaccacgttt cttggagtac tctacgggtg agtgttattt cttcaatggg 60
   acggagcggg tgcggttcct ggacagatac ttctataacc aagaggagta cgtgcgcttc 120
   gacagcgacg tgggggagta ccgggcggtg acggagctgg ggcggcctag cgccgagtac 180
   tggaacagcc agaaggactt cctggaagac aggcgggccc tggtggacac ctactgcaga 240
   cacaactacg gggttggtga gagcttcacg gtgcagcggc gagtccatcc taaggtgact 300
   gtgtatcctt caaagaccca gcccctgcag caccacaacc tcctggtctg ttctgtgagt 360
   ggtttctatc caggcagcat tgaagtcagg tggttccgga atggccagga agagaagact 420
   ggggtggtgt ccacaggcct gatccacaat ggagactgga ccttccagac cctggtgatg 480
   ctggaaacag ttcctcggag tggagaggtt tacacctgcc aagtggagca cccaagcg 538
<210> 664
   <211> 801
   <212> DNA
   <213> Homo sapiens
<400> 664
   atggtgtgtc tgaggctccc tggaggctcc tgcatggcag ttctgacagt gacactgatg 60
   gtgctgagct ccccactggc tttggctggg gacaccagac cacgtttctt ggagtactct 120
   acgggtgagt gttatttctt caatgggacg gagcgggtgc ggttcctgga cagatacttc 180
   tataaccaag aggagtacgt gcgcttcgac agcgacgtgg gggagtaccg ggcggtgacg 240
   gagctggggc ggcctgatgc cgagtactgg aacagccaga aggacttcct ggaagacagg 300
   cgggccctgg tggacaccta ctgcagacac aactacgggg ttggtgagag cttcacggtg 360
   cagcggcgag tccatcctaa ggtgactgtg tatccttcaa agacccagcc cctgcagcac 420
   cacaacctcc tggtctgttc tgtgagtggt ttctatccag gcagcattga agtcaggtgg 480
   ttccggaatg gccaggaaga gaagactggg gtggtgtcca caggcctgat ccacaatgga 540
   gactggacct tccagaccct ggtgatgctg gaaacagttc ctcggagtgg agaggtttac 600
   acctgccaag tggagcaccc aagcgtgaca agccctctca cagtggaatg gagtgcacgg 660
   tctgaatctg cacagagcaa gatgctgagt ggagtcgggg gctttgtgct gggcctgctc 720
   ttccttgggg ccgggctgtt catctacttc aggaatcaga aaggacactc tggacttcag 780
   ccaacaggat tcctgagctg a 801
<210> 665
   <211> 552
   <212> DNA
   <213> Homo sapiens
<400> 665
   cacgtttctt ggagtactct acgggtgagt gttatttctt caatgggacg gagcgggtgc 60
   ggttcctgga cagatacttc tataaccaag aggagtacgt gcgcttcgac agcgacgtgg 120
   gggagtaccg ggcggtgacg gagctggggc ggcctgatgc cgagtactgg aacagccaga 180
   aggacttcct ggaagacagg cgggccctgg tggacaccta ctgcagacac aactacgggg 240
   ttggtgagag cttcacagtg cagcggcgag tccatcctaa ggtgactgtg tatccttcaa 300
   agacccagcc cctgcagcac cacaacctcc tggtctgttc tgtgagtggt ttctatccag 360
   gcagcattga agtcaggtgg ttccggaatg gccaggaaga gaagactggg gtggtgtcca 420
   caggcctgat ccacaatgga gactggacct tccagaccct ggtgatgctg gaaacagttc 480
   ctcggagtgg agaggtttac acctgccaag tggagcaccc aagcgtgaca agccctctca 540
   cagtggaatg ga 552
<210> 666
   <211> 258
   <212> DNA
   <213> Homo sapiens
<400> 666
   cgtttcttgg agtactctac gggtgagtgt tatttcttca atgggacgga gcgggtgcgg 60
   ttcctggaca gatacttcta taaccaagag gagtacgtgc gcttcgacag cgacgtgggg 120
   gagtaccggg cggtgacaga gctggggcgg cctgatgccg agtactggaa cagccagaag 180
   gacttcctgg aagacaggcg ggccctggtg gacacctact gcagacacaa ctacggggtt 240
   ggtgagagct tcacggtg 258
<210> 667
   <211> 565
   <212> DNA
   <213> Homo sapiens
<400> 667
   ggggacacca gaccacgttt cttggagtac tctacgggtg agtgttattt cttcaatggg 60
   acggagcggg tgcggttcct ggacagatac ttctataacc aagaggagta cgtgcgcttc 120
   gacagcgacg tgggggagta ccgggcggtg acggagctgg ggcggcctag cgccgagtac 180
   tggaacagcc agaaggacat cctggaagac aggcgggccc tggtggacac ctactgcaga 240
   cacaactacg gggttggtga gagcttcaca gtgcagcggc gagtccatcc taaggtgact 300
   gtgtatcctt caaagaccca gcccctgcag caccacaacc tcctggtctg ttctgtgagt 360
   ggtttctatc caggcagcat tgaagtcagg tggttccgga atggccagga agagaagact 420
   ggggtggtgt ccacaggcct gatccacaat ggagactgga ccttccagac cctggtgatg 480
   ctggaaacag ttcctcggag tggagaggtt tacacctgcc aagtggagca cccaagcgtg 540
   acaagccctc tcacagtgga atgga 565
<210> 668
   <211> 304
   <212> DNA
   <213> Homo sapiens
<400> 668
   ggggacacca gaccacgttt cttggagtac tctacgggtg agtgttattt cttcaatggg 60
   acggagcggg tgcggttcct ggacagatac ttctataacc aagaggagta cgtgcgcttc 120
   gacagcgacg tgggggagta ccgggcggtg acggagctgg ggcggcctga tgccgagtac 180
   tggaacagcc agaaggactt cctggaagac aggcgggccc tggtggacac ctactgcaga 240
   cacaactacg gggttgtgga gagcttcaca gtgcagcggc gagtccatcc taaggtgact 300
   gtgt 304
<210> 669
   <211> 228
   <212> DNA
   <213> Homo sapiens
<400> 669
   ttcaatggga cggagcgggt gcggttcctg gacagatact tctataacca agaggagtac 60
   gtgcgcttcg acagcgacgt gggggagtac cgggcggtga cggagctggg gcggcctgat 120
   gccgagtact ggaacagcca gaaggacttc ctggaagaca ggcgggccct ggtggacacc 180
   tactgcagac acaactacgg ggttgttgag agcttcacag tgcagcgg 228
<210> 670
   <211> 269
   <212> DNA
   <213> Homo sapiens
<400> 670
   cacgtttctt ggagtactct acgggtgagt gttatttctt caatgggacg gagcgggtgc 60
   ggttcctgga cagatacttc tataaccaag aggagtacgt gcgcttcgac agcgacgtgg 120
   gggagtaccg ggcggtgacg gagctggggc ggcctgatgc cgagtactgg aacagccaga 180
   aggacttcct ggaagacagg cgggccctgg tggacaccta ctgcagacac aactacgggg 240
   ttgttgagag cttcacggtg cagcggcga 269
<210> 671
   <211> 269
   <212> DNA
   <213> Homo sapiens
<400> 671
   cacgtttctt ggagtactct acgggtgagt gttatttctt caatgggacg gagcgggtgc 60
   ggttcctgga cagatacttc tataaccaag aggagtacgt gcgcttcgac agcgacgtgg 120
   gggagtaccg ggcggtgacg gagctggggc ggcctgatgc cgagtactgg aacagccaga 180
   aggacttcct ggaagacagg cgggccctgg tggacaccta ctgcagacac aactacgggg 240
   ttgtggagag cttcacggtg cagcggcga 269
<210> 672
   <211> 245
   <212> DNA
   <213> Homo sapiens
<400> 672
   cacgtttctt ggagtactct acgggtgagt gttatttctt caatgggacg gagcgggtgc 60
   ggttcctgga cagatacttc tataaccaag aggagtacgt gcgcttcgac agcgacgtgg 120
   gggagtaccg ggcggtgacg gagctggggc ggcctagcgc cgagtactgg aacagccaga 180
   aggacttcct ggaagacagg cgggccgcgg tggacaccta ctgcagacac aactacgggg 240
   ttggt 245
<210> 673
   <211> 271
   <212> DNA
   <213> Homo sapiens
<400> 673
   ccacgtttct tggagtactc tacgggtgag tgttatttct tcaatgggac ggagcgggtg 60
   cggttcctgg acagatactt ctataaccaa gaggagtacg tgcgcttcga cagcgacgtg 120
   ggggagtacc gggcggtgac ggagctgggg cggcctagcg ccgagtactg gaacagccag 180
   aaggacttcc tggaagacag gcgggccctg gtggacacct actgcagaca caactacggg 240
   gttgtggaga gcttcacagt gcagcggcga g 271
<210> 674
   <211> 260
   <212> DNA
   <213> Homo sapiens
<400> 674
   cacgtttctt ggagtactct acgggtgagt gttatttctt caatgggacg gagcgggtgc 60
   ggttcctgga cagatacttc tataaccaag aggagtacgt gcgcttcgac agcgacgtgg 120
   gggagtaccg ggcggtgacg gagctggggc ggcctgttgc cgagtactgg aacagccaga 180
   aggacttcct ggaagacagg cgggccctgg tggacaccta ctgcagacac aactacgggg 240
   ttggtgagag cttcacggtg 260
<210> 675
   <211> 240
   <212> DNA
   <213> Homo sapiens
<400> 675
   ttggagtact ctacgggtga gtgttatttc ttcaatggga cggagcgggt gcggttcctg 60
   gacagatact tctataacca agaggagtac gtgcgcttcg acagcgacgt gggggagtac 120
   cgggcggtga cggagctggg gcggcctgct gcggagcact ggaacagcca gaaggacttc 180
   ctggaagaca ggcgggccct ggtggacacc tactgcagac acaactacgg ggttggtgag 240
<210> 676
   <211> 270
   <212> DNA
   <213> Homo sapiens
<400> 676
   cacgtttctt ggagtactct acgggtgagt gttatttctt caatgggacg gagcgggtgc 60
   ggttcctgga cagatacttc cataaccagg aggagttcgt gcgcttcgac agcgacgtgg 120
   gggagtaccg ggcggtgacg gagctggggc ggcctgatgc cgagtactgg aacagccaga 180
   aggacttcct ggaagacagg cgggccctgg tggacaccta ctgcagacac aactacgggg 240
   ttggtgagag cttcacggtg cagcggcgag 270
<210> 677
   <211> 270
   <212> DNA
   <213> Homo sapiens
<400> 677
   cacgtttctt ggagtactct acgggtgagt gttatttctt caatgggacg gagcgggtgc 60
   ggttcctgga cagatacttc tataaccaag aggagtacgt gcgcttcgac agcgacgtgg 120
   gggagtaccg ggcggtgacg gagctggggc ggcctagcgc cgagtactgg aacagccaga 180
   aggacatcct ggaagacagg cgggccctgg tggacaccta ctgcagacac aactacgggg 240
   ttgtggagag cttcacagtg cagcggcgag 270
<210> 678
   <211> 260
   <212> DNA
   <213> Homo sapiens
<400> 678
   cacgtttctt ggagtactct acgggtgagt gttatttctt caatgggacg gagcgggtgc 60
   ggttcctgga cagatacttc tataaccaag aggagtacgt gcgcttcgac agcgacgtgg 120
   gggagtaccg ggcggtgacg gagctggggc ggcctgctgc cgagtactgg aacagccaga 180
   aggacttcct ggaagacagg cgggccctgg tggacaccta ctgcagacac aactacgggg 240
   ttggtgagag cttcacggtg 260
<210> 679
   <211> 270
   <212> DNA
   <213> Homo sapiens
<400> 679
   cacgtttctt ggagtactct acgggtgagt gttatttctt caatgggacg gagcgggtgc 60
   ggttcctgga cagatacttc tataaccaag aggagtacgt gcgcttcgac agcgacgtgg 120
   gggagtaccg ggcggtgacg gagctggggc ggcctagcgc cgagtactgg aacagccaga 180
   aggacatcct ggaagacagg cgggccctgg tggacaccta ctgcagacac aactacgggg 240
   ctgtggagag cttcacagtg cagcggcgag 270
<210> 680
   <211> 242
   <212> DNA
   <213> Homo sapiens
<400> 680
   tcttggagta ctctacgggt gagtgttatt tcttcaatgg gacggagcgg gtgcggttcc 60
   tggacagata cttctataac caagaggagt acgtgcgctt cgacagcgac gtgggggagt 120
   accgggcggt gacggagctg gggcggcctg atgccgagta ctggaacagc cagaaggacc 180
   tcctggaaga caggcgggcc ctggtggaca cctactgcag acacaactac ggggttggtg 240
   ag 242
<210> 681
   <211> 260
   <212> DNA
   <213> Homo sapiens
<400> 681
   cacgtttctt ggagtactct aggggtgagt gttatttctt caatgggacg gagcgggtgc 60
   ggttcctgga cagatacttc tataaccaag aggagtacgt gcgcttcgac agcgacgtgg 120
   gggagtaccg ggcggtgacg gagctggggc ggcctagcgc cgagtactgg aacagccaga 180
   aggacatcct ggaagacagg cgggccctgg tggacaccta ctgcagacac aactacgggg 240
   ttggtgagag cttcacagtg 260
<210> 682
   <211> 242
   <212> DNA
   <213> Homo sapiens
<400> 682
   tttcttggag tactctacgg gtgagtgtta tttcttcaat gggacggagc gggtgcggtt 60
   cctggacaga tacttctata accaagagga gtacgtgcgc ttcgacagcg acgtggggga 120
   gtaccgggcg gtgacggagc tggggcggcc tgatgcggag cactggaaca gccagaagga 180
   catcctggaa gacaggcggg ccctggtgga cacctactgc agacacaact acggggttgg 240
   tg 242
<210> 683
   <211> 270
   <212> DNA
   <213> Homo sapiens
<400> 683
   cacgtttctt ggagtactct acgggtgagt gttatttctt caatgggacg gagcgggtgc 60
   ggttcctgga cagatacttc tataaccaag aggaggacgt gcgcttcgac agcgacgtgg 120
   gggagtaccg ggcggtgacg gagctggggc ggcctagcgc cgagtactgg aacagccaga 180
   aggacttcct ggaagacagg cgggccctgg tggacaccta ctgcagacac aactacgggg 240
   ttggtgagag cttcacggtg cagcggcgag 270
<210> 684
   <211> 246
   <212> DNA
   <213> Homo sapiens
<400> 684
   cacgtttctt ggagtactct acgggtgagt gttatttctt caatgggacg gagcgggtgc 60
   ggttcctgga cagatacttc tataaccaag aggagtacgt gcgcttcgac agcgacgtgg 120
   gggagttccg ggcggtgacg gagctggggc ggcctagcgc cgagtactgg aacagccaga 180
   aggacttcct ggaagacagg cgggccctgg tggacaccta ctgcagacac aactacgggg 240
   ttggtg 246
<210> 685
   <211> 270
   <212> DNA
   <213> Homo sapiens
<400> 685
   cacgtttctt ggagtactct acgggtgagt gttatttctt caatgggacg gagcgggtgc 60
   ggttcctgga cagatacttc tataaccaag aggagtacgt gcgcttcgac agcgacgtgg 120
   gggagtaccg ggcggtgacg gagctggggc ggcctagcgc cgagtactgg aacagccaga 180
   aggacatcct ggaagacagg cgggccgcgg tggacaccta ctgcagacac aactacgggg 240
   ttggtgagag cttcacagtg cagcggcgag 270
<210> 686
   <211> 257
   <212> DNA
   <213> Homo sapiens
<400> 686
   tttcttggag tactctacgg gtgagtgtta tttcttcaat gggacggagc gggtgcggtt 60
   cctggacaga tacttctata accaagagga gtacgtgcgc ttcgacagcg acgtggggga 120
   gtaccgggcg gtgacggagc tggggcggcc tatcgccgag tactggaaca gccagaagga 180
   catcctggaa gacaggcggg ccctggtgga cacctactgc agacacaact acggggttgg 240
   tgagagcttc acagtgc 257
<210> 687
   <211> 269
   <212> DNA
   <213> Homo sapiens
<400> 687
   cacgtttctt ggagtactct acgtctgagt gtcatttctt caatgggacg gagcgggtgc 60
   ggttcctgga cagatacttc tataaccaag aggagtacgt gcgcttcgac agcgacgtgg 120
   gggagtaccg ggcggtgacg gagctggggc ggcctgatgc cgagtactgg aacagccaga 180
   aggacttcct ggaagacagg cgggccctgg tggacaccta ctgcagacac aactacgggg 240
   ttgtggagag cttcacagtg cagcggcga 269
<210> 688
   <211> 269
   <212> DNA
   <213> Homo sapiens
<400> 688
   cacgtttctt ggagtactct atgggtgagt gttatttctt caatgggacg gagcgggtgc 60
   ggttcctgga cagatacttc cataaccagg aggagttcgt gcgcttcgac agcgacgtgg 120
   gggagtaccg ggcggtgacg gagctggggc ggcctgatgc cgagtactgg aacagccaga 180
   aggacttcct ggaagacagg cgggccctgg tggacaccta ctgcagacac aactacgggg 240
   ttggtgagag cttcacggtg cagcggcga 269
<210> 689
   <211> 270
   <212> DNA
   <213> Homo sapiens
<400> 689
   cacgtttctt ggagtactct acgggtgagt gttatttctt caatgggacg gagcgggtgc 60
   ggttcctgga cagatacttc tataaccaag aggagtacgt gcgcttcgac agcgacgtgg 120
   gggagtaccg ggcggtgacg gagctggggc ggcctagcgc cgagtactgg aacagccaga 180
   aggacttcct ggaagacagg cgggccctgg tggacaccta ctgcagacac aactacgggg 240
   ctgtggagag cttcacggtg cagcggcgag 270
<210> 690
   <211> 270
   <212> DNA
   <213> Homo sapiens
<400> 690
   cacgtttctt ggagtactct acgggtgagt gttatttctt caatgggacg gagcgggtgc      60
   ggttcctgga cagatacttc tataaccaag aggagtacgt gcgcttcgac agcgacgtga     120
   gggagtaccg ggcggtgacg gagctggggc ggcctagcgc cgagtactgg aacagccaga     180
   aggacatcct ggaagacagg cgggccctgg tggacaccta ctgcagacac aactacgggg     240
   ttggtgagag cttcacagtg cagcggcgag                                      270
<210> 691
   <211> 266
   <212> DNA
   <213> Homo sapiens
<400> 691
   cacgtttctt ggagtactct acgggtgagt gttatttctt caatgggacg gagcgggtgc      60
   ggttcctgga cagatacttc tataaccaag aggagtacgt gcgcttcgac agcgacgtgg     120
   gggagtaccg ggcggtgacg gagctggggc ggcctgatgc cgagtactgg aacagccaga     180
   aggacttcct ggaagacagg cgggccgcgg tggacaccta ctgcagacac aactacgggg     240
   ttggtgagag cttcacagtg cagcgg                                          266
<210> 692
   <211> 714
   <212> DNA
   <213> Homo sapiens
<400> 692
<210> 693
   <211> 801
   <212> DNA
   <213> Homo sapiens
<400> 693
   ttctatccag gcagcattga agtcaggtgg ttccggaacg gccaggaaga gaagactggg      60
   gtggtgtcca cgggcctgat ccagaatgga gactggacct tccagaccct ggtgatgctg     120
   gaaacagttc ctcagagtgg agaggtttac acctgccaag tggagcaccc aagtgtgatg     180
   agccctctca cagtggaatg gagagcacgg tctgaatctg cacagagcaa gatgctgagt     240
   ggagtcgggg gctttgtgct gggcctgctc ttccttgggg ccgggctgtt cggggacacc     300
   agaccacgtt tcttggagga ggttaagttt gagtgtcatt tcttcaacgg gacggagcgg     360
   gtgcggttgc tggaaagacg cgtccataac caagaggagt acgcgcgcta cgacagcgac     420
   gtgggggagt accgggcggt gacggagctg gggcggcctg atgccgagta ctggaacagc     480
   cagaaggacc tcctggagcg gaggcgtgcc gcggtggaca cctactgcag acacaactac     540
   ggggttggtg agagcttcac agtgcagcgg cgagttcaac ctaaggtgac tgtgatggtg     600
   tgtctgaggc tccctggagg ctcctgcatg gcagttctga cagtgacact gatggtgctg     660
   agctccccac tggctttggc ttatccttca aagacccagc ccctgcagca ccacaacctc     720
   ctggtctgtt ctgtgaatgg tatctacttc aggaatcaga aaggacactc tggacttccg     780
   ccaacaggat tcctgagctg a                                               801
<210> 694
   <211> 270
   <212> DNA
   <213> Homo sapiens
<400> 694
   cacgtttctt ggaggaggtt aagtttgagt gtcatttctt caacgggacg gagcgggtgc      60
   ggttgctgga aagacgcgtc cataaccaag aggagtacgc gcgctacgac agcgacgtgg     120
   gggagtaccg ggcggtgacg gagctggggc ggcctgatgc cgagtactgg aacagccaga     180
   aggacctcct ggagcggagg cgcgccgcgg tggacaccta ctgcagacac aactacgggg     240
   ttggtgagag cttcacagtg cagcggcgag                                      270
<210> 695
   <211> 801
   <212> DNA
   <213> Homo sapiens
<400> 695
   atggtgtgtc tgaggctccc tggaggctcc tgcatggcag ttctgacagt gacactgatg 60
   gtgctgagct ccccactggc tttggctggg gacaccagac cacgtttctt ggagtactct 120
   acgtctgagt gtcatttctt caatgggacg gagcgggtgc ggttcctgga cagatacttc 180
   tataaccaag aggagtacgt gcgcttcgac agcgacgtgg gggagttccg ggcggtgacg 240
   gagctggggc ggcctgatga ggagtactgg aacagccaga aggacttcct ggaagacagg 300
   cgggccgcgg tggacaccta ctgcagacac aactacgggg ttggtgagag cttcacagtg 360
   cagcggcgag tccatcctaa ggtgactgtg tatccttcaa agacccagcc cctgcagcac 420
   cacaacctcc tggtctgttc tgtgagtggt ttctatccag gcagcattga agtcaggtgg 480
   ttccggaatg gccaggaaga gaagactggg gtggtgtcca caggcctgat ccacaatgga 540
   gactggacct tccagaccct ggtgatgctg gaaacagttc ctcggagtgg agaggtttac 600
   acctgccaag tggagcaccc aagcgtgaca agccctctca cagtggaatg gagagcacgg 660
   tctgaatctg cacagagcaa gatgctgagt ggagtcgggg gctttgtgct gggcctgctc 720
   ttccttgggg ccgggctgtt catctacttc aggaatcaga aaggacactc tggacttcag 780
   ccaagaggat tcctgagctg a 801
<210> 696
   <211> 304
   <212> DNA
   <213> Homo sapiens
<400> 696
   ggggacacca gaccacgttt cttggagtac tctacgtctg agtgtcattt cttcaatggg 60
   acggagcggg tgcggttcct ggacagatac ttctataacc aagaggagta cgtgcgcttc 120
   gacagcgacg tgggggagtt ccgggcggtg acggagctgg ggcggcctga tgaggagtac 180
   tggaacagcc agaaggactt cctggaagac aggcgggccg cggtggacac ctactgcaga 240
   cacaactacg gggttggtga gagcttcacg gtgcagcggc gagtccatcc taaggtgact 300
   gtgt 304
<210> 697
   <211> 270
   <212> DNA
   <213> Homo sapiens
<400> 697
   cacgtttctt ggagtactct acgtctgagt gtcatttctt caatgggacg gagcgggtgc 60
   ggttcctgga cagatacttc tataaccaag aggagtacgt gcgcttcgac agcgacgtgg 120
   gggagttccg ggcggtgacg gagctggggc ggcctgatga ggagtactgg aacagccaga 180
   aggacttcct ggaagacagg cgcgccgcgg tggacaccta ctgcagacac aactacgggg 240
   ttggtgagag cttcacagtg cagcggcgag 270
<210> 698
   <211> 268
   <212> DNA
   <213> Homo sapiens

<400> 698
   cgtttcttgg agtactctac gtctgagtgt catttcttca acgggacgga gcgggtgcgg     60
   ttcctggaca gatacttcta taaccaagag gagtacgtgc gcttcgacag cgacgtgggg    120
   gagttccggg cggtgacgga gctggggcgg cctgatgagg agtactggaa cagccagaag    180
   gacttcctgg aagacaggcg ggccgcggtg gacacctact gcagacacaa ctacggggtt    240
   ggtgagagct tcacagtgca gcggcgag                                        268
<210> 699
   <211> 714
   <212> DNA
   <213> Homo sapiens
<400> 699
<210> 700
   <211> 801
   <212> DNA
   <213> Homo sapiens
<400> 700
   atggtgtgtc tgaggctccc tggaggctcc tgcatggcag ttctgacagt gacactgatg 60
   gtgctgagct ccccactggc tttggctggg gacaccagac cacgtttctt ggagtactct 120
   acgtctgagt gtcatttctt caatgggacg gagcgggtgc ggttcctgga cagatacttc 180
   tataaccaag aggagtacgt gcgcttcgac agcgacgtgg gggagttccg ggcggtgacg 240
   gagctggggc ggcctgatga ggagtactgg aacagccaga aggacttcct ggaagacgag 300
   cgggccgcgg tggacaccta ctgcagacac aactacgggg ttgtggagag cttcacagtg 360
   cagcggcgag tccatcctaa ggtgactgtg tatccttcaa agacccagcc cctgcagcac 420
   cacaacctcc tggtctgttc tgtgagtggt ttctatccag gcagcattga agtcaggtgg 480
   ttccggaatg gccaggaaga gaagactggg gtggtgtcca caggcctgat ccacaatgga 540
   gactggacct tccagaccct ggtgatgctg gaaacagttc ctcggagtgg agaggtttac 600
   acctgccaag tggagcaccc aagcgtgaca agccctctca cagtggaatg gagagcacgg 660
   tctgaatctg cacagagcaa gatgctgagt ggagtcgggg gctttgtgct gggcctgctc 720
   ttccttgggg ccgggctgtt catctacttc aggaatcaga aaggacactc tggacttcag 780
   ccaagaggat tcctgagctg a 801
<210> 701
   <211> 282
   <212> DNA
   <213> Homo sapiens
<400> 701
   ggggacacca gaccacgttt cttggagtac tctacgtctg agtgtcattt cttcaatggg 60
   acggagcggg tgcggttcct ggacagatac ttctataacc aagaggagta cgtgcgcttc 120
   gacagcgacg tgggggagtt ccgggcggtg acggagctgg ggcggcctga tgaggagtac 180
   tggaacagcc agaaggactt cctggaagac aggcgggccg cggtggacac ctactgcaga 240
   cacaactacg gggttgtgga gagcttcaca gtgcagcggc ga 282
<210> 702
   <211> 309
   <212> DNA
   <213> Homo sapiens
<400> 702
   ggggacacca gaccacgttt cttggagtac tctacgtctg agtgtcattt cttcaatggg     60
   acggagcggg tgcggttcct ggacagatac ttctataacc aagaggagta cgtgcgcttc    120
   gacagcgacg tgggggagtt ccgggcggtg acggagctgg ggcggcctga tgaggagtac    180
   tggaacagcc agaaggactt cctggaagac aggcgggccg cggtggacac ctactgcaga    240
   cacaactacg gggttgtgga gagcttcacg gtgcagcggc gagtccatcc taaggtgact    300
   gtgtatcct                                                            309
<210> 703
   <211> 270
   <212> DNA
   <213> Homo sapiens
<400> 703
   ccacgtttct tggagtactc tacgggtgag tgtcatttct tcaatgggac ggagcgggtg     60
   cggttcctgg acagatactt ctataaccaa gaggagtacg tgcgcttcga cagcgacgtg    120
   ggggagttcc gggcggtgac ggagctgggg cggcctgatg aggagtactg gaacagccag    180
   aaggacttcc tggaagacag gcgggccgcg gtggacacct actgcagaca caactacggg    240
   gttggtgaga gcttcacagt gcagcggcga                                     270
<210> 704
   <211> 255
   <212> DNA
   <213> Homo sapiens
<400> 704
   cgtttcttgg agtactctac gtctgagtgt catttcttca atgggacgga gcgggtgcgg     60
   ttcctggaca gatacttcta taaccaagag gagtacgtgc gcttcgacag cgacgtgggg    120
   gagttccggg cggtgacgga gctggggcgg cctgatgagg agtactggaa cagccagaag    180
   gacttcctgg aagacaggcg ggccgcggtg gacacctact gcagacacaa ctacggggct    240
   gtggagagct tcaca                                                     255
<210> 705
   <211> 262
   <212> DNA
   <213> Homo sapiens
<400> 705
   ttggagtact ctacgtctga gtgtcatttc ttcaatggga cggagcgggt gcggttcctg 60
   gacagatact tctataacca agaggagtac gtgcgcttcg acagcgacgt gggggagttc 120
   cgggcggtga cggagctggg gcggcctgat gaggagtact ggaacagcca gaaggacctc 180
   ctggagcaga agcggggccg ggtggacaac tactgcagac acaactacgg ggttgtggag 240
   agcttcacag tgcagcggcg ag 262
<210> 706
   <211> 238
   <212> DNA
   <213> Homo sapiens
<400> 706
   gtctgagtgt catttcttca atgggacgga gcgggtgcgg ttcctggaca gatacttcta 60
   taaccaagag gagtacgtgc gcttcgacag cgacgtgggg gagttccggg cggtgacgga 120
   gctggggcgg cctgatgagg agtactggaa cagccagaag gacctcctgg aagacaggcg 180
   ggccgcggtg gacacctact gcagacacaa ctacggggtt ggtgagagct tcacagtg 238
<210> 707
   <211> 238
   <212> DNA
   <213> Homo sapiens
<400> 707
   gtctgagtgt catttcttca atgggacgga gcgggtgcgg ttcctggaca gatacttcta 60
   taaccaagag gagtacgtgc gcttcgacag cgacgtgggg gagttccggg cggtgacgga 120
   gctggggcgg cctgatgagg agtactggaa cagccagaag gacctcctgg aagacaggcg 180
   ggccgcggtg gacacctact gcagacacaa ctacggggtt ggtgagagct tcacggtg 238
<210> 708
   <211> 231
   <212> DNA
   <213> Homo sapiens
<400> 708
   catttcttca atgggacgga gcgggtgcgg ttcctggaca gatacttcca taaccaggag 60
   gagaacgtgc gcttcgacag cgacgtgggg gagttccggg cggtgacgga gctggggcgg 120
   cctgatgagg agtactggaa cagccagaag gacttcctgg aagacaggcg ggccgcggtg 180
   gacacctact gcagacacaa ctacggggtt ggtgagagct tcacagtgca g 231
<210> 709
   <211> 219
   <212> DNA
   <213> Homo sapiens
<400> 709
   gagtgtcatt tcttcaatgg gacggagcgg gtgcggttcc tggacagata cttccataac 60
   caggaggagt tcgtgcgctt cgacagcgac gtgggggagt tccgggcggt gacggagctg 120
   gggcggcctg atgaggagta ctggaacagc cagaaggact tcctggaaga caggcgggcc 180
   gcggtggaca cctactgcag acacaactac ggggttggt 219
<210> 710
   <211> 240
   <212> DNA
   <213> Homo sapiens
<400> 710
   gtctgagtgt catttcttca atgggacgga gcgggtgcgg ttcctggaca gatacttcta 60
   taaccaagag gagtacgtgc gcttcgacag cgacgtgggg gagttccggg cggtgacgga 120
   gctggggcgg cctgatgagg agtactggaa cagccagaag gacttcctgg aagacgagcg 180
   ggccgcggtg gacacctact gcagacacaa ctacggggtt ggtgagagct tcacagtgca 240
<210> 711
   <211> 219
   <212> DNA
   <213> Homo sapiens
<400> 711
   gagtgtcatt tcttcaatgg gacggagcgg gtgcggttcc tggacagata cttctataac 60
   caagaggagt tcgtgcgctt cgacagcgac gtgggggagt tccgggcggt gacggagctg 120
   gggcggcctg atgaggagta ctggaacagc cagaaggact tcctggaaga caggcgggcc 180
   gcggtggaca cctactgcag acacaactac ggggttggt 219
<210> 712
   <211> 270
   <212> DNA
   <213> Homo sapiens
<400> 712
   cacgtttctt ggagtactct acgtctgagt gtcatttctt caatgggacg gagcgggtgc 60
   ggttcctgga cagatacttc tataaccagg aggagttcgt gcgcttcgac agcgacgtgg 120
   gggagttccg ggcggtgacg gagctggggc ggcctgatga ggagtactgg aacagccaga 180
   aggacttcct ggaagacagg cgggccgcgg tggacaccta ctgcagacac aactacgggg 240
   ttggtgagag cttcacagtg cagcggcgag 270
<210> 713
   <211> 282
   <212> DNA
   <213> Homo sapiens
<400> 713
   ggggacacca gaccacgttt cttggagtac tctacgtctg agtgtcattt cttcaatggg 60
   acggagcggg tgcggttcct ggacagatac ttccataacc aggaggagtt cgtgcgcttc 120
   gacagcgacg tgggggagtt ccgggcggtg acggagctgg ggcggcctga tgaggagtac 180
   tggaacagcc agaaggacct cctggagcgg aggcgggccg cggtggacac ctattgcaga 240
   cacaactacg gggttgtgga gagcttcaca gtgcagcggc ga 282
<210> 714
   <211> 279
   <212> DNA
   <213> Homo sapiens
<400> 714
   ggggacacca gaccacgttt cttggagtac tctacgtctg agtgtcattt cttcaatggg 60
   acggagcggg tgcggttcct ggacagatac ttctataacc aagaggagta cgtgcgcttc 120
   gacagcgacg tgggggagtt ccgggcggtg acggagctgg ggcggcctga tgaggagtac 180
   tggaacagcc agaaggacat cctggaagac gagcgggccg cggtggacac ctactgcaga 240
   cacaactacg gggttggtga gagcttcaca gtgcagcgg 279
<210> 715
   <211> 338
   <212> DNA
   <213> Homo sapiens
<400> 715
   ggggacacca gaccacgttt cttggagtac tctacgtctg agtgtcattt cttcaatggg 60
   acggagcggg tgcggttcct ggacagatac ttctataacc aagaggagga cttgcgcttc 120
   gacagcgacg tgggggagtt ccgggcggtg acggagctgg ggcggcctga tgaggagtac 180
   tggaacagcc agaaggactt cctggaagac aggcgggccg cggtggacac ctactgcaga 240
   cacaactacg gggttggtga gagcttcaca gtgcagcggc gagtccatcc taaggtgact 300
   gtgtatcctt caaagaccca gcccctgcag caccacaa 338
<210> 716
   <211> 270
   <212> DNA
   <213> Homo sapiens
<400> 716
   cacgtttctt ggagtactct acgtctgagt gtcatttctt caatgggacg gagcgggtgc 60
   ggttcctgga cagatacttc cataaccagg aggagaacgt gcgcttcgac agcgacgtgg 120
   gggagttccg ggcggtgacg gagctggggc ggcctgatga ggagtactgg aacagccaga 180
   aggacatcct ggaagacgag cgggccgcgg tggacaccta ctgcagacac aactacgggg 240
   ttgtggagag cttcacagtg cagcggcgag 270
<210> 717
   <211> 338
   <212> DNA
   <213> Homo sapiens
<400> 717
   ggggacacca gaccacgttt cttggagtac tctacgtctg agtgtcattt cttcaatggg 60
   acggagcggg tgcggttcct ggacagatac ttccataacc aggaggagtt cgtgcgcttc 120
   gacagcgacg tgggggagta ccgggcggtg acggagctgg ggcggcctga tgaggagtac 180
   tggaacagcc agaaggacct cctggagcgg aggcgggccg aggtggacac ctattgcaga 240
   cacaactacg gggttgtgga gagcttcaca gtgcagcggc gagtccatcc taaggtgact 300
   gtgtatcctt caaagaccca gcccctgcag caccacaa 338
<210> 718
   <211> 266
   <212> DNA
   <213> Homo sapiens
<400> 718
   tttcttggag tactctacgt ctgagtgtca tttcttcaat gggacggagc gggtgcggtt 60
   cctggacaga tacttctata accaagagga gtacgtgcgc ttcgacagcg acgtggggga 120
   gttccgggcg gtgacggagc tggggcggcc tgatgaggag tactggaaca gccagaagga 180
   catcctggaa gacaggcggg ccgcggtgga cacctactgc agacacaact acggggttgt 240
   ggagagcttc acagtgcagc ggcgag 266
<210> 719
   <211> 266
   <212> DNA
   <213> Homo sapiens
<400> 719
   tttcttggag tactctacgt ctgagtgtca tttcttcaat gggacggagc gggtgcggtt 60
   cctggacaga tacttctata accaagagga gtacgtgcgc ttcgacagcg acgtggggga 120
   gttccgggcg gtgacggagc tggggcggcc tgatgaggag tactggaaca gccagaagga 180
   catcctggaa gacaggcggg ccgcggtgga cacctactgc agacacaact acggggttgg 240
   tgagagcttc acagtgcagc ggcgag 266
<210> 720
   <211> 256
   <212> DNA
   <213> Homo sapiens
<400> 720
   ttggagtact ctacgtctga gtgtcatttc ttcaatggga cggagcgggt gcggttcctg 60
   gacagatact tccataacca ggaggagaac gtgcgcttcg acagcgacgt gggggagttc 120
   cgggcggtga cggagctggg gcggcctgat gaggagtact ggaacagcca gaaggacatc 180
   ctggaagacg agcgggccgc ggtggacacc tactgcagac acaactacgg ggttggtgag 240
   agcttcacag tgcagc 256
<210> 721
   <211> 241
   <212> DNA
   <213> Homo sapiens
<400> 721
   ttggagtact ctacgtctga gtgtcatttc ttcaatggga cggagcgggt gcggttcctg 60
   gacagatact tctataacca agaggagtac gtgcgcttcg acagcgacgt gggggagttc 120
   cgggcggtga cggagctggg gcggcctgat gaggagtact ggaacagcca gaaggacatc 180
   ctggaagacg agcgggccgc ggtggacacc tactgcagac acaactacgg ggctgtggag 240
   a 241
<210> 722
   <211> 250
   <212> DNA
   <213> Homo sapiens
<400> 722
   cacgtttctt ggagcaggtt aaacatgagt gtcatttctt caatgggacg gagcgggtgc 60
   ggttcctgga cagatacttc tataaccagg aggagtacgt gcgcttcgac agcgacgtgg 120
   gggagttccg ggcggtgacg gagctggggc ggcctgatga ggagtactgg aacagccaga 180
   aggacttcct ggaagacagg cgggccgcgg tggacaccta ctgcagacac aactacgggg 240
   ttggtgagag 250
<210> 723
   <211> 247
   <212> DNA
   <213> Homo sapiens
<400> 723
   ccacgtttct tggagtactc tacgtctgag tgtcatttct tcaatgggac ggagcgggtg 60
   cggttcctgg acagatactt ctataaccaa gaggagtacg tgcgcttcga cagcgacgtg 120
   ggggagttcc gggcggtgac ggagctgggg cggcctgatg aggagtactg gaacagccag 180
   aaggacttcc tggaagacag gcgggccctg gtggacacct actgcagaca caactacggg 240
   gttggtg 247
<210> 724
   <211> 251
   <212> DNA
   <213> Homo sapiens
<400> 724
   ttcttggagt actctacgtc tgagtgtcat ttcttcaatg ggacggagcg ggtgcggttc 60
   ctggacagat acttctataa ccaagaggag gacgtgcgct tcgacagcga cgtgggggag 120
   ttccgggcgg tgacggagct ggggcggcct gatgaggagt actggaacag ccagaaggac 180
   ttcctggaag acaggcgggc cgcggtggac acctactgca gacacaacta cggggttggt 240
   gagagcttca c 251
<210> 725
   <211> 270
   <212> DNA
   <213> Homo sapiens
<400> 725
   cacgtttctt ggagtactct acgtctgagt gtcatttctt caatgggacg gagcgggtgc 60
   ggttcctgga cagatacttc tataaccaag aggagtacgt gcgcttcgac agcgacgtgg 120
   gggagttccg ggcggtgacg gagctggggc ggcctgatga ggagtactgg aacagccaga 180
   aggacttcct ggaagacagg cgggccctgg tggacaccta ctgcagacac aactacgggg 240
   ttgtggagag cttcacagtg cagcggcgag 270
<210> 726
   <211> 240
   <212> DNA
   <213> Homo sapiens
<400> 726
   ttggagtact ctacgtctga gtgtcatttc ttcaatggga cggagcgggt gcggttcctg 60
   gacagatact tctataacca agaggagtac gtgcgcttcg acagcgacgt gggggagttc 120
   cgggcggtga cggagctggg gcggcctgat gaggagtact ggaacagcca gaaggacctc 180
   ctggagcaga ggcgggccgc ggtggacacc tactgcagac acaactacgg ggttggtgag 240
<210> 727
   <211> 246
   <212> DNA
   <213> Homo sapiens
<400> 727
   tttcttggag tactctacgt ctgagtgtca tttcttcaat gggacggagc gggtgcggtt 60
   cctggacaga tacttctata accaagagga gtacgtgcgc ttcgacagcg acgtggggga 120
   gttccgggcg gtgacggagc tggggcggcc tgatgaggag tactggaaca gccagaagga 180
   cttcctggaa gacaggcggg ccgcggtgga caattactgc agacacaact acggggttgg 240
   tgagag 246
<210> 728
   <211> 270
   <212> DNA
   <213> Homo sapiens
<400> 728
   cacgtttctt ggagtactct acgtctgagt gtcatttctt caatgggacg gagcgggtgc 60
   ggttcctgga cagatacttc tataaccaag aggagtacgt gcgcttcgac agcgacgtgg 120
   gggagttccg ggcggtgacg gagctggggc ggcctgatga ggagtactgg aacagccaga 180
   aggacttcct ggaagacagg cgggccgcgg tggacaacta ctgcagacac aactacgggg 240
   ttggtgagag cttcacagtg cagcggcgag 270
<210> 729
   <211> 270
   <212> DNA
   <213> Homo sapiens
<400> 729
   cacgtttctt ggagtactct acgtctgagt gtcatttctt caatgggacg gagcgggtgc 60
   ggttcctgga cagatacttc tataaccaag aggagaacgt gcgcttcgac agcgacgtgg 120
   gggagttccg ggcggtgacg gagctggggc ggcctgatga ggagtactgg aacagccaga 180
   aggacttcct ggaagacagg cgggccgcgg tggacaccta ctgcagacac aactacgggg 240
   ttggtgagag cttcacagtg cagcggcgag 270
<210> 730
   <211> 270
   <212> DNA
   <213> Homo sapiens
<400> 730
   cacgtttctt ggagtactct acgtctgagt gtcatttctt caatgggacg gagcgggtgc 60
   ggttcctgga cagatacttc tataaccaag aggagtccgt gcgcttcgac agcgacgtgg 120
   gggagttccg ggcggtgacg gagctggggc ggcctgatga ggagtactgg aacagccaga 180
   aggacttcct ggaagacagg cgggccgcgg tggacaccta ctgcagacac aactacgggg 240
   ttggtgagag cttcacagtg cagcggcgag 270
<210> 731
   <211> 269
   <212> DNA
   <213> Homo sapiens
<400> 731
   cacgtttctt ggagctgctt aagtctgagt gtcatttctt caatgggacg gagcgggtgc 60
   ggttcctgga cagatacttc tataaccaag aggagtacgt gcgcttcgac agcgacgtgg 120
   gggagttccg ggcggtgacg gagctggggc ggcctgatga ggagtactgg aacagccaga 180
   aggacttcct ggaagacagg cgggccgcgg tggacaccta ctgcagacac aactacgggg 240
   ttggtgagag cttcacagtg cagcggcga 269
<210> 732
   <211> 360
   <212> DNA
   <213> Homo sapiens
<400> 732
   ggggacacca gaccacgttt cttggagtac tctacgtctg agtgtcattt cttcaatggg 60
   acggagcggg tgcggttcct ggacagatac ttctataacc aagaggagta cgtgcgcttc 120
   gacagcgacg tgggggagtt ccgggcggtg acggagctgg ggcggcctga tgaggagcac 180
   tggaacagcc agaaggacat cctggaagac aggcgggccg cggtggacac ctactgcaga 240
   cacaactacg gggttggtga gagcttcaca gtgcagcggc gagtccatcc taaggtgact 300
   gtgtatcctt caaagaccca gcccctgcag caccacaacc tcctggtctg ttctgtgagt 360
<210> 733
   <211> 270
   <212> DNA
   <213> Homo sapiens
<400> 733
   cacgtttctt ggagtactct acgtctgagt gtcatttctt caatgggacg gagcgggtgc 60
   ggttcctgga cagatacttc tataaccaag aggagtacgt gcgcttcgac agcgacgtgg 120
   gggagttccg ggcggtgacg gagctggggc ggcctgatga ggagtactgg aacagccaga 180
   aggacttcct ggaagacagg cgggccgtgg tggacaccta ctgcagacac aactacgggg 240
   ttggtgagag cttcacagtg cagcggcgag 270
<210> 734
   <211> 259
   <212> DNA
   <213> Homo sapiens
<400> 734
   ttggagtact ctacgtctga gtgtcatttc ttcaatggga cggagcgggt gcggttcctg 60
   gacagatact tctataacca agaggagtac gtgcgcttcg acagcgacgt gggggagttc 120
   cgggcggtga cggagctggg gcggcctgat gaggactact ggaacagcca gaaggacttc 180
   ctggaagaca ggcgggccgc ggtggacacc tactgcagac acaactacgg ggttggtgag 240
   agcttcacag tgcagcggc 259
<210> 735
   <211> 269
   <212> DNA
   <213> Homo sapiens
<400> 735
   cacgtttctt ggagtactct acgtctgagt gtcatttctt caatgggacg gagcgggtgc 60
   ggttcctgga cagatacttc tataaccaag aggagtacgt gcgcttcgac agcgacgtgg 120
   gggagttccg ggcggtgacg gagctggggc ggcctgatga ggagtactgg aacagccaga 180
   aggacctcct ggagcagagg cgggccgcgg tggacaccta ctgcagacac aactacgggg 240
   ttgtggagag cttcacagtg cagcggcga 269
<210> 736
   <211> 259
   <212> DNA
   <213> Homo sapiens
<400> 736
   ttggagtact ctacgtctga gtgtcatttc ttcaatggga cggagcgggt gcggttcctg 60
   gacagatact tctataacca agaggagtac gtgcgcttcg acagcgacgt gggggagttc 120
   cgggcggtga cggagctggg gcggcctgat gaggactact ggaacagcca gaaggacttc 180
   ctggaagaca ggcgggccgc ggtggacacc tactgcagac acaactacgg ggttgtggag 240
   agcttcacag tgcagcggc 259
<210> 737
   <211> 267
   <212> DNA
   <213> Homo sapiens

<400> 737
   cgtttcttgg agtactctac gtctgagtgt catttcttca atgggacgga gcgggtgcgg 60
   ttcctggaca gatacttcta taaccaagag gagtacgtgc gcttcgacag cgacgtgggg 120
   gagttccggg cggtgacgga gctggggcgg cctgatgagg agtactggaa cagccagaag 180
   gacctcctgg aagacgagcg ggccgcggtg gacacctact gcagacacaa ctacggggtt 240
   gtggagagct tcacagtgca gcggcga 267
<210> 738
   <211> 270
   <212> DNA
   <213> Homo sapiens
<400> 738
   cacgtttctt ggagtactct acgtctgagt gtcatttctt caatgggacg gagcgggtgc 60
   ggttcctgga cagatacttc tataaccaag aggagtacgt gcgcttcgac agcgacgtgg 120
   gggagtaccg ggcggtgacg gagctggggc ggcctgatga ggagtactgg aacagccaga 180
   aggacttcct ggaagacagg cgggccgcgg tggacaccta ctgcagacac aactacgggg 240
   ttggtgagag cttcacagtg cagcggcgag 270
<210> 739
   <211> 270
   <212> DNA
   <213> Homo sapiens
<400> 739
   cacgtttctt ggagtactct acgtctgagt gtcatttctt caatgggacg gagcgggtgc 60
   ggttcctgga cagatacttc tataaccaag aggagtacgt gcgcttcgac agcgacgtgg 120
   gggagttccg ggcggtgacg gagctggggc ggcctgatga ggggtactgg aacagccaga 180
   aggacttcct ggaagacagg cgggccgcgg tggacaccta ctgcagacac aactacgggg 240
   ttgtggagag cttcacagtg cagcggcgag 270
<210> 740
   <211> 270
   <212> DNA
   <213> Homo sapiens
<400> 740
   cacgtttctt ggagtactct acgtctgagt gtcatttctt caatgggacg gagcgggtgc 60
   ggttcctgga cagatacttc tataaccaag aggagtacgt gcgcttcgac agcgacgtgg 120
   gggagttccg ggcggtgagg gagctggggc ggcctgatga ggagtactgg aacagccaga 180
   aggacttcct ggaagacagg cgggccgcgg tggacaccta ctgcagacac aactacgggg 240
   ttggtgagag cttcacagtg cagcggcgag 270
<210> 741
   <211> 246
   <212> DNA
   <213> Homo sapiens
<400> 741
   cacgtttctt ggagtactct acgtctgagt gtcatttctt caatgggacg gagcgggtgc 60
   ggttcctgga cagatacttc cataaccagg aggagaacgt gcgcttcgac agcgacgtgg 120
   gggagttccg ggcggtgacg gagctggggc ggcctgatga ggagtactgg aacagccaga 180
   aggacttcct ggaagacgag cgggccgcgg tggacaccta ctgcagacac aactacgggg 240
   ttgtgg 246
<210> 742
   <211> 266
   <212> DNA
   <213> Homo sapiens
<400> 742
   tttcttggag tactctacgt ctgagtgtca tttcttcaat gggacggagc gggtgcggtt 60
   cctggacaga tacttctata accaagagga gtacgtgcgc ttcgacagcg acgtggggga 120
   gtaccgggcg gtgacggagc tggggcggcc tgatgaggag tactggaaca gccagaagga 180
   cttcctggaa gacgagcggg ccgcggtgga cacctactgc agacacaact acggggttgt 240
   ggagagcttc acagtgcagc ggcgag 266
<210> 743
   <211> 270
   <212> DNA
   <213> Homo sapiens
<400> 743
   cacgtttctt ggagtactct acgtctgagt gtcatttctt caatgggacg gagcgggtgc 60
   ggttcctgga cagatacttc tataaccaag aggagtacgt gcgcttcgac agcgacgtgg 120
   gggagttccg ggcggtgacg gagctggggc ggcctgatga ggagtactgg aacagccaga 180
   aggacctcct ggaagacagg cgggccgcgg tggacaccta ctgcagacac aactacgggg 240
   ttgtggagag cttcacagtg cagcggcgag 270
<210> 744
   <211> 801
   <212> DNA
   <213> Homo sapiens
<400> 744
   atggtgtgtc tgaggctccc tggaggctcc tgcatggcag ttctgacagt gacactgatg 60
   gtgctgagct ccccactggc tttggctggg gacaccagac cacgtttctt ggagtactct 120
   acgggtgagt gttatttctt caatgggacg gagcgggtgc ggttactgga gagacacttc 180
   cataaccagg aggagctcct gcgcttcgac agcgacgtgg gggagttccg ggcggtgacg 240
   gagctggggc ggcctgtcgc cgagtcctgg aacagccaga aggacatcct ggaagacagg 300
   cgcgccgcgg tggacaccta ttgcagacac aactacgggg ctgtggagag cttcacagtg 360
   cagcggcgag tccatcctaa ggtgactgtg tatccttcaa agacccagcc cctgcagcac 420
   cacaacctcc tggtctgttc tgtgagtggt ttctatccag gcagcattga agtcaggtgg 480
   ttccggaatg gccaggaaga gaagactggg gtggtgtcca cgggcctgat ccacaatgga 540
   gactggacct tccagaccct ggtgatgctg gaaacagttc ctcggagtgg agaggtttac 600
   acctgccaag tggagcaccc aagcgtgaca agccctctca cagtggaatg gagagcacgg 660
   tctgaatctg cacagagcaa gatgctgagt ggagtcgggg gctttgtgct gggcctgctc 720
   ttccttgggg ccgggctgtt catctacttc aggaatcaga aaggacactc tggacttcag 780
   ccaagaggat tcctgagctg a 801
<210> 745
   <211> 801
   <212> DNA
   <213> Homo sapiens
<400> 745
   atggtgtgtc tgaggctccc tggaggctcc tgcatggcag ttctgacagt gacactgatg 60
   gtgctgagct ccccactggc tttggctggg gacaccagac cacgtttctt ggagtactct 120
   acgggtgagt gttatttctt caatgggacg gagcgggtgc ggttactgga gagacacttc 180
   cataaccagg aggagctcct gcgcttcgac agcgacgtgg gggagttccg ggcggtgacg 240
   gagctggggc ggcctgtcgc cgagtcctgg aacagccaga aggacatcct ggaagacagg 300
   cgggccgcgg tggacaccta ctgcagacac aactacgggg ctgtggagag cttcacagtg 360
   cagcggcgag tccatcctaa ggtgactgtg tatccttcaa agacccagcc cctgcagcac 420
   cacaacctcc tggtctgttc tgtgagtggt ttctatccag gcagcattga agtcaggtgg 480
   ttccggaatg gccaggaaga gaagactggg gtggtgtcca cgggcctgat ccacaatgga 540
   gactggacct tccagaccct ggtgatgctg gaaacagttc ctcggagtgg agaggtttac 600
   acctgccaag tggagcaccc aagcgtgaca agccctctca cagtggaatg gagagcacgg 660
   tctgaatctg cacagagcaa gatgctgagt ggagtcgggg gctttgtgct gggcctgctc 720
   ttccttgggg ccgggctgtt catctacttc aggaatcaga aaggacactc tggacttcag 780
   ccaagaggat tcctgagctg a 801
<210> 746
   <211> 245
   <212> DNA
   <213> Homo sapiens
<400> 746
   cacgtttctt ggagtactct acgggtgagt gttatttctt caatgggacg gagcgggtgc 60
   ggttactgga gagacacttc cataaccagg aggagctcct gcgcttcgac agcgacgtgg 120
   gggagttccg ggcggtgacg gagctggggc ggcctgtcgc cgagtcctgg aacagccaga 180
   aggacttcct ggaagacagg cgcgccgcgg tggacaccta ttgcagacac aactacgggg 240
   ctgtg 245
<210> 747
   <211> 243
   <212> DNA
   <213> Homo sapiens
<400> 747
   ttcttggagt actctacggg tgagtgttat ttcttcaatg ggacggagcg ggtgcggtta 60
   ctggagagac acttccataa ccaggaggag ctcctgcgct tcgacagcga cgtgggggag 120
   ttccgggcgg tgacggagct ggggcggcct gtcgccgagt cctggaacag ccagaaggac 180
   ttcctggaag acaggcgcgc cgcggtggac acctactgca gacacaacta cggggctgtg 240
   gag 243
<210> 748
   <211> 266
   <212> DNA
   <213> Homo sapiens
<400> 748
   cacgtttctt ggagtactct acgggtgagt gttatttctt caatgggacg gagcgggtgc      60
   ggttactgga gagacacttc cataaccagg aggagctcct gcgcttcgac agcgacgtgg     120
   gggagttccg ggcggtgacg gagctggggc ggcctgtcgc cgagtcctgg aacagccaga     180
   aggacatcct ggaagacagg cgcgccgcgg tggacaccta ctgcagacac aactacgggg     240
   ttgtggagag cttcacagtg cagcgg                                          266
<210> 749
   <211> 235
   <212> DNA
   <213> Homo sapiens
<400> 749
   gagtactcta cgggtgagtg ttatttcttc aatgggacgg agcgggtgcg gttactggag      60
   agacacttcc ataaccagga ggagctcctg cgcttcgaca gcgacgtggg ggagttccgg     120
   gcggtgacgg agctggggcg gcctgatgag gagtactgga acagccagaa ggacatcctg     180
   gaagacaggc gcgccgcggt ggacacctat tgcagacaca actacggggc tgtgg          235
<210> 750
   <211> 270
   <212> DNA
   <213> Homo sapiens
<400> 750
   cacgtttctt ggagtactct acgggtgagt gttatttctt caatgggacg gagcgggtgc      60
   ggttactgga gagacacttc cataaccagg aggagttcct gcgcttcgac agcgacgtgg     120
   gggagttccg ggcggtgacg gagctggggc ggcctgtcgc cgagtcctgg aacagccaga     180
   aggacatcct ggaagacagg cgcgccgcgg tggacaccta ttgcagacac aactacgggg     240
   ctgtggagag cttcacagtg cagcggcgag                                      270
<210> 751
   <211> 628
   <212> DNA
   <213> Homo sapiens
<400> 751
   ggggacacca gaccacgttt cttggagtac tctacgggtg agtgttattt cttcaatggg      60
   acggagcggg tgcggttact ggagagacac ttccataacc aggaggagct cctgcgcttc 120
   gacagcgacg tgggggagtt ccgggcggtg acggagctgg ggcggcctgt cgccgagtcc 180
   tggaacagcc agaaggacat cctggaagac aggcgcgccg cggtggacac ctattgcaga 240
   cacaactacg gggctgtgga gagcttcaca gtgcagcggc gagtccatcc taaggtgact 300
   gtgtatcctt caaagaccca gcccctgcag caccacaacc tcctggtctg ttctgtgagt 360
   ggtttctatc caggcagcat tgaagtcagg tggttccgga atggccagga agagaagact 420
   ggggtggtgt ccacgggcct gatccagaat ggagactgga ccttccagac cctggtgatg 480
   ctggaaacag ttcctcggag tggagaggtt tacacctgcc aagtggagca cccaagcgtg 540
   acaagccctc tcacagtgga atggagagca cggtctgaat ctgcacagag caagatgctg 600
   agtggagtcg ggggctttgt gctgggcc 628
<210> 752
   <211> 525
   <212> DNA
   <213> Homo sapiens
<400> 752
   cacgtttctt ggagtactct acgggtgagt gttatttctt caatgggacg gagcgggtgc 60
   ggttactgga gagacacttc cataaccagg aggagctcct gcgcttcgac agcgacgtgg 120
   gggagttccg ggcggtgacg gagctggggc ggcctgtcgc cgagtcctgg aacagccaga 180
   aggacatcct gggagacagg cgcgccgcgg tggacaccta ttgcagacac aactacgggg 240
   ctgtggagag cttcacagtg cagcggcgag tccatcctaa ggtgactgtg tatccttcaa 300
   agacccagcc cctgcagcac cacaacctcc tggtctgttc tgtgagtggt ttctatccag 360
   gcagcattga agtcaggtgg ttccggaatg gccaggaaga gaagactggg gtggtgtcca 420
   cgggcctgat ccacaatgga gactggacct tccagaccct ggtgatgctg gaaacagttc 480
   ctcggagtgg agaggtttac acctgccaag tggagcaccc aagcg 525
<210> 753
   <211> 270
   <212> DNA
   <213> Homo sapiens
<400> 753
   cacgtttctt ggagtactct acgggtgagt gttatttctt caatgggacg gagcgggtgc 60
   ggttcctgga gagacacttc cataaccagg aggagctcct gcgcttcgac agcgacgtgg 120
   gggagttccg ggcggtgacg gagctggggc ggcctgtcgc cgagtcctgg aacagccaga 180
   aggacatcct ggaagacagg cgcgccgcgg tggacaccta ttgcagacac aactacgggg 240
   ctgtggagag cttcacagtg cagcggcgag 270
<210> 754
   <211> 714
   <212> DNA
   <213> Homo sapiens
<400> 754
   ggggacacca gaccacgttt cttggagtac tctacgtctg agtgtcattt cttcaatggg      60
   acggagcggg tgcggttcct ggacagatac ttccataacc aggaggagaa cgtgcgcttc     120
   gacagcgacg tgggggagtt ccgggcggtg acggagctgg ggcggcctga tgccgagtac     180
   tggaacagcc agaaggacat cctggaagac gagcgggccg cggtggacac ctactgcaga     240
   cacaactacg gggttgtgga gagcttcaca gtgcagcggc gagtccatcc taaggtgact     300
   gtgtatcctt caaagaccca gcccctgcag caccacaacc tcctggtctg ttctgtgagt     360
   ggtttctatc caggcagcat tgaagtcagg tggttccgga atggccagga agagaagact     420
   ggggtggtgt ccacaggcct gatccacaat ggagactgga ccttccagac cctggtgatg     480
   ctggaaacag ttcctcggag tggagaggtt tacacctgcc aagtggagca cccaagcgtg     540
   acaagccctc tcacagtgga atggagagca cggtctgaat ctgcacagag caagatgctg     600
   agtggagtcg ggggctttgt gctgggcctg ctcttccttg gggccgggct gttcatctac     660
   ttcaggaatc agaaaggaca ctctggactt cagccaagag gattcctgag ctga           714
<210> 755
   <211> 270
   <212> DNA
   <213> Homo sapiens
<400> 755
   cacgtttctt ggagtactct acgtctgagt gtcatttctt caatgggacg gagcgggtgc      60
   ggttcctgga cagatacttc cataaccagg aggagaacgt gcgcttcgac agcgacgtgg     120
   gggagttccg ggcggtgacg gagctggggc ggcctgatgc cgagtactgg aacagccaga     180
   aggacatcct ggaagacgag cgggctgcgg tggacaccta ctgcagacac aactacgggg     240
   ttgtggagag cttcacagtg cagcggcgag                                      270
<210> 756
   <211> 714
   <212> DNA
   <213> Homo sapiens
<400> 756
   ggggacacca gaccacgttt cttggagtac tctacgtctg agtgtcattt cttcaatggg 60
   acggagcggg tgcggttcct ggacagatac ttccataacc aggaggagaa cgtgcgcttc 120
   gacagcgacg tgggggagtt ccgggcggtg acggagctgg ggcggcctga tgccgagtac 180
   tggaacagcc agaaggacat cctggaagac gagcgggccg cggtggacac ctactgcaga 240
   cacaactacg gggttggtga gagcttcaca gtgcagcggc gagtccatcc taaggtgact 300
   gtgtatcctt caaagaccca gcccctgcag caccacaacc tcctggtctg ttctgtgagt 360
   ggtttctatc caggcagcat tgaagtcagg tggttccgga atggccagga agagaagact 420
   ggggtggtgt ccacaggcct gatccacaat ggagactgga ccttccagac cctggtgatg 480
   ctggaaacag ttcctcggag tggagaggtt tacacctgcc aagtggagca cccaagcgtg 540
   acaagccctc tcacagtgga atggagagca cggtctgaat ctgcacagag caagatgctg 600
   agtggagtcg ggggctttgt gctgggcctg ctcttccttg gggccgggct gttcatctac 660
   ttcaggaatc agaaaggaca ctctggactt cagccaagag gattcctgag ctga 714
<210> 757
   <211> 266
   <212> DNA
   <213> Homo sapiens
<400> 757
   cacgtttctt ggagtactct acgtctgagt gtcatttctt caatgggacg gagcgggtgc 60
   ggttcctgga cagatacttc cataaccagg aggagaacgt gcgcttcgac agcgacgtgg 120
   gggagttccg ggcggtgacg gagctggggc ggcctgatgc cgagtactgg aacagccaga 180
   aggacatcct ggaagacgag cgcgccgcgg tggacaccta ctgcagacac aactacgggg 240
   ttggtgagag cttcacagtg cagcgg 266
<210> 758
   <211> 304
   <212> DNA
   <213> Homo sapiens
<400> 758
   ggggacacca gaccacgttt cttggagtac tctacgtctg agtgtcattt cttcaatggg 60
   acggagcggg tgcggttcct ggacagatac ttctataacc aagaggagta cgtgcgcttc 120
   gacagcgacg tgggggagta ccgggcggtg acggagctgg ggcggcctag cgccgagtac 180
   tggaacagcc agaaggacat cctggaagac aagcgggccg cggtggacac ctactgcaga 240
   cacaactacg gggttggtga gagcttcacg gtgcagcggc gagtccatcc taaggtgact 300
   gtgt 304
<210> 759
   <211> 262
   <212> DNA
   <213> Homo sapiens
<400> 759
   ttggagtact ctacgtctga gtgtcatttc ttcaatggga cggagcgggt gcggttcctg     60
   gacagatact tctataacca agaggagtac gtgcgcttcg acagcgacgt gggggagtac    120
   cgggcggtga cggagctggg gcggcctagc gccgagtact ggaacagcca gaaggacatc    180
   ctggaagaca agcgggccgc ggtggacacc tactgcagac acaactacgg ggttggtgag    240
   agcttcacag tgcagcggcg ag                                             262
<210> 760
   <211> 304
   <212> DNA
   <213> Homo sapiens
<400> 760
   ggggacacca gaccacgttt cttggagtac tctacgtctg agtgtcattt cttcaatggg     60
   acggagcggg tgcggttcct ggacagatac ttctataacc aagaggagta cgtgcgcttc    120
   gacagcgacg tgggggagtt ccgggcggtg acggagctgg ggcggcctag cgccgagtac    180
   tggaacagcc agaaggacat cctggaagac gagcgggccg cggtggacac ctactgcaga    240
   cacaactacg gggttgtgga gagcttcaca gtgcagcggc gagtccatcc taaggtgact    300
   gtgt                                                                 304
<210> 761
   <211> 268
   <212> DNA
   <213> Homo sapiens
<400> 761
   cgtttcttgg agtactctac gtctgagtgt catttcttca atgggacgga gcgggtgcgg     60
   ttcctggaca gatacttcca taaccaggag gagaacgtgc gcttcgacag cgacgtgggg    120
   gagttccggg cggtgacgga gctggggcgg cctgatgccg agtactggaa cagccagaag    180
   gacttcctgg aagacaggcg ggccgcggtg gacacctact gcagacacaa ctacggggtt    240
   ggtgagagct tcacagtgca gcggcgag                                       268
<210> 762
   <211> 228
   <212> DNA
   <213> Homo sapiens
<400> 762
   tgtcatttct tcaatgggac ggagcgggtg cggttcctgg acagatactt ccataaccag     60
   gaggagaacg tgcgcttcga cagcgacgtg ggggagttcc gggcggtgac ggagctgggg    120
   cggcctgatg ccgagtactg gaacagccag aaggacatcc tggaagacag gcgggccgcg    180
   gtggacacct actgcagaca caactacggg gttgtggaga gcttcaca                 228
<210> 763
   <211> 270
   <212> DNA
   <213> Homo sapiens
<400> 763
   cacgtttctt ggagtactct acgtctgagt gtcatttctt caatgggacg gagcgggtgc     60
   ggttcctgga cagatacttc tataaccaag aggagtacgt gcgcttcgac agcgacgtgg    120
   gggagtaccg ggcggtgacg gagctggggc ggcctgatgc cgagtactgg aacagccaga    180
   aggacttcct ggaagacagg cgggccgcgg tggacaccta ctgcagacac aactacgggg    240
   ttggtgagag cttcacagtg cagcggcgag                                     270
<210> 764
   <211> 268
   <212> DNA
   <213> Homo sapiens
<400> 764
   cacgtttctt ggagtactct acgtctgagt gtcatttctt caatgggacg gagcgggtgc     60
   ggttcctgga cagatacttc tataaccaag aggagtacgt gcgcttcgac agcgacgtgg    120
   gggagtaccg ggcggtgacg gagctggggc ggcctgacgc tgagtactgg aacagccaga    180
   aggacttcct ggaagacagg cgggccgcgg tggacaccta ctgcagacac aactacgggg    240
   ttggtgagag cttcacagtg cagcggcg                                       268
<210> 765
   <211> 255
   <212> DNA
   <213> Homo sapiens
<400> 765
   ttcttggagt actctacgtc tgagtgtcat ttcttcaatg ggacggagcg ggtgcggttc 60
   ctggacagat acttccataa ccaggaggag ttcgtgcgct tcgacagcga cgtgggggag 120
   taccgggcgg tgacggagct ggggcggcct gatgccgagt actggaacag ccagaaggac 180
   atcctggaag acgagcgggc cgcggtggac acctactgca gacacaacta cggggttgtg 240
   gagagcttca cagtg 255
<210> 766
   <211> 256
   <212> DNA
   <213> Homo sapiens
<400> 766
   tttcttggag tactctacgt ctgagtgtca tttcttcaat gggacggagc gggtgcggtt 60
   cctggacaga tacttccata accaggagga gaacgtgcgc ttcgacagcg acgtggggga 120
   gttccgggcg gtgacggagc tggggcggcc tgatgccgag tactggaaca gccagaagga 180
   catcctggag caggcgcggg ccgcggtgga cacctactgc agacacaact acggggttgt 240
   ggagagcttc acagtg 256
<210> 767
   <211> 270
   <212> DNA
   <213> Homo sapiens
<400> 767
   cacgtttctt ggagtactct acgtctgagt gtcatttctt caatgggacg gagcgggtgc 60
   ggttcctgga cagatacttc cataaccagg aggagaacgt gcgcttcgac agcgacgtgg 120
   gggagttccg ggcggtgacg gagctggggc ggcctgatgc cgagtactgg aacagccaga 180
   aggacatcct ggaagacaag cgggccgcgg tggacaccta ctgcagacac aactacgggg 240
   ttgtggagag cttcacagtg cagcggcgag 270
<210> 768
   <211> 270
   <212> DNA
   <213> Homo sapiens
<400> 768
   cacgtttctt ggagtactct acgtctgagt gtcatttctt caatgggacg gagcgggtgc 60
   ggttcctgga cagatacttc tataaccaag aggagtacgt gcgcttcgac agcgacgtgg 120
   gggagttccg ggcggtgacg gagctggggc ggcctgatgc cgagtactgg aacagccaga 180
   aggacttcct ggaagacagg cgggccgcgg tggacaccta ctgcagacac aactacgggg 240
   ttgtggagag cttcacagtg cagcggcgag 270
<210> 769
   <211> 270
   <212> DNA
   <213> Homo sapiens
<400> 769
   cacgtttctt ggagtactct acgtctgagt gtcatttctt caatgggacg gagcgggtgc 60
   ggttcctgga cagatacttc tataaccaag aggagtacgt gcgcttcgac agcgacgtgg 120
   gggagtaccg ggcggtgacg gagctggggc ggcctagcgc cgagtactgg aacagccaga 180
   aggacatcct ggaagacagg cgggccgcgg tggacaccta ctgcagacac aactacgggg 240
   ttggtgagag cttcacagtg cagcggcgag 270
<210> 770
   <211> 262
   <212> DNA
   <213> Homo sapiens
<400> 770
   cacgtttctt ggagtactct acgtctgagt gtcatttctt caatgggacg gagcgggtgc 60
   ggttcctgga cagatacttc tataaccaag aggagtacgt gcgcttcgac agcgacgtgg 120
   gggagtaccg ggcggtgacg gagctggggc ggcctagcgc cgagtactgg aacagccaga 180
   aggacatcct ggaagacagg cgggccctgg tggacaccta ctgcagacac aactacgggg 240
   ttggtgagag cttcacagtg ca 262
<210> 771
   <211> 227
   <212> DNA
   <213> Homo sapiens
<400> 771
   tacgtctgag tgtcatttct tcaatgggac ggagcgggtg cggttcctgg acagatactt 60
   ctataaccaa gaggagtacg tgcgcttcga cagcgacgtg ggggagttcc gggcggtgac 120
   ggagctgggg cggcctgatg ccgagtactg gaacagccag aaggacttcc tggaagacag 180
   gcgggccgcg gtggacacct actgcagaca caactacggg gttggtg 227
<210> 772
   <211> 270
   <212> DNA
   <213> Homo sapiens
<400> 772
   cacgtttctt ggagtactct acgtctgagt gtcatttctt caatgggacg gagcgggtgc 60
   ggttcctgga cagatacttc tataaccaag aggagtacgt gcgcttcgac agcgacgtgg 120
   gggagttccg ggcggtgacg gagctggggc ggcctgacgc tgagtactgg aacagccaga 180
   aggacttcct ggaagacagg cgggccgcgg tggacaccta ctgcagacac aactacgggg 240
   ttggtgagag cttcacagtg cagcggcgag 270

<210> 773
   <211> 254
   <212> DNA
   <213> Homo sapiens
<400> 773
   tttcttggag tactctacgt ctgagtgtca tttcttcaat gggacggagc gggtgcggtt 60
   cctggagaga tacttccata accaggagga gaacgtgcgc ttcgacagcg acgtggggga 120
   gttccgggcg gtgacggagc tggggcggcc tgatgccgag tactggaaca gccagaagga 180
   catcctggaa gacgagcggg ccgcggtgga cacctactgc agacacaact acggggttgt 240
   ggagagcttc acag 254
<210> 774
   <211> 247
   <212> DNA
   <213> Homo sapiens
<400> 774
   ggagtactct acgtctgagt gtcatttctt caatgggacg gagcgggtgc ggttcctgga 60
   cagatacttc cataaccagg aggagaacgt gcgcttcgac agcgacgtgg gggagttccg 120
   ggcggtgacg gagctggggc ggcctgatgc cgagtactgg aacagccaga aggacatcct 180
   ggaagacgag cgggccgcgg tggacaccta ctgcagacac aactacgggg ttgatgagag 240
   cttcaca 247
<210> 775
   <211> 343
   <212> DNA
   <213> Homo sapiens
<400> 775
   ggggacacca gaccacgttt cttggagtac tctacgggtg agtgttattt cttcaatggg 60
   acggagcggg tgcggttcct ggacagatac ttctataacc aagaggagta cgtgcgcttc 120
   gacagcgacg tgggggagtt ccgggcggtg acggagctgg ggcggcctga tgccgagtac 180
   tggaacagcc agaaggacat cctggaagac gagcgggccg cggtggacac ctactgcaga 240
   cacaactacg gggttgtgga gagcttcaca gtgcagcggc gagtccatcc taaggtgact 300
   gtgtatcctt caaagaccca gcccctgcag caccacaacc tcc 343
<210> 776
   <211> 270
   <212> DNA
   <213> Homo sapiens
<400> 776
   cacgtttctt ggagtactct acgtctgagt gtcatttctt caatgggacg gagcgggtgc 60
   ggttcctgga cagatacttc cataaccagg aggagaacgt gcgcttcgac agcgacgtgg 120
   gggagttccg ggcggtgacg gagctggggc ggcctgatgc cgagtactgg aacagccaga 180
   aggacttcct ggaagacagg cgggccctgg tggacaccta ctgcagacac aactacgggg 240
   ttgtggagag cttcacagtg cagcggcgag 270
<210> 777
   <211> 326
   <212> DNA
   <213> Homo sapiens
<400> 777
   ggggacacca gaccacgttt cttggagtac tctacgtctg agtgtcattt cttcaatggg 60
   acggagcggg tgcggttcct ggagagatac ttccataacc aggaggagtt cgtgcgcttc 120
   gacagcgacg tgggggagta ccgggcggtg acggagctgg ggcggcctga tgccgagtac 180
   tggaacagcc agaaggacat cctggaagac gagcgggccg cggtggacac ctactgcaga 240
   cacaactacg gggttgtgga gagcttcaca gtgcagcggc gagtccatcc taaggtgact 300
   gtgtatcctt caaagaccca gcccct 326
<210> 778
   <211> 266
   <212> DNA
   <213> Homo sapiens
<400> 778
   cacgtttctt ggagtactct acgtctgagt gtcatttctt caatgggacg gagcgggtgc 60
   ggttcctgga cagatacttc cataaccagg aggagaacgt gcgcttcgac agcgacgtgg 120
   gggagttccg ggcggtgacg gagctggggc ggcctgatgc cgagtactgg aacagccaga 180
   aggacctcct ggaagacgag cgggccgcgg tggacaccta ctgcagacac aactacgggg 240
   ttgtggagag cttcacagtg cagcgg 266
<210> 779
   <211> 359
   <212> DNA
   <213> Homo sapiens
<400> 779
   ggggacacca gaccacgttt cttggagtac tctacgtctg agtgtcattt cttcaatggg 60
   acggagcggg tgcggttcct ggacagatac ttctataacc aagaggagta cgtgcgcttc 120
   gacagcgacg tgggggagtt ccgggcggtg acggagctgg ggcggcctag cgccgagtac 180
   tggaacagcc agaaggactt cctggaagac aggcgggccg cggtggacac ctactgcaga 240
   cacaactacg gggttggtga gagcttcaca gtgcagcggc gagtccatcc taaggtgact 300
   gtgtatcctt caaagaccca gcccctgcag caccacaacc tcctggtctg ttctgtgag 359
<210> 780
   <211> 273
   <212> DNA
   <213> Homo sapiens
<400> 780
   gaccacgttt cttggagtac tctacgtctg agtgtcattt cttcaatggg acggagcggg 60
   tgcggttcct ggacagatac ttctataacc aagaggagta cgtgcgcttc gacagcgacg 120
   tgggggagtt ccgggcggtg acggagctgg ggcggcctga tgccgagtac tggaacagcc 180
   agaaggacat cctggaagac gagcgggccg cggtggacac ctactgcaga cacaactacg 240
   gggttgtgga gagcttcaca gtgcagcggc gag 273
<210> 781
   <211> 265
   <212> DNA
   <213> Homo sapiens
<400> 781
   cgtttcttgg agtactctac gtctgagtgt catttcttca atgggacgga gcgggtgcgg 60
   ttcctggaca gatacttcta taaccaagag gagtacgtgc gcttcgacag cgacgtgggg 120
   gagttccggg cggtgacgga gctggggcgg cctgatgccg agtactggaa cagccagaag 180
   gacatcctgg aagacgagcg ggccgcggtg gacacctact gcagacacaa ctacggggtt 240
   ggtgagagct tcacggtgca gcggc 265
<210> 782
   <211> 265
   <212> DNA
   <213> Homo sapiens
<400> 782
   cgtttcttgg agtactctac gtctgagtgt catttcttca atgggacgga gcgggtgcgg 60
   ttcctggaca gatacttcta taaccaagag gagtacgtgc gcttcgacag cgacgtgggg 120
   gagttccggg cggtgacgga gctggggcgg cctgatgccg agtactggaa cagccagaag 180
   gacttcctgg aagacgagcg ggccgcggtg gacacctact gcagacacaa ctacggggtt 240
   gtggagagct tcacagtgca gcggc 265
<210> 783
   <211> 249
   <212> DNA
   <213> Homo sapiens
<400> 783
   cacgtttctt ggagtactct acgtctgagt gtcatttctt caatgggacg gagcgggtgc 60
   ggttcctgga cagatacttc tataaccaag aggagtacgt gcgcttcgac agcgacgtgg 120
   gggagttccg ggcggtgacg gagctggggc ggcctgatgc cgagtactgg aacagccaga 180
   aggacctcct ggaagacagg cgggccgcgg tggacaccta ctgcagacac aactacgggg 240
   ttggtgaga 249
<210> 784
   <211> 270
   <212> DNA
   <213> Homo sapiens
<400> 784
   cacgtttctt ggagtactct acgtctgagt gtcatttctt caatgggacg gagcgggtgc 60
   ggttcctgga gagatacttc cataaccagg aggagaacgt gcgcttcgac agcgacgtgg 120
   gggagtaccg ggcggtgacg gagctggggc ggcctgacgc tgagtactgg aacagccaga 180
   aggacttcct ggaagacagg cgcgccgcgg tggacaccta ctgcagacac aactacgggg 240
   ttggtgagag cttcacagtg cagcggcgag 270
<210> 785
   <211> 270
   <212> DNA
   <213> Homo sapiens
<400> 785
   cacgtttctt ggagtactct acgtctgagt gtcatttctt caatgggacg gagcgggtgc 60
   ggtacctgga cagatacttc cataaccagg aggagaacgt gcgcttcgac agcgacgtgg 120
   gggagttccg ggcggtgacg gagctggggc ggcctgatgc cgagtactgg aacagccaga 180
   aggacatcct ggaagacgag cgggccgcgg tggacaccta ctgcagacac aactacgggg 240
   ttgtggagag cttcacagtg cagcggcgag 270
<210> 786
   <211> 248
   <212> DNA
   <213> Homo sapiens
<400> 786
   ttggagtact ctacgtctga gtgtcatttc ttcaatggga cggagcgggt gcggttcctg 60
   gacagatact tccataacca ggaggagaac gtgcgcttcg acagcgacgt gggggagttc 120
   cgggcggtga cggagctggg gcggcctgat gccgagtact ggaacagcca gaaggacatc 180
   ctggaagacg agcgggccgc ggtggacacc tactgcagac acaactaccg ggttgtggag 240
   agcttcac 248
<210> 787
   <211> 270
   <212> DNA
   <213> Homo sapiens
<400> 787
   cacgtttctt ggagtactct acgtctgagt gtcatttctt caatgggacg gagcgggtgc 60
   ggttcctgga cagatacttc cataaccagg aggagaacgt gcgcttcgac agcgacgtgg 120
   gggagttccg ggcggtgacg gagctggggc ggcctgatgc cgagtactgg aacagccaga 180
   aggacctcct ggaagacgag cgggccgcgg tggacaccta ctgcagacac aactacgggg 240
   ttggtgagag cttcacagtg cagcggcgag 270
<210> 788
   <211> 253
   <212> DNA
   <213> Homo sapiens
<400> 788
<210> 789
   <211> 269
   <212> DNA
   <213> Homo sapiens
<400> 789
   cacgtttctt ggagtactct acgtctgagt gtcatttctt caatgggacg gagcgggtgc 60
   ggttcctgga cagatacttc cataaccagg aggagaacgt gcgcttcgac agcgacgtgg 120
   gggagttccg ggcggtgacg gagctggggc ggcctgtcgc cgagtactgg aacagccaga 180
   aggacatcct ggaagacgag cgggccgcgg tggacaccta ctgcagacac aactacgggg 240
   ttggtgagag cttcacagtg cagcggcga 269
<210> 790
   <211> 270
   <212> DNA
   <213> Homo sapiens
<400> 790
   cacgtttctt ggagtactct acgtctgagt gtcatttctt caatgggacg gagcgggtgc 60
   ggttcctgga cagatacttc cataaccagg aggagaacgt gcgcttcgac agcgacgtgg 120
   gggagtaccg ggcggtgacg gagctggggc ggcctagcgc cgagtactgg aacagccaga 180
   aggacatcct ggaagacgag cgggccgcgg tggacaccta ctgcagacac aactacgggg 240
   ttgtggagag cttcacagtg cagcggcgag 270
<210> 791
   <211> 246
   <212> DNA
   <213> Homo sapiens
<400> 791
   cacgtttctt ggagtactct acgtctgagt gtcatttctt caatgggacg gagcgggtgc 60
   ggttcctgga cagatacttc tataaccaag aggagtacgt gcgcttcgac agcgacgtgg 120
   gggagtaccg ggcggtgacg gagctggggc ggcctagcgc cgagtactgg aacagccaga 180
   aggacatcct ggaagacaag cgggccgcgg tggacaacta ctgcagacac aactacgggg 240
   ttggtg 246
<210> 792
   <211> 270
   <212> DNA
   <213> Homo sapiens
<400> 792
   cacgtttctt ggagtactct acgtctgagt gtcatttctt caatgggacg gagcgggtgc 60
   ggttcctgga cagatacttc cataaccagg aggagaacct gcgcttcgac agcgacgtgg 120
   gggagttccg ggcggtgacg gagctggggc ggcctgatgc cgagtactgg aacagccaga 180
   aggacatcct ggaagacgag cgggccgcgg tggacaccta ctgcagacac aactacgggg 240
   ttggtgagag cttcacagtg cagcggcgag 270
<210> 793
   <211> 266
   <212> DNA
   <213> Homo sapiens
<400> 793
   cacgtttctt ggagtactct acgtctgagt gtcatttctt caatgggacg gagcgggtgc 60
   ggttcctgga cagatacttc cataaccagg aggagaacgt gcgcttcgac agcgacgtgg 120
   gggagttcct ggcggtgacg gagctggggc ggcctgatgc cgagtactgg aacagccaga 180
   aggacatcct ggaagacgag cgggccgcgg tggacaccta ctgcagacac aactacgggg 240
   ttgtggagag cttcacagtg cagcgg 266
<210> 794
   <211> 270
   <212> DNA
   <213> Homo sapiens
<400> 794
   cacgtttctt ggagtactct acgtctgagt gtcatttctt caatgggacg gagcgggtgc 60
   ggttcctgga cagatacttc cataaccagg aggagaacgt gcgcttcgac agcgacgtgg 120
   gggagtaccg ggcggtgacg gagctggggc ggcctgatgc cgagtactgg aacagccaga 180
   aggacatcct ggaagacgag cgggccgcgg tggacaccta ctgcagacac aactacgggg 240
   ttggtgagag cttcacagtg cagcggcgag 270
<210> 795
   <211> 269
   <212> DNA
   <213> Homo sapiens
<400> 795
   cacgtttctt ggagtactct acgtctgagt gtcatttctt caatgggacg gagcgggtgc 60
   ggttcctgga cagatacttc tataaccaag aggagtacgt gcgcttcgac agcgacgtgg 120
   gggagtaccg ggcggtgacg gagctggggc ggcctgatgc cgagtactgg aacagccaga 180
   aggacatcct ggaagacaag cgggccgcgg tggacaccta ctgcagacac aactacgggg 240
   ttggtgagag cttcacggtg cagcggcga 269
<210> 796
   <211> 245
   <212> DNA
   <213> Homo sapiens
<400> 796
   cacgtttctt ggagtactct acgtctgagt gtcatttctt caatgggacg gagcgggtgc 60
   ggttcctgga cagatacttc tataaccaag aggagtacgt gcgcttcgac agcgacgtgg 120
   gggagtaccg ggcggtgacg gagctggggc ggcctagcgc cgagtactgg aacagccaga 180
   aggacatcct ggaagacgag cgggccgcgg tggacaccta ctgcagacac aactacgggg 240
   ttggt 245
<210> 797
   <211> 270
   <212> DNA
   <213> Homo sapiens
<400> 797
   cacgtttctt ggagtactct acgtctgagt gtcatttctt caatgggacg gagcgggtgc 60
   ggttcctgga cagatacttc cataaccagg aggagaacgt gcgcttcgac agcgacgtgg 120
   gggagttccg ggcggtgacg gagctggggc ggcctgatgc cgagtcctgg aacagccaga 180
   aggacatcct ggaagacgag cgggccgcgg tggacaccta ctgcagacac aactacgggg 240
   ttggtgagag cttcacagtg cagcggcgag 270
<210> 798
   <211> 260
   <212> DNA
   <213> Homo sapiens
<400> 798
   ttggagtact ctacgtctga gtgtcatttc ttcaatggga cggagcgggt gcggttcctg 60
   gacagatact tccataacca ggaggagaac gtgcgcttcg acagcgacgt gggggagtac 120
   cgggcggtga cggagctggg gcggcctgat gccgagtact ggaacagcca gaaggacatc 180
   ctggaagacg agcgggccgc ggtggacacc tactgcagac acaactacgg ggttgtggag 240
   agcttcacag tgcagcggcg 260
<210> 799
   <211> 270
   <212> DNA
   <213> Homo sapiens
<400> 799
   cacgtttctt ggagtactct acgtctgagt gtcatttctt caatgggacg gagcgggtgc 60
   ggtacctgga cagatacttc cataaccagg aggagaacgt gcgcttcgac agcgacgtgg 120
   gggagttccg ggcggtgacg gagctggggc ggcctgatgc cgagtactgg aacagccaga 180
   aggacatcct ggaagacgag cgggccgcgg tggacaccta ctgcagacac aactacgggg 240
   ttggtgagag cttcacagtg cagcggcgag 270
<210> 800
   <211> 270
   <212> DNA
   <213> Homo sapiens
<400> 800
   cacgtttctt ggagtactct acgtctgagt gtcatttctt caatgggacg gagcgggtgc 60
   ggttcctgga cagatacttc cataaccagg aggagaacgt gcgcttcgac agcgacgtgg 120
   gggagttccg ggcggtgacg gagctggggc ggcctgatgc cgagtactgg aacagccaga 180
   aggacttcct ggaagacagg cgggccgcgg tggacaccta ctgcagacac aactacgggg 240
   ttgtggagag cttcacagtg cagcggcgag 270
<210> 801
   <211> 269
   <212> DNA
   <213> Homo sapiens
<400> 801
   cacgtttctt ggagtactct acgtctgagt gtcatttctt caatgggacg gagcgggtgc 60
   ggttcctgga cagatacttc cataaccagg aggagaacgt gcgcttcgac agcgacgtgg 120
   gggagttccg ggcggtgacg gagctggggc ggcctgctgc ggagcactgg aacagccaga 180
   aggacctcct ggaagacgag cgggccgcgg tggacaccta ctgcagacac aactacgggg 240
   ttgtggagag cttcacagtg cagcggcga 269
<210> 802
   <211> 270
   <212> DNA
   <213> Homo sapiens
<400> 802
   cacgtttctt ggagtactct acgtctgagt gtcatttctt caatgggacg gagcgggtgc 60
   ggttcctgga cagatacttc tataaccaag aggagtacgt gcgcttcgac agcgacgtgg 120
   gggagttccg ggcggtgacg gagctggggc ggcctgatgc cgagtactgg aacagccaga 180
   aggacctcct ggagcagagg cgggccgcgg tggacaccta ctgcagacac aactacgggg 240
   ttgtggagag cttcacagtg cagcggcgag 270
<210> 803
   <211> 242
   <212> DNA
   <213> Homo sapiens
<400> 803
   ttggagtact ctacgtctga gtgtcatttc ttcaatggga cggagcgggt gcggttcctg 60
   gacagatact tctataacca agaggagtac gtgcgcttcg acagcgacgt gggggagttc 120
   cgggcggtga cggagctggg gcggcctgct gcggagcact ggaacagcca gaaggacatc 180
   ctggaagacg agcgggccgc ggtggacacc tactgcagac acaactacgg ggttggtgag 240
   ag 242
<210> 804
   <211> 270
   <212> DNA
   <213> Homo sapiens
<400> 804
   cacgtttctt ggagtactct acgtctgagt gtcatttctt caatgggacg gagcgggtgc 60
   ggttcctgga cagatacttc tataaccaag aggagtacgt gcgcttcgac agcgacgtgg 120
   gggagttccg ggcggtgacg gagctggggc ggcctgtcgc cgagtactgg aacagccaga 180
   aggacttcct ggaagacagg cgggccgcgg tggacaccta ctgcagacac aactacgggg 240
   ttggtgagag cttcacagtg cagcggcgag 270
<210> 805
   <211> 270
   <212> DNA
   <213> Homo sapiens
<400> 805
   cacgtttctt ggagtactct acgtctgagt gtcatttctt caatgggacg gagcgggtgc 60
   ggttcctgga cagatacttc tataaccaag aggagtacgt gcgcttcgac agcgacgtgg 120
   gggagtaccg ggcggtgacg gagctggggc ggcctgatgc cgagtactgg aacagccaga 180
   aggacttcct ggaagacagg cgggccctgg tggacaccta ctgcagacac aactacgggg 240
   ttggtgagag cttcacggtg cagcggcgag 270
<210> 806
   <211> 270
   <212> DNA
   <213> Homo sapiens
<400> 806
   cacgtttctt ggagtactct acgtctgagt gtcatttctt caatgggacg gagcgggtgc 60
   ggttcctgga cagatacttc tataaccaag aggagtacgt gcgcttcgac agcgacgtgg 120
   gggagtaccg ggcggtgacg gagctggggc ggcctagcgc cgagtactgg aacagccaga 180
   aggacatcct ggaagacgag cgggccgcgg tggacaccta ctgcagacac aactacgggg 240
   ttgtggagag cttcacagtg cagcggcgag 270
<210> 807
   <211> 266
   <212> DNA
   <213> Homo sapiens
<400> 807
   cacgtttctt ggagtactct acgtctgagt gtcatttctt caatgggacg gagcgggtgc 60
   ggttcctgga cagatacttc tataaccaag aggagtacgt gcgcttcgac agcgacgtgg 120
   gggagtaccg ggcggtgacg gagctggggc ggcctagcgc cgagtactgg aacagccaga 180
   aggacttcct ggaagacagg cgggccgcgg tggacaccta ctgcagacac aactacgggg 240
   ttggtgagag cttcacagtg cagcgg 266
<210> 808
   <211> 282
   <212> DNA
   <213> Homo sapiens
<400> 808
   ggggacacca gaccacgttt cttggagtac tctacgtctg agtgtcattt cttcaatggg 60
   acggagcggg tgcggttcct ggacagatac ttccataacc aggaggagtt cgtgcgcttc 120
   gacagcgacg tgggggagta ccgggcggtg acggagctgg ggcggcctgc tgcggagcac 180
   tggaacagcc agaaggacct cctggagcgg aggcgggccg aggtggacac ctattgcaga 240
   cacaactacg gggttgtgga gagcttcaca gtgcagcggc ga 282
<210> 809
   <211> 246
   <212> DNA
   <213> Homo sapiens
<400> 809
   cacgtttctt ggagtactct acgtctgagt gtcatttctt caatgggacg gagcgggtgc 60
   ggttcctgga cagatacttc cataaccagg aggagttcgt gcgcttcgac agcgacgtgg 120
   gggagtaccg ggcggtgacg gagctggggc ggcctgctgc ggagcactgg aacagccaga 180
   aggacctcct ggagcggagg cgggccgagg tggacaccta ctgcagacac aactacgggg 240
   ttgtgg 246
<210> 810
   <211> 282
   <212> DNA
   <213> Homo sapiens
<400> 810
   ggggacacca gaccacgttt cttggagtac tctacgtctg agtgtcattt cttcaatggg      60
   acggagcggg tgcggttcct ggagagatac ttccataacc aggaggagaa cgtgcgcttc     120
   gacagcgacg tgggggagta ccgggcggtg acggagctgg ggcggcctga tgccgagtac     180
   tggaacagcc agaaggacct cctggagcag aggcgggccg cggtggacac ctactgcaga     240
   cacaactacg gggttggtga gagcttcaca gtgcagcggc ga                        282
<210> 811
   <211> 282
   <212> DNA
   <213> Homo sapiens
<400> 811
   ggggacacca gaccacgttt cttggagtac tctacgtctg agtgtcattt cttcaatggg      60
   acggagcggg tgcggttcct ggagagatac ttccataacc aggaggagaa cgtgcgcttc     120
   gacagcgacg tgggggagta ccgggcggtg acggagctgg ggcggcctga tgccgagtac     180
   tggaacagcc agaaggacct cctggaagac aggcgggccc tggtggacac ctactgcaga     240
   cacaactacg gggttggtga gagcttcaca gtgcagcggc ga                        282
<210> 812
   <211> 270
   <212> DNA
   <213> Homo sapiens
<400> 812
   cacgtttctt ggagtactct acgggtgagt gttatttctt caatgggacg gagcgggtgc      60
   ggttcctgga cagatacttc cataaccagg aggagttcgt gcgcttcgac agcgacgtgg     120
   gggagtaccg ggcggtgacg gagctggggc ggcctgctgc ggagcactgg aacagccaga     180
   aggacctcct ggagcggagg cgggccgagg tggacaccta ttgcagacac aactacgggg     240
   ttgtggagag cttcacagtg cagcggcgag                                    270
<210> 813
   <211> 266
   <212> DNA
   <213> Homo sapiens

<400> 813
   tttcttggag tactctacgt ctgagtgtca attcttcaat gggacggagc gggtgcggtt 60
   cctggacaga tacttccata accaggagga gttcgtgcgc ttcgacagcg acgtggggga 120
   gtaccgggcg gtgacggagc tggggcggcc tgatgctgag tactggaaca gccagaagga 180
   cctcctggag cggaggcggg ccgaggtgga cacctattgc agacacaact acggggttgt 240
   ggagagcttc acagtgcagc ggcgag 266
<210> 814
   <211> 266
   <212> DNA
   <213> Homo sapiens
<400> 814
   tttcttggag tactctacgt ctgagtgtca tttcttcaat gggacggagc gggtgcggtt 60
   cctggagaga tacttccata accaggagga gaacgtgcgc ttcgacagcg acgtggggga 120
   gtaccgggcg gtgacggagc tggggcggcc tgatgccgag tactggaaca gccagaagga 180
   cctcctggag cagaggcggg ccgcggtgga cacctactgc agacacaact acggggttgt 240
   ggagagcttc acagtgcagc ggcgag 266
<210> 815
   <211> 235
   <212> DNA
   <213> Homo sapiens
<400> 815
   gagtactcta cgtctgagtg tcatttcttc aatgggacgg agcgggtgcg gttcctggac 60
   agatacttcc ataaccagga ggagttcgtg cgcttcgaca gcgacgtggg ggagtaccgg 120
   gcggtgacgg agctggggcg gcctgctgcg gagcactgga acagccagaa ggacctcctg 180
   gagcggaggc gggccgaggt ggacacctat tgcagacaca actacggggt tggtg 235
<210> 816
   <211> 261
   <212> DNA
   <213> Homo sapiens
<400> 816
   ttcttggagt actctacgtc tgagtgtcat ttcttcaatg ggacggagcg ggtgcggttc 60
   ctggacagat acttccataa ccaggaggag ttcgtgcgct tcgacagcga cgtgggggag 120
   taccgggcgg tgacggagct ggggcggcct gatgcggagc actggaacag ccagaaggac 180
   ctcctggagc ggaggcgggc cgaggtggac acctattgca gacacaacta cggggttgtg 240
   gagagcttca cagtgcagcg g 261
<210> 817
   <211> 261
   <212> DNA
   <213> Homo sapiens
<400> 817
   ttcttggagt actctacgtc tgagtgtcat ttcttcaatg ggacggagcg ggtgcggttc 60
   ctggacagat acttccataa ccaggaggag aacgtgcgct tcgacagcga cgtgggggag 120
   taccgggcgg tgacggagct ggggcggcct gatgccgagt actggaacag ccagaaggac 180
   ctcctggagc agaggcgggc cgcggtggac acctactgca gacacaacta cggggttggt 240
   gagagcttca cagtgcagcg g 261
<210> 818
   <211> 261
   <212> DNA
   <213> Homo sapiens
<400> 818
   ttcttggagc aggttaaaca tgagtgtcat ttcttcaatg ggacggagcg ggtgcggttc 60
   ctggacagat acttccataa ccaggaggag ttcgtgcgct tcgacagcga cgtgggggag 120
   taccgggcgg tgacggagct ggggcggcct gctgcggagc actggaacag ccagaaggac 180
   ctcctggagc ggaggcgggc cgaggtggac acctattgca gacacaacta cggggttgtg 240
   gagagcttca cagtgcagcg g 261
<210> 819
   <211> 235
   <212> DNA
   <213> Homo sapiens
<400> 819
   gagtactcta cgggtgagtg ttatttcttc aatgggacgg agcgggtgcg gttcctggac 60
   agatacttcc ataaccagga ggagttcgtg cgcttcgaca gcgacgtggg ggagtaccgg 120
   gcggtgacgg agctggggcg gcctgatgag gagtactgga acagccagaa ggacctcctg 180
   gagcggaggc gggccgaggt ggacacctat tgcagacaca actacggggt tgtgg 235
<210> 820
   <211> 224
   <212> DNA
   <213> Homo sapiens
<400> 820
   gtctgagtgt catttcttca atgggacgga gcgggtgcgg ttcctggaga gatacttcca 60
   taaccaggag gagaacgtgc gcttcgacag cgacgtgggg gagtaccggg cggtgacgga 120
   gctggggcgg cctgatgccg agtactggaa cagccagaag gacctcctgg aagacaggcg 180
   ggccctggtg gacacctact gcagacacaa ctacggggtt gtgg 224
<210> 821
   <211> 235
   <212> DNA
   <213> Homo sapiens
<400> 821
   gagtactcta cgtctgagtg tcatttcttc aatgggacgg agcgggtgcg gttcctggag 60
   agatacttcc ataaccagga ggagaacgtg cgcttcgaca gcgacgtggg ggagtaccgg 120
   gcggtgacgg agctggggcg gcctagcgcc gagtactgga acagccagaa ggacctcctg 180
   gagcagaggc gggccgcggt ggacacctac tgcagacaca actacggggt tggtg 235
<210> 822
   <211> 255
   <212> DNA
   <213> Homo sapiens
<400> 822
   ttcttggagt actctacgtc tgagtgtcat ttcttcaatg ggacggagcg ggtgcggttc 60
   ctggacagat acttccataa ccaggaggag ttcgtgcgct tcgacagcga cgtgggggag 120
   taccgggcgg tgacggagct ggggcggcct gatgccgagt actggaacag ccagaaggac 180
   ctcctggagc ggaggcgggc cgaggtggac acctattgca gacacaacta cggggttggt 240
   gagagcttca cagtg 255
<210> 823
   <211> 247
   <212> DNA
   <213> Homo sapiens
<400> 823
   ctctacgggt gagtgttatt tcttcaatgg gacggagcgg gtgcggttcc tggacagata 60
   cttccataac caggaggagt tcgtgcgctt cgacagcgac gtgggggagt accgggcggt 120
   gacggagctg gggcggcctg atgccgagta ctggaacagc cagaaggact tcctggaaga 180
   caggcgggcc ctggtggaca cctactgcag acacaactac ggggttgtgg agagcttcac 240
   agtgcag 247
<210> 824
   <211> 240
   <212> DNA
   <213> Homo sapiens
<400> 824
   ttggagtact ctacgtctga gtgtcatttc ttcaatggga cggagcgggt gcggttcctg 60
   gacagatact tccataacca ggaggagttc gtgcgcttcg acagcgacgt gggggagtac 120
   cgggcggtga cggagctggg gcggcctgct gcggagcact ggaacagcca gaaggacatc 180
   ctggaagacg agcgggccgc ggtggacacc tactgcagac acaactacgg ggttgtggag 240
<210> 825
   <211> 266
   <212> DNA
   <213> Homo sapiens
<400> 825
   cacgtttctt ggagtactct acgtctgagt gtcatttctt caatgggacg gagcgggtgc 60
   ggttcctgga cagatacttc cataaccagg aggagaacgt gcgcttcgac agcgacgtgg 120
   gggagttccg ggcggtgacg gagctggggc ggcctgatgc cgagtactgg aacagccaga 180
   aggacctcct ggagcagagg cgggccgcgg tggacaccta ctgcagacac aactacgggg 240
   ttgtggagag cttcacagtg cagcgg 266
<210> 826
   <211> 258
   <212> DNA
   <213> Homo sapiens
<400> 826
   gagtactcta cgtctgagtg tcatttcttc aatgggacgg agcgggtgcg gttcctggag 60
   agatacttcc ataaccagga ggagaacgtg cgcttcgaca gcgacgtggg ggagtaccgg 120
   gcggtgacgg agctggggcg gcctgatgct gagtactgga acagccagaa ggacctcctg 180
   gagcggaggc gggccgaggt ggacacctat tgcagacaca actacggggt tgtggagagc 240
   ttcacagtgc agcggcga 258
<210> 827
   <211> 270
   <212> DNA
   <213> Homo sapiens
<400> 827
   ggggacacca gaccacgttt cttggagtac tctacgtctg agtgtcattt cttcaatggg 60
   acggagcggg tgcggttcct ggagagatac ttccataacc aggaggagaa cgtgcgcttc 120
   gacagcgacg tgggggagta ccgggcggtg acggagctgg ggcggcctga tgccgagtac 180
   tggaacagcc agaaggacct cctggagcag aagcgggccg cggtggacac ctactgcaga 240
   cacaactacg gggttggtga gagcttcaca 270
<210> 828
   <211> 241
   <212> DNA
   <213> Homo sapiens
<400> 828
   ttggagtact ctacgtctga gtgtcatttc ttcaatggga cggagcgggt gcggttcctg 60
   gagagatact tccataacca ggaggagttc gtgcgcttcg acagcgacgt gggggagtac 120
   cgggcggtga cggagctggg gcggcctgat gccgagtact ggaacagcca gaaggacctc 180
   ctggagcaga ggcgggccgc ggtggacacc tactgcagac acaactacgg ggttgtggag 240
   a 241
<210> 829
   <211> 241
   <212> DNA
   <213> Homo sapiens
<400> 829
   ttggagtact ctacgtctga gtgtcatttc ttcaatggga cggagcgggt gcggttcctg 60
   gacagatact tccataacca ggaggagaac gtgcgcttcg acagcgacgt gggggagttc 120
   cgggcggtga cggagctggg gcggcctgat gccgagtact ggaacagcca gaaggacctc 180
   ctggagcaga agcgggccgc ggtggacacc tactgcagac acaactacgg ggttgtggag 240
   a 241
<210> 830
   <211> 270
   <212> DNA
   <213> Homo sapiens
<400> 830
   cacgtttctt ggagtactct acgtctgagt gtcatttctt caatgggacg gagcgggtgc    60
   ggttcctgga cagatacttc cataaccagg aggagttcgt gcgcttcgac agcgacgtgg   120
   gggagtaccg ggcggtgacg gagctggggc ggcctgctgc ggagcactgg aacagccaga   180
   aggacttcct ggaagacagg cgggccgcgg tggacaccta ctgcagacac aactacgggg   240
   ttggtgagag cttcacagtg cagcggcgag                                    270
<210> 831
   <211> 270
   <212> DNA
   <213> Homo sapiens
<400> 831
   cacgtttctt ggagtactct acgtctgagt gtcatttctt caatgggacg gagcgggtgc    60
   ggttcctgga cagatacttc cataaccagg aggagttcgt gcgcttcgac agcgacgtgg   120
   gggagtaccg ggcggtgacg gagctggggc ggcctgatgc cgagtactgg aacagccaga   180
   aggacctcct ggagcggagg cgggccgagg tggacaccta ttgcagacac aactacgggg   240
   ttgtggagag cttcacagtg cagcggcgag                                    270
<210> 832
   <211> 288
   <212> DNA
   <213> Homo sapiens
<400> 832
   ttcttggagt actctacgtc tgagtgtcat ttcttcaatg ggacggagcg ggtgcggttc    60
   ctggagagat acttccataa ccaggaggag aacgtgcgct tcgacagcga cgtgggggag   120
   taccgggcgg tgacggagct ggggcggcct gatgccgagt actggaacag ccagaaggac   180
   atcctggagc aggcgcgggc cgcggtggac acctactgca gacacaacta cggggttggt   240
   gagagcttca cagtgcagcg gcgagtccat cctaaggtga ctgtgtat                288
<210> 833
   <211> 235
   <212> DNA
   <213> Homo sapiens
<400> 833
   gagtactcta cgtctgagtg tcatttcttc aatgggacgg agcgggtgcg gttcctggac 60
   agatacttct ataaccaaga ggagtacgtg cgcttcgaca gcgacgtggg ggagtaccgg 120
   gcggtgacgg agctggggcg gcctgctgcg gagcactgga acagccagaa ggacttcctg 180
   gaagacaggc gggccgcggt ggacacctac tgcagacaca actacggggt tggtg 235
<210> 834
   <211> 270
   <212> DNA
   <213> Homo sapiens
<400> 834
   cacgtttctt ggagtactct acgtctgagt gtcatttctt caatgggacg gagcgggtgc 60
   agttcctgga cagatacttc cataaccagg aggagttcgt gcgcttcgac agcgacgtgg 120
   gggagtaccg ggcggtgacg gagctggggc ggcctgctgc ggagcactgg aacagccaga 180
   aggacctcct ggagcggagg cgggccgagg tggacaccta ttgcagacac aactacgggg 240
   ttgtggagag cttcacagtg cagcggcgag 270
<210> 835
   <211> 270
   <212> DNA
   <213> Homo sapiens
<400> 835
   cacgtttctt ggagtactct acgtctgagt gtcatttctt caatgggacg gagcgggtgc 60
   ggttcctgga gagatacttc cataaccagg aggagaacgt gcgcttcgac agcgacgtgg 120
   gggagtaccg ggcggtgacg gagctggggc ggcctgatgc cgagtactgg aacagccaga 180
   aggacttcct ggaagacagg cgggccctgg tggacaccta ctgcagacac aactacgggg 240
   ttggtgagag cttcacagtg cagcggcgag 270
<210> 836
   <211> 257
   <212> DNA
   <213> Homo sapiens
<400> 836
   cacgtttctt ggagtactct acgggtgagt gttatttctt caatgggacg gagcgggtgc 60
   ggttcctgga cagatacttc cataaccagg aggagttcgt gcgcttcgac agcgacgtgg 120
   gggagtaccg ggcggtgacg gagctggggc ggcctgctgc ggagcactgg aacagccaga 180
   aggacctcct ggagcggagg cgggccgagg tggacaccta ttgcagacac aactacgggg 240
   ctgtggagag cttcaca 257
<210> 837
   <211> 270
   <212> DNA
   <213> Homo sapiens
<400> 837
   cacgtttctt ggagtactct acgtctgagt gtcatttctt caatgggacg gagcgggtgc 60
   ggttcctgga gagatacttc cataaccagg aggagaacgt gcgcttcgac agcgacgtgg 120
   gggagtaccg ggcggtgacg gagctggggc ggcctgatgc cgagtactgg aacagccaga 180
   aggacctcct ggagcagagg cgggccgcgg tggacaccta ctgcagacac aactacgggg 240
   ctgtggagag cttcacagtg cagcggcgag 270
<210> 838
   <211> 253
   <212> DNA
   <213> Homo sapiens
<400> 838
   tttcttggag tactctacgt ctgagtgtca tttcttcaat gggacggagc gggtgcggtt 60
   cctggacaga tacttccata accaggagga gaacgtgcgc ttcgacagcg acgtggggga 120
   gttccgggcg gtgacggagc tggggcggcc tgatgccgag tactggaaca gccagaagga 180
   cctcctggag cagaggcggg ccgcggtgga cacctactgc agacacaact acggggttgg 240
   tgagagcttc aca 253
<210> 839
   <211> 253
   <212> DNA
   <213> Homo sapiens
<400> 839
   tttcttggag tactctacgg gtgagtgtta tttcttcaat gggacggagc gggtgcggtt 60
   cctggacaga tacttccata accaggagga gttcgtgcgc ttcgacagcg acgtggggga 120
   gtaccgggcg gtgacggagc tggggcggcc tgctgcggag cactggaaca gccagaagga 180
   cctcctggag cggaggcggg ccgcggtgga cacctattgc agacacaact acggggttgt 240
   ggagagcttc aca 253
<210> 840
   <211> 270
   <212> DNA
   <213> Homo sapiens
<400> 840
   cacgtttctt ggagtactct acgtctgagt gtcatttctt caatgggacg gagcgggtgc 60
   ggttcctgga cagatacttc cataaccagg aggagttcgt gcgcttcgac agcgacgtgg 120
   gggagtaccg ggcggtgacg gagctggggc ggcctgctgc ggagcactgg aacagccaga 180
   aggacctcct ggagcggagg cgggccgcgg tggacaccta ctgcagacac aactacgggg 240
   ttgtggagag cttcacagtg cagcggcgag 270
<210> 841
   <211> 259
   <212> DNA
   <213> Homo sapiens
<400> 841
   ttggagtact ctacgtctga gtgtcatttc ttcaatggga cggagcgggt gcggttcctg 60
   gacagatact tccataacca ggaggagaac gtgcgcttcg acagcgacgt gggggagttc 120
   cgggcggtga cggagctggg gcggcctgat gccgagtact ggaacagcca gaaggacctc 180
   ctggagcaga ggcgggccga ggtggacacc tactgcagac acaactacgg ggttgtggag 240
   agcttcacag tgcagcggc 259
<210> 842
   <211> 270
   <212> DNA
   <213> Homo sapiens
<400> 842
   cacgtttctt ggagtactct acgtctgagt gtcatttctt caatgggacg gagcgggtgc 60
   ggttcctgga cagatacttc cataaccagg aggagttcgt gcgcttcgac agcgacgtgg 120
   gggagtaccg ggcggtgacg gagctggggc ggcctgatgc ggagcactgg aacagccaga 180
   aggacctcct ggagcggagg cgggccgcgg tggacaccta ttgcagacac aactacgggg 240
   ttgtggagag cttcacagtg cagcggcgag 270
<210> 843
   <211> 266
   <212> DNA
   <213> Homo sapiens
<400> 843
   cacgtttctt ggagtactct acgtctgagt gtcatttctt caatgggacg gagcgggtgc 60
   ggttcctgga cagatacttc cataaccagg aggagttcgt gcgcttcgac agcgacgtgg 120
   gggagttccg ggcggtgacg gagctggggc ggcctgctgc ggagcactgg aacagccaga 180
   aggacctcct ggagcggagg cgggccgagg tggacaccta ttgcagacac aactacgggg 240
   ttgtggagag cttcacagtg cagcgg 266
<210> 844
   <211> 270
   <212> DNA
   <213> Homo sapiens
<400> 844
   cacgtttctt ggagtactct acgtctgagt gtcatttctt caatgggacg gagcgggtgc 60
   ggttcctgga cagatacttc cataaccagg aggagttcgt gcgcttcgac agcgacgtgc 120
   gggagtaccg ggcggtgacg gagctggggc ggcctgatgc cgagtactgg aacagccaga 180
   aggacctcct ggagcggagg cgggccgagg tggacaccta ttgcagacac aactacgggg 240
   ttggtgagag cttcacagtg cagcggcgag 270
<210> 845
   <211> 270
   <212> DNA
   <213> Homo sapiens
<400> 845
   cacgtttctt ggagtactct acgtctgagt gtcaattctt caatgggacg gagcgggtgc 60
   ggttcctgga cagatacttc cataaccagg aggagttcgt gcgcttcgac agcgacgtgg 120
   gggagtaccg ggcggtgacg gagctggggc ggcctgatgc tgagtactgg aacagccaga 180
   aggacatcct ggagcaggcg cgggccgcgg tggacaccta ctgcagacac aactacgggg 240
   ttgtggagag cttcacagtg cagcggcgag 270
<210> 846
   <211> 270
   <212> DNA
   <213> Homo sapiens
<400> 846
   cacgtttctt ggagtactct acgtctgagt gtcatttctt caatgggacg gagcgggtgc 60
   ggttcctgga cagatacttc cataaccagg aggagttcgt gcgcttcgac agcgacgtgg 120
   gggagtaccg ggcggtgacg gagctggggc ggcctgctgc ggagcactgg aacagccaga 180
   aggacctcct ggagcggagg cgggccgagg tggacaatta ctgcagacac aactacgggg 240
   ttgtggagag cttcacagtg cagcggcgag 270
<210> 847
   <211> 270
   <212> DNA
   <213> Homo sapiens
<400> 847
   cacgtttctt ggagtaccct acgtctgagt gtcatttctt caatgggacg gagcgggtgc 60
   ggttcctgga cagatacttc cataaccagg aggagttcgt gcgcttcgac agcgacgtgg 120
   gggagtaccg ggcggtgacg gagctggggc ggcctgctgc ggagcactgg aacagccaga 180
   aggacctcct ggagcggagg cgggccgagg tggacaccta ttgcagacac aactacgggg 240
   ttgtggagag cttcacagtg cagcggcgag 270
<210> 848
   <211> 270
   <212> DNA
   <213> Homo sapiens
<400> 848
   cacgtttctt ggagtactct acgtctgagt gtcatttctt caatgggacg gagcgggtgc 60
   ggttcctgga gagatacttc cataaccagg aggagttcgt gcgcttcgac agcgacgtgg 120
   gggagtaccg ggcggtgacg gagctggggc ggcctgatgc cgagtactgg aacagccaga 180
   aggacctcct ggaagacagg cgggccctgg tggacaccta ctgcagacac aactacgggg 240
   ttggtgagag cttcacagtg cagcggcgag 270
<210> 849
   <211> 801
   <212> DNA
   <213> Homo sapiens
<400> 849
   atggtgtgtc tgaagctccc tggaggctcc tgcatgacag cgctgacagt gacactgatg 60
   gtgctgagct ccccactggc tttgtctggg gacacccgac cacgtttcct gtggcagcct 120
   aagagggagt gtcatttctt caatgggacg gagcgggtgc ggttcctgga cagatacttc 180
   tataaccagg aggagtccgt gcgcttcgac agcgacgtgg gggagttccg ggcggtgacg 240
   gagctggggc ggcctgacgc tgagtactgg aacagccaga aggacatcct ggagcaggcg 300
   cgggccgcgg tggacaccta ctgcagacac aactacgggg ttgtggagag cttcacagtg 360
   cagcggcgag tccaacctaa ggtgactgta tatccttcaa agacccagcc cctgcagcac 420
   cacaacctcc tggtctgctc tgtgagtggt ttctatccag gcagcattga agtcaggtgg 480
   ttcctgaacg gccaggaaga gaaggctggg atggtgtcca caggcctgat ccagaatgga 540
   gactggacct tccagaccct ggtgatgctg gaaacagttc ctcgaagtgg agaggtttac 600
   acctgccaag tggagcaccc aagcgtgaca agccctctca cagtggaatg gagagcacgg 660
   tctgaatctg cacagagcaa gatgctgagt ggagtcgggg gctttgtgct gggcctgctc 720
   ttccttgggg ccgggctgtt catctacttc aggaatcaga aaggacactc tggacttcag 780
   ccaacaggat tcctgagctg a 801
<210> 850
   <211> 266
   <212> DNA
   <213> Homo sapiens
<400> 850
   cacgtttcct gtggcagcct aagagggagt gtcatttctt caatgggacg gagcgggtgc      60
   ggttcctgga cagatacttc tataaccagg aggagtccgt gcgcttcgac agcgacgtgg     120
   gggagttccg ggcggtgacg gagctggggc ggcctgacgc tgagtactgg aacagccaga     180
   aggacatcct ggagcaggcg cgggccgcgg tggacaccta ctgcagacac aactacggag     240
   ttgtggagag cttcacagtg cagcgg                                          266
<210> 851
   <211> 266
   <212> DNA
   <213> Homo sapiens
<400> 851
   cacgtttcct gtggcagcct aagagggagt gtcatttctt caatgggacg gagcgggtgc      60
   ggttcctgga cagatacttc tataaccagg aggagtccgt gcgcttcgac agcgacgtgg     120
   gggagttccg ggcggtgacg gagctggggc ggcctgacgc tgagtactgg aacagccaga     180
   aggacatcct ggagcaggcg cgggccgcgg tggacaccta ttgcagacac aactacgggg     240
   ttgtggagag cttcacagtg cagcgg                                          266
<210> 852
   <211> 714
   <212> DNA
   <213> Homo sapiens
<400> 852
   ggggacaccc gaccacgttt cctgtggcag cctaagaggg agtgtcattt cttcaatggg      60
   acggagcggg tgcggttcct ggacagatac ttctataacc aggaggagtc cgtgcgcttc     120
   gacagcgacg tgggggagtt ccgggcggtg acggagctgg ggcggcctga cgctgagtac     180
   tggaacagcc agaaggacat cctggagcag gcgcgggccg cggtggacac ctactgcaga     240
   cacaactacg gggttggtga gagcttcaca gtgcagcggc gagtccaacc taaggtgact     300
   gtatatcctt caaagaccca gcccctgcag caccacaacc tcctggtctg ctctgtgagt     360
   ggtttctatc caggcagcat tgaagtcagg tggttcctga acggccagga agagaaggct     420
   gggatggtgt ccacaggcct gatccagaat ggagactgga ccttccagac cctggtgatg     480
   ctggaaacag ttcctcgaag tggagaggtt tacacctgcc aagtggagca cccaagcgtg     540
   acaagccctc tcacagtgga atggagagca cggtctgaat ctgcacagag caagatgctg     600
   agtggagtcg ggggctttgt gctgggcctg ctcttccttg gggccgggct gttcatctac       660
   ttcaggaatc agaaaggaca ctctggactt cagccaacag gattcctgag ctga          714

<210> 853
   <211> 220
   <212> DNA
   <213> Homo sapiens
<400> 853
   gagtgtcatt tcttcaatgg gacggagcgg gtgcggttcc tggacagata cttctataac     60
   caggaggagt ccgtgcgctt cgacagcgac gtgggggagt tccgggcggt gacggagctg    120
   gggcggcctg atgccgagta ctggaacagc cagaaggaca tcctggagca ggcgcgggcc    180
   gcggtggaca cctactgcag acacaactac ggggttggtg                          220
<210> 854
   <211> 246
   <212> DNA
   <213> Homo sapiens
<400> 854
   cacgtttcct gtggcagcct aagagggagt gtcatttctt caatgggacg gagcgggtgc     60
   ggttcctgga cagatacttc tataatcagg aggagtccgt gcgcttcgac agcgacgtgg    120
   gggagttccg ggcggtgacg gagctggggc ggcctgacgc tgagtactgg aacagccaga    180
   aggacatcct ggagcaggcg cgggccgcgg tggacaccta ctgcagacac aactacgggg    240
   ttggtg                                                               246
<210> 855
   <211> 294
   <212> DNA
   <213> Homo sapiens
<400> 855
   ggggacaccc gaccacgttt cctgtggcag cctaagaggg agtgtcattt cttcaatggg     60
   acggagcggg tgcggttcct ggacagacac ttctataacc aggaggagtc cgtgcgcttc    120
   gacagcgacg tgggggagtt ccgggcggtg acggagctgg ggcggcctga cgctgagtac    180
   tggaacagcc agaaggacat cctggagcag gcgcgggccg cggtggacac ctactgcaga    240
   cacaactacg gggttgtgga gagcttcaca gtgcagcggc gagtccaacc taag          294
<210> 856
   <211> 255
   <212> DNA
   <213> Homo sapiens
<400> 856
   ttcctgtggc agcctaagag ggagtgtcat ttcttcaatg ggacggagcg ggtgcggttc 60
   ctggacagat acttctataa ccaggaggag tccgtgcgct tcgacagcga cgtgggggag 120
   ttccgggcgg tgacggagct ggggcggcct gacgctgagt actggaacag ccagaaggac 180
   ttcctggagc aggcgcgggc cgcggtggac acctactgca gacacaacta cggggttgtg 240
   gagagcttca cagtg 255
<210> 857
   <211> 261
   <212> DNA
   <213> Homo sapiens
<400> 857
   ttcctgtggc agcctaagag ggagtgtcat ttcttcaatg ggacggagcg ggtgcggttc 60
   ctggacagat acttctataa ccaggaggag tccgtgcgct tcgacagcga cgtgggggag 120
   ttccgggcgg tgacggagct ggggcggcct gacgctgagt actggaacag ccagaaggac 180
   ctcctggagc aggcgcgggc cgcggtggac acctactgca gacacaacta cggggttgtg 240
   gagagcttca cagtgcagcg g 261
<210> 858
   <211> 242
   <212> DNA
   <213> Homo sapiens
<400> 858
   ctgtggcagc ctaagaggga gtgtcatttc ttcaatggga cggagcgggt gcggttcctg 60
   gacagatact tctataacca ggaggagtcc gtgcgcttcg acagcgacgt gggggagttc 120
   cgggcggcga cggagctggg gcggcctgac gctgagtact ggaacagcca gaaggacatc 180
   ctggagcagg cgcgggccgc ggtggacacc tactgcagac acaactacgg ggttgtggag 240
   ag 242
<210> 859
   <211> 247
   <212> DNA
   <213> Homo sapiens
<400> 859
   tttcctgtgg cagcctaaga gggagtgtca tttcttcaat gggacggagc gggtgcggtt 60
   cctggacaga tacttctata accaggagga gtccgtgcgc ttcgacagcg acgtggggga 120
   gtaccgggcg gtgacggagc tggggcggcc tgacgctgag tactggaaca gccagaagga 180
   catcctggag caggcgcggg ccgcggtgga cacctactgc agacacaact acggggttgt 240
   ggagagc 247
<210> 860
   <211> 270
   <212> DNA
   <213> Homo sapiens
<400> 860
   cacgtttcct gtggcagcct aagagggagt gtcatttctt caatgggacg gagcgggtgc 60
   ggttcctgga cagatacttc tataaccagg aggagtccgt gcgcttcgac agcgacgtgg 120
   gggagttccg ggcggtgacg gagctggggc ggcctgacgc tgagtactgg aacagccaga 180
   agaacatcct ggagcaggcg cgggccgcgg tggacaccta ctgcagacac aactacgggg 240
   ttggtgagag cttcacagtg cagcggcgag 270
<210> 861
   <211> 270
   <212> DNA
   <213> Homo sapiens
<400> 861
   cacgtttcct gtggcagcct aagagggagt gtcatttctt caatgggacg gagcgggtgc 60
   ggttcctgga cagatacttc tataaccagg aggagtccgt gcgcttcgac agcgacgtgg 120
   gggagttcca ggcggtgacg gagctggggc ggcctgacgc tgagtactgg aacagccaga 180
   aggacatcct ggagcaggcg cgggccgcgg tggacaccta ctgcagacac aactacgggg 240
   ttgtggagag cttcacagtg cagcggcgag 270
<210> 862
   <211> 248
   <212> DNA
   <213> Homo sapiens
<400> 862
   gtttcctgtg gcagcctaag agggagtgtc atttcttcaa tgggacggag cgggtgcggt 60
   tcctggacag atacttctat aaccaggagg agtccgtgcg cttcgacagc gacgtggggg 120
   agttccgggc ggtgacggag ctggggcggc ctgacgctga gtactggaac agccagaagg 180
   acatcctgga agacgagcgg gccgcggtgg acacctactg cagacacaac tacggggttg 240
   tggagagc 248
<210> 863
   <211> 270
   <212> DNA
   <213> Homo sapiens
<400> 863
   cacgtttcct gtggcagcct aagagggagt gtcatttctt caatgggacg gagcgggtgc 60
   ggttcctgga cagatacttc tataaccagg aggagtccgt gcgcttcgac agcgacgtgg 120
   gggagtaccg ggcggtgacg gagctggggc ggcctgacgc tgagtactgg aacagccaga 180
   aggacatcct ggagcaggcg cgggccgcgg tggacaccta ctgcagacac aactacgggg 240
   ttggtgagag cttcacagtg cagcggcgag 270
<210> 864
   <211> 801
   <212> DNA
   <213> Homo sapiens
<400> 864
   atggtgtgtc tgaagctccc tggaggctcc tgcatgacag cgctgacagt gacactgatg 60
   gtgctgagct ccccactggc tttggctggg gacacccgac cacgtttcct gtggcagcct 120
   aagagggagt gtcatttctt caatgggacg gagcgggtgc ggttcctgga cagatacttc 180
   tataaccagg aggagtccgt gcgcttcgac agcgacgtgg gggagtaccg ggcggtgacg 240
   gagctggggc ggcctgacgc tgagtactgg aacagccaga aggacttcct ggaagacagg 300
   cgcgccgcgg tggacaccta ctgcagacac aactacgggg ttggtgagag cttcacagtg 360
   cagcggcgag tccaacctaa ggtgactgta tatccttcaa agacccagcc cctgcagcac 420
   cacaacctcc tggtctgctc tgtgagtggt ttctatccag gcagcattga agtcaggtgg 480
   ttcctgaacg gccaggaaga gaaggctggg atggtgtcca caggcctgat ccagaatgga 540
   gactggacct tccagaccct ggtgatgctg gaaacagttc ctcgaagtgg agaggtttac 600
   acctgccaag tggagcaccc aagcgtgaca agccctctca cagtggaatg gagagcacgg 660
   tctgaatctg cacagagcaa gatgctgagt ggagtcgggg gctttgtgct gggcctgctc 720
   ttccttgggg ccgggctgtt catctacttc aggaatcaga aaggacactc tggacttcag 780
   ccaacaggat tcctgagctg a 801
<210> 865
   <211> 255
   <212> DNA
   <213> Homo sapiens
<400> 865
   cgtttcctgt ggcagcctaa gagggagtgt catttcttca atgggacgga gcgggtgcgg    60
   ttcctggaca gatacttcta taaccaggag gagtccgtgc gcttcgacag cgacgtgggg   120
   gagtaccggg cggtgacgga gctggggcgg cctgacgctg agtactggaa cagccagaag   180
   gacttcctgg aagacaggcg ggccgcggtg gacacctact gcagacacaa ctacggggtt   240
   ggtgagagct tcaca                                                    255
<210> 866
   <211> 801
   <212> DNA
   <213> Homo sapiens
<400> 866
   atggtgtgtc tgaagctccc tggaggctcc tgcatgacag cgctgacagt gacactgatg    60
   gtgctgagct ccccactggc tttggctggg gacacccgac cacgtttcct gtggcagcct   120
   aagagggagt gtcatttctt caatgggacg gagcgggtgc ggttcctgga cagatacttc   180
   tataaccagg aggagtccgt gcgcttcgac agcgacgtgg gggagtaccg ggcggtgacg   240
   gagctggggc ggcctgacgc tgagtactgg aacagccaga aggacctcct ggaagacagg   300
   cgcgccgcgg tggacaccta ctgcagacac aactacgggg ttggtgagag cttcacagtg   360
   cagcggcgag tccaacctaa ggtgactgta tatccttcaa agacccagcc cctgcagcac   420
   cacaacctcc tggtctgctc tgtgagtggt ttctatccag gcagcattga agtcaggtgg   480
   ttcctgaacg gccaggaaga gaaggctggg atggtgtcca caggcctgat ccagaatgga   540
   gactggacct tccagaccct ggtgatgctg gaaacagttc ctcgaagtgg agaggtttac   600
   acctgccaag tggagcaccc aagcgtgaca agccctctca cagtggaatg gagagcacgg   660
   tctgaatctg cacagagcaa gatgctgagt ggagtcgggg gctttgtgct gggcctgctc   720
   ttccttgggg ccgggctgtt catctacttc aggaatcaga aaggacactc tggacttcag   780
   ccaacaggat tcctgagctg a                                             801
<210> 867
   <211> 242
   <212> DNA
   <213> Homo sapiens
<400> 867
   tttcctgtgg cagcctaaga gggagtgtca tttcttcaat gggacggagc gggtgcggtt 60
   cctggacaga tacttctata accaggagga gtccgtgcgc ttcgacagcg acgtggggga 120
   gtaccgggcg gtgacggagc tggggcggcc tgacgctgag tactggaaca gccagaagga 180
   cctcctggaa gacaggcggg ccgcggtgga cacctactgc agacacaact acggggttgg 240
   tg 242
<210> 868
   <211> 785
   <212> DNA
   <213> Homo sapiens
<400> 868
   atggtgtgtc tgaagctccc tggaggctcc tgcatgacag cgctgacagt gacactgatg 60
   gtgctgagct ccccactggc tttggctggg gacacccgac cacgtttcct gtggcagcct 120
   aagagggagt gtcatttctt caatgggacg gagcgggtgc ggttcctgga cagatacttc 180
   tataaccagg aggagtccgt gcgcttcgac agcgacgtgg gggagtaccg ggcggtgacg 240
   gagctggggc ggcctgacgc tgagtactgg aacagccaga aggacttcct ggaagacagg 300
   gccgccgcgg tggacaccta ctgcagacac aactacgggg ttggtgagag cttcacagtg 360
   cagcggcgag tccaacctaa ggtgactgta tatccttcaa agacccagcc cctgcagcac 420
   cacaacctcc tggtctgctc tgtgagtggt ttctatccag gcagcattga agtcaggtgg 480
   ttcctgaacg gccaggaaga gaaggctggg atggtgtcca caggcctgat ccagaatgga 540
   gactggacct tccagaccct ggtgatgctg gaaacagttc ctcgaagtgg agaggtttac 600
   acctgccaag tggagcaccc aagcgtgaca agccctctca cagtggaatg gagagcacgg 660
   tctgaatctg cacagagcaa gatgctgagt ggagtcgggg gctttgtgct gggcctgctc 720
   ttccttgggg ccgggctgtt catctacttc aggaatcaga aaggacactc tggacttcag 780
   ccaac 785
<210> 869
   <211> 235
   <212> DNA
   <213> Homo sapiens
<400> 869
   tggcagccta agagggagtg tcatttcttc aatgggacgg agcgggtgcg gttcctggac 60
   agatacttct ataaccagga ggagtccgtg cgcttcgaca gcgacgtggg ggagtaccgg 120
   gcggtgacgg agctggggcg gcctgacgct gagtactgga acagccagaa ggacttcctg 180
   gaagacaggc gggccctggt ggacacctac tgcagacaca actacggggt tggtg 235
<210> 870
   <211> 240
   <212> DNA
   <213> Homo sapiens
<400> 870
   ctgtggcagc ctaagaggga gtgtcatttc ttcaatggga cggagcgggt gcggttcctg      60
   gacagatact tctataacca ggaggagtcc gtgcgcttcg acagcgacgt gggggagtac     120
   cgggcggtga cggagctggg gcggcctgac gctgagtact ggaacagcca gaaggacatc     180
   ctggaagaca ggcgcgccgc ggtggacacc tactgcagac acaactacgg ggttggtgag     240
<210> 871
   <211> 262
   <212> DNA
   <213> Homo sapiens
<400> 871
   cacgtttcct gtggcagcct aagagggagt gtcatttctt caatgggacg gagcgggtgc      60
   ggttcccgga cagatacttc tataaccagg aggagtccgt gcgcttcgac agcgacgtgg     120
   gggagtaccg ggcggtgacg gagctggggc ggcctgacgc tgagtactgg aacagccaga     180
   aggacatcct ggaagacagg cgcgccgcgg tggacaccta ctgcagacac aactacgggg     240
   ttggtgagag cttcacagtg ca                                              262
<210> 872
   <211> 270
   <212> DNA
   <213> Homo sapiens
<400> 872

**Table of Sequence No. referred to name of each gene**

| Seq. No. | Name of Gene | Seq. No. | Name of Gene | Seq. No. | Name of Gene | Seq. No. | Name of Gene |
|---|---|---|---|---|---|---|---|
| 1 | DPB1*01011 | 57 | DPB1*5201 | 113 | DPB1*11012 | 169 | DPB1*6801 |
| 2 | DPB1*01012 | 58 | DPB1*5301 | 114 | DPB1*1301 | 170 | DPB1*6901 |
| 3 | DPB1*02012 | 59 | DPB1*5401 | 115 | DPB1*1401 | 171 | DPB1*7001 |
| 4 | DPB1*02013 | 60 | DPB1*5501 | 116 | DPB1*1501 | 172 | DPB1*7101 |
| 5 | DPB1*02014 | 61 | DPB1*5601 | 117 | DPB1*1601 | 173 | DPB1*7201 |
| 6 | DPB1*0202 | 62 | DPB1*5701 | 118 | DPB1*1701 | 174 | DPB1*7301 |
| 7 | DPB1*03011 | 63 | DPB1*5801 | 119 | DPB1*1801 | 175 | DPB1*7401 |
| 8 | DPB1*03012 | 64 | DPB1*5901 | 120 | DPB1*1901 | 176 | DPB1*7501 |
| 9 | DPB1*0401 | 65 | DPB1*6001 | 121 | DPB1*20011 | 177 | DPB1*7601 |
| 10 | DPB1*0402 | 66 | DPB1*6101N | 122 | DPB1*20012 | 178 | DPB1*7701 |
| 11 | DPB1*0501 | 67 | DPB1*6201 | 123 | DPB1*2101 | 179 | DPB1*7801 |
| 12 | DPB1*0601 | 68 | DPB1*6301 | 124 | DPB1*2201 | 180 | DPB1*7901 |
| 13 | DPB1*0801 | 69 | DPB1*6401N | 125 | DPB1*2301 | 181 | DPB1*8001 |
| 14 | DPB1*0901 | 70 | DPB1*6501 | 126 | DPB1*2401 | 182 | DPB1*8101 |
| 15 | DPB1*1001 | 71 | DPB1*6601 | 127 | DPB1*2501 | 183 | DPB1*8201 |
| 16 | DPB1*11011 | 72 | DPB1*6701 | 128 | DPB1*26011 | 184 | DPB1*8301 |
| 17 | DPB1*11012 | 73 | DPB1*6801 | 129 | DPB1*26012 | 185 | DPB1*8401 |
| 18 | DPB1*1301 | 74 | DPB1*6901 | 180 | DPB1*2701 | 186 | DPB1*8501 |
| 19 | DPB1*1401 | 75 | DPB1*7001 | 131 | DPB1*2801 | 187 | DPB1*8601 |
| 20 | DPB1*1501 | 76 | DPB1*7101 | 132 | DPB1*2901 | 188 | DPB1*8701 |
| 21 | DPB1*1601 | 77 | DPB1*7201 | 133 | DPB1*3001 | 189 | DPB1*8801 |
| 22 | DP81*1701 | 78 | DPB1*7301 | 134 | DPB1*3101 | 190 | DPB1*8901 |
| 23 | DPB1*1801 | 79 | DPB1*7401 | 135 | DPB1*3201 | 191 | DPB1*9001 |
| 24 | DPB1*1901 | 80 | DPB1*7501 | 136 | DPB1*330t | 192 | DPB1*9101 |
| 25 | DPB1*20011 | 81 | DPB1*7601 | 137 | DPB1*3401 | 193 | DQB1*05011 |
| 26 | DPB1*20012 | 82 | DPB1*7701 | 138 | DPB1*3501 | 194 | DQB1*05012 |
| 27 | DPB1*2101 | 83 | DPB1*7801 | 139 | DPB1*3601 | 195 | DQB1*0502 |
| 28 | DPB1*2201 | 84 | DPB1*7901 | 140 | DPB1*3701 | 196 | DQB1*05031 |
| 29 | DPB1*2301 | 85 | DPB1*8001 | 141 | DPB1*3801 | 197 | DQB1*05032 |
| 30 | DPB1*2401 | 86 | DPB1*8101 | 142 | DPB1*3901 | 198 | DQB1*0504 |
| 31 | DPB1*2501 | 87 | DPB1*8201 | 143 | DPB1*4001 | 199 | DQB1*0201 |
| 32 | DPB1*26011 | 88 | DPB1*8301 | 144 | DPB1*4101 | 200 | DQB1*0202 |
| 33 | DPB1*26012 | 89 | DPB1*8401 | 145 | DPB1*4401 | 201 | DQB1*0203 |
| 34 | DPB1*2701 | 90 | DPB1*8501 | 146 | DPB1*4501 | 202 | DQB1*03011 |
| 35 | DPB1*2801 | 91 | DPB1*8601 | 147 | DPB1*4601 | 203 | DQB1*03012 |
| 36 | DPB1*2901 | 92 | DPB1*8701 | 148 | DPB1*4701 | 204 | DQB1*0302 |
| 37 | DPB1*3001 | 93 | DPB1*8801 | 149 | DPB1*480 | 205 | DQB1*03032 |
| 38 | DPB1*3101 | 94 | DPB1*8901 | 150 | DPB1*4901 | 206 | DQB1*03033 |
| 39 | DPB1*3201 | 95 | DPB1*9001 | 151 | DPB1*5001 | 207 | DQB1*0304 |
| 40 | DPB1*3301 | 96 | DPB1*9101 | 152 | DPB1*5101 | 208 | DQB1*03051 |
| 41 | DPB1*3401 | 97 | DPB1*01011 | 153 | DPB1*5201 | 209 | DQB*03052 |
| 42 | DPB1*3501 | 98 | DPB1*01012 | 154 | DPB1*5301 | 210 | DQB1*0306 |
| 43 | DPB1*3601 | 99 | DPB1*02012 | 155 | DPB1*5401 | 211 | DQB1*0307 |
| 44 | DPB1*3701 | 100 | DPB1*02013 | 156 | DPB1*5501 | 212 | DQB1*0308 |
| 45 | DPB1*3801 | 101 | DPB1*02014 | 157 | DPB1*5601 | 213 | DQB1*0309 |
| 46 | DPB1*3901 | 102 | DPB1*0202 | 158 | DPB1*5701 | 214 | DQB1*0310 |
| 47 | DPB1*4001 | 103 | DPB1*03011 | 159 | DPB1*5801 | 215 | DQB1*0401 |
| 48 | DPB1*4101 | 104 | DPB1*03012 | 160 | DPB1*5901 | 216 | DQB1*0402 |
| 49 | DPB1*4401 | 105 | DPB1*0401 | 161 | DPB1*6001 | 217 | DQB1*06011 |
| 50 | DPB1*4501 | 106 | DPB1*0402 | 162 | DPB1*6101N | 218 | DQB1*06012 |
| 51 | DPB1*4601 | 107 | DPB1*0501 | 163 | DPB1*6201 | 219 | DQB1*06013 |
| 52 | DPB1*4701 | 108 | DPB1*0601 | 164 | DPB1*6301 | 220 | DQB1*0602 |
| 53 | DPB1*4801 | 109 | DPB1*0801 | 165 | DPB1*6401N | 221 | DQB1*0603 |
| 54 | DPB1*4901 | 110 | DPB1*0901 | 166 | DPB1*6501 | 222 | DQB1*06041 |
| 55 | DPB1*5001 | 111 | DPB1*1001 | 167 | DPB1*6601 | 223 | DQB*06042 |
| 56 | DPB1*5101 | 112 | DPB1*11011 | 168 | DPB1*6701 | 224 | DQB1*06051 |
| 225 | DQB1*06052 | 281 | DQB1*0613 | 337 | DRB1*0415 | 393 | DRB1*0819 |
| 226 | DQB1*0606 | 282 | DQB1*0614 | 338 | DRB1*041 | 394 | DRB1*0820 |
| 227 | DQB1*0607 | 283 | DQB1*0615 | 339 | DRB1*0417 | 395 | DRB1*0821 |
| 228 | DQB1*0608 | 284 | DQB1*0616 | 340 | DRB1*0418 | 396 | DRB1*0822 |
| 229 | DQB1*0609 | 285 | DQB1*0617 | 341 | DRB1*0419 | 397 | DRB1*0823 |
| 230 | DQB1*0610 | 286 | DQB1*0619 | 342 | DRB1*0420 | 398 | DRB1*0824 |
| 231 | DQB1*06111 | 287 | DRB1*0101 | 343 | DRB1*0421 | 399 | DRB1*09012 |
| 232 | DQB1*06112 | 288 | DRB1*01021 | 344 | DRB1*0422 | 400 | DRB1*10011 |
| 233 | DQB1*0612 | 289 | DRB1*01022 | 345 | DRB1*0423 | 401 | DRB1*10012 |
| 234 | DQB1*0613 | 290 | DRB1*0103 | 346 | DRB1*0424 | 402 | DRB1*11011 |
| 235 | DQB1*0614 | 291 | DRB1*0104 | 347 | DRB1*0425 | 403 | DRB1*11012 |
| 236 | DQB1*0615 | 292 | DRB1*0105 | 348 | DRB1*0426. | 404 | DRB1*11013 |
| 237 | DQB1*0616 | 293 | DRB1*0106 | 349 | DRB1*0427 | 405 | DRB1*11014 |
| 238 | DQB1*0617 | 294 | DRB1*0107 | 350 | DRB1*0428 | 406 | DRB1*1102 |
| 239 | DQB1*0619 | 295 | DRB1*0108 | 351 | DRB1*0429 | 407 | DRB1*1103 |
| 240 | DQB1*05011 | 296 | DRB1*03011 | 352 | DRB1*0430 | 408 | DRB1*11041 |
| 241 | DQB1*05012 | 297 | DRB1*03012 | 353 | DRB1*0431 | 409 | DRB1*11042 |
| 242 | DQB1*0502 | 298 | DRB1*03021 | 354 | DRB1*0432 | 410 | DRB1*1105 |
| 243 | DQB1*05031 | 299 | DRB1*03022 | 355 | DRB1*0433 | 411 | DRB1*1106 |
| 244 | DQB1*05032 | 300 | DRB1*0303 | 356 | DRB1*0434 | 412 | DRB1*1107 |
| 245 | DQB1*0504 | 301 | DRB1*0304 | 357 | DRB1*0435 | 413 | DRB1*11081 |
| 246 | DQB1*0201 | 302 | DRB1*03051 | 358 | DRB1*0436 | 414 | DRB1*11082 |
| 247 | DQB1*0202 | 303 | DRB1*03052 | 359 | DRB1*0437 | 415 | DRB1*1109 |
| 248 | DQB1#0203 | 304 | DRB1*0306 | 360 | ORB1*0438 | 416 | DRB1*1110 |
| 249 | DQB1*03011 | 305 | DRB1*0307 | 361 | DRB1*0439 | 417 | DRB1*1111 |
| 250 | DQB1*03012 | 306 | DRB1*0308 | 362 | DRB1*0440 | 418 | DRB1*11121 |
| 251 | DQB1*0302 | 307 | DRB1*0309 | 363 | ORB1*0441 | 419 | DRB1*11122 |
| 252 | DQB1*03032 | 308 | DRB1*0310 | 364 | DRB1*0442 | 420 | DRB1*1113 |
| 253 | DQB1*03033 | 309 | DRB1*0311 | 365 | DRB1*07011 | 421 | DRB1*1114 |
| 254 | DQB1*0304 | 310 | DRB1*0312 | 366 | DRB1*07012 | 422 | DRB1*1115 |
| 255 | DQB1*03051 | 311 | DRB1*0313 | 367 | DRB1*0703 | 423 | DRB1*1116 |
| 256 | DQB1*03052 | 312 | DRB1*0314 | 368 | DRB1*0704 | 424 | DRB1*1117 |
| 257 | DQB1*0306 | 313 | DRB1*0315 | 369 | DRB1*0705 | 425 | DRB1*1118 |
| 258 | DQB1*0307 | 314 | DRB1*0316 | 370 | DRB1*0801 | 426 | DRB1*1119 |
| 259 | DQHB1*0308 | 315 | DRB1*0317 | 371 | DRB1*08021 | 427 | DRB1*1120 |
| 260 | DQB1*0309 | 316 | DRB1*0318 | 372. | DRB1*08022 | 428 | DRB1*1121 |
| 261 | DQB1*0310 | 317 | DRB1*0319 | 373 | DRB1*08023 | 429 | DRB1*1122 |
| 262 | DQB1*0401 | 318 | DRB1*0320 | 374 | DRB1*08032 | 430 | DRB1*1123 |
| 263 | DQB1*0402 | 319 | DRB1*04011 | 375 | DRB1*08041 | 431 | DRB1*1124 |
| 264 | DQB1*06011 | 320 | DRB1*04012 | 376 | DRB1*08042 | 432 | DRB1*1125 |
| 265 | DQB1*06012 | 321 | DRB1*0402 | 377 | DRB1*08043 | 433 | DRB1*1126 |
| 266 | DQB1*06013 | 322 | BRB1*04031 | 378 | DRB1*08044 | 434 | DRB1*11271 |
| 267 | DQB1*0602 | 323 | DRB1*04032 | 379 | DRB1*805 | 435 | DRB1*11272 |
| 268 | DQB1*0603 | 324 | DRB1*0404 | 380 | DRB1*0806 | 436 | DRB1*1128 |
| 269 | DQB1*06041 | 325 | DRB1*04051 | 381 | DRB1*0807 | 437 | DRB1*1129 |
| 270 | DQB1*06042 | 326 | DRB1*04052 | 382 | DRB1*0808 | 438 | DRB1*1130 |
| 271 | DQB1*06051 | 327 | DRB1*0406 | 383 | DRB1*0809 | 439 | DRB1*1131 |
| 272 | DQB1*06052 | 328 | DR1*04071 | 38 | DRB1*0810 | 440 | DRB1*1132 |
| 273 | DQB1*0606 | 329 | DRB1*04072 | 385 | DRB1*0811 | 441 | DRB1*1133 |
| 274 | DQB1*0607 | 330 | DRB1*0408 | 386 | DRB1*0812 | 442 | ORB1*1134 |
| 275 | DQB1*0608 | 331 | DRB1*0409 | 387 | DRB1*0813 | 443 | DRB1*1135 |
| 276 | DQB1*0609 | 332 | DRB1*0410 | 388 | DRB1*0814 | 444 | DRB1*1136 |
| 277 | DQB1*0610 | 333 | DRB1*0411 | 389 | DRB1*0815 | 445 | DRB1*1137 |
| 278 | DQB1*06111 | 334 | DRB1*0412 | 390 | DRB1*0816 | 446 | DRB1*1138 |
| 279 | DQB1*06112 | 335 | DRB1*0413 | 391 | DRB1*0817 | 447 | DRB1*1139 |
| 280 | DQB1*0612 | 336 | DRB1*0414 | 392 | DRB1*0818 | 448 | DRB1*1140 |
| 449 | DRB1*1141 | 505 | DRB1*1340 | 561 | DRB1*15023 | 617 | DRB1*0404 |
| 450 | DRB1*1142 | 506 | DR1*1341 | 562 | DRB1*1503 | 618 | DRB1*04051 |
| 451 | DRB1*12011 | 507 | DRB1*1342 | 563 | DRB1*1504 | 619 | DRB1*04052 |
| 452 | DRB1*12012 | 508 | DRB1*1343 | 564 | DRB1*1505 | 620 | DRB1*0406 |
| 453 | DRB1*12021 | 509 | DRB1*1344 | 565 | DRB1*1506 | 621 | DRB1*04071 |
| 454 | DRB1*12022 | 510 | DRB1*1345 | 566 | DRB1*1507 | 622 | DRB1*04072 |
| 455 | DRB1*12032 | 511 | DRB1*1346 | 567 | DRB1*1508 | 623 | DRB1*0408 |
| 456 | DRB1*1204 | 512 | DRB1*1347 | 568 | DRB1*1509 | 624 | DRB1*0409 |
| 457 | DRB1*1205 | 513 | DR61*1348 | 569 | BRB1*1510 | 625 | DRB1*0410 |
| 458 | DRB1*1206 | 514 | DRB1*1349 | 570 | DRB1*1511 | 626 | DRB1*0411 |
| 459 | DRB1*1207 | 515 | DRB1*14011 | 571 | DRB1*16011 | 627 | DRB1*0412 |
| 460 | DRB1*1208 | 516 | DRB1*14012 | 572 | DRB1*16012 | 628 | DRB1*0413 |
| 461 | DRB1*13011 | 517 | DRB1*1402 | 573 | DRB1*16021 | 629 | DRB1*0414 |
| 462 | DRB1*13012 | 518 | DRB1*1403 | 574 | DRB1*16022 | 630 | DRB1*0415 |
| 463 | DRB1*13021 | 519 | DRB1*1404 | 575 | DRB1*1603 | 631 | DRB1*0416 |
| 464 | DRB1*13022 | 520 | DRB1*1405 | 576 | DRB1*1604 | 632 | DRB1*0417 |
| 465 | DRB1*13031 | 521 | DRB1*1406 | 577 | DRB1*1605 | 633 | DRB1*0418 |
| 466 | DRB1*13032 | 522 | DRB1*1407 | 578 | DRB1*1607 | 634 | DRB1*0419 |
| 467 | DRB1*1304 | 523 | DRB1*1408 | 579 | DRB1*1608 | 635 | DR61*0420 |
| 468 | DRB1*1305 | 524 | DRB1*1409 | 580 | DRB1*0101 | 636 | DRB1*0421 |
| 469 | DRB1*1306 | 525 | DRB1*1410 | 581 | DRB1*01021 | 637 | DRB1*0422 |
| 470 | DRB1*13071 | 526 | DRB1*1411 | 582 | DRB1*01022 | 638 | DRB1*0423 |
| 471 | DRB1*13072 | 527 | DRB1*1412 | 583 | DRB1*0103 | 639 | DRB1*0424 |
| 472 | DRB1*1308 | 528 | DRB1*1413 | 584 | DRB1*0104 | 640 | DRB1*0425 |
| 473 | DRB1*1309 | 529 | DRB1*1414 | 585 | DRB1*0105 | 641 | DRB1*0426 |
| 474 | DRB1*1310 | 530 | DRB1*1415 | 586 | DRB1*0106 | 642 | DRB1*0427 |
| 475 | DRB1*1311 | 531 | DRB1*1416 | 587 | DRB1*0107 | 643 | DRB1*0428 |
| 476 | DRB1*1312 | 532 | DRB1*1417 | 588 | DRB1*0108 | 644 | DRB1*0429 |
| 477 | DRB1*1313 | 533 | DRB1*1418 | 589 | DRB1*03011 | 645 | DRB1*0430 |
| 478 | DRB1*13141 | 534 | DRB1*1419 | 590 | DRB1*03012 | 646 | DRB1*0431 |
| 479 | DRB1*13142 | 535 | DRB1*1420 | 591 | DRB1*03021 | 647 | DRB1*0432 |
| 480 | DRB1*1315 | 536 | DRB1*1421 | 592 | DRB1*03022 | 648 | DRB1*0433 |
| 481 | DRB1*1316 | 537 | DRB1*1422 | 593 | DRB1*0303 | 649 | DRB1*0434 |
| 482 | DRB1*1317 | 538 | DRB1*1423 | 594 | DRB1*0304 | 650 | DRB1*0435 |
| 483 | DRB1*1318 | 539 | DRB1*1424 | 595 | DRB1*03051 | 651 | DRB1*0436 |
| 484 | DRB1*1319 | 540 | DRB1*1425 | 596 | DRB1*03052 | 652 | DRB1*0437 |
| 485 | DRB1*1320 | 541 | DRB1*1426 | 597 | DRB1*0306 | 653 | DRB1*0438 |
| 486 | DRB1*1321 | 542 | DRB1*1427 | 598 | DRB1*0307 | 654 | DRB1*0439 |
| 487 | DRB1*1322 | 543 | DRB1*1428 | 599 | DRB1*0308 | 655 | DRB1*0440 |
| 488 | DRB1*1323 | 544 | DRB1*1429 | 600 | DRB1*0309 | 656 | DRB1*0441 |
| 489 | DRB1*1324 | 545 | DRB1*1430 | 601 | DRB1*0310 | 657 | DRB1*0442 |
| 490 | DRB1*1325 | 546 | DRB1*1431 | 602 | DRB1*0311 | 658 | DRB1*07011 |
| 491 | DRB1*41326 | 547 | DRB1*1432 | 603 | DRB1*0312 | 659 | DRB1*07012 |
| 492 | DRB1*1327 | 548 | DRB1*1433 | 604 | DRB1*0313 | 660 | DRB1*0703 |
| 493 | DRB1*1328 | 549 | DRB1*1434 | 605 | DRB1*0314 | 661 | DRB1*0704 |
| 494 | DRB1*1329 | 550 | DRB1*1435 | 606 | DRB1*0315 | 662 | DRB1*0705 |
| 495 | DRB1*1330 | 551 | DRB1*1436 | 607 | DRB1*0316 | 663 | DRB1*0801 |
| 496 | DRB1*1331 | 552 | DRB1*1437 | 608 | DRB1*0317 | 664 | DRB1*08021 |
| 497 | DRB1*1332 | 553 | DRB1*1438 | 609 | DRB1*0318 | 665 | DRB1*08022 |
| 498 | DRB1*1333 | 554 | DRB1*1439 | 610 | DRB1*0319 | 666 | DRB1*08023 |
| 499 | DRB1*1334 | 555 | DRB1*1440 | 611 | DRB1*0320 | 667 | DRB1*08032 |
| 500 | DRB1*1335 | 556 | DRB1*15011 | 612 | DRB1*04011 | 668 | DRB1*08041 |
| 501 | DRB1*1336 | 557 | DRB1*15012 | 613 | DRB1*04012 | 669 | DRB1*08042 |
| 502 | DRB1*1337 | 558 | DRB1*15013 | 614 | DRB1*0402 | 670 | DRB1*08043 |
| 503 | DRB1*1338 | 559 | DRB1*15021 | 615 | DRB1*04031 | 671 | DRB1*08044 |
| 504 | DRB1*1339 | 560 | DRB1*15022 | 616 | DRB1*04032 | 672 | DRB1*0805 |
| 673 | DRB1*0806 | 729 | DRB1*1128 | 735 | DRB1*1327 | 841 | DRB1*1433 |
| 674 | DRB1*0807 | 730 | DRB1*1129 | 786 | DRB1*1328 | 842 | DRB1*1434 |
| 675 | DRB1*0808 | 731 | DRB1*1130 | 787 | DRB1*1329 | 843 | DRB1*1435 |
| 676 | DRB1*0809 | 732 | DRB1*1311 | 788 | DRB1*1330 | 844 | DRB1*1436 |
| 677 | DRB1*0810 | 733 | DRB1*1132 | 789 | DRB1*1331 | 845 | DRB1*1437 |
| 678 | DRB1*0811 | 734 | DRB1*1133 | 790 | DRB1*1332 | 846 | DRB1*1438 |
| 679 | DRB1*0812 | 735 | DRB1*1134 | 791 | DRB1*1333 | 847 | DRB1*1439 |
| 680 | DRB1*0813 | 736 | DRB1*1135 | 792 | DRB1*1334 | 848 | DRB1*1440 |
| 681 | DRB1*0814 | 737 | DRB1*1136 | 793 | DRB1*1335 | 849 | DRB1*15011 |
| 682 | DRB1*0815 | 738 | DRB1*1137 | 794 | DRB1*1336 | 850 | DRB1*15012 |
| 683 | DRB1*0816 | 739 | DRB1*1138 | 795 | DRB1*1337 | 851 | DRB1*15013 |
| 684 | DRB1*0817 | 740 | DRB1*1139 | 796 | DRB1*1338 | 852 | DRB1*15021 |
| 685 | DRB1*0818 | 741 | DRB1*1140 | 797 | DRB1*1339 | 853 | DRB1*15022 |
| 686 | DRB1*0819 | 742 | DRB1*1141 | 798 | DRB1*1340 | 854 | DRB1*15023 |
| 687 | DRB1*0820 | 743 | DRB1*1142 | 799 | DRB1*1341 | 855 | DRB1*1503 |
| 688 | DRB1*0821 | 744 | DRB1*12011 | 800 | DRB1*1342 | 856 | DRB1*1504 |
| 689 | DRB1*0822 | 745 | DRB1*12012 | 801 | DRB1*1343 | 857 | DRB1*1505 |
| 690 | DRB1*0823 | 746 | DRB1*12021 | 802 | DRB1*1344 | 858 | DRB1*1506 |
| 691 | DRB1*0824 | 747 | DRB1*12022 | 803 | DRB1*1345 | 859 | DRB1*1507 |
| 692 | DRB1*09012 | 748 | DRB1*12032 | 804 | DRB1*1346 | 860 | DRB1*1508 |
| 693 | DRB1*10011 | 749 | DRB1*1204 | 805 | DRB1*1347 | 861 | DRB1*1509 |
| 694 | DRB1*10012 | 750 | DRB1*1205 | 806 | DRB1*1348 | 862 | DRB1*1510 |
| 695 | DRB1*11011 | 751 | DRB1*1206 | 807 | DRB1*1349 | 863 | DRB1*1511 |
| 696 | DRB1*11012 | 752 | DRB1*1207 | 808 | DRB1*14011 | 864 | DRB1*16011 |
| 697 | DRB1*11013 | 753 | DRB1*1208 | 809 | DRB1*14012 | 865 | DRB1*16012 |
| 698 | DRB1*11014 | 754 | DRB1*13011 | 810 | DRB1*1402 | 866 | DRB1*16021 |
| 699 | DRB1*1102 | 755 | DRB1*13012 | 811 | DRB1*1403 | 867 | DRB1*16022 |
| 700 | DRB1*1103 | 756 | DRB1*13021 | 812 | DRB1*1404 | 868 | DRB1*1603 |
| 701 | DRB1*11041 | 757 | DRB1*13022 | 813 | DRB1*1405 | 869 | DRB1*1604 |
| 702 | DRB1*11042 | 758 | DRB1*13031 | 814 | DRB1*1406 | 870 | DRB1*1605 |
| 703 | DRB1*1105 | 759 | DRB1*13032 | 815 | DRB1*140 | 871 | DRB1*1607 |
| 704 | DRB1*1106 | 760 | DRB1*1304 | 816 | DRB1*1408 | 872 | DRB1*11608 |
| 705 | DRB1*1107 | 761 | DRB1*1305 | 817 | DRB1*1409 | | |
| 706 | DRB1*11081 | 762 | DRB1*1306 | 818 | DRB1*1410 | | |
| 707 | DRB1*11082 | 763 | DRB1*13071 | 819 | DRB1*1411 | | |
| 708 | DRB1*1109 | 764 | DRB1*13072 | 820 | DRB1*1412 | | |
| 709 | DRB1*1110 | 765 | DRB1*1308 | 821 | DRB1*1413 | | |
| 710 | DRB1*1111 | 766 | DRB1*1309 | 822 | DRB1*1414 | | |
| 711 | DRB1*11121 | 767 | DRB1*1310 | 823 | DRB1*1415 | | |
| 712 | DRB1*11122 | 768 | DRB1*1311 | 824 | DRB1*1416 | | |
| 713 | DRB1*1113 | 769 | DRB1*1312 | 825 | DRB1*1417 | | |
| 714 | DRB1*1114 | 770 | DRB1*1313 | 826 | DRB1*1418 | | |
| 715 | DRB1*1115 | 771 | DRB1*13141 | 827 | DRB1*1419 | | |
| 716 | DRB1*1116 | 772 | DRB1*13142 | 828 | DRB1*1420 | | |
| 717 | DRB1*1117 | 773 | DRB1*1315 | 829 | DRB1*1421 | | |
| 718 | DRB1*1118 | 774 | DRB1*1316 | 830 | DRB1*1422 | | |
| 719 | DRB1*1119 | 715 | DRB1*1317 | 831 | DRB1*423 | | |
| 720 | DRB1*1120 | 776 | DRB1*1318 | 832 | DRB1*1424 | | |
| 721 | DRB1*1121 | 777 | DRB1*1319 | 833 | DRB1*1425 | | |
| 722 | DRB1*1122 | 778 | DRB1*1320 | 834 | DRB1*1426 | | |
| 723 | DRB1*1123 | 779 | DRB1*1321 | 835 | DRB1*1427 | | |
| 724 | DRB1*1124 | 780 | DRB1*1322 | 836 | DRB1*1428 | | |
| 725 | DRB1*1125 | 781 | DRB1*1323 | 837 | DRB1*1429 | | |
| 726 | DRB1*1126 | 782 | DRB1*1324 | 838 | DRB1*1430 | | |
| 727 | DRB1*11271 | 783 | DRB1*1325 | 839 | DRB1*1431 | | |
| 728 | DRB1*11272 | 784 | DRB1*1326 | 840 | DRB1*1432 | | |

## Claims

1. A screening method to determine effective stomach cancer curative medicines, wherein amino acid variations of DQB1*gene are used as a marker, and wherein said amino acid variations are at positions 57 and 67 of the amino acid of HLA DQB1*gene, and wherein methods for treatment of the human or animal body by surgery or therapy and diagnostic methods practised on the human or animal body are excluded.

2. The method according to claim 1, wherein said effective cancer treatment medicines are immunological medicines, with the proviso that Asp is encoded at position 57 and Val is encoded at position 67 of the HLA DQB1*gene.

## Patentansprüche

1. Screeningverfahren zur Bestimmung von wirksamen Arzneimitteln zur Heilung von Magenkrebs, wobei Aminosäureänderungen des DQB1*-Gens als Markierung benutzt werden, und wobei sich diese Aminosäureänderungen an den Positionen 57 und 67 der Aminosäure des HLA DQB1*-Gens befinden, und wobei Verfahren zur chirurgischen oder therapeutischen Behandlung des menschlichen oder tierischen Körpers und Diagnostizierverfahren, die am menschlichen oder tierischen Körper vorgenommen werden, ausgeschlossen sind.

2. Verfahren nach Anspruch 1, wobei die wirksamen Arzneimittel zur Behandlung von Krebs immunologische Arzneimittel sind, mit der Maßgabe, dass im HLA DQB1*-Gen an Position 57 Asp codiert ist und an Position 67 Val codiert ist.

## Revendications

1. Procédé de criblage permettant de déterminer des médicaments curatifs efficaces pour le cancer de l'estomac, dans lequel des variations d'acides aminés du gène DQB1* sont utilisées en tant que marqueur, et dans lequel lesdites variations d'acides aminés sont en positions 57 et 67 de la séquence d'acides aminés du gène HLA-DQB1*, et où des procédés de traitement du corps humain ou animal par chirurgie ou thérapie et des procédés de diagnostic pratiqués sur le corps humain ou animal sont exclus.

2. Procédé selon la revendication 1, dans lequel lesdits médicaments thérapeutiques efficaces du cancer sont des médicaments immunologiques, à condition que Asp soit codé en position 57 et Val soit codé en position 67 du gène HLA-DQB1*.
